# FASCICULE DE BREVET EUROPEEN

(11) **EP 1 697 507 B1**
(45) Date de publication et mention de la délivrance du brevet: **19.09.2012**
(21) Numéro de dépôt: 04805624.6
(22) Date de dépôt: 02.12.2004
(51) Int. Cl.: C12N 7/00, C07K 14/165, G01N 33/569

(54) **UTILISATION DES PROTEINES ET DES PEPTIDES CODES PAR LE GENOME D'UNE NOUVELLE SOUCHE DE CORONAVIRUS ASSOCIE AU SRAS**
VERWENDUNG VON PROTEINEN UND PEPTIDEN, KODIERT IM GENOM EINES NEUEN MIT SARS VERBUNDENEN CORONAVIRUS-STAMMES
USE OF PROTEINS AND PEPTIDES CODED BY THE GENOME OF A NOVEL STRAIN OF SARS-ASSOCIATED CORONAVIRUS

(30) Priorité: 02.12.2003 FR 0314152; 02.12.2003 FR 0314151
(43) Date de publication de la demande: 06.09.2006
(60) Demande divisionnaire: 10012268.8
(73) Titulaire: INSTITUT PASTEUR, 75724 Paris Cedex 15 (FR); CENTRE NATIONAL DE LA RECHERCHE SCIENTIFIQUE (CNRS), 75794 Paris Cedex 16 (FR); UNIVERSITE PARIS VII, F-75251 Paris Cedex 05 (FR)
(72) Inventeur: VAN DER WERF, Sylvie, F-91190 Gif-sur-yvette (FR); ESCRIOU, Nicolas, F-75014 Paris (FR); CRESCENZO-CHAIGNE, Bernadette, F-92200 Neuilly-sur-seine (FR); MANUGUERRA, Jean-Claude, F-75018 Paris (FR); KUNST, Frederik, Institut Pasteur, 25-28 rue Dr. Roux 75724 Cedex 15 Paris (FR); CALLENDRET, Benoît, 92000 Nanterre (FR); BETTON, Jean-Michel, F-75014 Paris (FR); LORIN, Valérie, F-92120 Montrouge (FR); GERBAUD, Sylvie, F-94100 Saint Maur Des Fosses (FR); BURGUIERE, Ana Maria, F-92140 Clamart (FR); AZEBI, Saliha, F-94400 Vitry-sur-seine (FR); CHARNEAU, Pierre, F-75005 Paris (FR); TANGY, Frédéric, F-93260 Les Lilas (FR); COMBREDET, Chantal, F-75017 Paris (FR); DELAGNEAU, Jean-François, F-78170 La Celle Saint Cloud (FR); MARTIN, Monique, F-92290 Chatenay Malabry (FR)
(74) Mandataire: Marcadé, Véronique
(86) Numéro de dépôt international: PCT/FR2004/003105
(87) Numéro de publication internationale: WO 2005/056781

(56) Documents cités:
- DATABASE UNIPROT 10 octobre 2003 (2003-10-10), XP002294755 Database accession no. P59594
- MARRA M A ET AL: "The genome sequence of the SARS-associated coronavirus" SCIENCE, AMERICAN ASSOCIATION FOR THE ADVANCEMENT OF SCIENCE,, US, vol. 300, no. 5624, 30 mai 2003 (2003-05-30), pages 1399-1404, XP002269483 ISSN: 0036-8075
- WANG J ET AL: "ASSESSMENT OF IMMUNOREACTIVE SYNTHETIC PEPTIDES FROM THE STRUCTURAL PROTEINS OF SEVERE ACUTE RESPIRATORY SYNDROME CORONAVIRUS" CLINICAL CHEMISTRY, AMERICAN ASSOCIATION FOR CLINICAL CHEMISTRY. WINSTON, US, vol. 49, no. 12, 13 novembre 2003 (2003-11-13), pages 1989-1996, XP001182489 ISSN: 0009-9147

## Description

La présente invention est relative à une nouvelle souche de coronavirus associé au syndrome respiratoire aigu sévère (SRAS), issue d'un prélèvement répertorié sous le n° 031589 et prélevé à Hanoi (Vietnam), à des molécules d'acide nucléique issues de son génome, aux protéines et peptides codés par lesdites molécules d'acide nucléique ainsi qu'à leurs applications, notamment en tant que réactifs de diagnostic et/ou comme vaccin.

Le coronavirus est un virus à ARN monocaténaire, de polarité positive, d'approximativement 30 kilobases qui se réplique dans le cytoplasme des cellules hôtes ; l'extrémité 5' du génome a une structure en coiffe et l'extrémité 3' comporte une queue polyA. Ce virus est enveloppé et comprend, à sa surface, des structures péplomériques dénommées spicules.

Le génome comprend les cadres ouverts de lecture ou ORF suivants, de son extrémité 5' vers son extrémité 3' : ORF1a et ORF1b correspondant aux protéines du complexe de transcription-réplication, et ORF-S, ORF-E, ORF-M et ORF-N correspondant aux protéines structurales S, E, M et N. Il comprend également des ORFs correspondant à des protéines de fonction inconnue codées par : la région située entre l'ORF-S et l'ORF-E et chevauchant cette dernière, la région située entre l'ORF-M et l'ORF-N, et la région incluse dans l'ORF-N.

La protéine S est une glycoprotéine membranaire (200-220 kDa) qui se présente sous la forme de spicules ou "Spike" émergeant de la surface de l'enveloppe virale. Elle est responsable de l'attachement du virus aux récepteurs de la cellule hôte et de l'induction de la fusion de l'enveloppe virale avec la membrane cellulaire.

La petite protéine d'enveloppe (E) également dénommée sM (*small membrane*) qui est une protéine trans-membranaire non glycosylée d'environ 10 kDa, est la protéine présente en plus faible quantité dans le virion. Elle joue un rôle moteur dans le processus de bourgeonnement des coronavirus qui se produit au niveau du compartiment intermédiaire dans le réticulum endoplasmique et l'appareil de Golgi

La protéine M ou protéine de matrice (25-30 kDa) est une glycoprotéine membranaire plus abondante qui est intégrée dans la particule virale par une interaction M/E, tandis que l'incorporation de S dans les particules est dirigée par une interaction S/M. Elle semble être importante pour la maturation virale des coronavirus et pour la détermination du site au niveau duquel les particules virales sont assemblées.

La protéine N ou protéine de nucléocapside (45-50 kDa) qui est la plus conservée parmi les protéines structurales des coronavirus, est nécessaire pour encapsider l'ARN génomique puis pour diriger son incorporation dans le virion. Cette protéine est vraisemblablement également impliquée dans la réplication de l'ARN.

Lorsqu'une cellule hôte est infectée, le cadre de lecture (ORF) situé en 5' du génome viral est traduit en une polyprotéine qui est clivée par les protéases virales et libère alors plusieurs protéines non-structurales telles que l'ARN-polymérase ARN dépendante (Rep) et l'ATPase hélicase (Hel). Ces deux protéines sont impliquées dans la réplication du génome viral ainsi que dans la génération de transcrits qui sont utilisés dans la synthèse des protéines virales. Les mécanismes par lesquels ces ARNms sub-génomiques sont produits, ne sont pas complètement compris ; cependant des faits récents indiquent que les séquences de régulation de la transcription à l'extrémité 5' de chaque gène représentent des signaux qui régulent la transcription discontinue des ARNms sub-génomiques.

Les protéines de la membrane virale (protéines S, E et M) sont insérées dans le compartiment intermédiaire, alors que l'ARN répliqué (brin +) s'assemble avec la protéine N (nucléocapside). Ce complexe protéine-ARN s'associe ensuite avec la protéine M incluse dans les membranes du réticulum endoplasmique et les particules virales se forment lorsque le complexe de la nucléocapside bourgeonne dans le réticulum endoplasmique. Le virus migre ensuite à travers le complexe du Golgi et éventuellement sort de la cellule, par exemple par exocytose. Le site de l'attachement du virus à la cellule hôte se trouve au niveau de la protéine S.

Les coronavirus sont responsables de 15 à 30 % des rhumes chez l'Homme et d'infections respiratoires ou digestives chez les animaux, notamment le chat (FIPV : *Feline infectious peritonitis virus),* la volaille (IBV : *Avian Infectious bronchitis virus*), la souris (MHV : *Mouse Hepatitis virus*), le porc (TGEV : Transmissible gastroenterititis virus, PEDV : *Porcine Epidemic Diarrhea virus,* PRCoV : *Porcine Respiratory Coronavirus,* HEV : *Hemagglutinating encephalomyelitis Virus*) et les bovins (BcoV *: Bovine coronavirus*).

En général, chaque coronavirus n'affecte qu'une seule espèce ; chez les individus immunocompétents, l'infection induit des anticorps éventuellement neutralisants et une immunité cellulaire, capables de détruire les cellules infectées.

Une épidémie de pneumonie atypique, dénommée syndrome respiratoire aigu sévère (SARS ou *Severe acute respiratory syndrome,* SRAS en français) s'est propagée dans différents pays (Vietnam, Hong-Kong, Singapour, Thaïlande et Canada) au cours du premier trimestre 2003, à partir d'un foyer initial apparu en Chine dans le dernier trimestre de 2002. La sévérité de cette maladie est telle que son taux de mortalité est d'environ 3 à 6 %. La détermination de l'agent causatif de cette maladie a été entreprise par de nombreux laboratoires, à travers le monde.

En mars 2003, un nouveau coronavirus (SARS-CoV, SARS virus ou virus SRAS, en français) a été isolé, en association avec des cas de syndrome respiratoire aigu sévère (T.G.KSIAZEK et al., The New England Journal of Medicine, 2003, 348, 1319-1330 ; C. DROSTEN et al., The New England Journal of Medicine, 2003, 348, 1967-1976 ; Peiris et al., Lancet, 2003, 361, 1319-).

Des séquences génomiques de ce nouveau coronavirus ont ainsi été obtenues, notamment celles de l'isolat Urbani (Genbank n° d'accès AY274119.3 et A. MARRA et al., Science, May 1, 2003, 300, 1399-1404) et de l'isolat de Toronto (Tor2, Genbank n° d'accès AY 278741 et A. ROTA et al., Science, 2003, 300, 1394-1399).

L'organisation du génome est comparable à celle des autres coronavirus connus permettant ainsi de confirmer l'appartenance du SARS-CoV à la famille des *Coronaviridae ;* les cadres ouverts de lecture ORF1a et 1b et les cadres ouverts de lecture correspondant aux protéines S, E, M, et N, ainsi qu'à des protéines codées par : la région située entre l'ORF-S et l'ORF-E (ORF3), la région située entre l'ORF-S et l'ORF-E et chevauchant l'ORF-E (ORF4), la région située entre l'ORF-M et l'ORF-N (ORF7 à ORF11) et la région correspondant à l'ORF-N (ORF13 et ORF14), ont notamment été identifiées.

Sept différences ont été mises en évidence entre les séquences des isolats Tor2 et Urbani ; 3 correspondent à des mutations silencieuses (c/t en position 16622 et a/g en position 19064 de l'ORF1b, t/c en position 24872 de l'ORF-S) et 4 modifient la séquence en acides aminés de respectivement : les protéines codées par l'ORF1a (c/t en position 7919 correspondant à la mutation A/V), la protéine S (g/t en position 23220 correspondant à la mutation A/S), la protéine codée par l'ORF3 (a/g en position 25298 correspondant à la mutation R/G) et de la protéine M (t/c en position 26857 correspondant à la mutation S/P).

En outre, l'analyse phylogénétique montre que le SARS-CoV est éloigné des autres coronavirus et qu'il est apparu, ni par mutation de coronavirus respiratoires humains, ni par recombinaison entre des coronavirus connus (pour une revue, voir Holmes, J.C.I., 2003, 111, 1605-1609).

La mise en évidence et la prise en compte de nouveaux variants sont importantes pour la mise au point de réactifs de détection et de diagnostic du SRAS suffisamment sensibles et spécifiques ainsi qu'à des compositions immunogènes aptes à protéger des populations contre des épidémies de SRAS.

Les Inventeurs ont maintenant mis en évidence une autre souche de coronavirus associé au SRAS, qui se distingue des isolats Tor2 et Urbani.

La présente invention est telle que définie par les revendications 1 à 22 ci-après.

Les Inventeurs décrivent une souche isolée ou purifiée de coronavirus humain associé au syndrome respiratoire aigu sévère, caractérisée en ce que son génome présente sous la forme d'ADN complémentaire un codon sérine en position 23220-23222 du gène de la protéine S ou un codon glycine en position 25298-25300 du gène de l'ORF3, et un codon alanine en position 7918-7920 de l'ORF1a ou un codon sérine en position 26857-26859 du gène de la protéine M, lesdites positions étant indiquées en référence à la séquence Genbank AY274119.3.

Selon un mode de réalisation avantageux de ladite souche, l'équivalent ADN de son génome présente une séquence correspondant à la séquence SEQ ID NO : 1 ; cette souche de coronavirus est issue du prélèvement de lavage bronchoalvéolaire d'un patient atteint de SRAS, répertorié sous le n° 031589 et effectué à l'hôpital français de Hanoi (Vietnam).

Ladite séquence SEQ ID NO:1 est celle de l'acide désoxyribonucléique correspondant à la molécule d'acide ribonucléique du génome de la souche isolée de coronavirus telle que définie ci-dessus.

La séquence SEQ ID NO : 1 se distingue de la séquence Genbank AY274119.3 (isolat Tor2) en ce qu'elle possède les mutations suivantes :
- g/t en position 23220 ; le codon alanine (gct) en position 577 de la séquence en acides aminés de la protéine S de Tor2 est remplacé par un codon sérine (tct),
- a/g en position 25298 ; le codon arginine (aga) en position 11 de la séquence en acide aminés de la protéine codée par l'ORF3 de Tor 2 est remplacé par un codon glycine (gga).

En outre, la séquence SEQ ID NO : 1 se distingue de la séquence Genbank AY278741 (isolat Urbani) en ce qu'elle possède les mutations suivantes :
- t/c en position 7919 ; le codon valine (gtt) en position 2552 de la séquence en acides aminés de la protéine codée par l'ORF1a est remplacé par un codon alanine (gct),
- t/c en position 16622 : cette mutation ne modifie pas la séquence en acides aminés des protéines codées par l'ORF1b (mutation silencieuse),
- g/a en position 19064 : cette mutation ne modifie pas la séquence en acides aminés des protéines codées par l'ORF1b (mutation silencieuse),
- c/t en position 24872 : cette mutation ne modifie pas la séquence en acides aminés de la protéine S, et
- c/t en position 26857 : le codon proline (ccc) en position 154 de la séquence en acides aminés de la protéine M est remplacé par un codon sérine (tcc).

En l'absence de mention particulière, les positions des séquences nucléotidiques et peptidiques sont indiquées en référence à la séquence Genbank AY274119.3.

Les Inventeurs décrivent également un polynucléotide isolé ou purifié, caractérisé en ce que sa séquence est celle du génome de la souche isolée de coronavirus telle que définie ci-dessus.

Selon un mode de réalisation avantageux dudit polynucléotide il présente la séquence SEQ ID NO : 1.

Les Inventeurs décrivent également un polynucléotide isolé ou purifié, caractérisé en ce que sa séquence hybride dans des conditions de forte stringence avec la séquence du polynucléotide tel que défini ci-dessus.

Les termes « isolé ou purifié » signifient modifié « par la main de l'homme » à partir de l'état naturel ; autrement dit si un objet existe dans la nature, il est dit isolé ou purifié s'il a été modifié ou extrait de son environnement naturel ou les deux. Par exemple, un polynucléotide ou une protéine/un peptide naturellement présent dans un organisme vivant n'est ni isolé, ni purifié ; en revanche le même polynucléotide ou protéine /peptide séparé des molécules coexistantes dans son environnement naturel, obtenu par clonage, amplification et/ou synthèse chimique est isolé au sens de la présente invention. De plus, un polynucléotide ou une protéine/peptide qui est introduit dans un organisme par transformation, manipulation génétique ou par toute autre méthode, est « isolé » même s'il est présent dans ledit organisme. Le terme purifié tel qu'utilisé dans la présente invention, signifie que les protéines /peptides selon l'invention sont essentiellement libres d'association avec les autres protéines ou polypeptides, comme l'est par exemple le produit purifié de la culture de cellules hôtes recombinantes ou le produit purifié à partir d'une source non-recombinante.

On entend par conditions d'hybridation de forte stringence, des conditions de température et de force ionique choisies de telle manière qu'elles permettent le maintien de l'hybridation spécifique et sélective entre polynucléotides complémentaires.

A titre d'illustration, des conditions de forte stringence aux fins de définir les polynucléotides ci-dessus, sont avantageusement les suivantes : l'hybridation ADN-ADN ou ADN-ARN est réalisée en deux étapes : (1) préhybridation à 42°C pendant 3 heures en tampon phosphate (20 mM pH 7,5) contenant 5 x SSC (1 x SSC correspond à une solution 0,15 M NaCl + 0, 015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation pendant 20 heures à 42°C suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C.

Les Inventeurs décrivent également un fragment représen tatif du polynucléotide tel que défini ci-dessus, caractérisé en ce qu'il est susceptible d'être obtenu, soit par l'utilisation d'enzymes de restriction dont les sites de reconnaissance et de coupure sont présents dans ledit polynucléotide tel que défini ci-dessus, soit par amplification à l'aide d'amorces oligonucléotidiques spécifiques dudit polynucléotide tel que défini ci-dessus, soit par transcription *in vitro,* soit par synthèse chimique.

Selon un mode de réalisation avantageux dudit fragment, il est sélectionné dans le groupe constitué par : l'ADNc correspondant à au moins un cadre ouvert de lecture (ORF) choisi parmi : ORF1a, ORF1b, ORF-S, ORF-E, ORF-M, ORF-N, ORF3, ORF4, ORF7 à ORF11, ORF13 et ORF 14, et l'ADNc correspondant aux extrémités 5' ou 3' non-codantes dudit polynucléotide.

Selon une disposition avantageuse de ce mode de réalisation, ledit fragment présente une séquence sélectionnée dans le groupe constitué par :
- les séquences SEQ ID NO : 2 et 4 représentant l'ADNc correspondant à l'ORF-S qui code pour la protéine S,
- les séquences SEQ ID NO : 13 et 15 représentant l'ADNc correspondant à l'ORF-E qui code pour la protéine E,
- les séquences séquence SEQ ID NO: 16 et 18 représentant l'ADNc correspondant à l'ORF-M qui code pour la protéine M,
- les séquences SEQ ID NO : 36 et 38 représentant l'ADNc correspondant à l'ORF-N qui code pour la protéine N,
- les séquences représentant les ADNc correspondant respectivement : aux ORF1a et ORF1b (ORF1ab, SEQ ID NO : 31), aux ORF3 et ORF4 (SEQ ID NO : 7, 8), aux ORF 7 à 11 (SEQ ID NO: 19,20), à l'ORF13 (SEQ ID NO : 32) et à l'ORF14 (SEQ ID NO : 34), et
- les séquences représentant les ADNc correspondant respectivement aux extrémités 5'(SEQ ID NO : 39 et 72) et 3' non-codantes (SEQ ID NO : 40, 73) dudit polynucléotide.

Les Inventeurs décrivent également un fragment de l'ADNc codant pour la protéine S, tel que défini ci-dessus, caractérisé en ce qu'il présente une séquence sélectionnée dans le groupe constitué par les séquences SEQ ID NO : 5 et 6 (fragments Sa et Sb).

Les Inventeurs décrivent également un fragment de l'ADNc correspondant aux ORF1a et ORF1b tel que défini ci-dessus, caractérisé en ce qu'il présente une séquence sélectionnée dans le groupe constitué par les séquences SEQ ID NO : 41 à 54 (fragments L0 à L12).

Les Inventeurs décrivent également un fragment du polynucléotide tel que défini ci dessus, caractérisé en ce qu'il présente au moins 15 bases ou paires de bases consécutives de la séquence du génome de ladite souche incluant au moins une de celles situées en position 7979, 16622, 19064, 23220, 24872, 25298 et 26857. De préférence, il s'agit d'un fragment de 20 à 2500 bases ou paires de bases, de manière préférée de 20 à 400.

Selon un mode de réalisation avantageux dudit fragment, il inclut au moins un couple de bases ou de paires de bases correspondant aux positions suivantes : 7919 et 23220, 7919 et 25298, 16622 et 23220, 19064 et 23220, 16622 et 25298, 19064 et 25298, 23220 et 24872, 23220 et 26857, 24872 et 25298, 25298 et 26857.

Les Inventeurs décrivent également des amorces d'au moins 18 bases aptes à amplifier un fragment du génome d'un coronavirus associé au SRAS ou de l'équivalent ADN de celui-ci.

Selon un mode de réalisation desdites amorces, elles sont sélectionnées dans le groupe constitué par :
- la paire d'amorces n° 1 correspondant respectivement aux positions 28507 à 28522 (amorce sens, SEQ ID NO : 60) et 28774 à 28759 (amorce anti-sens, SEQ ID NO : 61) de la séquence du polynucléotide tel que défini ci-dessus,
- la paire d'amorces n° 2 correspondant respectivement aux positions 28375 à 28390 (amorce sens, SEQ ID NO : 62) et 28702 à 28687 (amorce anti-sens, SEQ ID NO : 63) de la séquence du polynucléotide tel que défini ci-dessus., et
- la paire d'amorces constituée des amorces SEQ ID Nos 55 et 56.

Les Inventeurs décrivent également une sonde apte à détecter la présence du génome d'un coronavirus associé au SRAS ou d'un fragment de celui-ci, caractérisée en ce qu'elle est sélectionnée dans le groupe constitué par : les fragments tels que définis ci-dessus et les fragments correspondant aux positions suivantes de la séquence du polynucléotide tel que défini ci-dessus : 28561 à 28586, 28588 à 28608, 28541 à 28563 et 28565 à 28589 (SEQ ID NO : 64 à 67).

Les sondes et amorces peuvent être marquées directement ou indirectement par un composé radioactif ou non radioactif par des méthodes bien connues de l'Homme du Métier, afin d'obtenir un signal détectable et/ou quantifiable. Parmi les isotopes radioactifs utilisés, on peut citer le ³²P, le ³³P, le ³⁵S, le ³H ou l'¹²⁵I. Les entités non radioactives sont sélectionnées parmi les ligands tels que la biotine, l'avidine, la streptavidine, la digoxygénine, les haptènes, les colorants, les agents luminescents tels que les agents radioluminescents, chémoluminescents, bioluminescents, fluorescents, phosphorescents.

Les sondes et amorces englobent les sondes et les amorces marquées dérivées des séquences précédentes.

De telles sondes et amorces sont utiles pour le diagnostic de l'infection par un coronavirus associé au SRAS.

Les Inventeurs décrivent également une méthode de détection d'un coronavirus associé au SRAS, à partir d'un échantillon biologique, laquelle méthode est caractérisée en ce qu'elle comprend au moins :
(a) l'extraction d'acides nucléiques présents dans ledit échantillon biologique,
(b) l'amplification d'un fragment de l'ORF-N par RT-PCR à l'aide d'une paire d'amorces telle que définie ci-dessus, et
(c) la détection par tout moyen approprié des produits d'amplifications obtenus en (b).

Les produits d'amplifications (amplicons) en (b) sont de 268 pb pour la paire d'amorces n° 1 et de 328 pb pour la paire d'amorces n°2.

Selon un mode de mise en oeuvre avantageux dudit procédé, l'étape (b) de détection est réalisée à l'aide d'au moins une sonde correspondant aux positions 28561 à 28586, 28588 à 28608, 28541 à 28563 et 28565 à 28589 de la séquence du polynucléotide tel que défini ci-dessus.

De préférence, le génome du coronavirus associé au SRAS est détecté et éventuellement quantifié par PCR en temps réel, à l'aide de la paire d'amorces n°2 et des sondes correspondant aux positions 28541 à 28563 et 28565 à 28589 marquées avec des composés différents, notamment des agents fluorescents différents.

La RT-PCR en temps réel qui met en oeuvre cette paire d'amorces et cette sonde est très sensible puisqu'elle permet de détecter 10² copies d'ARN et jusqu'à 10 copies d'ARN, elle est en outre fiable et reproductible.

La présente demande englobe les polydésoxyribonucléotides et les polyribonucléotides simple-brin, double-brin et tripe-brin correspondant à la séquence du génome de la souche isolée de coronavirus et de ses fragments tels que définis ci-dessus, ainsi qu'à leurs séquences complémentaires, sens ou anti-sens, notamment les ARN et les ADNc correspondant à la séquence du génome et de ses fragments tels que définis ci-dessus.

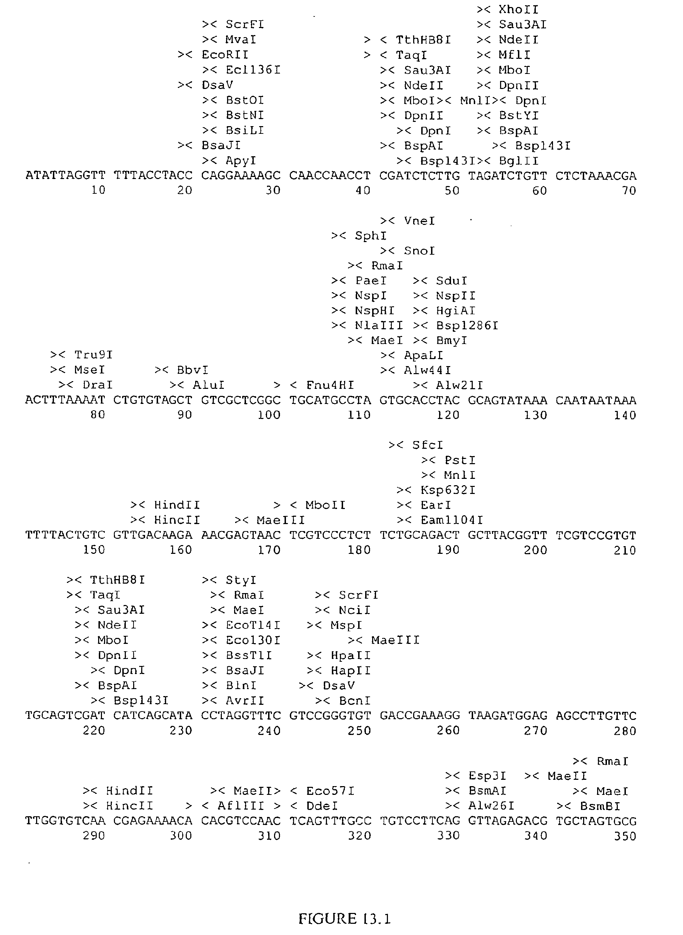

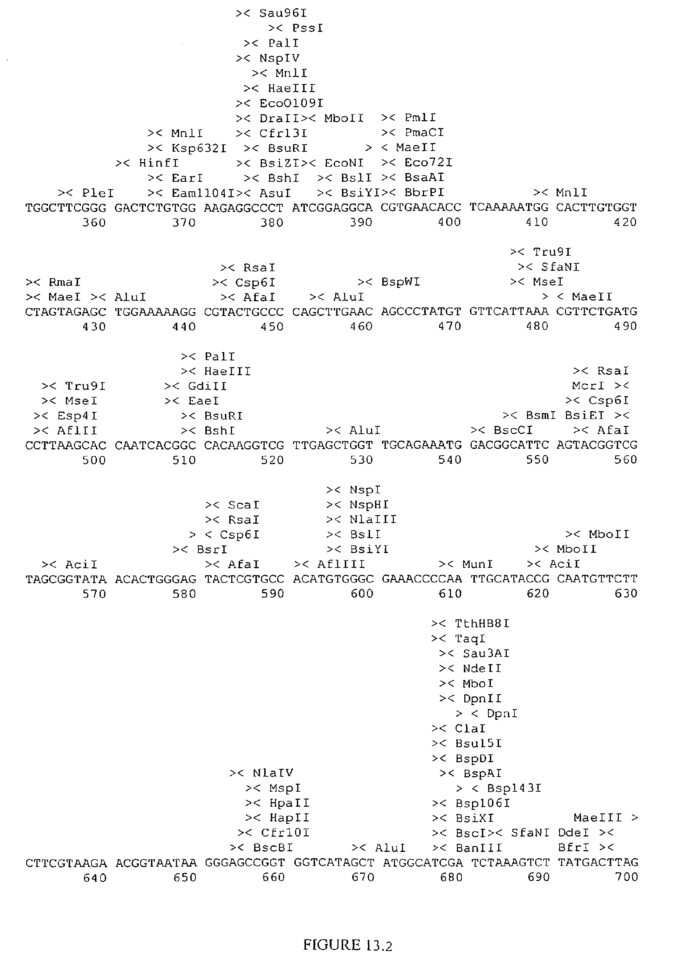

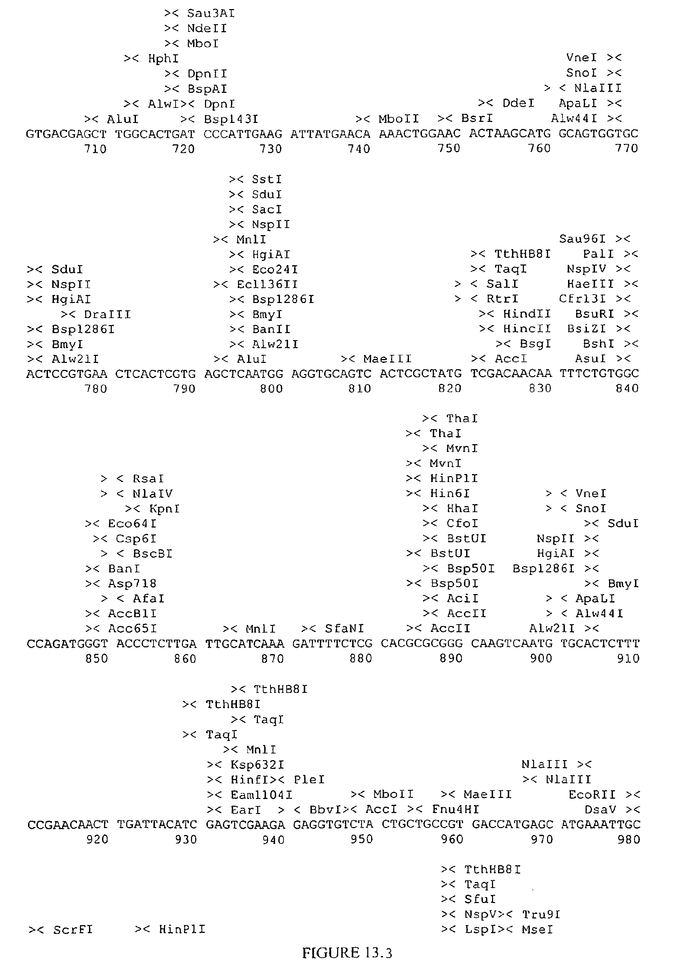

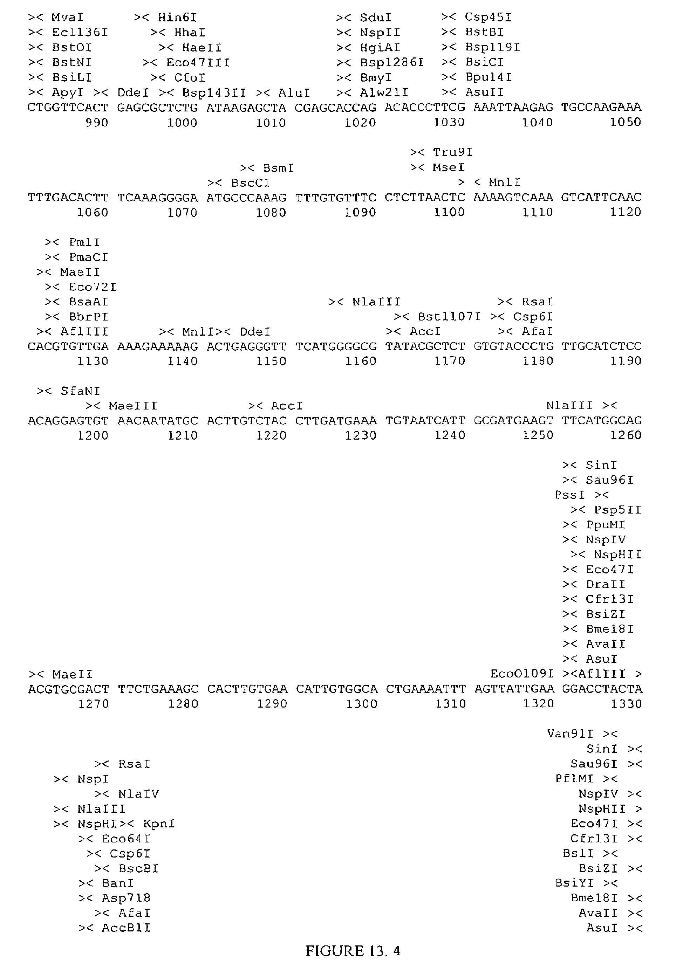

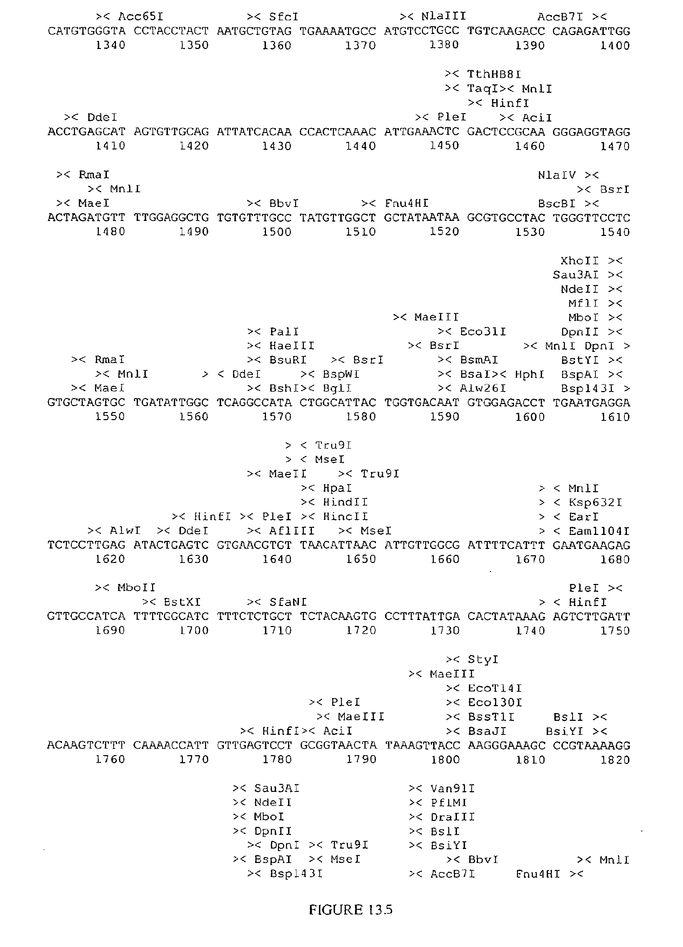

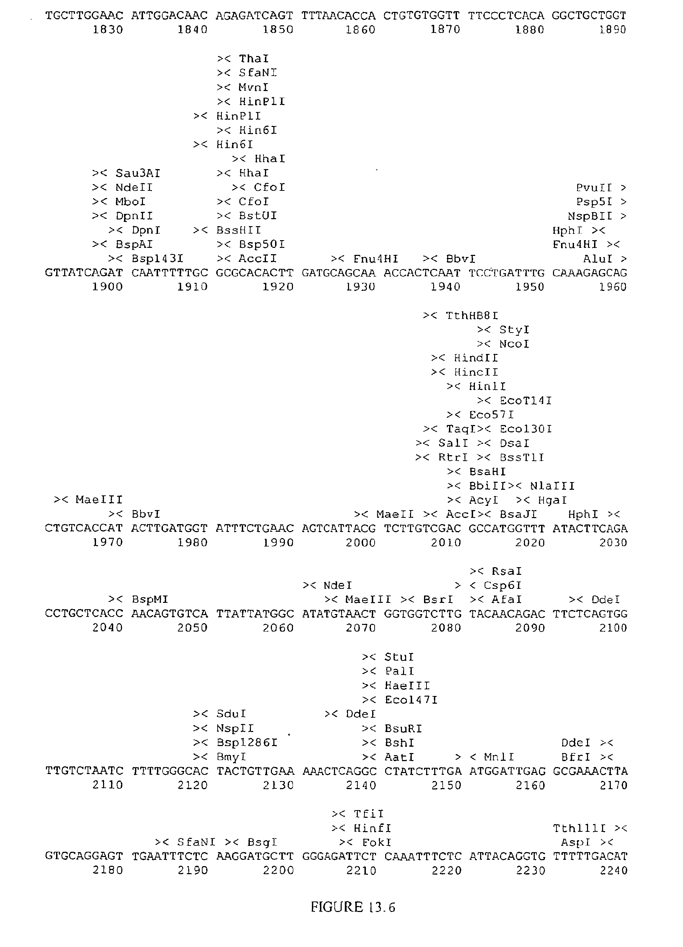

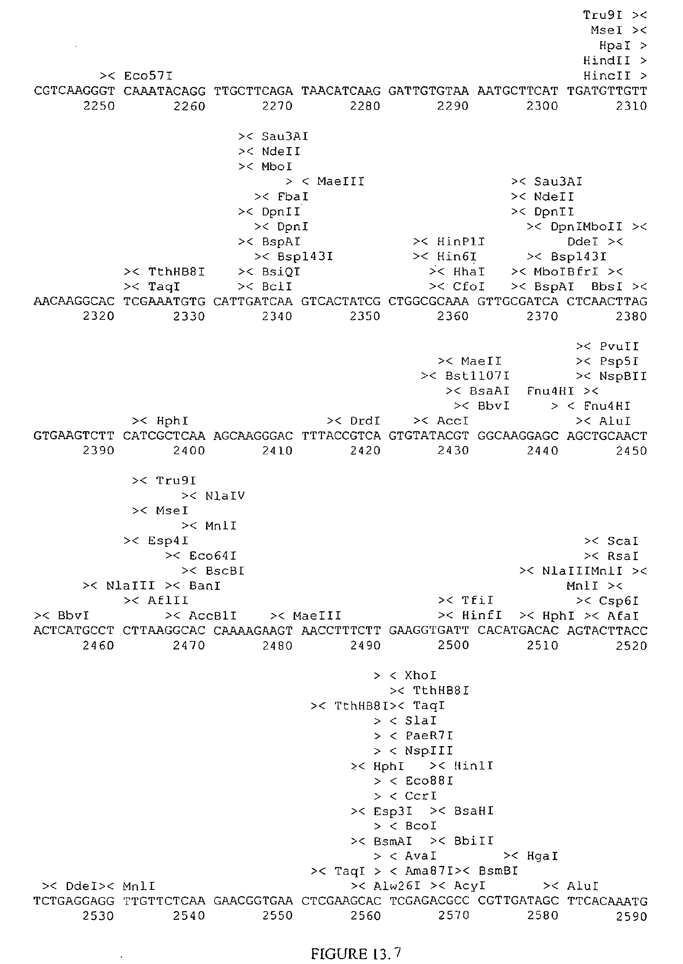

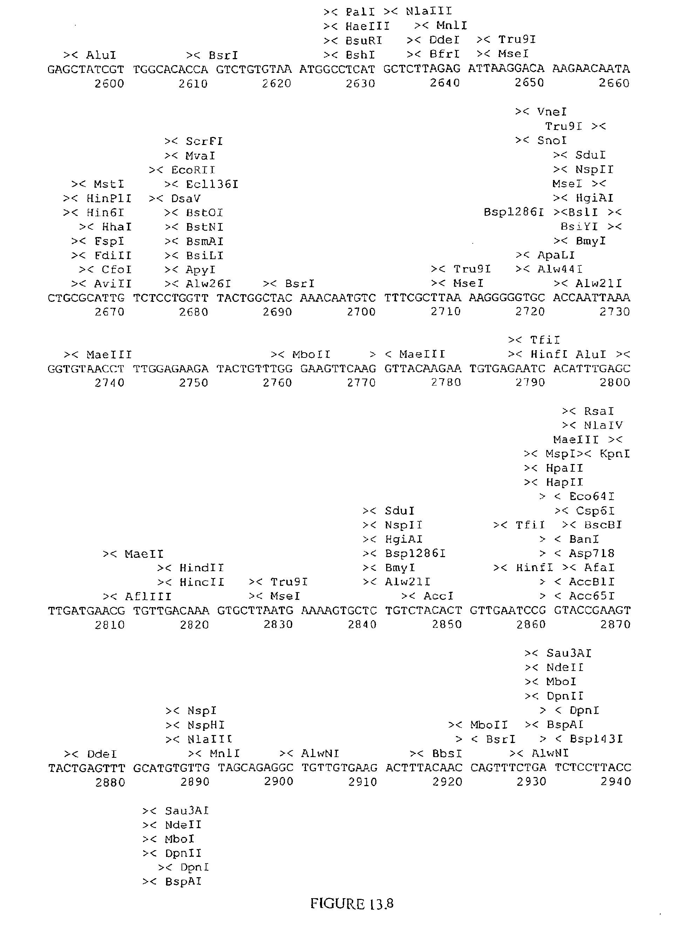

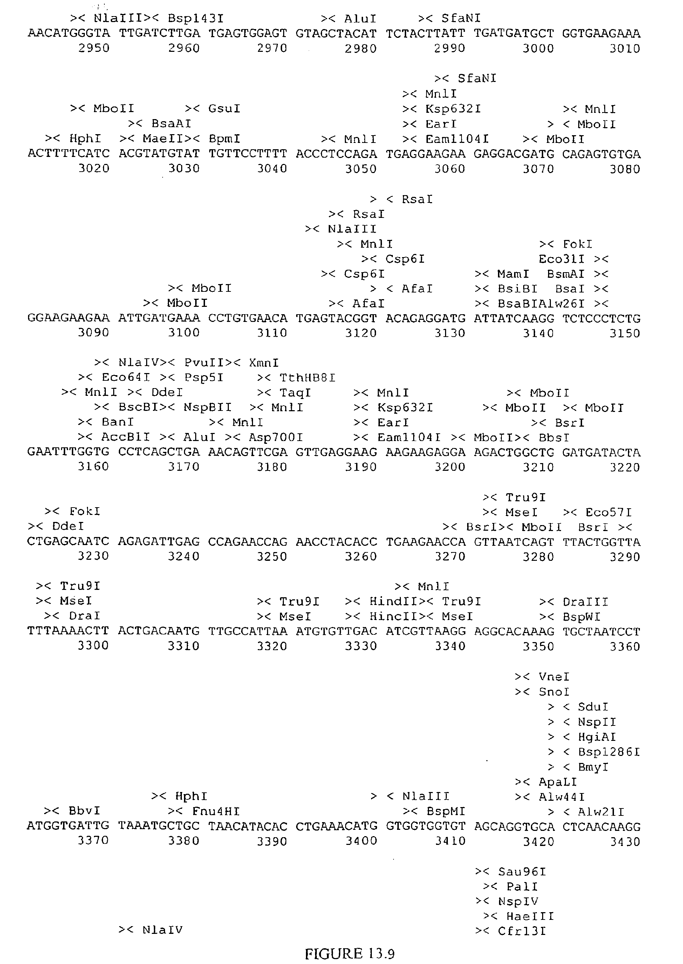

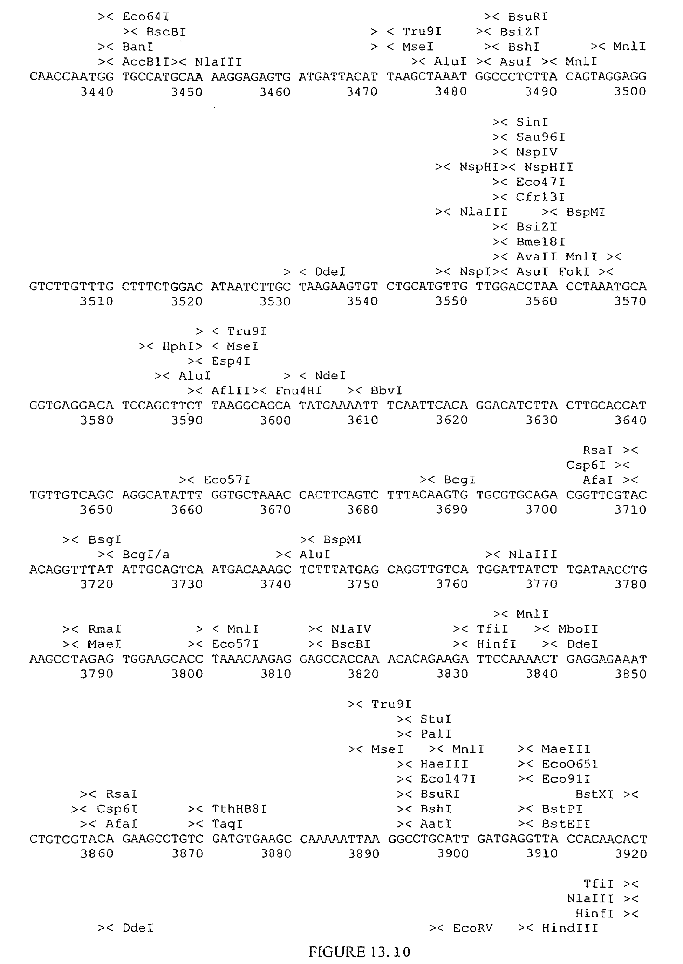

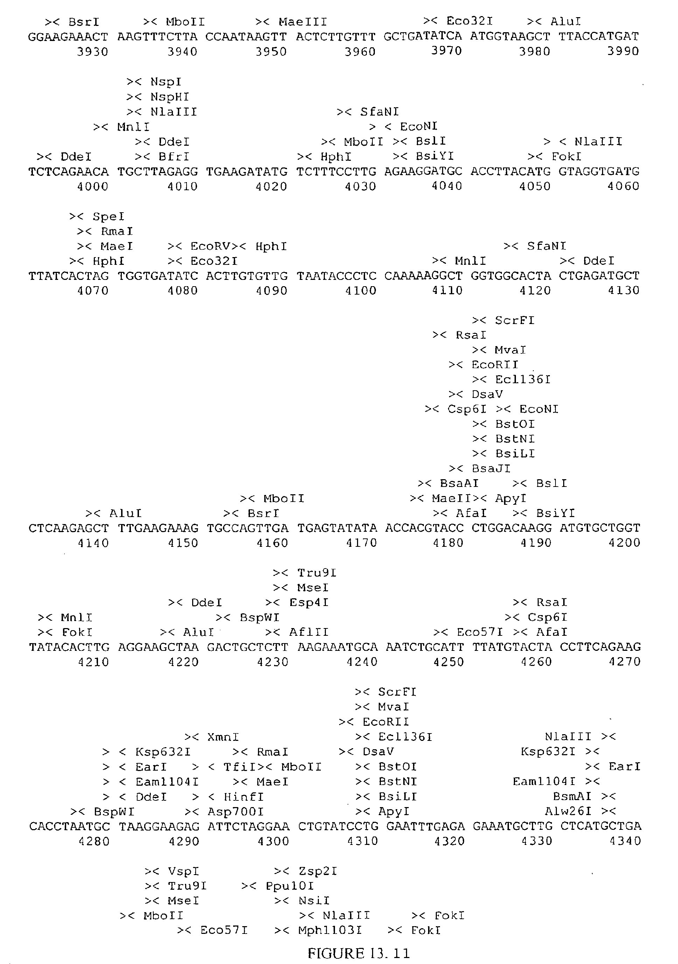

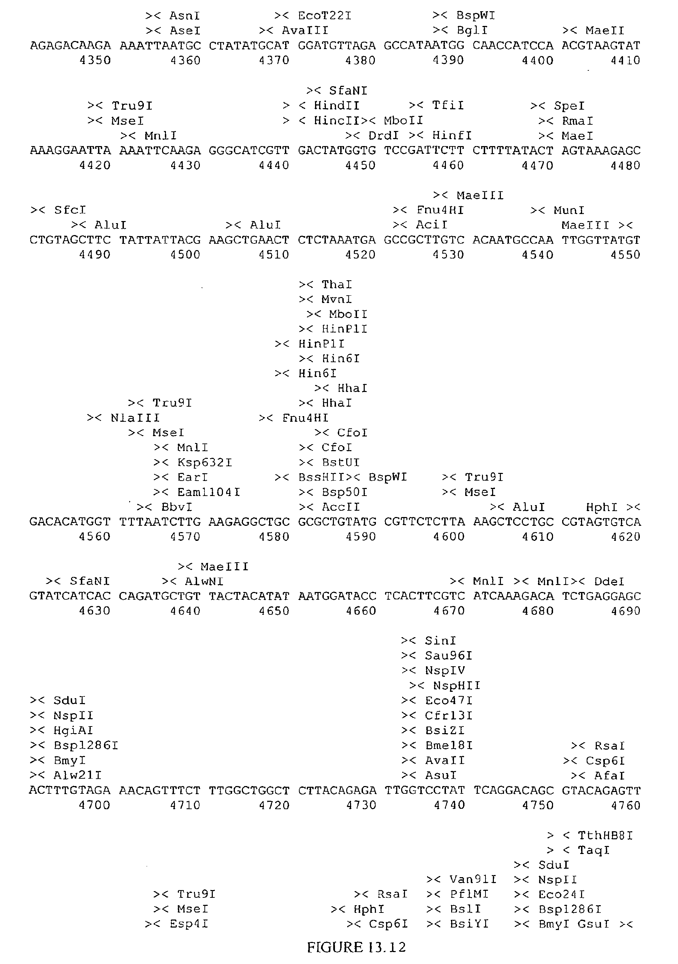

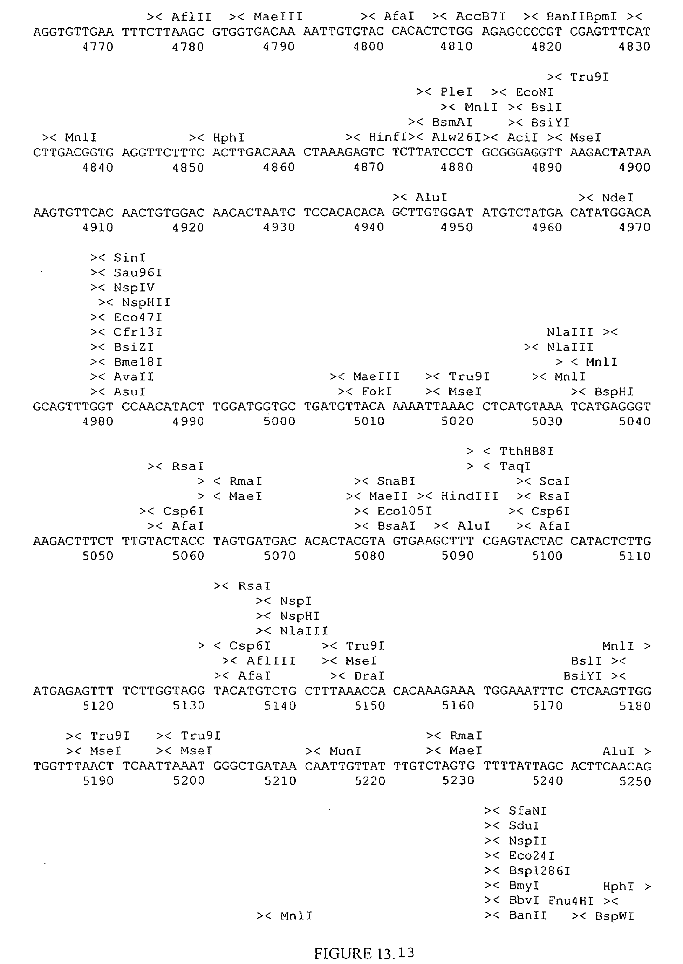

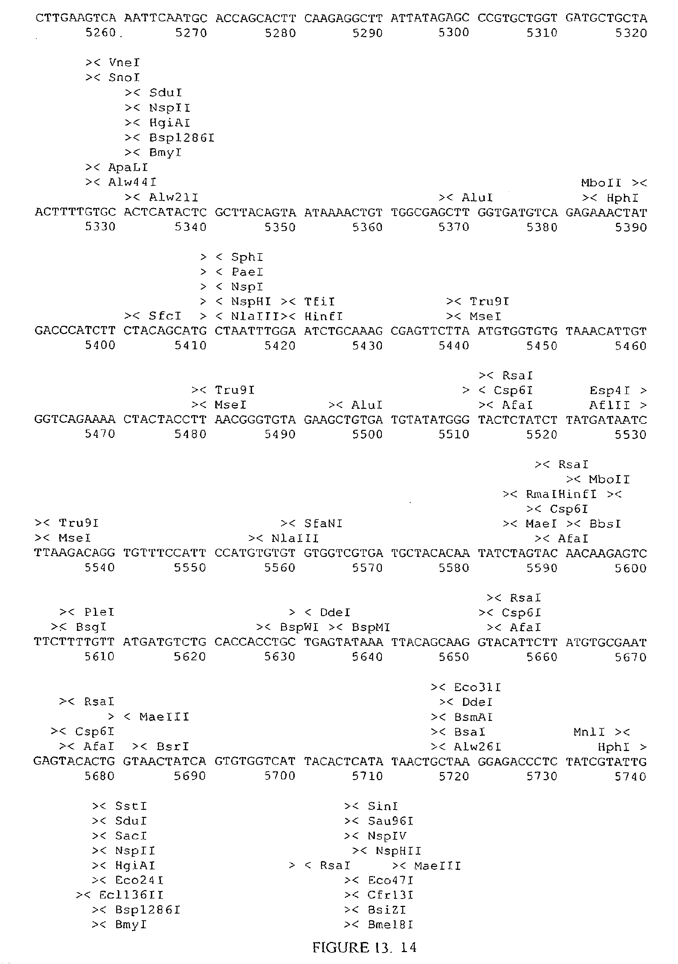

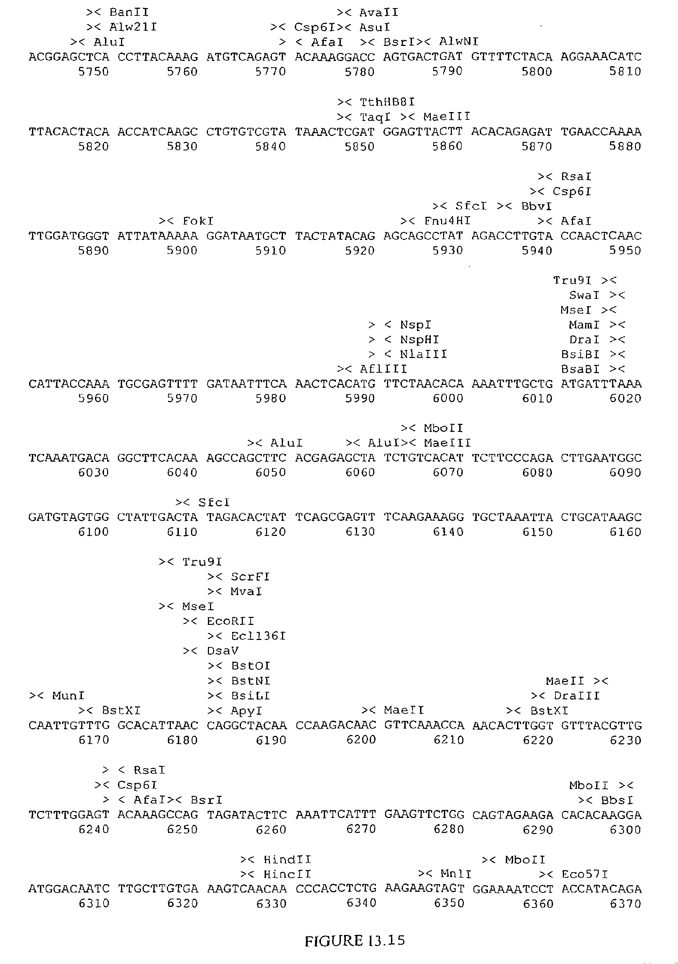

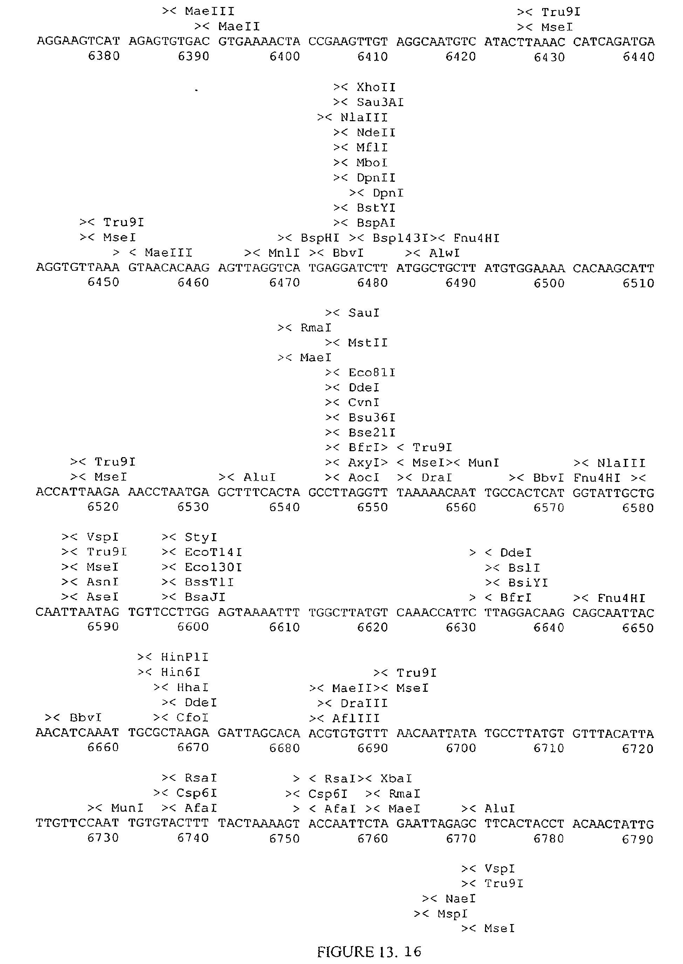

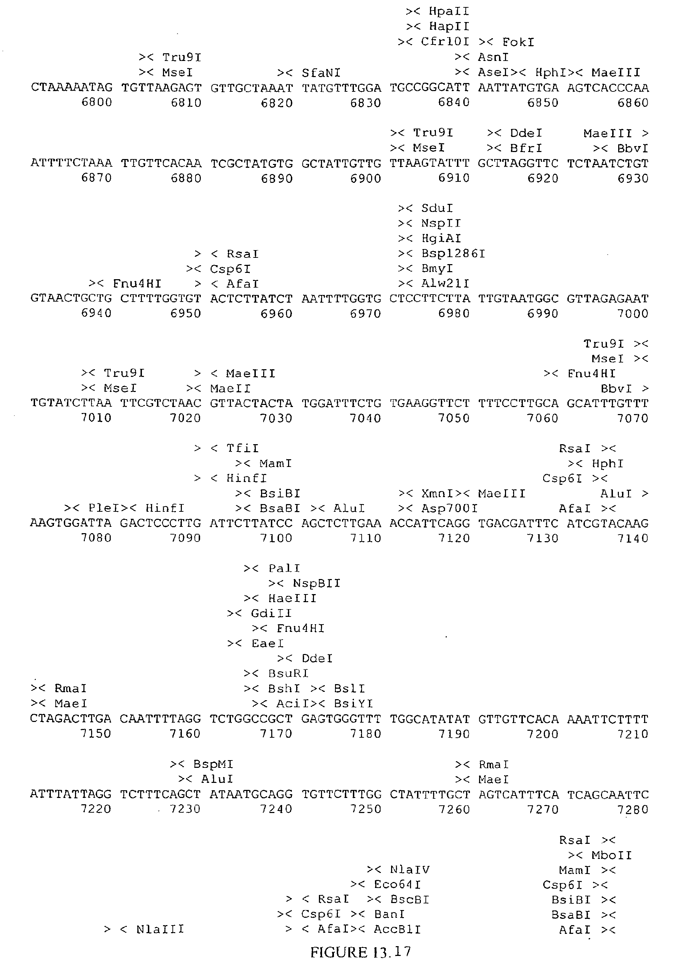

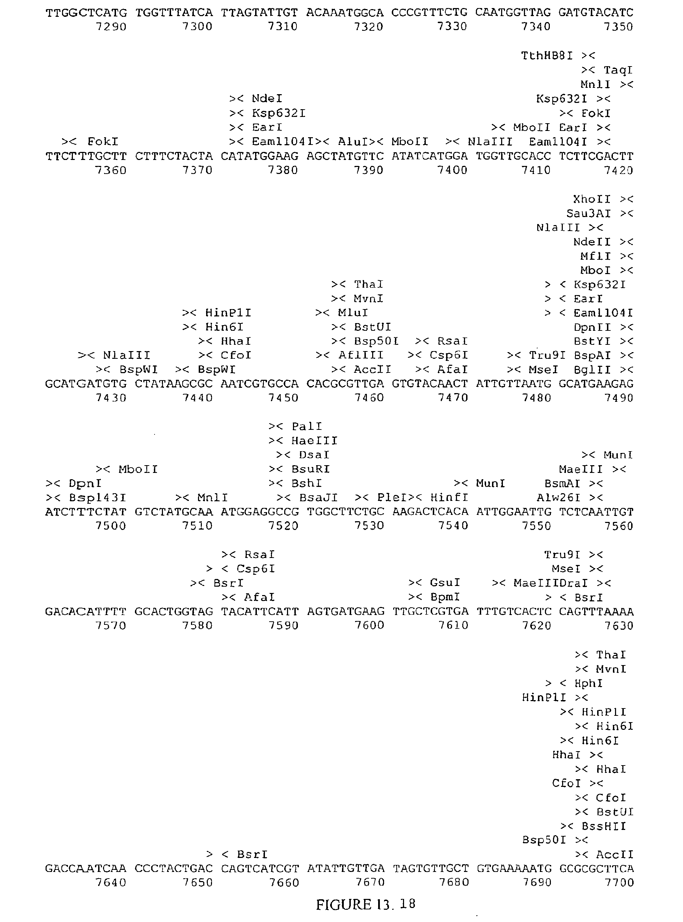

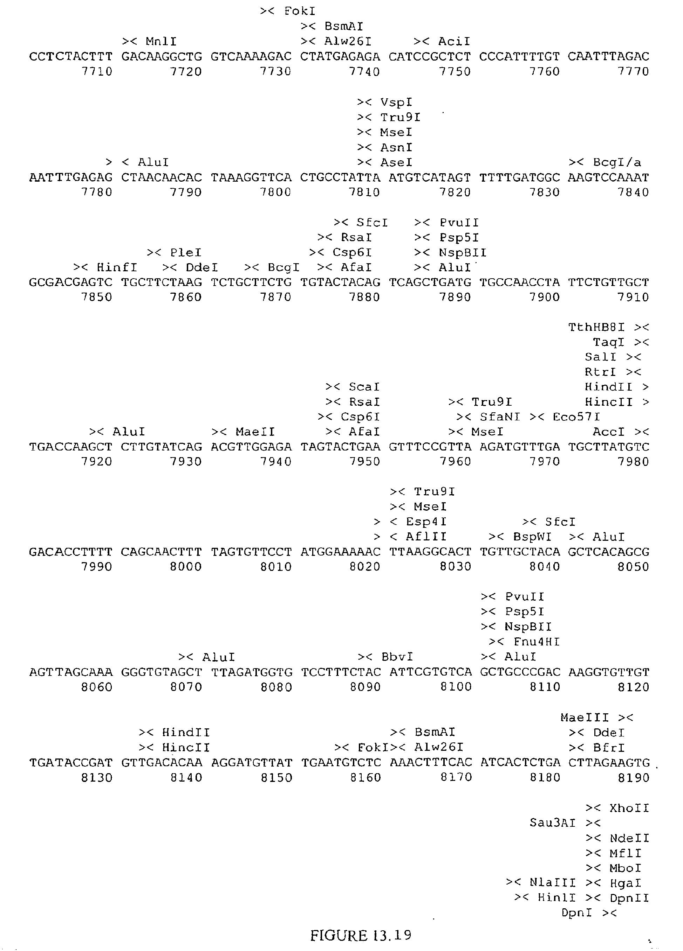

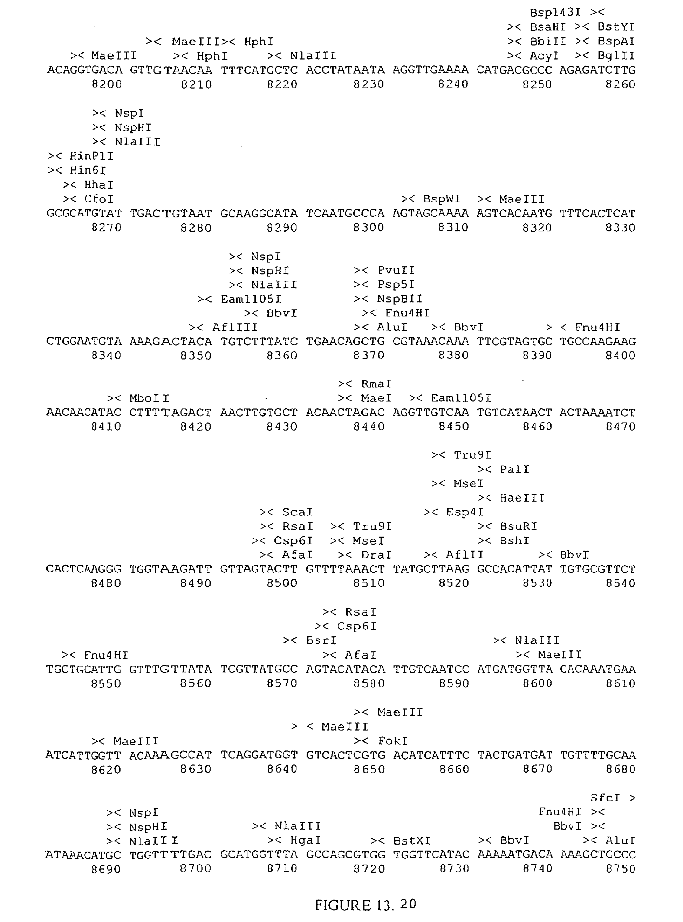

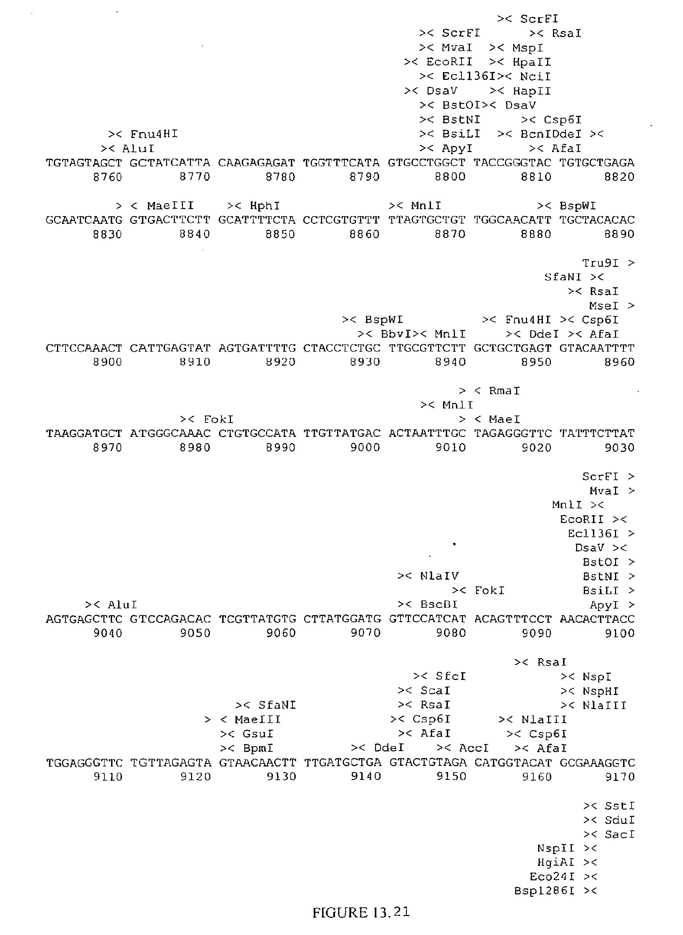

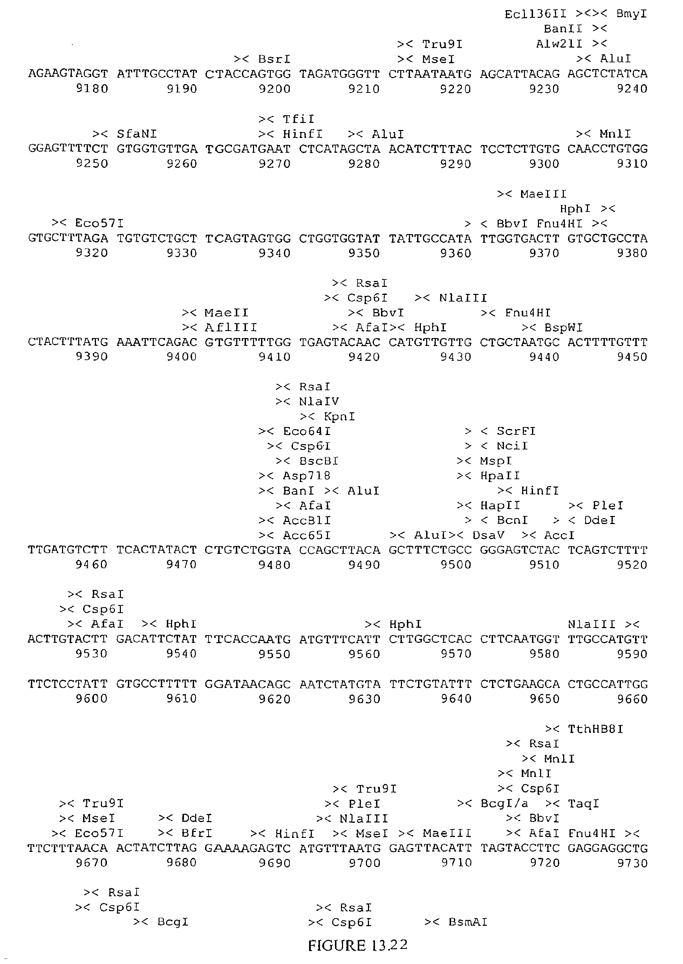

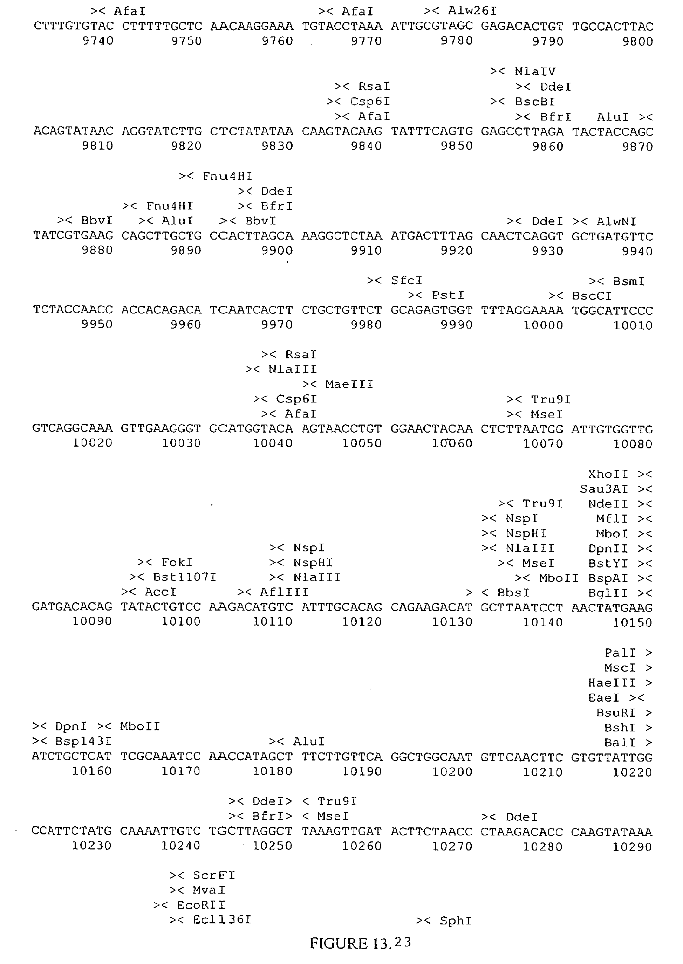

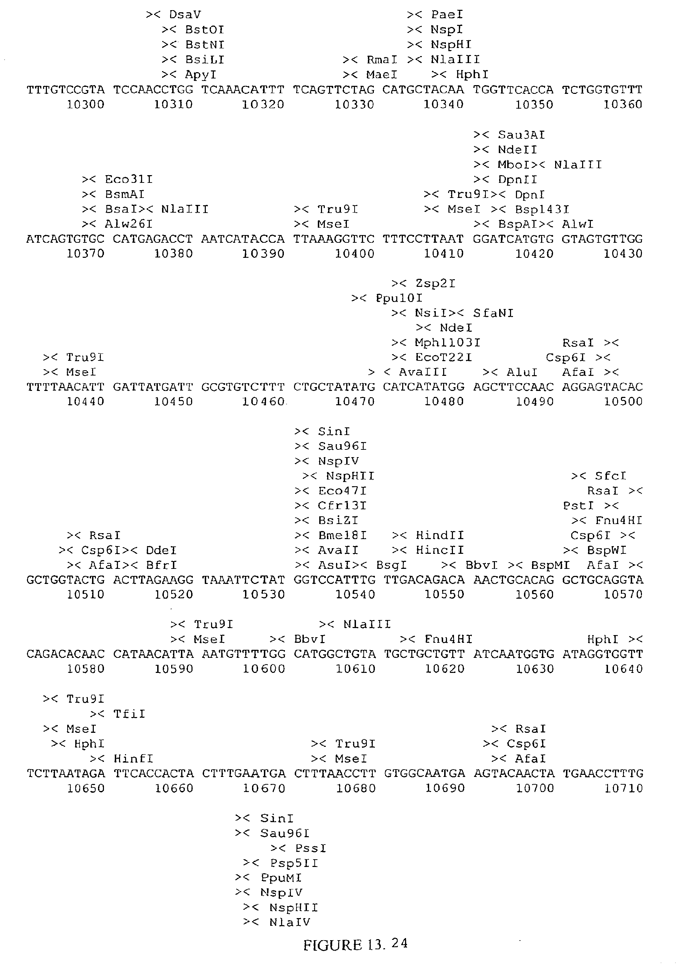

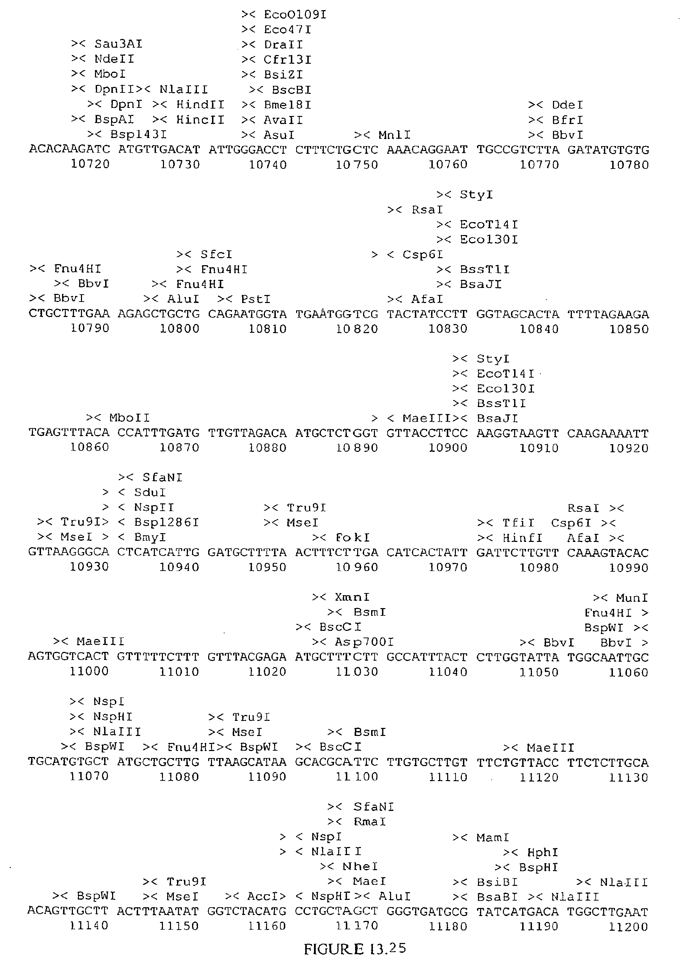

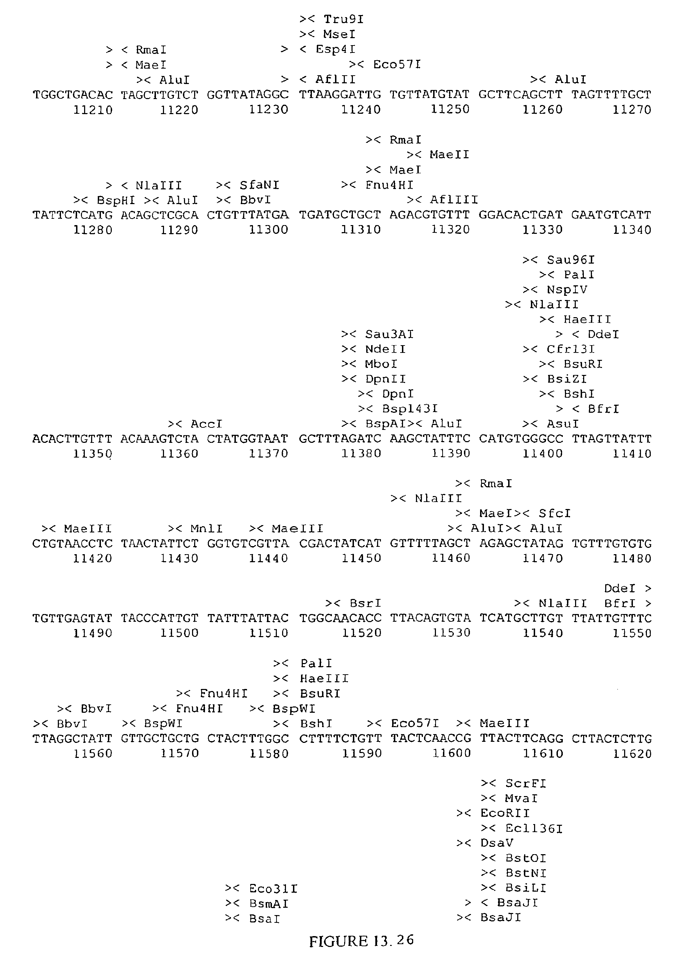

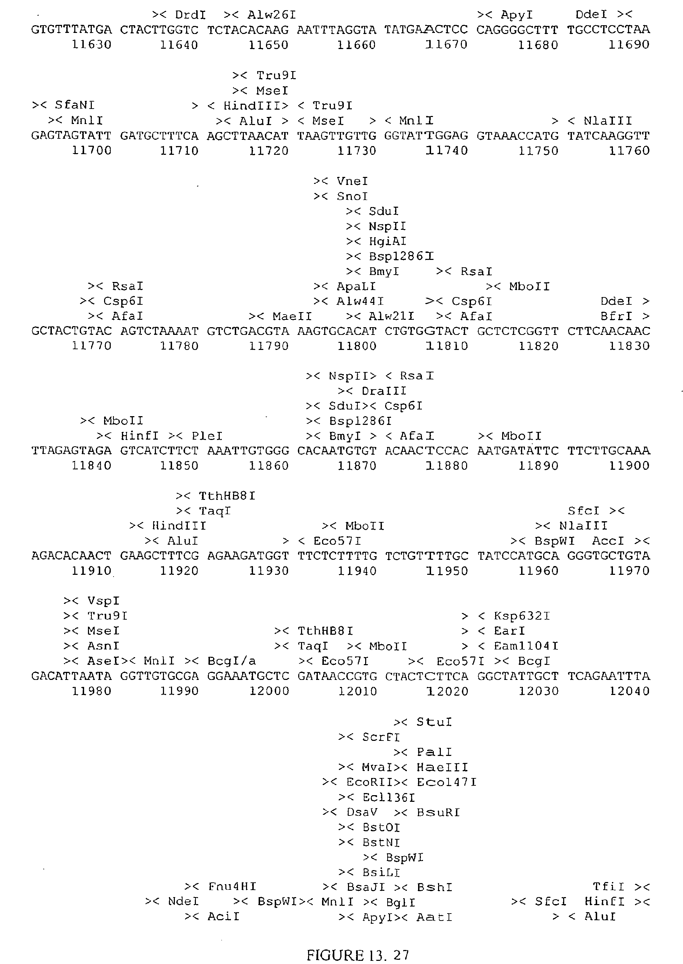

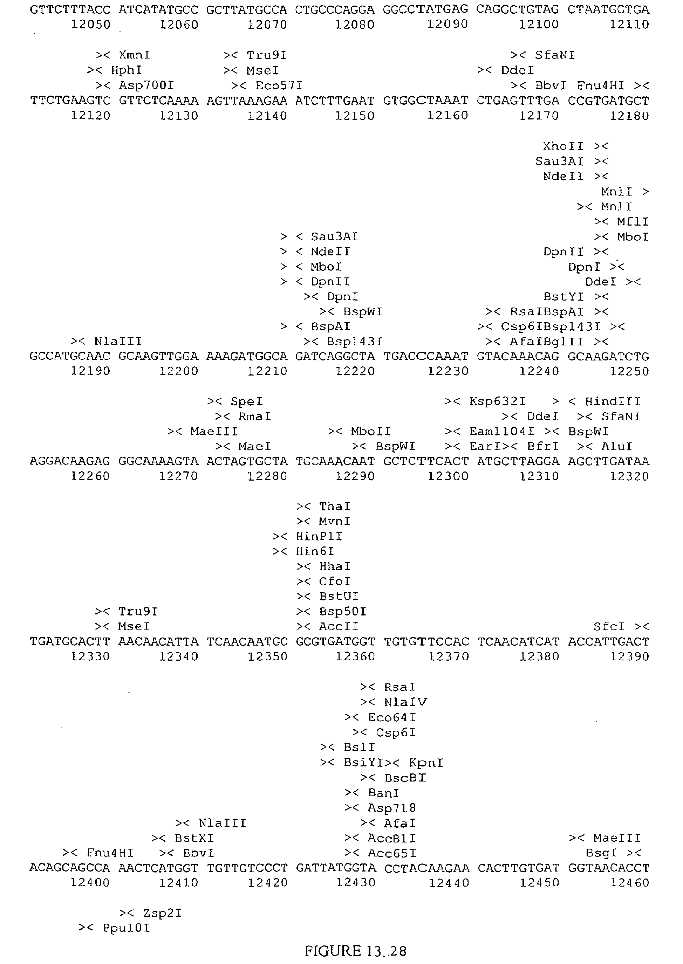

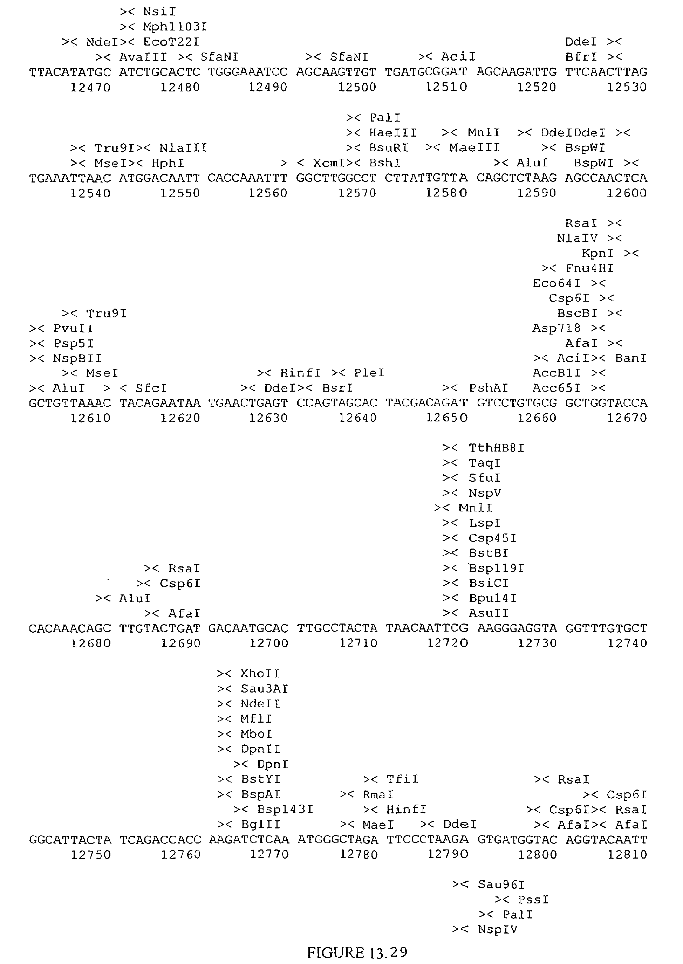

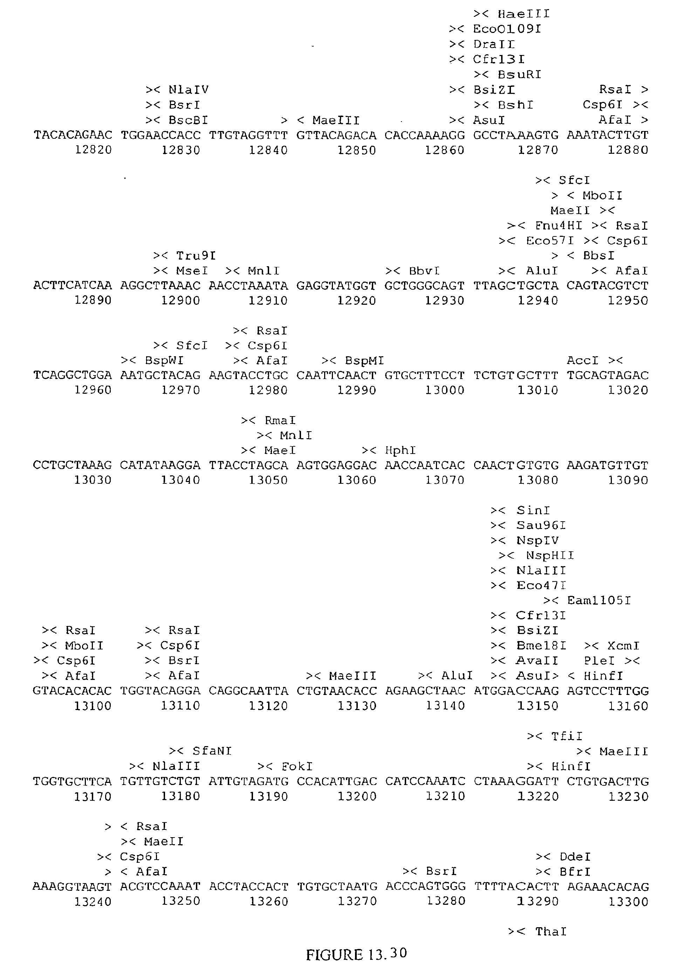

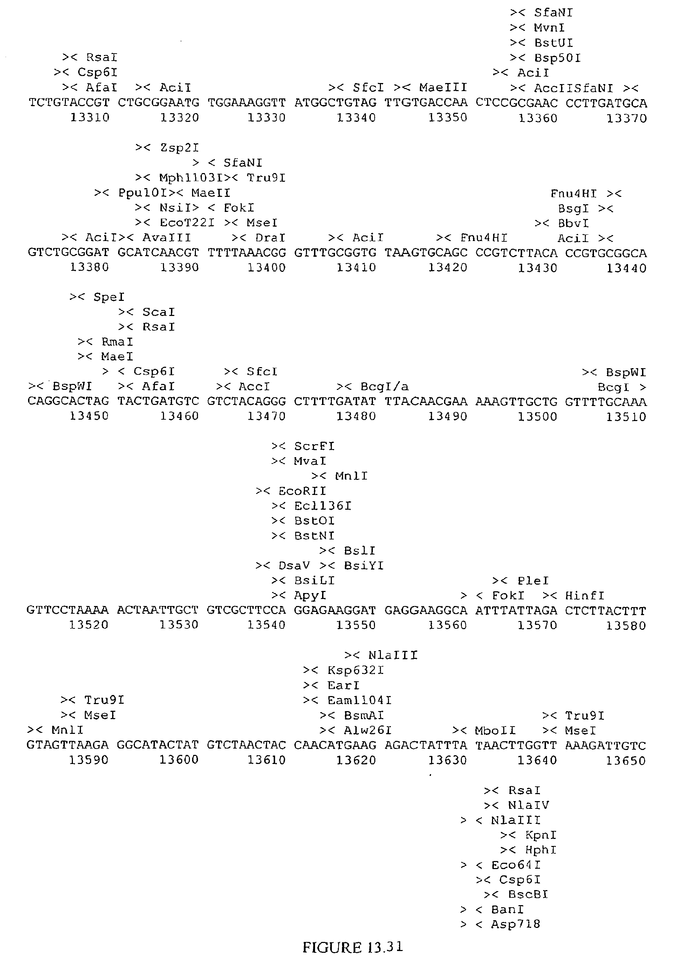

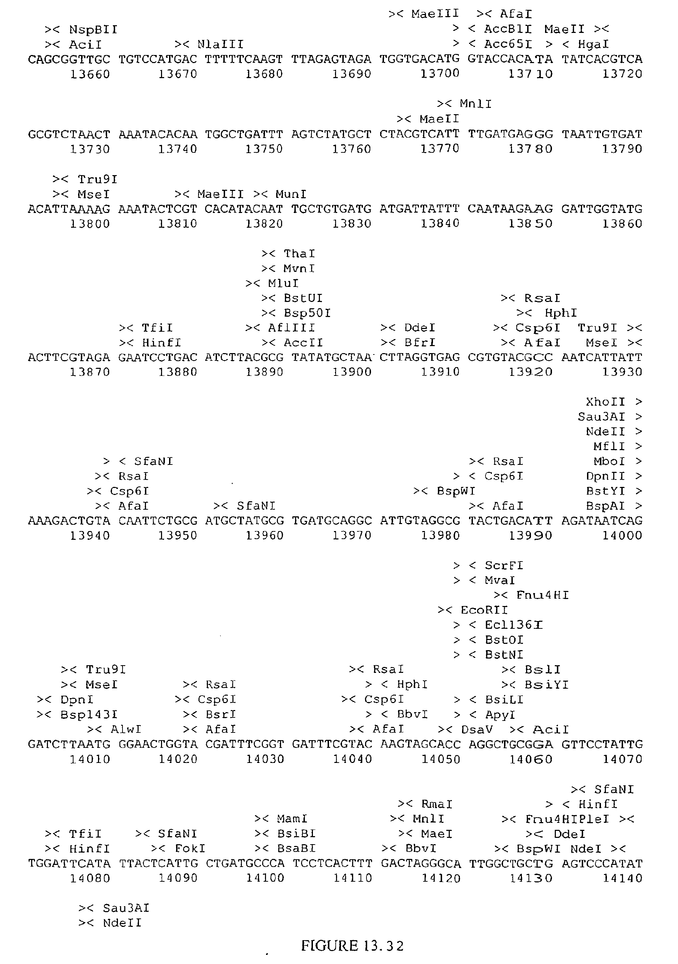

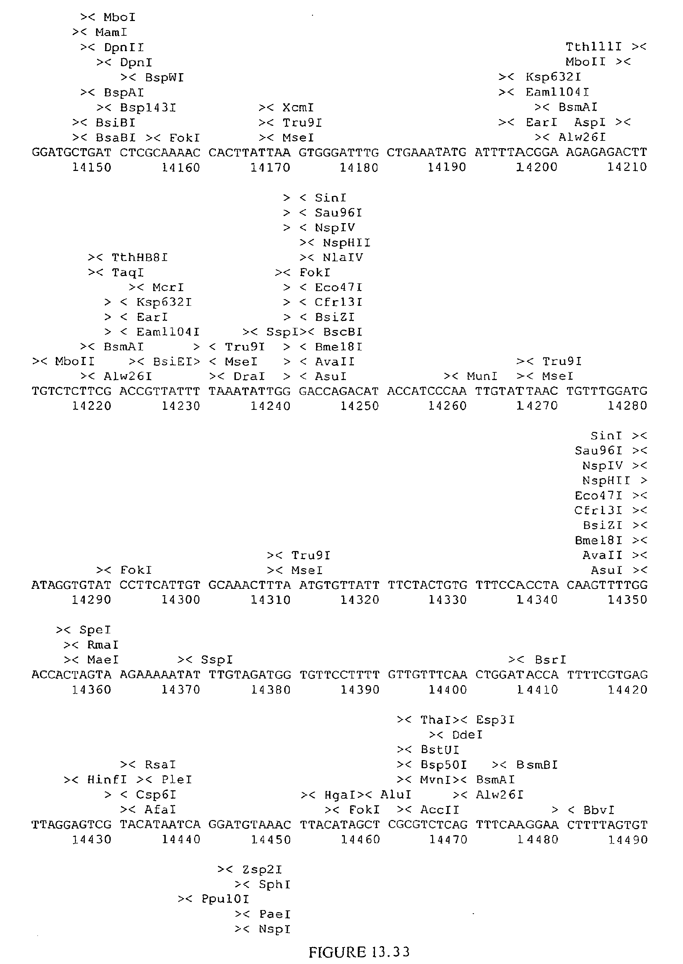

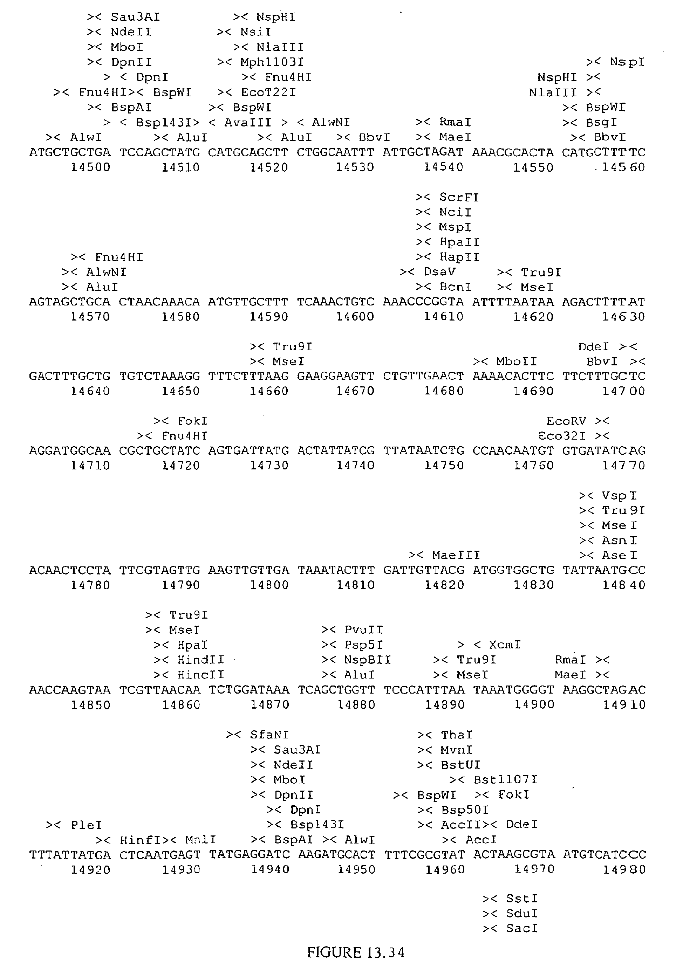

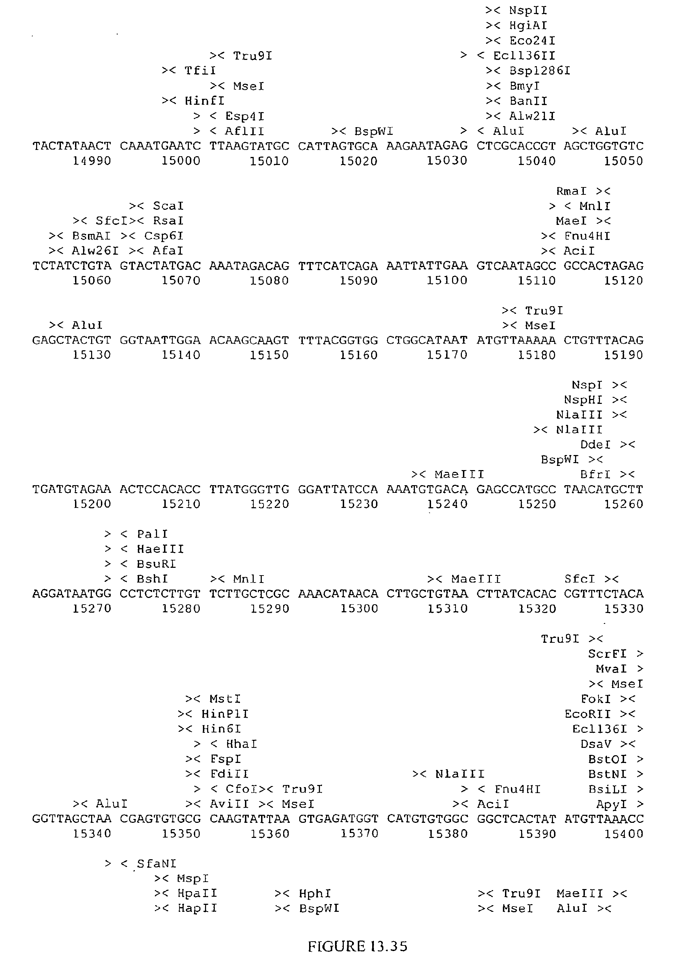

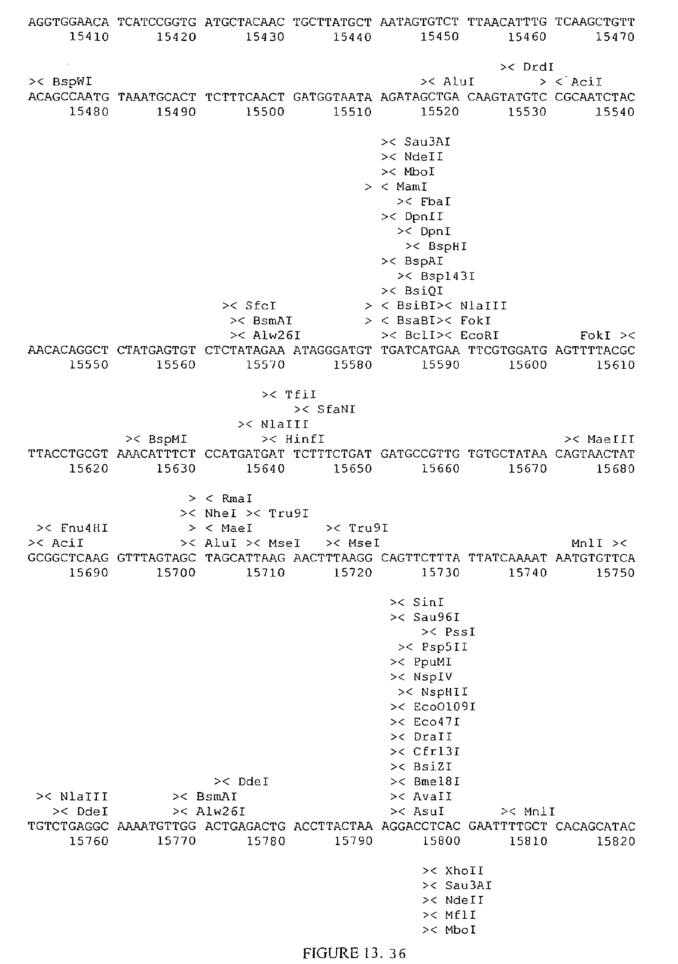

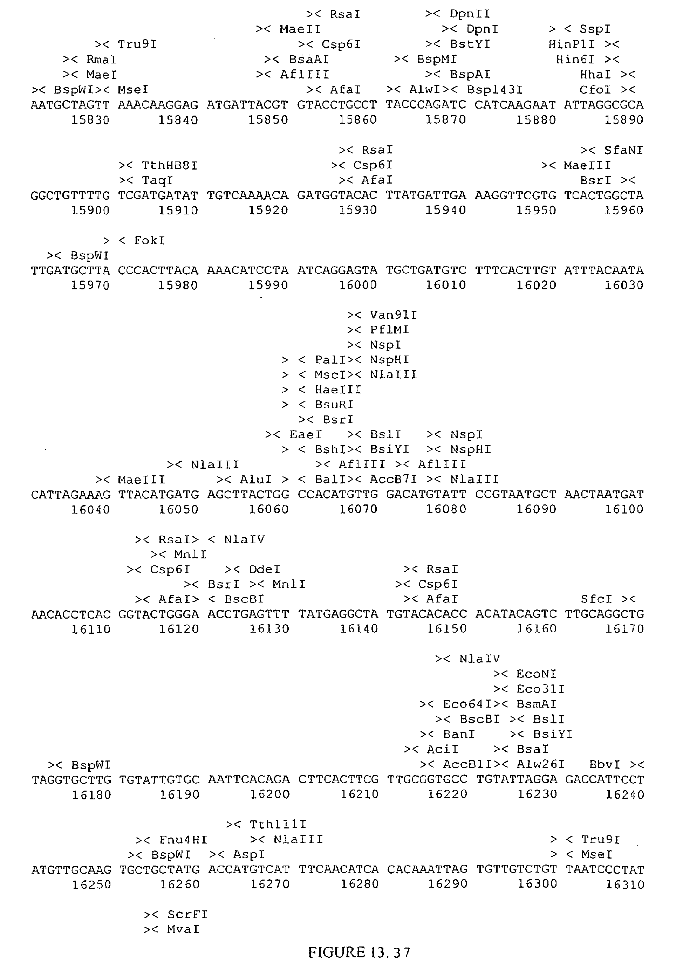

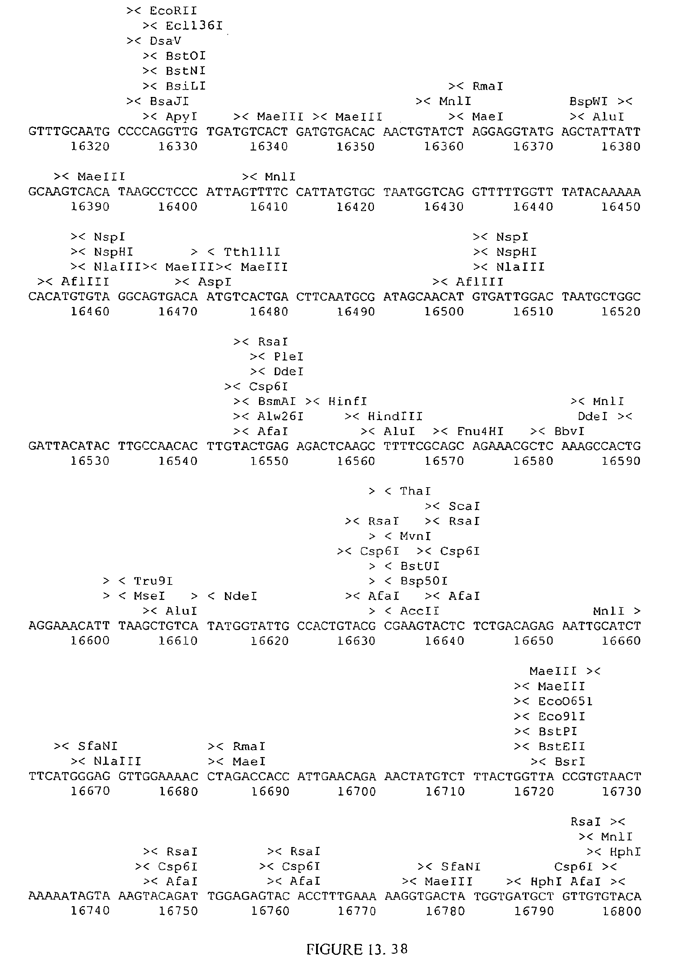

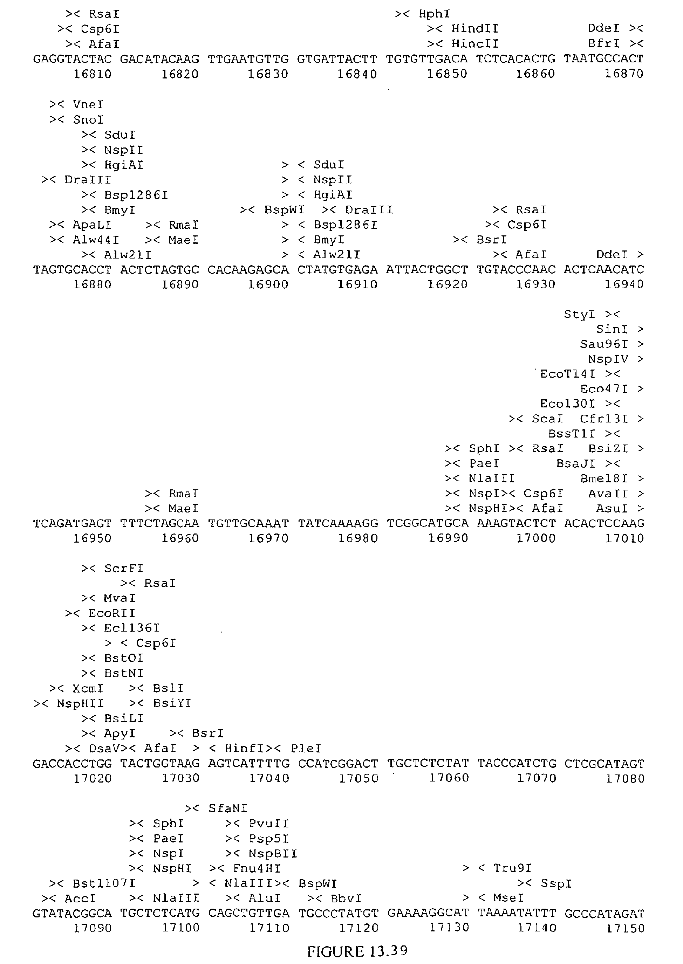

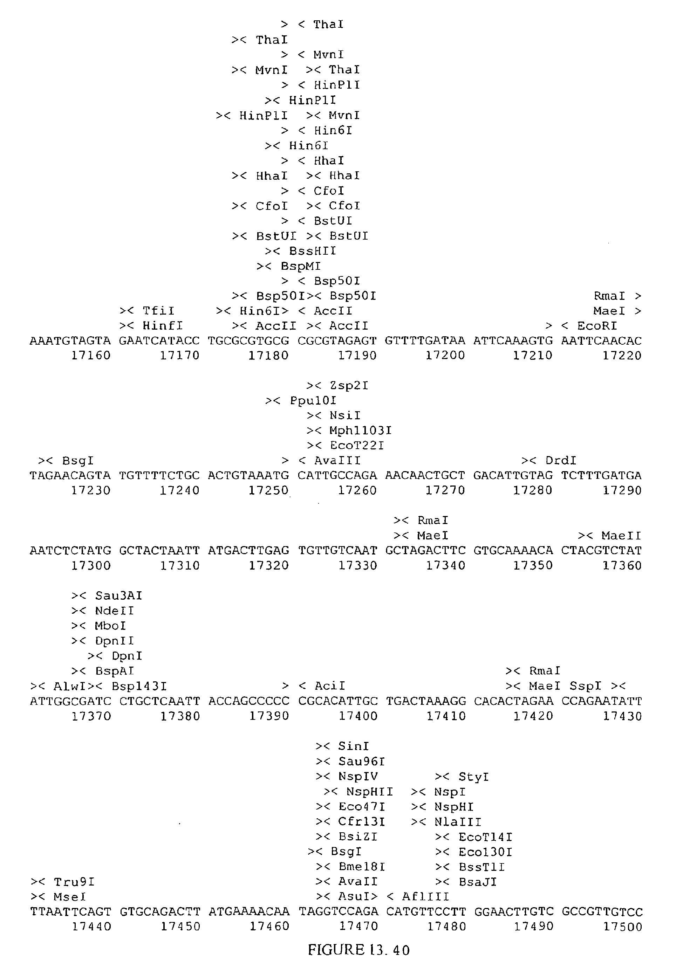

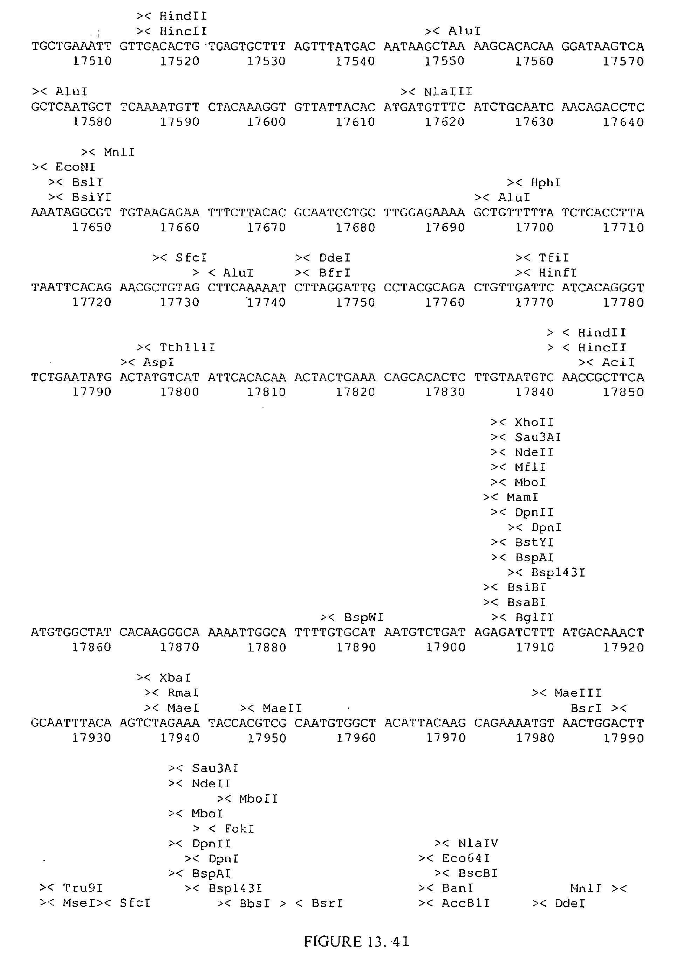

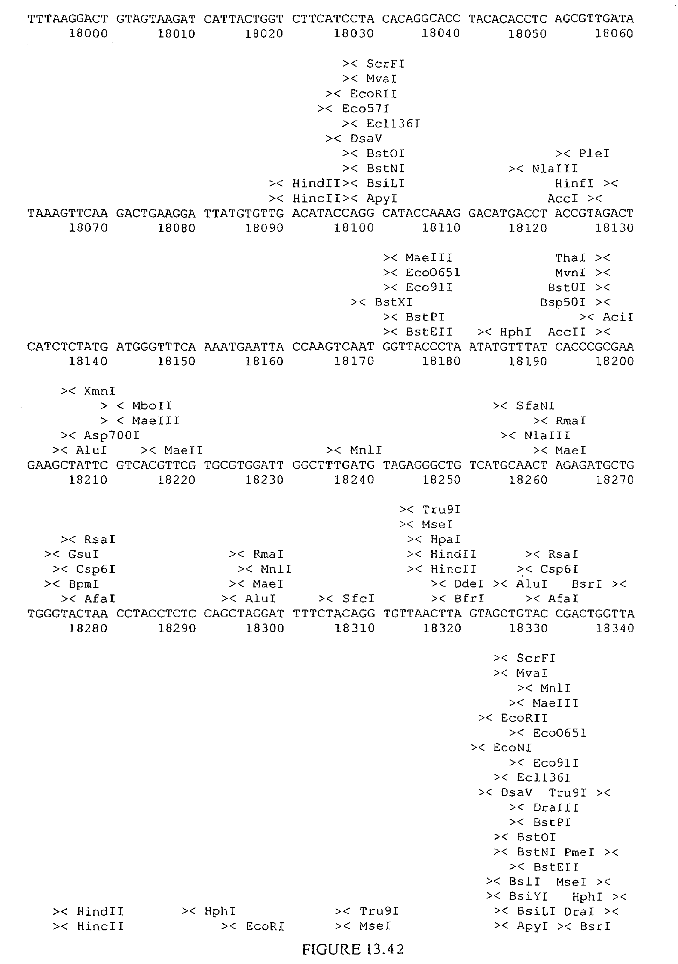

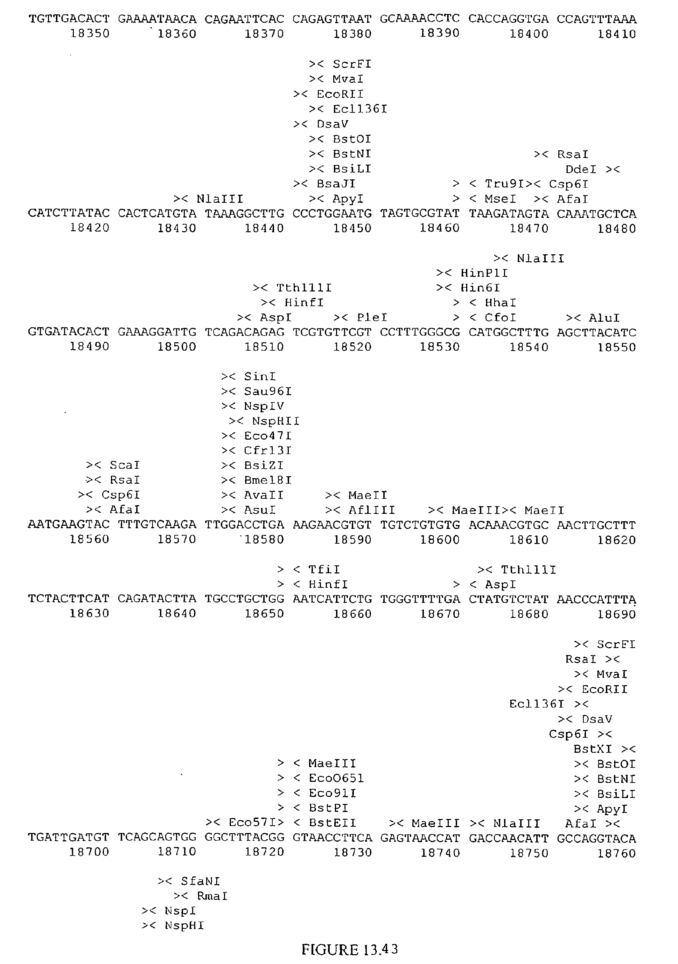

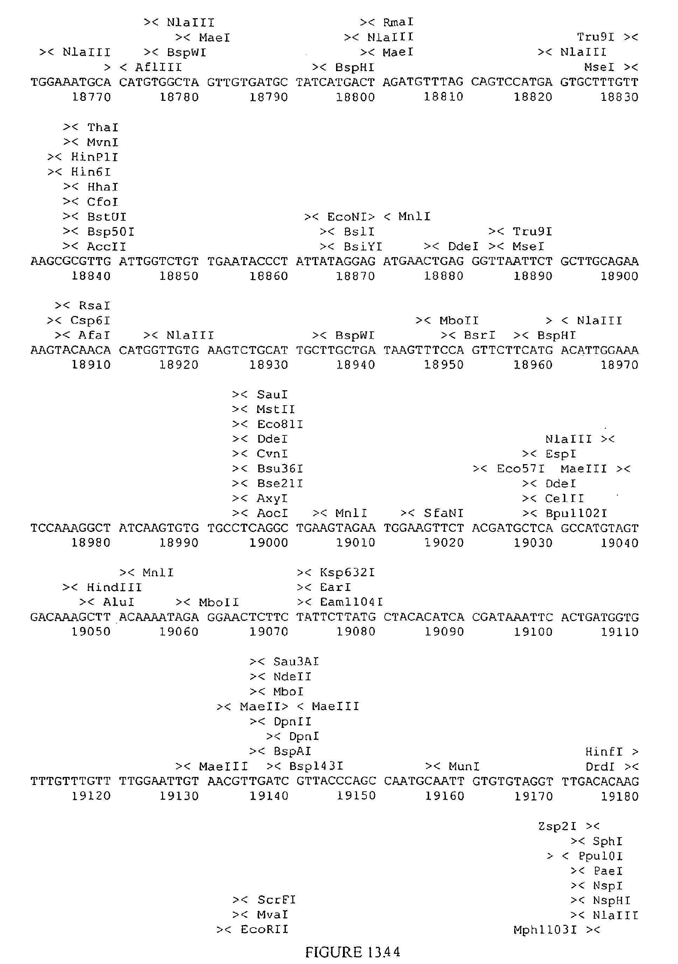

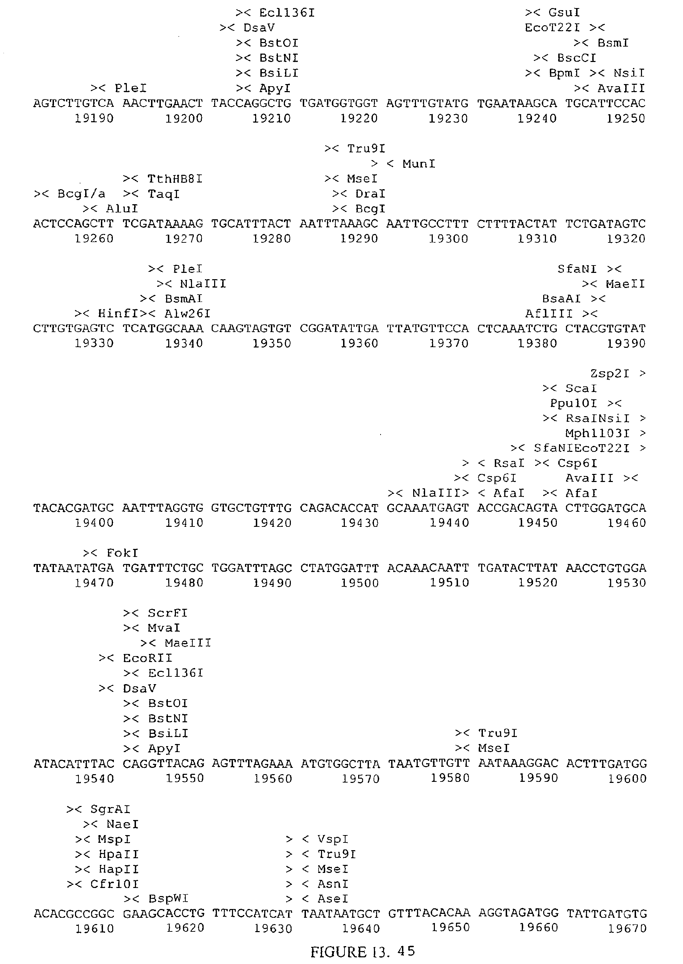

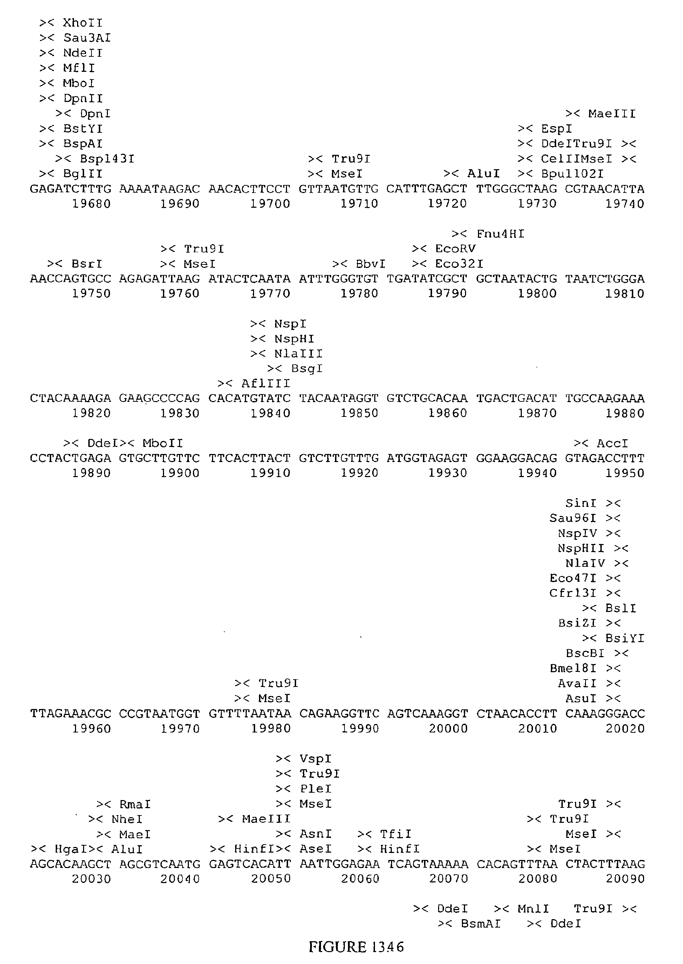

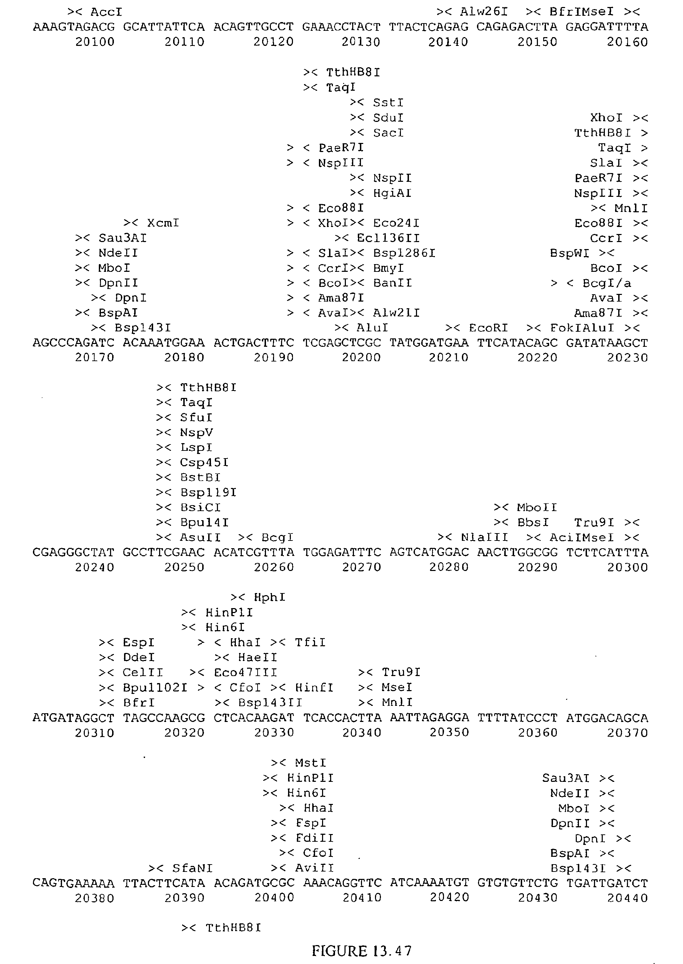

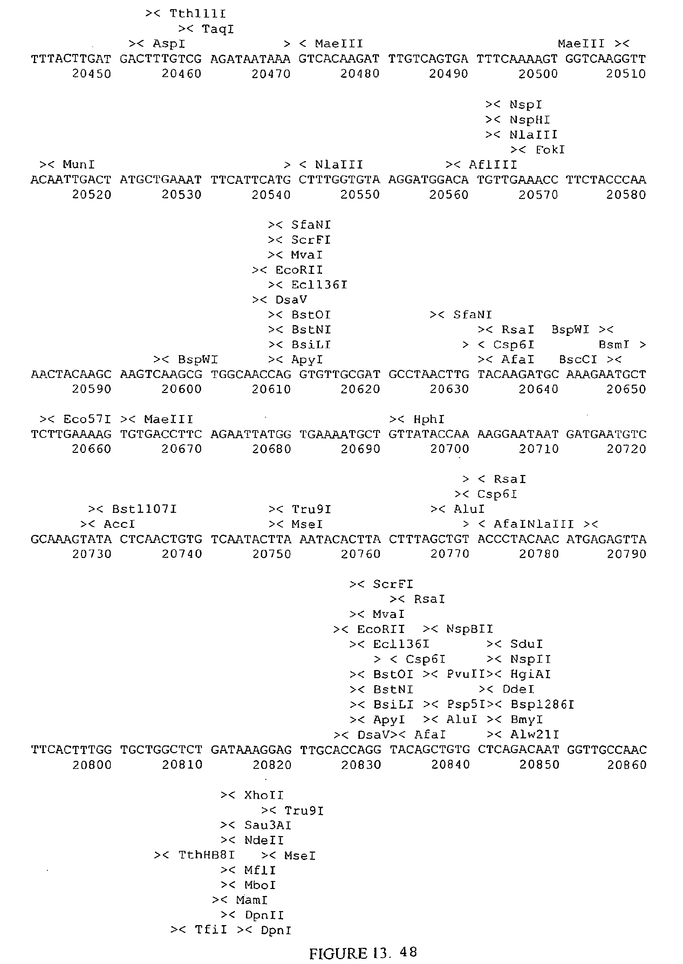

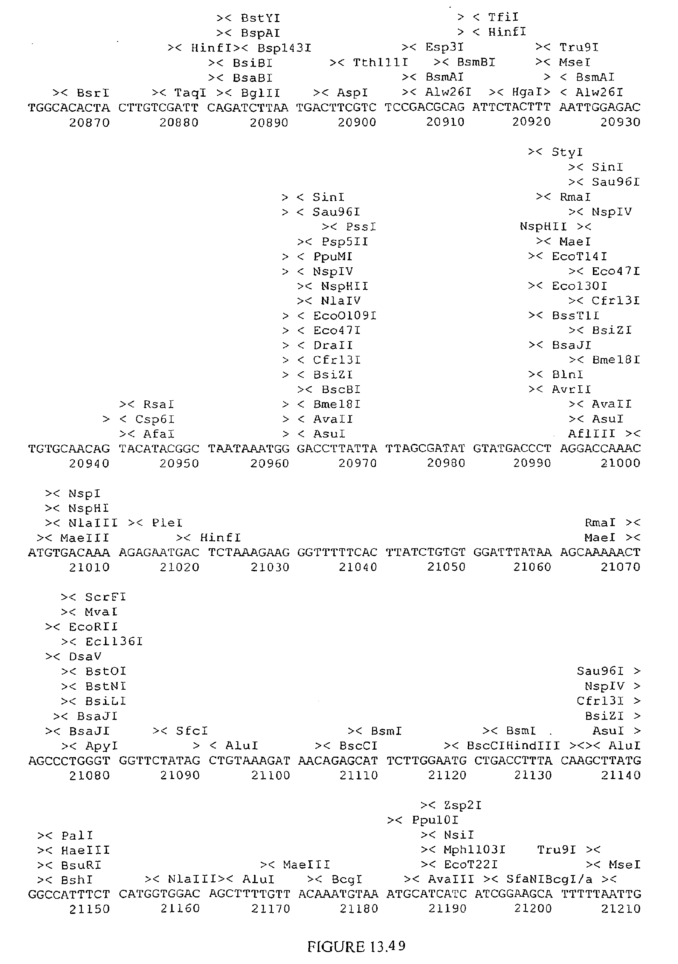

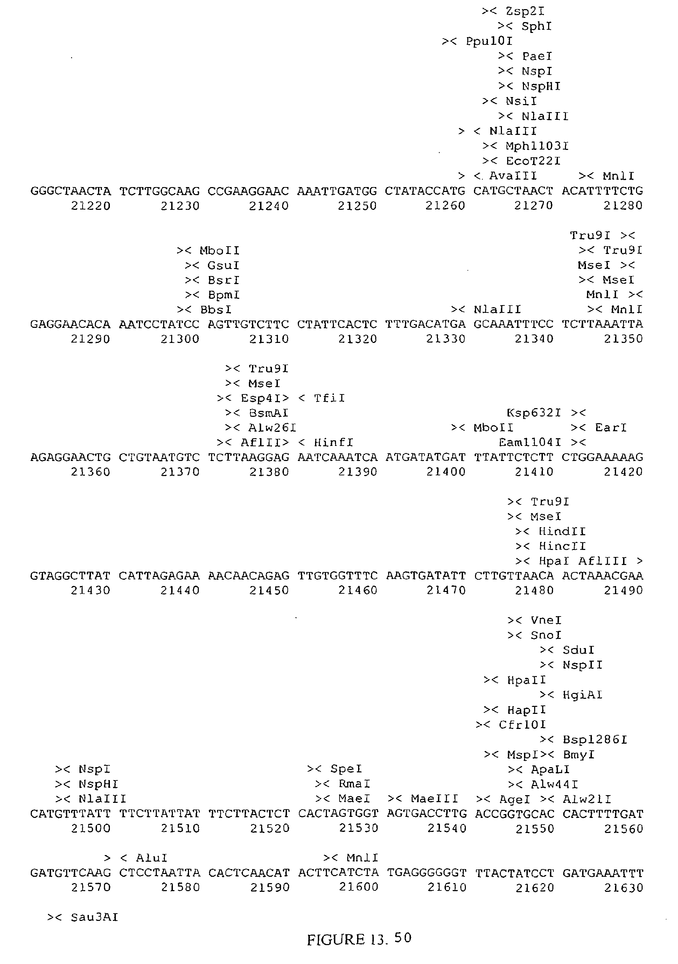

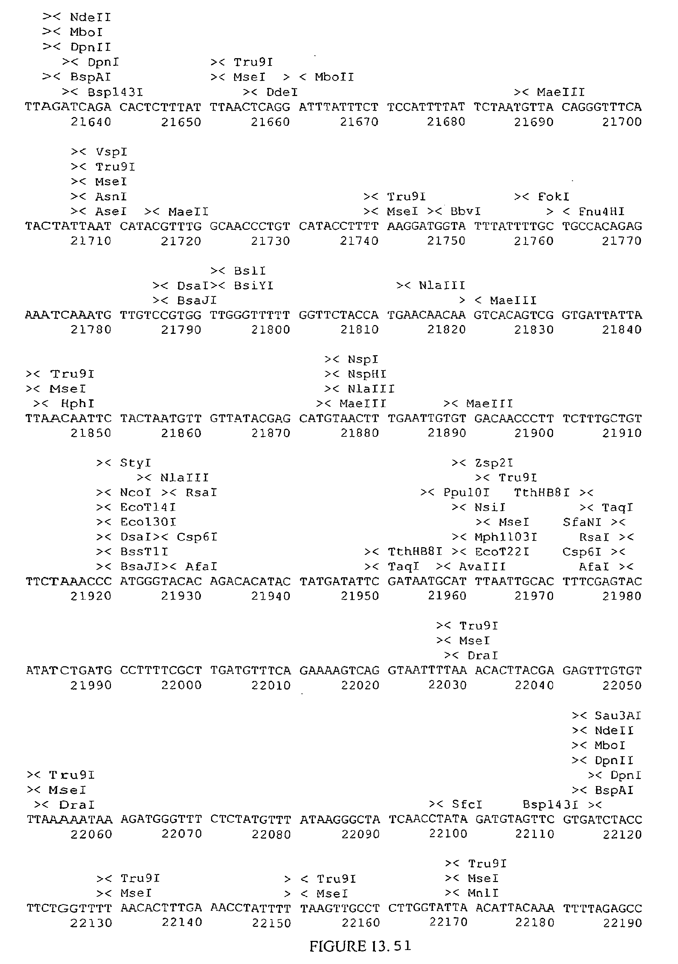

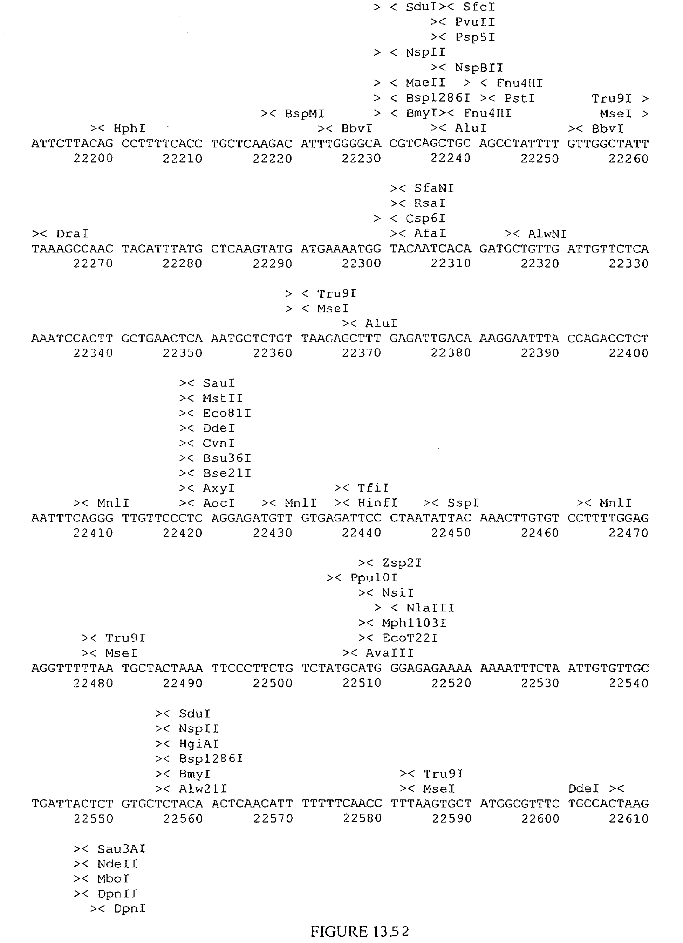

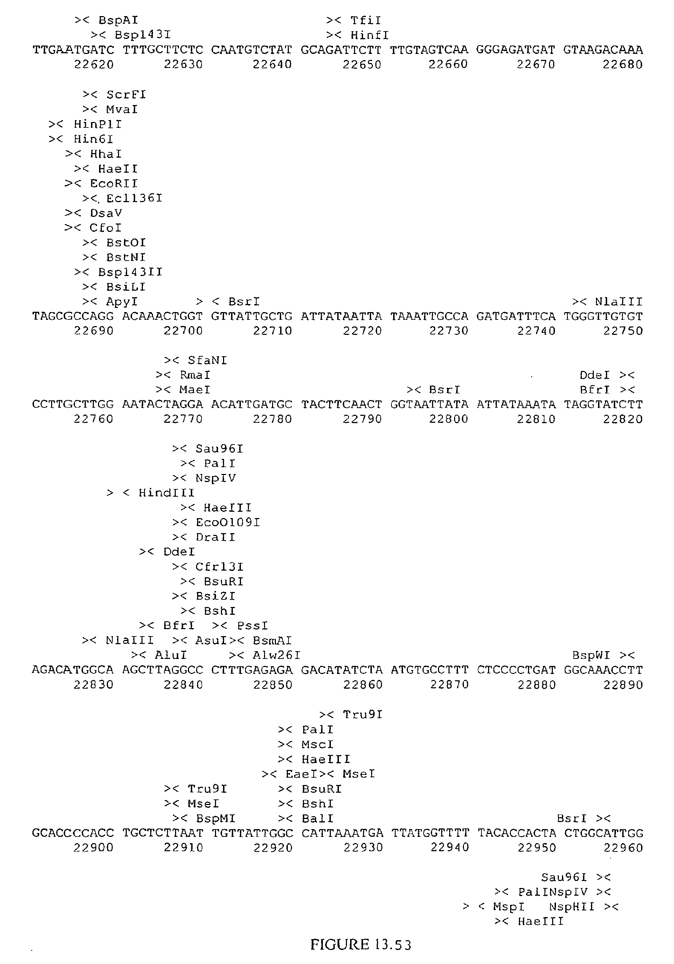

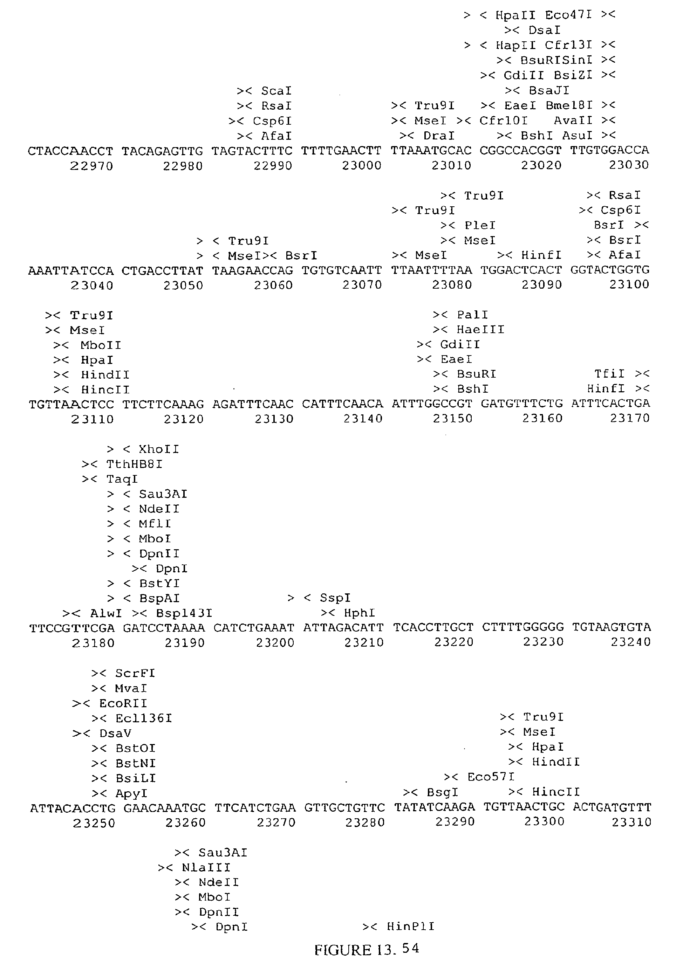

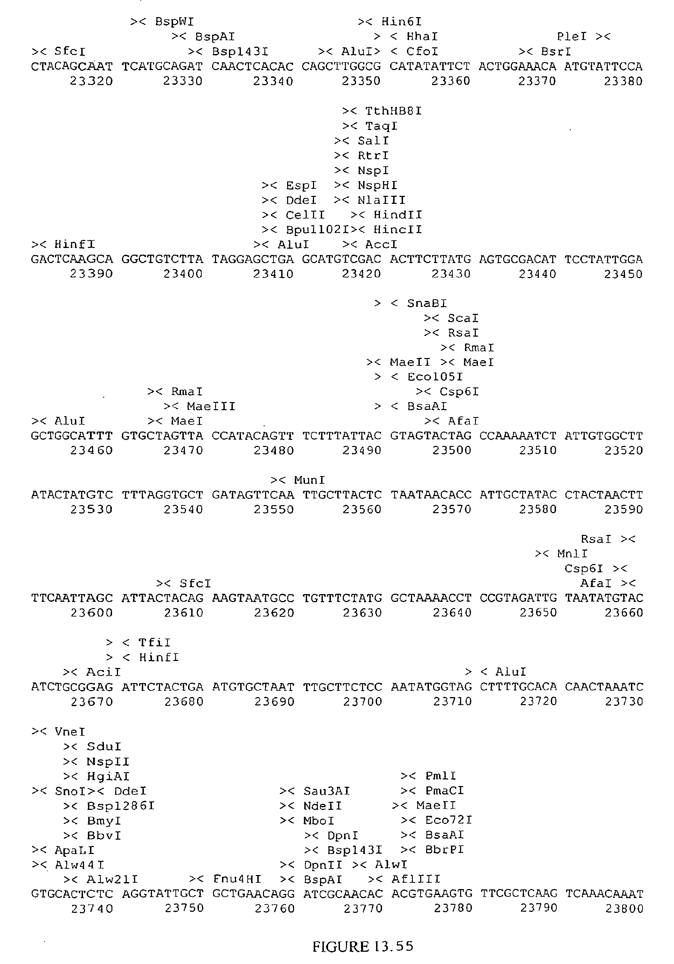

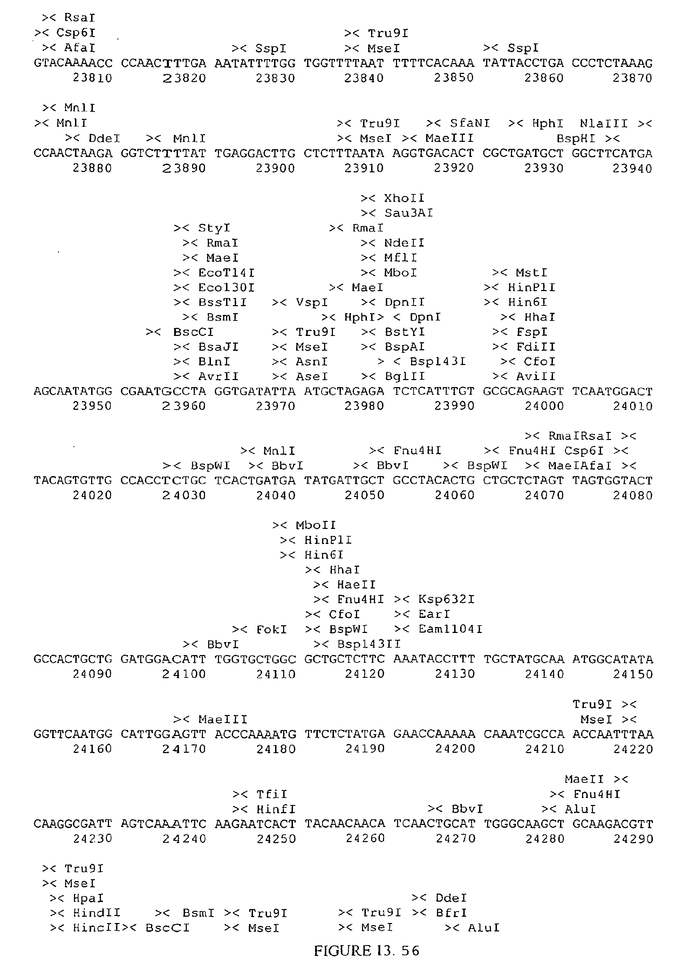

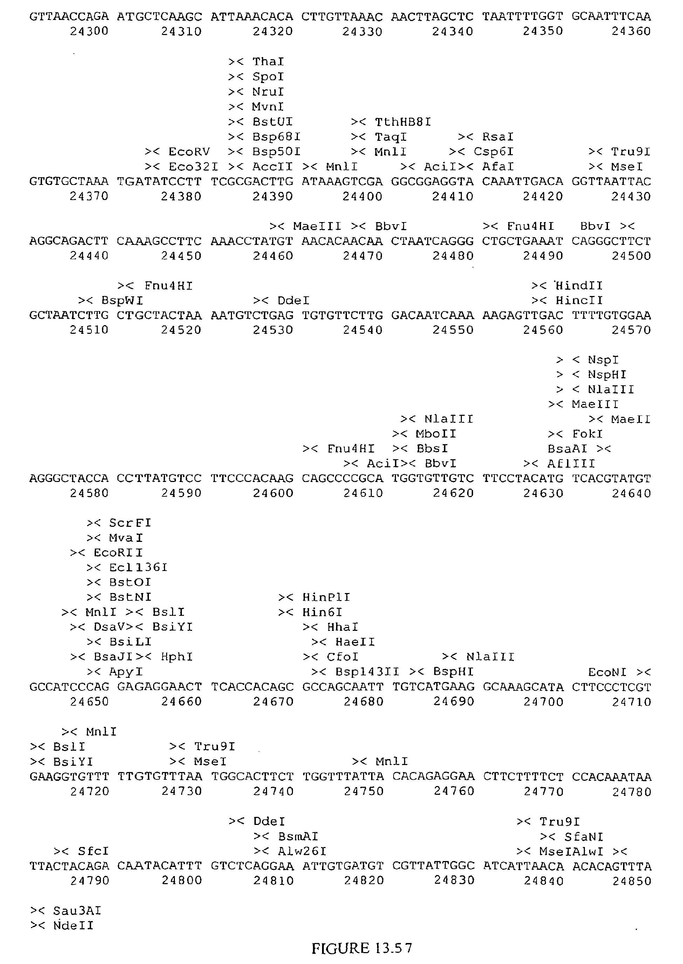

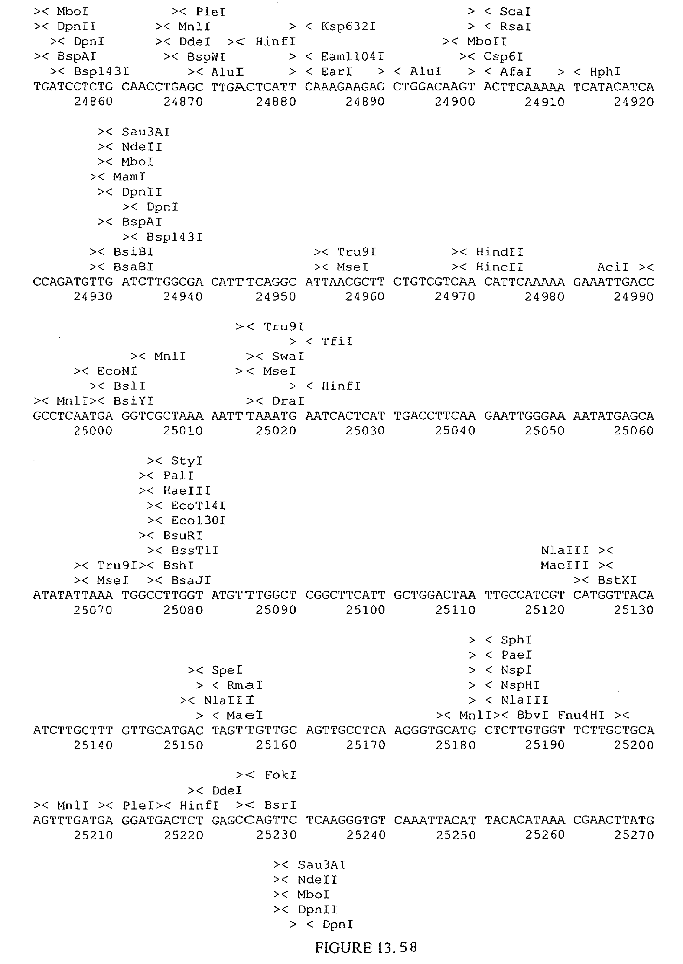

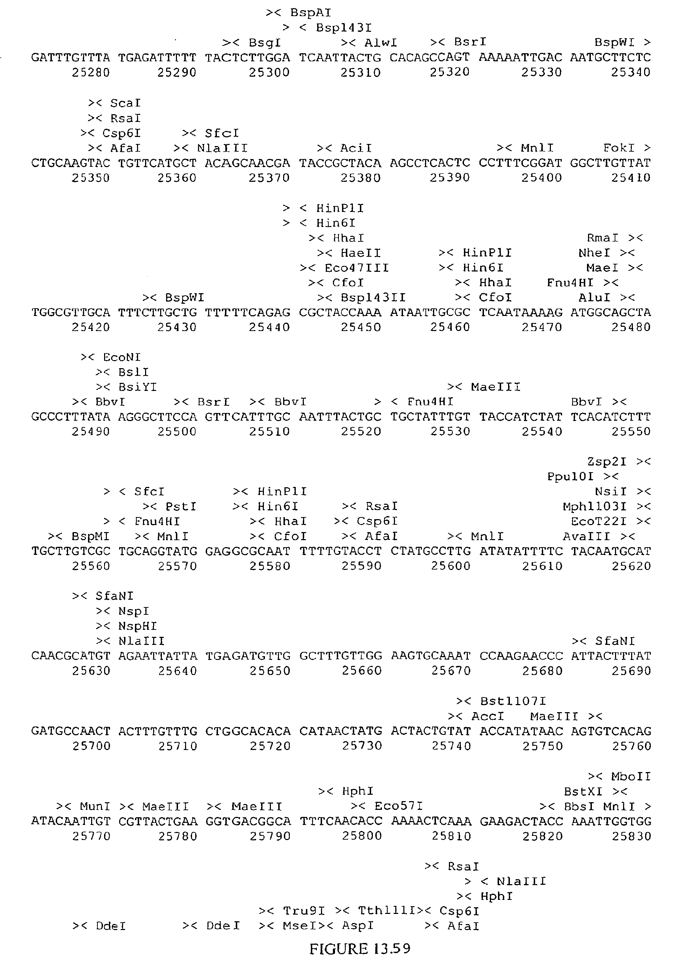

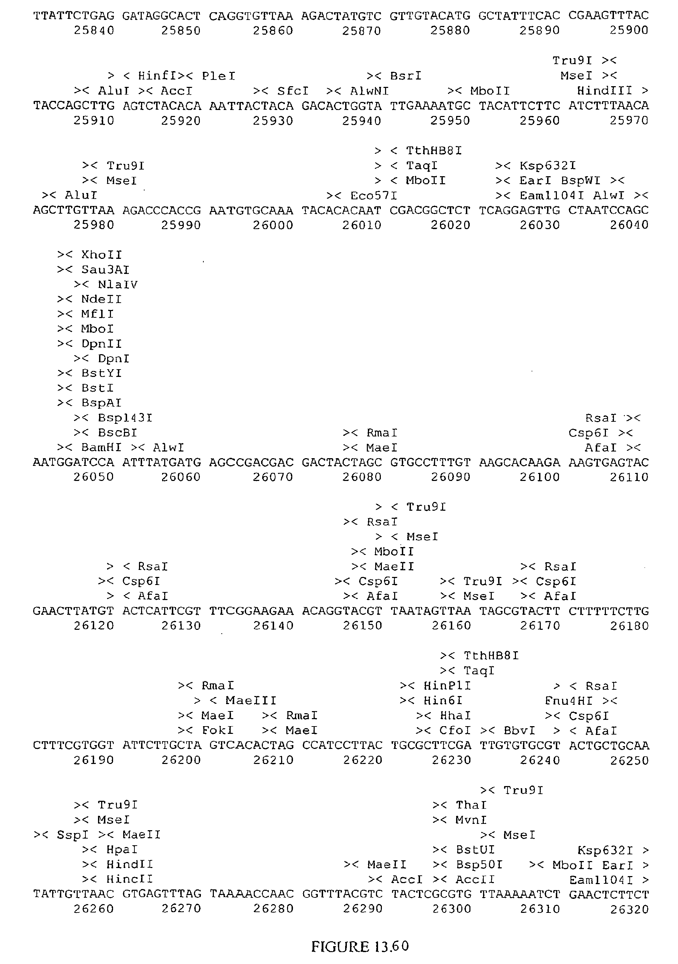

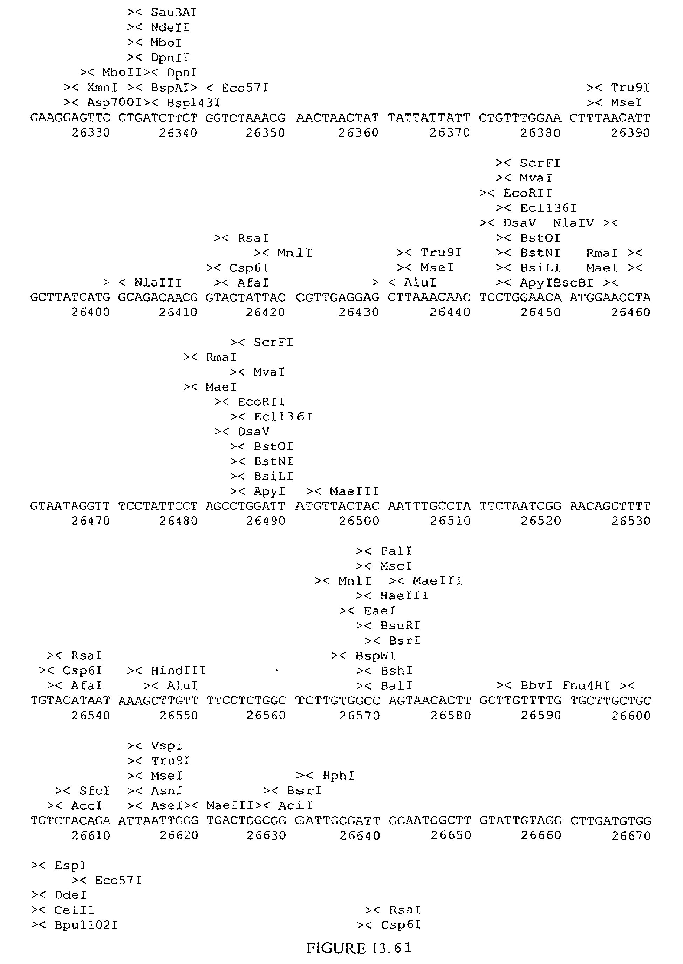

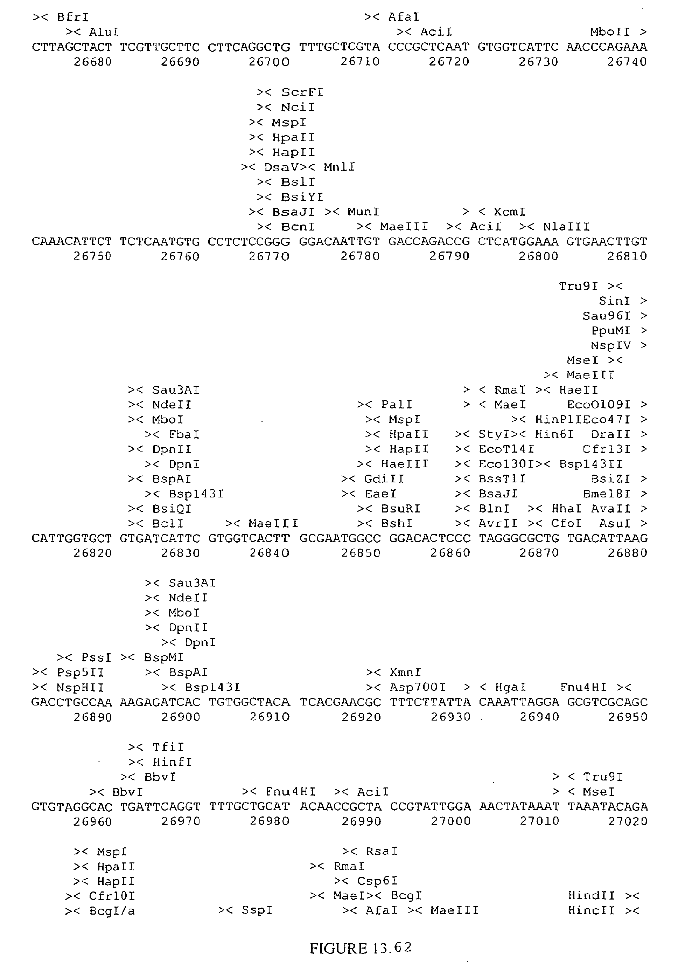

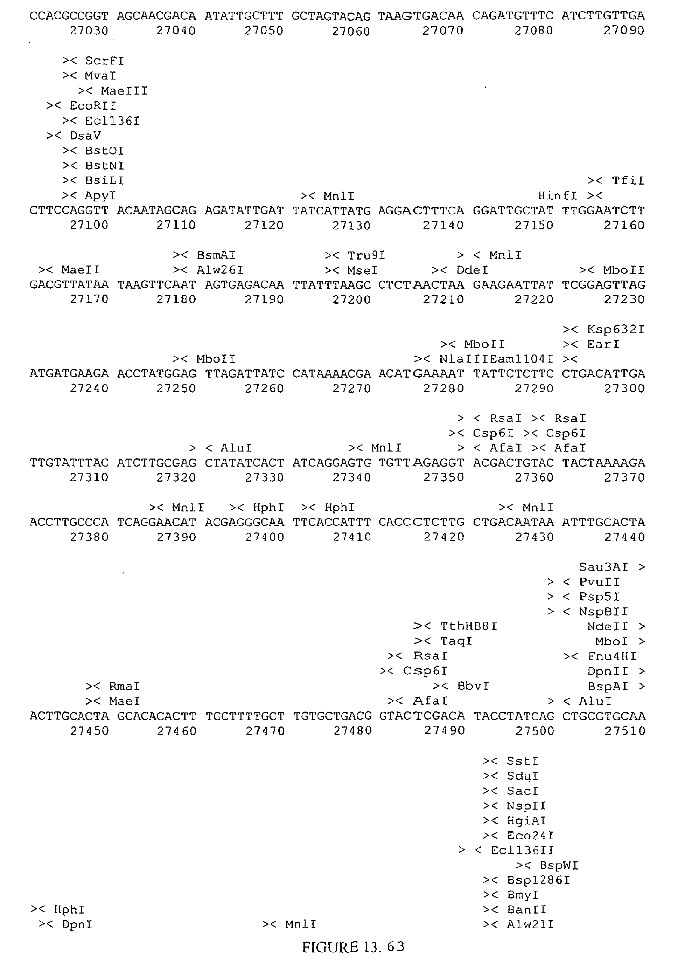

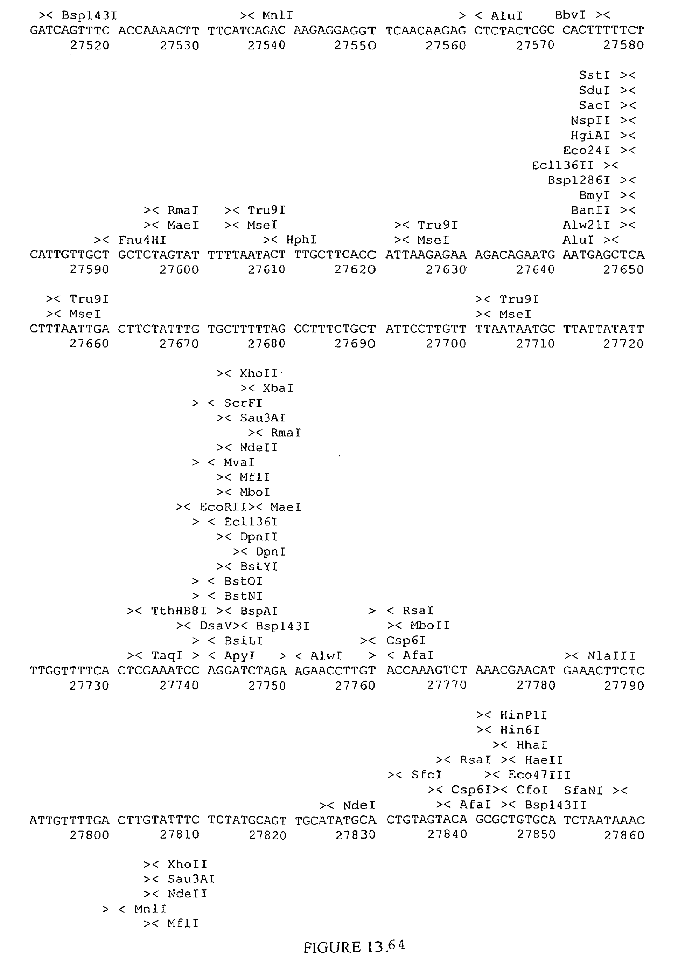

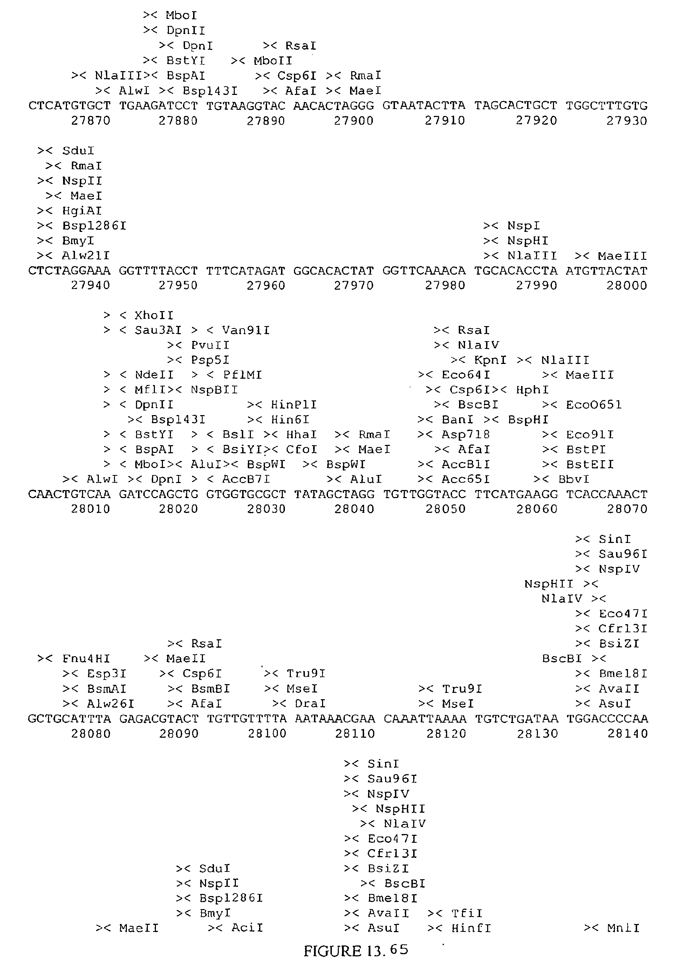

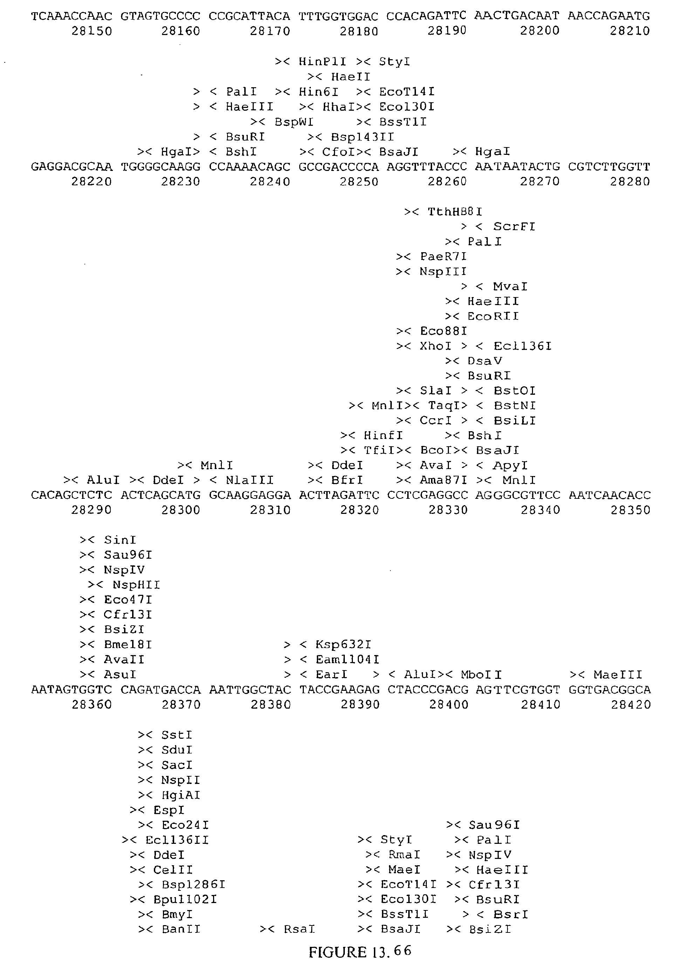

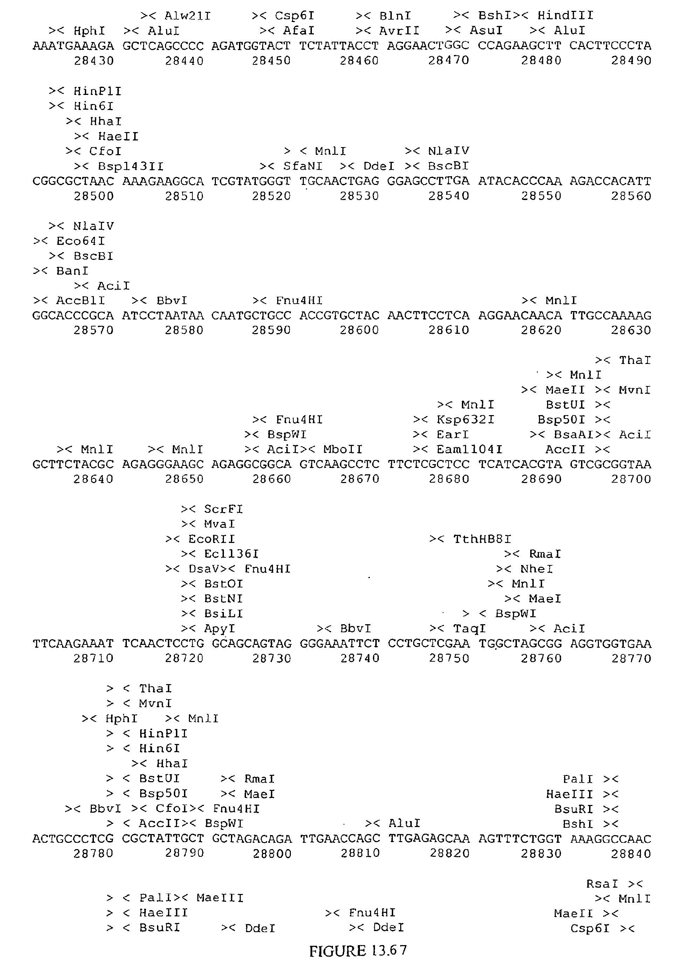

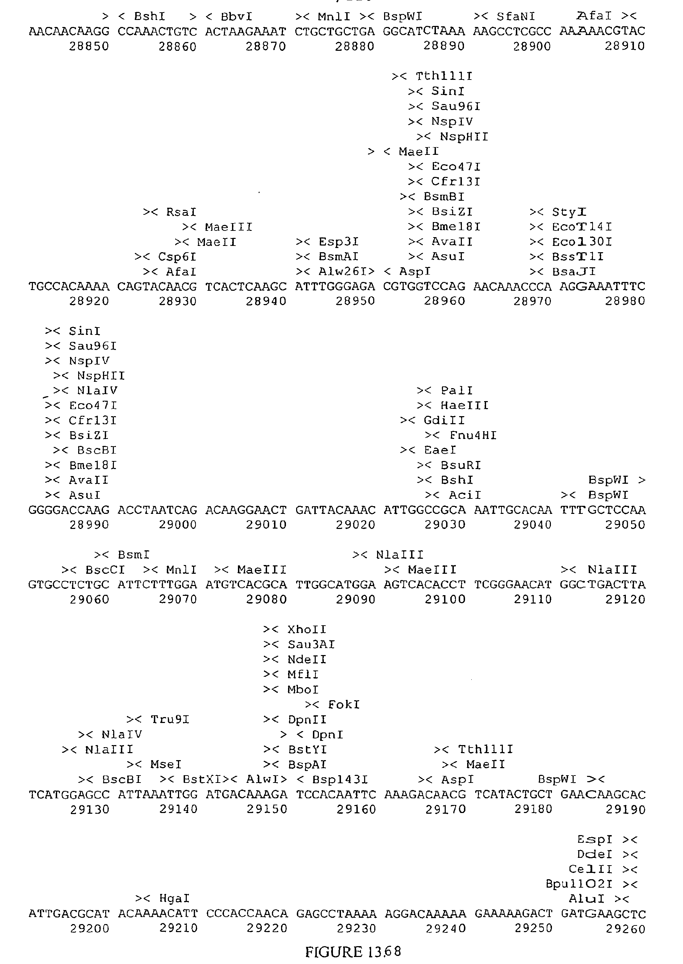

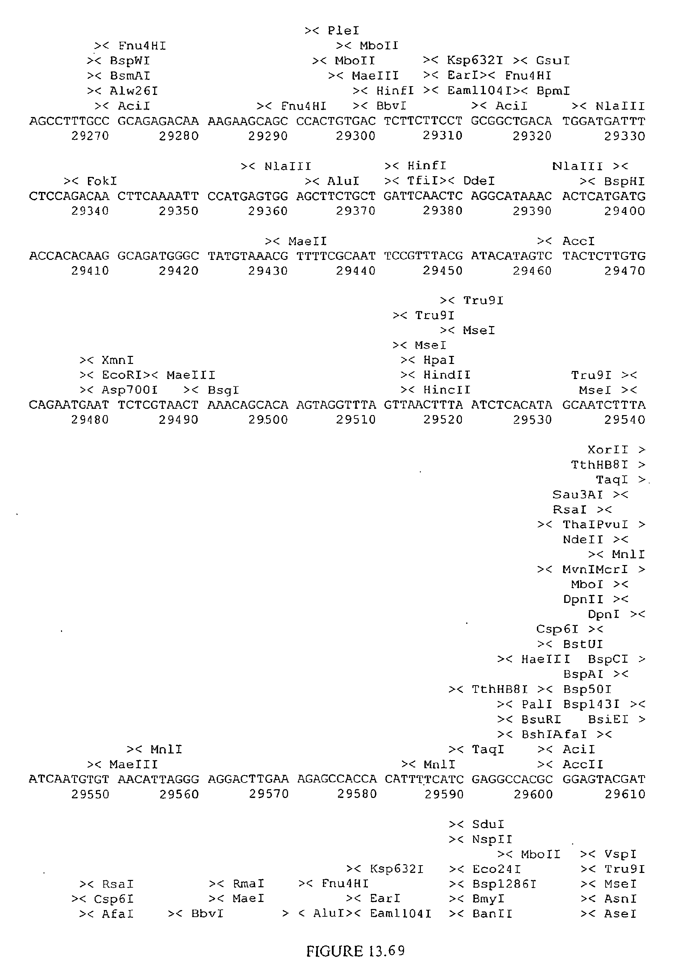

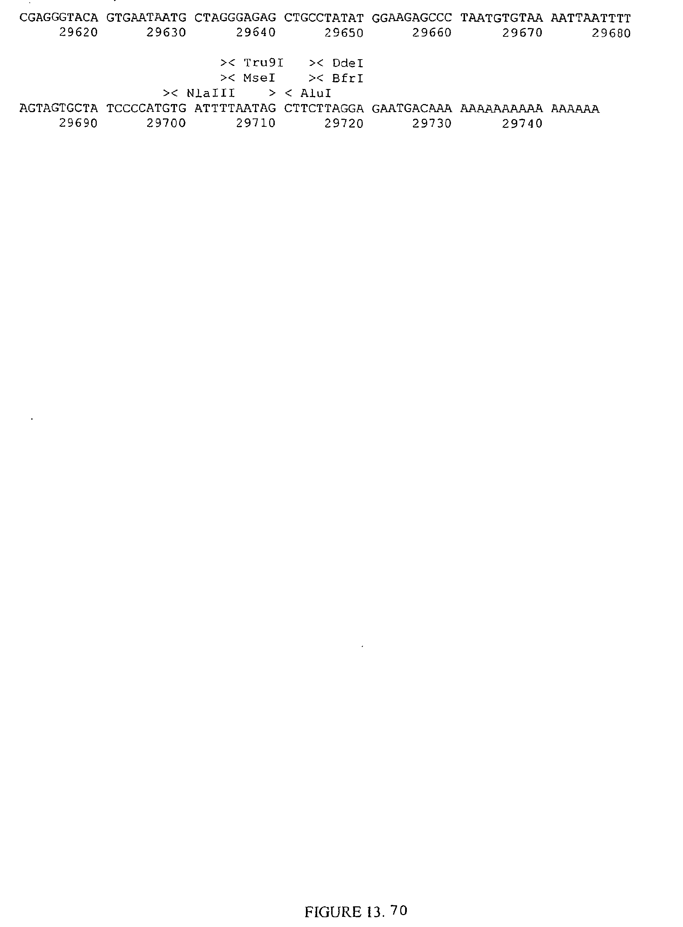

Les Inventeurs décrivent également les fragments d'amplification obtenus à l'aide d'amorces spécifiques du génome de la souche purifiée ou isolée tel que défini ci-dessus, notamment à l'aide d'amorces et de paires d'amorces telles que définies ci-dessus, les fragments de restriction constitués par ou comprenant la séquence des fragments tels que définis ci-dessus, les fragments obtenus par transcription *in vitro* à partir d'un vecteur contenant la séquence SEQ ID NO : 1 ou un fragment tel que défini ci-dessus, ainsi que des fragments obtenus par synthèse chimique. Des exemples de fragments de restriction sont déduits de la carte de restriction de la séquence SEQ ID NO : 1 illustrée par la figure 13. Lesdits fragments sont, soit sous forme de fragments isolés, soit sous forme de mélanges de fragments. La présente demande englobe également les fragments modifiés, par rapport aux précédents, par enlèvement, ou addition de nucléotides dans une proportion d'environ 15 %, par rapport à la longueur des fragments ci-dessus et/ou modifiés au niveau de la nature des nucléotides, dès lors que les fragments nucléotidiques modifiés conservent une capacité d'hybridation avec les séquences d'ARN génomiques ou antigénomiques de l'isolat tel que défini ci-dessus.

Les molécules d'acide nucléique selon l'invention sont obtenues par les méthodes classiques, connues en elles-mêmes, en suivant les protocoles standards tels que ceux décrits dans *Current Protocols in Molecular Biology (Frederick M. AUSUBEL,2000, Wiley and son Inc, Library of Congress, USA).* Par exemple, elles peuvent être obtenues par amplification d'une séquence nucléique par PCR ou RT-PCR ou bien par synthèse chimique totale ou partielle.

Les Inventeurs décrivent également une puce ou filtre à ADN ou à ARN, caractérisé en ce qu'il comprend au moins un polynucléotide ou l'un de ses fragments tels que définis ci-dessus.

Les puces ou filtres à ADN ou à ARN sont préparés par les méthodes classiques, connues en elles-mêmes, comme par exemple greffage chimique ou électrochimique d'oligonucléotides sur support de verre ou de nylon.

La présente invention a également pour objet un vecteur de clonage et/ou d'expression recombinant, notamment un plasmide, un virus, un vecteur viral ou un phage comprenant un fragment d'acide nucléique selon la présente invention. De préférence, ledit vecteur recombinant est un vecteur d'expression dans lequel ledit fragment d'acide nucléique est placé sous le contrôle d'éléments régulateurs de la transcription et de la traduction appropriés. En outre, ledit vecteur peut comprendre des séquences (étiquettes ou *tag*) fusionnées en phase avec l'extrémité 5' et/ou 3' dudit insert, utiles pour l'immobilisation, et/ou la détection et/ou la purification de la protéine exprimée à partir dudit vecteur.

Ces vecteurs sont construits et introduits dans des cellules hôtes par les méthodes classiques d'ADN recombinant et de génie génétique, qui sont connues en elles-mêmes. De nombreux vecteurs dans lesquels on peut insérer une molécule d'acide nucléique d'intérêt afin de l'introduire et de la maintenir dans une cellule hôte, sont connus en eux-mêmes ; le choix d'un vecteur approprié dépend de l'utilisation envisagée pour ce vecteur (par exemple réplication de la séquence d'intérêt, expression de cette séquence, maintien de la séquence sous forme extrachromosomique ou bien intégration dans le matériel chromosomique de l'hôte), ainsi que de la nature de la cellule hôte.

Les Inventeurs décrivent les plasmides suivants :
- le plasmide, dénommé SARS-S, compris dans la souche bactérienne déposée sous le n° 1-3059, le 20 juin 2003, auprès de la Collection Nationale de Cultures de Microorganismes, 25 rue du Docteur Roux, 75724 Paris Cedex 15 ; il contient la séquence d'ADNc codant pour la protéine S de la souche de SARS-CoV issue du prélèvement répertorié sous le n° 031589, laquelle séquence correspondant aux nucléotides des positions 21406 à 25348 (SEQ ID NO : 4), en référence à la séquence Genbank AY274119.3,
- le plasmide, dénommé SARS-S1, compris dans la souche bactérienne déposée sous le n° I-3020, le 12 mai 2003, auprès de la Collection Nationale de Cultures de Microorganismes, 25 rue du Docteur Roux, 75724 Paris Cedex 15 ; il contient un fragment 5' de la séquence d'ADNc codant pour la protéine S de la souche de SARS-CoV issue du prélèvement répertorié sous le n° 031589, telle que définie ci-dessus, lequel fragment correspondant aux nucléotides des positions 21406 à 23454 (SEQ ID NO :5), en référence à la séquence Genbank AY274119.3 Tor2,
- le plasmide, dénommé SARS-S2, compris dans la souche bactérienne déposée sous le n° I-3019, le 12 mai 2003, auprès de la Collection Nationale de Cultures de Microorganismes, 25 rue du Docteur Roux, 75724 Paris Cedex 15 ; il contient un fragment 3'de la séquence d'ADNc codant pour la protéine S de la souche de SARS-CoV issue du prélèvement répertorié sous le n° 031589, telle que définie ci-dessus, lequel fragment correspondant aux nucléotides des positions 23322 à 25348 (SEQ ID NO :6), en référence à la séquence Genbank n° d'accès AY274119.3,
- le plasmide, dénommé SARS-SE, compris dans la souche bactérienne déposée sous le n° 1-3126, le, 13 novembre 2003, auprès de la Collection Nationale de Cultures de Microorganismes, 25 rue du Docteur Roux, 75724 Paris Cedex 15; il contient l'ADNc correspondant à la région située entre l'ORF-S et l'ORF-E et chevauchant l'ORF-E de la souche de SARS-CoV issue du prélèvement répertorié sous le n° 031589, telle que définie ci-dessus, laquelle région correspondant aux nucléotides des positions 25110 à 26244 (SEQ ID NO :8), en référence à la séquence Genbank n° d'accès AY274119.3,
- le plasmide, dénommé SARS-E, compris dans la souche bactérienne déposée sous le n° 1-3046, le 28 mai 2003, auprès de la Collection Nationale de Cultures de Microorganismes, 25 rue du Docteur Roux, 75724 Paris Cedex 15 ; il contient la séquence d'ADNc codant pour la protéine E de la souche de SARS-CoV issue du prélèvement répertorié sous le n° 031589, telle que définie ci-dessus, laquelle séquence correspondant aux nucléotides des positions 26082 à 26413 (SEQ ID NO :15), en référence à la séquence Genbank n° d'accès AY274119.3,
- le plasmide, dénommé SARS-M ; compris dans la souche bactérienne déposée sous le n° 1-3047, le 28 mai 2003, auprès de la Collection Nationale de Cultures de Microorganismes, 25 rue du Docteur Roux, 75724 Paris Cedex 15 ; il contient la séquence d'ADNc codant pour la protéine M de la souche de SARS-CoV issue du prélèvement répertorié sous le n° 031589, telle que définie ci-dessus; laquelle séquence correspondant aux nucléotides des positions 26330 à 27098 (SEQ ID NO :18), en référence à la séquence Genbank n° d'accès AY274119.3,
- le plasmide dénommé SARS-MN, compris dans la souche bactérienne déposée sous le n° I-3125, le 13 novembre 2003, auprès de la Collection Nationale de Cultures de Microorganismes, 25 rue du Docteur Roux, 75724 Paris Cedex 15 ; il contient la séquence d'ADNc correspondant à la région située entre l'ORF-M et l'ORF-N de la souche de SARS-CoV issue du prélèvement répertorié sous le n° 031589 et prélevée à Hanoi, telle que définie ci-dessus, laquelle séquence correspondant aux nucléotides des positions 26977 à 28218 (SEQ ID NO :20), en référence à la séquence Genbank n° d'accès AY274119.3,
- le plasmide dénommé SARS-N, compris dans la souche bactérienne déposée sous le n° 1-3048, le 5 juin 2003, auprès de la Collection Nationale de Cultures de Microorganismes, 25 rue du Docteur Roux, 75724 Paris Cedex 15 ; il contient l'ADNc codant pour la protéine N de la souche de SARS-CoV issue du prélèvement répertorié sous le n° 031589, telle que définie ci-dessus, laquelle séquence correspondant aux nucléotides des positions 28054 à 29430 (SEQ ID NO :38), en référence à la séquence Genbank n° d'accès AY274119.3 ; ainsi ce plasmide comprend un insert de séquence SEQ ID NO :38 et est compris dans une souche bactérienne qui et qu'il a été déposée sous le n° 1-3048, le 5 juin 2003, auprès de la Collection Nationale de Cultures de Microorganismes, 25 rue du Docteur Roux, 75724 Paris Cedex 15,
- le plasmide dénommé SARS-5'NC, compris dans la souche bactérienne déposée sous le n° I- 3124, le 7 novembre 2003, auprès de la Collection Nationale de Cultures de Microorganismes, 25 rue du Docteur Roux, 75724 Paris Cedex 15 ; il contient l'ADNc correspondant à l'extrémité 5'non codante du génome de la souche de SARS-CoV issue du prélèvement répertorié sous le n° 031589, telle que définie ci-dessus, laquelle séquence correspondant aux nucléotides des positions 1 à 204 (SEQ ID NO :39), en référence à la séquence Genbank n° d'accès AY274119.3,
- le plasmide dénommé SARS-3'NC, compris dans la souche bactérienne déposée sous le n° 1-3123 le 7 novembre 2003, auprès de la Collection Nationale de Cultures de Microorganismes, 25 rue du Docteur Roux, 75724 Paris Cedex 15. ; il contient la séquence d'ADNc correspondant à l'extrémité 3'non codante du génome de la souche de SARS-CoV issue du prélèvement répertorié sous le n° 031589, telle que définie ci-dessus, laquelle séquence correspondant à celle située entre le nucléotide en position 28933 à 29727 (SEQ ID NO :40), en référence à la séquence Genbank n° d'accès AY274119.3, se termine par une série de nucléotides a.,
- le plasmide d'expression dénommé pIV2.3N, contenant un fragment d'ADNc codant pour une fusion C-terminale de la protéine N (SEQ ID NO : 37) avec une étiquette polyhistidine,
- le plasmide d'expression dénommé pIV2.3S_{C}, contenant un fragment d'ADNc codant pour une fusion C-terminale du fragment correspondant aux positions 475 à 1193 de la séquence en acides aminés de la protéine S (SEQ ID NO : 3) avec une étiquette polyhistidine,
- le plasmide d'expression pIV2.3S_{L}, contenant un fragment d'ADNc codant pour une fusion C-terminale du fragment correspondant aux positions 14 à 1193 de la séquence en acides aminés de la protéine S (SEQ ID NO : 3) avec une étiquette polyhistidine,
- le plasmide d'expression dénommé pIV2.4N, contenant un fragment d'ADNc codant pour une fusion N-terminale de la protéine N (SEQ ID NO : 3) avec une étiquette polyhistidine,
- le plasmide d'expression dénommé pIV2.4S_{C} ou pIV2.4S₁, contenant un insert codant pour une fusion N-terminale du fragment correspondant aux positions 475 à 1193 de la séquence en acides aminés de la protéine S (SEQ ID NO : 3) avec une étiquette polyhistidine, et
- le plasmide d'expression dénommé pIV2.4S_{L} contenant un fragment d'ADNc codant pour une fusion N-terminale du fragment correspondant aux positions 14 à 1193 de la séquence en acides aminés de la protéine S (SEQ ID NO : 3) avec une étiquette polyhistidine.

Selon une disposition avantageuse du plasmide d'expression tel que défini ci-dessus, il est compris dans une souche bactérienne qui a été déposée sous le n° I- 3117, le 23 octobre 2003, auprès de la Collection Nationale de Cultures de Microorganismes, 25 rue du Docteur Roux, 75724 Paris Cedex 15.

Selon une autre disposition avantageuse du plasmide d'expression tel que défini ci-dessus, il est compris dans une souche bactérienne qui a été déposée sous le n° I- 3118, le 23 octobre 2003, auprès de la Collection Nationale de Cultures de Microorganismes, 25 rue du Docteur Roux, 75724 Paris Cedex 15.

Selon une autre disposition du plasmide d'expression tel que défini ci-dessus, il est compris dans une souche bactérienne qui a été déposée à la CNCM, 25 rue du Docteur Roux, 75724 Paris Cedex 15 sous les numéros suivants :
a) souche n° I- 3118, déposée le 23 octobre 2003,
b) souche n° I- 3019, déposée le 12 mai 2003,
c) souche n° I- 3020, déposée le 12 mai 2003,
d) souche n°I-3059, déposée le 20 juin 2003,
e) souche n° I-3323, déposée le 22 novembre 2004,
f) souche n° 1-3324, déposée le 22 novembre 2004,
g) souche n° I-3326, déposée le 1^{er} décembre 2004,
h) souche n° 1-3327, déposée le 1^{er} décembre 2004,
i) souche n° I-3332, déposée le 1^{er} décembre 2004,
j) souche n° I-3333, déposée le 1^{er} décembre 2004,
k) souche n°I-3334, déposée le 1^{er} décembre 2004,
l) souche n° I-3335, déposée le 1^{er} décembre 2004,
m) souche n° I-3336, déposée le 1^{er} décembre 2004,
n) souche n° 1-3337, déposée le 1^{er} décembre 2004,
o) souche n° I-3338, déposée le 2 décembre 2004,
p) souche n° 1-3339, déposée le 2 décembre 2004,
q) souche n° I-3340, déposée le 2 décembre 2004,
r) souche n° I-3341, déposée le 2 décembre 2004.

Les Inventeurs décrivent également un insérat d'acide nucléique d'origine virale, caractérisé en ce qu'il est contenu dans l'une quelconque des souches telles que définies ci-dessus en a)-r).

Les Inventeurs décrivent également un acide nucléique comportant un gène synthétique permettant une expression optimisée de la protéine S dans des cellules eucaryotes, caractérisé en ce qu'il possède la séquence SEQ ID NO: 140.

Les Inventeurs décrivent également un vecteur d'expression comportant un acide nucléique comportant un gène synthétique permettant une expression optimisée de la protéine S, lequel vecteur est contenu dans la souche bactérienne déposée à la CNCM, le 1^{er} décembre 2004, sous le n°I-3333.

Selon un mode de réalisation dudit vecteur d'expression, il s'agit d'un vecteur viral, sous forme de particule virale ou sous forme de génome recombinant.

Selon une disposition avantageuse de ce mode de réalisation, il s'agit d'une particule virale recombinante ou d'un génome viral recombinant susceptible d'être obtenu par transfection d'un plasmide selon les alinéas g), h) et k) à r) tels que définis ci-dessus, dans un système cellulaire approprié, c'est-à-dire, par exemple, des cellules transfectées avec un ou plusieurs autres plasmide(s), destinés à trancomplémenter certaines fonctions du virus délétées dans le vecteur et nécessaires à la formation des particules virales.

On entend ici par « famille de la protéine S » la protéine S complète, son ectodomaine et des fragments de cet ectodomaine qui sont de préférence produits dans un système eucaryote.

Les Inventeurs décrivent également un vecteur lentiviral codant pour un polypeptide de la famille de la protéine S, telle que définie ci-dessus.

Les Inventeurs décrivent également un virus rougeole recombinant codant pour un polypeptide de la famille de la protéine S, telle que définie ci-dessus.

Les Inventeurs décrivent également un virus vaccine recombinant codant pour un polypeptide de la famille de la protéine S, telle que définie ci-dessus.

Les Inventeurs décrivent également l'utilisation d'un vecteur selon les alinéas e) à r) tels que définis ci-dessus, ou d'un vecteur comportant un gène synthétique de la protéine S, tel que défini ci-dessus, pour la production, en système eucaryote, de la protéine S du coronavirus associé au SRAS, ou d'un fragment de cette protéine.

Les Inventeurs décrivent également une méthode de production de la protéine S en système eucaryote, comportant une étape de transfection de cellules eucaryotes en culture par un vecteur choisi parmi les vecteurs contenus dans les souches bactériennes mentionnées aux alinéas e) à r) ci-dessus ou un vecteur comportant un gène synthétique permettant une expression optimisée de la protéine S.

Les Inventeurs décrivent également une banque d'ADNc caractérisée en ce qu'elle comprend des fragments tels que définis ci-dessus, en particulier des fragments d'amplification ou des fragments de restriction, clonés dans un vecteur recombinant, notamment un vecteur d'expression (banque d'expression).

Les Inventeurs décrivent également des cellules, notamment des cellules procaryotes, modifiées par un vecteur recombinant tel que défini ci-dessus.

Les Inventeurs décrivent également une cellule eucaryote génétiquement modifiée exprimant une protéine ou un polypeptide tels que définis ci-après. Bien évidemment, les termes "cellule eucaryote génétiquement modifiée" ne désignent pas une cellule modifiée par un virus sauvage.

Selon un mode de réalisation avantageux de ladite cellule, elle est susceptible d'être obtenue par transfection par l'un quelconque des vecteurs mentionnés aux alinéas k) à n) ci-dessus.

Selon une disposition avantageuse de ce mode de réalisation, il s'agit de la cellule FRhK4-Ssol-30, déposée à la CNCM le 22 novembre 2004, sous le n°I-3325.

Les vecteurs recombinants tels que définis ci-dessus et les cellules transformées par lesdits vecteurs d'expression sont avantageusement utilisés pour la production des protéines et des peptides correspondants. Les banques d'expression dérivées desdits vecteurs, ainsi que les cellules transformées par lesdites banques d'expression sont avantageusement utilisées pour identifier les épitopes immunogènes (épitopes B et T) des protéines du coronavirus associé au SRAS.

Les Inventeurs décrivent également les protéines et les peptides purifiées ou isolées, caractérisés en ce qu'ils sont codés par le polynucléotide ou l'un de ses fragments tels que définis ci-dessus.

Selon un mode de réalisation avantageux, ladite protéine est sélectionnée dans le groupe constitué par :
- la protéine S de séquence SEQ ID NO :3 ou son ectodomaine
- la protéine E de séquence SEQ ID NO :14
- la protéine M de séquence SEQ ID NO :17
- la protéine N de séquence SEQ ID NO : 37
- les protéines codées par les ORFs : ORF1a, ORF1b, ORF3, ORF4 et ORF7 à ORF11, ORF 13 et ORF 14 de séquence respectivement, SEQ ID NO :74, 75, 10, 12, 22, 24, 26, 28, 30, 33 et 35.

On utilisera ci-après indifféremment les termes « ectodomaine de la protéine S » et « forme soluble de la protéine S ».

Selon un mode de réalisation avantageux, ledit polypeptide est constitué des acides aminés correspondant aux positions 1 à 1193 de la séquence en acides aminés de la protéine S

Selon un autre mode de réalisation avantageux, ledit peptide est sélectionné dans le groupe constitué par :
a) les peptides correspondant aux positions 14 à 1193 et 475 à 1193 de la séquence en acides aminés de la protéine S,
b) les peptides correspondant aux positions 2 à 14 (SEQ ID NO : 69) et 100 à 221 de la séquence en acides aminés de la protéine M ; ces peptides correspondent respectivement à l'ectodomaine et à l'endodomaine de la protéine M, et
c) les peptides correspondant aux positions 1 à 12 (SEQ ID NO : 70) et 53 à 76 (SEQ ID NO : 71) de la séquence en acides aminés de la protéine E ; ces peptides correspondent respectivement à l'ectodomaine et à l'extrémité C-terminale de la protéine E, et
d) les peptides de 5 à 50 acides aminés consécutifs, de préférence de 10 à 30 acides aminés, inclus ou chevauchant partiellement ou totalement la séquence des peptides tels que définis en a), b) ou c).

Les Inventeurs décrivent également un peptide caractérisé en ce qu'il présente une séquence de 7 à 50 acides aminés incluant un résidu d'acide aminé sélectionné dans le groupe constitué par :
- l'alanine située en position 2552 de la séquence en acides aminés de la protéine codée par l'ORF1a.
- la sérine située en position 577 de la séquence en acides aminés de la protéine S de la souche de SARS-CoV telle que définie ci-dessus,
- la glycine en position 11 de la séquence en acides aminés de la protéine codée par l'ORF3 de la souche de SARS-CoV telle que définie ci-dessus,
- la sérine en position 154 de la séquence en acides aminés de la protéine M de la souche de SARS-CoV telle que définie ci-dessus.

Les Inventeurs décrivent a également un anticorps ou un fragment d'anticorps polyclonal ou monoclonal, susceptible d'être obtenu par immunisation d'un animal avec un vecteur recombinant tel que défini ci-dessus, une banque d'ADNc telle que définie ci-dessus ou bien une protéine ou un peptide tels que définis ci-dessus, caractérisé en ce qu'il se lie avec l'une au moins des protéines codées par le SARS-CoV telles que définies ci-dessus.

Un anticorps tel que défini ci-dessus englobe les anticorps polyclonaux, les anticorps monoclonaux, les anticorps chimériques tels que les anticorps humanisés, ainsi que leurs fragments (Fab, Fv, scFv).

Les Inventeurs décrivent également un hybridome produisant un anticorps monoclonal contre la protéine N, caractérisé en ce qu'il est choisi parmi les hybridomes suivants :
- l'hybridome produisant l'anticorps monoclonal 87, déposé à la CNCM le 1^{er} décembre 2004 sous le numéro 1-3328,
- l'hybridome produisant l'anticorps monoclonal 86, déposé à la CNCM le 1^{er} décembre 2004 sous le numéro 1-3329,
- l'hybridome produisant l'anticorps monoclonal 57, déposé à la CNCM le 1^{er} décembre 2004 sous le numéro I-3330, et
- l'hybridome produisant l'anticorps monoclonal 156, déposé à la CNCM le 1^{er} décembre 2004 sous le numéro I-3331.

Les Inventeurs décrivent également un anticorps ou fragment d'anticorps polyclonal ou monoclonal dirigé contre la protéine N, caractérisé en ce qu'il est produit par un hybridome tel que défini ci-dessus.

On entend par anticorps chimérique, relativement à un anticorps d'une espèce animale particulière ou d'une classe particulière d'anticorps, un anticorps comprenant tout ou partie d'une chaîne lourde et/ou d'une chaîne légère d'un anticorps d'une autre espèce animale ou d'une autre classe d'anticorps.

On entend par anticorps humanisé une immmunoglobuline humaine dans laquelle les résidus des CDRs (*Complementary-Determining Regions*) qui forment le site de liaison à l'antigène sont remplacés par ceux d'un anticorps monoclonal non-humain possédant la spécificité, l'affinité ou l'activité recherchées. Par comparaison avec les anticorps non-humains, les anticorps humanisés sont moins immunogènes et possèdent une demi-vie prolongée chez l'Homme car ils ne possèdent qu'une faible proportion de séquences non-humaines étant donné que la quasi-totalité des résidus des régions FR (Framework) et de la région constante (Fc) de ces anticorps sont ceux d'une séquence consensus d'immunoglobulines humaines.

Les Inventeurs décrivent également une puce ou filtre à protéine, caractérisé en ce qu'il comprend une protéine, un peptide ou bien un anticorps tels que définis ci-dessus.

Les puces à protéine sont préparées par les méthodes classiques, connues en elles-mêmes. Parmi les supports appropriés sur lesquels peuvent être immobilisés des protéines, on peut citer ceux en matière plastique ou en verre, notamment sous la forme de microplaques.

Les Inventeurs décrivent également des réactifs dérivés de la souche isolée de coronavirus associé au SRAS, issue du prélèvement répertorié sous le n° 031589, utiles pour l'étude et le diagnostic de l'infection provoquée par un coronavirus associé au SRAS, lesquels réactifs sont sélectionnés dans le groupe constitué par :
(a) une paire d'amorces, une sonde ou une puce à ADN telles que définies ci-dessus,
(b) un vecteur recombinant ou une cellule modifiée tels que définis ci-dessus,
(c) une souche isolée de coronavirus ou un polynucléotide tels que définis ci-dessus,
(d) une protéine ou un peptide tel que défini ci-dessus,
(e) un anticorps ou fragment d'anticorps tels que définis ci-dessus, et
(f) une puce à protéine telle que définie ci-dessus.

Ces différents réactifs sont préparés et utilisés selon les techniques classiques de biologie moléculaire et d'immunologie, en suivant les protocoles standards tels que ceux décrits dans *Current Protocols in Molecular Biology* (Frederick M. AUSUBEL, 2000, Wiley and Son Inc., Library of Congress, USA), dans *Current Protocols in Immunology* (John E. Cologan, 2000, Wiley and Son Inc. Library of Congress, USA) et dans *Antibodies : A Laboratory Manual* (E. Howell and D Lane, Cold Spring Harbor Laboratory, 1988)

Les fragments d'acide nucléique selon l'invention sont préparés et utilisés selon les techniques classiques telles que définies ci-dessus. Les peptides et les protéines selon l'invention sont préparés par les techniques d'ADN recombinant, connues de l'Homme du métier, notamment à l'aide des vecteurs recombinants tels que définis ci-dessus. Alternativement, les peptides selon l'invention peuvent être préparés par les techniques classiques de synthèse en phase solide ou liquide, connues de l'Homme du métier.

Les anticorps polyclonaux sont préparés par immunisation d'un animal approprié avec une protéine ou un peptide tels que définis ci-dessus, éventuellement couplé à la KLH ou à l'albumine et/ou associé à un adjuvant approprié tel que l'adjuvant de Freund (complet ou incomplet) ou l'hydroxyde d'alumine ; après obtention d'un titre en anticorps satisfaisant, les anticorps sont récoltés par prélèvement du sérum des animaux immunisés et enrichis en IgG par précipitation, selon les techniques classiques, puis les IgG spécifiques des protéines du SARS-CoV sont éventuellement purifiées par chromatographie d'affinité sur une colonne appropriée sur laquelle sont fixés ledit peptide ou ladite protéine, tels que définis ci-dessus, de façon à obtenir une préparation d'IgG monospécifiques.

Les anticorps monoclonaux sont produits à partir d'hybridomes obtenus par fusion de lymphocytes B d'un animal immunisé par une protéine ou un peptide tels que définis ci-dessus avec des myélomes, selon la technique de Köhler et Milstein (Nature, 1975, 256, 495-497) ; les hybridomes sont cultivés *in vitro,* notamment dans des fermenteurs ou produits *in vivo*, sous forme d'ascite ; alternativement lesdits anticorps monoclonaux sont produits par génie génétique comme décrit dans le brevet américain US 4,816,567.

Les anticorps humanisés sont produits par des méthodes générales comme celles décrites dans la Demande Internationale WO 98/45332.

Les fragments d'anticorps sont produits à partir des régions V_{H} et V_{L} clonées, à partir des ARNm d'hybridomes ou de lymphocytes spléniques d'une souris immunisée ; par exemple, les fragments Fv, scFv ou Fab sont exprimés à la surface de phages filamenteux selon la technique de Winter et Milstein (Nature, 1991, 349, 293-299) ; après plusieurs étapes de sélection, les fragments d'anticorps spécifiques de l'antigène sont isolés et exprimés dans un système d'expression approprié, par les techniques classiques de clonage et d'expression d'ADN recombinant.

Les anticorps ou leur fragments tels que définis ci-dessus, sont purifiés par les techniques classiques connues de l'Homme du métier, telles que la chromatographie d'affinité.

Les Inventeurs décrivent en outre l'utilisation d'un produit sélectionné dans le groupe constitué par : une paire d'amorces, une sonde, une puce à ADN, un vecteur recombinant, une cellule modifiée, une souche isolée de coronavirus, un polynucléotide, une protéine ou un peptide, un anticorps ou un fragment d'anticorps et une puce à protéine tels que définis ci-dessus, pour la préparation d'un réactif de détection et éventuellement de génotypage/sérotypage, d'un coronavirus associé au SRAS.

Les protéines et les peptides tels que définis ci-dessus, qui sont aptes à être reconnus et/ou à induire la production d'anticorps spécifiques du coronavirus associé au SRAS, sont utiles pour le diagnostic de l'infection par un tel coronavirus ; l'infection est détectée, par une technique appropriée- notamment EIA, ELISA, RIA, immunofluorescence-, à partir d'un échantillon biologique prélevé chez un individu susceptible d'être infecté.

Selon une disposition avantageuse de ladite utilisation, lesdites protéines sont sélectionnées dans le groupe constitué par les protéines S, E, M et/ou N et les peptides tels que définis ci-dessus.

Les protéines S, E, M et/ou N et les peptides dérivés de ces protéines tels que définis ci-dessus, par exemple la protéine N, sont utilisées pour le diagnostic indirect d'une infection à coronavirus associé au SRAS (diagnostic sérologique ; détection d'anticorps spécifiques du SARS-CoV), notamment par une méthode immunoenzymatique (ELISA).

Les anticorps et les fragments d'anticorps tels que définis ci-dessus, notamment ceux dirigés contre les protéines S, E, M et/ou N et les peptides dérivés tels que définis ci-dessus, sont utiles pour le diagnostic direct d'une infection à coronavirus associé au SRAS ; la détection de protéine(s) du SARS-CoV est réalisée par une technique appropriée, notamment EIA, ELISA, RIA, immunofluorescence à partir d'un échantillon biologique prélevé chez un individu susceptible d'être infecté.

Les Inventeurs décrivent également une méthode de détection d'un coronavirus associé au SRAS, à partir d'un échantillon biologique, laquelle méthode est caractérisée en ce qu'elle comprend au moins :
(a) la mise en contact dudit échantillon biologique avec au moins un anticorps ou un fragment d'anticorps, une protéine, un peptide ou bien une puce ou un filtre à protéine ou à peptide tels que définis ci-dessus, et
(b) la révélation par tout moyen approprié des complexes antigène-anticorps formés en (a), par exemple par EIA, ELISA, RIA, ou par immunofluorescence.

Selon un mode de mise en oeuvre avantageux dudit procédé l'étape (a) comprend :
(a₁) la mise en contact dudit échantillon biologique avec au moins un premier anticorps ou un fragment d'anticorps qui est fixé sur un support approprié, notamment une microplaque,
(a₂) le lavage de la phase solide, et
(a₃) l'addition d'au moins un second anticorps ou un fragment d'anticorps, différent du premier, ledit anticorps ou fragment d'anticorps étant éventuellement marqué de façon appropriée.

Ce procédé qui permet de capturer les particules virales présentes dans l'échantillon biologique est également dénommé procédé d'immunocapture.

Par exemple :
- l'étape (a₁) est réalisée avec au moins un premier anticorps monoclonal ou polyclonal ou un fragment de ceux-ci, dirigé contre la protéine S, M, et/ou E, et/ou un peptide correspondant à l'ectodomaine de l'une de ces protéines (peptides M2-14 ou E1-12)
- l'étape (a₃) est réalisée avec au moins un anticorps ou un fragment d'anticorps dirigé contre un autre épitope de la même protéine ou de préférence contre une autre protéine, de manière préférée contre une protéine interne telle que la nucléoprotéine N ou l'endodomaine de la protéine E ou M, de manière encore plus préférée il s'agit d'anticorps ou de fragments d'anticorps dirigés contre la protéine N qui est très abondante dans la particule virale ; lorsqu'un anticorps ou un fragment d'anticorps dirigé contre une protéine interne (N) ou contre l'endodomaine des protéines E ou M est utilisé, le dit anticorps est incubé en présence de détergent, comme le Tween 20 par exemple, à des concentrations de l'ordre de 0,1 %.
- l'étape (b) de révélation des complexes antigène-anticorps formés est réalisée, soit directement à l'aide d'un second anticorps marqué par exemple avec de la biotine ou une enzyme appropriée telle que la peroxydase ou la phosphatase alcaline, soit indirectement à l'aide d'un sérum anti-immunoglobulines marqué comme ci-dessus. Les complexes ainsi formés sont révélés à l'aide d'un substrat approprié.

Selon une mise en oeuvre préférée de cette méthode, l'échantillon biologique est mélangé à l'anticorps monoclonal de révélation préàlablement à sa mise en contact avec les anticorps monoclonaux de capture. Le cas échéant, le mélange sérum-anticorps de révélation est incubé pendant au moins 10 minutes à température ambiante avant d'être appliqué sur la plaque.

Les Inventeurs décrivent également un test d'immunocapture destiné à détecter une infection par le coronavirus associé au SRAS par détection de la nucléoprotéine native (protéine N), en particulier caractérisé en ce que l'anticorps utilisé pour la capture de la nucléoprotéine virale native est un anticorps monoclonal spécifique de la région centrale et/ou d'un épitope conformationnel.

Selon un mode de réalisation dudit test, l'anticorps utilisé pour la capture de la protéine N est l'anticorps monoclonal Acm87, produit par l'hybridome déposé à la CNCM le 1^{er} décembre 2004 sous le numéro I-3328.

Selon un autre mode de réalisation dudit test d'immunocapture, l'anticorps utilisé pour la capture de la protéine N est l'anticorps monoclonal Acm86, produit par l'hybridome déposé à la CNCM le 1^{er} décembre 2004 sous le numéro 1-3329.

Selon un autre mode de réalisation dudit test d'immunocapture, les anticorps monoclonaux Acm86 et Acm87 sont utilisés pour la capture de la protéine N.

Dans les tests d'immunocapture, on peut utiliser pour la révélation de la protéine N, l'anticorps monoclonal Acm57, produit par l'hybridome déposé à la CNCM le 1^{er} décembre 2004 sous le numéro 1-3330, ledit anticorps étant conjugué à une molécule ou une particule révélatrice.

Conformément audit test d'immunocapture, une combinaison des anticorps Acm57 et Acm87, conjugués à une molécule ou une particule révélatrice, est utilisée pour la révélation de la protéine N.

Une molécule révélatrice peut être un atome radioactif, un colorant, une molécule fluorescente, un fluorophore, une enzyme ; une particule révélatrice peut être, par exemple : de l'or colloïdal, une particule magnétique ou une bille de latex.

Les Inventeurs décrivent également un réactif de détection d'un coronavirus associé au SRAS, caractérisé en ce qu'il est sélectionné dans le groupe constitué par :
(a) une paire d'amorces ou une sonde telles que définies ci-dessus,
(b) un vecteur recombinant tel que défini ci-dessus ou une cellule modifiée telle que définie ci-dessus,
(c) une souche isolée de coronavirus telle que définie ci-dessus ou un polynucléotide tel que défini ci-dessus,
(d) un anticorps ou un fragment d'anticorps tel que défini ci-dessus,
(e) une combinaison d'anticorps comprenant les anticorps monoclonaux Acm86 et/ou Acm87, et l'anticorps monoclonal Acm57, telle que définie ci-dessus,
(f) une puce ou un filtre tels que définis ci-dessus.

Les Inventeurs décrivent également une méthode de détection d'une infection par un coronavirus associé au SRAS, à partir d'un échantillon biologique, par ELISA IgG indirect utilisant la protéine N, laquelle méthode est caractérisée en ce que les plaques sont sensibilisées par une solution de protéine N à une concentration comprise entre 0,5 et 4µg/mL, de préférence 2µg/mL, dans un tampon PBS 10mM pH 7,2, rouge de phénol à 0,25mL /L.

Les Inventeurs décrivent, en outre, une méthode de détection d'une infection par un coronavirus associé au SRAS, à partir d'un échantillon biologique, par ELISA double épitope, caractérisée en ce que le sérum à tester est mélangé à l'antigène de révélation, ledit mélange étant ensuite mis au contact de l'antigène fixé sur un support solide.

Selon une variante des tests de détection des coronavirus associé au SRAS, ces tests combinent un ELISA utilisant la protéine N, et un autre ELISA utilisant la protéine S, tel que décrit plus bas.

Les Inventeurs décrivent aussi un complexe immun formé d'un anticorps ou d'un fragment d'anticorps polyclonal ou monoclonal tel que défini ci-dessus, et d'une protéine ou d'un peptide du coronavirus associé au SRAS.

Les Inventeurs décrivent en outre un kit de détection d'un coronavirus associé au SRAS, caractérisé en ce qu'il comprend au moins un réactif sélectionné dans le groupe constitué par : une paire d'amorces, une sonde, une puce à ADN ou à ARN, un vecteur recombinant, une cellule modifiée, une souche isolée de coronavirus, un polynucléotide, une protéine ou un peptide, un anticorps, et une puce à protéine tels que définis ci-dessus.

Les Inventeurs décrivent en outre, une composition immunogène, caractérisée en ce qu'elle comprend au moins un produit sélectionné dans le groupe constitué par :
a) une protéine ou un peptide tels que définis ci-dessus,
b) un polynucléotide de type ADN ou ARN ou l'un de ses fragments représentatifs tels que définis ci-dessus, de séquence choisie parmi :
   (i) la séquence SEQ ID NO : 1 ou son équivalent ARN
   (ii) la séquence hybridant dans des conditions de forte stringence avec la séquence SEQ ID NO : 1,
   (iii) la séquence complémentaire de la séquence SEQ ID NO : 1 ou de la séquence hybridant dans des conditions de forte stringence avec la séquence SEQ ID NO : 1, .
   (iv) la séquence nucléotidique d'un fragment représentatif du polynucléotide tel que défini en (i), (ii) ou (iii),
   (v) la séquence telle que définie en (i), (ii), (iii) ou (iv), modifiée, et
c) un vecteur d'expression recombinant comprenant un polynucléotide tel que défini en b), et
d) une banque d'ADNc telle que définie ci-dessus,
ladite composition immunogène étant capable d'induire une immunité humorale ou cellulaire protectrice spécifique du coronavirus associé au SRAS, notamment la production d'un anticorps dirigé contre un épitope spécifique du coronavirus associé au SRAS.

Les protéines et les peptides tels que définis ci-dessus, notamment les protéines S, M, E et/ou N et les peptides dérivés, ainsi que les molécules d'acide nucléique (ADN ou ARN) codant lesdites protéines ou lesdits peptides, sont de bons candidats vaccin et peuvent être utilisées dans des compositions immunogènes pour la production d'un vaccin contre le coronavirus associé au SRAS.

Selon un mode de réalisation avantageux des compositions selon l'invention, elles contiennent en outre, au moins un véhicule pharmaceutiquement acceptable et éventuellement des substances porteuses et/ou des adjuvants.

Les véhicules pharmaceutiquement acceptables, les substances porteuses et les adjuvants sont ceux classiquement utilisés.

Les adjuvants sont avantageusement choisis dans le groupe constitué par des émulsions huileuses, de la saponine, des substances minérales, des extraits bactériens, de l'hydroxyde d'alumine et le squalène.

Les substances porteuses sont avantageusement sélectionnées dans le groupe constitué par des liposomes unilamellaires, des liposomes multilamellaires, des micelles de saponine ou des microsphères solides de nature saccharidique ou aurifère.

Les compositions selon l'invention, sont administrées par voie générale, notamment intramusculaire ou sous-cutanée ou bien par voie locale notamment nasale (aérosol).

Les Inventeurs décrivent également l'utilisation d'une protéine ou d'un peptide isolé ou purifié présentant une séquence sélectionnée dans le groupe constitué par les séquences SEQ ID NO : 3, 10, 12, 14, 17, 22, 24, 26, 28, 30, 33, 35, 37, 69, 70, 71, 74 et 75 pour former un complexe immun avec un anticorps dirigé spécifiquement contre un épitope du coronavirus associé au SRAS.

Les Inventeurs décrivent également un complexe immun formé d'une protéine ou d'un peptide isolé ou purifié présentant une séquence sélectionnée dans le groupe constitué par les séquences SEQ ID NO : 3, 10, 12, 14, 17, 22, 24, 26, 28, 30, 33, 35, 37, 69, 70, 71, 74 et 75, et d'un anticorps dirigé spécifiquement contre un épitope du coronavirus associé au SRAS.

Les Inventeurs décrivent également l'utilisation d'une protéine ou d'un peptide isolé ou purifié présentant une séquence sélectionnée dans le groupe constitué par les séquences SEQ ID NO : 3, 10, 12, 14, 17, 22, 24, 26, 28, 30, 33, 35, 37, 69, 70, 71, 74 et 75 pour induire la production d'un anticorps capable de reconnaître spécifiquement un épitope du coronavirus associé au SRAS.

Les Inventeurs décrivent également l'utilisation d'un polynucléotide isolé ou purifié présentant une séquence sélectionnée dans le groupe constitué par les séquences SEQ ID NO : 1, 2, 4, 7, 8, 13, 15, 16, 18, 19, 20, 31, 36 et 38 pour induire la production d'un anticorps dirigé contre la protéine codée par ledit polynucléotide et capable de reconnaître spécifiquement un épitope du coronavirus associé au SRAS

Les Inventeurs décrivent également des anticorps monoclonaux reconnaissant la protéine S native d'un coronavirus associé au SRAS.

Les Inventeurs décrivent également l'utilisation d'une protéine ou d'un polypeptide de la famille de la protéine S, telle que définie ci-dessus, ou d'un anticorps reconnaissant la protéine S native, tel que défini ci-dessus, pour détecter une infection par un coronavirus associé au SRAS, à partir d'un échantillon biologique.

Les Inventeurs décrivent également une méthode de détection d'une infection par un coronavirus associé au SRAS, à partir d'un échantillon biologique, caractérisée en ce que la détection est effectuée par ELISA utilisant la protéine S recombinante, exprimée dans un système eucaryote.

Selon un mode de mise en oeuvre avantageux de ladite méthode, il s'agit d'une méthode par ELISA double épitope, et le sérum à tester est mélangé à l'antigène de révélation, ledit mélange étant ensuite mis au contact de l'antigène fixé sur un support solide.

Les Inventeurs décrivent aussi un complexe immun formé d'un anticorps ou d'un fragment d'anticorps monoclonal reconnaissant la protéine S native, et d'une protéine ou d'un peptide du coronavirus associé au SRAS.

Les Inventeurs décrivent également un complexe immun formé d'une protéine ou d'un polypeptide de la famille de la protéine S, telle que définie ci-dessus, et d'un anticorps dirigé spécifiquement contre un épitope du coronavirus associé au SRAS.

Les Inventeurs décrivent en outre Les Inventeurs décrivent un kit ou coffret de détection d'un coronavirus associé au SRAS, caractérisé en ce qu'il comprend au moins un réactif sélectionné dans le groupe constitué par : une protéine ou polypeptide de la famille de la protéine S, telle que définie ci-dessus, un acide nucléique codant pour une protéine ou polypeptide de la famille de la protéine S, telle que définie ci-dessus, une cellule exprimant une protéine ou polypeptide de la famille de la protéine S, telle que définie ci-dessus, ou un anticorps reconnaissant la protéine S native d'un coronavirus associé au SRAS.

Les Inventeurs décrivent une composition immunogène et/ou vaccinale, caractérisée en ce qu'elle comprend un polypeptide ou une protéine recombinante de la famille de la protéine S, telle que définie ci-dessus, obtenu dans un système d'expression eucaryote.

Les Inventeurs décrivent également une composition immunogène et/ou vaccinale, caractérisée en ce qu'elle comprend un vecteur ou virus recombinant, exprimant une protéine ou un polypeptide de la famille de la protéine S, telle que définie ci-dessus.

Outre les dispositions qui précèdent, l'invention comprend encore d'autres dispositions, qui ressortiront de la description qui va suivre, qui se réfère à des exemples de mise en oeuvre du polynucléotide représentant le génome de la souche de SARS-CoV issue du prélèvement répertorié sous le numéro 031589, et des fragments d'ADNc dérivés objets de la présente invention, ainsi qu'au Tableau I présentant la liste des séquences :

**Tableau I : Liste des séquences**

| **numéro d'identification** | **Séquence** | **Position de l'ADNc en référence à Genbank AY274119.3** | **Numéro de dépôt à la CNCM du plasmide correspondant** |
|---|---|---|---|
| SEQ ID NO : 1 | génome de la souche issue du prélèvement 031589 | - | - |
| SEQ ID NO : 2 | ORF-S* | 21406-25348 | - |
| SEQ ID NO : 3 | Protéine S | - | - |
| SEQ ID NO : 4 | ORF-S** | 21406-25348 | I-3059 |
| SEQ ID NO: 5 | fragment Sa | 21406-23454 | I-3020 |
| SEQ ID NO : 6 | fragment Sb | 23322-25348 | I-3019 |
| SEQ ID NO : 7 | ORF-3+ORF-4* | 25110-26244 | - |
| SEQ ID NO : 8 | ORF-3+ORF-4** | 25110-26244 | I-3126 |
| SEQ ID NO : 9 | ORF3 | - | - |
| SEQ ID NO : 10 | Protéine ORF-3 | - | - |
| SEQ ID NO : 11 | ORF4 | - | - |
| SEQ ID NO : 12 | Protéine ORF-4 | - | - |
| SEQ ID NO: 13 | ORF-E* | 26082-26413 | - |
| SEQ ID NO : 14 | Protéine E | - | - |
| SEQ ID NO : 15 | ORF-E** | 26082-26413 | I-3046 |
| SEQ ID NO : 16 | ORF-M* | 26330-27098 | - |
| SEQ ID NO : 17 | Protéine M | - | - |
| SEQ ID NO : 18 | ORF-M** | 26330-27098 | I-3047 |
| SEQ ID NO : 19 | ORF7 à 11* | 26977-28218 | - |
| SEQ ID NO : 20 | ORF7 à 11** | 26977-28218 | I-3125 |
| SEQ ID NO : 21 | ORF7 | - | - |
| SEQ ID NO : 22 | Protéine ORF7 | - | - |
| SEQ ID NO : 23 | ORF8 | - | - |
| SEQ ID NO : 24 | Protéine ORF8 | - | - |
| SEQ ID NO : 25 | ORF9 | - | - |
| SEQ ID NO : 26 | Protéine ORF9 | - | - |
| SEQ ID NO : 27 | ORF10 | - | - |
| SEQ ID NO : 28 | Protéine ORF10 | - | - |
| SEQ ID NO : 29 | ORF11 | - | - |
| SEQ ID NO : 30 | Protéine ORF11 | - | - |
| SEQ ID NO : 31 | OrF1ab | 265-21485 | - |
| SEQ ID NO : 32 | ORF13 | 28130-28426 | - |
| SEQ ID NO : 33 | Protéine ORF13 | - | - |
| SEQ ID NO : 34 | ORF14 | - | - |
| SEQ ID NO : 35 | Protéine ORF14 | 28583-28795 | - |
| SEQ ID NO : 36 | ORF-N* | 28054-29430 | |
| SEQ ID NO : 37 | Protéine N | - | - |
| SEQ ID NO : 38 | ORF-N** | 28054-29430 | I-3048 |
| SEQ ID NO : 39 | 5'non-codante** | 1-204 | I-3124 |
| SEQ ID NO : 40 | 3'non-codante** | 28933-29727 | I-3123 |
| SEQ ID NO : 41 | **ORF1ab** Fragment L0 | 30-500 | - |
| SEQ ID NO : 42 | Fragment L1 | 211-2260 | - |
| SEQ ID NO : 43 | Fragment L2 | 2136-4187 | - |
| SEQ ID NO : 44 | Fragment L3 | 3892-5344 | - |
| SEQ ID NO : 45 | Fragment L4b | 4932-6043 | - |
| SEQ ID NO : 46 | Fragment L4 | 5305-7318 | - |
| SEQ ID NO : 47 | Fragment L5 | 7275-9176 | - |
| SEQ ID NO : 48 | Fragment L6 | 9032-11086 | - |
| SEQ ID NO : 49 | Fragment L7 | 10298-12982 | - |
| SEQ ID NO : 50 | Fragment L8 | 12815-14854 | - |
| SEQ ID NO : 51 | Fragment L9 | 14745-16646 | - |
| SEQ ID NO : 52 | Fragment L10 | 16514-18590 | - |
| SEQ ID NO : 53 | Fragment L11 | 18500-20602 | - |
| SEQ ID NO : 54 | Fragment L12 | 20319-22224 | - |
| SEQ ID NO : 55 | Amorce N sens | - | - |
| SEQ ID NO : 56 | Amorce N antisens | - | - |
| SEQ ID NO : 57 | Amorce S_{C} sens | - | - |
| SEQ ID NO : 58 | Amorce S_{L} sens | - | - |
| SEQ ID NO : 59 | Amorce S_{C} e t S_{L} antisens | - | - |
| SEQ ID NO : 60 | Amorce sens série 1 | 28507-28522 | - |
| SEQ ID NO : 61 | Amorce antisens série 1 | 28774-28759 | |
| SEQ ID NO : 62 | Amorce sens série 2 | 28375-28390 | - |
| SEQ ID NO : 63 | Amorce antisens série 2 | 28702-28687 | - |
| SEQ ID NO : 64 | Sonde 1/série 1 | 28561-28586 | - |
| SEQ ID NO : 65 | Sonde 2/série 1 | 28588-28608 | - |
| SEQ ID NO : 66 | Sonde 1/série 2 | 28541-28563 | - |
| SEQ ID NO : 67 | Sonde 2/série 2 | 28565-28589 | - |
| SEQ ID NO : 68 | Amorce ancre 14T | | |
| SEQ ID NO : 69 | Peptide M2-14 | - | - |
| SEQ ID NO : 70 | Peptide E1-12 | - | - |
| SEQ ID NO : 71 | Peptide E53-76 | - | - |
| SEQ ID NO : 72 | 5'non-codante* | 1-204 | - |
| SEQ ID NO : 73 | 3'non-codante* | 28933-29727 | - |
| SEQ ID NO : 74 | Protéine ORF1a | - | - |
| SEQ ID NO : 75 | Protéine ORF1b | - | - |
| SEQ ID NO:76-139 | Amorces | | |
| SEQ ID NO:140 | Pseudogène de S | | |
| SEQ ID NO:141-148 | amorces | | |
| SEQ ID NO:149 | Aa1-13 de S | | |
| SEQ ID NO:150 | polypeptide | | |
| SEQ ID NO:151-158 | amorces | | |
| | | | |
| | | | |
| | | | |
| | | | |

| | | | |
|---|---|---|---|
| * produit d'amplification PCR (amplicon) ** insert cloné dans le plasmide déposé à la CNCM | | | |

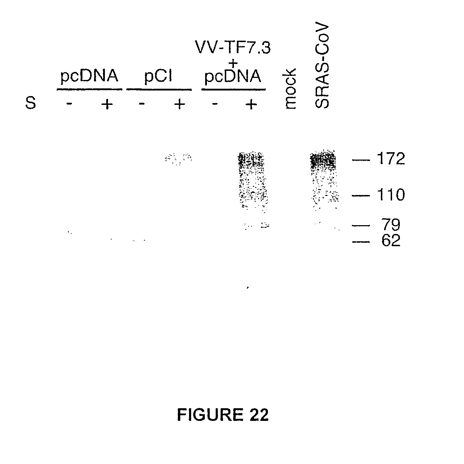

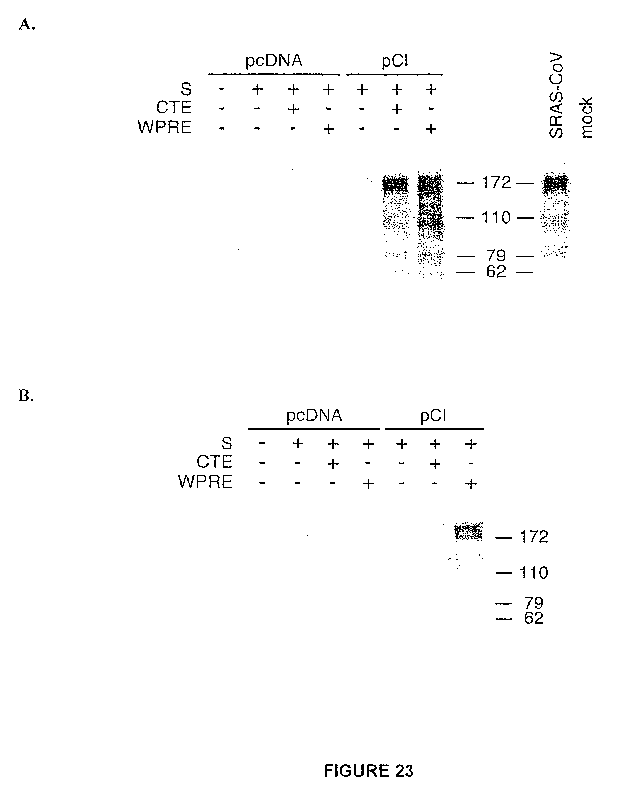

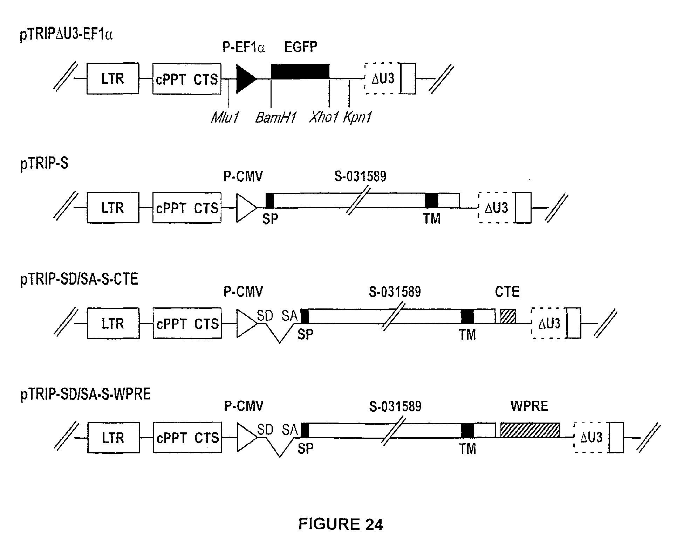

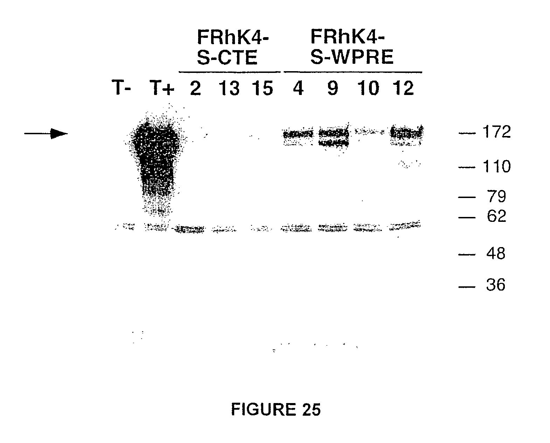

ainsi qu'aux dessins annexés dans lesquels :

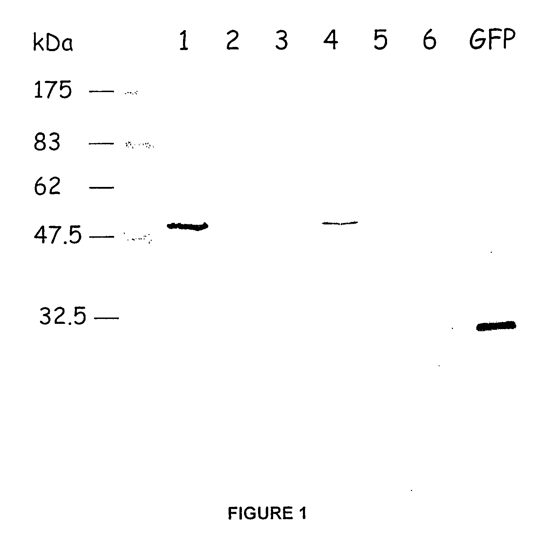

- la figure 1 illustre l'analyse par Western-blot de l'expression *in vitro* des protéines recombinantes N, S_{C} et S_{L} à partir des vecteurs d'expression pIVEX. Piste 1 : pIV2.3N. Piste 2 : pIV2.3S_{C}. Piste 3 : pIV2.3S_{L}. Piste 4 : pIV2.4N. Piste 5 : pIV2.4S₁ ou pIV2.4S_{C}. Piste 6 : pIV2.4S_{L}. L'expression de la protéine GFP exprimée à partir du même vecteur est utilisée comme contrôle.

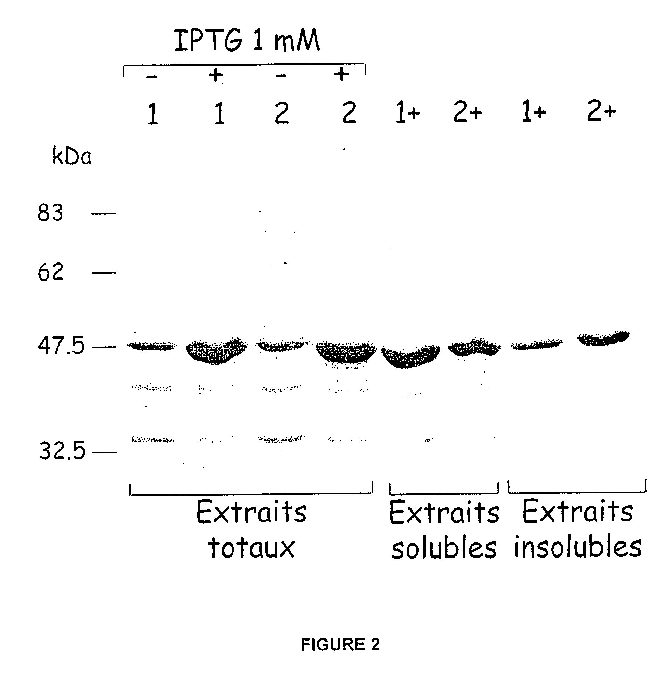

- la figure 2 illustre l'analyse par électrophorèse en gel de polyacrylamide en conditions dénaturantes (SDS-PAGE) et coloration au bleu de Coomassie, de l'expression *in vivo* de la protéine N à partir des vecteurs d'expression pIVEX. La souche d'*E.coli* BL21(DE3)pDIA17 transformée par les vecteurs pIVEX recombinants est cultivée à 30°C dans du milieu LB, en présence ou en l'absence d'inducteur (IPTG 1mM). Piste 1 : pIV2.3N Piste 2 : pIV2.4N.

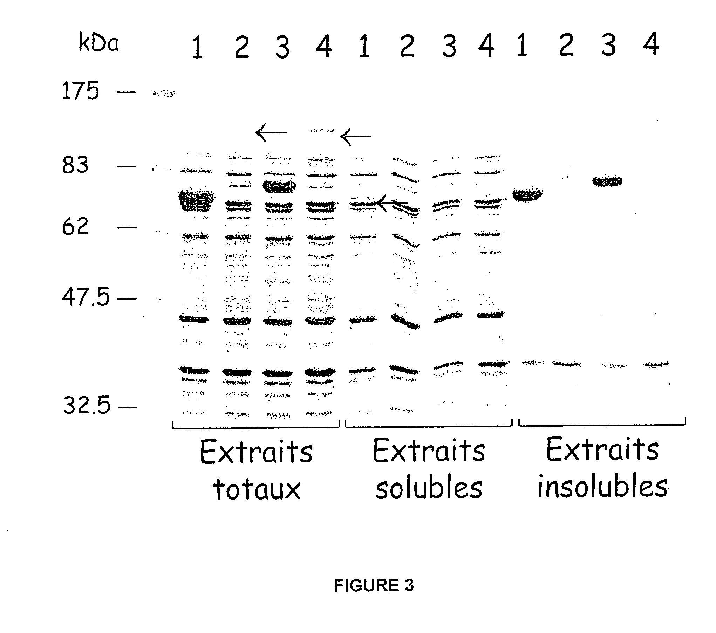

- la figure 3 illustre l'analyse par électrophorèse en gel de polyacrylamide en conditions dénaturantes (SDS-PAGE) et coloration au bleu de Coomassie, de l'expression *in vivo* des polypeptides S_{L} et S_{C} à partir des vecteurs d'expression pIVEX. La souche d'*E.coli* BL21(DE3)pDIA17 transformée par les vecteurs pIVEX recombinants est cultivée à 30°C dans du milieu LB, en présence ou en l'absence d'inducteur (IPTG 1mM). Piste 1 : pIV2.3S_{C} Piste 2 : pIV2.3S_{L}. Piste 3 : pIV2.4S₁ Piste 4 : pIV2.4S_{L}.

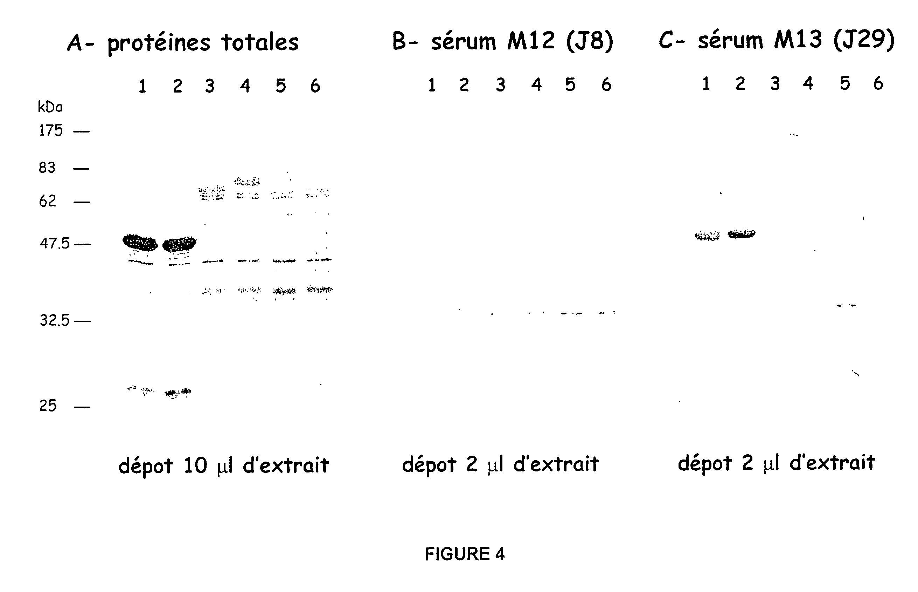

- la figure 4 illustre l'activité antigénique des protéines N, S_{L} et S_{C} recombinantes produites dans la souche *E. coli* BL21(DE3)pDIA17 transformée par les vecteurs pIVEX recombinants. A : électrophorèse (SDS-PAGE) des lysats bactériens. B et C : Western-blot avec les sérums, provenant d'un même patient infecté par le SARS-CoV, prélevés respectivement 8 jours (B : sérum M12) et 29 jours-(C : sérum M13) après le début des symptômes du SRAS. Piste 1 : pIV2.3N. Piste 2 : pIV2.4N. Piste 3 : pIV2.3S_{C}. Piste 4 : pIV2.4 S₁. Piste 5 : pIV2.3S_{L}. Piste 6 : pIV2.4S_{L}

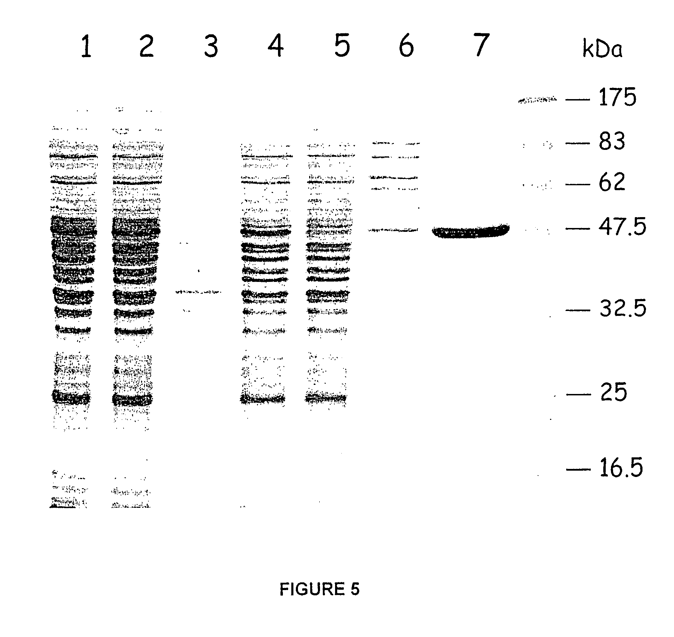

- la figure 5 illustre la purification sur colonne Ni-NTA agarose de la protéine N recombinante produite dans la souche *E. coli* BL21(DE3)pDIA17 à partir du vecteur pIV2.3N. Piste 1 : Extrait bactérien total. Piste 2 : Extrait soluble. Piste 3 : Extrait insoluble. Piste 4 : Extrait déposé sur la colonne Ni-NTA. Piste 5 : protéines non-retenues. Piste 6 : Fractions du pic 1. Piste 7 : Fractions du pic 2.

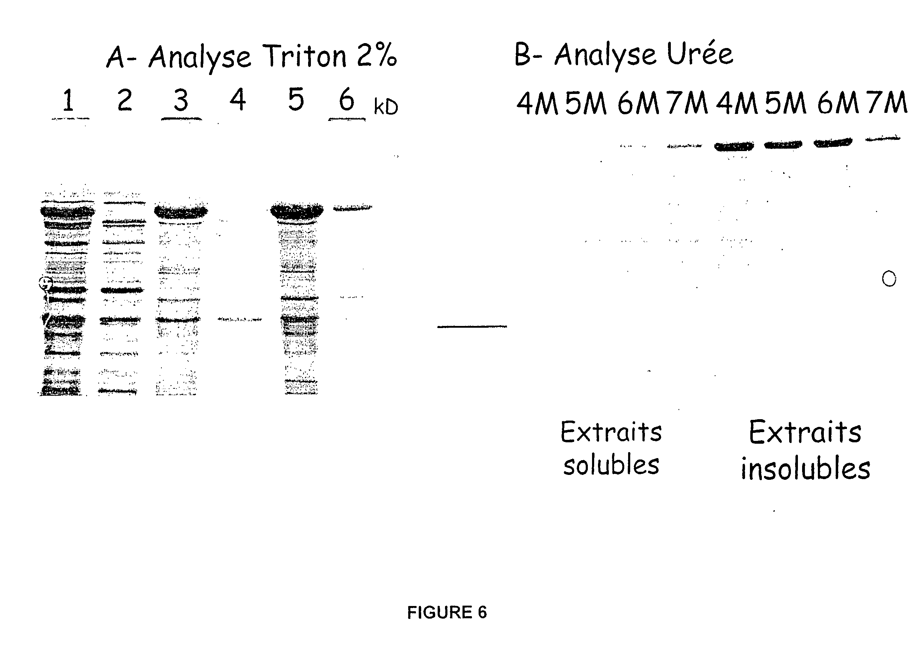

- la figure 6 illustre la purification de la protéine S_{C} recombinante à partir des corps d'inclusions produits dans la souche *E. coli* BL21(DE3)pDIA17 transformée par le pIV2.4S₁.A. Traitement au Triton X-100 (2%) : Piste 1 : Extrait bactérien total. Piste 2 : Extrait soluble. Piste 3 : Extrait insoluble. Piste 4 : Surnageant après traitement au Triton X-100 (2 %). Pistes 5 et 6 : Culot après traitement au Triton X-100 (2 %).B : Traitement à l'urée 4M, 5M, 6M et 7M des extraits solubles et insolubles.

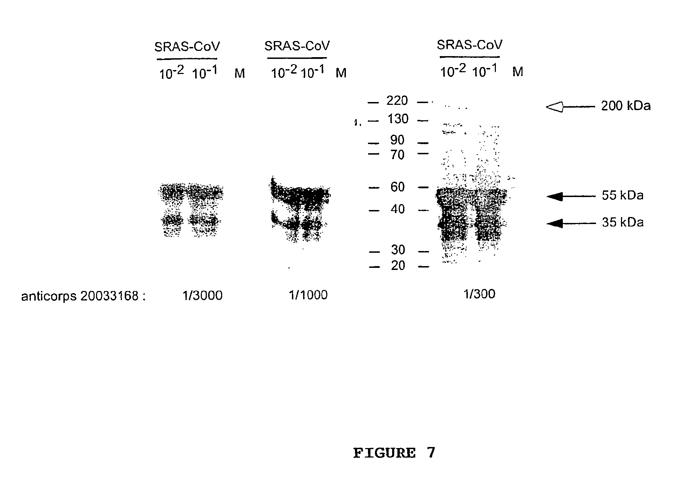

- la figure 7 représente l'immunoempreinte réalisée à l'aide d'un lysat de cellules infectées par le SARS-CoV et d'un sérum de patient atteint de pneumopathie atypique.

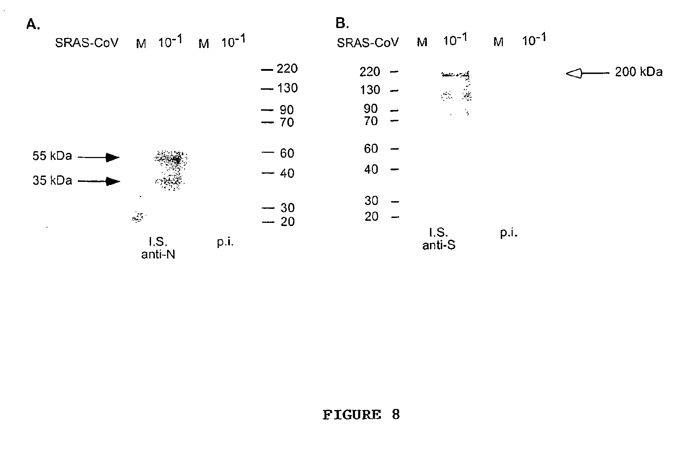

- la figure 8 représente des immunoempreintes réalisées à l'aide d'un lysat de cellules infectées par le SARS-CoV et d'immunsérums de lapins spécifiques de la nucléoprotéine N (A) et de la protéine de spicule S (B). I.S. : sérum immun. p.i. : sérum pré-immun. L'immunsérum anti-N a été utilisé au 1/50000 et l'immunsérum anti-S au 1/10000.

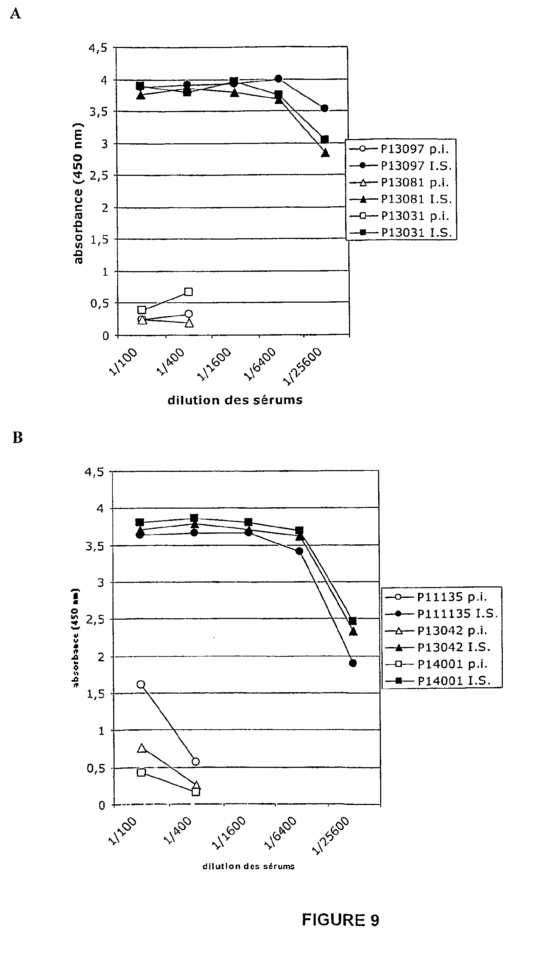

- la figure 9 illustre la réactivité en ELISA des sérums polyclonaux monospécifiques de lapin dirigés contre la protéine N ou le fragment court de la protéine S (S_{C}), vis-à-vis des protéines recombinantes correspondantes utilisées pour l'immunisation. A : lapins P13097, P13081, et P13031 immunisés avec la protéine N recombinante purifié. B : lapins P11135, P13042, et P14001 immunisés avec une préparation de corps d'inclusions correspondants au fragment court de la protéine S (S_{C}). I.S. : sérum immun. p.i. : sérum pré-immun.

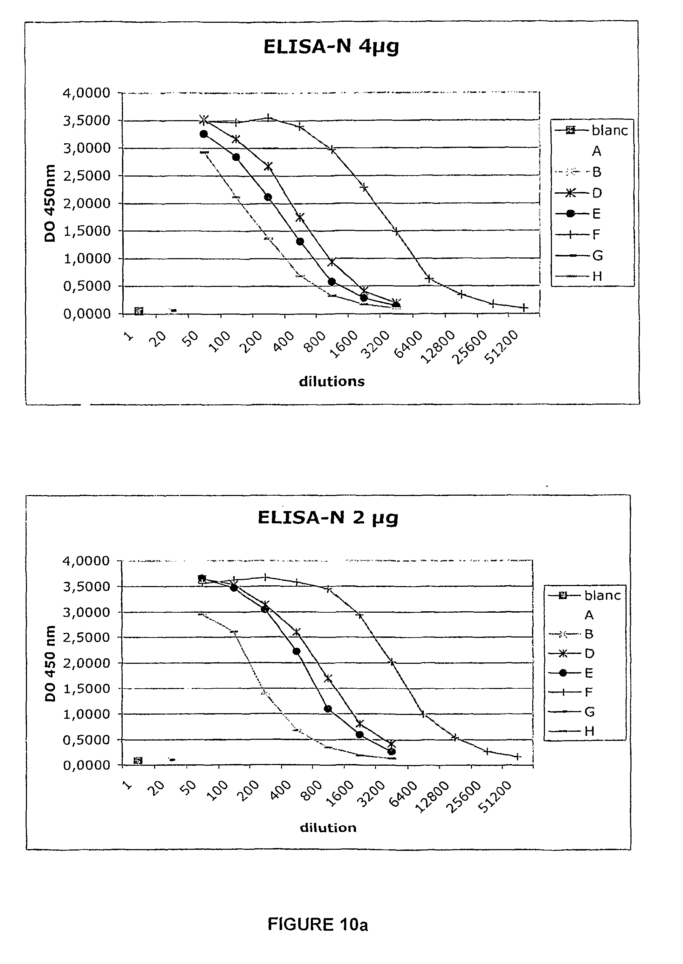

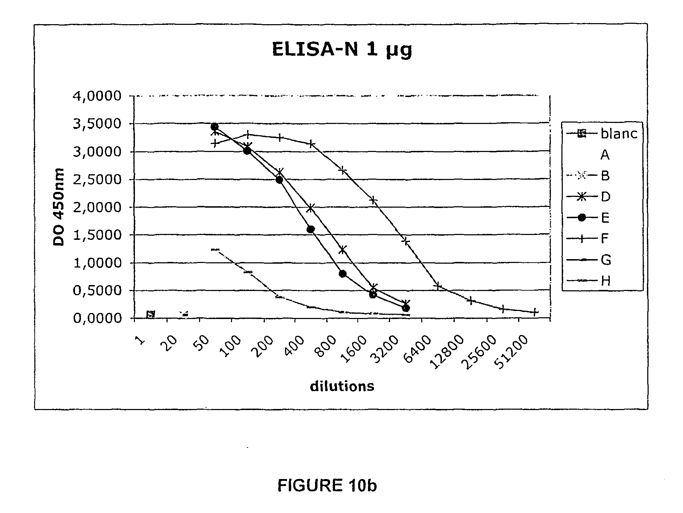

- la figure 10 illustre la réactivité en ELISA de la protéine N recombinante purifiée, vis-à-vis de sérum de patients atteints de pneumonie atypique causée par le SARS-CoV. Figure 10a : plaques ELISA préparés avec la protéine N à la concentration de 4 µg/ml et 2 µg/ml. Figure 10b : plaque ELISA préparée avec la protéine N à la concentration de 1 µg/ml. Les sérums désignés A, B, D, E, F, G, H correspondent à ceux du Tableau IV.

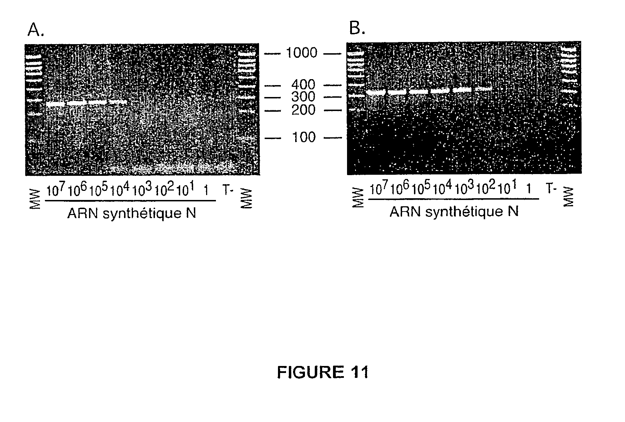

- la figure 11 illustre l'amplification par RT-PCR de quantités décroissantes d'ARN synthétique du gène N du SARS-CoV (10⁷ à 1 copie), à l'aide des couples d'amorces n° 1 (N/+/28507,N/-/28774) (A) et n° 2 (N/+/28375,N/-/28702) (B). T : amplification réalisée en l'absence d'ARN. MW : marqueur d'ADN.

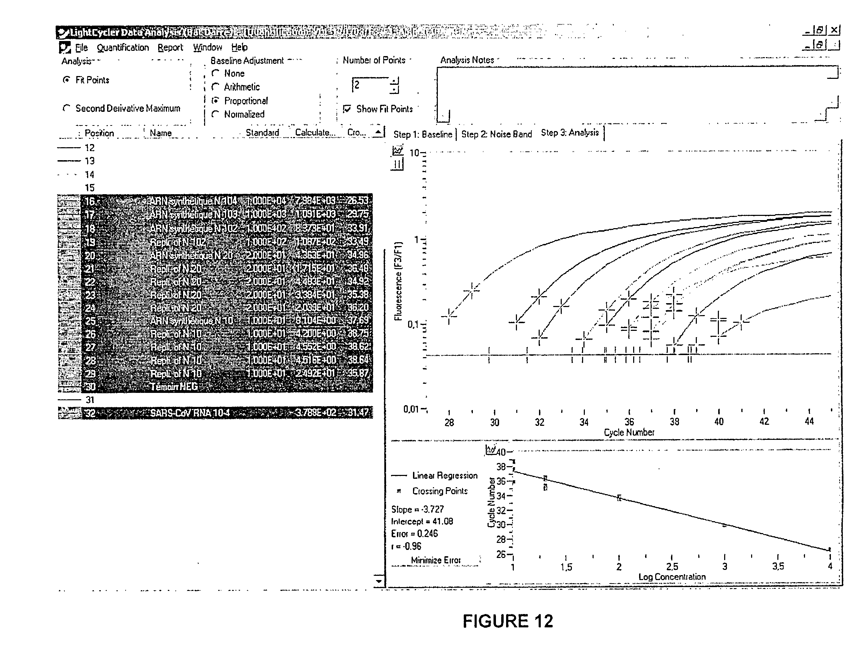

- la figure 12 illustre l'amplification par RT-PCR en temps réel d'ARN synthétique du gène N du SARS-CoV : des quantités décroissantes d'ARN synthétique en répliquat (repli. ; pistes 16 à 29) ainsi que de l'ARN viral dilué au 1/20x10⁻⁴ (piste 32) ont été amplifiés par RT-PCR en temps réel à l'aide du kit "Light Cycler RNA Amplification Kit Hybridization Probes" et des couples d'amorces et de sondes de la série n° 2, dans les conditions décrites à l'exemple 8.
- la figure 13 (figure 13.1 à 13.70) représente la carte de restriction de la séquence SEQ ID NO : 1 correspondant à l'équivalent ADN du génome de la souche de SARS-CoV issue du prélèvement répertorié sous le numéro 031589.

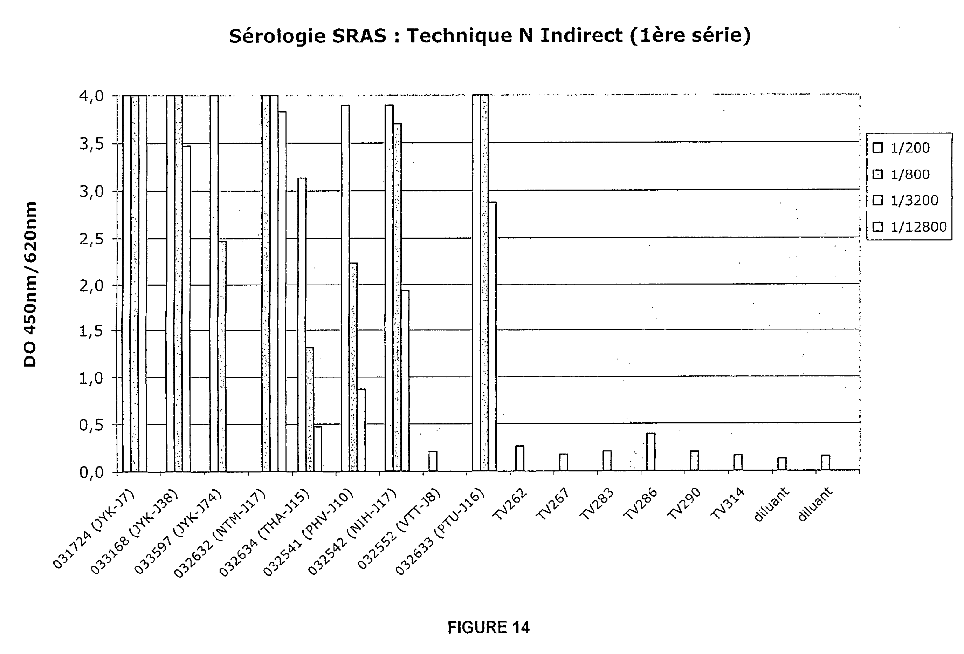

- la figure 14 montre le résultat du test de sérologie SRAS par ELISA N indirect (1ère série de sérums testés)

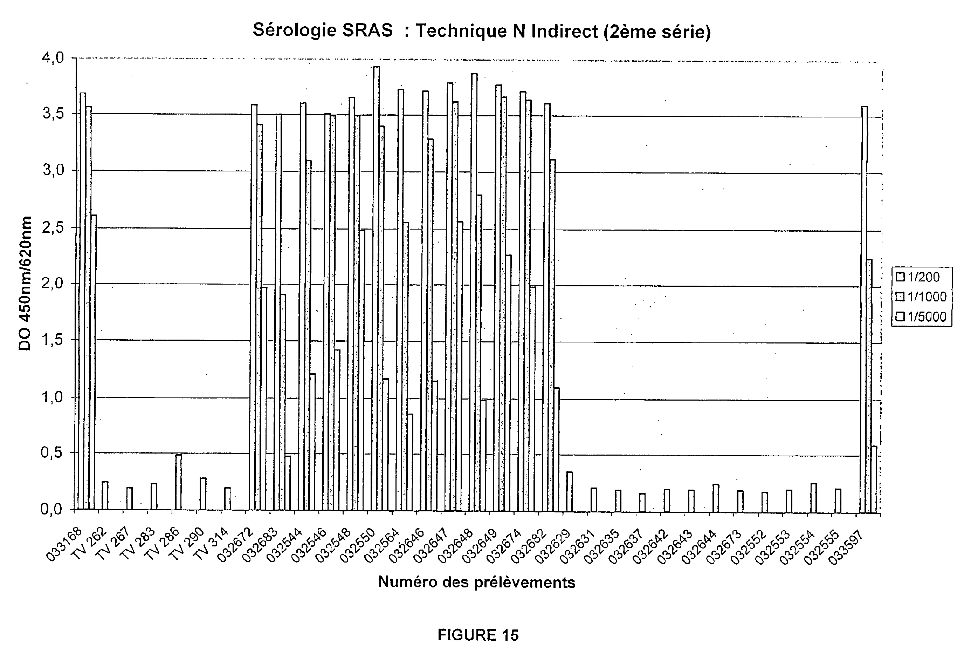

- la figure 15 montre le résultat du test de sérologie SRAS par ELISA N indirect (2ème série de sérums testés)

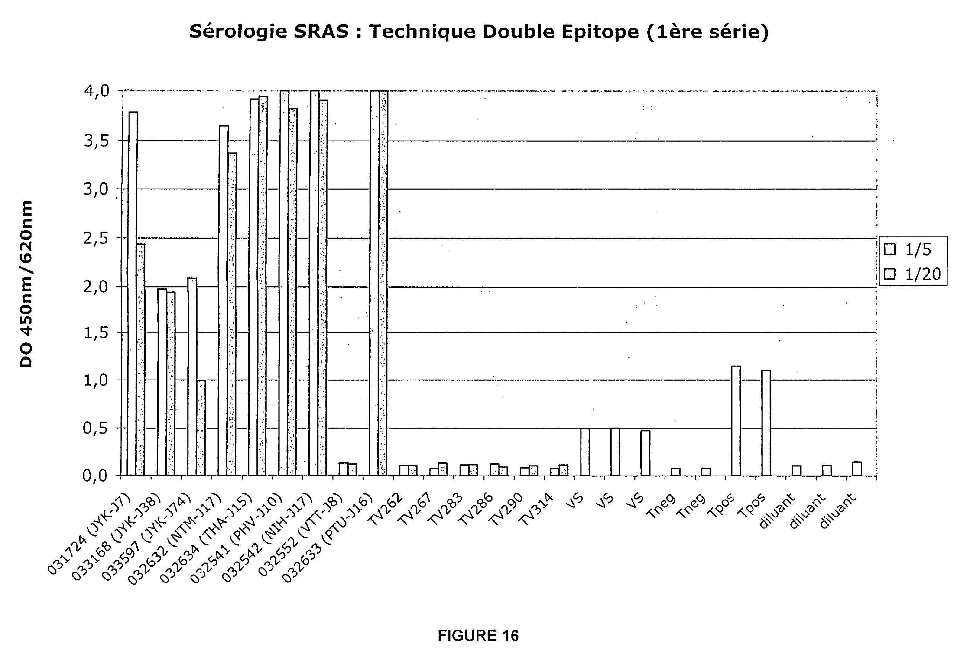

- la figure 16 présente le résultat du test de sérologie SRAS par ELISA N double épitope (1ère série de sérums testés)

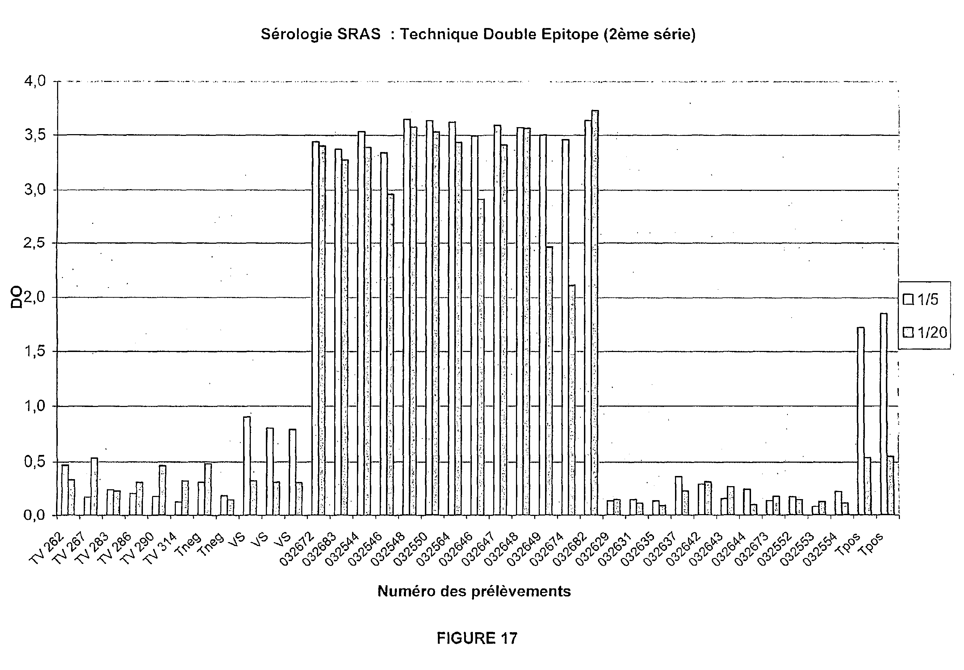

- la figure 17 montre le résultat du test de sérologie SRAS par ELISA N double épitope (2ème série de sérums testés)

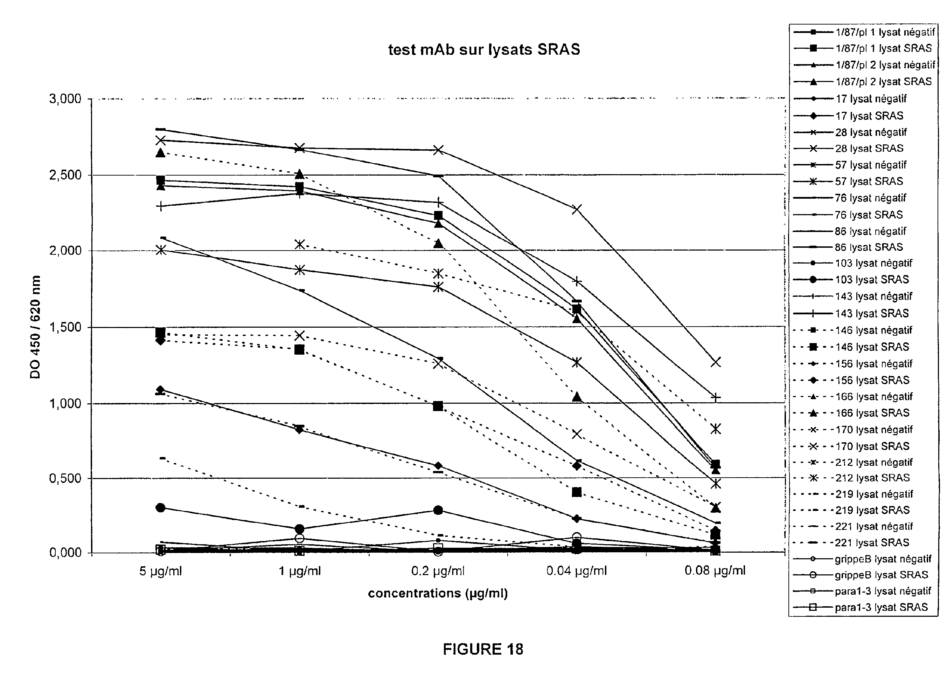

- la figure 18 illustre le test de réactivité des anticorps monoclonaux anti-N par ELISA sur la nucléoprotéine N native du SRAS-CoV. Les anticorps ont été testés sous la forme de surnageants de culture d'hybridomes par un ELISA indirect utilisant un lysat irradié de cellules VeroE6 infectées par le SRAS-CoV comme antigène (courbes lysat SRAS). Un contrôle négatif de réactivité est réalisé pour chaque anticorps sur un lysat de cellules VeroE6 non infectées (courbes lysat négatif). Plusieurs anticorps monoclonaux de spécificité connue ont été utilisés comme anticorps témoins négatifs : para1-3 dirigé contre les antigènes des virus parainfluenza de type 1-3 (Bio-Rad) et grippe B dirigé contre les antigènes du virus de la grippe de type B (Bio-Rad).

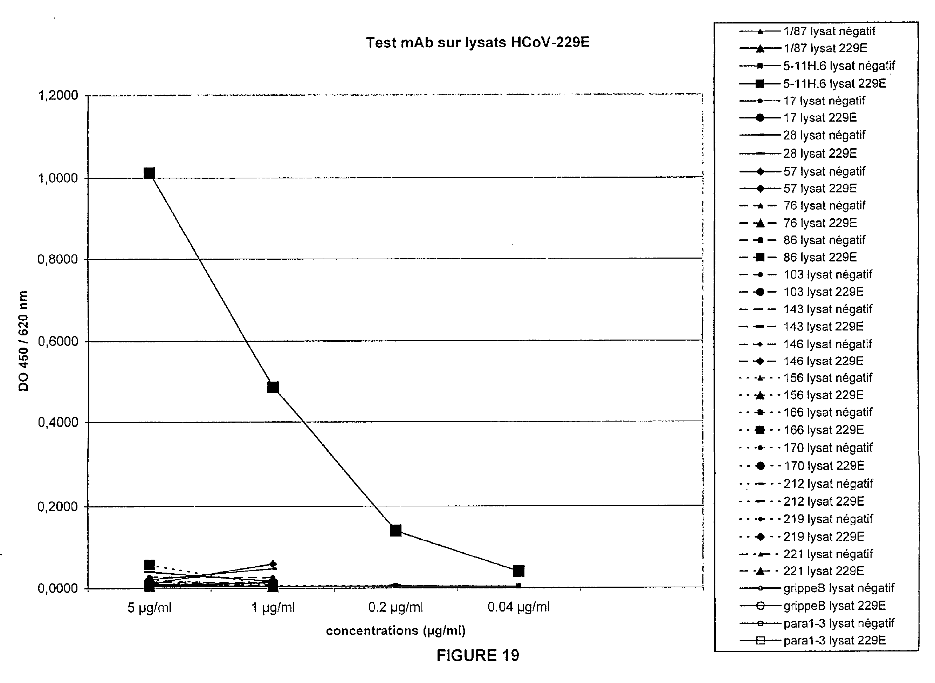

- la figure 19 illustre le test de réactivité des anticorps monoclonaux anti-N du SRAS-CoV par ELISA sur les antigènes natifs du coronavirus humain 229E (HCoV-229E). Les anticorps ont été testés sous la forme de surnageants de culture d'hybridomes par un test ELISA indirect utilisant un lysat de cellules MRC-5 infectées par le coronavirus humain 229E comme antigène (courbes lysat 229E). Un contrôle négatif d'immunoréactivité est réalisé pour chaque anticorps sur un lysat de cellules MRC-5 non infectées (courbes lysat négatif). L'anticorps monoclonal 5-11H.6 dirigé contre la protéine S du coronavirus humain 229E (Sizun et al. 1998, J. Virol. Met. 72 : 145-152) est utilisé comme anticorps témoin positif. Les anticorps para1-3 dirigé contre les antigènes des virus parainfluenza de type 1-3 (Bio-Rad) et grippe B dirigé contre les antigènes du virus de la grippe de type B (Bio-Rad) ont été ajoutés au panel des anticorps monoclonaux testés.

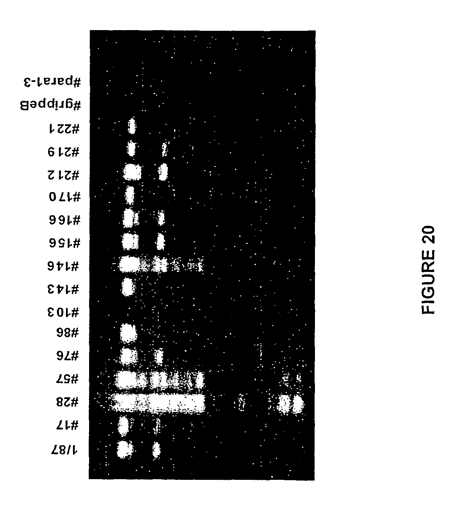

- la figure 20 montre un test de réactivité des anticorps monoclonaux anti-N du SRAS-CoV par western blot sur la nucléoprotéine N native du SRAS-CoV dénaturée. Un lysat de cellules VeroE6 infectées par le SRAS-CoV a été préparé dans le tampon de dépôt selon Laemmli et mis à migrer dans un gel SDS à 12% de polyacrylamide puis les protéines ont été transférées sur membrane de PVDF. Les anticorps monoclonaux anti-N testés ont été utilisés pour l'immunoessai à la concentration de 0.05 µg/ml. La révélation est faite avec des anticorps anti-IgG(H+L) de souris couplés à la peroxydase (NA93IV, Amersham) et le système ECL+. Deux anticorps monoclonaux ont été utilisés comme témoins négatifs de réactivité : grippe B dirigé contre les antigènes du virus de la grippe de type B (Bio-Rad) et para1-3 dirigé contre les antigènes des virus parainfluenza de type 1-3 (Bio-Rad).

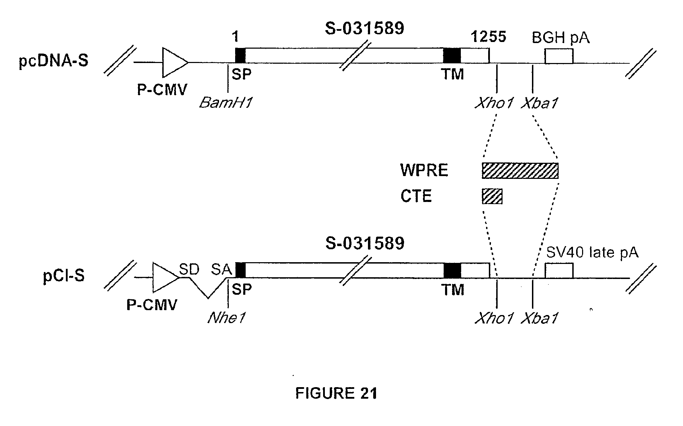

- la figure 21 présente les plasmides d'expression en cellules de mammifères de la protéine S du SRAS-CoV. Le cDNA de la S du SRAS-CoV a été inséré entre les sites BamH1 et Xho1 du plasmide d'expression pcDNA3.1(+) (Clontech) pour obtenir le plasmide pcDNA-S et entre les sites Nhe1 et Xho1 du plasmide d'expression pCI (Promega) pour obtenir le plasmide pCI-S. Les séquences WPRE et CTE ont été insérées dans chacun des deux plasmides pcDNA-S et pCI-S entre les sites Xho1 et Xba1 pour obtenir respectivement les plasmides pcDNA-S-CTE, pcDNA-S-WPRE, pCI-S-CTE et pCI-S-WPRE.
   SP : peptide signal prédit (aa 1-13) avec le logiciel signalP v2.0 (Nielsen et al., 1997, Protein Engineering, 10 : 1-6)
   TM : région transmembranaire prédite (aa 1196-1218) avec le logiciel TMHMM v2.0 (Sonnhammer et al., 1998, Proc. of Sixth Int. Conf. on Intelligent Systems for Molecular Biology, p 175-182, AAAI Press). Il faut noter que les acides aminés W1194 et P1195 font possiblement partie de la région transmembranaire avec des probabilités respectives de 0,13 et 0,42
   P-CMV : promoteur immédiat/précoce du cytomégalovirus. BGH pA : signal de polyadénylation du gène de l'hormone de croissance bovine
   SV40 late pA : signal de polyadénylation tardif du virus SV40
   SD/SA : sites donneur et accepteur d'épissage
   WPRE : séquences du "Woodchuck Hepatitis Virus posttranscriptional regulatory element" du virus de l'hépatite de la marmotte
   CTE : séquences du "constitutive transport element" du rétrovirus simien de Mason-Pfizer
- la figure 22 illustre l'expression de la protéine S après transfection de cellules VeroE6. Des extraits cellulaires ont été préparés 48 heures après transfection de cellulesVeroE6 par les plasmides pcDNA, pcDNA-S, pCI et pCI-S. Des extraits cellulaires ont également été préparés 18 heures après infection par le virus recombinant de la vaccine VV-TF7.3 et transfection par les plasmides pcDNA ou pcDNA-S. A titre de contrôle, des extraits de cellules VeroE6 ont été préparés 8 heures après infection par le SRAS-CoV à une multiplicité d'infection de 3. Ils ont été séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). Une échelle de masse moléculaire (kDa) est portée sur la figure.
   SRAS-CoV : extrait de cellules VeroE6 infectées par le SRAS-CoV
   Mock : extrait contrôle de cellules non infectées
- la figure 23 illustre l'effet des séquences CTE et WPRE sur l'expression de la protéine S après transfection de cellules VeroE6 et 293T. Des extraits cellulaires ont été préparés 48 heures après transfection de cellulesVeroE6 (A) ou 293T (B) par les plasmides pcDNA, pcDNA-S, pcDNA-S-CTE, pcDNA-S-WPRE, pCI-S, pCI-S-CTE et pCI-S-WPRE, séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). Une échelle de masse moléculaire (kDa) est portée sur la figure.
   SRAS-CoV : extrait de cellules VeroE6 préparés 8 heures après infection par le SRAS-CoV à une multiplicité d'infection de 3.
   Mock : extrait contrôle de cellules VeroE6 non infectées
- la figure 24 présente des vecteurs lentiviraux défectifs à DNA flap central pour l'expression de la S du SRAS-CoV. Le cADN de la protéine S du SRAS-CoV a été cloné sous la forme d'un fragment BamH1-Xho1 dans le plasmide pTRIPΔU3-CMV contenant un vecteur lentiviral défectif TRIP à DNA flap central (Sirven et al, 2001, Mol. Ther., 3 :438-448) pour obtenir le plasmide pTRIP-S. Les cassettes d'expression optimales constituées du promoteur immédiat/précoce du virus CMV, d'un signal d'épissage, du cDNA de la S et de l'un ou l'autre des signaux post-transcriptionnels CTE ou WPRE ont été substituées à la cassette EF1α-EGFP du vecteur d'expression lentiviral défectif à DNA FLAP central TRIPΔU3-EF1 α (Sirven et al, 2001, Mol. Ther., 3 :438-448) pour obtenir les plasmides pTRIP-SD/SA-S-CTE et pTRIP-SD/SA-S-WPRE.
   SP : peptide signal
   TM : région transmembranaire
   P-CMV : promoteur immédiat/précoce du cytomégalovirus
   P-EF1α : promoteur du gène EF1α
   SD/SA : sites donneur et accepteur d'épissage
   WPRE : séquences du "Woodchuck Hepatitis Virus posttranscriptional regulatory element" du virus de l'hépatite de la marmotte
   CTE : séquences du "constitutive transport element" du rétrovirus simien de Mason-Pfizer
   LTR : « Long terminal repeat »
   ΔU3 : LTR délété des séquences « promoter/enhancer »
   cPPT : « polypurine tract cis-active sequence »
   CTS : « central termination sequence »
- la figure 25 montre l'analyse par western blot de l'expression de la S du SRAS-CoV par des lignées cellulaires transduites par les vecteurs lentiviraux TRIP-SD/SA-S-WPRE et TRIP-SD/SA-S-CTE. Des extraits cellulaires ont été préparés à partir de lignées FrhK4-S-CTE et FrhK4-S-WPRE établies après transduction par les vecteurs lentiviraux TRIP-SD/SA-S-CTE et TRIP-SD /SA-S-WPRE respectivement. Ils ont été séparés sur un gel SDS à 8% d'acrylamide et analysés par western blot à l'aide d'un anticorps polyclonal de lapin anti-S et d'un conjugué anti-IgG(H+L) de lapin couplé à la peroxidase. Une échelle de masse moléculaire (kDa) est portée sur la figure.
   T- : extrait contrôle de cellules FrhK-4
   T+ : extrait de cellules FrhK-4 préparées 24h après infection par le SRAS-CoV à une multiplicité d'infection de 3.

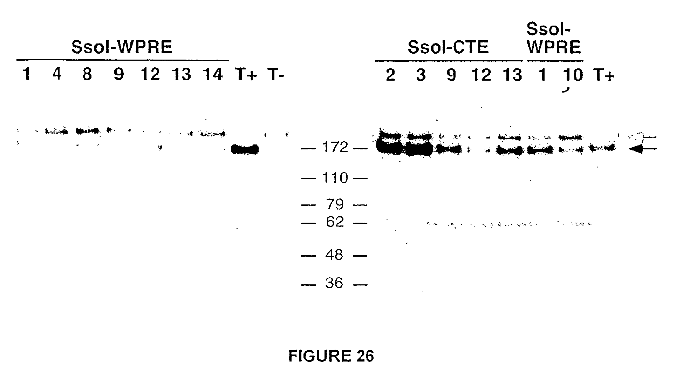

- la figure 26 concerne l'analyse de l'expression de polypeptide Ssol par des lignées cellulaires transduites par les vecteurs lentiviraux TRIP-SD/SA-Ssol-WPRE et TRIP-SD/SA-Ssol-CTE. La sécrétion du polypeptide Ssol a été recherchée dans le surnageant d'une série de clones cellulaires isolés après transduction de cellules FRhK-4 par les vecteurs lentiviraux TRIP-SD/SA-SsoI-WPRE et TRIP-SD/SA-Ssol-CTE. 5 µl de surnageant dilués au 1/2 dans du tampon de dépôt selon Laemmli ont été analysés par western blot révélé par un anticorps monoclonal anti-FLAG (M2, Sigma) et un conjugué anti-IgG(H+L) de souris couplé à la peroxidase. T- : surnageant de la lignée FRhK-4 parentale. T+ : surnageant de cellules BHK infectées par un virus recombinant de la vaccine exprimant le polypeptide Ssol. La flèche pleine indique le polypeptide Ssol, tandis que la flèche creuse indique une réaction croisée avec une protéine d'origine cellulaire.

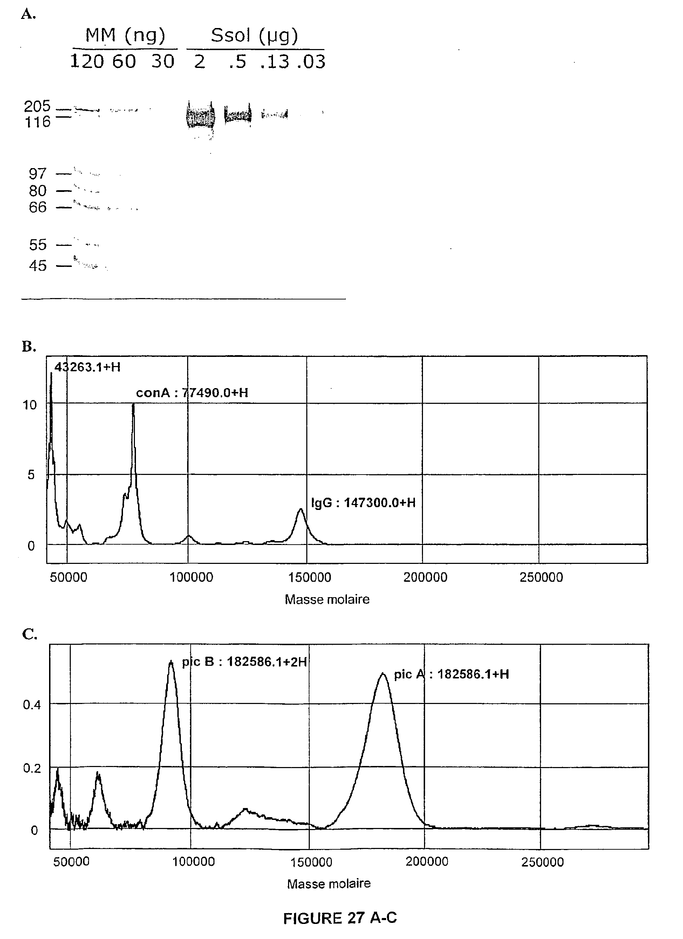

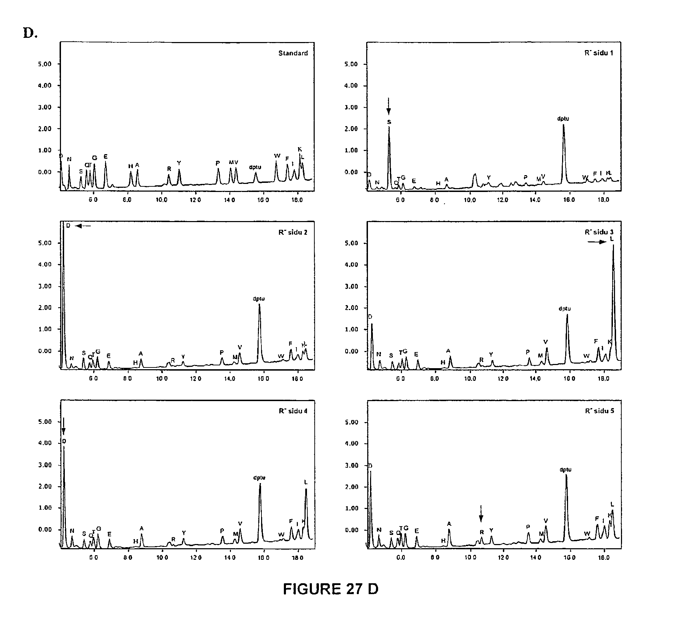

- la figure 27 montre les résultats relatifs à l'analyse du polypeptide Ssol purifié
   A. 8, 2, 0.5 et 0.125 µg de polypeptide recombinant Ssol purifié par chromatographie d'affinité anti-FLAG et gel filtration (G75) ont été séparés sur gel SDS à 8% de polyacrylamide. Le polypeptide Ssol ainsi que des quantités variables de marqueurs de masse moléculaire (MM) ont été révélés par coloration au nitrate d'argent (Gelcode SilverSNAP stain kit II, Pierce).
   B. Marqueurs étalons pour l'analyse par spectrométrie de masse SELDI-TOF
      IgG : IgG bovine de MM 147300
      ConA : conalbumine de MM 77490
      HRP : péroxidase de raifort analysée à titre de contrôle et de MM 43240
   C. Analyse par spectrométrie de masse (SELDI-TOF) du polypeptide recombinant Ssol.
      Les pics A et B correspondent au polypeptide Ssol simplement et doublement chargé.
   D. Séquençage de l'extrémité N-terminale du polypeptide recombinant Ssol. 5 cycles de dégradation d'Edman en phase liquide ont été réalisés sur un séquenceur ABI494 (Applied Biosystems).

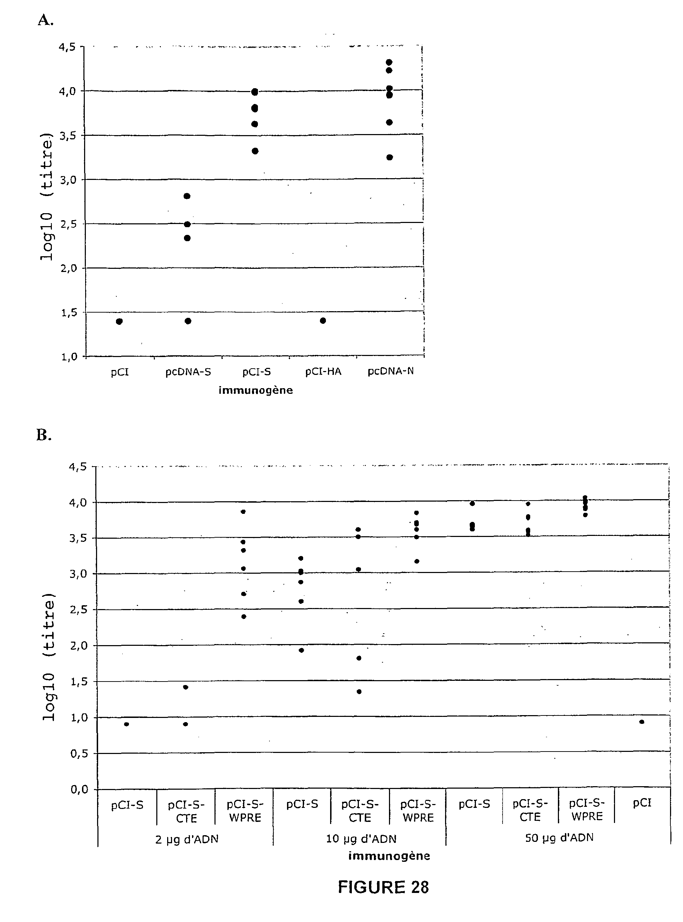

- la figure 28 illustre l'influence d'un signal d'épissage et des séquences CTE et WPRE sur l'efficacité de l'immunisation génique à l'aide d'ADN plasmidique codant la S du SRAS-CoV
   A.Des groupes de 7 souris BALB/c ont été immunisés à deux reprises à 4 semaines d'intervalle à l'aide de 50 µg d'ADN plasmidique de pCI, pcDNA-S, pCI-S, pcDNA-N et pCI-HA.
   B. Des groupes de 6 souris BALB/c ont été immunisés à deux reprises à 4 semaines d'intervalle à l'aide de 2 µg, 10µg ou 50 µg d'ADN plasmidique de pCI, pCI-S, pCI-S-CTE et pCI-S-WPRE.

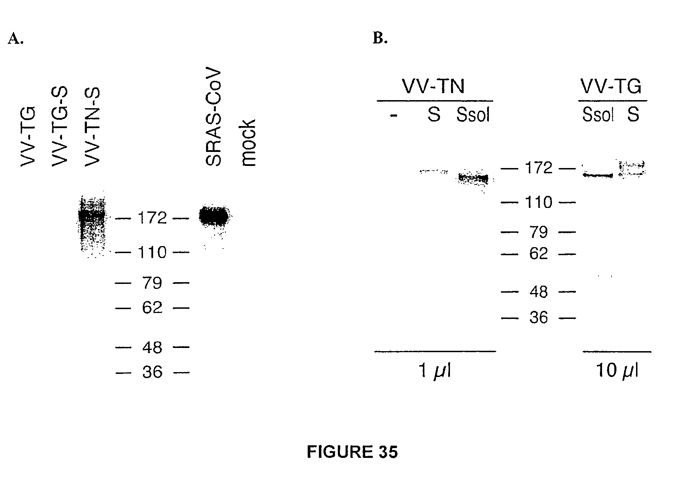

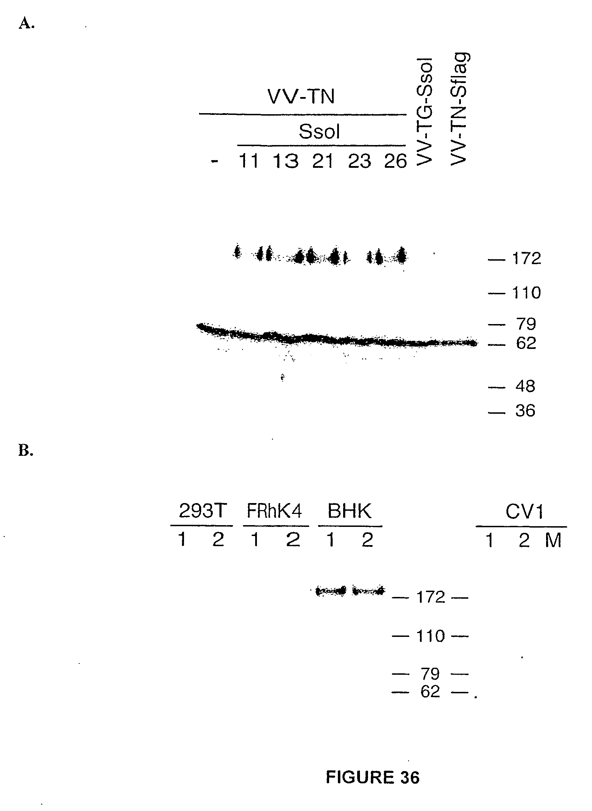

   Les sérums immuns prélevés 3 semaines après la deuxième immunisation ont été analysés par ELISA indirect en utilisant un lysat de cellules VeroE6 infectées par le SRAS-CoV comme antigène. Les titres en anticorps anti-SRAS-CoV sont calculés comme l'inverse de la dilution produisant une DO spécifique de 0,5 après révélation par un anticorps polyclonal anti-IgG de souris couplé à la peroxidase (NA931V, Amersham) et du TMB (KPL).

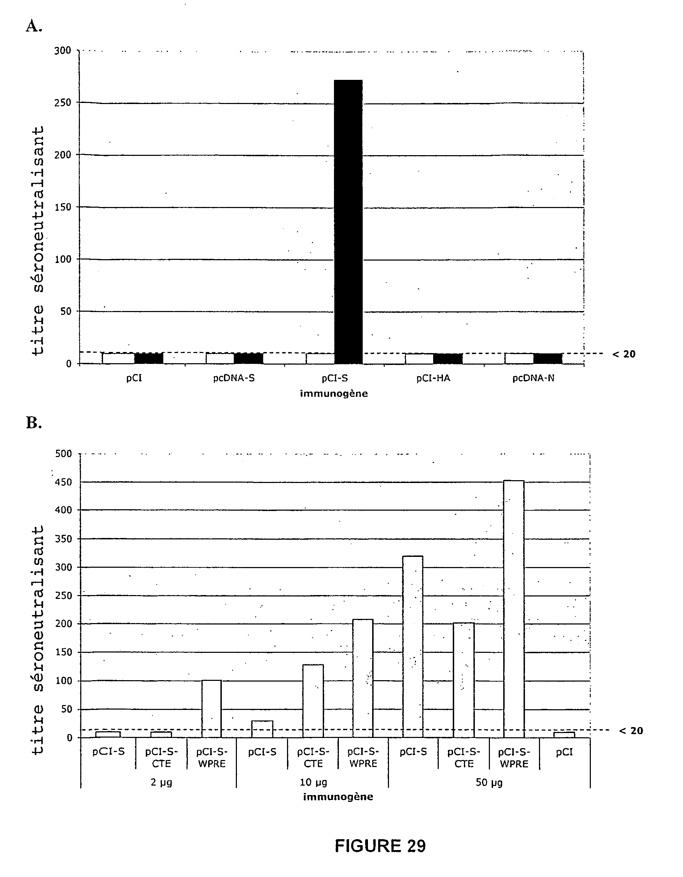

- la figure 29 montre la séroneutralisation de l'infectivité du SRAS-CoV par les anticorps induits chez la souris après immunisation génique à l'aide d'ADN plasmidique codant la S du SRAS-CoV. Des pools de sérums immuns prélevés 3 semaines après la deuxième immunisation ont été réalisés pour chacun des groupes des expériences décrites dans la figure 28 et évalués pour leur capacité à séroneutraliser l'infectivité de 100 TCID50 du SRAS-CoV sur cellules FRhK-4. 4 points sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4.
   A. Groupes de souris BALB/c immunisés à deux reprises à 4 semaines d'intervalle à l'aide de 50 µg d'ADN plasmidique de pCI, pcDNA-S, pCI-S, pcDNA-N et pCI-HA. □ : sérum préimmun. ■ : sérum immun.
   B. Groupes de souris BALB/c immunisés à deux reprises à 4 semaines d'intervalle à l'aide de 2 µg, 10µg ou 50 µg d'ADN plasmidique de pCI, pCI-S, pCI-S-CTE et pCI-S-WPRE.

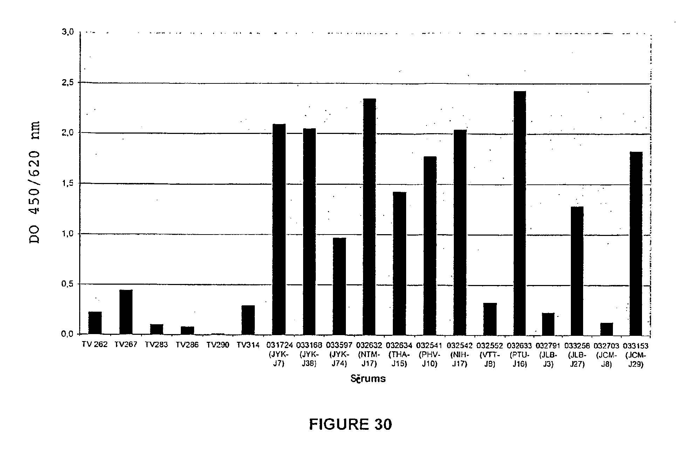

- la figure 30 illustre l'immunoréactivité du polypeptide recombinant Ssol vis à vis de sérums de patients atteints de SRAS. La réactivité de sérums de patients a été analysée par test ELISA indirect contre des phases solides préparées à l'aide du polypeptide recombinant Ssol purifié. Les anticorps de patients réagissant avec la phase solide à une dilution de 1/400 sont révélés par un anticorps polyclonal anti-IgG(H+L) humain couplé à la peroxidase (Amersham NA933V) et du TMB plus H2O2 (KPL). Les sérums de cas probables de SRAS sont identifiés par un numéro d'ordre du Centre National de Référence des Virus Influenzae ainsi que par les initiales du patient et le nombre de jours écoulés depuis le début des symptômes, le cas échéant. Les sérums TV sont des sérums témoins de sujets qui ont été prélevés en France avant l'épidémie de SRAS survenue en 2003.

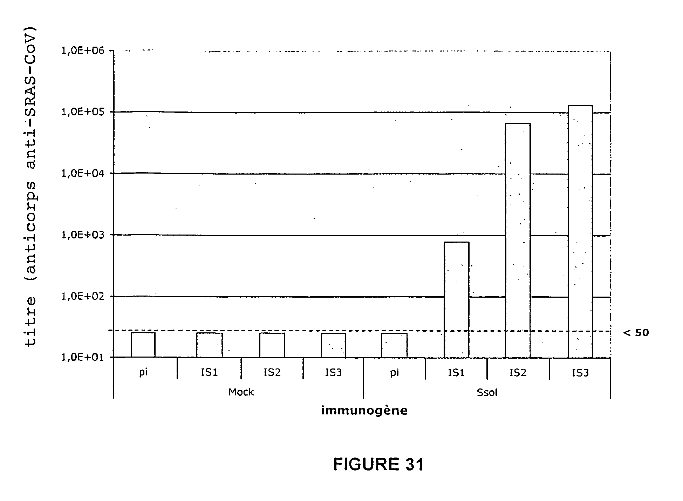

- La figure 31 montre l'induction d'anticorps dirigés contre le SRAS-CoV après immunisation avec le polypeptide recombinant Ssol. Deux groupe de 6 souris ont été immunisés à 3 semaines d'intervalle avec 10 µg de polypeptide recombinant Ssol (groupe Ssol) adjuvé avec de l'hydroxyde d'aluminium ou, à titre de contrôle, de l'adjuvant seul (groupe mock).Trois immunisations successives ont été réalisées et les sérums immuns ont été prélevés 3 semaines après chacune des trois immunisations (IS1, IS2, IS3). Les sérums immuns ont été analysés par pool pour chacun des 2 groupes par ELISA indirect en utilisant un lysat de cellules VeroE6 infectées par le SRAS-CoV comme antigène. Les titres en anticorps anti-SRAS-CoV sont calculés comme l'inverse de la dilution produisant une DO spécifique de 0,5 après révélation par un anticorps polyclonal anti-IgG de souris couplé à la peroxidase (Amersham) et du TMB (KPL).

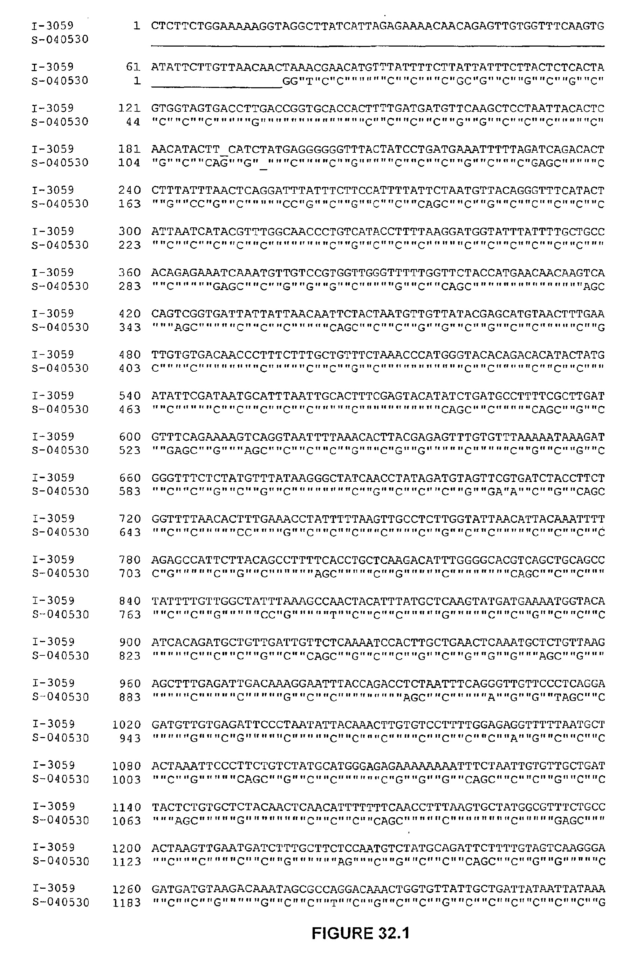

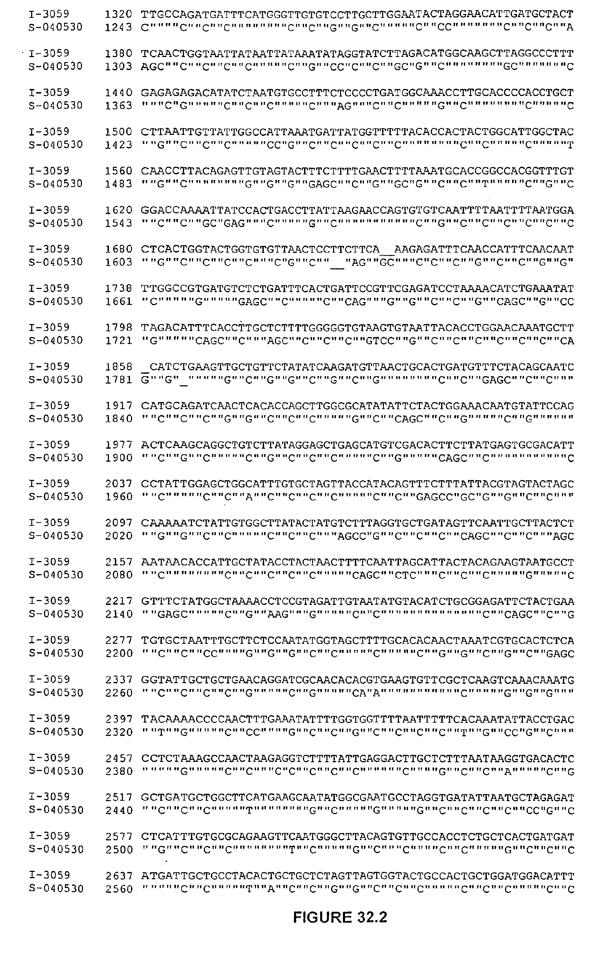

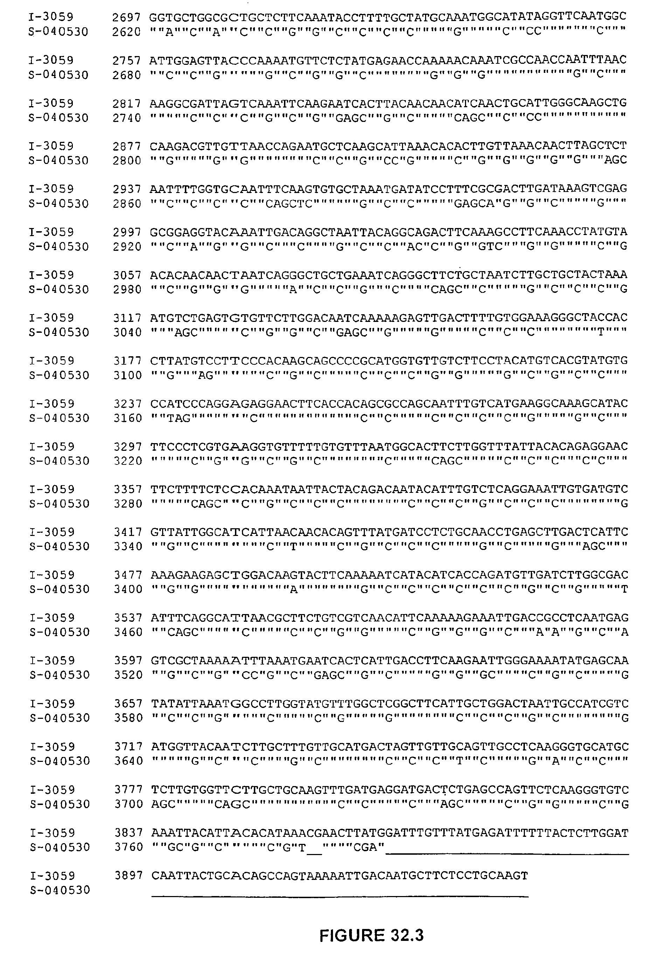

- La figure 32 présente l'alignement nucléotidique des séquences du gène synthétique 040530 avec la séquence du gène sauvage de l'isolat 031589 du SRAS-CoV. I-3059 correspond aux nucléotides 21406-25348 de l'isolat 031589 du SRAS-CoV déposé à la C.N.C.M. sous le numéro 1-3059 (SEQ ID NO : 4, plasmide pSRAS-S). S-040530 est la séquence du gène synthétique 040530.

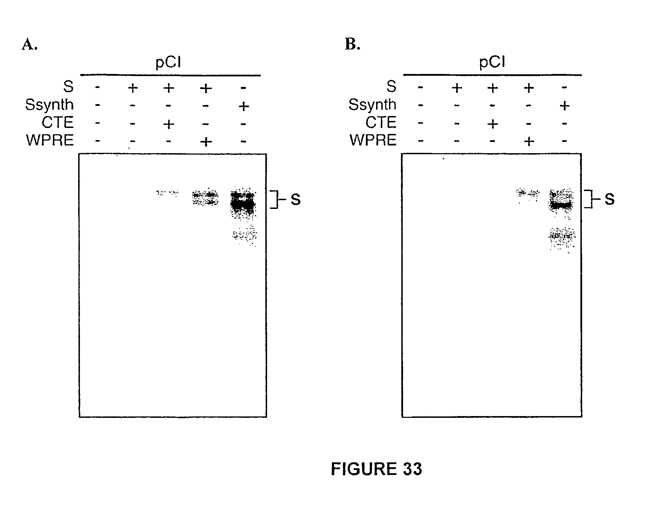

- la figure 33 illustre l'utilisation d'un gène synthétique pour l'expression de la S du SRAS-CoV. Des extraits cellulaires préparés 48 heures après transfection de cellulesVeroE6 (A) ou 293T (B) par les plasmides pCI, pCI-S, pCI-S-CTE, pCI-S-WPRE et pCI-Ssynth ont été séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). Le western blot est révélé par luminescence (ECL+, Amersham) et acquisition sur un dispositif d'imagerie numérique (FluorS, BioRad). Les niveaux d'expression de la protéine S ont été mesurés en quantifiant les 2 bandes majoritaires repérées sur l'image.

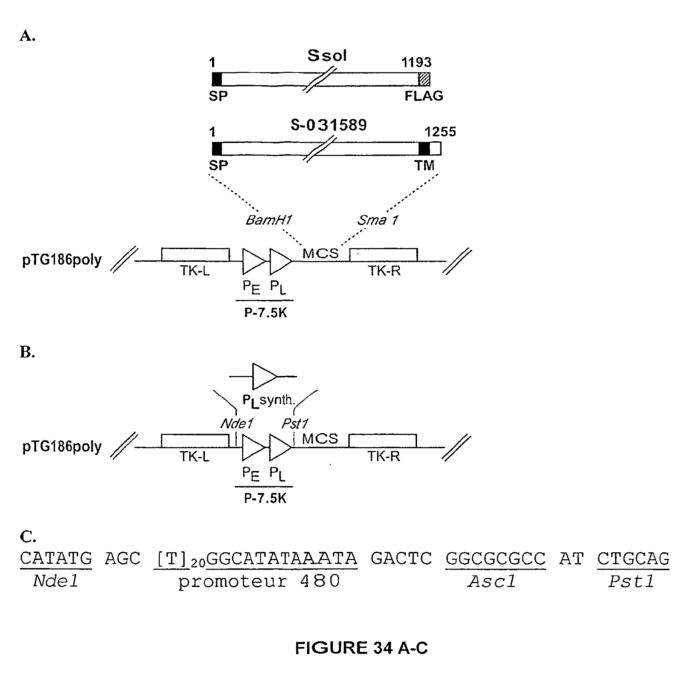

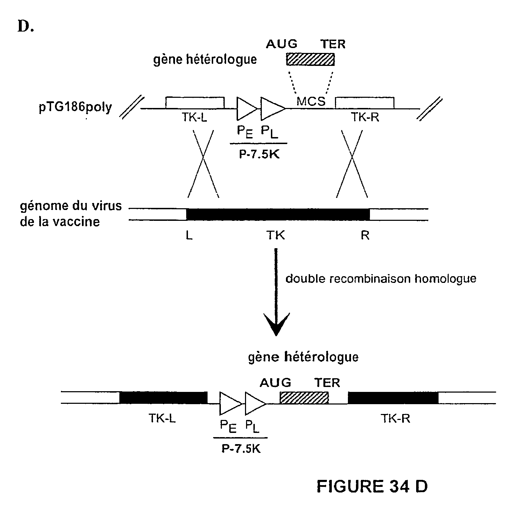

- la figure 34 présente un schéma de la construction des virus vaccine recombinants VV-TG-S, VV-TG-Ssol, VV-TN-S et VV-TN-Ssol
   A. Les cDNA de la protéine S et du polypeptide Ssol du SRAS-CoV ont été inséré entre les sites BamH1 et Sma1 du plasmide de transfert pTG186 pour obtenir les plasmides pTG-S et pTG-Ssol.
   B. Les séquences du promoteur synthétique 480 ont ensuite été substituées à celles du promoteur 7.5 par échange du fragment Nde1-Pst1 des plasmides pTG186poly, pTG-S et pTG-Ssol pour obtenir les plasmides de transfert pTN480, pTN-S et pTN -Ssol.
   C. Séquence du promoteur synthétique 480 tel que contenu entre les sites Nde1 et Pst1 des plasmides de transfert de la série pTN. Un site Asc1 a été inséré pour faciliter les manipulations ultérieures. Les sites de restriction ainsi que la séquence du promoteur sont soulignés
   D. Les virus recombinants de la vaccine sont obtenus par double recombinaison homologue *in vivo* entre la cassette TK des plasmides de transfert des séries pTG et pTN et le gène TK de la souche Copenhague du virus de la vaccine.
      SP : peptide signal prédit (aa 1-13) avec le logiciel signalP v2.0 (Nielsen et al., 1997, Protein Engineering, 10 : 1-6)
      TM : région transmembranaire prédite (aa 1196-1218) avec le logiciel TMHMM v2.0 (Sonnhammer et al., 1998, Proc. of Sixth Int. Conf. on Intelligent Systems for Molecular Biology, p 175-182, AAAI Press). Il faut noter que les acides aminés W1194 et P1195 font possiblement partie de la région transmembranaire avec des probabilités respectives de 0,13 et 0,42.
      TK-L, TK-R : parties gauche et droite du gène de la thymidine kinase du virus de la vaccine
      MCS : site multiple de clonage
      PE : promoteur précoce
      PL : promoteur tardif
      PL synth : promoteur tardif synthétique 480
- la figure 35 illustre l'expression de la protéine S par les virus vaccine recombinants, analysée par western blot. Des extraits cellulaires ont été préparés 18 heures après infection de cellules CV1 par les virus vaccine recombinants VV-TG, VV-TG-S et VV-TN-S à une M.O.I. de 2 (A). A titre de contrôle, des extraits de cellules VeroE6 ont été préparés 8 heures après infection par le SRAS-CoV à une multiplicité d'infection de 2. Des extraits cellulaires ont également été préparés 18 heures après infection de cellules CV1 par les virus vaccine recombinants VV-TG-S, VV-TG-Ssol, VV-TN, VV-TN-S et VV-TN-Ssol (B). Ils ont été séparés sur des gels SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). « 1µl » et « 10µl » indique les quantités d'extraits cellulaires déposées sur le gel. Une échelle de masse moléculaire (kDa) est portée sur la figure.
   SRAS-CoV : extrait de cellules VeroE6 infectées par le SRAS-CoV
   Mock : extrait contrôle de cellules non infectées
- la figure 36 montre le résultat d'une analyse par western blot de la sécrétion du polypeptide Ssol par les virus vaccine recombinants.
   A. Des surnageants de cellules CV1 infectées par le virus vaccine recombinant VV-TN, différents clones du virus VV-TN-Ssol et par les virus VV-TG-Ssol ou VV-TN-Sflag ont été récoltés 18 heures après infection de cellules CV1 à une M.O.I. de 2.
   B. Des surnageants de cellules 293T, FRhK-4, BHK-21 et CV1 infectées en dupliqués (1,2) par le virus vaccine recombinant VV-TN-Ssol à une M .O.I. de 2 ont été récoltés 18 heures après infection. Le surnageant de cellules CV1 infectées par le virus VV-TN a également été récolté à titre de contrôle (M).

   Tous les surnageants ont été séparés sur un gel SDS à 8% d'acrylamide selon Laemmli et analysés par western blot à l'aide d'un anticorps monoclonal de souris anti-FLAG et d'un anticorps polyclonal anti-IgG(H+L) de souris couplé à la peroxidase (NA931V, Amersham) (A) ou à l'aide d'un anticorps polyclonal de lapin anti-S et d'un anticorps polyclonal anti-IgG(H+L) de lapin couplé à la peroxidase (NA934V, Amersham) (B).
   Une échelle de masse moléculaire (kDa) est portée sur la figure.

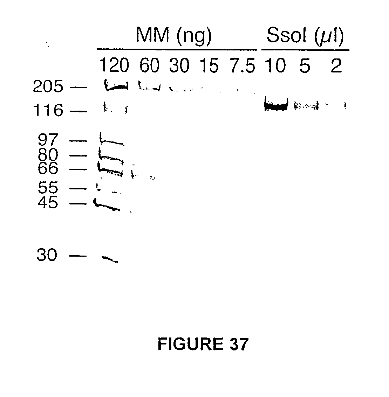

- la figure 37 montre l'analyse du polypeptide Ssol, purifié par gel SDS de polyacrylamide
   10, 5 et 2 µl de polypeptide recombinant Ssol purifié par chromatographie d'affinité anti-FLAG ont été séparés sur gel SDS en gradient de 4 à 15 % de polyacrylamide. Le polypeptide Ssol ainsi que des quantités variables de marqueurs de masse moléculaire (MM) ont été révélés par coloration au nitrate d'argent (Gelcode SilverSNAP stain kit II, Pierce).

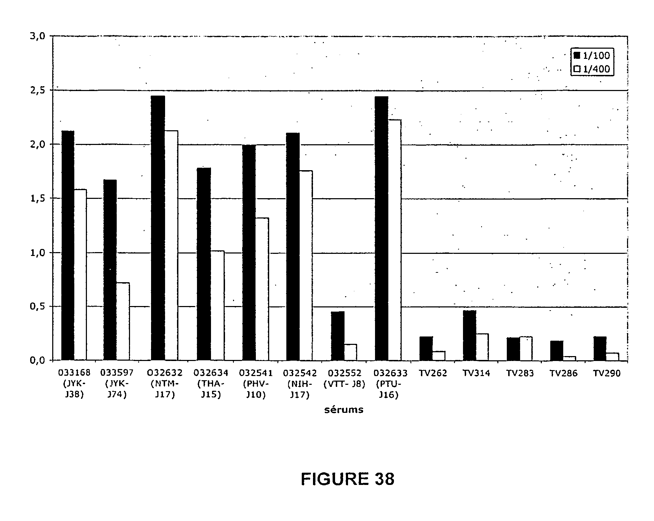

- la figure 38 illustre l'immunoréactivité du polypeptide recombinant Ssol produit par le virus vaccine recombinant VV-TN-Ssol vis-à-vis de sérums de patients atteints de SRAS. La réactivité de sérums de patients a été analysée par test ELISA indirect contre des phases solides préparées à l'aide du polypeptide recombinant Ssol purifié. Les anticorps de patients réagissant avec la phase solide à une dilution de 1/100 et 1/400 sont révélés par un anticorps polyclonal anti-IgG(H+L) humain couplé à la peroxidase (Amersham NA933V) et du TMB plus H2O2 (KPL). Les sérums de cas probables de SRAS sont identifiés par un numéro d'ordre du Centre National de Référence des Virus Influenzae ainsi que par les initiales du patient et le nombre de jours écoulés depuis le début des symptômes, le cas échéant. Les sérums TV sont des sérums témoins de sujets qui ont été prélevés en France avant l'épidémie de SRAS survenue en 2003.

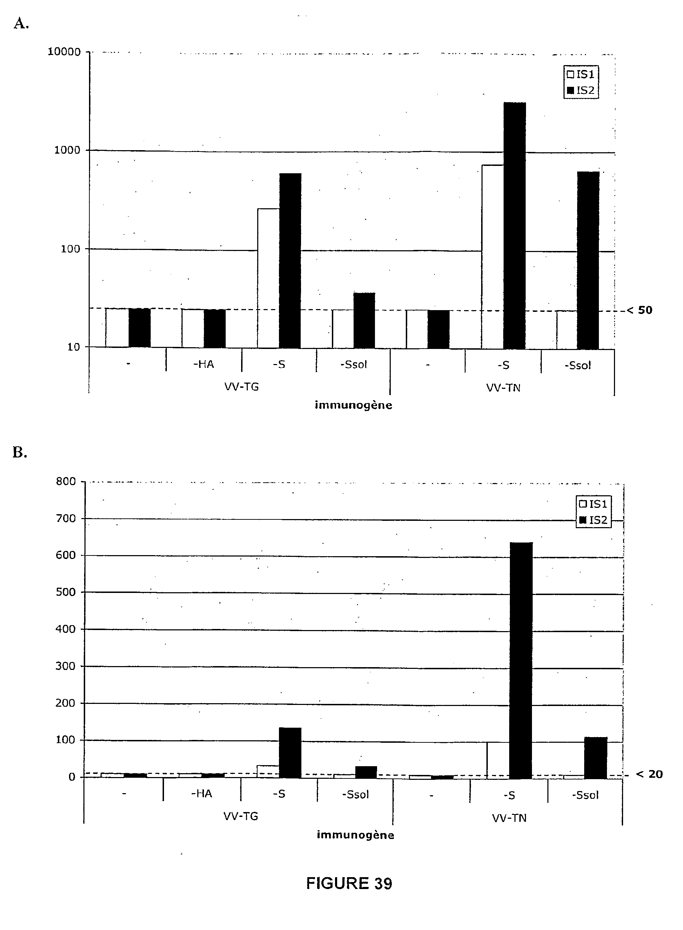

- la figure 39 montre la réponse en anticorps anti-SRAS-CoV chez la souris après immunisation par les virus vaccine recombinants. Des groupes de 7 souris BALB/c ont été immunisés par voie i.v. à deux reprises à 4 semaines d'intervalle par 106 u.f.p. de virus vaccine recombinants VV-TG, VV-TG-HA, VV-TG-S, VV-TG-Ssol, VV-TN, VV-TN-S, VV-TN-Ssol.
   A. Des pools de sérums immuns prélevés 3 semaines après chacune des deux immunisations ont été réalisés pour chacun des groupes et ont été analysés par ELISA indirect en utilisant un lysat de cellules VeroE6 infectées par le SRAS-CoV comme antigène. Les titres en anticorps anti-SRAS-CoV sont calculés comme l'inverse de la dilution produisant une DO spécifique de 0,5 après révélation par un anticorps polyclonal anti-IgG de souris couplé à la peroxidase (NA931V, Amersham) et du TMB (KPL).
   B. Les pools de sérums immuns ont été évalués pour leur capacité à séroneutraliser l'infectivité de 100 TCID50 du SRAS-CoV sur cellules FRhK-4. 4 points sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4.

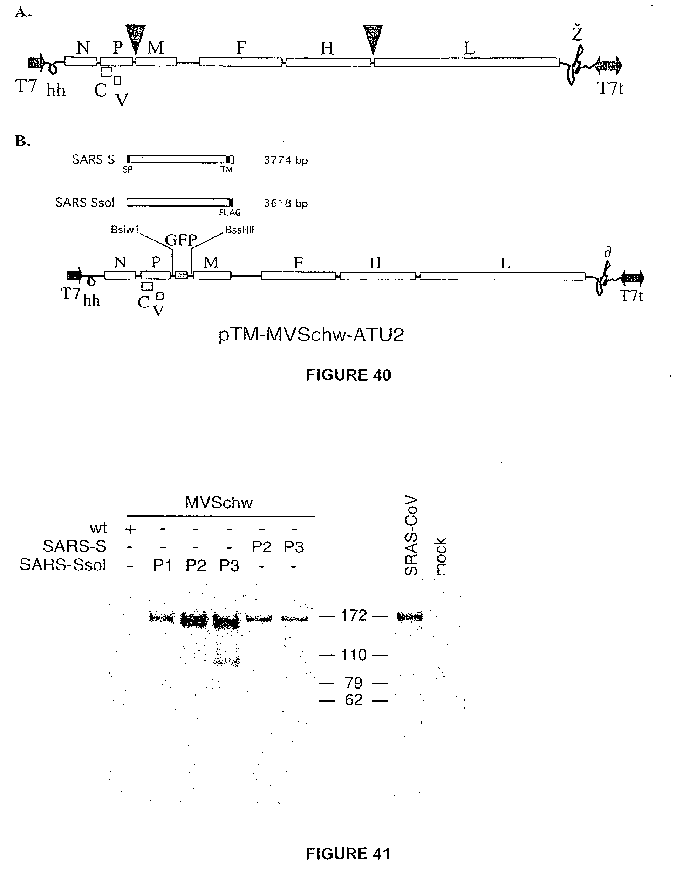

- la figure 40 décrit la construction des virus recombinants MVSchw2-SARS-S et MVSchw2-SARS-Ssol.
   A. Le vecteur rougeole est un génome complet de la souche vaccinale Schwarz du virus de la rougeole (MV) dans lequel une unité de transcription supplémentaire a été introduite (Combredet, 2003, Journal of Virology, 77 : 11546-11554). L'expression des phases ouvertes de lecture (ORF) supplémentaires est contrôlée par les éléments agissant en cis nécessaires à la transcription, à la formation de la coiffe et à la polyadénylation du transgène, qui ont été copiés des éléments présents à la jonction N/P. 2 vecteurs différents permettent l'insertion entre les gènes P (phosphoprotéine) et M (matrice) d'une part et H (hémagglutinine) et L (polymérase) d'autre part.
   B. Les génomes recombinants MVSchw2-SARS-S et MVSchw2-SARS-Ssol du virus de la rougeole ont été construits par l'insertion des ORFs de la protéine S et du polypeptide Ssol au sein d'une unité de transcription supplémentaire localisée entre les gènes P et M du vecteur.

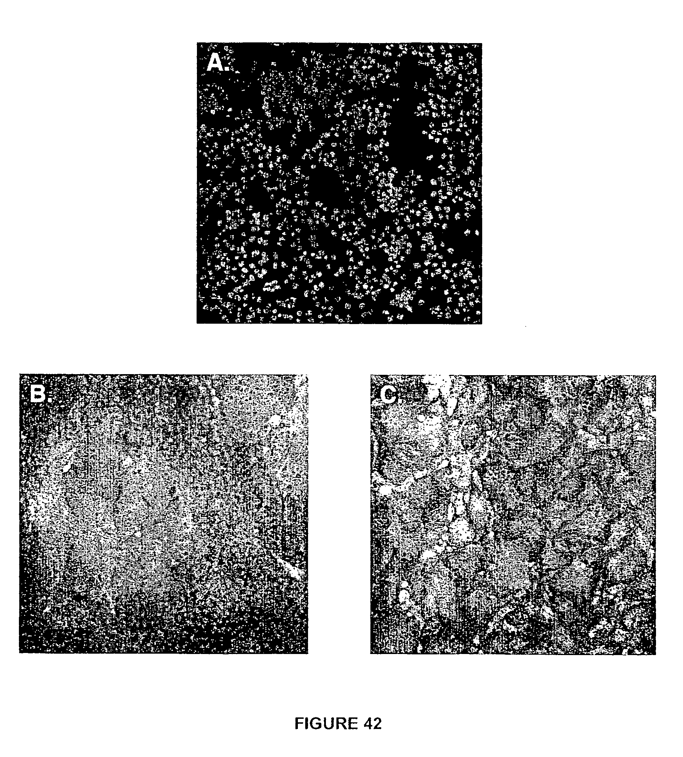

   Les différents gènes du virus de la rougeole (MV) sont indiqués : N (nucléoprotéine), PVC (phosphoprotéine et protéine V/C), M (matrice), F (fusion), H (hémagglutinine), L (polymérase). T7 = promoteur de l'ARN polymérase T7, hh = ribozyme hammerhead, T7t = séquence terminatrice de l'ARN polymérase du phage T7, ∂ = ribozyme du virus de l'hépatite ∂, (2), (3) = unités de transcription supplémentaires (ATU).
   Taille du génome du MV : 15894 nt.
   SP : peptide signal
   TM : région transmembranaire
   FLAG : étiquette FLAG
- la figure 41 illustre l'expression de la protéine S par les virus rougeole recombinants, analysée par western blot.
   Des extraits cytoplasmiques ont été préparés après infection de cellules Vero par différents passages des virus MVSchw2-SARS-S et MVSchw2-SARS-Ssol et le virus sauvage MWSchw à titre de contrôle. Des extraits cellulaires en tampon de dépôt selon Laemmli ont également été préparés 8 heures après infection de cellules VeroE6 par le SRAS-CoV à une multiplicité d'infection de 3. Ils ont été séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham).
   Une échelle de masse moléculaire (kDa) est portée sur la figure.
   Pn : nième passage du virus après coculture de cellules 293-3-46 et Vero.
   SRAS-CoV : extrait de cellules VeroE6 infectées par le SRAS-CoV
   Mock : extrait contrôle de cellules VeroE6 non infectées
- la figure 42 montre l'expression de la protéine S par les virus rougeole recombinants, analysée par immunofluorescence
   Des cellules Vero en monocouches sur lamelles de verre ont été infectées par le virus sauvage MWSchw (A) ou les virus MVSchw2-SARS-S (B) et MVSchw2-SARS-Ssol (C). Quand les syncytia ont atteint 30 à 40% de confluence (A., B.) ou 90-100% (C), les cellules ont été fixées, perméabilisées et marquées par des anticorps polyclonaux de lapins anti-SRAS-CoV et un conjugué anti-IgG(H+L) de lapin couplé au FITC (Jackson).

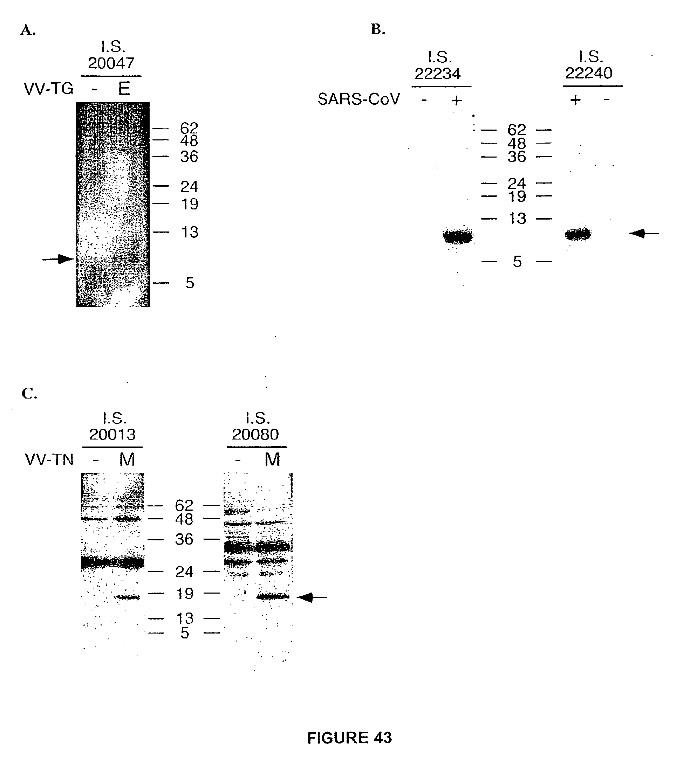

- la figure 43 illustre l'analyse par western blot de l'immunoréactivité de sérums de lapins dirigés contre les peptides E1-12, E53-76 et M2-14. Le lapin 20047 a été immunisé avec le peptide E1-12 couplé à la KLH. Les lapins 22234 et 22240 ont été immunisés avec le peptide E53-76 couplé à la KLH. Les lapins 20013 et 20080 ont été immunisés avec le peptide M2-14 couplé à la KLH. Les immunsérums ont été analysés par western blot à l'aide d'extraits de cellules infectées par le SRAS-CoV (B) ou à l'aide d'extraits de cellules infectées par un virus recombinant de la vaccine exprimant la protéine E (A) ou M (C) de l'isolat 031589 du SRAS-CoV. Les immunoempreintes ont été révélées à l'aide d'un conjugué anti-IgG(H+L) de lapin couplé à la peroxidase (NA934V, Amersham).
   La position des protéines E etM est indiquée par une flèche.
   Une échelle de masse moléculaire (kDa) est portée sur la figure.
   Il doit être bien entendu, toutefois, que ces exemples sont donnés uniquement à titre d'illustration de l'objet de l'invention, dont ils ne constituent en aucune manière une limitation.

### Exemple 1 : Clonage et séquençage du génome de la souche de SARS-CoV issue du prélèvement répertorié sous le numéro 031589

L'ARN de la souche de SARS-CoV a été extrait à partir du prélèvement de lavage bronchoalvéolaire répertorié sous le numéro 031589, effectué sur un patient de l'hôpital français de Hanoi (Vietnam) atteint de SRAS.

L'ARN isolé a été utilisé comme matrice pour amplifier les ADNc correspondant aux différents cadres ouverts de lecture du génome (ORF 1a, ORF1b, ORF-S, ORF-E, ORF-M, ORF-N (incluant les ORF-13 et ORF-14), ORF3, ORF4, ORF7 à ORF11), et aux extrémités 5' et 3' non-codantes. Les séquences des amorces et des sondes utilisées pour l'amplification/détection ont été définies d'après la séquence nucléotidique disponible du SARS-CoV.

Dans ce qui suit les amorces et les sondes sont identifiées par : la lettre S, suivie d'une lettre qui indique la région correspondante du génome (L pour l'extrémité 5'incluant ORF1a et ORF1b ; S, M et N pour les ORF-S, ORF-M, ORF-N, SE et MN pour les régions intergéniques correspondantes), puis éventuellement de Fn, Rn, avec n inclus entre 1 et 6 correspondant aux amorces utilisées pour la PCR nichée ou imbriquée (paire F1 + R1 pour la première amplification, paire F2 + R2 pour la deuxième amplification, etc...), puis de /+/ ou /-/ correspondant à une amorce sens ou antisens et enfin des positions des amorces en référence à la séquence Genbank AY27411.3 ; pour les amorces S et N sens et antisens et les autres amorces sens uniquement, lorsqu'une seule position est indiquée elle correspond à celle de l'extrémité 5' d'une sonde ou d'une amorce d'environ 20 bases ; pour les amorces antisens autres que les amorces S et N, lorsqu'une seule position est indiquée elle correspond à celle de l'extrémité 3' d'une sonde ou d'une amorce d'environ 20 bases.

Les produits d'amplifications ainsi générés ont été séquencés à l'aide d'amorces spécifiques afin de déterminer la séquence complète du génome de la souche de SARS-CoV issue du prélèvement répertorié sous le numéro 031589. Ces produits d'amplification, à l'exception de ceux correspondant aux ORF1a et ORF1b, ont ensuite été clonés dans des vecteurs d'expression afin de produire les protéines virales correspondantes et les anticorps dirigés contre ces protéines, notamment par immunisation à base d'ADN.

### 1. Extraction des ARN

Les ARN ont été extraits à l'aide du kit *QIamp viral RNA extraction mini* (QIAGEN) en suivant les recommandations du fabricant. De manière plus précise : 140 µl du prélèvement et 560 µl de tampon AVL ont été mélangés vigoureusement pendant 15 secondes, incubés 10 min à température ambiante puis centrifugés brièvement à vitesse maximale. 560 µl d'éthanol à 100% ont été ajoutés au surnageant et le mélange ainsi obtenu a été agité très vigoureusement pendant 15 sec. 630 µl du mélange ont ensuite été déposés sur la colonne.

La colonne a été placée sur un tube de 2 ml, centrifugée 1 min à 8000 rpm, puis le reste du mélange précédent a été déposé sur la même colonne, centrifugé à nouveau, 1 min à 8000 rpm et la colonne a été transférée sur un tube de 2 ml propre. Ensuite, 500 µl de tampon AW1 ont été ajoutés sur la colonne, puis la colonne a été centrifugée 1 min à 8000 rpm et l'éluat a été éliminé. 500 µl de tampon AW2 ont été ajoutés sur la colonne qui a ensuite été centrifugée 3 min à 14000 rpm et transférée sur un tube de 1,5 ml. Enfin, 60 µl de tampon AVE ont été ajoutés sur la colonne qui a été incubée 1 à 2 min à température ambiante puis centrifugée 1 min à 8000 rpm. L'éluat correspondant à l'ARN purifié a été récupéré et congelé à-20°C.

### 2. Amplification, séquençage et clonage des ADNc

### 2.1) ADNc codant pour la protéine S

Les ARN extraits à partir du prélèvement ont été soumis à une transcription inverse à l'aide d'oligonucléotides hexamériques de séquence aléatoire (pdN6), afin de produire des fragments d'ADNc.

La séquence codant pour la glycoprotéine S du SARS-CoV a été amplifiée sous la forme de deux fragments d'ADN chevauchants : fragment 5' (SRAS-Sa, SEQ ID NO:5) et fragment 3'(SRAS-Sb, SEQ ID NO:6), en réalisant deux amplifications successives à l'aide d'amorces imbriquées. Les amplicons ainsi obtenus ont été séquencés, clonés dans le vecteur plasmidique PCR 2.1-TOPO™ (IN VITROGEN), puis la séquence des ADNc clonés a été déterminée.

### a)clonage et séquençage des fragments Sa et Sb

### a₁) synthèse de l'ADNc

Le mélange réactionnel contenant : ARN (5 µl), H₂O ppi (3,5 µl), tampon de transcriptase inverse5X (4 µl, ), dNTP 5 mM (2 µl), pdN6 100 ug/ml (4 µl), RNasin 40 UI/ul (0,5 µl) et transcriptase inverse AMV-RT, 10 UI/ul, PROMEGA (1µl) a été incubé dans un thermocycleur dans les conditions suivantes : 45 min à 42°C, 15 min à 55°C, 5 min à 95°C, puis l'ADNc obtenu a été maintenu à +4°C.

### a₂) première amplification PCR

Les extrémités 5' et 3' du gène S ont été amplifiées respectivement avec les paires d'amorces S/F1/+/ 21350-21372 et S/R1/-/ 23518-23498, S/F3/+/ 23258-23277 et S/R3/-/25382-25363. Le mélange réactionnel de 50 µl contenant : ADNc (2 µl), amorces 50 µM (0,5 µl), tampon 10 X (5 µl), dNTP 5 mM (2 µl), Taq Expand High Fidelity, Roche (0,75 µl) et H₂0 (39, 75 µl) a été amplifié dans un thermocycleur, dans les conditions suivantes : une étape initiale de dénaturation à 94°C pendant 2 min a été suivie de 40 cycles comprenant : une étape de dénaturation à 94°C pendant 30 sec, une étape d'hybridation à 55°C pendant 30 sec puis une étape d'élongation à 72°C pendant 2 min 30 sec, avec 10 sec d'élongation supplémentaire à chaque cycle, puis d'une étape finale d'élongation à 72°C pendant 5 min.

### a₃) deuxième amplification PCR

Les produits de la première amplification PCR (amplicons 5' et 3') ont subi une seconde étape d'amplification PCR (PCR nichée) dans des conditions identiques à celles de la première amplification, avec les paires d'amorces S/F2/+/21406-21426 et S/R2/-/23454-23435, et S/F4/+/23322-23341 et S/R4/-/25348-25329, respectivement pour l'amplicon 5' et l'amplicon 3'.

### a₄) clonage et séquençage des fragments Sa et Sb

Les amplicons Sa (extrémité 5') et Sb (extrémité 3') ainsi obtenus ont été purifiés à l'aide du kit *QIAquick PCR purification* (QIAGEN), en suivant les recommandations du fabricant, puis ils ont été clonés dans le vecteur PCR2.1-TOPO (kit Invitrogen), pour donner les plasmides dénommés SRAS-S1 et SRAS-S2.

L'ADN des clones Sa et Sb a été isolé puis l'insert correspondant a été séquencé à l'aide du Kit Big Dye, Applied Biosystem® et des amorces universelles M13 forward et M13 reverse, ainsi que des amorces: S/S/+/21867, S/S/+/22353, S/S/+/22811, S/S/+/23754, S/S/+/24207, S/S/+/24699, S/S/+/24348, S/S/-/24209, S/S/-/23630, S/S/-/23038, S/S/-/22454, S/S/-/21815, S/S/-/24784, S/S/+/21556, S/S/+/23130 et S/S/+/24465, en suivant les instructions du fabricant ; les séquences des fragments Sa et Sb ainsi obtenues correspondent aux séquences SEQ ID NO :5 et SEQ ID NO :6 dans la liste de séquences jointe en annexe.

Le plasmide, dénommé SARS-S1 a été déposé sous le n° 1-3020, le 12 mai 2003, auprès de la Collection Nationale de Cultures de Microorganismes, 25 rue du Docteur Roux, 75724 Paris Cedex 15 ; il contient un fragment 5' de la séquence du gène S de la souche de SARS-CoV issue du prélèvement répertorié sous le n° 031559, telle que définie ci-dessus, lequel fragment dénommé Sa correspondant aux nucléotides des positions 21406 à 23454 (SEQ ID NO :5), en référence à la séquence Genbank AY274119.3 Tor2.

Le plasmide, dénommé TOP10F'-SARS-S2 a été déposé sous le n° 1-3019, le 12 mai 2003, auprès de la Collection Nationale de Cultures de Microorganismes, 25 rue du Docteur Roux, 75724 Paris Cedex 15 ; il contient un fragment 3'de la séquence du gène S de la souche de SARS-CoV issue du prélèvement répertorié sous le n° 031589, telle que définie ci-dessus, lequel fragment dénommé Sb correspondant aux nucléotides des positions 23322 à 25348 (SEQ ID NO : 6), en référence à la séquence Genbank n° d'accès AY274119.3.

### b) clonage et séquençage de l'ADNc complet (clone SRAS-S de 4 kb)

L'ADNc S complet a été obtenu à partir des clones SARS-S1 et SARS-S2 précités, de la façon suivante :
1) une réaction d'amplification PCR a été réalisée sur un clone SARS-S2 en présence de l'amorce S/R4/-/25348-25329 précitée et de l'amorce S/S/+/24696-24715: un amplicon de 633 pb a été obtenu,
2) une autre réaction d'amplification PCR a été réalisée sur un autre clone SARS-S2, en présence des amorces S/F4/+/23322-23341 précitée et S/S/- /24803-24784: un amplicon de 1481 pb a été obtenu,
   La réaction d'amplification a été réalisée dans les conditions telles que définies ci-dessus pour l'amplification des fragments Sa et Sb, à l'exception que 30 cycles d'amplifications comprenant une étape de dénaturation à 94° C pendant 20 sec et une étape d'élongation à 72° C pendant 2 min 30 sec ont été effectués.
3) les 2 amplicons (633 pb et 1481 pb) ont été purifiés dans les conditions telles que définies ci-dessus pour les fragments Sa et Sb.
4) une autre réaction d'amplification PCR à l'aide des amorces S/F4/+/23322-23341 et S/R4/-/25348-25329 précitées, a été réalisée sur les amplicons purifiés obtenus en 3). La réaction d'amplification a été réalisée dans les conditions telles que définies ci-dessus pour l'amplification des fragments Sa et Sb, à l'exception que 30 cycles d'amplifications ont été effectués.
   L'amplicon de 2026 pb ainsi obtenu a été purifié, cloné dans le vecteur PCR2.1-TOPO puis séquencé comme ci-dessus, à l'aide des amorces telles que définies ci-dessus pour les fragments Sa et Sb. Le clone ainsi obtenu a été dénommé clone 3'.
5) Le clone SARS-S1 précédemment obtenu et le clone 3'ont été digérés par *EcoR I*, les bandes d'environ 2kb ainsi obtenues ont été purifiées sur gel puis amplifiées par PCR avec les amorces S/F2/+/21406-21426 et S/R4/-/25348-25329 précitées. La réaction d'amplification a été réalisée dans les conditions telles que définies ci-dessus pour l'amplification des fragments Sa et Sb, à l'exception que 30 cycles d'amplifications ont été effectués. L'amplicon d'environ 4 kb a été purifié et séquencé. Il a ensuite été cloné dans le vecteur PCR2.1-TOPO pour donner le plasmide, dénommé SARS-S, et l'insert contenu dans ce plasmide a été séquencé comme ci-dessus, à l'aide des amorces telles que définies ci-dessus pour les fragments Sa et Sb. Les séquences d'ADNc de l'insert et de l'amplicon codant pour la protéine S, correspondent respectivement aux séquences SEQ ID NO : 4 et SEQ ID NO : 2 dans la liste de séquences jointe en annexe, elles codent pour la protéine S (SEQ ID NO : 3).

La séquence de l'amplicon correspondant à l'ADNc codant pour la protéine S de la souche de SARS-CoV issue du prélèvement n°031589 présente les deux mutations suivantes par rapport aux séquences correspondantes de respectivement les isolats Tor2 et Urbani, les positions des mutations étant indiquées en référence à la séquence complète du génome de l'isolat Tor2 (Genbank AY274119.3) :
- g/t en position 23220 ; le codon alanine (gct) en position 577 de la séquence en acides aminés de la protéine S de Tor2 est remplacé par un codon sérine (tct),
- c/t en position 24872 : cette mutation ne modifie pas la séquence en acides aminés de la protéine S, et

Le plasmide, dénommé SARS-S, a été déposé sous le n° 1-3059, le 20 juin 2003, auprès de la Collection Nationale de Cultures de Microorganismes, 25 rue du Docteur Roux, 75724 Paris Cedex 15 ; il contient la séquence d'ADNc codant pour la protéine S de la souche de SARS-CoV issue du prélèvement répertorié sous le n° 031589, laquelle séquence correspondant aux nucléotides des positions 21406 à 25348 (SEQ ID NO :4), en référence à la séquence Genbank AY274119.3.

### 2.2) ADNc codant pour les protéines M et E

Les ARN issus du prélèvement 031589, extraits comme ci-dessus, ont été soumis à une transcription inverse, associée, lors de la même étape (kit *Titan One Step RT-PCR*®, Roche), à une réaction d'amplification par PCR, à l'aide des couples d'amorces :
- S/E/F1/+/26051-26070 et S/E/R1/-/26455-26436 pour amplifier l'ORF-E, et
- S/M/F1/+/26225-26244 et S/M/R1/-/27148-27129 pour amplifier l'ORF-M.

Un premier mélange réactionnel contenant : 8,6 µl d'H₂Oppi, 1 µl de dNTP (5mM), 0,2 µl de chacune des amorces (50µM), 1,25 µl de DTT (100mM) et 0,25 µl de RNAsin (40UI/µl) a été combiné avec un deuxième mélange réactionnel contenant : 1 µl d'ARN, 7 µl d'H₂Oppi, 5 µl de tampon de RT-PCR 5X et 0,5 µl de mélange d'enzyme et les mélanges combinés ont été incubés dans un thermocycleur dans les conditions suivantes : 30 min à 42°C, 10 min à 55°C, 2 min à 94°C suivi de 40 cycles comprenant une étape de dénaturation à 94°C pendant 10 sec, une étape d'hybridation à 55°C pendant 30 sec et une étape d'élongation à 68°C pendant 45 sec, avec 3 sec d'incrément par cycle et enfin une étape d'élongation terminale à 68°C pendant 7 min.

Les produits d'amplification ainsi obtenus (amplicons M et E) ont subi une deuxième amplification PCR (PCR nichée) en utilisant le kit Expand High-Fi®, Roche), à l'aide des couples d'amorces :
- S/E/F2/+/26082-26101 et S/E/R2/-/26413-26394 pour l'amplicon E, et
- S/M/F2/+/26330-26350 et S/M/R2/-/27098-27078 pour l'amplicon M.
Le mélange réactionnel contenant : 2 µl du produit de la première PCR, 39,25 µl d'H₂Oppi, 5 µl de tampon 10X contenant du MgCl₂, 2 µl de dNTP (5mM), 0,5µl de chacune des amorces (50 µM) et 0,75µl de mélange d'enzyme a été incubé dans un thermocycleur dans les conditions suivantes : une étape de dénaturation à 94°C pendant 2 min a été suivie de 30 cycles comprenant une étape de dénaturation à 94°C pendant 15 sec, une étape d'hybridation à 60°C pendant 30 sec et une étape d'élongation à 72°C pendant 45 sec, avec 3 sec d'incrément par cycle, et enfin une étape d'élongation terminale à 72°C pendant 7 min. Les produits d'amplification obtenus correspondant aux ADNc codant pour les protéines E et M ont été séquencés comme ci-dessus, à l'aide des amorces: S/E/F2/+/26082 et S/E/R2/-/26394, S/M/F2/+/26330, S/M/R2/-/27078 précitées et des amorces S/M/+/26636-26655 et S/M/-/26567-26548. Ils ont ensuite été clonés, comme ci-dessus, pour donner les plasmides dénommés SARS-E et SARS-M. L'ADN de ces clones a ensuite été isolé et séquencé à l'aide des amorces universelles M13 forward et M13 reverse ainsi que des amorces S/M/+/26636 et S/M/-/26548 précitées.

La séquence de l'amplicon représentant l'ADNc codant pour la protéine E (SEQ ID NO : 13) de la souche de SARS-CoV issue du prélèvement n°031589 ne comporte pas de différences par rapport aux séquences correspondantes des isolats AY274119.3-Tor2 et AY278741-Urbani. La séquence de la protéine E de la souche de SARS-CoV 031589 correspond à la séquence SEQ ID NO : 14 dans la liste de séquences jointe en annexe.

Le plasmide, dénommé SARS-E a été déposé sous le n° 1-3046, le 28 mai 2003, auprès de la Collection Nationale de Cultures de Microorganismes, 25 rue du Docteur Roux, 75724 Paris Cedex 15 ; il contient la séquence d'ADNc codant pour la protéine E de la souche de SARS-CoV issue du prélèvement répertorié sous le n° 031589, telle que définie ci-dessus, laquelle séquence correspondant aux nucléotides des positions 26082 à 26413 (SEQ ID NO : 15), en référence à la séquence Genbank n° d'accès AY274119.3.

La séquence de l'amplicon représentant l'ADNc codant pour la M (SEQ ID NO : 16) de la souche de SARS-CoV issue du prélèvement n°031589 ne comporte pas de différences par rapport à la séquence correspondante de l'isolat AY274119.3-Tor2. En revanche, en position 26857, l'isolat AY278741-Urbani comporte un c et la séquence de la souche de SARS-CoV issue du prélèvement répertorié sous le n°031589 un t. Cette mutation aboutit à une modification de la séquence en acides aminés de la protéine correspondante: en position 154, une proline (AY278741-Urbani) est changée en sérine dans la souche de SARS-CoV issue du prélèvement répertorié sous le n°031589. La séquence de la protéine M de la souche de SARS-CoV issue du prélèvement répertorié sous le n°031589 correspond à la séquence SEQ ID NO : 17 dans la liste de séquences jointe en annexe.

Le plasmide, dénommé SARS-M a été déposé sous le n° 1-3047, le 28 mai 2003, auprès de la Collection Nationale de Cultures de Microorganismes, 25 rue du Docteur Roux, 75724 Paris Cedex 15 ; il contient la séquence d'ADNc codant pour la protéine M de la souche de SARS-CoV issue du prélèvement répertorié sous le n° 031589, telle que définie ci-dessus ; laquelle séquence correspondant aux nucléotides des positions 26330 à 27098 (SEQ ID NO : 18), en référence à la séquence Genbank n° d'accès AY274119.3.

### 2.3) ADNc correspondant aux ORF3, ORF4, ORF7 à ORF11

La même stratégie d'amplification, de clonage et de séquençage a été utilisée pour obtenir les fragments d'ADNc correspondant respectivement aux ORF suivantes: ORF 3, ORF4, ORF7, ORF8, ORF9, ORF10 et ORF11. Les couples d'amorces utilisées pour la première amplification sont :
- ORF3 et ORF4 : S/SE/F1/+/25069-25088 et S/SE/R1/-/26300-26281
- ORF7 à ORF11 : S/MN/F1/+/26898-26917 et S/MN/R1/-/28287-28266

Les couples d'amorces utilisées pour la deuxième amplification sont :
- ORF3 et ORF4 : S/SE/F2/+/25110-25129 et S/SE/R2/-/26244-26225
- ORF7 à ORF11 : S/MN/F2/+/26977-26996 et S/MN/R2/-/28218-28199

Les conditions de la première amplification (RT-PCR) sont les suivantes : 45 min à 42°C, 10 min à 55°C, 2 min à 94°C suivi de 40 cycles comprenant une étape de dénaturation à 94°C pendant 15 sec, une étape d'hybridation à 58°C pendant 30 sec et une étape d'élongation à 68°C pendant 1 min, avec 5 sec d'incrément par cycle et enfin une étape d'élongation terminale à 68°C pendant 7 min.
Les conditions de la PCR nichée sont les suivantes : une étape de dénaturation à 94°C pendant 2 min a été suivie de 40 cycles comprenant une étape de dénaturation à 94°C pendant 20 sec, une étape d'hybridation à 58°C pendant 30 sec et une étape d'élongation à 72°C pendant 50 sec, avec 4 sec d'incrément par cycle et enfin une étape d'élongation terminale à 72°C pendant 7 min.

Les produits d'amplification obtenus correspondant aux ADNc contenant respectivement les ORF3 et 4 et les ORF7 à 11 ont été séquencés à l'aide des amorces : S/SE/+/25363, S/SE/+/25835, S/SE/-/25494, S/SE/-/25875, S/MN/+/27839, S/MN/+/27409, S/MN/-/27836 S/MN/-/27799 et clonés comme ci-dessus pour les autres ORF, pour donner les plasmides dénommés SARS-SE et SARS-MN. L'ADN de ces clones a été isolé et séquencé à l'aide de ces mêmes amorces et des amorces universelles M 13 sens et M 13 anti-sens.

La séquence de l'amplicon représentant l'ADNc de la région contenant les ORF 3 et 4 (SEQ ID NO :7) de la souche de SARS-CoV issue du prélèvement n°031589 comporte une différence nucléotidique par rapport à la séquence correspondante de l'isolat AY274119-Tor2. Cette mutation en position 25298 aboutit à une modification de la séquence en acides aminés de la protéine correspondante (ORF 3): en position 11, une arginine (AY274119-Tor2) est changée en glycine dans la souche de SARS-CoV issue du prélèvement n°031589. En revanche, aucune mutation n'a été identifiée par rapport à la séquence correspondante de l'isolat AY278741-Urbani. Les séquences des ORF 3 et 4 la souche de SARS-CoV issue du prélèvement n°031589 correspondent respectivement aux séquences SEQ ID NO : 10 et 12 dans la liste de séquences jointe en annexe.

Le plasmide, dénommé SARS-SE a été déposé sous le n° I-3126, le 13 novembre 2003, auprès de la Collection Nationale de Cultures de Microorganismes, 25 rue du Docteur Roux, 75724 Paris Cedex 15 ; il contient l'ADNc correspondant à la région située entre l'ORF-S et l'ORF-E et chevauchant l'ORF-E de la souche de SARS-CoV issue du prélèvement répertorié sous le n° 031589, telle que définie ci-dessus, laquelle région correspondant aux nucléotides des positions 25110 à 26244 (SEQ ID NO :8), en référence à la séquence Genbank n° d'accès AY274119.3,

La séquence de l'amplicon représentant l'ADNc correspondant à la région contenant les ORF7 à ORF11 (SEQ ID NO :19) de la souche de SARS-CoV issue du prélèvement n°031589 ne comporte pas de différences par rapport aux séquences correspondantes des isolats AY274119-Tor2 et AY278741-Urbani. Les séquences des ORF7 à 11 de la souche de SARS-CoV issue du prélèvement n°031589 correspondent respectivement aux séquences SEQ ID NO : 22, 24, 26, 28 et 30 dans la liste de séquences jointe en annexe.

Le plasmide dénommé SARS-MN a été déposé sous le n° I-3125, le 13 novembre 2003, auprès de la Collection Nationale de Cultures de Microorganismes, 25 rue du Docteur Roux, 75724 Paris Cedex 15 ; il contient la séquence d'ADNc correspondant à la région située entre l'ORF-M et l'ORF-N de la souche de SARS-CoV issue du prélèvement répertorié sous le n° 031589 et prélevée à Hanoi, telle que définie ci-dessus, laquelle séquence correspondant aux nucléotides des positions 26977 à 28218 (SEQ ID NO :20 ), en référence à la séquence Genbank n° d'accès AY274119.3.

La séquence de l'amplicon représentant l'ADNc correspondant à la région contenant les ORF7 à ORF11 (SEQ ID NO :19) de la souche de SARS-CoV issue du prélèvement n°031589 ne comporte pas de différences par rapport aux séquences correspondantes des isolats AY274119-Tor2 et AY278741-Urbani. Les séquences des ORF7 à 11 de la souche de SARS-CoV issue du prélèvement n°031589 correspondent respectivement aux séquences SEQ ID NO : 22, 24, 26, 28 et 30 dans la liste de séquences jointe en annexe.

### 2.4) ADNc codant pour la protéine N et incluant les ORF13 et ORF14

L'ADNc a été synthétisé et amplifié comme décrit ci-dessus pour les fragments Sa et Sb. De manière plus précise, le mélange réactionnel contenant : 5 µl d'ARN, 5 µl d'H₂O ppi 4 µl de tampon de reverse transcriptase 5X, 2 µl de dNTP (5 mM), 2 µl d'oligo 20T (5 µM), 0,5 µl de RNasin (40 UI/ul) et 1, 5 µl de AMV-RT (10 UI/ul Promega) a été incubé dans un thermocycleur dans les conditions suivantes : 45 min à 42°C, 15 min à 55°C, 5 min à 95°C, puis il a été maintenu à +4°C.

Une première amplification PCR a été réalisée avec la paire d'amorces S/N/F3/+/28023 et S/N/R3/-/29480.

Le mélange réactionnel comme ci-dessus pour l'amplification des fragments S1 et S2 a été incubé dans un thermocycleur, dans les conditions suivantes : une étape initiale de dénaturation à 94°C pendant 2 min a été suivie de 40 cycles comprenant une étape de dénaturation à 94°C pendant 20 sec, une étape d'hybridation à 55°C pendant 30 sec puis une étape d'élongation à 72°C pendant 1 min 30 sec avec 10 sec d'élongation supplémentaire à chaque cycle, puis d'une étape finale d'élongation à 72°C pendant 5 min.

L'amplicon obtenu à la première amplification PCR a subi une seconde étape d'amplification PCR (PCR nichée) avec la paires d'amorce S/N/F4/+/28054 et S/N/R4/-/29430 dans des conditions identiques à celles de la première amplification.

Le produit d'amplification obtenu correspondant à l'ADNc codant pour la protéine N de la souche de SARS-CoV issue du prélèvement n°031589a été séquencé à l'aide des amorces: S/N/F4/+/28054, S/N/R4/-/29430, S/N/+/28468, S/N/+/28918 et S/N/-/28607 et cloné comme ci-dessus pour les autres ORF, pour donner le plasmide dénommé SARS-N. L'ADN de ces clones a été isolé et séquencé à l'aide des amorces universelles M13 sens et M13 anti-sens, ainsi que des amorces S/N/+/28468, S/N/+/28918 et S/N/-/28607.

La séquence de l'amplicon représentant l'ADNc correspondant à l'ORF-N et incluant les ORF13 et ORF14 (SEQ ID NO :36) de la souche de SARS-CoV issue du prélèvement n°031589 ne comporte pas de différences par rapport aux séquences correspondantes des isolats AY274119.3-Tor2 et AY278741-Urbani. La séquence de la protéine N de la souche de SARS-CoV issue du prélèvement n°031589 correspond à la séquence SEQ ID NO : 37 dans la liste de séquences jointe en annexe.

Les séquences des ORF13 et 14 de la souche de SARS-CoV issue du prélèvement n°031589 correspondent respectivement aux séquences SEQ ID NO : 32 et 34 dans la liste de séquences jointe en annexe.

Le plasmide dénommé SARS-N a été déposé sous le n° I-3048, le 5 juin 2003, auprès de la Collection Nationale de Cultures de Microorganismes, 25 rue du Docteur Roux, 75724 Paris Cedex 15 ; il contient l'ADNc codant pour la protéine N de la souche de SARS-CoV issue du prélèvement répertorié sous le n° 031589, telle que définie ci-dessus, laquelle séquence correspondant aux nucléotides des positions 28054 à 29430 (SEQ ID NO :38), en référence à la séquence Genbank n° d'accès AY274119.3.

### 2.5) extrémités 5' et 3' non-codantes

### a) extrémité 5'non-codante (5'NC)

### a₁) synthèse de l'ADNc

Les ARN issus du prélèvement 031589, extraits comme ci-dessus, ont été soumis à une transcription inverse dans les conditions suivantes :
L'ARN (15 µl) et l'amorce S/L/-/443 (3 µl à la concentration de 5µm, ont été incubés 10 min à 75°C.

Ensuite, du Tampon de transcriptase inverse 5X (6 µl, INVITROGEN), des dNTP 10 mM (1 µl), du DTT 0,1M (3 µl) ont été ajoutés et le mélange a été incubé à 50°C pendant 3 min.

Enfin la transcriptase inverse (3 µl de Superscript®, INVITROGEN) a été ajoutée au mélange précédent qui a été incubé à 50°C pendant 1h30 puis à 90 °C pendant 2 min.

L'ADNc ainsi obtenu a été purifié à l'aide du kit *QIAquick PCR purification* (QIAGEN), selon les recommandations du fabricant.

### b₁) Réaction à la Terminal Transferase (TdT)

L'ADNc (10 µl) est incubé 2 min à 100°C, conservé dans la glace, puis sont ajoutés : H₂0 (2,5 µl), tampon TdT 5X (4 µl, AMERSHAM), dATP 5mM (2 µl) et TdT (1,5 µl, AMERSHAM). Le mélange ainsi obtenu est incubé 45 min à 37°C puis 2 min à 65°C.

Le produit obtenu est amplifié par une première réaction PCR à l'aide des amorces : S/L/-/225-206 et ancre 14T : 5'-AGATGAATTCGGTACCTTTTTTTTTTTTTT-3' (SEQ ID NO :68). Les conditions de l'amplification sont les suivantes : une étape initiale de dénaturation à 94°C pendant 2 min est suivie de 10 cycles comprenant une étape de dénaturation à 94°C pendant 10 sec, une étape d'hybridation à 45°C pendant 30 sec puis une étape d'élongation à 72°C pendant 30 sec puis de 30 cycles comprenant une étape de dénaturation à 94°C pendant 10 sec, une étape d'hybridation à 50°C pendant 30 sec puis une étape d'élongation à 72°C pendant 30 sec, puis d'une étape finale d'élongation à 72°C pendant 5 min.

Le produit de la première amplification PCR a subi une seconde étape d'amplification à l'aide des amorces : S/L/-/204-185 et ancre 14T précitée dans des conditions identiques à celles de la première amplification. L'amplicon ainsi obtenu a été purifié, séquencé à l'aide de l'amorce S/L/-/182-163 puis il a été cloné comme ci-dessus pour les différentes ORF, pour donner le plasmide dénommé SARS-5'NC. L'ADN de ce clone a été isolé et séquencé à l'aide des amorces universelles M13 sens et M13 anti-sens et de l'amorce S/L/-/182-163 précitée.

L'amplicon représentant l'ADNc correspondant à l'extrémité 5'NC de la souche de SARS-CoV issue du prélèvement répertorié sous le n° 031589 correspond à la séquence SEQ ID NO : 72 dans la liste de séquences jointe en annexe ; cette séquence ne comporte pas de différences par rapport aux séquences correspondantes des isolats AY274119.3-Tor2 et AY278741-Urbani.

Le plasmide dénommé SARS-5'NC a été déposé sous le n° I- 3124, le 7 novembre 2003, auprès de la Collection Nationale de Cultures de Microorganismes, 25 rue du Docteur Roux, 75724 Paris Cedex 15 ; il contient l'ADNc correspondant à l'extrémité 5'non codante du génome de la souche de SARS-CoV issue du prélèvement répertorié sous le n° 031589, telle que définie ci-dessus, laquelle séquence correspondant aux nucléotides des positions 1 à 204 (SEQ ID NO :39), en référence à la séquence Genbank n° d'accès AY274119.3.

### b) extrémité 3'non-codante (3'NC)

### a₁) synthèse de l'ADNc

Les ARN issus du prélèvement 031589, extraits comme ci-dessus, ont été soumis à une transcription inverse, selon le protocole suivant : le mélange réactionnel contenant : ARN (5 µl), H₂O (5 µl), tampon de transcriptase inverse 5X (4 µl), dNTP 5 mM (2 µl), Oligo 20T 5µM (2 µl), RNasin 40 U/ µl (0,5 µl) et RT-AMV 10 UI/ µl (1,5 µl, PROMEGA) a été incubé dans un thermocycleur, dans les conditions suivantes : 45 min à 42°C, 15 min à 55°C, 5 min à 95°C, puis il a été maintenu à +4°C.

L'ADNc obtenu a été amplifié par une première réaction PCR à l'aide des amorces S/N/+/28468-28487 et ancre 14T précitée. Les conditions de l'amplification sont les suivantes : une étape initiale de dénaturation à 94°C pendant 2 min est suivie de 10 cycles comprenant une étape de dénaturation à 94°C pendant 20 sec, une étape d'hybridation à 45°C pendant 30 sec puis une étape d'élongation à 72°C pendant 50 sec puis de 30 cycles comprenant une étape de dénaturation à 94°C pendant 20 sec, une étape d'hybridation à 50°C pendant 30 sec puis une étape d'élongation à 72°C pendant 50 sec, puis d'une étape finale d'élongation à 72°C pendant 5 min.

Le produit de la première amplification PCR a subi une seconde étape d'amplification à l'aide des amorces S/N/+/28933-28952 et ancre 14T précitée, dans des conditions identiques à celles de la première amplification. L'amplicon ainsi obtenu a été purifié, séquencé à l'aide de l'amorce S/N/+/29257-29278 et cloné comme ci-dessus pour les différentes ORF, pour donner le plasmide dénommé SARS-3'NC. L'ADN de ce clone a été isolé et séquencé à l'aide des amorces universelles M13 sens et M13 anti-sens et de l'amorce S/N/+/29257-29278 précitée.

L'amplicon représentant l'ADNc correspondant à l'extrémité 3'NC de la souche de SARS-CoV issue du prélèvement répertorié sous le n° 031589 correspond à la séquence SEQ ID NO :73 dans la liste de séquences jointe en annexe ; cette séquence ne comporte pas de différences par rapport aux séquences correspondantes des isolats AY274119.3-Tor2 et AY278741-Urbani.

Le plasmide dénommé SARS-3'NC a été déposé sous le n° I-3123 le 7 novembre 2003, auprès de la Collection Nationale de Cultures de Microorganismes, 25 rue du Docteur Roux, 75724 Paris Cedex 15. ; il contient la séquence d'ADNc correspondant à l'extrémité 3'non codante du génome de la souche de SARS-CoV issue du prélèvement répertorié sous le n° 031589, telle que définie ci-dessus, laquelle séquence correspondant à celle située entre le nucléotide en position 28933 à 29727 (SEQ ID NO :40), en référence à la séquence Genbank n° d'accès AY274119.3, se termine par une série de nucléotides a.

### 2.6) ORFla et ORF1b

L'amplification de la région 5' contenant les ORF1a et ORF1b du génome du SARS-CoV issu du prélèvement 031589 a été réalisée en pratiquant des réactions de RT-PCR suivies de PCR nichées selon les mêmes principes que ceux précédemment décrits pour les autres ORF. Les fragments amplifiés sont chevauchants sur plusieurs dizaines de bases, permettant ainsi la reconstruction informatique de la séquence complète de cette partie du génome. En moyenne, les fragments amplifiés sont de deux kilobases.

14 fragments chevauchants dénommés L0 à L12 ont ainsi été amplifiés à l'aide des amorces suivantes :

**Tableau II: Amorces utilisées pour l'amplification de la région 5'(ORF1a et ORF1b)**

| **REGION AMPLIFIEE ET SEQUENCEE (ne tient pas compte des amorces)** | **Amorce sens RT-PCR** | **Amorce antisens RT-PCR** | **Amorce sens PCR nichée** | **Amorce antisens PCR nichée** |
|---|---|---|---|---|
| **L0** **50-480** | S/L0/F1/+30 | S/L0/R1/-481 | | |
| **L1** **231-2240** | S/L1/F1/+147 | S/L1/R1/-2336 | S/L1/F2/+211 | S/L1/R2/-2241 |
| **L2** **2156-4167** | S/L2/F1/+2033 | S/L2/R1/-4192 | S/L2/F2/+2136 | S/L2/R2/-4168 |
| **L3** **3913-5324** | S/L3bis/F1/+3850 | S/L3bis/R1/-5365 | S/L3bis/F2/+3892 | S/L3bis/R2/-5325 |
| **L4b** **4952-6023** | S/L4b/F1/+4878 | S/L4b/R1/-6061 | S/L4b/F2/+4932 | S/L4b/R2/-6024 |
| **L4** **5325-7318** | S/L4/F1/+5272 | S/L4/R1/-7392 | S/L4/F2/+5305 | S/L4/R2/-7323 |
| **L5** **7296-9156** | S/L5/F1/+7111 | S/L5/R1/-9253 | S/L5/F2/+7275 | S/L5/R2/-9157 |
| **L6** **9053-11066** | S/L6/F1/+8975 | S/L6/R1/-11151 | S/L6/F2/+9032 | S/L6/R2/-11067 |
| **L7** **10928-12962** | S/L7/F1/+10883 | S/L7/R1/-13050 | S/L7/F2/+10928 | S/L7/R2/-12963 |
| **L8** **12835-14834** | S/L8/F1/+12690 | S/L8/R1/-14857 | S/L8/F2/+12815 | S/L8/R2/-14835 |
| **L9** **14765-16624** | S/L9/F1/+14688 | S/L9/R1/-16678 | S/L9/F2/+14745 | S/L9/R2/-16625 |
| **L10** **16534-18570** | S/L10/F1/+16451 | S/L10/R1/-18594 | S/L10/F2/+16514 | S/L10/R2/-18571 |
| **L11** **18521-20582** | S/L11/F1/+18441 | S/L11/R1/-20612 | S/L11/F2/+18500 | S/L11/R2/-20583 |
| **L12** **20338-22205.** | S/L12/F1/+20279 | S/L12/R1/-22229 | S/L12/F2/+20319 | S/L12/R2/-22206 |

Tous les fragments ont été amplifiés dans les conditions suivantes, excepté le fragment L0 qui a été amplifié comme décrit ci-dessus pour l'ORF-M :
- RT-PCR: 30 min à 42°C, 15 min à 55°C, 2 min à 94°C, puis l'ADNc obtenu est amplifié dans les conditions suivantes : 40 cycles comprenant : une étape de dénaturation à 94°C pendant 15 sec, une étape d'hybridation à 58°C pendant 30 sec puis une étape d'élongation à 68°C pendant 1 min 30 sec, avec 5 sec d'élongation supplémentaire à chaque cycle, puis une étape finale d'élongation à 68°C pendant 7 min.
- PCR nichée : une étape initiale de dénaturation à 94°C pendant 2 min est suivie de 35 cycles comprenant : une étape de dénaturation à 94°C pendant 15 sec, une étape d'hybridation à 60°C pendant 30 sec puis une étape d'élongation à 72°C pendant 1 min 30 sec, avec 5 sec d'élongation supplémentaire à chaque cycle, puis une étape finale d'élongation à 72°C pendant 7 min.

Les produits d'amplifications ont été séquencés à l'aide des amorces définies dans le Tableau III ci-après :

**Tableau III : Amorces utilisées pour le séquençage de la région 5' (ORF1a et ORF1b)**

| Noms | Séquences (SEQ ID NO : 76 à 139) |
|---|---|
| S/L3/+/4932 | 5'-CCACACACAGCTTGTGGATA-3' |
| S/L4/+/6401 | 5'-CCGAAGTTGTAGGCAATGTC-3' |
| S/L4/+/6964 | 5'-TTTGGTGCTCCTTCTTATTG-3' |
| S/L4/-/6817 | 5'-CCGGCATCCAAACATAATTT-3' |
| S/L5/-/7633 | 5'-TGGTCAGTAGGGTTGATTGG-3' |
| S/L5/-/8127 | 5'-CATCCTTTGTGTCAACATCG-3' |
| S/L5/-/8633 | 5'-GTCACGAGTGACACCATCCT-3' |
| S/L5/+/7839 | 5'-ATGCGACGAGTCTGCTTCTA-3' |
| S/L5/+/8785 | 5'-TTCATAGTGCCTGGCTTACC-3' |
| S/L5/+/8255 | 5'-ATCTTGGCGCATGTATTGAC-3' |
| S/L6/-/9422 | 5'-TGCATTAGCAGCAACAACAT-3' |
| S/L6/-/9966 | 5'-TCTGCAGAACAGCAGAAGTG-3' |
| S/L6/-/10542 | 5'-CCTGTGCAGTTTGTCTGTCA-3' |
| S/L6/+/10677 | 5'-CCTTGTGGCAATGAAGTACA-3' |
| S/L6/+/10106 | 5'-ATGTCATTTGCACAGCAGAA-3' |
| S/L6/+/9571 | 5'-CTTCAATGGTTTGCCATGTT-3' |
| S/L7/-/11271 | 5'-TGCGAGCTGTCATGAGAATA-3' |
| S/L7/-/11801 | 5'-AACCGAGAGCAGTACCACAG-3' |
| S/L7/-/12383 | 5'-TTTGGCTGCTGTAGTCAATG-3' |
| S/L7/+/12640 | 5'-CTACGACAGATGTCCTGTGC-3' |
| S/L7/+/12088 | 5'-GAGCAGGCTGTAGCTAATGG-3' |
| S/L7/+111551 | 5'-TTAGGCTATTGTTGCTGCTG-3' |
| S/L8/-13160 | 5'-CAGACAACATGAAGCACCAC-3' |
| S/L8/-/13704 | 5'-CGCTGACGTGATATATGTGG-3' |
| S/L8/-14284 | 5'-TGCACAATGAAGGATACACC-3' |
| S/L8/+/14453 | 5'-ACATAGCTCGCGTCTCAGTT-3' |
| S/L8/+/13968 | 5'-GGCATTGTAGGCGTACTGAC-3' |
| S/L8/+/13401 | 5'-GTTTGCGGTGTAAGTGCAG-3' |
| S/L9/-15098 | 5'-TAGTGGCGGCTATTGACTTC-3' |
| S/L9/-15677 | 5'-CTAAACCTTGAGCCGCATAG-3' |
| S/L9/-16247 | 5'-CATGGTCATAGCAGCACTTG-3' |
| S/L9/+16323 | 5'-CCAGGTTGTGATGTCACTGAT-3' |
| S/L9/+15858 | 5'-CCTTACCCAGATCCATCAAG-3' |
| S/L9/+15288 | 5'-CGCAAACATAACACTTGCTG-3' |
| S/L10/-16914 | 5'-AGTGTTGGGTACAAGCCAGT-3' |
| S/L10/-17466 | 5'-GTTCCAAGGAACATGTCTGG-3' |
| S/L 10/-18022 | 5'-AGGTGCCTGTGTAGGATGAA-3' |
| S/L10/+18245 | 5'-GGGCTGTCATGCAACTAGAG-3' |
| S/L10/+17663 | 5'-TCTTACACGCAATCCTGCTT-3' |
| S/L101+17061 | 5'-TACCCATCTGCTCGCATAGT-3' |
| S/L11/-/18877 | 5'-GCAAGCAGAATTAACCCTCA-3' |
| S/L11/-19396 | 5'-AGCACCACCTAAATTGCATC-3' |
| S/L11/-20002 | 5'-TGGTCCCTTTGAAGGTGTTA-3' |
| S/L11/+20245 | 5'-TCGAACACATCGTTTATGGA-3' |
| S/L11/+/19611 | 5'-GAAGCACCTGTTTCCATCAT-3' |
| S/L11/+/19021 | 5'-ACGATGCTCAGCCATGTAGT-3' |
| SARS/L1/F3/+800 | 5'-GAGGTGCAGTCACTCGCTAT-3' |
| SARS/L1/F4/+1391 | 5'-CAGAGATTGGACCTGAGCAT-3' |
| SARS/L1/F5/+1925 | 5'-CAGCAAACCACTCAATTCCT-3' |
| SARS/L1/R3/-1674 | 5'-AAATGATGGCAACCTCTTCA-3' |
| SARS/L1/R4/-1107 | 5'-CACGTGGTTGAATGACTTTG-3' |
| SARS/L1/R5/-520 | 5'-ATTTCTGCAACCAGCTCAAC-3' |
| SARS/L2/F3/+2664 | 5'-CGCATTGTCTCCTGGTTTAC-3' |
| SARS/L2/F4/+3232 | 5'-GAGATTGAGCCAGAACCAGA-3' |
| SARS/L2/F5/+3746 | 5'-ATGAGCAGGTTGTCATGGAT-3' |
| SARS/L2/R3/-3579 | 5'-CTGCCTTAAGAAGCTGGATG-3' |
| SARS/L2/R4/-2991 | 5'-TTTCTTCACCAGCATCATCA-3' |
| SARS/L2/R5/-2529 | 5'-CACCGTTCTTGAGAACAACC-3' |
| SARS/L3/F3/+4708 | 5'-TCTTTGGCTGGCTCTTACAG-3' |
| SARS/L3/F4/+5305 | 5'-GCTGGTGATGCTGCTAACTT-3' |
| SARS/L3/F5/+5822 | 5'-CCATCAAGCCTGTGTCGTAT-3' |
| SARS/L3/R3/-5610 | 5'-CAGGTGGTGCAGACATCATA-3' |
| SARS/L3/R4/-4988 | 5'-AACATCAGCACCATCCAAGT-3' |
| SARS/L3/R5/-4437 | 5'-ATCGGACACCATAGTCAACG-3' |

Les séquences des fragments L0 à L12 de la souche de SARS-CoV issue du prélèvement répertorié sous le n° 031589, correspondent respectivement aux séquences SEQ ID NO :41 à SEQ ID NO :54 dans la liste de séquences jointe en annexe. Parmi ces séquences, seule celle correspondant aux fragments L5 comporte une différence nucléotidique par rapport à la séquence correspondante de l'isolat AY278741-Urbani. Cette mutation t/c en position 7919 aboutit à une modification de la séquence en acides aminés de la protéine correspondante, codée par l'ORF 1a: en position 2552, une valine (codon gtt ; AY278741) est changée en alanine (codon gct) dans la souche de SARS-CoV 031589. En revanche, aucune mutation n'a été identifiée par rapport à la séquence correspondante de l'isolat AY274119.3-Urbani. Les autres fragments ne présentent pas de différences par rapport aux séquences correspondantes des isolats Tor2 et Urbani.

### Exemple 2 : Production et purification de protéines N et S recombinantes de la souche de SARS-CoV issue du prélèvement répertorié sous le numéro 031589

La protéine entière N et deux fragments polypeptidiques de la protéine S de la souche de SARS-CoV issue du prélèvement répertorié sous le numéro 031589 ont été produites chez *E. coli*, sous forme de protéines de fusion comprenant une étiquette polyhistidine N-ou C-terminale. Dans les deux polypeptides S, les séquences hydrophobes N et C-terminales de la protéine S (peptide signal : positions 1 à 13 et hélice transmembranaire : positions 1196 à 1218) ont été délétées alors que l'hélice β (positions 565 à 687) et les deux motifs de type coiled-coils (positions 895 à 980 et 1155 à 1186) de la protéine S ont été préservés. Ces deux polypeptides sont constitués par : un fragment long (S_{L}) correspondant aux positions 14 à 1193 de la séquence en acides aminés de la protéine S et un fragment court (S_{C}) correspondant aux positions 475 à 1193 de la séquence en acides aminés de la protéine S.

### 1) Clonage des ADNc N, S_{L} et S_{C} dans les vecteurs d'expression pIVEX2.3 et pIVEX2.4

Les ADNc correspondant à la protéine N et aux fragments S_{L} et S_{C} ont été amplifiés par PCR dans des conditions standard, à l'aide de l'ADN polymérase Platinium Pfx® (INVITROGEN). Les plasmides SRAS-N et SRAS-S ont été utilisés comme matrice et les oligonucléotides suivants comme amorces :
5'-CCCATATGTCTGATAATGGACCCCAATCAAAC-3' (N sens, SEQ ID NO :55)
5'-CCCCCGGGTGCCTGAGTTGAATCAGCAGAAGC-3' (N antisens, SEQ ID NO :56)
5'-CCCATATGAGTGACCTTGACCGGTGCACCAC-3' (S_{C} sens, SEQ ID NO :57)
5'-CCCATATGAAACCTTGCACCCCACCTGCTC-3' (S_{L} sens, SEQ ID NO :58)
5'-CCCCCGGGTTTAATATATTGCTCATATTTTCCC-3' (S_{C} et S_{L} antisens, SEQ ID NO :59).

Les amorces sens introduisent un site *NdeI* (souligné) alors que les amorces antisens introduisent un site *XmaI* ou *SmaI* (souligné). Les 3 produits d'amplification on été purifiés sur colonne (kit *QIAquick PCR Purification,* QIAGEN) et clonés dans un vecteur approprié. L'ADN plasmidique purifié des 3 constructions (kit *QIAFilter Midi Plasmid,* QIAGEN) a été vérifié par séquençage et digéré par les enzymes *NdeI* et *XmaI.* Les 3 fragments correspondants aux ADNc N, S_{L} et S_{C} ont été purifiés sur gel d'agarose puis insérés dans les plasmides pIVEX2.3MCS (étiquette polyhistidine C-terminale) et pIVEX2.4d (étiquette polyhistidine N-terminale) préalablement digérés par les mêmes enzymes. Après vérification des constructions, les 6 vecteurs d'expressions ainsi obtenus (pIV2.3N, pIV2.3S_{C}, pIV2.3S_{L}, pIV2.4N, pIV2.4S_{C} également dénommé pIV2.4S_{I}, pIV2.4S_{L}) ont été ensuite utilisés, d'une part pour tester l'expression des protéines *in-vitro,* et d'autre part pour transformer la souche bactérienne BL21(DE3)pDIA17 (NOVAGEN). Ces constructions codent pour des protéines dont la masse moléculaire attendue est la suivante : pIV2.3N (47174 Da), pIV2.3S_{C} (82897 Da), pIV2.3S_{L} (132056 Da), pIV2.4N (48996 Da), pIV2.4S_{I} (81076 Da) et pIV2.4S_{L}(133877 Da). Des bactéries transformées par pIV2.3N ont été déposées à la CNCM le 23 octobre 2003, sous le numéro I-3117, et des bactéries transformées par pIV2.4S₁ ont été déposées à la CNCM le 23 octobre 2003, sous le numéro I-3118.

### 2) Analyse de l'expression des protéines recombinantes in-vitro et in vivo

L'expression de protéines recombinantes à partir des 6 vecteurs recombinants a été testée, dans un premier temps, dans un système *in-vitro* (RTS100, Roche). Les protéines produites *in vitro,* après une incubation des vecteurs recombinants pIVEX, 4h à 30°C, dans le système RTS100, ont été analysées par western-blot à l'aide d'un anticorps anti-(his)₆ couplé à la péroxydase. Le résultat d'expression *in-vitro* (Figure 1) montre que seule la protéine N est exprimée en quantités importantes, cela quelle que soit la position, N- ou C-terminale, de l'étiquette polyhistidine. Dans une seconde étape, l'expression des protéines N et S a été testée *in-vivo* à 30°C dans du milieu LB, en présence ou en l'absence d'inducteur (IPTG 1mM). La protéine N est très bien produite dans ce système bactérien (Figure 2) et se retrouve principalement dans une fraction soluble après lyse des bactéries. En revanche, la version longue de S (S_{L}) est très peu produite et complètement insoluble (Figure 3). La version courte (S_{C}) présente également une très faible solubilité, mais un taux d'expression beaucoup plus élevé que celui de la version longue. Par ailleurs, la construction S_{C} fusionnée à une étiquette polyhistidine en position C-terminale présente une taille plus faible que celle attendue. Une expérience d'immunodétection avec un anticorps anti-polyhistidine a montré que cette construction était incomplète. En conclusion, les deux constructions, pIV2.3N et pIV2.4S_{I}, exprimant respectivement la protéine N entière fusionnée à l'étiquette polyhistidine en C-terminal et la protéine S courte fusionnée à l'étiquette polyhistidine en N-terminal, ont été retenues pour produire les deux protéines en grande quantité afin de les purifier. Les plasmides pIV2.3N et pIV2.4S_{I} ont été déposés respectivement sous le n° I-3117 et I-3118 auprès de la CNCM, 25 rue du Docteur Roux, 75724 PARIS 15, le 23 octobre 2003.

### 3) Analyse de l'activité antigénique des protéines recombinantes

L'activité antigénique des protéines N, S_{L} et S_{C} a été testée par western-blot, à l'aide de deux échantillons de sérum, provenant d'un même patient infecté par le SARS-CoV, prélevés 8 jours (M12) et 29 jours-(M13) après le début des symptômes du SRAS. Le protocole expérimental est comme décrit à l'exemple 3. Les résultats illustrés par la figure 4 montrent (i) la séroconversion du patient, et (ii) que la protéine N possède une plus forte réactivité antigénique que la protéine S courte.

### 4) Purification de la protéine N à partir de pIV2.3N

Plusieurs expériences de purification de la protéine N, produite à partir du vecteur pIV2.3N, ont été réalisées selon le protocole suivant. Les bactéries BL21(DE3)pDIA17, transformées par le vecteur d'expression pIV2.3N, ont été cultivées à 30°C dans 1 litre de milieu de culture contenant 0,1 mg/ml d'ampicilline, et induites par 1 mM IPTG quand la densité cellulaire, équivalente à A₆₀₀ = 0,8, est atteinte (environ 3 heures). Après 2 heures de culture en présence d'inducteur, les cellules ont été récupérées par centrifugation (10 min à 5000 rpm), remises en suspension dans le tampon de lyse (50 mM NaH₂PO₄, NaCl 0,3 M, 20 mM imidazole, pH 8 contenant le mélange d'inhibiteurs de protéases *Complete*®, Roche), et lysées par la presse de French (12000 psi). Après centrifugation du lysat bactérien (15 min à 12000 rpm), le surnageant (50 ml) a été déposé à un débit de 1ml/min sur une colonne (15 ml) de chélation métallique (Ni-NTA superflow, Qiagen), équilibrée par le tampon de lyse. Après lavage de la colonne par 200 ml de tampon de lyse, la protéine N a été éluée par un gradient d'imidazole (20 →250 mM) en 10 volumes de colonne. Les fractions contenant la protéine N ont été rassemblées et analysées par électrophorèse en gel de polyacrylamide en conditions dénaturantes puis coloration au bleu de Coomassie. Les résultats illustrés par la figure 5 montrent que le protocole employé permet de purifier la protéine N avec une homogénéité très satisfaisante (95%) et un rendement moyen de 15 mg de protéine par litre de culture.

### 5) Purification de la protéine S_{C} à partir de pIV2.4S_{C} (pIV2.4S₁)

Le protocole suivi pour purifier la protéine S courte est très différent de celui décrit ci-dessus car la protéine est fortement agrégée dans le système bactérien (corps d'inclusion). Les bactéries BL21(DE3)pDIA17, transformées par le vecteur d'expression pIV2.4S₁ ont été cultivées à 30°C dans 1 litre de milieu de culture contenant 0,1 mg/ml d'ampicilline, et induites par 1 mM IPTG quand la densité cellulaire, équivalente à A₆₀₀ = 0,8, est atteinte (environ 3 heures). Après 2 heures de culture en présence d'inducteur, les cellules ont été récupérées par centrifugation (10 min à 5000 rpm), remises en suspension dans le tampon de lyse (0,1 M Tris-HCl, EDTA 1 mM, pH 7,5), et lysées par la presse de French (1200 psi). Après centrifugation du lysat bactérien (15 min à 12000 rpm), le culot a été remis en suspension dans 25 ml de tampon de lyse contenant 2% Triton X100 et 10 mM β-mercaptoéthanol, puis centrifugé pendant 20 min à 12000 rpm. Le culot a été remis en suspension dans un tampon Tris-HCl 10 mM contenant 7 M urée, et mis en agitation douce pendant 30 min à température ambiante. Ce dernier lavage des corps d'inclusion avec 7 M urée est nécessaire pour éliminer la plupart des protéines membranaires d'*E*. *coli* qui co-sédimentent avec la protéine S_{C} agrégée. Après une dernière centrifugation pendant 20 min à 12000 rpm, le culot final est remis en suspension dans le tampon Tris-HCl 10 mM. L'analyse électrophorétique de cette préparation (Figure 6) montre que la protéine S courte peut être purifiée avec une homogénéité satisfaisante (environ 90%) à partir des corps d'inclusion (extrait insoluble).

### Exemple 3 : Immunodominance de la protéine N

La réactivité des anticorps présents dans le sérum des patients atteints de pneumopathie atypique causée par le coronavirus associé au SRAS (SARS-CoV), vis-à-vis des différentes protéines de ce virus, a été analysée par westem-blot dans les conditions décrites ci-après.

### 1) Matériel

### a) lysat de cellules infectées par le SARS-CoV

Des cellules Vero E6 (2x10⁶) ont été infectées par le SARS-CoV (isolat répertorié sous le numéro FFM/MA104) à une multiplicité d'infection (M.O.I.) de 10⁻¹ ou 10⁻² puis incubées dans du milieu DMEM contenant 2% de SVF, à 35°C dans une atmosphère contenant 5% de CO₂. 48 heures plus tard, le tapis cellulaire a été lavé avec du PBS puis lysé avec 500 µl de tampon de dépôt préparé selon Laemmli et contenant du ß-mercaptoéthanol. Les échantillons ont ensuite été bouillis 10 minutes puis soniqués 3 fois 20 secondes.

### b) anticorps

### b₁) sérum de patient atteint de pneumopathie atypique

Le sérum référencé au Centre National de Référence des virus influenzae (Région-Nord) sous le N° 20033168 est celui d'un patient français atteint d'une pneumopathie atypique causée par le SARS-CoV prélevé au jour 38 après le début des symptômes ; le diagnostic d'infection par le SARS-CoV a été réalisé par RT-PCR nichée et PCR quantitative.

### b₂) sérums polyclonaux de lapin monospécifiques dirigés, contre la protéine N ou la protéine S

Les sérums sont ceux produits à partir des protéines recombinantes N et S_{C} (exemple 2), selon le protocole d'immunisation décrit à l'exemple 4 ; il s'agit du sérum du lapin P13097 (sérum anti-N) et du sérum du lapin P11135 (sérum anti-S).

### 2) Méthode

20 µl de lysat de cellules infectées par le SARS-CoV à des M.O.I. de 10⁻¹ et 10⁻² et, à titre de contrôle, 20 µl d'un lysat de cellules non infectées (mock) ont été séparés sur un gel SDS à 10% de polyacrylamide puis transférés sur une membrane de nitrocellulose. Après blocage dans une solution de PBS/lait 5%/Tween 0,1 % et lavage en PBS/Tween 0,1 %, cette membrane a été hybridée pendant une nuit à 4°C avec : (i) l'immun-sérum N° 20033168 dilué au 1/300, 1/1000 et 1/3000 dans le tampon PBS/BSA 1%/Tween 0,1%, (ii) le sérum du lapin P13097 (sérum anti-N) dilué au 1/50000 dans le même tampon et (iii) le sérum du lapin P11135 (sérum anti-S) dilué au 1/10000 dans le même tampon. Après lavage en PBS/Tween, une hybridation secondaire a été réalisée à l'aide, soit d'anticorps polyclonaux de mouton dirigés contre les chaînes lourdes et légères des immunoglobulines G humaines et couplés à la peroxidase (NA933V, Amersham), soit d'anticorps polyclonaux d'âne dirigés contre les chaînes lourdes et légères des immunoglobulines G de lapin et couplés à la peroxidase (NA934V, Amersham). Les anticorps fixés ont été révélés à l'aide du kit ECL+ (Amersham) et de films d'autoradiographie Hyperfilm MP (Amersham). Une échelle de masse moléculaire (kDa) est portée sur la figure.

### 3) Résultats

La figure 7 montre que trois polypeptides de masse moléculaire apparente 35, 55 et 200 kDa sont détectés spécifiquement dans les extraits de cellules infectées par le SARS-CoV.

Afin d'identifier ces polypeptides, deux autres immunoempreintes (figure 8) ont été réalisées sur les mêmes échantillons et dans les mêmes conditions avec des anticorps polyclonaux de lapins spécifique de la nucléoprotéine N (lapin P13097, figure 8A) et de la protéine de spicule S (lapin P11135, figure 8B) Cette expérience montre que le polypeptide de 200 kDa correspond à la glycoprotéine de spicule S du SARS-CoV, que le polypeptide de 55 kDa correspond à la nucléoprotéine N tandis que le polypeptide de 35 kDa représente vraisemblablement une forme tronquée ou dégradée de la N.

Les données présentées dans la figure 7 montrent donc que le sérum 20033168 réagit fortement avec la N et beaucoup plus faiblement avec la S du SARS-CoV, puisque les polypeptides de 35 et 55 kDa sont révélés sous la forme de bandes intenses pour des dilutions de 1/300, 1/1000 et 1/3000 de l'immunsérum alors que le polypeptide de 200 kDa n'est que faiblement révélé pour une dilution de 1/300. On peut noter également qu'aucun autre polypeptide du SARS-CoV n'est détecté pour des dilutions supérieures au 1/300 du sérum 20033168.

Cette expérience indique que la réponse en anticorps spécifique de la N du SARS-CoV domine les réponses en anticorps spécifiques des autres polypeptides du SARS-CoV et en particulier la réponse en anticorps dirigée contre la glycoprotéine S. Elle indique une immunodominance de la nucléoprotéine N lors des infections humaines par le SARS-CoV.

### Exemple 4 : Préparation d'anticorps polyclonaux monospécifiques dirigés contre les protéines N et S du coronavirus associé au SRAS (SARS-CoV)

### 1) Matériel et méthode

Trois lapins (P13097, P13081, P13031) ont été immunisés avec le polypeptide recombinant purifié correspondant à l'intégralité de la nucléoprotéine (N), préparé selon le protocole décrit à l'exemple 2. Après une première injection de 0,35 mg par lapin de protéine émulsionnée en adjuvant complet de Freund (voie intradermique), les animaux ont reçus 3 injections de rappel à 3 puis 4 semaines d'intervalle, de 0,35 mg de protéine recombinante émulsionnée en adjuvant incomplet de Freund.

Trois lapins (P11135, P13042, P14001) ont été immunisés avec le polypeptide recombinant correspondant au fragment court de la protéine S (S_{C}), produit comme décrit à l'exemple 2. Comme ce polypeptide est retrouvé principalement sous la forme de corps d'inclusion dans le cytoplasme bactérien, les animaux ont reçus 4 injections intra-dermiques à 3-4 semaines d'intervalle d'une préparation de corps d'inclusion correspondant à 0,5 mg de protéine recombinante émulsionnée en adjuvant incomplet de Freund. Les 3 premières injections ont été réalisées avec une préparation de corps d'inclusion préparés selon le protocole décrit à l'exemple 2, tandis que la quatrième injection a été réalisée avec une préparation de corps d'inclusion qui ont été préparés selon le protocole décrit à l'exemple 2 puis purifiés sur gradient de saccharose et lavés en 2 % Triton X100.

Pour chaque lapin, un sérum pré-immun (p.i.) a été préparé avant la première immunisation et un immun-sérum (I.S.) 5 semaines après la quatrième immunisation.

Dans un premier temps, la réactivité des sérums a été analysée par test ELISA vis à vis de préparations de protéines recombinantes semblables à celles utilisées pour les immunisations ; les tests ELISA ont été réalisés selon le protocole et avec les réactifs tels que décrits à l'exemple 6.

Dans un deuxième temps, la réactivité des sérums a été analysée en réalisant une immunoempreinte (western blot) d'un lysat de cellules infectées par le SARS-CoV, en suivant le protocole tel que décrit à l'exemple 3.

### 2) Résultats

Les tests ELISA (figure 9) démontrent que les préparations de protéine N recombinante et de corps d'inclusion du fragment court de la protéine S (S_{C}) sont immunogènes chez l'animal et que le titre des sérums immuns est élevé (plus de 1/25000).

L'immunoempreinte (figure 8) montre que le sérum immun du lapin P13097 reconnaît deux polypeptides présents dans les lysats de cellules infectées par le SARS-CoV : un polypeptide dont la masse moléculaire apparente (50-55 kDa selon les expériences) est compatible avec celle de la nucléoprotéine N (422 résidus, masse moléculaire prédite de 46 kDa) et un polypeptide de 35 kDa, qui représente vraisemblablement une forme tronquée ou dégradée de la N.

Cette expérience montre également que le sérum du lapin P11135 reconnaît principalement un polypeptide dont la masse moléculaire apparente (180-220 kDa selon les expériences) est compatible avec une forme glycosylée de la S (1255 résidus, chaîne polypeptidique non glycosylée de 139 kDa), ainsi que des polypeptides plus légers, qui représentent vraisemblablement des formes tronquées et/ou non glycosylées de la S.

En conclusion, l'ensemble de ces expériences démontrent que des polypeptides recombinants exprimés chez *E. coli* et correspondant aux protéines N et S du SARS-CoV permettent d'induire chez l'animal des anticorps polyclonaux capables de reconnaître les formes natives de ces protéines.

### Exemple 5 : Préparation d'anticorps polyclonaux monospécifiques dirigés contre les protéines M et E du coronavirus associé au SRAS (SARS-CoV)

### 1) Analyse de la structure des protéines M et E

### a) Protéine E

La structure de la protéine E du SARS-CoV (76 acides aminés) a été analysée *in silico*, à l'aide de différents logiciels comme signalP v1.1, NetNGlyc 1.0, THMM 1.0 et 2.0 (Krogh et al., 2001, J. Mol. Biol., 305(3):567-580) ou encore TOPPRED (von Heijne, 1992, J. Mol. Biol. 225, 487-494). L'analyse montre que ce polypeptide non glycosylé est une protéine membranaire de type 1, contenant une seule hélice transmembranaire (aa 12-34 d'après THMM), et dont la plus grande partie du domaine hydrophile (42 résidus) est localisée à l'extrémité C-terminale et vraisemblablement à l'intérieur de la particule virale (endodomaine). On peut noter une inversion dans la topologie prédite par les versions 1.0 (N-ter est externe) et 2.0 (N-ter est interne) du logiciel THMM, mais que d'autres algorithmes, notamment TOPPRED et THUMBUP (Zhou et Zhou, 2003, Protein Science 12 :1547-1555) confirment une localisation externe de l'extrémité N-terminale de E.

### b) Protéine M

Une analyse similaire réalisée sur la protéine M du SARS-CoV (221 acides aminés) montre que ce polypeptide ne possède pas de peptide signal (d'après le logiciel signalP v1.1) mais trois domaines transmembranaires (résidus 15-37, 50-72, 77-99 d'après THMM2.0) et un grand domaine hydrophile (aa 100-221) localisé à l'intérieur de la particule virale (endodomaine). Elle est vraisemblablement glycosylée sur l'asparagine en position 4 (d'après NetNGlyc 1.0).

Ainsi, en accord avec les données expérimentales connues pour les autres coronavirus, il est remarquable que les deux protéines M et E présentent des endodomaines correspondant à la majeure partie des polypeptides et des ectodomaines de très petite taille.
- l'ectodomaine de E correspond vraisemblablement aux résidus 1 à 11 ou 1 à 12 de la protéine : MYSFVSEETGT(L), SEQ ID NO : 70. En effet, la probabilité associée à la localisation transmembranaire du résidu 12 est intermédiaire (0,56 d'après THMM 2.0).
- l'ectodomaine de M correspond vraisemblablement aux résidus 2 à 14 de la protéine : ADNGTITVEELKQ, SEQ ID NO : 69. En effet, la méthionine N-terminale de M est très probablement clivée du polypeptide mature car le résidu en position 2 est une Alanine (Varshavsky, 1996, 93:12142-12149).

Par ailleurs, l'analyse de l'hydrophobicité (Kyte & Doolittle, Hopp & Woods) de la protéine E met en évidence que l'extrémité C-terminale de l'endodomaine de E est hydrophile et donc vraisemblablement exposée à la surface de ce domaine. Ainsi, un peptide synthétique correspondant à cette extrémité est un bon candidat immunogène pour induire chez l'animal des anticorps dirigés contre l'endodomaine de E. En conséquence, un peptide correspondant aux 24 résidus C-terminaux de E a été synthétisé.

### 2) Préparation d'anticorps dirigés contre l'ectodomaine des protéines M et E et l'endodomaine de la protéine E

Les peptides M2-14 (ADNGTITVEELKQ, SEQ ID NO : 69), E1-12 (MYSFVSEETGTL, SEQ ID NO: 70) et E53-76 (KPTVYVYSRV KNLNSSEGVP DLLV, SEQ ID NO : 71) ont été synthétisés par Neosystem. Ils ont été couplés à la KLH (*Keylzole Limpet Hemocyanin*) à l'aide du MBS (m-maleimido-benzoyl-N-hydroxysuccinimide ester) via une cystéine ajoutée au cours de la synthèse soit en N-terminal du peptide (cas de E53-76) soit en C-terminal (cas de M2-14 et E1-12).

Deux lapins ont été immunisés avec chacun des conjugués, en suivant le protocole d'immunisation suivant : après une première injection de 0,5 mg de peptide couplé à la KLH et émulsionné en adjuvant complet de Freund (voie intradermique), les animaux reçoivent 2 à 4 injections de rappel à 3 ou 4 semaines d'intervalle de 0,25 mg de peptide couplé à la KLH et émulsionné en adjuvant incomplet de Freund.

Pour chaque lapin, un sérum pré-immun (p.i.) a été préparé avant la première immunisation et un immun-sérum (I.S.) est préparé 3 à 5 semaines après les injections de rappel.

La réactivité des sérums a été analysée par western blot à l'aide d'extraits de cellules infectées par le SRAS-CoV (figure 43B) ou à l'aide d'extraits de cellules infectées par un virus recombinant de la vaccine exprimant la protéine E (VV-TG-E, figure 43A) ou M (VV-TN-M, figure 43C) de l'isolat 031589 du SRAS-CoV.

Les immunsérums des lapins 22234 et 22240, immunisés par le conjugué KLH-E53-76, reconnaissent un polypeptide d'environ 9 à 10kD, qui est présent dans les extraits de cellules infectées par le SRAS-CoV mais absent dans les extraits de cellules non infectées (figure 43B). La masse apparente de ce polypeptide est compatible avec la masse prédite de la protéine E, qui est de 8,4 kD. De façon similaire, l'immunsérum du lapin 20047, immunisé par le conjugué KLH-E1-12, reconnaît un polypeptide présent dans les extraits de cellules infectées par le virus VV-TG-E, dont la masse molaire apparente est compatible avec celle de la protéine E (figure 43A).

L'immunsérum des lapin 20013 et 20080, immunisés par le conjugué KLH-M2-14, reconnaît un polypeptide présent dans les extraits de cellules infectées par le virus VV-TN-M (figure 43C), dont la masse molaire apparente (18 kD environ) est compatible avec celle de la glycoprotéine M, qui est de 25,1 kD et présente un point isoélectrique élevé (9.1 pour le polypeptide nu).

Ces résultats démontrent que les peptides E1-12 et E53-76 d'une part et le peptide M2-14 d'autre part permettent d'induire chez l'animal des anticorps polyclonaux capables de reconnaître les formes natives des protéines E et M respectivement du SRAS-CoV.

### Exemple 6 : Analyse de la réactivité en ELISA de la protéine N recombinante, vis-à-vis de sérums de patients atteints de SRAS

### 1) Matériel

L'antigène utilisé pour préparer les phases solides est la nucléoprotéine N recombinante purifiée préparée selon le protocole décrit à l'exemple 2.

Les sérums à tester (Tableau IV) ont été choisis sur la base des résultats d'analyse de leur réactivité par immunofluorescence (titre IF-SRAS), vis-à-vis de cellules infectées par le SARS-CoV.

**Tableau IV: Sérums testés en ELISA**

| **Référence** | **N° sérum** | **Type de sérum** | **Date du Sérum***** | **Titre IF-SRAS** |
|---|---|---|---|---|
| 3050 | A | Témoin | na* | nt** |
| 3048 | B | Témoin | na | nt |
| 033168 | D | Patient 1- SRAS | 27/04/03 (J38) | 320 |
| 033397 | E | Patient-1 SRAS | 11/05/03 (J52) | 320 |
| 032632 | F | Patient-2 SRAS | 21/03/03 (J17) | 2500 |
| 032791 | G | Patient-3 SRAS | 04/04/03 (J3) | <40 |
| 033258 | H | Patient-3 SRAS | 28/04/03 (J27) | 160 |

| | | | | |
|---|---|---|---|---|
| *na : non-applicable. ** nt : non-testé. *** les dates indiquées correspondent au nombre de jours après le début des symptômes de SRAS. | | | | |

### 2) Méthode

La protéine N (100 µl) diluée à différentes concentrations dans du tampon carbonate 0,1 M, pH 9,6 (1, 2 ou 4 µg/ml) est distribuée dans les puits de plaques ELISA, puis les plaques sont incubées une nuit à température du laboratoire. Les plaques sont lavées avec du tampon PBS-Tween, saturées avec du tampon PBS-lait écrémé-saccharose (5 %). Les sérums à tester (100 µl) préalablement dilués (1/50, 1/100, 1/200, 1/400, 1/800, 1/1600 et 1/3200) sont ajoutés, puis les plaques sont incubées 1 h à 37° C. Après 3 lavages, le conjugué anti-IgG humaines marqué à la peroxydase (référence 209-035-098, JACKSON) dilué au 1/18000 est ajouté puis les plaques sont incubées 1h à 37 °C. Après 4 lavages, le chromogène (TMB) et le substrat (H₂0₂) sont ajoutés et les plaques sont incubées 30min à température ambiante, à l'abri de la lumière. La réaction est ensuite arrêtée puis l'absorbance à 450 nm est mesurée à l'aide d'un lecteur automatique.

### 3) Résultats

Les tests ELISA (figure 10) démontrent que la préparation de protéine N recombinante est reconnue spécifiquement par les anticorps de sérums de patients atteints de SRAS prélevés en phase tardive de l'infection (≥ 17 jours après le début des symptômes) alors qu'elle n'est pas reconnue de façon significative par les anticorps d'un sérum de patient prélevé en phase précoce de l'infection (3 jours après le début des symptômes) ni par des sérums témoins de sujets non atteints de STRAS.

### Exemple 7 : Tests ELISA élaborés pour une détection très spécifique et sensible d'une infection par le coronavirus associé au SRAS, à partir de sérums de patients

### 1) Test ELISA IgG indirect

### a) Réactifs

### Préparation des plaques

Les plaques sont sensibilisées par une solution de protéine N à 2µg/mL dans un tampon PBS 10mM pH 7,2, rouge de phénol à 0,25mL /L. 100 µL de solution sont déposés dans les puits et laissés incubés à température ambiante pendant une nuit. La saturation se fait par un prélavage en tampon PBS 10mM / tween 0,1%, suivi d'un lavage avec une solution de saturation PBS, 25% lait /saccharose.

### Diluant serums

Tampon TRIS 0,48g/L, PBS 10mM, EDTA 3,7g/L,lait 15% v/v , pH 6,7

### Diluant conjugué

Tampon citrate (15g/L), tween 0,5% , serum bovin 25%, NaCl 12%, lait écrémé 6% v/v PH 6,5

### Conjugué

Conjugué anti-IgG humaines 50X, commercialisés par Bio-Rad : kit Platelia H. pylori ref 72778

### Autres solutions:

Solution de lavage R2, Solutions de révélation au TMB R8 diluant, R9 chromogène, R10 solution d'arrêt : réactifs commercialisés commercialisées par Bio-Rad (ex : kit *Platelia pylori,* ref 72778)

### b) Mode opératoire

Diluer les sérums au **1/200** dans le diluant échantillons
Distribuer 100µL/puits
Incubation 1h à 37°C
3 lavages en solution de LAVAGE R2 10x préalablement diluée 10 fois en eau déminéralisée (*i.e.,* solution de lavage 1X)
Distribuer 100µL de conjugué (conjugué 50x à diluer extemporanément dans le diluant conjugué fourni)
Incubation 1h à 37°C
4 lavages en solution de lavage 1X
distribuer 200µL/puits de solution de révélation (à diluer extemporanément ex:1 mL de R9 dans 10mL de R8)
Incubation 30 min à température ambiante à l'obscurité
arrêter la réaction avec 100µL/puits de R10
LECTURE à 450/620nm

Les résultats peuvent être interprétés en prenant un serum SEUIL donnant une réponse au delà de laquelle les serums testés seront considérés comme positifs. Ce sérum est choisi et dilué de façon à donner un signal significativement supérieur au bruit de fond.

### 2) Test ELISA DOUBLE EPITOPE

### a) Réactifs

### Préparation des plaques

Les plaques sont sensibilisées par une solution de protéine N à 1µg/mL dans un tampon PBS 10mM pH 7,2, rouge de phénol à 0,25mL /L. 100 µL de solution sont déposés dans les puits et laissés incubés à température ambiante pendant une nuit. La saturation se fait par un prélavage en tampon PBS 10mM / 0,1% tween suivi d'un lavage avec une solution de saturation PBS 10mM, lait 25% (V/V)

### Diluant serums et conjugué

Tampon TRIS saline 50mM pH8, lait 2%

### Conjugué

Il s'agit de la protéine N recombinante purifiée, couplée à la peroxidase selon le protocole de Nakane (Nakane P.K. and Kawaoi A; (1974) : Peroxydase-labeled antibody, a new method of conjugation. The Journal of Histochemistry and Cytochemistry Vol22, N)23, pp 1084-1091.), dans des ratios molaires respectifs 1/2. Ce conjugué ProtN POD est utilisé à une concentration de 2µg/mL dans du diluant serum/conjugué.

### Autres solutions :

Solution de lavage R2, Solutions de révélation au TMB R8, diluant, R9 chromogène, R10 solution d'arrêt : réactifs commercialisées par Bio-Rad (ex kit platelia pylori ref 72778).

### b) Mode opératoire

### 1ere étape en plaque de "prédilution"

■ Diluer chaque sérum au 1/5 dans la plaque de prédilution (48 µL de diluant +12 µL de sérum).
■ Après avoir dilués l'ensemble des serums, distribuer 60µL de conjugué
■ Le cas échéant, le mélange sérum + conjugué est laissé à incuber.

### 2eme étape en plaque de "réaction"

■ Transférer 100µLde mélange/puits dans la plaque de réaction
■ Incubation 1h 37°C
■ 5 lavages en solution de LAVAGE R2 10x préalablement diluée 10 fois en eau déminéralisée (--> solution de lavage 1x)
■ distribuer 200µL/puits de solution de révélation (à diluer extemporanément ex:1 mL de R9 dans 10mL de R8)
■ incubation 30min à température ambiante à l'abri de la lumière
■ arrêter la réaction avec 100µL/puits de R10
■ LECTURE à 450/620nm

De même que pour le test ELISA indirect, les résultats peuvent être interprétés en utilisant un serum "valeur seuil". Tout serum ayant une réponse supérieure au sérum valeur seuil sera considéré comme positif.

### 2) Résultats

Les sérums de patients classés comme cas probables de SRAS de l'hôpital français de Hanoi, Vietnam ou en relation avec l'hôpital français de Hanoi (JYK) ont été analysés en utilisant le test IgG-N indirect et le test N double épitope.

Les résultats du test IgG-N indirect (figures 14 et 15) et N double épitope (figures 16 et 17) montrent une excellente corrélation entre eux ainsi qu'avec un test ELISA indirect comparant la réactivité des sérums vis-à-vis d'un lysat de cellules VeroE6 non infectées ou infectées par le SRAS-CoV (ELISA-lysat SRAS-CoV ; voir le Tableau V ci-après). Tous les sérums prélevés 12 jours ou plus après le début des symptômes ont été trouvés positifs, y compris chez des patients pour lesquels l'infection par le virus du SRAS-CoV n'avait pas pu être documentée par analyse de prélèvements respiratoires par RT-PCR, vraisemblablement en raison d'un prélèvement trop tardif au cours de l'infection (≥ J12). Dans le cas du patient TTH pour lequel un prélèvement nasal réalisé à J7 a été trouvé négatif par RT-PCR, la qualité du prélèvement pourrait être en cause.

Certains sérums ont été trouvés négatifs alors que la présence de SRAS-CoV a été détectée par RT-PCR. Il s'agit dans tous les cas de sérums précoces prélevés moins de 10 jours après le début des symptômes (ex : sérum # 032637). Dans le cas d'un patient PTTH (sérum # 032673), seule une suspicion de SRAS était évoquée au moment où les prélèvements ont été réalisés.

En conclusion, les tests sérologiques IgG-N indirect et N-double épitope permettent de documenter l'infection par le SRAS-CoV chez tous les patients pour des sérums prélevés 12 jours ou plus après l'infection.

**Tableau V : Résultats des tests ELISA**

| **Num Pvt** | **Patient** | **Jour** | **PCR-SARS (1)** | **ELISA lysat SRAS-CoV (2)** | **IgG-N (2^{ème} série)** | **2Xepitope (2^{ème} série)** |
|---|---|---|---|---|---|---|
| 033168 | JYK | 38 | POS | +++ | >5000 | NT |
| 033597 | JYK | 74 | POS | NT | ≈ 5000 | NT |
| 032552 | VTT | 8 | NEG-J3&J8&J12 | NEG | <200 | <5 |
| 032544 | CTP | 16 | NEG J16&J20 | ++ | >5000 | >>20 |
| 032546 | CJF | 15 | NEG J15&J19 | ++ | >5000 | >>20 |
| 032548 | PTL | 17 | NEG J17&J21 | ++ | >5000 | >>20 |
| 032550 | NTH | 17 | NEG-J17&J21 | ++ | >5000 | >>20 |
| 032553 | VTT | 8 | NEG-J3&J8&J12 | NEG | <200 | <5 |
| 032554 | NTBV | 4 | POS | NEG | <200 | <5 |
| 032555 | NTBV | 4 | POS | NEG | <200 | |
| 032564 | NTP | 15 | POS | ++ | >5000 | >>20 |
| 032629 | NVH | 4 | POS | NEG | <200 | <5 |
| 032631 | BTTX | 9 | POS | NEG | <200 | <5 |
| 032635 | NHH | 4 | POS | NEG | <200 | <5 |
| 032637 | NHB | 10 | POS | NEG | <200 | <5 |
| 032642 | BTTX | 9 | POS | NEG | <200 | <5 |
| 032643 | LTDH | 1 | POS | NEG | <200 | <5 |
| 032644 | NTBV | 4 | POS | NEG | <200 | <5 |
| 032646 | TTH | 12 | NEG J7&J12&J16 | ++ | >5000 | >>20 |
| 032647 | DTH | 17 | NEG J17&J21 | ++ | >5000 | >>20 |
| 032648 | NNT | 15 | NEG J15&J19 | ++ | >5000 | >>20 |
| 032649 | PTH | 17 | NEG J17&J21 | ++ | >5000 | >>20 |
| 032672 | LVV | 16 | NEG J16&J20 | + | >5000 | >>20 |
| 032673 | PTTH | NA | NEG | NEG | <200 | <5 |
| 032674 | PNB | 17 | NEG J17&J21 | ++ | >5000 | >>20 |
| 032682 | VTH | 12 | NEG J12&J16 | ++ | >5000 | >>20 |
| 032683 | DTV | 17 | NEG J17&J21 | + | >1000 | >>20 |

| | | | | | | |
|---|---|---|---|---|---|---|
| Notes: (1) : Les analyses par RT-PCR ont été réalisées par RT-PCR nichée BNI, LC Artus et LC-N sur des écouvillonnages nasaux ou pharyngés; POS signifie qu'au moins un prélèvement a été trouvé positif chez ce patient. (2) : La réactivité des sérums dans le test ELISA utilisant un lysat de cellules infectées par le SRAS-CoV a été classée en très fortement réactif (+++), fortement réactif (++), réactif (+) et négatif en fonction de la valeur DO obtenue aux dilutions testées | | | | | | |

### Exemple 8 : Détection du coronavirus associé au SRAS (SARS-CoV) par RT-PCR

### 1) Mise au point de conditions de RT-PCR en temps réel à l'aide d'amorces spécifiques du gène de la protéine de nucléocapside - test "Light Cycler N"

### a) conception des amorces et des sondes

La conception des amorces et sondes a été réalisée à partir de la séquence du génome de la souche de SARS-CoV issue du prélèvement répertorié sous le numéro 031589, à l'aide du programme "Light Cycler Probe Design (Roche)". Ainsi les deux séries d'amorces et de sondes suivantes ont été sélectionnées :

### - série 1 (SEQ ID NO : 60, 61, 64, 65):

- amorce sens : N/+/28507 : 5'-GGC ATC GTA TGG GTT G-3' [28507-28522]
- amorce antisens : N/-/28774 : 5'-CAG TTT CAC CAC CTC C-3' [28774-28759]
- sonde 1 : 5'-GGC ACC CGC AAT CCT AAT AAC AAT GC-fluorescéine 3' [28561-28586]
- sonde 2 : 5' Red705 -GCC ACC GTG CTA CAA CTT CCT-phosphate [28588-28608]

### - série 2 (SEQ ID NO : 62, 63, 66, 67)

- amorce sens : N/+/28375 : 5'-GGC TAC TAC CGA AGA G-3' [28375-28390]
- amorce antisens : N/-/28702 : 5'-AAT TAC CGC GAC TAC G-3' [28702-28687]
- sonde 1 : SRAS/N/FL : 5'-ATA CAC CCA AAG ACC ACA TTG GC - fluorescéine 3' [28541-28563]
- sonde 2 : SRAS/N/LC705 : 5' Red705 -CCC GCA ATC CTA ATA ACA ATG CTG C-phosphate 3' [28565-28589]

### b) analyse de l'efficacité des deux couples amorces

Afin de tester l'efficacité respective des deux couples d'amorces, une amplification par RT-PCR a été réalisée sur un ARN synthétique correspondant aux nucléotides 28054-29430 du génome de la souche de SARS-CoV issue du prélèvement répertorié sous le numéro 031589et contenant la séquence du gène N.

De manière plus précise :
Cet ARN synthétique a été préparé par transcription *in vitro* à l'aide de l'ARN polymérase du phage T7, d'une matrice d'ADN obtenu par linéarisation du plasmide SRAS-N avec l'enzyme *Bam H1.* Après élimination de la matrice d'ADN par digestion à l'aide de DNAse 1, les ARN synthétiques sont purifiés par une extraction au phénol-chloroforme suivie de deux précipitations successives en acétate d'ammonium et isopropanol. Ils sont alors quantifiés par mesure de l'absorbance à 260 nm et leur qualité est contrôlée par le rapport des absorbances à 260 et 280 nm ainsi que par une électrophorèse en gel d'agarose. Ainsi, la concentration de la préparation d'ARN synthétique utilisée pour ces études est de 1,6 mg/ml, ce qui correspond à 2,1.10¹⁵ copies/ml d'ARN.

Des quantités décroissantes d'ARN synthétique ont été amplifiés par RT-PCR à l'aide du kit "Superscript™ One-Step RT-PCR with Platinum® Taq" et les couples d'amorces n° 1 (N/+/28507, N/-/28774) (figure 1A) et n° 2 (N/+/28375, N//28702) (figure 1B), en suivant les indications du fournisseur. Les conditions d'amplification utilisées sont les suivantes : l'ADNc a été synthétisé par incubation 30 min à 45 °C, 15 min à 55°C puis 2 min à 94 °C puis il a été amplifié par 5 cycles comprenant : une étape de dénaturation à 94°C pendant 15 sec, une étape d'hybridation à 45°C pendant 30 sec puis une étape d'élongation à 72°C pendant 30 sec, suivis de 35 cycles comprenant : une étape de dénaturation à 94°C pendant 15 sec, une étape d'hybridation à 55°C pendant 30 sec puis une étape d'élongation à 72°C pendant 30 sec, avec 2 sec d'élongation supplémentaire à chaque cycle, et d'une étape finale d'élongation à 72°C pendant 5 min. Les produits d'amplification obtenus ont ensuite été maintenus à 10°C.

Les résultats présentés à la figure 11 montrent que le couple d'amorces n° 2 (N/+/28375, N/-/28702) permet de détecter jusqu'à 10 copies d'ARN (bande de faible intensité) ou 10² copies (bande de bonne intensité) contre 10⁴ copies pour le couple d'amorces n° 1 (N/+/28507, N/-/28774). Les amplicons sont respectivement de 268 pb (couple 1) et de 328 pb (couple 2).

### c) mise au point de la RT-PCR en temps réel

Une RT-PCR en temps réel a été mise au point à l'aide du couple d'amorces n°2 et du couple de sonde constitué par SRAS/N/FL et SRAS/N/LC705 (figure 2).

L'amplification a été réalisée sur un LightCycler™ (Roche) à l'aide du kit "Light Cycler RNA Amplification Kit Hybridization Probes " (référence 2 015 145, Roche) dans les conditions optimisées suivantes. Un Mélange réactionnel contenant : H₂O (6,8 µl), MgCl₂ 25 mM (0,8 µl, 4 µM final de Mg2+), mélange réactionnel 5X (4 µl), sonde SRAS/N/FL 3µM (0,5 µl, 0,075 µM final), sonde SRAS/N/LC705 3 µM (0,5 µl, 0,075 µM final), amorce N/+/28375 10 µM (1 µl, 0,5 µM final), amorce N/-/28702 10 µM (1 µl, 0,5 µM final), mélange d'enzyme (0,4 µl) et échantillon (ARN viral, 5 µl) a été amplifié en suivant le programme suivant :
- Transcription inverse : **50°C** 10:00min analysis mode: none
- Dénaturation : 95°C 30sec x1 analysis mode: none
- Amplification : 95°C 2sec }
   **50°C** 15sec analysis mode: quantification*} x45
   72°C 13sec rampe thermique 2,0°C/sec }
- refroidissement : 40°C 30sec x1 analysis mode: none

   *La mesure de fluorescence se fait à la fin de l'hybridation et à chaque cycle (en mode SINGLE).

Les résultats présentés à la figure 12 montrent que cette RT-PCR en temps réel est très sensible puisqu'elle permet de détecter 10² copies d'ARN synthétique dans 100% des 5 échantillons analysés (29/29 échantillons dans 8 expériences) et jusqu'à 10 copies d'ARN dans 100% des 5 échantillons analysés (40/45 échantillons dans 8 expériences). Elle montre également que cette RT-PCR permet de détecter la présence du génome du SARS-CoV dans un échantillon et de quantifier le nombre de génomes présents. A titre d'exemple, l'ARN viral d'un stock de SARS-CoV cultivé sur cellules Vero E6 a été extrait à l'aide du kit "Qiamp viral RNA extraction" (Qiagen), dilué à 0,05.10⁻⁴ et analysé par RT-PCR en temps réel selon le protocole décrit ci-dessus; l'analyse présentée à la figure 12 montre que ce stock de virus contient 6,5.10⁹ génomes -équivalents/ml (geq/ml), ce qui est tout à fait similaire à la valeur de 1,0.10¹⁰ geq/ml mesurée à l'aide du kit "RealArt™ HPA-Coronavirus LC RT PCR Reagents" commercialisé par Artus.

### 2) Mise au point de conditions de RT-PCR nichée ciblant le gène de l'ARN polymérase - test "RT-PCR nichée CDC (Centers for Disease Control and Prévention) /IP"

### a) Extraction de l'ARN viral

Echantillon clinique : QIAamp viral RNA Mini Kit (QIAGEN) selon les indications du fabricant, ou une technique équivalente. L'ARN est élué dans un volume de 60 µl.

### b) RT-PCR nichée "SNE/SAR"

### Première étape : RT-PCR couplée « SNE »

Le kit Invitrogen "*Superscript*™ *One-Step RT-PCR with Platinum*® *Taq*"a été utilisé, mais le kit "*Titan*" de Roche Boehringer peut lui être substitué avec des résultats similaires.
Oligonucléotides :
- SNE-S1 5' GGT TGG GAT TAT CCA AAA TGT GA 3'
- SNE-AS 1 5'GCA TCA TCA GAA AGA ATC ATC ATG 3'
- > Taille attendue :**440 pb**

1. Préparer un mix :

| | |
|---|---|
| H2O | 6,5 µl |
| Reaction mix 2X | 12,5 µl |
| Oligo SNE-S1 50 µM | 0,2 µl |
| Oligo SNE-AS1 50 µM | 0,2 µl |
| RNAsin 40 U/µl | 0,12 µl |
| RT/Platinum Taq mix | 0, 5 µl |

2. A 20 µl du mix, ajouter 5 µl d'ARN et procéder à l'amplification sur thermocycleur (conditions ABI 9600) :

| | | | |
|---|---|---|---|
| 2.1 | 45°C | 30 min. | |
| | 55°C | 15 min. | |
| | 94°C | 2 min. | |
| 2.2. | 94°C | 15 sec. | } |
| | 45°C | 30 sec. | } x 5 cycles |
| | 72°C | 30 sec. | } |
| 2.3. | 94°C | 15 sec. | } |
| | 55°C | 30 sec. | } x 35 cycles |
| | 72°C | 30 sec. + 2sec./cycle | } |
| 2.4. | 72°C | 5 min. | |
| 2.5 | 10°C | ∞ | |

Conservation à +4°C.

La RNAsin (N2511/N2515) de Promega a été utilisée comme inhibiteurs de RNase.

Des ARN synthétiques ont servi de témoin positif. A titre de contrôle, 10³, 10² et 10 copies d'ARN synthétique R_{SNE} ont été amplifiées dans chaque expérience.

### Seconde étape : PCR nichée "STAR"

Oligonucléotides :
- SAR1-S 5' CCT CTC TTG TTC TTG CTC GCA 3'
- SAR1-AS 5' TAT AGT GAG CCG CCA CAC ATG 3'
- > Taille attendue : **121 pb**

1. Préparer un mix :

| | |
|---|---|
| H2O | 35,8 µl |
| Tampon Taq 10X | 5 µl |
| MgCl₂ 25 mM | 4 µl |
| Mix dNTPs 5 mM | 2 µl |
| Oligo SAR1-S 50 µM | 0,5 µl |
| Oligo SAR1-AS 50 µM | 0,5 µl |
| Taq ADN pol 5 U/µl | 0,25 µl |

L'*AmpliTaq DNA Pol* d'Applied Biosystems a été utilisée (tampon 10X sans MgCl₂, réf 27216601).
2. A 48 µl du mix, ajouter 2 µl du produit de la première PCR et procéder à l'amplification (conditions ABI 9600) :

| | | | |
|---|---|---|---|
| 2.1. | 94°C | 2 min. | |
| 2.2. | 94°C | 30 sec. | } |
| | 45°C | 45 sec. | } x 5 cycles |
| | 72°C | 30 sec. | } |
| 2.3. | 94°C | 30 sec. | } |
| | 55°C | 30 sec. | } x 35 cycles |
| | 72°C | 30 sec. + lsec./cycle | } |
| 2.4. | 72°C | 5 min. | |
| 2.5 | 10°C | ∞ | |

3. Analyser 10 µl du produit réactionnel sur gel "*low-melting*" (type Seakem GTG) à 3% d'agarose.
La sensibilité du test niché est en routine, dans les conditions décrites, de 10 copies d'ARN.
4. Les fragments peuvent ensuite être purifiés sur QIAquick PCR kit (QIAGEN) et séquencés avec les oligos SAR1-S et SAR1-AS.

### 3) Détection de l'ARN du SARS-CoV par PCR à partir de prélèvements respiratoires

### a) Première étude comparative

Une étude comparative a été réalisée sur une série de prélèvements respiratoires reçus par le Centre National de Référence du Virus Influenzae (région nord) et susceptibles de contenir du SARS-CoV. Pour ce faire, l'ARN a été extrait des prélèvements à l'aide du kit "Qiamp viral RNA extraction" (Qiagen) et analysé par RT-PCR en temps réel, d'une part à l'aide des couples d'amorces et de sondes de la série n° 2 dans les conditions décrites ci-dessus d'une part, et d'autre part à l'aide du kit "LightCycler SARS-CoV quantification kit" commercialisé par Roche (référence 03 604 438). Les résultats sont résumés dans le Tableau VI ci-dessous. Ils montrent que 18 des 26 prélèvements sont négatifs et 5 des 26 prélèvements sont positifs pour les deux kits, tandis qu'un prélèvement est positif pour le seul kit Roche et deux pour les seuls réactifs N "série2". En outre, pour 3 prélèvements (20032701, 20032712, 20032714) les quantités d'ARN détectés sont nettement supérieures avec les réactifs (sondes et amorces) de la série n°2. Ces résultats indiquent que les amorces et sondes N "série2" sont plus sensibles pour la détection du génome du SARS-CoV dans des prélèvements biologiques que celles du kit actuellement disponible.

**Tableau VI: Analyse par RT-PCR en temps réel des ARN extraits d'une série de prélèvements de 5 patients à l'aide des couples d'amorces et de sondes de la série n° 2 (N "série 2") ou du kit "LightCycler SARS-CoV quantification kit" (Roche). Le type de prélèvement est indiqué ainsi que le nombre de copies de génome viral mesurées dans chacun des deux tests. NEG : RT-PCR négative.**

| **Prélèvements n°** | **Patient** | **Type de prélèvement** | **KIT ROCHE** | **N "série2"** |
|---|---|---|---|---|
| 20033082 | K | nasal | NEG | NEG |
| 20033083 | K | pharyngé | NEG | NEG |
| 20033086 | K | nasal | NEG | NEG |
| 20033087 | K | pharyngé | NEG | NEG |
| 20032802 | M | nasal | NEG | NEG |
| 20032803 | M | expectoration | NEG | NEG |
| 20032806 | M | nasal ou pharyngé | NEG | NEG |
| 20031746ARN2 | C | pharyngé | NEG | NEG |
| 20032711 | C | nasal ou pharyngé | **39** | NEG |
| 20032910 | B | nasal | NEG | NEG |
| 20032911 | B | pharyngé | NEG | NEG |
| 20033356 | V | expectoration | NEG | NEG |
| 20033357 | V | expectoration | NEG | NEG |
| 20031725 | K | asp. endotrachéale | NEG | 150 |
| 20032657 | K | asp. endotrachéale | NEG | NEG |
| 20032698 | K | asp. endotrachéale | NEG | NEG |
| 20032720 | K | asp. endotrachéale | **3** | **5** |
| 20033074 | K | selles | **115** | **257** |
| 20032701 | M | pharyngé | **443** | **1676** |
| 20032702 | M | expectoration | NEG | 249 |
| 20031747ARN2 | C | pharyngé | NEG | NEG |
| 20032712 | C | inconnu | **634** | **6914** |
| 20032714 | C | pharyngé | **17** | **223** |
| 20032800 | B | nasal | NEG | NEG |
| 20033353 | V | nasal | NEG | NEG |
| 20033384 | V | nasal | NEG | NEG |

### b) Deuxième étude comparative

Les performances de différentes méthodes de RT-PCR nichée et de RT-PCR en temps réel ont ensuite été comparées pour 121 prélèvements respiratoires de cas possibles de SRAS de l'hôpital français de Hanoi, Vietnam, réalisés entre le 4^{ème} et le 17^{ème} jour après le début des symptômes. Parmi ces prélèvements, 14 avaient été trouvés positifs lors d'un premier test utilisant la méthode de RT-PCR nichée ciblant l'ORF1b (codant pour la réplicase) telle que décrite initialement par le Bernhard Nocht Institute (RT-PCR nichée BNI). Des informations concernant ce test sont disponibles sur internet, à l'adresse http://www15.bni-hamburg.de/bni/bni2/neu2/getfile.acgi?area engl=diagnostics&pid=4112.

Les différents tests comparés dans cette étude sont :
- la méthode de RT-PCR quantitative selon l'invention, avec les amorces et sonde N "série 2" décrite ci-dessus (colonne Light Cycler N),
- le test de RT-PCR nichée ciblant le gène de l'ARN polymérase décrit ci-dessus, développé par le CDC, le BNI et l'Institut Pasteur (RT-PCR nichée CDC/IP),
- le kit ARTUS de référence "HPA Corona LC RT-PCR Kit # 5601-02", qui est un test de RT-PCR en temps réel ciblant le gène ORF1b,
- le test de RT-PCR nichée du BNI, ciblant également le gène de l'ARN polymérase, mentionné ci-dessus.

Les inventeurs ont constaté :
1) une variabilité inter-test pour une même technique, liée à la dégradation de la préparation d'ARN lors de décongélations répétées, notamment pour les échantillons contenant les quantités d'ARN les plus faibles,
2) une sensibilité réduite de la RT-PCR nichée CDC/IP par rapport à la RT-PCR nichée BNI, et
3) une sensibilité comparable du test de RT-PCR quantitative selon l'invention (Light Cycler N) par rapport au test Light Cycler (LC) Artus.

Ces résultats, présentés dans le Tableau VII ci-dessous, montrent que le test par RT-PCR quantitative selon l'invention constitue un excellent complément - ou une alternative - aux tests actuellement disponibles. En effet, le coronavirus lié au SRAS est un virus émergent, susceptible d'évoluer rapidement. En particulier, le gène de la RNA polymérase du coronavirus lié au SRAS, qui est ciblé dans la plupart des tests actuellement disponibles, peut recombiner avec celui d'autres coronavirus non liés au SRAS. L'utilisation d'un test ciblant exclusivement ce gène pourrait alors conduire à l'obtention de faux négatifs.

Le test par RT-PCR quantitative selon l'invention ne cible pas la même région génomique que le kit ARTUS, puisqu'il cible le gène codant pour la protéine N. En réalisant un test de diagnostique ciblant deux gènes différents du coronavirus lié au SRAS, on peut donc espérer s'affranchir de résultats de type faux négatifs, qui pourraient être dus à l'évolution génétique du virus.

De plus, il apparaît particulièrement avantageux de cibler le gène de la protéine de nucléocapside, car il est très stable, du fait de la forte pression de sélection liée aux contraintes structurales élevées concernant cette protéine.

**Tableau VII: Comparaison de différentes méthodes d'analyse par amplification génique, à partir de 121 Prélèvements de cas probables de SRAS de l'hôpital français de Hanoi, Vietnam (épidémie 2003)**

| **N° CNR** | **Type prélèvement (1)** | **Jour prélèvement** | **Patient** | **RT-PCR nichée CDC/IP** | **RT-PCR nichée BNI** | **kit Light Cycler Artus** | **Light Cycler N (IP)** |
|---|---|---|---|---|---|---|---|
| 107 prélèvements | N et P | | | Négatif | Négatif | Négatif | Négatif |
| 032529 | P | 10 | NHB | Négatif | Positif | Négatif | Négatif |
| 032530 | N | 10 | NHB | Positif | Positif | 3,10E+01 | 4,20E+01 |
| 032531 | P | 7 | LP | Positif | Positif | 7,70E+00 | 3,10E+00 |
| 032534 | N | 15 | BND | Positif | Positif | 1,60E+00 | Négatif |
| 032600 | P | 4 | NHH | Négatif | Positif | Négatif | 1,30E+02 |
| 032612 | P | 17 | NTS | Négatif | Positif | Négatif | Négatif |
| 032688 | P | 9 | BTX | Positif | Positif | Négatif | Négatif |
| 032689 | N | 4 | NVH | Positif | Positif | 1,20E+01 | 2,30E+02 |
| 032690 | P | 4 | NVH | Négatif | Positif | 1,60E+00 | Négatif |
| 032727 | P | 8 | NVH | Positif | Positif | 2,30E+02 | 4,00E+02 |
| 032728 | N | 8 | NVH | Positif | Positif | 1,10E+03 | 1,60E+04 |
| 032729 | P | 14 | NHB | Positif | Positif | 5,90E+00 | 3,40E+01 |
| 032730 | N | 14 | NHB | Positif | Positif | 1,30E+02 | 4,80E+02 |
| 032741 | P | 8 | NHH | Positif | Positif | 2,10E+02 | 1,30E+02 |
| | | | | | | | |
| positifs | | | | 10 | 14 | 10 | 9 |
| fraction détectée des 14 positifs | | | | 71,4% | 100,0% | 71,4% | 64,3% |

| | | | | | | | |
|---|---|---|---|---|---|---|---|
| (1) P= écouvillonage pharyngé N= écouvillonage nasal | | | | | | | |

### Exemple 9 : Obtention et caractérisation d'anticorps monoclonaux dirigés contre la protéine N

Des souris Balb C ont été immunisées à l'aide de la protéine N recombinante purifiée et leurs cellules spléniques fusionnées avec un myélome murin approprié selon les techniques de Köhler et Milstein.

Dix-neuf hybridomes sécréteurs d'anticorps anti N ont été présélectionnés et leurs immuno réactivités précisées. Ces anticorps reconnaissent bien la protéine N recombinante (en ELISA) avec des intensités variables, ainsi que la protéine virale, naturelle N en ELISA et/ou en Western Blot. Les figures 18 à 20 montrent les résultats de ces tests pour 15 de ces 19 anticorps monoclonaux.

Les clones 12, 17, 28, 57, 72, 76, 86, 87, 98, 103, 146, 156, 166, 170, 199, 212, 218, 219 et 222, fortement réactifs, ont été sous clonés. Les études de spécificité ont été poursuivies avec les outils appropriés afin de préciser les épitopes reconnus et vérifier l'absence de réactivité vis-à-vis des autres Coronavirus humains et de certains virus respiratoires.

Les études de cartographie (*mapping*) épitopique (réalisées sur membrane spot, à l'aide de peptides chevauchants de 15 aa) et les études supplémentaires réalisées sur la protéine N naturelle en Western Blot ont révélé l'existence de 4 groupes d'anticorps monoclonaux :
1° Anticorps monoclonaux spécifiques d'un épitope linéaire majeur en position N-ter (75-81, séquence : INTNSVP)
   Le représentant de ce groupe est l'anticorps 156. L'hybridome produisant cet anticorps a été déposé à la Collection Nationale de Cultures de Microorganismes (CNCM) de l'Institut Patseur (Paris, France) le 1^{er} décembre 2004, sous le numéro I-3331. Ce même épitope est également reconnu par un sérum de lapin (polyclonal anti N) obtenu par immunisation classique à l'aide de cette même protéine N.
2° Anticorps monoclonaux spécifiques d'un épitope linéaire majeur situé en position centrale (position 217-224, séquence : ETALALL); les représentants de ce groupe sont les anticorps monoclonaux 87 et 166. L'hybridome produisant l'anticorps 87 a été déposé à la CNCM le 1^{er} décembre 2004, sous le numéro I-3328.
3° Anticorps monoclonaux spécifiques d'un épitope linéaire majeur situé en position C-terminale (position 403-408, séquence : DFFRQL), les représentants de ce groupe sont les anticorps 28, 57 et 143. L'hybridome produisant l'anticorps 57 a été déposé à la CNCM le 1^{er} décembre 2004, sous le numéro I-3330.
4° Anticorps monoclonaux spécifiques d'un épitope discontinu, conformationel. Ce groupe d'anticorps ne reconnaît aucun des peptides recouvrant la séquence de la protéine N, mais réagissent fortement sur la protéine naturelle non dénaturée. Le représentant de ce dernier groupe est l'anticorps 86. L'hybridome produisant cet anticorps a été déposé à la CNCM le 1^{er} décembre 2004, sous le numéro I-3329.

Le Tableau VIII ci-après résume les résultats de cartographie épitopique obtenus :

**Tableau VIII : Mapping épitopique des anticorps monoclonaux**

| **Anticorps** | **Epitope** | **Position** | **Région** |
|---|---|---|---|
| **28** | **DFSRQL** *Q* | **403 ... 408** | **C - Ter.** |
| **143** | **DFSRQL** *Q* | | |
| **76** | **DFSRQL** *Q* | | |
| **57** | **DFSRQL** *Q* | | |
| | ***FFGMS** RI* | **315 ... 319** | |
| **146** | **LPQRQ** | **383 ... 387** | |
| **166** | **ETALALL***LL* | **217 ... 224** | **centrale** |
| **87** | **ETALALL** | **217 ... 224** | |
| ***156*** | ***INTNSGP*** | **75 ... 81** | ***N - Ter.*** |
| ***86*** | ***Conformationnel*** | | |
| ***212*** | ***Conformationnel*** | | |
| **170** | **Conformationnel** | | |

En outre, comme illustré notamment aux figures 18 et 19, ces anticorps ne présentent pas de réactivité en ELISA et/ou en WB, vis à vis de la protéine N du coronavirus humain 229 E.

### Exemple 10 : Combinaisons des anticorps monoclonaux pour le développement d'un test d'immunocapture sensible et spécifique de l'antigène viral N dans le sérum ou les fluides biologiques des patients contaminés par le virus SARS Co V

Les anticorps listés ci-dessous ont été retenus en raison de leurs propriétés bien particulières pour une étude supplémentaire de capture et détection de la protéine virale N, dans le sérum des sujets ou patients.

Ces anticorps ont été produits en ascite sur souris, purifiés par chromatographie d'affinité et utilisés seuls ou en combinaison comme anticorps de capture, et comme anticorps signal.

Liste des anticorps sélectionnés pour le propos :
- Acm anti région C-ter (n° 28, 57, 143)
- Acm anti région centrale (n° 87, 166)
- Acm anti région N-ter (n° 156)
- Acm anti épitope discontinu, conformationnel (86)

### 1) Préparation des réactifs :

### a) Plaques ELISA immunocapture

Les plaques sont sensibilisées avec les solutions d'anticorps à 5 µg/ml en tampon carbonate 0,1 M, pH.9,6. Les solutions (monovalentes ou plurivalentes) sont déposées sous volume de 100 µl dans les puits et incubées pendant une nuit à température ambiante. Ces plaques sont ensuite lavées en tampon PBS (10 mM pH 7,4 additionné de 0,1% de Tween 20) puis saturées avec une solution de PBS additionnée de 0,3% de BSA et de 5% de Saccharose). Les plaques sont ensuite séchées puis conditionnées dans un sachet en présence d'un dessicant. Elles sont prêtes à l'emploi.

### b) Conjugués

Les anticorps purifiés ont été couplés à la péroxydase selon le protocole de Nakane (Nakane et al. - 1974, J.of Histo and cytochemistry, vol.22, p1084-1091) dans un rapport de une molécule d'IgG pour 3 molécules de péroxydase. Ces conjugués ont été purifiés par chromatographie d'exclusion et conservés concentrés (concentration comprise entre 1 à 2 mg/ml) en présence de 50% de glycerol et à -20°C. Ils sont dilués pour leur mise en oeuvre dans les essais à la concentration finale de 1 ou 2 µg/ml en tampon PBS (pH 7,4) additionné de 1% de BSA.

### c) Autres réactifs

- Sérums humains négatifs pour tous les marqueurs sériques des virus HIV, HBV, HCV et THLV
- Pool de sérums humains négatifs additionné de 0,5% de Triton X 100
- Ag viral inactivé : surnageant de culture virale inactivé par irradiation et inactivation vérifiée après mise en culture sur cellules sensibles - titre de la suspension avant inactivation environ 10⁷ particules infectieuses par ml ou encore environ 5x10⁹ particules virales physiques par ml d'antigène
- Les échantillons d'Ag dilués en sérum humain négatif : ces échantillons ont été préparés par dilution au 1:100^{e} puis par dilution en série de raison 5.
   Ces échantillons non infectieux miment des échantillons humains supposés contenir des concentrations faibles à très faibles de nucléoprotéine virale N. De tels échantillons ne sont pas accessibles pour les travaux de routine
- Solution de lavage R2, solution de révélation TMB R8, chromogène R9 et solution d'arrêt R10, sont les réactifs génériques commercialisés par Bio-Rad dans ses trousses ELISA (ex : trousse *Platelia Pylori* ref.72778).

### 2) Mode opératoire

Les échantillons de sérums humains surchargés en Ag viral inactivé sont distribués à raison de 100 µl par cupule, directement dans les plaques sensibilisées, prêtes à l'emploi puis incubés pendant 1 heure à 37°C (incubation Bio-Rad IPS).

Le matériel non retenu par la phase solide est éliminé par 3 lavages (lavage avec solution R2 diluée, laveur automatique LP 35).

Les conjugués appropriés, dilués à la concentration finale de 1 ou 2 µg/ml sont distribués à raison de 100 µl par cupule et les plaques incubées à nouveau pendant une heure à 37° C (incubation IPS).

L'excès de conjugué est éliminé par 4 lavages successifs (solution R2 diluée - laveur LP 35).

La présence de conjugué fixé sur les plaques est révélée après addition de 100 µl de solution de révélation préparée avant emploi (1 ml de R9 et 10 ml de R8) et après incubation pendant 30 minutes, à température ambiante et à l'abri de la lumière.

La réaction enzymatique est finalement bloquée par addition de 100 µl de réactif R10 (H2SO4 1N) dans toutes les cupules.

La lecture est effectuée à l'aide d'un lecteur de microplaque approprié à double longueur d'onde (450/620 nm).

Les résultats peuvent être interprétés en utilisant comme valeur seuil provisoire la moyenne d'au moins deux contrôles négatifs multiplié par un facteur 2 ou encore la moyenne de 100 sérums négatifs additionnée d'un incrément correspondant à 6 SD (*Standart Deviation* calculée sur les 100 mesures individuelles).

### 3) Résultats

Différentes combinaisons anticorps de capture et anticorps signal ont été testées en se fondant sur les propriétés des anticorps sélectionnés, et en évitant les combinaisons d'anticorps spécifiques des mêmes épitopes en phase solide et en conjugués.

Les meilleurs résultats ont été obtenus avec les 4 combinaisons listées ci-dessous. Ces résultats sont reproduits dans le tableau IX ci-après.
1. Combinaison F/28
   Phase solide (Acm 166 + 87 région centrale) : conjugué anticorps 28 (C-ter)
2. Combinaison G/28
   Phase solide (Acm 86 - épitope conformationel) : conjugué anticorps 28 (C-ter)
3. Combinaison H/28
   Phase solide (Acms 86, 166 et 87 région centrale et épitope conformationel) : conjugué anticorps 28 (C-ter)
4. Combinaison H/28 + 87
   Phase solide (Acms 86, 166 et 87 région centrale et épitope conformationel) : conjugué mixte anticorps 28 (C-ter) et 87 (central)
5. Combinaison G/87
   Phase solide (Acm 86 - épitope conformationel) : conjugué anticorps 87 (région centrale)

Les 4 premières combinaisons présentent des performances équivalentes et reproduites, supérieures aux autres combinaisons mises en oeuvre (comme par exemple la combinaison G/87). Bien entendu, dans ces combinaisons, un anticorps monoclonal peut être remplacé par un autre anticorps reconnaissant le même épitope. Ainsi, on peut citer les variantes suivantes :
6. Variante de la combinaison F/28
   Phase solide (Acm 87 uniquement) : conjugué anticorps 57 (C-ter)
7. Variante de la combinaison G/28
   Phase solide (Acm 86 - épitope conformationel) : conjugué anticorps 57 (C-ter)
8. Variante de la combinaison H/28
   Phase solide (Acms 86 et 87 région centrale et épitope conformationel) : conjugué anticorps 57 (C-ter)
9. Variante de la combinaison H/28 + 87
   Phase solide (Acms 86 et 87 région centrale et épitope conformationel) : conjugué mixte anticorps 57 (C-ter) et 87 (central)

**Tableau IX : Contrôle d'immunoréactivité des Acm anti nucléoprotéines SARS CoV : densités optiques mesurées avec chaque combinaison d'anticorps, en fonction des dilutions de l'antigène viral inactivé.**

| **N°** | **Dilution** | **F/28** | **G/28** | **G/87** | **H/28** | **H/28+87** |
|---|---|---|---|---|---|---|
| 0 | 1/100 | 5 | 5 | 3,495 | 3,900 | 5 |
| 1 | 1/500 | 3,795 | 3,814 | 1,379 | 3,702 | 3,804 |
| 2 | ½ 500 | 2,815 | 2,950 | 0,275 | 3,268 | 2,680 |
| 3 | 1/12500 | 0,987 | 1,038 | 0,135 | 1,374 | 0,865 |
| 4 | 1/62500 | 0,404 | 0,348 | 0,125 | 0,480 | 0,328 |
| 5 | 1/312500 | 0,285 | 0,211 | 0,123 | 0,240 | 0,215 |
| 6 | Témoin | 0,210 | 0,200 | 0,098 | 0,186 | 0,156 |
| 7 | Témoin | 0,269 | 0,153 | 0,104 | 0,193 | 0,202 |

La limite de détection de ces 4 essais expérimentaux correspond à la dilution d'antigène en sérum négatif 1:62500. Une extrapolation rapide laisse supposer la détection de moins de 10³ particules infectieuses par ml de sérums.

De cette étude, il ressort que les anticorps les mieux appropriés pour la capture de la nucleoprotéine virale native sont les anticorps spécifiques de la région centrale et/ou d'un épitope conformationnel, l'un et l'autre étant des anticorps sélectionnés également pour leur forte affinité pour l'antigène natif.

Ayant déterminé les meilleurs anticorps pour la composition de la phase solide, les anticorps à retenir en priorité pour la détection des antigènes fixés sur la phase solide, sont les anticorps complémentaires spécifiques d'un épitope dominant en région C-ter. L'emploi de tout autre anticorps complémentaire, mais spécifique des épitopes localisés en région N-ter de la protéine conduit à des résultats moyens ou médiocres.

### Exemple 11 : Systèmes d'expression eucaryotes de la protéine de spicule (S) du coronavirus associé au SRAS (SRAS-CoV)

### 1) Optimisation des conditions d'expression de la S du SRAS-CoV en cellules de mammifères.

Les conditions d'expression transitoire de la protéine de spicule (S) du SRAS-CoV ont été optimisées en cellules de mammifères (293T, VeroE6).

Pour cela, un fragment d'ADN contenant le cDNA de la S du SRAS-CoV a été amplifié par PCR à l'aide des oligonucléotides 5'-ATAGGATCCA CCATGTTTAT TTTCTTATTA TTTCTTACTC TCACT-3' et 5'-ATACTCGAGTT ATGTGTAATG TAATTTGACA CCCTTG-3' à partir du plasmide pSARS-S (C.N.C.M. n° 1-3059) puis inséré entre les sites BamH1 et Xho1 du plasmide pTRIPΔU3-CMV contenant un vecteur lentiviral TRIP (Sirven, 2001, Mol. Ther., 3, 438-448) pour obtenir le plasmide pTRIP-S. Le fragment BamH1 et Xho1 contenant le cDNA de la S a ensuite été sous-cloné entre les BamH1 et Xho1 du plasmide d'expression eucaryote pcDNA3.1(+) (Clontech) pour obtenir le plasmide pcDNA-S. Le fragment Nhe1 et Xho1 contenant le cADN de la S a ensuite été sous-cloné entre les sites correspondants du plasmide d'expression pCI (Promega) pour obtenir le plasmide pCI-S. Les séquences WPRE du virus de l'hépatite de la marmotte ("*Woodchuck Hepatitis Virus posttranscriptional regulatory element*") et les séquences CTE ("*constitutive transport element*") du rétrovirus simien de Mason-Pfizer ont été insérées dans chacun des deux plasmides pcDNA-S et pCI-S entre les sites Xho1 et Xba1 pour obtenir respectivement les plasmides pcDNA-S-CTE, pcDNA-S-WPRE, pCI-S-CTE et pCI-S-WPRE (figure 21). Le plasmide pCI-S-WPRE a été déposé à la CNCM, le 22 novembre 2004, sous le numéro 1-3323. Tous les inserts ont été séquencés à l'aide d'un kit BigDye Terminator v1.1 (Applied Biosystems) et d'un séquenceur automatique ABI377.

La capacité des plasmides construits à diriger l'expression de la S du SRAS-CoV dans des cellules de mammifères a été recherchée après transfection de cellules VeroE6 (figure 22). Dans cette expérience, des monocouches de 5x10⁵ cellules VeroE6 en boites de Pétri de 35mm ont été transfectées avec 2 µg des plasmides pcDNA (à titre de contrôle), pcDNA-S, pCI et pCI-S et 6 µl de réactif Fugene6 selon les indications du fabricant (Roche). Après 48 heures d'incubation à 37°C et sous 5% de CO₂, des extraits cellulaires ont été préparé en tampon de dépôt selon Laemmli, séparés sur un gel SDS à 8% de polyacrylamide, puis transférés sur une membrane de PVDF (BioRad). La détection de cette immunoempreinte (« western blot ») a été réalisée à l'aide d'un sérum polyclonal de lapin anti-S (immun-sérum du lapin P11135 : cf exemple 4 ci-dessus) et d'anticorps polyclonaux d'âne dirigés contre les IgG de lapin et couplés à la peroxidase (NA934V, Amersham). Les anticorps fixés ont été révélés par luminescence à l'aide du kit ECL+ (Amersham) et de films d'autoradiographie Hyperfilm MP (Amersham).

Cette expérience (figure 22) montre que le plasmide pcDNA-S ne permet pas de diriger l'expression de la S du SRAS-CoV à des niveaux détectables tandis que le plasmide pCI-S permet une expression faible, proche de la limite de détection, qui peut être mise en évidence lorsque le film est surexposé. Des résultats similaires ont été obtenus lorsque l'expression de la S a été recherchée par immunofluorescence (données non montrées). Cette impossibilité de détecter une expression efficace de la S ne peut pas être imputée aux techniques de détection utilisées puisque la protéine S peut être mise en évidence à la taille attendue (180 kDa) dans un extrait de cellules infectées par le SRAS-CoV ou dans un extrait de cellules VeroE6 infectées par le virus recombinant de la vaccine VV-TF7.3 et transfectées par le plasmide pcDNA-S. Dans cette dernière expérience, le virus VV-TF7.3 exprime l'ARN polymérase du phage T7 et permet la transcription cytoplasmique d'un ARN non coiffé susceptible d'être traduit efficacement. Cette expérience suggère que les défauts d'expression décrits ci-dessus sont dus à une incapacité intrinsèque du cADN de la S à être exprimé efficacement lorsque l'étape de transcription en ARN messager est réalisée au niveau nucléaire.

Dans une seconde expérience, l'effet des signaux CTE et WPRE sur l'expression de la S a été recherchée après transfection de cellules VeroE6 (figure 23A) et 293T (figure 23B) et selon un protocole similaire à celui décrit ci-dessus. Alors que l'expression de la S ne peut pas être mis en évidence après transfection des plasmides pcDNA-S-CTE et pcDNA-S-WPRE dérivés de pcDNA-S, l'insertion des signaux WPRE et CTE améliore fortement l'expression de la S dans le contexte du plasmide d'expression pCI-S.

Pour préciser ce résultat, une deuxième série d'expériences a été réalisée, où l'immunoempreinte est révélée de façon quantitative par luminescence et acquisition sur un dispositif d'imagerie numérique (FluorS, BioRad). L'analyse des résultats obtenus avec le logiciel QuantityOne v4.2.3 (BioRad) montre que les séquences WPRE et CTE augmentent respectivement l'expression de la S d'un facteur 20 à 42 et 10 à 26 en cellules Vero E6 (Tableau X). En cellules 293T (Tableau X), l'effet de la séquence CTE est plus modéré (4 à 5 fois) tandis que celui de la séquence WPRE reste important (13 à 28 fois).

**Tableau X analyse quantitative de l'effet des signaux CTE et WPRE sur l'expression de la S du SRAS-CoV : Des extraits cellulaires ont été préparés 48 heures après transfection de cellulesVeroE6 ou 293T par les plasmides pCI, pCI-S, pCI-S-CTE et pCI-S-WPRE et analysés par western blot comme décrit dans la légende de la figure 22. Le western blot est révélé par luminescence (ECL+, Amersham) et acquisition sur un dispositif d'imagerie numérique (FluorS, BioRad). Les niveaux d'expression sont indiqués en fonction d'une échelle arbitraire où la valeur de 1 représente le niveau mesuré après transfection du plasmide pCI-S. Deux expériences indépendantes ont été réalisées pour chacun des deux types cellulaires. Dans l'expérience 1 sur cellules VeroE6, les transfections ont été réalisées en dupliqués et les résultats sont indiqués sous la forme de moyennes et écart-types des niveaux d'expression mesurés.**

| **Plasmide** | **Cellule** | **exp. 1** | **exp. 2** |
|---|---|---|---|
| **PCI** | VeroE6 | 0,0 | 0,0 |
| **pCI-S** | VeroE6 | 1,0±0,1 | 1,0 |
| **pCI-S-CTE** | VeroE6 | 9,8 ±0,9 | 26,4 |
| **pCI-S-WPRE** | VeroE6 | 20,1±2,0 | 42,3 |
| **PCI** | 293T | 0,0 | 0,0 |
| **PCI-S** | 293T | 1,0 | 1,0 |
| **PCI-S-CTE** | 293T | 4,6 | 4,0 |
| **PCI-S-WPRE** | 293T | 27,6 | 12,8 |

En résumé, l'ensemble de ces résultats montre que l'expression dans des cellules de mammifères du cADN de la S du SRAS-CoV sous la dépendance de séquences promotrices de l'ARN polymérase II, requiert, pour être efficace, la présence d'un signal d'épissage ainsi que de l'une ou l'autre des séquences WPRE et CTE.

### 2) Obtention de lignées stables permettant l'expression de la S du SRAS-CoV

Le cADN de la protéine S du SRAS-CoV a été cloné sous la forme d'un fragment BamH1-Xho1 dans le plasmide pTRIPΔU3-CMV contenant un vecteur lentiviral défectif TRIP à DNA flap central (Sirven et al, 2001, Mol. Ther., 3 :438-448) pour obtenir le plasmide pTRIP-S (figure 24). La co-transfection transitoire selon Zennou et al (2000, Cell, 101 : 173-185) de ce plasmide, d'un plasmide d'encapsidation (p8.2) et d'un plasmide d'expression de la glycoprotéine d'enveloppe G du VSV (pHCMV-G) dans des cellules 293T a permis la préparation de pseudoparticules rétrovirales contenant le vecteur TRIP -S et pseudotypées par la protéine d'enveloppe G. Ces vecteurs TRIP-S pseudotypés ont été utilisés pour transduire des cellules 293T et FRhK-4 : aucune expression de la protéine S n'a pu être mis en évidence par western blot et immunofluorescence dans les cellules transduites (données non présentées).

Les cassettes d'expression optimales constituées du promoteur immédiat/précoce du virus CMV, d'un signal d'épissage, du cDNA de la S et de l'un ou l'autre des signaux post-transcriptionnels WPRE ou CTE décrites ci-dessus ont alors été substituées à la cassette EF1α-EGFP du vecteur d'expression lentiviral défectif à DNA FLAP central TRIPΔU3-EF1α (Sirven et al, 2001, Mol. Ther., 3 :438-448) (figure 25). Ces substitutions ont été réalisées par une série de sous-clonages successifs des cassettes d'expression de S qui ont été excisées des plasmides pCT-S-CTE (BglII-Ap1) ou respectivement pCI-S-WPRE (BglII-Sal1) puis insérées entre les sites Mlu1 et Kpn1 ou respectivement Mlu1 et Xho1 du plasmide TRIPΔU3-EF1α pour obtenir les plasmides pTRIP-SD/SA-S-CTE et pTRIP-SD/SA-S-WPRE, déposés à la CNCM, le 1^{er} décembre 2004, sous les numéros I-3336 et 1-3334, respectivement. Des vecteurs pseudotypés ont été produits selon Zennou et al (2000, Cell, 101 : 173-185) et utilisés pour transduire des cellules 293T (10000 cellules) et FRhK-4 (15000 cellules) selon une série de 5 cycles successifs de transduction avec une quantité de vecteur correspondant à 25 ng (TRIP-SD/SA-S-CTE) ou 22 ng TRIP-SD/SA-S-WPRE) de p24 par cycle.

Les cellules transduites ont été clonées par dilution limite et une série de clones ont été analysés pour l'expression de la S du SRAS-CoV qualitativement par immunofluorescence (données non montrées), puis quantitativement par western blot (figure 25) à l'aide d'un sérum polyclonal de lapin anti-S. Les résultats présentés dans la figure 25 montrent que les clones 2 et 15 de cellules FrhK4-s-CTE transduites par TRIP-SD/SA-S-CTE et les clones 4, 9 et 12 de cellules FRhK4-S-WPRE transduites par TRIP-SD/SA-S-WPRE permettent l'expression de la S du SRAS-CoV à des niveaux respectivement faibles et modérés si on les compare à ceux que l'on peut observer au cours de l'infection par le SRAS-CoV.

En résumé, les vecteurs TRIP-SD/SA-S-CTE et TRIP-SD/SA-S-WPRE permettent l'obtention de clones stables de cellules FRhK-4 et de façon similaire 293T exprimant la S du SRAS-CoV, alors que les essais réalisés avec le vecteur "de base" TRIP-S sont restés infructueux, ce qui démontre la nécessité d'un signal d'épissage ainsi que de l'une, ou l'autre, des séquences CTE et WPRE pour l'obtention de clones cellulaires stables exprimant la protéine S.

En outre, ces modifications du vecteur TRIP (insertion d'un signal d'épissage et d'un signal post-transcriptionnel comme CTE et WPRE) pourraient s'avérer intéressantes pour améliorer l'expression d'autres cDNA que celui de la S.

### 3) Obtention de lignées stables permettant l'expression d'une forme soluble de la S du SRAS-CoV. Purification de cet antigène recombinant

Un cDNA codant pour une forme soluble de la protéine S (Ssol) a été obtenu en fusionnant les séquences codant pour l'ectodomaine de la protéine (acides aminés 1 à 1193) à celles d'une étiquette (FLAG : DYKDDDDK) via un linker BspE1 codant pour le dipeptide SG. Pratiquement, pour obtenir le plasmide pcDNA-Ssol, un fragment d'ADN codant pour l'ectodomaine de la S du SRAS-CoV a été amplifié par PCR à l'aide des oligonucléotides 5'-ATAGGATCCA CCATGTTTAT TTTCTTATTA TTTCTTACTC TCACT-3' et 5'-ACCTCCGGAT TTAATATATT GCTCATATTT TCCCAA-3' à partir du plasmide pcDNA-S, puis inséré entre les sites uniques BamH1 et BspE1 uniques d'un plasmide d'expression eucaryote pcDNA3.1 (+) (Clontech) modifié contenant entre ses sites BamH1 et Xho1 la séquence de l'étiquette FLAG :

Les fragments Nhe1-Xho1 et BamH1-Xho1, contenant le cADN de la forme soluble de la S, ont ensuite été excisés du plasmide pcDNA-Ssol, et sous-clonés entre les sites correspondants du plasmide pTRIP-SD/SA-S-CTE et du plasmide pTRIP-SD/SA-S-WPRE, respectivement, pour obtenir les plasmides pTRIP-SD/SA-Ssol-CTE et pTRIP-SD/SA-Ssol-WPRE, déposés à la CNCM, le 1^{er} décembre 2004, sous les numéros 1-3337 et 1-3335, respectivement.

Des vecteurs pseudotypés ont été produits selon Zennou et coll. (2000, Cell, 101 : 173-185) et utilisés pour transduire des cellules FRhK-4 (15000 cellules) selon une série de 5 cycles successifs de transduction (15000 cellules) avec une quantité de vecteur correspondant à 24 ng (TRIP-SD/SA-Ssol-CTE) ou 40 ng TRIP-SD/SA-Ssol-WPRE) de p24 par cycle. Les cellules transduites ont été clonées par dilution limite et une série de 16 clones transduits par TRIP-SD/SA-Ssol-CTE et de 15 clones par TRIP-SD/SA-Ssol-WPRE ont été analysés pour l'expression du polypeptide Ssol par western blot révélé par un anticorps monoclonal anti-FLAG (figure 26 et données non montrées), ainsi que par un ELISA-capture spécifique du polypeptide Ssol qui a été mis au point dans ce but (Tableau XI et données non montrées). Une partie du processus de sélection des meilleurs clones sécréteurs est montré dans la figure 26. L'ELISA-capture repose sur l'utilisation de phases solides recouvertes d'anticorps polyclonaux de lapins immunisés par du SRAS-CoV purifié et inactivé. Ces phases solides permettent la capture du polypeptide Ssol sécrété dans les surnageants cellulaires, dont la présence est ensuite révélée par une série d'étapes impliquant successsivement la fixation d'un anticorps monoclonal anti-FLAG (M2, SIGMA), d'anticorps biotinylés polyclonaux de lapin anti-IgG(H+L) de souris (Jackson) et d'un conjugué streptavidine-peroxidase (Amersham) puis l'addition de chromogène et de substrat (TMB + H₂0₂, KPL).

**Tableau XI : analyse de l'expression de polypeptide Ssol par des lignées cellulaires transduites par les vecteurs lentiviraux TRIP-SD/SA-Ssol-WPRE et TRIP-SD/SA-Ssol-CTE. La sécrétion du polypeptide Ssol a été recherchée dans le surnageant d'une série de clones cellulaires isolés après transduction de cellules FRhK-4 par les vecteurs lentiviraux TRIP-SD/SA-Ssol-WPRE et TRIP-SD/SA-Ssol-CTE. Les surnageants dilués au 1/50 ont été analysés par un test ELISA-capture spécifique de la S du SRAS-CoV.**

| Vecteur | Clone | DO (450 nm) |
|---|---|---|
| Témoin | - | 0,031 |
| TRIP-SD/SA-Ssol-CTE | CTE2 | 0,547 |
| | CTE3 | 0,668 |
| | CTE9 | 0,171 |
| | CTE12 | 0,208 |
| | CTE13 | 0,133 |
| TRIP-SD/SA-Ssol-WPRE | WPRE1 | 0,061 |
| | WPRE10 | 0,134 |

La lignée cellulaire sécrétant les quantités les plus élevées de polypeptide Ssol dans le surnageant de culture est la lignée FRhK4-Ssol-CTE3. Elle a été soumise à une deuxième série de 5 cycles de transduction par le vecteur TRIP-SD/SA-Ssol-CTE dans des conditions similaires à celles décrites ci-dessus puis clonée. Le sous-clone sécrétant les quantités les plus élevés de Ssol a été sélectionné par une combinaison d'analyse par western blot et ELISA-capture : il s'agit du sous-clone FRhK4-Ssol-30, qui a été déposé à la CNCM, le 22 novembre 2004, sous le numéro I-3325.

La lignée FRhK4-Ssol-30 permet la production et la purification en quantité du polypeptide recombinant Ssol. Dans une expérience type où les conditions expérimentales de croissance, de production et de purification ont été optimisées, les cellules de la lignée FRhK4-Ssol-30 sont ensemencées en milieu de culture standard (DMEM sans pyruvate contenant 4.5g/l de glucose et supplémenté par 5% de SVF, 100 U/ml de pénicilline et 100 µg/ml de streptomycine) sous la forme d'une monocouche sous-confluente (1 million de cellules pour chaque 100 cm² dans 20 ml de milieu). A confluence, le milieu standard est remplacé par le milieu de sécrétion où la quantité de SVF est abaissée à 0.5% et la quantité de milieu réduite à 16 ml pour chaque 100 cm². Le surnageant de culture est prélevé après 4 à 5 jours d'incubation à 35°C et sous 5% de CO₂. Le polypeptide recombinant Ssol est purifié à partir du surnageant par l'enchaînement d'étapes de filtration sur membrane de polyethersulfone (PES) de 0.1 µm, de concentration par ultrafiltration sur une membrane de PES de point de coupure 50kD, de chromatographie d'affinité sur matrice anti-FLAG avec élution par une solution de peptide FLAG (DYKDDDDK) à 100 µg/ml en TBS (Tris 50mM pH 7.4, 150 mMNaCl) puis de chromatographie de gel filtration en TBS sur billes de sephadex G-75 (Pharmacia). La concentration du polypeptide recombinant Ssol purifié a été déterminée par test micro-BCA (Pierce) puis ses caractéristiques biochimiques analysées.

L'analyse par gel SDS à 8 % d'acrylamide coloré au nitrate d'argent met en évidence un polypeptide majoritaire dont la masse moléculaire est d'environ 180kD et dont le degré de pureté peut être évalué à 98% (figure 27A). Par spectrométrie de masse SELDI-TOF (Cyphergen), deux pics principaux sont mis en évidence : ils correspondent à des formes simplement, et doublement chargées d'un polypeptide majoritaire dont la masse moléculaire est ainsi déterminée à 182,6 ± 3,7 kD (figures 27B et C). Après transfert sur membrane Prosorb et rincage en TFA 0, 1 %, l'extrémité N-terminale du polypeptide Ssol a été séquencée en phase liquide par dégradation d'Edman sur 5 résidus (ABI494, Applied Biosystems) et déterminée comme étant SDLDR (figure 27D). Ceci démontre que le peptide signal localisé à l'extrémité N-terminale de la protéine S du SRAS-CoV, composé des aa 1 à 13 (MFIFLLFLTLTSG) d'après une analyse réalisée avec le logiciel signalP v2.0 (Nielsen et al., 1997, Protein Engineering, 10 : 1-6), est clivé du polypeptide Ssol mature. Le polypeptide recombinant Ssol est donc constitué des acides aminés 14 à 1193 de la protéine S du SRAS-CoV fusionnés en C-terminal à une séquence SGDYKDDDDK contenant la séquence de l'étiquette FLAG (soulignée). L'écart entre la masse molaire théorique du polypeptide Ssol nu (132,0 kD) et la masse molaire réelle du polypeptide mature (182,6 kD) suggère que le polypeptide Ssol est glycosylé.

Une préparation de polypeptide Ssol purifié et dont la concentration protéique a été déterminée par test micro-BCA, permet de réaliser une gamme étalon pour mesurer à l'aide du test ELISA-capture décrit plus haut les concentrations de Ssol présent dans les surnageants de culture et de revisiter les caractéristiques des lignées sécrétrices. Selon ce test, la lignée FRhK4-Ssol-CT3 sécrète 4 à 6 µg/ml de polypeptide Ssol tandis que la lignée FRhK4-Ssol-30 sécrète 9 à 13 µg/ml de Ssol après 4 à 5 jours de culture à confluence. En outre, le schéma de purification présenté plus haut permet en routine de purifier de 1 à 2 mg de polypeptide Ssol par litre de surnageant de culture.

### Exemple 12 : Immunisation génique visant la protéine de spicule (S) du coronavirus associé au SRAS (SRAS-CoV)

L'effet d'un signal d'épissage et des signaux post-transcriptionnels WPRE et CTE a été analysé après immunisation génique de souris BALB/c (figure 28).

Pour cela, des souris BALB/c ont été immunisées à des intervalles de 4 semaines par injection dans le *tibialis anterior* d'une solution saline de 50 µg d'ADN plasmidique de pcDNA-S et pCI-S ainsi que, à titre de contrôle, par 50 µg d'ADN plasmidique de pcDNA-N (dirigeant l'expression de la N du SRAS-CoV) ou de pCI-HA (dirigeant l'expression de la HA du virus grippal A/PR/8/34) et les sérums immuns collectés 3 semaines après la 2ème injection. La présence d'anticorps dirigés contre la S du SRAS-CoV a été recherchée par ELISA indirect en utilisant comme antigène un lysat de cellules VeroE6 infectées par le SRAS-CoV et à titre de contrôle, un lysat de cellules VeroE6 non infectées. Les titres (TI) en anticorps anti-SRAS-CoV sont calculés comme l'inverse de la dilution produisant une DO spécifique de 0,5 (différence entre DO mesurée sur lysat de cellules infectées et DO mesurée sur lysat de cellules non infectées) après révélation par un anticorps polyclonal anti-IgG de souris couplé à la peroxidase (NA931V, Amersham) et du TMB additionné de H₂O₂ (KPL) (figure 28A).

Dans ces conditions, le plasmide d'expression pcDNA-S ne permet l'induction que de faibles titres d'anticorps dirigées contre la S du SRAS-CoV chez 3 souris sur 6 (LOG₁₀(TI)=1,9±0,6) alors que le plasmide pcDNA-N permet l'induction d'anticorps anti-N à des titres élevés (LOG₁₀(TI)= 3,9±0,3) chez tous les animaux, et les plasmides contrôles (pCI, pCI-HA) n'entraînent aucun anticorps détectable (LOG₁₀(TI)<1,7). Le plasmide pCI-S muni d'un signal d'épissage permet l'induction d'anticorps à des titres élevés (LOG₁₀(TI)= 3,7±0,2), qui sont environ 60 fois supérieurs à ceux observés après injection du plasmide pcDNA-S (p<10⁻⁵).

L'efficacité des signaux post-transcriptionnels a été étudiée en réalisant une étude dose-réponse des titres en anticorps anti-S induits chez la souris BALB/c en fonction de la quantité d'ADN plasmidique utilisé comme immunogène (2µg, 10 µg et 50 µg). Cette étude (figure 28B) démontre que le signal post-transcriptionnel WPRE améliore fortement l'efficacité de l'immunisation génique lorsque de faibles doses d'ADN sont utilisées (p<10⁻⁵ pour une dose de 2 µg d'ADN et p<10⁻² pour une dose de 10 µg), alors que l'effet du signal CTE reste marginal (p=0,34 pour une dose de 2 µg d'ADN).

Enfin, les anticorps induits chez la souris après immunisation génique neutralisent l'infectivité du SRAS-CoV *in vitro* (figures 29A et 29B) à des titres qui sont en rapport avec les titres mesurés par ELISA.

En résumé, l'utilisation d'un signal d'épissage et du signal post-transcriptionnel WPRE du virus de l'hépatite de la marmotte améliore de façon considérable l'induction d'anticorps neutralisants dirigés contre le SRAS-CoV après immunisation génique à l'aide d'ADN plasmidique dirigeant l'expression du cADN de la S du SRAS-CoV.

### Exemple 13 : Applications diagnostiques de la protéine S

La réactivité en ELISA du polypeptide recombinant Ssol a été analysée vis-à-vis de sérums de patients atteints de SRAS.

Les sérums de cas probables de SRAS testés ont été choisis sur la base des résultats (positifs ou négatifs) d'analyse de leur réactivité spécifique vis-à-vis des antigènes natifs du SRAS-CoV par test d'immunofluorescence sur cellules VeroE6 infectées par le SRAS-CoV et/ou par test ELISA indirect en utilisant comme antigène un lysat de cellules VeroE6 infectées par le SRAS-CoV. Les sérums de ces patients sont identifiés par un numéro d'ordre du Centre National de Référence des Virus Influenzae ainsi que par les initiales du patient et le nombre de jours écoulés depuis le début des symptomes. Tous les sérums de cas probables (cf Tableau XI) reconnaissent les antigènes natifs du SRAS-CoV, à l'exception du sérum 032552 du patient VTT, pour lequel l'infection par le SRAS-CoV n'a pas pu être confirmée par RT-PCR réalisée sur prélèvements respiratoires des jours 3, 8 et 12. Un panel de sérums témoin a été utilisé à titre de contrôle (sérums TV): il s'agit de sérums prélevés en France avant l'épidémie de SRAS survenue en 2003.

**Tableau XII : sérums de cas probables de SRAS**

| **sérum** | **patient** | **jour de prélèvement** |
|---|---|---|
| 031724 | JYK | 7 |
| 033168 | JYK | 38 |
| 033597 | JYK | 74 |
| 032632 | NTM | 17 |
| 032634 | THA | 15 |
| 032541 | PHV | 10 |
| 032542 | NIH | 17 |
| 032552 | VTT | 8 |
| 032633 | PTU | 16 |
| 032791 | JLB | 3 |
| 033258 | JLB | 27 |
| 032703 | JCM | 8 |
| 033153 | JCM | 29 |

Des phases solides sensibilisées par le polypeptide recombinant Ssol ont été préparées par adsorption d'une solution de polypeptide Ssol purifié à 2 µg/ml en PBS dans les puits d'une plaque ELISA, puis les plaques sont incubées une nuit à 4°C et lavées avec du tampon PBS-Tween (PBS, 0,1% Tween20). Après saturation des plaques ELISA par une solution de PBS-lait écrémé à 10% (poids/volume) et lavage en PBS-tween, les sérums à tester (100 µl) sont dilués au 1/400 dans du tampon PBS-lait écrémé-Tween (PBS, 3% lait écrémé, 0,1% Tween) puis ajoutés dans les puits de la plaque ELISA sensibilisée. Les plaques sont incubées 1h à 37°C. Après 3 lavages avec du tampon PBS-Tween, le conjugué anti-IgG humaines marqué à la peroxidase (réf NA933V, Amersham) dilué au 1/4000 dans du tampon PBS-lait écrémé-Tween est ajouté, puis les plaques sont incubées une heure à 37°C. Après 6 lavages avec du tampon PBS-Tween, le chromogène (TMB) et le substrat (H₂O₂) sont ajoutés et les plaques sont incubées 10 minutes à l'abri de la lumière. La réaction est arrêtée par ajout d'une solution 1N de H₃PO₄, puis l'absorbance est mesurée à 450 nm avec un référence à 620nm.

Les tests ELISA (figure 30) démontrent que le polypeptide recombinant Ssol est reconnu spécifiquement par les anticorps sériques de patients atteints de SRAS prélevés en phase moyenne ou tardive de l'infection (≥ 10 jours après le début des symptômes alors qu'il n'est pas reconnu de façon significative par les anticorps sériques de deux patients (JLB et JCM) prélevés en phase précoce de l'infection (3 à 8 jours après le début des symptômes) ni par des sérums témoins de sujets non atteints de SRAS. Les anticorps sériques des patients JLB et JCM montrent une séroconversion entre les jours 3 et 27 pour le premier et 8 et 29 pour le second après le début des symptômes, ce qui confirme la spécificité de la réactivité de ces sérums vis-à-vis du polypeptide Ssol.

En conclusion, ces résultats démontrent que le polypeptide recombinant Ssol peut être utilisé comme antigène pour la mise au point d'un test ELISA de diagnostic sérologique de l'infection par le SRAS-CoV.

### Exemple 14 : Applications vaccinales de la protéine S soluble recombinante

L'immunogénicité du polypeptide recombinant Ssol a été étudiée chez la souris.

Pour cela, un groupe de 6 souris a été immunisé à 3 semaines d'intervalle avec 10 µg de polypeptide recombinant Ssol adjuvé par 1 mg d'hydroxyde d'aluminium (Alu-gel-S, Serva) dilué en PBS. Trois immunisations successives ont été réalisées et les sérums immuns ont été prélevés 3 semaines après chacune des immunisations (IS1, IS2, IS3). A titre de contrôle, un groupe de souris (groupe mock) a reçu de l'hydroxide d'aluminium seul selon le même protocole.

Les sérums immuns ont été analysés par pool pour chacun des 2 groupes par ELISA indirect en utilisant un lysat de cellules VeroE6 infectées par le SRAS-CoV comme antigène et à titre de contrôle, un lysat de cellules VeroE6 non infectées. Les titres en anticorps anti-SRAS-CoV sont calculés comme l'inverse de la dilution produisant une DO spécifique de 0,5 après révélation par un anticorps polyclonal anti-IgG(H+L) de souris couplé à la peroxidase (NA931V, Amersham) et du TMB additionné de H₂O₂ (KPL). Cette analyse (figure 31) montre que l'immunisation par le polypeptide Ssol induit chez la souris dès la première immunisation des anticorps dirigés contre la forme native de la protéine de spicule du SRAS-CoV présente dans le lysat de cellules VeroE6 infectées. Après 2 puis 3 immunisations, les titres en anticorps anti-S deviennent très élevés.

Les sérums immuns ont été analysés par pool pour chacun des 2 groupes pour leur capacité à séroneutraliser l'infectivité du SRAS-CoV. 4 points de séroneutralisation sur cellules FRhK-4 (100 TCID50 de SRAS-CoV) sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4. Cette analyse montre que les anticorps induits chez la souris par le polypeptide Ssol sont neutralisants : les titres observés sont très élevés après 2 puis 3 immunisations (supérieurs à 2560 et 5120 respectivement, Tableau XIII).

**Tableau XIII : induction d'anticorps dirigés contre le SRAS-CoV après immunisation avec le polypeptide recombinant Ssol. Les sérums immuns ont été analysés par pool pour chacun des 2 groupes pour leur capacité à séroneutraliser l'infectivité de 100 TCID50 du SRAS-CoV sur cellules FRhK-4. 4 points sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4.**

| **groupe** | **sérums** | **Ac. Neutralisants** |
|---|---|---|
| Mock | pi | < 20 |
| | IS1 | < 20 |
| | IS2 | < 20 |
| | IS3 | < 20 |
| Ssol | pi | < 20 |
| | IS1 | 57 |
| | IS2 | > 2560 |
| | IS3 | > 5120 |

Les titres neutralisants observés chez les souris immunisées avec le polypeptide Ssol atteignent des niveaux très supérieurs aux titres observés par Yang et coll. chez la souris (2004, Nature, 428: 561-564) et à ceux observés par Buchholz chez le hamster (2004, PNAS 101 : 9804-9809), qui protègent respectivement la souris et le hamster de l'infection par le SRAS-CoV. Il est donc vraisemblable que les anticorps neutralisants induits chez la souris après immunisation par le polypeptide Ssol protègent ces animaux contre l'infection par le SRAS-CoV.

### Exemple 15 : Gène synthétique optimisé pour l'expression en cellules de mammifères de la protéine de spicule (S) du coronavirus associé au SRAS (SRAS-CoV).

### 1) Conception du gène synthétique

Un gène synthétique codant pour la protéine de spicule du SRAS-CoV a été conçu à partir du gène de l'isolat 031559 (plasmide pSARS-S, C.N.C.M. n° 1-3059) de façon à permettre des niveaux d'expression élevés dans des cellules de mammifères et en particulier dans les cellules d'origine humaine.

Pour cela :
- l'usage des codons du gène sauvage de l'isolat 031589 a été modifié de façon à se rapprocher du biais observé chez l'homme et à améliorer l'efficacité de traduction du mRNA correspondant
- le contenu global en GC du gène a été augmenté de façon à prolonger la demi-vie du mRNA correspondant
- les motifs, éventuellement cryptiques, susceptibles d'interférer avec une expression efficace du gène ont été supprimés (sites donneurs et accepteurs d'épissage, signaux de polyadénylation, séquences très riches (>80%) ou très pauvres (<30%) en GC, séquences répétées, séquences impliquées dans la formation de structures secondaires de l'ARN, boites TATA)
- un deuxième codon STOP a été ajouté pour permettre une terminaison efficace de la traduction.

En outre, des motifs CpG ont été introduits dans le gène de façon à augmenter son immunogénicité comme vaccin à ADN. Afin de faciliter la manipulation du gène synthétique, deux sites de restriction BamH1 et Xho1 ont été placés de part et d'autre de la phase ouverte de lecture de la protéine S, et les sites de restriction BamH1, Xho1, Nhe1, Kpn1, BspE1 et Sall ont été évités dans le gène synthétique.

La séquence du gène synthétique conçu (gène 040530) est donnée en SEQ ID No: 140.

Un alignement du gène synthétique 040530 avec la séquence du gène sauvage de l'isolat 031589 du SRAS-CoV déposé à la C.N.CM. sous le numéro 1-3059 (SEQ ID NO : 4, plasmide pSRAS-S) est présenté dans la figure 32.

### 2) Constructions plasmidiques

Le gène synthétique SEQ ID No: 140 a été assemblé à partir d'oligonucléotides synthétiques et cloné entre les sites Kpn1 et Sac1 du plasmide pUC-Kana pour donner le plasmide 040530pUC-kana. La séquence nucléotidique de l'insert du plasmide 040530pUC-kana a été vérifiée par séquençage automatique (Applied).

Un fragment Kpn1-Xho1 contenant le gène synthétique 040530 a été excisé du plasmide 040530pUC-kana et sous-clone entre les sites Nhe1 et Xho1 du plasmide d'expression pCI (Promega) pour obtenir le plasmide pCI-SSYNTH, déposé à la CNCM le 1^{er} décembre 2004, sous le numéro I-3333.

Un gène synthétique codant pour la forme soluble de la protéine S a ensuite été obtenu en fusionnant les séquences synthétiques codant pour l'ectodomaine de la protéine S (acides aminés 1 à 1193) à celles de l'étiquette (FLAG : DYKDDDDK) via un linker BspE1 codant pour le dipeptide SG. Pratiquement, un fragment d'ADN codant pour l'ectodomaine de la S du SRAS-CoV a été amplifié par PCR à l'aide des oligonucléotides 5'-ACTAGCTAGC GGATCCACCA TGTTCATCTT CCTG -3' et 5'- AGTATCCGGAC TTGATGTACT GCTCGTACTT GC-3' à partir du plasmide 040530pUC-kana, digéré par Nhe1 et BspE1 puis inséré entre les sites uniques Nhe1 et BspE1 du plasmide pCI-Ssol, pour donner le plasmide pCI-SCUBE, déposé à la CNCM le 1^{er} décembre 2004, sous le numéro I-3332. (Les plasmides pCI-Ssol, pCI-Ssol-CTE et pCI-Ssol-WPRE (déposé à la CNCM, le 22 novembre 2004, sous le numéro 1-3324) avaient été précédemment obtenus par sous-clonage du fragment Kpn1-Xho1 excisé du plasmide pcDNA-Ssol (voir note technique de la DI 2004-106) entre les sites Nhe1 et Xho1 des plasmides pCI, pCI-S-CTE et pCI-S-WPRE respectivement.)

Les plasmides pCI-Scube et pCI-Ssol codent pour le même polypeptide recombinant Ssol.

### 3) Résultats

La capacité du gène synthétique codant pour la protéine S à diriger efficacement l'expression de la S du SRAS-CoV dans des cellules de mammifères a été comparée à celle du gène sauvage après transfection transitoire de cellules de primates (VeroE6) et de cellules humaines (293T).

Dans l'expérience présentée par la figure 33 et au Tableau XIV, des monocouches de 5x10⁵ cellules VeroE6 ou 7x10⁵ cellules 293T en boites de Pétri de 35mm ont été transfectées avec 2 µg des plasmides pCI (à titre de contrôle), pCI-S, pCI-S-CTE, pCI-S-WPRE et pCI-Ssynth et 6 µl de réactif Fugene6 selon les indications du fabricant (Roche). Après 48 heures d'incubation à 37°C et sous 5% de CO₂, des extraits cellulaires ont été préparés en tampon de dépôt selon Laemmli, séparés sur un gel SDS à 8% de polyacrylamide puis transférés sur une membrane de PVDF (BioRad). La détection de cette immunoempreinte (« western blot ») a été réalisée à l'aide d'un sérum polyclonal de lapin anti-S (immun-sérum du lapin P11135 : cf exemple 4 ci-dessus) et d'anticorps polyclonaux d'âne dirigés contre les IgG de lapin et couplés à la peroxidase (NA934V, Amersham). L'immunoempreinte est révélée de façon quantitative par luminescence à l'aide du kit ECL+ (Amersham) et acquisition sur un dispositif d'imagerie numérique (FluorS, BioRad).

L'analyse des résultats obtenus avec le logiciel QuantityOne v4.2.3 (BioRad) montre que dans cette expérience, le plasmide pCI-Synth permet l'expression transitoire de la protéine S à des niveaux élevés dans les cellules VeroE6 et 293T, alors que le plasmide pCI-S ne permet pas d'induire une expression à des niveaux suffisants pour être détectée. Les niveaux d'expression observés sont de l'ordre de 2 fois supérieurs à ceux observés avec le plasmide pCI-S-WPRE.

**Tableau XIV : utilisation d'un gène synthétique pour l'expression de la S du SRAS-CoV. Des extraits cellulaires préparés 48 heures après transfection de cellulesVeroE6 ou 293T par les plasmides pCI, pCI-S, pCI-S-CTE, pCI-S-WPRE et pCI-Ssynth ont été séparés sur un gel SDS à 8% d'acrylamide et analysés par western blot à l'aide d'un anticorps polyclonal de lapin anti-S et d'un anticorps polyclonal anti-IgG(H+L) de lapin couplé à la peroxidase (NA934V, Amersham). Le western blot est révélé par luminescence (ECL+, Amersham) et acquisition sur un dispositif d'imagerie numérique (FluorS, BioRad). Les niveaux d'expression de la protéine S ont été mesurés en quantifiant les 2 bandes majoritaires repérées sur l'image (voir figure 33) et sont indiqués en fonction d'une échelle arbitraire où la valeur de 1 représente le niveau mesuré après transfection du plasmide pCI-S-WPRE.**

| **plasmide** | **VeroE6** | **293T** |
|---|---|---|
| **pCI** | 0,0 | 0,0 |
| **pCI-S** | ≤ 0,1 | ≤ 0,1 |
| **pCI-S-CTE** | 0,5 | ≤ 0,1 |
| **pCI-S-WPRE** | 1,0 | 1,0 |
| **PCI-Ssynth** | 1,8 | 1,9 |

Dans un second temps, la capacité du gène synthétique Scube à diriger efficacement la synthèse et la sécrétion du polypeptide Ssol par des cellules de mammifères a été comparée à celle du gène sauvage après transfection transitoire de cellules de hamster (BHK-21) et de cellules humaines (293T).

Dans l'expérience présentée par le Tableau XV, des monocouches de 6x10⁵ cellules BHK-21 et de 7x10⁵ cellules 293T en boites de Pétri de 35mm ont été transfectées avec 2 µg des plasmides pCI (à titre de contrôle), pCI-Ssol, pCI-Ssol-CTE, pCI-Ssol-WPRE et pCI-Scube et 6 µl de réactif Fugene6 selon les indications du fabricant (Roche). Après 48 heures d'incubation à 37°C et sous 5% de CO2, les surnageants cellulaires ont été prélevés et analysés de façon quantitative pour la sécrétion du polypeptide Ssol par un test ELISA-capture spécifique du polypeptide Ssol.

L'analyse des résultats montre que, dans cette expérience, le plasmide pCI-Scube permet l'expression du polypeptide Ssol à des niveaux 8 fois (cellules BHK-21) à 20 fois (cellules 293T) plus élevés que le plasmide pCI-Ssol. Les niveaux d'expression observés sont de l'ordre de 2 fois (cellules 293T) à 5 fois (cellules BHK-21) supérieurs à ceux observés avec le plasmide pCI-Ssol-WPRE.

**Tableau XV : utilisation d'un gène synthétique pour l'expression du polypeptide Ssol. Les surnageants ont été récoltés 48 heures après transfection de cellules BHK ou 293T par les plasmides pCI, pCI-Ssol, pCI-Ssol-CTE, pCI-Ssol-WPRE et pCI-Scube et analysés de façon quantitative pour la sécrétion du polypeptide Ssol par un test ELISA-capture spécifique du polypeptide Ssol. Les transfections ont été réalisées en dupliqués et les résultats sont indiqués sous la forme de moyennes et écart-types des concentrations de polypeptide Ssol (ng/ml) mesurées dans les surnageants.**

| **Plasmide** | **BHK** | **293T** |
|---|---|---|
| **Pci** | < 20 | < 20 |
| **pCI-Ssol** | < 20 | 56 ± 10 |
| **pCI-Ssol-CTE** | < 20 | 63 ± 8 |
| **pCI-Ssol-WPRE** | 28 ± 1 | 531 ± 15 |
| **PCI-Scube** | 152 ± 6 | 1140 ± 20 |

En résumé, ces résultats montrent que l'expression dans des cellules de mammifères du gène synthétique 040530 codant pour la S du SRAS-CoV sous la dépendance de séquences promotrices de l'ARN polymérase II est bien plus efficace que celle du gène sauvage de l'isolat 031589. Cette expression est même plus efficace que celle dirigée par le gène sauvage en présence des séquences WPRE du virus de l'hépatite de la marmotte.

### 4) Applications

L'utilisation du gène synthétique 040530 codant pour la S du SRAS-CoV ou de sa variante Scube codant pour le polypeptide Ssol est susceptible de remplacer avantageusement le gène sauvage dans de nombreuses applications où l'expression de la S est nécessaire à des niveaux élevés. En particulier pour :
- améliorer l'efficacité de l'immunisation génique par des plasmides du type pCI-Ssynth voire pCI-Ssynth-CTE ou pCI-Ssynth-WPRE
- établir de nouvelles lignées cellulaires exprimant des quantités plus élevées de la protéine S ou du polypeptide Ssol à l'aide de vecteurs lentiviraux recombinants porteurs du gène Ssynth ou du gène Scube respectivement
- améliorer l'immunogénicité des vecteurs lentiviraux recombinants permettant l'expression de la protéine S ou du polypeptide Ssol
- améliorer l'immunogénicité de vecteurs vivants permettant l'expression de la protéine S ou du polypeptide Ssol comme des virus recombinants de la vaccine ou des virus rougeole recombinants (voir exemples 16 et 17 ci-après)

### Exemple 16 : Expression de la protéine de spicule (S) du coronavirus associé au SRAS (SRAS-CoV) à l'aide de virus recombinants de la Vaccine.

### Application vaccinale.

**Application à la production d'une forme soluble de la protéine de spicule (S) et conception d'un test de sérologie du SRAS.**

### 1) Introduction

Le but de cet exemple est d'évaluer la capacité de virus recombinants de la vaccine (VV) exprimant différents antigènes du coronavirus associé au SRAS (SRAS-CoV) à constituer de nouveaux candidats vaccins contre le SRAS et un moyen de produire des antigènes recombinants en cellules de mammifères.

Pour cela, les inventeurs se sont intéressés à la protéine de spicule (S) du SRAS-CoV, qui permet d'induire après immunisation génique chez l'animal des anticorps neutralisants l'infectivité du SRAS-CoV, ainsi qu'à une forme soluble et sécrétée de cette protéine, le polypeptide Ssol, qui est composé de l'ectodomaine (aa 1-1193) de la S fusionné en son extrémité C-ter à une étiquette FLAG (DYKDDDDK) via un linker BspE1 codant pour le dipeptide SG. Ce polypeptide Ssol présente une antigénicité similaire à celle de la protéine S et permet, après injection à la souris sous la forme d'une protéine purifiée adjuvée en hydroxide d'aluminium, l'induction de titres élevés d'anticorps neutralisants contre le SRAS-CoV.

Les différentes formes du gène S ont été placées sous la dépendance du promoteur du gène 7.5K puis introduites au sein du locus de la thymidine kinase (TK) de la souche Copenhague du virus de la vaccine par double recombinaison homologue *in vivo.* Afin d'améliorer l'immunogénicité des virus vaccine recombinants, un promoteur tardif synthétique a été choisi à la place du promoteur 7.5K, pour augmenter la production de S et Ssol au cours des phases tardives du cycle viral.

Après avoir isolé les virus vaccine recombinants et vérifié leur capacité à exprimer l'antigène S du SRAS-CoV, leur capacité à induire chez la souris une réponse immunitaire contre le SRAS a été testée. Après avoir purifié l'antigène Ssol du surnageant de cellules infectées, un test ELISA de sérodiagnostic du SRAS a été conçu, et son efficacité a été évaluée à l'aide de sérums de cas probables de SRAS.

### 2) Construction des virus recombinants

Des virus recombinants de la vaccine dirigeant l'expression de la glycoprotéine S de l'isolat 031589 du SRAS-CoV et d'une forme soluble et sécrétée de cette protéine, le polypeptide Ssol, sous la dépendance du promoteur 7.5K ont été obtenus. Dans le but d'augmenter les niveaux d'expression de S et Ssol, des virus recombinants dans lesquels les cDNA de S et de Ssol sont placés sous la dépendance d'un promoteur synthétique tardif ont également été obtenus.

Le plasmide pTG186poly est un plasmide de transfert pour la construction de virus recombinants de la vaccine (Kieny, 1986, Biotechnology, 4 :790-795). A ce titre, il contient le gène de la thymidine kinase du VV dans lequel a été inséré le promoteur du gène 7.5K suivi d'un site multiple de clonage permettant l'insertion de gènes hétérologues (figure 34A). Le promoteur du gène 7.5K contient en fait un tandem de deux séquences promotrices actives respectivement durant les phases précoces (P_{E}) et tardives (P_{L}) du cycle de réplication du virus de la vaccine. Les fragments BamH1-Xho1 ont été excisés des plasmides pTRIP-S et pcDNA-Ssol respectivement et insérés entre les sites BamH1 et Sma1 du plasmide pTG186poly pour donner les plasmides pTG-S et pTG-Ssol (figure 34A). Les plasmides pTG-S et pTG-Ssol ont été déposés à la CNCM, le 2 décembre 2004, sous les numéros I-3338 et I-3339, respectivement.

Les plasmides pTN480, pTN-S et pTN-Ssol ont été obtenus à partir des plasmides pTG186poly, pTG-S et pTG-Ssol respectivement, en substituant le fragment Nde1-Pst1 contenant le promoteur 7.5K par un fragment d'ADN contenant le promoteur tardif synthétique 480, qui a été obtenu par hybridation des oligonucléotides 5'- TATGAGCTTT TTTTTTTTTT TTTTTTTGGC ATATAAATAG ACTCGGCGCG CCATCTGCA-3' et 5'- GATGGCGCGC CGAGTCTATT TATATGCCAA AAAAAAAAAA AAAAAAAAGC TCA-3' (figure 34B). L'insert a été séquencé à l'aide d'un kit BigDye Terminator v1.1 (Applied Biosystems) et d'un séquenceur automatique ABI377. La séquence du promoteur synthétique tardif 480 tel que cloné dans les plasmides de transfert de la série pTN est indiquée figure 34C. Les plasmides pTN-S et pTN-Ssol ont été déposés à la CNCM, le 2 décembre 2004, sous les numéros 1-3340 et I-3341, respectivement.

Les virus recombinants de la vaccine ont été obtenus par double recombinaison homologue *in vivo* entre la cassette TK des plasmides de transfert des séries pTG et pTN et le gène TK de la souche Copenhague du virus de la vaccine selon une procédure décrite par Kieny et coll. (1984, Nature, 312 : 163-166). Brièvement, des cellules CV-1 sont transfectées à l'aide de DOTAP (Roche) par de l'ADN génomique de la souche copenhague du virus de la vaccine et chacun des plasmides de transfert des séries pTG et pTN décrits ci-dessus, puis surinfectées par le virus vaccine auxiliaire VV-ts7 pendant 24 heures à 33°C. Le virus auxiliaire est contre-sélectionné par incubation à 40°C pendant 2 jours puis les virus recombinants (phénotype TK-) sélectionnés par deux cycles de clonage sous milieu gélosé sur cellules 143Btk- en présence de BuDr (25 µg/ml). Les 6 virus VV-TG, VV-TG-S, VV-TG-Ssol, VV-TN, VV-TN-S et VV-TN-Ssol ont été respectivement obtenus à l'aide des plasmides de transfert pTG186poly, pTG-S, pTG-Ssol, pTN480, pTN-S, pTN-Ssol. Les virus VV-TG et VV-TN n'expriment aucun gène hétérologue et ont été utilisés comme contrôle TK- dans les expériences. Les préparations de virus recombinants ont été réalisés sur monocouches de cellules CV-1 ou BHK-21 et le titre en unités formant plage (u.f.p.) déterminé sur cellules CV-1 selon Earl et Moss (1998, Current Protocols in Molecular Biology, 16.16.1-16.16.13).

### 3) Caractérisation des virus recombinants

L'expression des transgènes codant la protéine S et le polypeptide Ssol a été recherchée par western blot.

Des monocouches de cellules CV-1 ont été infectées à une multiplicité de 2 par les différents virus vaccine recombinants VV-TG, VV-TG-S, VV-TG-Ssol, VV-TN, VV-TN-S et VV-TN-Ssol. Après 18 heures d'incubation à 37°C et sous 5% de CO2, des extraits cellulaires ont été préparés en tampon de dépôt selon Laemmli, séparés sur un gel SDS à 8% de polyacrylamide puis transférés sur une membrane de PVDF (BioRad). La détection de cette immunoempreinte (« western blot ») a été réalisée à l'aide d'un sérum polyclonal de lapin anti-S (immun-sérum du lapin P11135 : cf exemple 4) et d'anticorps polyclonaux d'âne dirigés contre les IgG de lapin et couplés à la peroxidase (NA934V, Amersham). Les anticorps fixés ont été révélés par luminescence à l'aide du kit ECL+ (Amersham) et de films d'autoradiographie Hyperfilm MP (Amersham).

Comme le montre la figure 35A, le virus recombinant VV-TN-S dirige l'expression de la protéine S à des niveaux qui sont comparables à ceux que l'on peut observer 8h après infection par le SRAS-CoV mais qui sont bien plus élevés que ceux que l'on peut observer après infection par VV-TG-S. Dans une deuxième expérience (figure 35B), l'analyse de quantités variables d'extraits cellulaires montre que les niveaux d'expression observés après infection par les virus de la série TN (VV-TN-S et VV-TN-Ssol) sont environ 10 fois plus élevés que ceux observés avec les virus de la série TG (VV-TG-S et W-TG-Ssol respectivement). En outre, le polypeptide Ssol est sécrété dans le surnageant de cellules CV-1 infectées par le virus VV-TN-Ssol plus efficacement que dans le surnageant de cellules infectées par VV-TG-Ssol (figure 36A). Dans cette expérience, le virus VV-TN-Sflag a été utilisé à titre de contrôle, car il exprime la forme membranaire de la protéine S fusionnée en son extrémité C-ter à l'étiquette FLAG. La protéine Sflag n'est pas détectée dans le surnageant des cellules infectées par VV-TN-Sflag, démontrant que le polypeptide Ssol est bien sécrété de façon active après infection par W-TN-Ssol.

Ces résultats démontrent que les virus vaccine recombinants sont bien porteurs des transgènes et permettent l'expression de la glycoprotéine du SRAS sous sa forme membranaire (S) ou sous une forme soluble et sécrétée (Ssol). Les virus vaccine porteurs du promoteur synthétique 480 permettent l'expression de S et la sécrétion de Ssol à des niveaux bien plus élevés que les virus porteurs du promoteur du gène 7.5K.

### 4) Application à la production d'une forme soluble de la S du SRAS-CoV.

### Purification de cet antigène recombinant et applications diagnostiques.

La lignée BHK-21 est la lignée cellulaire qui sécrète les quantités les plus élevées de polypeptide Ssol après infection par le virus VV-TN-Ssol parmi les lignées testées (BHK-21, CV1, 293T et FrhK-4, figure 36B) ; elle permet la production et la purification en quantité du polypeptide recombinant Ssol. Dans une expérience type où les conditions expérimentales d'infection, de production et de purification ont été optimisées, les cellules BHK-21 sont ensemencées en milieu de culture standard (DMEM sans pyruvate contenant 4.5g/l de glucose et supplémenté par 5% de TPB, 5% de SVF, 100 U/ml de pénicilline et 100 µg/ml de streptomycine) sous la forme d'une monocouche sous-confluente (10 millions de cellules pour chaque 100 cm² dans 25 ml de milieu). Après 24h d'incubation à 37°C sous 5% de CO₂, les cellules sont infectées à une M.O.I. de 0.03 et le milieu standard remplacé par le milieu de sécrétion où la quantité de SVF est abaissée à 0.5% et le TPB supprimé. Le surnageant de culture est prélevé après 2,5 jours d'incubation à 35°C et sous 5% de CO₂ et le virus de la vaccine inactivé par addition de triton X-100 (0,1%). Après filtration sur membrane de polyethersulfone (PES) de 0.1 µm, le polypeptide recombinant Ssol est purifié par une chromatographie d'affinité sur matrice anti-FLAG avec élution par une solution de peptide FLAG (DYKDDDDK) à 100 µg/ml en TBS (Tris 50mM pH 7.4, 150 mMNaCl).

L'analyse par gel SDS à 8 % d'acrylamide coloré au nitrate d'argent a mis en évidence un polypeptide majoritaire dont la masse moléculaire est d'environ 180kD et dont le degré de pureté est supérieur à 90% (figure 37).La concentration du polypeptide recombinant Ssol purifié a été déterminée par comparaison avec les marqueurs de masse moléculaire et estimée à 24 ng/µl.

Cette préparation de polypeptide Ssol purifié permet de réaliser une gamme étalon pour mesurer à l'aide d'un test ELISA-capture les concentrations de Ssol présentes dans les surnageants de culture. Selon ce test, la lignée BHK-21 sécrète environ 1 µg/ml de polypeptide Ssol dans les conditions de production décrites plus haut. En outre, le schéma de purification présenté permet de purifier de l'ordre de 160 µg de polypeptide Ssol par litre de surnageant de culture.

La réactivité en ELISA du polypeptide recombinant Ssol a été analysée vis-à-vis de sérums de patients atteints de STRAS.

Les sérums de cas probables de SRAS testés ont été choisis sur la base des résultats (positifs ou négatifs) d'analyse de leur réactivité spécifique vis à vis des antigènes natifs du SRAS-CoV par test d'immunofluorescence sur cellules VeroE6 infectées par le SRAS-CoV et/ou par test ELISA indirect en utilisant comme antigène un lysat de cellules VeroE6 infectées par le SRAS-CoV. Les sérums de ces patients sont identifiés par un numéro d'ordre du Centre National de Référence des Virus Influenzae ainsi que par les initiales du patient et le nombre de jours écoulés depuis le début des symptômes. Tous les sérums de cas probables (cf Tableau XVI) reconnaissent les antigènes natifs du SRAS-CoV à l'exception du sérum 032552 du patient VTT, pour lequel l'infection par le SRAS-CoV n'a pas pu être confirmée par RT-PCR réalisée sur prélèvements respiratoires des jours 3, 8 et 12. Un panel de sérums témoin a été utilisé à titre de contrôle (sérums TV): il s'agit de sérums prélevés en France avant l'épidémie de SRAS survenue en 2003.

**Tableau XVI : sérums de cas probables de SRAS**

| **sérum** | **patient** | **jour de prélèvement** |
|---|---|---|
| 033168 | JYK | 38 |
| 033597 | JYK | 74 |
| 032632 | NTM | 17 |
| 032634 | THA | 15 |
| 032541 | PHV | 10 |
| 032542 | NIH | 17 |
| 032552 | VTT | 8 |
| 032633 | PTU | 16 |

Des phases solides sensibilisées par le polypeptide recombinant Ssol ont été préparées par adsorption d'une solution de polypeptide Ssol purifié à 4 µg/ml en PBS dans les puits d'une plaque ELISA. Les plaques sont incubées une nuit à 4°C puis lavées avec du tampon PBS-Tween (PBS, 0,1% Tween20). Après lavage en PBS-tween, les sérums à tester (100 µl) sont dilués au 1/100 et 1/400 dans du tampon PBS-lait écrémé-Tween (PBS, 3% lait écrémé, 0,1% Tween) puis ajoutés dans les puits de la plaque ELISA sensibilisée. Les plaques sont incubées 1h à 37°C. Après 3 lavages avec du tampon PBS-Tween, le conjugué anti-IgG humaines marqué à la peroxidase (réf NA933V, Amersham) dilué au 1/4000 dans du tampon PBS-lait écrémé-Tween est ajouté puis les plaques sont incubées une heure à 37°C. Après 6 lavages avec du tampon PBS-Tween, le chromogène (TMB) et le substrat (H₂O₂) sont ajoutés et les plaques sont incubées 10 minutes à l'abri de la lumière. La réaction est arrêtée par ajout d'une solution 1M de H₃PO₄ puis l'absorbance est mesurée à 450 nm avec une référence à 620nm.

Les tests ELISA (figure 38) démontrent que le polypeptide recombinant Ssol est reconnu spécifiquement par les anticorps sériques de patients atteints de SRAS prélevés en phase moyenne ou tardive de l'infection (≥ 10 jours après le début des symptômes), alors qu'il n'est pas reconnu de façon significative par les anticorps sériques des sérums témoins de sujets non atteints de SRAS.

En conclusion, ces résultats démontrent que le polypeptide recombinant Ssol peut être purifié à partir du surnageant de cellules de mammifères infectées par le virus vaccine recombinant VV-TN-Ssol et être utilisé comme antigène pour la mise au point d'un test ELISA de diagnostic sérologique de l'infection par le SRAS-CoV.

### 5) Applications vaccinales

L'immunogénicité des virus vaccine recombinants a été étudiée chez la souris.

Pour cela, des groupes de 7 souris BALB/c ont été immunisés par voie i.v. à deux reprises à 4 semaines d'intervalle par 10⁶ u.f.p. de virus vaccine recombinants VV-TG, VV-TG-S, VV-TG-Ssol, VV-TN, VV-TN-S et VV-TN-Ssol ainsi que, à titre de contrôle, VV-TG-HA qui dirige l'expression de l'hémagglutinine de la souche A/PR/8/34 du virus de la grippe. Les sérums immuns ont été prélevés 3 semaines après chacune des immunisations (IS1, IS2).

Les sérums immuns ont été analysés par pool pour chacun des groupes par ELISA indirect en utilisant un lysat de cellules VeroE6 infectées par le SRAS-CoV comme antigène et à titre de contrôle, un lysat de cellules VeroE6 non infectées. Les titres (TI) en anticorps anti-SRAS-CoV sont calculés comme l'inverse de la dilution produisant une DO spécifique de 0,5 après révélation par un anticorps polyclonal anti-IgG(H+L) de souris couplé à la peroxidase (NA931 V, Amersham) et du TMB additionné de H₂O₂ (KPL). Cette analyse (figure 39A) montre que l'immunisation par le virus VV-TG-S et VV-TN-S induit chez la souris dès la première immunisation des anticorps dirigés contre la forme native de la protéine de spicule du SRAS-CoV présente dans le lysat de cellules VeroE6 infectées. Les réponses induites par le virus VV-TN-S sont plus élevées que celles induites par le virus VV-TG-S après la première (TI=740 et TI=270 respectivement) et la deuxième (TI=3230 et TI=600 respectivement) immunisation. Le virus VV-TN-Ssol induit de forts titres d'anticorps anti-SRAS-CoV après deux immunisations (TI=640), alors que le virus VV-TG-Ssol induit une réponse à la limite de la détection (TI=40).

Les sérums immuns ont été analysés par pool pour chacun des groupes pour leur capacité à séroneutraliser l'infectivité du SRAS-CoV. 4 points de séroneutralisation sur cellules FRhK-4 (100 TCID50 de SRAS-CoV) sont réalisés pour chacune des dilutions de raison 2 testées à partir du 1/20. Le titre séroneutralisant est calculé selon la méthode de Reed et Munsch comme l'inverse de la dilution neutralisant l'infectivité de 2 cupules sur 4. Cette analyse montre que les anticorps induits chez la souris par les virus vaccine exprimant la protéine S ou le polypeptide Ssol sont neutralisants et que les virus à promoteurs synthétiques sont des immunogènes plus efficaces que les virus porteurs du promoteur 7.5K : les titres les plus élevés (640) sont observés après 2 immunisations par le virus VV-TN-S (figure 39B).

Le pouvoir protecteur des anticorps neutralisants induits chez la souris après immunisation par les virus vaccine recombinants est évalué à l'aide d'une infection d'épreuve par le SRAS-CoV.

### 6) Autres applications

Des virus vaccine recombinants de troisième génération sont construits en substituant les séquences sauvages des gènes S et Ssol par des gènes synthétiques optimisés pour l'expression en cellules de mammifères, décrits ci-dessus. Ces virus vaccine recombinants sont susceptibles d'exprimer des quantités plus importantes des antigènes S et Ssol et donc de présenter une immunogénicité accrue.

Le virus vaccine recombinant VV-TN-Ssol peut être utilisé pour la production en quantité et la purification de l'antigène Ssol en vue d'applications diagnostiques (sérologie par ELISA) et vaccinales (vaccin sous-unitaire).

### Exemple 17 : Virus recombinant de la rougeole exprimant la protéine de spicule (S) du coronavirus associé au SRAS (SRAS-CoV). Applications vaccinales.

### 1) Introduction

Le vaccin rougeole (MV) induit chez l'homme une immunité protectrice de longue durée après une seule injection (Hilleman, 2002, Vaccine, 20 : 651-665). La protection conférée est très robuste et repose sur l'induction d'une réponse en anticorps et d'une réponse cellulaire CD4 et CD8. Le génome du MV est très stable et aucune réversion vers la virulence des souches vaccinales n'a jamais été observée. Le virus de la rougeole appartient au genre des *Morbillivirus* de la famille des *Paramyxoviridae ;* c'est un virus enveloppé dont le génome est un ARN monocaténaire de polarité négative de 16kb (figure 40A) et dont le cycle de réplication exclusivement cytoplasmique exclut toute possibilité d'intégration dans le génome de l'hôte. Le vaccin rougeole est ainsi l'un des vaccins vivants les plus efficaces et les plus sûrs utilisés dans la population humaine. L'équipe de Frédéric Tangy a développé récemment un vecteur d'expression sur la base de la souche Schwarz du virus de la rougeole, qui est la souche atténuée la plus sûre et la plus utilisée chez l'homme comme vaccin contre la rougeole. Cette souche vaccinale peut être isolée à partir d'un clone moléculaire infectieux en conservant son immunogénicité chez les primates ainsi que chez la souris susceptible à l'infection. Elle constitue, après insertion d'unités de transcription supplémentaires, un vecteur pour l'expression de séquences hétérologues (Combredet, 2003, J. Virol. 77 : 11546-11554). En outre, un MV Schwarz recombinant exprimant la glycoprotéine d'enveloppe du virus West Nile (WNV) induit une réponse en anticorps efficace et de longue durée qui protège la souris d'une infection d'épreuve létale par le WNV (Despres et al, 2004, J. Infect. Dis., sous presse). Toutes ces caractéristiques font de la souche atténuée Schwarz du virus de la rougeole un candidat vecteur extrêmement prometteur pour la construction de nouveaux vaccins vivants recombinants.

Le but de cet exemple est d'évaluer la capacité de virus recombinants de la rougeole (MV) exprimant différents antigènes du coronavirus associé au SRAS (SRAS-CoV) à constituer de nouveaux candidats vaccins contre le SRAS.

Les inventeurs se sont intéressés à la protéine de spicule (S) du SRAS-CoV, qui permet d'induire après immunisation génique chez l'animal des anticorps neutralisants l'infectivité du SRAS-CoV, ainsi qu'à une forme soluble et sécrétée de cette protéine, le polypeptide Ssol, qui est composé de l'ectodomaine (aa 1-1193) de la S fusionné en son extrémité C-ter à une étiquette FLAG (DYKDDDDK) via un linker BspE1 codant pour le dipeptide SG. Ce polypeptide Ssol présente une antigénicité similaire à celle de la protéine S et permet, après injection à la souris sous la forme d'une protéine purifiée adjuvée en hydroxide d'aluminium, l'induction de titres élevés d'anticorps neutralisants contre le SRAS-CoV.

Les différentes formes du gène S ont été introduites sous la forme d'une unité de transcription supplémentaire entre les gènes P (phosphoprotéine) et M (matrice) dans le cADN de la souche Schwarz du MV précédemment décrite (Combredet, 2003, J. Virol. 77 : 11546-11554 ; Demande EP N° 02291551.6 du 20 juin 2002, et Demande EP n° 02291550.8 du 20 juin 2002). Après avoir isolé les virus recombinants MVSchw2-SARS-S et MVSchw2-SARS-Ssol et vérifié leur capacité à exprimer l'antigène S du SRAS-CoV, leur capacité à induire chez la souris puis chez le singe une réponse immunitaire protectrice contre le SRAS est testée.

### 2) Construction des virus recombinants

Le plasmide pTM-MVSchw-ATU2 (figure 40B) contient un cADN infectieux correspondant à l'antigénome de la souche vaccinale Schwarz du virus de la rougeole (MV) dans lequel une unité de transcription supplémentaire (ATU) a été introduite entre les gènes P (phosphoprotéine) et M (matrice) (Combredet, 2003, Journal of Virology, 77 : 11546-11554). Des génomes recombinants MVSchw2-SARS-S et MVSchw2-SARS-Ssol du virus de la rougeole ont été construits par l'insertion des ORFs de la protéine S et du polypeptide Ssol au sein de l'unité de transcription supplémentaire du vecteur MVSchw-ATU2.

Pour cela, un fragment d'ADN contenant le cADN de la S du SRAS-CoV a été amplifié par PCR à l'aide des oligonucléotides 5'-ATACGTACGA CCATGTTTAT TTTCTTATTA TTTCTTACTC TCACT-3' et 5'-ATAGCGCGCT CATTATGTGT AATGTAATTT GACACCCTTG-3' en utilisant le plasmide pcDNA-S comme matrice puis inséré dans le plasmide pCR®2.1-TOPO (Invitrogen) pour obtenir le plasmide pTOPO-S-MV. Les deux oligonucléotides utilisés contiennent des sites de restriction BsiW1 et BssHII, de façon à permettre l'insertion ultérieure dans le vecteur rougeole, et ont été conçus de façon à générer une séquence de 3774 nt incluant les codons d'initiation et de terminaison, afin de respecter la règle des 6 qui stipule que la longueur du génome d'un virus rougeole doit être divisible par 6 (Calain & Roux, 1993, J. Virol., 67 : 4822-4830 ; Schneider et al., 1997, Virology, 227 : 314-322). L'insert a été séquencé à l'aide d'un kit BigDye Terminator v1.1 (Applied Biosystems) et d'un séquenceur automatique ABI377.

Afin d'exprimer une forme soluble et sécrétée de la S du SRAS-CoV, un plasmide contenant l'ADNc du polypeptide Ssol correspondant à l'ectodomaine (aa 1-1193) de la S du SRAS-CoV fusionné en son extrémité C-ter à la séquence d'une étiquette FLAG (DYKDDDDK) via un linker BspE1 codant pour le dipeptide SG a ensuite été obtenu. Pour cela, un fragment d'ADN a été amplifié à l'aide des oligonucléotides 5'-CCATTTCAAC AATTTGGCCG-3' et 5'-ATAGGATCCG CGCGCTCATT ATTTATCGTC GTCATCTTTA TAATC-3' à partir du plasmide pCDNA-Ssol puis inséré dans le plasmide pTOPO-S-MV entre les sites Sal1 et BamH1 pour obtenir le plasmide pTOPO-S-MV-SF. La séquence générée est longue de 3618 nt entre les sites BsiW1 et BssHII et respecte la règle des 6. L'insert a été séquencé comme indiqué ci-dessus.

Les fragments BsiW1-BssHII contenant les ADNc de la protéine S et du polypeptide Ssol ont ensuite été excisés par digestion des plasmides pTOPO-S-MV et pTOPO-S-MV-SF puis sous-clonés entre les sites correspondants du plasmide pTM-MVSchw-ATU2 pour donner les plasmides pTM-MVSchw2-SARS-S et pTM-MVSchw2-SARS-Ssol (figure 40B). Ces deux plasmides ont été déposés à la C.N.C.M. le 1^{er} décembre 2004, sous les numéros 1-3326 et 1-3327, respectivement.

Les virus rougeole recombinants correspondants aux plasmides pTM-MVSchw2-SARS-S et pTM-MVSchw2-SARS-Ssol ont été obtenus par génétique inverse selon le système reposant sur l'utilisation d'une lignée cellulaire auxiliaire, décrit par Radecke et coll. (1995, Embo J., 14, : 5773-5784) et modifié par Parks et coll. (1999, J. Virol., 73 : 3560-3566). Brièvement, les cellules auxiliaires 293-3-46 sont transfectées selon la méthode au phosphate de calcium par 5 µg des plasmides pTM-MVSchw2-SARS-S ou pTM-MVSchw2-SARS-Ssol et 0,02 µg du plasmide pEMC-La dirigeant l'expression de la polymérase L du MV (don de M.A. Billeter). Après une nuit d'incubation à 37°C, un choc thermique est réalisé pendant 2 heures à 43°C et les cellules transfectées sont transférées sur une monocouche de cellules Vero. Pour chacun des deux plasmides, des syncytia sont apparus après 2 à 3 jours de co-culture et ont été transférés successivement sur des monocouches de cellules Vero à 70% de confluence en boites de pétri de 35 mm puis en flacons de 25 et 75 cm². Quand les syncytia ont atteint 80-90% de confluence, les cellules sont récupérées à l'aide d'un grattoir puis congelées et décongelées une fois. Après une centrifugation à basse vitesse, le surnageant contenant le virus est conservé en aliquot à -80°C. Les titres des virus recombinants MVSchw2-SARS-S et MVSchw2-SARS-Ssol ont été déterminés par dilution limite sur cellules Vero et le titre en dose infectant 50% des cupules (TCID₅₀) calculé selon la méthode de Kärber.

### 3) Caractérisation des virus recombinants

L'expression des transgènes codant la protéine S et le polypeptide Ssol a été recherchée par western blot et immunofluorescence.

Des monocouches de cellules Vero en flacons T-25 ont été infectées à une multiplicité de 0,05 par différents passages des deux virus MVSchw2-SARS-S et MVSchw2-SARS-Ssol et le virus sauvage MWSchw à titre de contrôle. Quand les syncytia ont atteint 80 à 90% de confluence, des extraits cytoplasmiques ont été préparés dans un tampon d'extraction (150mMNaCl, 50 mM Tris-HCl pH 7,2, 1% triton-X-100, 0,1% SDS, 1% DOC) puis dilués en tampon de dépôt selon Laemmli, séparés sur un gel SDS à 8% de polyacrylamide et transférés sur une membrane de PVDF (BioRad). La détection de cette immunoempreinte (« western blot ») a été réalisée à l'aide d'un sérum polyclonal de lapin anti-S (immun-sérum du lapin P11135 : cf exemple 4 ci-dessus) et d'anticorps polyclonaux d'âne dirigés contre les IgG de lapin et couplés à la peroxidase (NA934V, Amersham). Les anticorps fixés ont été révélés par luminescence à l'aide du kit ECL+ (Amersham) et de films d'autoradiographie Hyperfilm MP (Amersham).

Des cellules Vero en monocouches sur lamelles de verre ont été infectées par les deux virus MVSchw2-SARS-S et MVSchw2-SARS-Ssol et le virus sauvage MWSchw à titre de contrôle à des multiplicités d'infection de 0,05. Quand les syncytia ont atteint 90 à 100% (virus MVSchw2-SARS-Ssol) ou 30 à 40% (MVSchw2-SARS-S,MWSchw) de confluence, les cellules ont été fixées dans une solution de PBS-PFA 4%, perméabilisées par une solution de PBS contenant 0,2% de triton puis marquées par des anticorps polyclonaux de lapins hyperimmunisés par des virions purifiés et inactivés du SRAS-CoV et par un conjugué d'anticorps de chèvre anti-IgG(H+L) de lapin couplé au FITC (Jackson).

Comme le montrent les figures 41 et 42, les virus recombinants MVSchw2-SARS-S et MVSchw2-SARS-Ssol dirigent l'expression de la protéine S et du polypeptide Ssol respectivement à des niveaux comparables à ceux que l'on peut observer 8h après infection par le SRAS-CoV. L'expression de ces polypeptides est stable après 3 passages des virus recombinants en culture cellulaire. Ces résultats démontrent que les virus rougeole recombinants sont bien porteurs des transgènes et permettent l'expression de la glycoprotéine du SRAS sous sa forme membranaire (S) ou sous une forme soluble (Ssol). On s'attend à ce que le polypeptide Ssol soit sécrété des cellules infectées par le virus MVSchw2-SARS-Ssol comme c'est le cas lorsque ce même polypeptide est exprimé dans des cellules de mammifères après transfection transitoire des séquences correspondantes (cf exemple 11 ci-dessus).

### 4) Applications

Ayant montré que les virus MVSchw2-SARS-S et MVSchw2-SARS-Ssol permettent l'expression de la S du SRAS-CoV, leur capacité à induire une réponse immunitaire protectrice contre le SRAS-CoV chez la souris CD46^{+/-} IFN-α̅βR^{-/-}, qui est susceptible à l'infection par le MV, est évaluée. La réponse en anticorps des souris immunisées est évaluée par test ELISA contre les antigènes natifs du SRAS-CoV et pour leur capacité à neutraliser l'infectivité du SRAS-CoV *in vitro,* en utilisant les méthodologies décrites ci-dessus. Le pouvoir protecteur de la réponse sera évalué en mesurant la réduction de la charge virale pulmonaire 2 jours après une infection d'épreuve non létale par le SRAS-CoV.

Des virus rougeole recombinants de seconde génération sont construits en substituant les séquences sauvages des gènes S et Sol par des gènes synthétiques optimisés pour l'expression en cellules de mammifères, décrits à l'exemple 15 ci-dessus. Ces virus rougeole recombinants sont susceptibles d'exprimer des quantités plus importantes des antigènes S et Ssol et donc de présenter une immunogénicité accrue.

Alternativement, les gènes sauvages ou synthétiques codant pour la protéine S ou le polypeptide Ssol peuvent être insérés dans le vecteur rougeole MVSchw-ATU3 sous la forme d'une unité de transcription supplémentaire localisée entre les gènes H et L, puis les virus recombinants produits et caractérisés de façon similaire. Cette insertion est susceptible de générer des virus recombinants possédant des caractéristiques (multiplication du virus, niveau d'expression du transgène) différentes et possiblement une immunogénicité améliorée par rapport à ceux obtenus après insertion des transgènes entre les gènes P et N.

Le virus rougeole recombinant MVSchw2-SARS-Ssol peut être utilisé pour la production en quantité et la purification de l'antigène Ssol en vue d'applications diagnostiques et vaccinales.

### Exemple 18 : Autres applications liées à la protéine S

a) Les vecteurs lentiviraux permettant l'expression de la S ou de Ssol (voire de fragments de la S) peuvent constituer un vaccin recombinant contre le SRAS-CoV, pour être utilisé en prophylaxie humaine et vétérinaire. Afin de démontrer la faisabilité d'un tel vaccin, l'immunogénicité des vecteurs lentiviraux recombinants TRIP-SD/SA-S-WPRE et TRIP-SD/SA-Ssol-WPRE est étudiée chez la souris.
b) Des anticorps monoclonaux sont produits à l'aide du polypeptide recombinant Ssol. D'après les résultats présentés à l'exemple 14 ci-dessus, ces anticorps ou du moins la majorité d'entre eux reconnaîtront la forme native de la S du SRAS-CoV et seront susceptibles d'applications diagnostiques et/ou prophylactiques.
c) Un test de sérologie du SRAS est mis au point avec le polypeptide Ssol utilisé comme antigène et la méthodologie du double épitope.

## Revendications

1. Polypeptide isolé et purifié, **caractérisé en ce qu'**il s'agit de l'ectodomaine de la protéine S de séquence SEQ ID NO: 3, et **en ce qu'**il est constitué des acides aminés correspondant aux positions 1 à 1193 de la séquence en acides aminés de ladite protéine S ou des acides aminés correspondant aux positions 14 à 1193 de la séquence en acides aminés de ladite protéine S.

2. Acide nucléique codant pour un polypeptide selon la revendication 1.

3. Vecteur d'expression recombinant, **caractérisé en ce qu'**il comprend un acide nucléique selon la revendication 2.

4. Vecteur d'expression recombinant selon la revendication 3, codant pour l'ectodomaine de la protéine S de séquence SEQ ID NO: 3 **caractérisé en ce qu'**il est choisi parmi les vecteurs contenus dans les souches bactériennes suivantes, déposées auprès de la Collection Nationale de Cultures de Microorganismes (CNCM), 25 rue du Docteur Roux, 75724 Paris Cedex 15
a) souche n° I-3324, déposée le 22 novembre 2004,
b) souche n° 1-3327, déposée le 1" décembre 2004,
c) souche n° 1-3332, déposée le 1" décembre 2004,
d) souche n° I-3335, déposée le 1^{er} décembre 2004,
e) souche n° I-3337, déposée le 1^{er} décembre 2004,
f) souche n° 1-3339, déposée le 2 décembre 2004, et
g) souche n° I-3341, déposée le 2 décembre 2004.

5. Vecteur d'expression selon la revendication 3, **caractérisé en ce qu'**il s'agit d'un vecteur viral, sous forme de particule virale ou sous forme de génome recombinant.

6. Vecteur viral, sous forme de particule virale ou sous forme de génome recombinant selon la revendication 5, **caractérisé en ce qu'**il s'agit d'une particule virale recombinante ou d'un génome viral recombinant susceptible d'être obtenu par transfection d'un plasmide selon les alinéas b) ou d) à g) de la revendication 4, dans un système cellulaire approprié.

7. Vecteur d'expression recombinant selon la revendication 3, **caractérisé en ce qu'**il s'agit d'un vecteur lentiviral.

8. Vecteur d'expression recombinant selon la revendication 3, **caractérisé en ce qu'**il s'agit d'un virus rougeole.

9. Vecteur d'expression recombinant selon la revendication 3, **caractérisé en ce qu'**il s'agit d'un virus vaccine.

10. Utilisation d'un vecteur contenu dans une souche bactérienne selon la revendication 4, pour la production *in vitro*, en système eucaryote, de l'ectodomaine de la protéine S du coronavirus associé au SRAS selon la revendication 1.

11. Méthode de production de l'ectodomaine de la protéine S selon la revendication 1 en système eucaryote, comportant une étape de transfection de cellules eucaryotes en culture par un vecteur choisi parmi les vecteurs contenus dans les souches bactériennes mentionnées à la revendication 5.

12. Cellule eucaryote isolée génétiquement modifiée exprimant un polypeptide selon la revendication 1.

13. Cellule selon la revendication 12, susceptible d'être obtenue par transfection par l'un quelconque des vecteurs mentionnés aux alinéas d) ou e) de la revendication 4.

14. Cellule selon la revendication 13, **caractérisée en ce qu'**il s'agit de la cellule FRhK4-Ssol-30, déposée à la CNCM le 22 novembre 2004, sous le n°I-3325.

15. Utilisation d'un polypeptide selon la revendication 1, pour détecter une infection par un coronavirus associé au SRAS, à partir d'un échantillon biologique.

16. Méthode de détection d'une infection par un coronavirus associé au SRAS ou d'anticorps dirigés contre un coronavirus associé au SRAS, à partir d'un échantillon biologique, **caractérisée en ce que** la détection est effectuée par ELISA utilisant un polypeptide selon la revendication 1, exprimé dans un système eucaryote.

17. Méthode de détection selon la revendication 16, comportant en outre une étape de détection par ELISA utilisant la protéine N recombinante.

18. Méthode selon la revendication 16 ou 17, **caractérisée en ce qu'**il s'agit d'une méthode par ELISA double épitope, et **en ce que** le sérum à tester est mélangé à l'antigène de révélation, ledit mélange étant ensuite mis au contact de l'antigène fixé sur un support solide.

19. Complexe immun isolé formé d'un polypeptide selon la revendication 1, et d'un anticorps dirigé spécifiquement contre un épitope du coronavirus associé au SRAS.

20. Kit ou coffret de détection d'un coronavirus associé au SRAS, **caractérisé en ce qu'**il comprend au moins un réactif sélectionné dans le groupe constitué par : un polypeptide selon la revendication 1, un acide nucléique selon la revendication 2, une cellule selon l'une quelconque des revendications 12 à 14.

21. Composition immunogène et/ou vaccinale, **caractérisée en ce qu'**elle comprend un polypeptide recombinant selon la revendication 1, obtenu dans un système d'expression eucaryote.

22. Composition immunogène et/ou vaccinale, **caractérisée en ce qu'**elle comprend un vecteur ou virus recombinant selon l'une quelconque des revendications 3, 4, et 5 à 9.

## Claims

1. Isolated and purified polypeptide, **characterised in that** it is the ectodomain of the protein S having the sequence SEQ ID NO: 3 and **in that** it consists of amino acids corresponding to positions 1 to 1193 in the amino acid sequence of the said protein S or amino acids corresponding to positions 14 to 1193 in the sequence of amino acids in the said protein S.

2. Nucleic acid coding for a polypeptide according to claim 1.

3. Recombinant expression vector, **characterised in that** it comprises a nucleic acid according to claim 2.

4. Recombinant expression vector according to claim 3, coding for the ectodomain of the protein S of sequence SEQ ID NO: 3, **characterised in that** it is selected from the vectors contained in the following bacterial strains filed with the [French] National Microorganisms Cultures Collection (CNCM), 25 rue du Docteur Roux, 75724 Paris Cedex 15
a) strain no. 1-3324, filed on the 22 November 2004,
b) strain no. 1-3327, filed on the 1 December 2004,
c) strain no. 1-3332, filed on the 1 December 2004,
d) strain no. 1-3335, filed on the 1 December 2004,
e) strain no. 1-3337, filed on the 1 December 2004,
f) strain no. 1-3339, filed on the 2 December 2004, and
g) strain no. 1-3341, filed on the 2 December 2004.

5. Expression vector according to claim 3, **characterised in that** it is a viral vector, in the form of a viral particle or in the form of a recombinant genome.

6. Viral vector in the form of a viral particle or recombinant genome according to claim 5, **characterised in that** it is a recombinant viral particle or a recombinant viral genome which can be obtained by the transfection of a plasmid according to subparagraphs b) or d) to g) of claim 4 in an appropriate cell system.

7. Recombinant expression vector according to claim 3, **characterised in that** it is a lentiviral vector.

8. Recombinant expression vector according to claim 3, **characterised in that** it is a measles virus.

9. Recombinant expression vector according to claim 3, **characterised in that** it is a vaccine virus.

10. Use of a vector contained in a bacterial strain according to claim 4 for the *in vitro* production in a eucaryote system of the ectodomain of the protein S of the coronavirus associated with SARS according to claim 1.

11. Method for production of the ectodomain of the protein S according to claim 1 in a eucaryote system comprising a step of transfection of eucaryote cells in culture by a vector selected from the vectors contained in the bacterial strains mentioned in claim 5.

12. Isolated Genetically-modified eucaryote cell expressing a polypeptide according to claim 1.

13. Cell according to claim 12, which can be obtained by transfection by any one of the vectors mentioned in subparagraphs d) or e) of claim 4.

14. Cell according to claim 13, **characterised in that** it is the FRhK4-Ssol-30 cell filed with the CNCM on the 22 November 2004 under no. 1-3325.

15. Use of a polypeptide according to claim 1, to detect an infection by a coronavirus associated with SARS from of a biological sample.

16. Method for the detection of an infection by a coronavirus associated with SARS or antibodies directed against a coronavirus associated with SARS, from a biological sample, **characterised in that** the detection is performed by ELISA using a polypeptide according to claim 1 expressed in an eucaryote system.

17. Method of detection according to claim 16, further comprising a step of detection by ELISA using N recombinant protein.

18. Method according to claim 16 or 17, **characterised in that** it is a double epitope ELISA method and **in that** the serum to be tested is mixed with the visualizing antigen, said mixture then being brought into contact with the antigen fixed on a solid support.

19. Isolated immune complex formed of a polypeptide according to claim 1, and an antibody directed specifically against an epitope of the coronavirus associated with SARS.

20. Kit or box for detecting a coronavirus associated with SARS, **characterised in that** it comprises at least one reagent selected from the group consisting of: a polypeptide according to claim 1, a nucleic acid according to claim 2, a cell according to any one of claims 12 to 14.

21. Immunogenic and/or vaccine composition, **characterised in that** it comprises a recombinant polypeptide according to claim 1 obtained in a eucaryote expression system.

22. Immunogenic and/or vaccine composition, **characterised in that** it comprises a recombinant vector or virus according to any one of claims 3, 4 and 5 to 9.

## Patentansprüche

1. Isoliertes und gereinigtes Polypeptid, **dadurch gekennzeichnet, dass** es sich um die Ektodomäne des Proteins S mit der Sequenz SEQ ID NO: 3 handelt, und dass es aus Aminosäuren, die Positionen 1 bis 1193 der Aminosäuresequenz des Proteins S entsprechen, oder Aminosäuren, die Positionen 14 bis 1193 der Aminosäuresequenz des Proteins S entsprechen, besteht.

2. Nukleinsäure, die für ein Polypeptid gemäß Anspruch 1 codiert.

3. Rekombinanter Expressionsvektor, **dadurch gekennzeichnet, dass** er eine Nukleinsäure gemäß Anspruch 2 umfasst.

4. Rekombinanter Expressionsvektor gemäß Anspruch 3, der für die Ektodomäne des Proteins S mit der Sequenz SEQ ID NO: 3 codiert, **dadurch gekennzeichnet, dass** er aus den Vektoren ausgewählt ist, die in der folgenden Bakterienstämmen enthalten sind, die bei der Collection Nationale de Cultures de Microorganismes (CNCM), 25 rue du Docteur Roux, 75724 Paris Cedex 15, hinterlegt wurden:
a) Stamm Nr. 1-3324, hinterlegt am 22. November 2004,
b) Stamm Nr. 1-3327, hinterlegt am 1. Dezember 2004,
c) Stamm Nr. 1-3332, hinterlegt am 1. Dezember 2004,
d) Stamm Nr. 1-3335, hinterlegt am 1. Dezember 2004,
e) Stamm Nr. 1-3337, hinterlegt am 1. Dezember 2004,
f) Stamm Nr. 1-3339, hinterlegt am 2. Dezember 2004 und
g) Stamm Nr. 1-3341, hinterlegt am 2. Dezember 2004.

5. Expressionsvektor gemäß Anspruch 3, **dadurch gekennzeichnet, dass** es sich um einen viralen Vektor, in der Form eines viralen Partikels oder in der Form eines rekombinanten Genoms, handelt.

6. Viraler Vektor, in der Form eines viralen Partikels oder in der Form eines rekombinanten Genoms, gemäß Anspruch 5, **dadurch gekennzeichnet, dass** es sich um ein rekombinantes virales Partikel oder ein rekombinantes virales Genom, das durch Transfektion eines Plasmids gemäß den Absätzen b) oder d) bis g) von Anspruch 4 in ein geeignetes zelluläres System erhalten werden kann, handelt.

7. Rekombinanter Expressionsvektor gemäß Anspruch 3, **dadurch gekennzeichnet, dass** es sich um einen lentiviralen Vektor handelt.

8. Rekombinanter Expressionsvektor gemäß Anspruch 3, **dadurch gekennzeichnet, dass** es sich um ein Masernvirus handelt.

9. Rekombinanter Expressionsvektor gemäß Anspruch 3, **dadurch gekennzeichnet, dass** es sich um ein Vacciniavirus handelt.

10. Verwendung eines Vektors, der in einem Bakterienstamm gemäß Anspruch 4 enthalten ist, für die in-vitro-Produktion der Ektodomäne des Proteins S des Koronavirus, assoziiert mit SARS, gemäß Anspruch 1 in einem eukaryotischen System.

11. Verfahren zur Herstellung der Ektodomäne des Proteins S gemäß Anspruch 1 in einem eukaryotischen System, umfassend eine Stufe der Transfektion von Eukaryotenzellen in Kultur mit einem Vektor, der aus den Vektoren ausgewählt ist, die in den Bakterienzellen, die in Anspruch 5 genannt sind, enthalten sind.

12. Isolierte Eukaryotenzelle, die genetisch modifiziert ist, sodass sie ein Polypeptid gemäß Anspruch 1 exprimiert.

13. Zelle gemäß Anspruch 12, die durch Transfektion durch einen der in den Absätzen d) oder e) des Anspruchs 4 genannten Vektoren erhalten werden kann.

14. Zelle gemäß Anspruch 13, **dadurch gekennzeichnet, dass** es sich um die Zelle FRhK4-Ssol-30 handelt, die bei der CNCM am 22. November 2004 unter der Nummer 1-3325 hinterlegt wurde.

15. Verwendung eines Polypeptids gemäß Anspruch 1, um eine Infektion durch ein Koronavirus, assoziiert mit SARS, ausgehend von einer biologischen Probe zu detektieren.

16. Verfahren zur Detektion einer Infektion durch ein Koronavirus, assoziiert mit SARS, oder von Antikörpern, die gegen ein Koronavirus, assoziiert mit SARS, gerichtet sind, ausgehend von einer biologischen Probe, **dadurch gekennzeichnet, dass** die Detektion durch ELISA durchgeführt wird, wobei ein Polypeptid gemäß Anspruch 1 verwendet wird, das in einem eukaryotischen System exprimiert wird.

17. Verfahren zur Detektion gemäß Anspruch 16, das außerdem eine Stufe der Detektion durch ELISA umfasst, der das rekombinante Protein N verwendet.

18. Verfahren gemäß Anspruch 16 oder 17, **dadurch gekennzeichnet, dass** es sich um ein Verfahren durch Doppel-Epitop-ELISA handelt, und dass das zu testende Serum mit dem Entwicklungsantigen gemischt wird, wobei das Gemisch dann mit dem Antigen, das an einem festen Träger fixiert ist, in Kontakt gebracht wird.

19. Isolierter Immunkomplex, der aus einem Polypeptid gemäß Anspruch 1 und einem Antikörper, der spezifisch gegen ein Epitop des Koronavirus, assoziiert mit SARS, gerichtet ist, gebildet ist.

20. Kit oder Packung zur Detektion eines Koronavirus, assoziiert mit SARS, **dadurch gekennzeichnet, dass** er/sie wenigstens ein Reagens, ausgewählt aus der Gruppe, bestehend aus einem Polypeptid gemäß Anspruch 1, einer Nukleinsäure gemäß Anspruch 2, einer Zelle gemäß einem der Ansprüche 12 bis 14, umfasst.

21. Immunogene Zusammensetzung und/oder Impfstoffzusammensetzung, **dadurch gekennzeichnet, dass** sie ein rekombinantes Polypeptid gemäß Anspruch 1, das in einem eukaryotischen Expressionssystem erhalten wurde, umfasst.

22. Immunogene Zusammensetzung und/oder Impfstoffzusammensetzung, **dadurch gekennzeichnet, dass** sie einen rekombinanten Vektor oder ein rekombinantes Virus gemäß einem der Ansprüche 3, 4 und 5 bis 9 umfasst.


### SEQUENCE LISTING

<110> INSTITUT PASTEUR
   CENTRE NATIONAL DE LA RECHERCHE SCIENTIFIQUE
   UNIVERSITE PARIS 7
   VAN DER WERF, Sylvie
   ESCRIOU, Nicolas
   CRESCENZO-CHAIGNE, Bernadette
   MANUGUERRA, Jean-Claude
   KUNST, Franck
   CALLENDRET, Benoît
   BETTON, Jean-Michel
   LORIN, Valérie
   GERBAUD, Sylvie
   BURGUIERE, Ana Maria
   AZEBI, Saliha
   CHARNEAU, Pierre
   TANGY, Frédéric
   COMBREDET, Chantal
   DELAGNEAU, Jean-François
   MARTIN, Monique
<120> UTILISATION DES PROTEINES ET DES PEPTIDES CODES PAR LE GENOME D'UNE
   NOUVELLE SOUCHE DE CORONAVIRUS ASSOCIE AU SARS
<130> 226-111ext
<150> FR 0314152
   <151> 2003-12-02
<150> FR 0314151
   <151> 2003-12-02
<160> 158
<170> PatentIn version 3.1
<210> 1
   <211> 29746
   <212> DNA
   <213> CORONAVIRUS
<400> 1
   atattaggtt tttacctacc caggaaaagc caaccaacct cgatctcttg tagatctgtt      60
   ctctaaacga actttaaaat ctgtgtagct gtcgctcggc tgcatgccta gtgcacctac     120
   gcagtataaa caataataaa ttttactgtc gttgacaaga aacgagtaac tcgtccctct     180
   tctgcagact gcttacggtt tcgtccgtgt tgcagtcgat catcagcata cctaggtttc     240
   gtccgggtgt gaccgaaagg taagatggag agccttgttc ttggtgtcaa cgagaaaaca     300
   cacgtccaac tcagtttgcc tgtccttcag gttagagacg tgctagtgcg tggcttcggg     360
   gactctgtgg aagaggccct atcggaggca cgtgaacacc tcaaaaatgg cacttgtggt     420
   ctagtagagc tggaaaaagg cgtactgccc cagcttgaac agccctatgt gttcattaaa     480
   cgttctgatg ccttaagcac caatcacggc cacaaggtcg ttgagctggt tgcagaaatg     540
   gacggcattc agtacggtcg tagcggtata acactgggag tactcgtgcc acatgtgggc     600
   gaaaccccaa ttgcataccg caatgttctt cttcgtaaga acggtaataa gggagccggt     660
   ggtcatagct atggcatcga tctaaagtct tatgacttag gtgacgagct tggcactgat     720
   cccattgaag attatgaaca aaactggaac actaagcatg gcagtggtgc actccgtgaa     780
   ctcactcgtg agctcaatgg aggtgcagtc actcgctatg tcgacaacaa tttctgtggc     840
   ccagatgggt accctcttga ttgcatcaaa gattttctcg cacgcgcggg caagtcaatg     900
   tgcactcttt ccgaacaact tgattacatc gagtcgaaga gaggtgtcta ctgctgccgt     960
   gaccatgagc atgaaattgc ctggttcact gagcgctctg ataagagcta cgagcaccag    1020
   acacccttcg aaattaagag tgccaagaaa tttgacactt tcaaagggga atgcccaaag    1080
   tttgtgtttc ctcttaactc aaaagtcaaa gtcattcaac cacgtgttga aaagaaaaag    1140
   actgagggtt tcatggggcg tatacgctct gtgtaccctg ttgcatctcc acaggagtgt    1200
   aacaatatgc acttgtctac cttgatgaaa tgtaatcatt gcgatgaagt ttcatggcag    1260
   acgtgcgact ttctgaaagc cacttgtgaa cattgtggca ctgaaaattt agttattgaa    1320
   ggacctacta catgtgggta cctacctact aatgctgtag tgaaaatgcc atgtcctgcc    1380
   tgtcaagacc cagagattgg acctgagcat agtgttgcag attatcacaa ccactcaaac    1440
   attgaaactc gactccgcaa gggaggtagg actagatgtt ttggaggctg tgtgtttgcc    1500
   tatgttggct gctataataa gcgtgcctac tgggttcctc gtgctagtgc tgatattggc    1560
   tcaggccata ctggcattac tggtgacaat gtggagacct tgaatgagga tctccttgag 1620
   atactgagtc gtgaacgtgt taacattaac attgttggcg attttcattt gaatgaagag 1680
   gttgccatca ttttggcatc tttctctgct tctacaagtg cctttattga cactataaag 1740
   agtcttgatt acaagtcttt caaaaccatt gttgagtcct gcggtaacta taaagttacc 1800
   aagggaaagc ccgtaaaagg tgcttggaac attggacaac agagatcagt tttaacacca 1860
   ctgtgtggtt ttccctcaca ggctgctggt gttatcagat caatttttgc gcgcacactt 1920
   gatgcagcaa accactcaat tcctgatttg caaagagcag ctgtcaccat acttgatggt 1980
   atttctgaac agtcattacg tcttgtcgac gccatggttt atacttcaga cctgctcacc 2040
   aacagtgtca ttattatggc atatgtaact ggtggtcttg tacaacagac ttctcagtgg 2100
   ttgtctaatc ttttgggcac tactgttgaa aaactcaggc ctatctttga atggattgag 2160
   gcgaaactta gtgcaggagt tgaatttctc aaggatgctt gggagattct caaatttctc 2220
   attacaggtg tttttgacat cgtcaagggt caaatacagg ttgcttcaga taacatcaag 2280
   gattgtgtaa aatgcttcat tgatgttgtt aacaaggcac tcgaaatgtg cattgatcaa 2340
   gtcactatcg ctggcgcaaa gttgcgatca ctcaacttag gtgaagtctt catcgctcaa 2400
   agcaagggac tttaccgtca gtgtatacgt ggcaaggagc agctgcaact actcatgcct 2460
   cttaaggcac caaaagaagt aacctttctt gaaggtgatt cacatgacac agtacttacc 2520
   tctgaggagg ttgttctcaa gaacggtgaa ctcgaagcac tcgagacgcc cgttgatagc 2580
   ttcacaaatg gagctatcgt tggcacacca gtctgtgtaa atggcctcat gctcttagag 2640
   attaaggaca aagaacaata ctgcgcattg tctcctggtt tactggctac aaacaatgtc 2700
   tttcgcttaa aggggggtgc accaattaaa ggtgtaacct ttggagaaga tactgtttgg 2760
   gaagttcaag gttacaagaa tgtgagaatc acatttgagc ttgatgaacg tgttgacaaa 2820
   gtgcttaatg aaaagtgctc tgtctacact gttgaatccg gtaccgaagt tactgagttt 2880
   gcatgtgttg tagcagaggc tgttgtgaag actttacaac cagtttctga tctccttacc 2940
   aacatgggta ttgatcttga tgagtggagt gtagctacat tctacttatt tgatgatgct 3000
   ggtgaagaaa acttttcatc acgtatgtat tgttcctttt accctccaga tgaggaagaa 3060
   gaggacgatg cagagtgtga ggaagaagaa attgatgaaa cctgtgaaca tgagtacggt 3120
   acagaggatg attatcaagg tctccctctg gaatttggtg cctcagctga aacagttcga 3180
   gttgaggaag aagaagagga agactggctg gatgatacta ctgagcaatc agagattgag 3240
   ccagaaccag aacctacacc tgaagaacca gttaatcagt ttactggtta tttaaaactt 3300
   actgacaatg ttgccattaa atgtgttgac atcgttaagg aggcacaaag tgctaatcct 3360
   atggtgattg taaatgctgc taacatacac ctgaaacatg gtggtggtgt agcaggtgca 3420
   ctcaacaagg caaccaatgg tgccatgcaa aaggagagtg atgattacat taagctaaat 3480
   ggccctctta cagtaggagg gtcttgtttg ctttctggac ataatcttgc taagaagtgt 3540
   ctgcatgttg ttggacctaa cctaaatgca ggtgaggaca tccagcttct taaggcagca 3600
   tatgaaaatt tcaattcaca ggacatctta cttgcaccat tgttgtcagc aggcatattt   3660
   ggtgctaaac cacttcagtc tttacaagtg tgcgtgcaga cggttcgtac acaggtttat   3720
   attgcagtca atgacaaagc tctttatgag caggttgtca tggattatct tgataacctg   3780
   aagcctagag tggaagcacc taaacaagag gagccaccaa acacagaaga ttccaaaact   3840
   gaggagaaat ctgtcgtaca gaagcctgtc gatgtgaagc caaaaattaa ggcctgcatt   3900
   gatgaggtta ccacaacact ggaagaaact aagtttctta ccaataagtt actcttgttt   3960
   gctgatatca atggtaagct ttaccatgat tctcagaaca tgcttagagg tgaagatatg   4020
   tctttccttg agaaggatgc accttacatg gtaggtgatg ttatcactag tggtgatatc   4080
   acttgtgttg taataccctc caaaaaggct ggtggcacta ctgagatgct ctcaagagct   4140
   ttgaagaaag tgccagttga tgagtatata accacgtacc ctggacaagg atgtgctggt   4200
   tatacacttg aggaagctaa gactgctctt aagaaatgca aatctgcatt ttatgtacta   4260
   ccttcagaag cacctaatgc taaggaagag attctaggaa ctgtatcctg gaatttgaga   4320
   gaaatgcttg ctcatgctga agagacaaga aaattaatgc ctatatgcat ggatgttaga   4380
   gccataatgg caaccatcca acgtaagtat aaaggaatta aaattcaaga gggcatcgtt   4440
   gactatggtg tccgattctt cttttatact agtaaagagc ctgtagcttc tattattacg   4500
   aagctgaact ctctaaatga gccgcttgtc acaatgccaa ttggttatgt gacacatggt   4560
   tttaatcttg aagaggctgc gcgctgtatg cgttctctta aagctcctgc cgtagtgtca   4620
   gtatcatcac cagatgctgt tactacatat aatggatacc tcacttcgtc atcaaagaca   4680
   tctgaggagc actttgtaga aacagtttct ttggctggct cttacagaga ttggtcctat   4740
   tcaggacagc gtacagagtt aggtgttgaa tttcttaagc gtggtgacaa aattgtgtac   4800
   cacactctgg agagccccgt cgagtttcat cttgacggtg aggttctttc acttgacaaa   4860
   ctaaagagtc tcttatccct gcgggaggtt aagactataa aagtgttcac aactgtggac   4920
   aacactaatc tccacacaca gcttgtggat atgtctatga catatggaca gcagtttggt   4980
   ccaacatact tggatggtgc tgatgttaca aaaattaaac ctcatgtaaa tcatgagggt   5040
   aagactttct ttgtactacc tagtgatgac acactacgta gtgaagcttt cgagtactac   5100
   catactcttg atgagagttt tcttggtagg tacatgtctg ctttaaacca cacaaagaaa   5160
   tggaaatttc ctcaagttgg tggtttaact tcaattaaat gggctgataa caattgttat   5220
   ttgtctagtg ttttattagc acttcaacag cttgaagtca aattcaatgc accagcactt   5280
   caagaggctt attatagagc ccgtgctggt gatgctgcta acttttgtgc actcatactc   5340
   gcttacagta ataaaactgt tggcgagctt ggtgatgtca gagaaactat gacccatctt   5400
   ctacagcatg ctaatttgga atctgcaaag cgagttctta atgtggtgtg taaacattgt   5460
   ggtcagaaaa ctactacctt aacgggtgta gaagctgtga tgtatatggg tactctatct   5520
   tatgataatc ttaagacagg tgtttccatt ccatgtgtgt gtggtcgtga tgctacacaa   5580
   tatctagtac aacaagagtc ttcttttgtt atgatgtctg caccacctgc tgagtataaa   5640
   ttacagcaag gtacattctt atgtgcgaat gagtacactg gtaactatca gtgtggtcat 5700
   tacactcata taactgctaa ggagaccctc tatcgtattg acggagctca ccttacaaag 5760
   atgtcagagt acaaaggacc agtgactgat gttttctaca aggaaacatc ttacactaca 5820
   accatcaagc ctgtgtcgta taaactcgat ggagttactt acacagagat tgaaccaaaa 5880
   ttggatgggt attataaaaa ggataatgct tactatacag agcagcctat agaccttgta 5940
   ccaactcaac cattaccaaa tgcgagtttt gataatttca aactcacatg ttctaacaca 6000
   aaatttgctg atgatttaaa tcaaatgaca ggcttcacaa agccagcttc acgagagcta 6060
   tctgtcacat tcttcccaga cttgaatggc gatgtagtgg ctattgacta tagacactat 6120
   tcagcgagtt tcaagaaagg tgctaaatta ctgcataagc caattgtttg gcacattaac 6180
   caggctacaa ccaagacaac gttcaaacca aacacttggt gtttacgttg tctttggagt 6240
   acaaagccag tagatacttc aaattcattt gaagttctgg cagtagaaga cacacaagga 6300
   atggacaatc ttgcttgtga aagtcaacaa cccacctctg aagaagtagt ggaaaatcct 6360
   accatacaga aggaagtcat agagtgtgac gtgaaaacta ccgaagttgt aggcaatgtc 6420
   atacttaaac catcagatga aggtgttaaa gtaacacaag agttaggtca tgaggatctt 6480
   atggctgctt atgtggaaaa cacaagcatt accattaaga aacctaatga gctttcacta 6540
   gccttaggtt taaaaacaat tgccactcat ggtattgctg caattaatag tgttccttgg 6600
   agtaaaattt tggcttatgt caaaccattc ttaggacaag cagcaattac aacatcaaat 6660
   tgcgctaaga gattagcaca acgtgtgttt aacaattata tgccttatgt gtttacatta 6720
   ttgttccaat tgtgtacttt tactaaaagt accaattcta gaattagagc ttcactacct 6780
   acaactattg ctaaaaatag tgttaagagt gttgctaaat tatgtttgga tgccggcatt 6840
   aattatgtga agtcacccaa attttctaaa ttgttcacaa tcgctatgtg gctattgttg 6900
   ttaagtattt gcttaggttc tctaatctgt gtaactgctg cttttggtgt actcttatct 6960
   aattttggtg ctccttctta ttgtaatggc gttagagaat tgtatcttaa ttcgtctaac 7020
   gttactacta tggatttctg tgaaggttct tttccttgca gcatttgttt aagtggatta 7080
   gactcccttg attcttatcc agctcttgaa accattcagg tgacgatttc atcgtacaag 7140
   ctagacttga caattttagg tctggccgct gagtgggttt tggcatatat gttgttcaca 7200
   aaattctttt atttattagg tctttcagct ataatgcagg tgttctttgg ctattttgct 7260
   agtcatttca tcagcaattc ttggctcatg tggtttatca ttagtattgt acaaatggca 7320
   cccgtttctg caatggttag gatgtacatc ttctttgctt ctttctacta catatggaag 7380
   agctatgttc atatcatgga tggttgcacc tcttcgactt gcatgatgtg ctataagcgc 7440
   aatcgtgcca cacgcgttga gtgtacaact attgttaatg gcatgaagag atctttctat 7500
   gtctatgcaa atggaggccg tggcttctgc aagactcaca attggaattg tctcaattgt 7560
   gacacatttt gcactggtag tacattcatt agtgatgaag ttgctcgtga tttgtcactc 7620
   cagtttaaaa gaccaatcaa ccctactgac cagtcatcgt atattgttga tagtgttgct 7680
   gtgaaaaatg gcgcgcttca cctctacttt gacaaggctg gtcaaaagac ctatgagaga 7740
   catccgctct cccattttgt caatttagac aatttgagag ctaacaacac taaaggttca 7800
   ctgcctatta atgtcatagt ttttgatggc aagtccaaat gcgacgagtc tgcttctaag 7860
   tctgcttctg tgtactacag tcagctgatg tgccaaccta ttctgttgct tgaccaagct 7920
   cttgtatcag acgttggaga tagtactgaa gtttccgtta agatgtttga tgcttatgtc 7980
   gacacctttt cagcaacttt tagtgttcct atggaaaaac ttaaggcact tgttgctaca 8040
   gctcacagcg agttagcaaa gggtgtagct ttagatggtg tcctttctac attcgtgtca 8100
   gctgcccgac aaggtgttgt tgataccgat gttgacacaa aggatgttat tgaatgtctc 8160
   aaactttcac atcactctga cttagaagtg acaggtgaca gttgtaacaa tttcatgctc 8220
   acctataata aggttgaaaa catgacgccc agagatcttg gcgcatgtat tgactgtaat 8280
   gcaaggcata tcaatgccca agtagcaaaa agtcacaatg tttcactcat ctggaatgta 8340
   aaagactaca tgtctttatc tgaacagctg cgtaaacaaa ttcgtagtgc tgccaagaag 8400
   aacaacatac cttttagact aacttgtgct acaactagac aggttgtcaa tgtcataact 8460
   actaaaatct cactcaaggg tggtaagatt gttagtactt gttttaaact tatgcttaag 8520
   gccacattat tgtgcgttct tgctgcattg gtttgttata tcgttatgcc agtacataca 8580
   ttgtcaatcc atgatggtta cacaaatgaa atcattggtt acaaagccat tcaggatggt 8640
   gtcactcgtg acatcatttc tactgatgat tgttttgcaa ataaacatgc tggttttgac 8700
   gcatggttta gccagcgtgg tggttcatac aaaaatgaca aaagctgccc tgtagtagct 8760
   gctatcatta caagagagat tggtttcata gtgcctggct taccgggtac tgtgctgaga 8820
   gcaatcaatg gtgacttctt gcattttcta cctcgtgttt ttagtgctgt tggcaacatt 8880
   tgctacacac cttccaaact cattgagtat agtgattttg ctacctctgc ttgcgttctt 8940
   gctgctgagt gtacaatttt taaggatgct atgggcaaac ctgtgccata ttgttatgac 9000
   actaatttgc tagagggttc tatttcttat agtgagcttc gtccagacac tcgttatgtg 9060
   cttatggatg gttccatcat acagtttcct aacacttacc tggagggttc tgttagagta 9120
   gtaacaactt ttgatgctga gtactgtaga catggtacat gcgaaaggtc agaagtaggt 9180
   atttgcctat ctaccagtgg tagatgggtt cttaataatg agcattacag agctctatca 9240
   ggagttttct gtggtgttga tgcgatgaat ctcatagcta acatctttac tcctcttgtg 9300
   caacctgtgg gtgctttaga tgtgtctgct tcagtagtgg ctggtggtat tattgccata 9360
   ttggtgactt gtgctgccta ctactttatg aaattcagac gtgtttttgg tgagtacaac 9420
   catgttgttg ctgctaatgc acttttgttt ttgatgtctt tcactatact ctgtctggta 9480
   ccagcttaca gctttctgcc gggagtctac tcagtctttt acttgtactt gacattctat 9540
   ttcaccaatg atgtttcatt cttggctcac cttcaatggt ttgccatgtt ttctcctatt 9600
   gtgccttttt ggataacagc aatctatgta ttctgtattt ctctgaagca ctgccattgg 9660
   ttctttaaca actatcttag gaaaagagtc atgtttaatg gagttacatt tagtaccttc 9720
   gaggaggctg ctttgtgtac ctttttgctc aacaaggaaa tgtacctaaa attgcgtagc 9780
   gagacactgt tgccacttac acagtataac aggtatcttg ctctatataa caagtacaag 9840
   tatttcagtg gagccttaga tactaccagc tatcgtgaag cagcttgctg ccacttagca 9900
   aaggctctaa atgactttag caactcaggt gctgatgttc tctaccaacc accacagaca 9960
   tcaatcactt ctgctgttct gcagagtggt tttaggaaaa tggcattccc gtcaggcaaa 10020
   gttgaagggt gcatggtaca agtaacctgt ggaactacaa ctcttaatgg attgtggttg 10080
   gatgacacag tatactgtcc aagacatgtc atttgcacag cagaagacat gcttaatcct 10140
   aactatgaag atctgctcat tcgcaaatcc aaccatagct ttcttgttca ggctggcaat 10200
   gttcaacttc gtgttattgg ccattctatg caaaattgtc tgcttaggct taaagttgat 10260
   acttctaacc ctaagacacc caagtataaa tttgtccgta tccaacctgg tcaaacattt 10320
   tcagttctag catgctacaa tggttcacca tctggtgttt atcagtgtgc catgagacct 10380
   aatcatacca ttaaaggttc tttccttaat ggatcatgtg gtagtgttgg ttttaacatt 10440
   gattatgatt gcgtgtcttt ctgctatatg catcatatgg agcttccaac aggagtacac 10500
   gctggtactg acttagaagg taaattctat ggtccatttg ttgacagaca aactgcacag 10560
   gctgcaggta cagacacaac cataacatta aatgttttgg catggctgta tgctgctgtt 10620
   atcaatggtg ataggtggtt tcttaataga ttcaccacta ctttgaatga ctttaacctt 10680
   gtggcaatga agtacaacta tgaacctttg acacaagatc atgttgacat attgggacct 10740
   ctttctgctc aaacaggaat tgccgtctta gatatgtgtg ctgctttgaa agagctgctg 10800
   cagaatggta tgaatggtcg tactatcctt ggtagcacta ttttagaaga tgagtttaca 10860
   ccatttgatg ttgttagaca atgctctggt gttaccttcc aaggtaagtt caagaaaatt 10920
   gttaagggca ctcatcattg gatgctttta actttcttga catcactatt gattcttgtt 10980
   caaagtacac agtggtcact gtttttcttt gtttacgaga atgctttctt gccatttact 11040
   cttggtatta tggcaattgc tgcatgtgct atgctgcttg ttaagcataa gcacgcattc 11100
   ttgtgcttgt ttctgttacc ttctcttgca acagttgctt actttaatat ggtctacatg 11160
   cctgctagct gggtgatgcg tatcatgaca tggcttgaat tggctgacac tagcttgtct 11220
   ggttataggc ttaaggattg tgttatgtat gcttcagctt tagttttgct tattctcatg 11280
   acagctcgca ctgtttatga tgatgctgct agacgtgttt ggacactgat gaatgtcatt 11340
   acacttgttt acaaagtcta ctatggtaat gctttagatc aagctatttc catgtgggcc 11400
   ttagttattt ctgtaacctc taactattct ggtgtcgtta cgactatcat gtttttagct 11460
   agagctatag tgtttgtgtg tgttgagtat tacccattgt tatttattac tggcaacacc 11520
   ttacagtgta tcatgcttgt ttattgtttc ttaggctatt gttgctgctg ctactttggc 11580
   cttttctgtt tactcaaccg ttacttcagg cttactcttg gtgtttatga ctacttggtc 11640
   tctacacaag aatttaggta tatgaactcc caggggcttt tgcctcctaa gagtagtatt 11700
   gatgctttca agcttaacat taagttgttg ggtattggag gtaaaccatg tatcaaggtt 11760
   gctactgtac agtctaaaat gtctgacgta aagtgcacat ctgtggtact gctctcggtt 11820
   cttcaacaac ttagagtaga gtcatcttct aaattgtggg cacaatgtgt acaactccac 11880
   aatgatattc ttcttgcaaa agacacaact gaagctttcg agaagatggt ttctcttttg 11940
   tctgttttgc tatccatgca gggtgctgta gacattaata ggttgtgcga ggaaatgctc 12000
   gataaccgtg ctactcttca ggctattgct tcagaattta gttctttacc atcatatgcc 12060
   gcttatgcca ctgcccagga ggcctatgag caggctgtag ctaatggtga ttctgaagtc 12120
   gttctcaaaa agttaaagaa atctttgaat gtggctaaat ctgagtttga ccgtgatgct 12180
   gccatgcaac gcaagttgga aaagatggca gatcaggcta tgacccaaat gtacaaacag 12240
   gcaagatctg aggacaagag ggcaaaagta actagtgcta tgcaaacaat gctcttcact 12300
   atgcttagga agcttgataa tgatgcactt aacaacatta tcaacaatgc gcgtgatggt 12360
   tgtgttccac tcaacatcat accattgact acagcagcca aactcatggt tgttgtccct 12420
   gattatggta cctacaagaa cacttgtgat ggtaacacct ttacatatgc atctgcactc 12480
   tgggaaatcc agcaagttgt tgatgcggat agcaagattg ttcaacttag tgaaattaac 12540
   atggacaatt caccaaattt ggcttggcct cttattgtta cagctctaag agccaactca 12600
   gctgttaaac tacagaataa tgaactgagt ccagtagcac tacgacagat gtcctgtgcg 12660
   gctggtacca cacaaacagc ttgtactgat gacaatgcac ttgcctacta taacaattcg 12720
   aagggaggta ggtttgtgct ggcattacta tcagaccacc aagatctcaa atgggctaga 12780
   ttccctaaga gtgatggtac aggtacaatt tacacagaac tggaaccacc ttgtaggttt 12840
   gttacagaca caccaaaagg gcctaaagtg aaatacttgt acttcatcaa aggcttaaac 12900
   aacctaaata gaggtatggt gctgggcagt ttagctgcta cagtacgtct tcaggctgga 12960
   aatgctacag aagtacctgc caattcaact gtgctttcct tctgtgcttt tgcagtagac 13020
   cctgctaaag catataagga ttacctagca agtggaggac aaccaatcac caactgtgtg 13080
   aagatgttgt gtacacacac tggtacagga caggcaatta ctgtaacacc agaagctaac 13140
   atggaccaag agtcctttgg tggtgcttca tgttgtctgt attgtagatg ccacattgac 13200
   catccaaatc ctaaaggatt ctgtgacttg aaaggtaagt acgtccaaat acctaccact 13260
   tgtgctaatg acccagtggg ttttacactt agaaacacag tctgtaccgt ctgcggaatg 13320
   tggaaaggtt atggctgtag ttgtgaccaa ctccgcgaac ccttgatgca gtctgcggat 13380
   gcatcaacgt ttttaaacgg gtttgcggtg taagtgcagc ccgtcttaca ccgtgcggca 13440
   caggcactag tactgatgtc gtctacaggg cttttgatat ttacaacgaa aaagttgctg 13500
   gttttgcaaa gttcctaaaa actaattgct gtcgcttcca ggagaaggat gaggaaggca 13560
   atttattaga ctcttacttt gtagttaaga ggcatactat gtctaactac caacatgaag 13620
   agactattta taacttggtt aaagattgtc cagcggttgc tgtccatgac tttttcaagt 13680
   ttagagtaga tggtgacatg gtaccacata tatcacgtca gcgtctaact aaatacacaa 13740
   tggctgattt agtctatgct ctacgtcatt ttgatgaggg taattgtgat acattaaaag 13800
   aaatactcgt cacatacaat tgctgtgatg atgattattt caataagaag gattggtatg 13860
   acttcgtaga gaatcctgac atcttacgcg tatatgctaa cttaggtgag cgtgtacgcc 13920
   aatcattatt aaagactgta caattctgcg atgctatgcg tgatgcaggc attgtaggcg 13980
   tactgacatt agataatcag gatcttaatg ggaactggta cgatttcggt gatttcgtac 14040
   aagtagcacc aggctgcgga gttcctattg tggattcata ttactcattg ctgatgccca 14100
   tcctcacttt gactagggca ttggctgctg agtcccatat ggatgctgat ctcgcaaaac 14160
   cacttattaa gtgggatttg ctgaaatatg attttacgga agagagactt tgtctcttcg 14220
   accgttattt taaatattgg gaccagacat accatcccaa ttgtattaac tgtttggatg 14280
   ataggtgtat ccttcattgt gcaaacttta atgtgttatt ttctactgtg tttccaccta 14340
   caagttttgg accactagta agaaaaatat ttgtagatgg tgttcctttt gttgtttcaa 14400
   ctggatacca ttttcgtgag ttaggagtcg tacataatca ggatgtaaac ttacatagct 14460
   cgcgtctcag tttcaaggaa cttttagtgt atgctgctga tccagctatg catgcagctt 14520
   ctggcaattt attgctagat aaacgcacta catgcttttc agtagctgca ctaacaaaca 14580
   atgttgcttt tcaaactgtc aaacccggta attttaataa agacttttat gactttgctg 14640
   tgtctaaagg tttctttaag gaaggaagtt ctgttgaact aaaacacttc ttctttgctc 14700
   aggatggcaa cgctgctatc agtgattatg actattatcg ttataatctg ccaacaatgt 14760
   gtgatatcag acaactccta ttcgtagttg aagttgttga taaatacttt gattgttacg 14820
   atggtggctg tattaatgcc aaccaagtaa tcgttaacaa tctggataaa tcagctggtt 14880
   tcccatttaa taaatggggt aaggctagac tttattatga ctcaatgagt tatgaggatc 14940
   aagatgcact tttcgcgtat actaagcgta atgtcatccc tactataact caaatgaatc 15000
   ttaagtatgc cattagtgca aagaatagag ctcgcaccgt agctggtgtc tctatctgta 15060
   gtactatgac aaatagacag tttcatcaga aattattgaa gtcaatagcc gccactagag 15120
   gagctactgt ggtaattgga acaagcaagt tttacggtgg ctggcataat atgttaaaaa 15180
   ctgtttacag tgatgtagaa actccacacc ttatgggttg ggattatcca aaatgtgaca 15240
   gagccatgcc taacatgctt aggataatgg cctctcttgt tcttgctcgc aaacataaca 15300
   cttgctgtaa cttatcacac cgtttctaca ggttagctaa cgagtgtgcg caagtattaa 15360
   gtgagatggt catgtgtggc ggctcactat atgttaaacc aggtggaaca tcatccggtg 15420
   atgctacaac tgcttatgct aatagtgtct ttaacatttg tcaagctgtt acagccaatg 15480
   taaatgcact tctttcaact gatggtaata agatagctga caagtatgtc cgcaatctac 15540
   aacacaggct ctatgagtgt ctctatagaa atagggatgt tgatcatgaa ttcgtggatg 15600
   agttttacgc ttacctgcgt aaacatttct ccatgatgat tctttctgat gatgccgttg 15660
   tgtgctataa cagtaactat gcggctcaag gtttagtagc tagcattaag aactttaagg 15720
   cagttcttta ttatcaaaat aatgtgttca tgtctgaggc aaaatgttgg actgagactg 15780
   accttactaa aggacctcac gaattttgct cacagcatac aatgctagtt aaacaaggag 15840
   atgattacgt gtacctgcct tacccagatc catcaagaat attaggcgca ggctgttttg 15900
   tcgatgatat tgtcaaaaca gatggtacac ttatgattga aaggttcgtg tcactggcta 15960
   ttgatgctta cccacttaca aaacatccta atcaggagta tgctgatgtc tttcacttgt 16020
   atttacaata cattagaaag ttacatgatg agcttactgg ccacatgttg gacatgtatt 16080
   ccgtaatgct aactaatgat aacacctcac ggtactggga acctgagttt tatgaggcta 16140
   tgtacacacc acatacagtc ttgcaggctg taggtgcttg tgtattgtgc aattcacaga 16200
   cttcacttcg ttgcggtgcc tgtattagga gaccattcct atgttgcaag tgctgctatg 16260
   accatgtcat ttcaacatca cacaaattag tgttgtctgt taatccctat gtttgcaatg 16320
   ccccaggttg tgatgtcact gatgtgacac aactgtatct aggaggtatg agctattatt 16380
   gcaagtcaca taagcctccc attagttttc cattatgtgc taatggtcag gtttttggtt 16440
   tatacaaaaa cacatgtgta ggcagtgaca atgtcactga cttcaatgcg atagcaacat 16500
   gtgattggac taatgctggc gattacatac ttgccaacac ttgtactgag agactcaagc 16560
   ttttcgcagc agaaacgctc aaagccactg aggaaacatt taagctgtca tatggtattg 16620
   ccactgtacg cgaagtactc tctgacagag aattgcatct ttcatgggag gttggaaaac 16680
   ctagaccacc attgaacaga aactatgtct ttactggtta ccgtgtaact aaaaatagta 16740
   aagtacagat tggagagtac acctttgaaa aaggtgacta tggtgatgct gttgtgtaca 16800
   gaggtactac gacatacaag ttgaatgttg gtgattactt tgtgttgaca tctcacactg 16860
   taatgccact tagtgcacct actctagtgc cacaagagca ctatgtgaga attactggct 16920
   tgtacccaac actcaacatc tcagatgagt tttctagcaa tgttgcaaat tatcaaaagg 16980
   tcggcatgca aaagtactct acactccaag gaccacctgg tactggtaag agtcattttg 17040
   ccatcggact tgctctctat tacccatctg ctcgcatagt gtatacggca tgctctcatg 17100
   cagctgttga tgccctatgt gaaaaggcat taaaatattt gcccatagat aaatgtagta 17160
   gaatcatacc tgcgcgtgcg cgcgtagagt gttttgataa attcaaagtg aattcaacac 17220
   tagaacagta tgttttctgc actgtaaatg cattgccaga aacaactgct gacattgtag 17280
   tctttgatga aatctctatg gctactaatt atgacttgag tgttgtcaat gctagacttc 17340
   gtgcaaaaca ctacgtctat attggcgatc ctgctcaatt accagccccc cgcacattgc 17400
   tgactaaagg cacactagaa ccagaatatt ttaattcagt gtgcagactt atgaaaacaa 17460
   taggtccaga catgttcctt ggaacttgtc gccgttgtcc tgctgaaatt gttgacactg 17520
   tgagtgcttt agtttatgac aataagctaa aagcacacaa ggataagtca gctcaatgct 17580
   tcaaaatgtt ctacaaaggt gttattacac atgatgtttc atctgcaatc aacagacctc 17640
   aaataggcgt tgtaagagaa tttcttacac gcaatcctgc ttggagaaaa gctgttttta 17700
   tctcacctta taattcacag aacgctgtag cttcaaaaat cttaggattg cctacgcaga 17760
   ctgttgattc atcacagggt tctgaatatg actatgtcat attcacacaa actactgaaa 17820
   cagcacactc ttgtaatgtc aaccgcttca atgtggctat cacaagggca aaaattggca 17880
   ttttgtgcat aatgtctgat agagatcttt atgacaaact gcaatttaca agtctagaaa 17940
   taccacgtcg caatgtggct acattacaag cagaaaatgt aactggactt tttaaggact 18000
   gtagtaagat cattactggt cttcatccta cacaggcacc tacacacctc agcgttgata 18060
   taaagttcaa gactgaagga ttatgtgttg acataccagg cataccaaag gacatgacct 18120
   accgtagact catctctatg atgggtttca aaatgaatta ccaagtcaat ggttacccta 18180
   atatgtttat cacccgcgaa gaagctattc gtcacgttcg tgcgtggatt ggctttgatg 18240
   tagagggctg tcatgcaact agagatgctg tgggtactaa cctacctctc cagctaggat 18300
   tttctacagg tgttaactta gtagctgtac cgactggtta tgttgacact gaaaataaca 18360
   cagaattcac cagagttaat gcaaaacctc caccaggtga ccagtttaaa catcttatac 18420
   cactcatgta taaaggcttg ccctggaatg tagtgcgtat taagatagta caaatgctca 18480
   gtgatacact gaaaggattg tcagacagag tcgtgttcgt cctttgggcg catggctttg 18540
   agcttacatc aatgaagtac tttgtcaaga ttggacctga aagaacgtgt tgtctgtgtg 18600
   acaaacgtgc aacttgcttt tctacttcat cagatactta tgcctgctgg aatcattctg 18660
   tgggttttga ctatgtctat aacccattta tgattgatgt tcagcagtgg ggctttacgg 18720
   gtaaccttca gagtaaccat gaccaacatt gccaggtaca tggaaatgca catgtggcta 18780
   gttgtgatgc tatcatgact agatgtttag cagtccatga gtgctttgtt aagcgcgttg 18840
   attggtctgt tgaataccct attataggag atgaactgag ggttaattct gcttgcagaa 18900
   aagtacaaca catggttgtg aagtctgcat tgcttgctga taagtttcca gttcttcatg 18960
   acattggaaa tccaaaggct atcaagtgtg tgcctcaggc tgaagtagaa tggaagttct 19020
   acgatgctca gccatgtagt gacaaagctt acaaaataga ggaactcttc tattcttatg 19080
   ctacacatca cgataaattc actgatggtg tttgtttgtt ttggaattgt aacgttgatc 19140
   gttacccagc caatgcaatt gtgtgtaggt ttgacacaag agtcttgtca aacttgaact 19200
   taccaggctg tgatggtggt agtttgtatg tgaataagca tgcattccac actccagctt 19260
   tcgataaaag tgcatttact aatttaaagc aattgccttt cttttactat tctgatagtc 19320
   cttgtgagtc tcatggcaaa caagtagtgt cggatattga ttatgttcca ctcaaatctg 19380
   ctacgtgtat tacacgatgc aatttaggtg gtgctgtttg cagacaccat gcaaatgagt 19440
   accgacagta cttggatgca tataatatga tgatttctgc tggatttagc ctatggattt 19500
   acaaacaatt tgatacttat aacctgtgga atacatttac caggttacag agtttagaaa 19560
   atgtggctta taatgttgtt aataaaggac actttgatgg acacgccggc gaagcacctg 19620
   tttccatcat taataatgct gtttacacaa aggtagatgg tattgatgtg gagatctttg 19680
   aaaataagac aacacttcct gttaatgttg catttgagct ttgggctaag cgtaacatta 19740
   aaccagtgcc agagattaag atactcaata atttgggtgt tgatatcgct gctaatactg 19800
   taatctggga ctacaaaaga gaagccccag cacatgtatc tacaataggt gtctgcacaa 19860
   tgactgacat tgccaagaaa cctactgaga gtgcttgttc ttcacttact gtcttgtttg 19920
   atggtagagt ggaaggacag gtagaccttt ttagaaacgc ccgtaatggt gttttaataa 19980
   cagaaggttc agtcaaaggt ctaacacctt caaagggacc agcacaagct agcgtcaatg 20040
   gagtcacatt aattggagaa tcagtaaaaa cacagtttaa ctactttaag aaagtagacg 20100
   gcattattca acagttgcct gaaacctact ttactcagag cagagactta gaggatttta 20160
   agcccagatc acaaatggaa actgactttc tcgagctcgc tatggatgaa ttcatacagc 20220
   gatataagct cgagggctat gccttcgaac acatcgttta tggagatttc agtcatggac 20280
   aacttggcgg tcttcattta atgataggct tagccaagcg ctcacaagat tcaccactta 20340
   aattagagga ttttatccct atggacagca cagtgaaaaa ttacttcata acagatgcgc 20400
   aaacaggttc atcaaaatgt gtgtgttctg tgattgatct tttacttgat gactttgtcg 20460
   agataataaa gtcacaagat ttgtcagtga tttcaaaagt ggtcaaggtt acaattgact 20520
   atgctgaaat ttcattcatg ctttggtgta aggatggaca tgttgaaacc ttctacccaa 20580
   aactacaagc aagtcaagcg tggcaaccag gtgttgcgat gcctaacttg tacaagatgc 20640
   aaagaatgct tcttgaaaag tgtgaccttc agaattatgg tgaaaatgct gttataccaa 20700
   aaggaataat gatgaatgtc gcaaagtata ctcaactgtg tcaatactta aatacactta 20760
   ctttagctgt accctacaac atgagagtta ttcactttgg tgctggctct gataaaggag 20820
   ttgcaccagg tacagctgtg ctcagacaat ggttgccaac tggcacacta cttgtcgatt 20880
   cagatcttaa tgacttcgtc tccgacgcag attctacttt aattggagac tgtgcaacag 20940
   tacatacggc taataaatgg gaccttatta ttagcgatat gtatgaccct aggaccaaac 21000
   atgtgacaaa agagaatgac tctaaagaag ggtttttcac ttatctgtgt ggatttataa 21060
   agcaaaaact agccctgggt ggttctatag ctgtaaagat aacagagcat tcttggaatg 21120
   ctgaccttta caagcttatg ggccatttct catggtggac agcttttgtt acaaatgtaa 21180
   atgcatcatc atcggaagca tttttaattg gggctaacta tcttggcaag ccgaaggaac 21240
   aaattgatgg ctataccatg catgctaact acattttctg gaggaacaca aatcctatcc 21300
   agttgtcttc ctattcactc tttgacatga gcaaatttcc tcttaaatta agaggaactg 21360
   ctgtaatgtc tcttaaggag aatcaaatca atgatatgat ttattctctt ctggaaaaag 21420
   gtaggcttat cattagagaa aacaacagag ttgtggtttc aagtgatatt cttgttaaca 21480
   actaaacgaa catgtttatt ttcttattat ttcttactct cactagtggt agtgaccttg 21540
   accggtgcac cacttttgat gatgttcaag ctcctaatta cactcaacat acttcatcta 21600
   tgaggggggt ttactatcct gatgaaattt ttagatcaga cactctttat ttaactcagg 21660
   atttatttct tccattttat tctaatgtta cagggtttca tactattaat catacgtttg 21720
   gcaaccctgt catacctttt aaggatggta tttattttgc tgccacagag aaatcaaatg 21780
   ttgtccgtgg ttgggttttt ggttctacca tgaacaacaa gtcacagtcg gtgattatta 21840
   ttaacaattc tactaatgtt gttatacgag catgtaactt tgaattgtgt gacaaccctt 21900
   tctttgctgt ttctaaaccc atgggtacac agacacatac tatgatattc gataatgcat 21960
   ttaattgcac tttcgagtac atatctgatg ccttttcgct tgatgtttca gaaaagtcag 22020
   gtaattttaa acacttacga gagtttgtgt ttaaaaataa agatgggttt ctctatgttt 22080
   ataagggcta tcaacctata gatgtagttc gtgatctacc ttctggtttt aacactttga 22140
   aacctatttt taagttgcct cttggtatta acattacaaa ttttagagcc attcttacag 22200
   ccttttcacc tgctcaagac atttggggca cgtcagctgc agcctatttt gttggctatt 22260
   taaagccaac tacatttatg ctcaagtatg atgaaaatgg tacaatcaca gatgctgttg 22320
   attgttctca aaatccactt gctgaactca aatgctctgt taagagcttt gagattgaca 22380
   aaggaattta ccagacctct aatttcaggg ttgttccctc aggagatgtt gtgagattcc 22440
   ctaatattac aaacttgtgt ccttttggag aggtttttaa tgctactaaa ttcccttctg 22500
   tctatgcatg ggagagaaaa aaaatttcta attgtgttgc tgattactct gtgctctaca 22560
   actcaacatt tttttcaacc tttaagtgct atggcgtttc tgccactaag ttgaatgatc 22620
   tttgcttctc caatgtctat gcagattctt ttgtagtcaa gggagatgat gtaagacaaa 22680
   tagcgccagg acaaactggt gttattgctg attataatta taaattgcca gatgatttca 22740
   tgggttgtgt ccttgcttgg aatactagga acattgatgc tacttcaact ggtaattata 22800
   attataaata taggtatctt agacatggca agcttaggcc ctttgagaga gacatatcta 22860
   atgtgccttt ctcccctgat ggcaaacctt gcaccccacc tgctcttaat tgttattggc 22920
   cattaaatga ttatggtttt tacaccacta ctggcattgg ctaccaacct tacagagttg 22980
   tagtactttc ttttgaactt ttaaatgcac cggccacggt ttgtggacca aaattatcca 23040
   ctgaccttat taagaaccag tgtgtcaatt ttaattttaa tggactcact ggtactggtg 23100
   tgttaactcc ttcttcaaag agatttcaac catttcaaca atttggccgt gatgtttctg 23160
   atttcactga ttccgttcga gatcctaaaa catctgaaat attagacatt tcaccttgct 23220
   cttttggggg tgtaagtgta attacacctg gaacaaatgc ttcatctgaa gttgctgttc 23280
   tatatcaaga tgttaactgc actgatgttt ctacagcaat tcatgcagat caactcacac 23340
   cagcttggcg catatattct actggaaaca atgtattcca gactcaagca ggctgtctta 23400
   taggagctga gcatgtcgac acttcttatg agtgcgacat tcctattgga gctggcattt 23460
   gtgctagtta ccatacagtt tctttattac gtagtactag ccaaaaatct attgtggctt 23520
   atactatgtc tttaggtgct gatagttcaa ttgcttactc taataacacc attgctatac 23580
   ctactaactt ttcaattagc attactacag aagtaatgcc tgtttctatg gctaaaacct 23640
   ccgtagattg taatatgtac atctgcggag attctactga atgtgctaat ttgcttctcc 23700
   aatatggtag cttttgcaca caactaaatc gtgcactctc aggtattgct gctgaacagg 23760
   atcgcaacac acgtgaagtg ttcgctcaag tcaaacaaat gtacaaaacc ccaactttga 23820
   aatattttgg tggttttaat ttttcacaaa tattacctga ccctctaaag ccaactaaga 23880
   ggtcttttat tgaggacttg ctctttaata aggtgacact cgctgatgct ggcttcatga 23940
   agcaatatgg cgaatgccta ggtgatatta atgctagaga tctcatttgt gcgcagaagt 24000
   tcaatggact tacagtgttg ccacctctgc tcactgatga tatgattgct gcctacactg 24060
   ctgctctagt tagtggtact gccactgctg gatggacatt tggtgctggc gctgctcttc 24120
   aaataccttt tgctatgcaa atggcatata ggttcaatgg cattggagtt acccaaaatg 24180
   ttctctatga gaaccaaaaa caaatcgcca accaatttaa caaggcgatt agtcaaattc 24240
   aagaatcact tacaacaaca tcaactgcat tgggcaagct gcaagacgtt gttaaccaga 24300
   atgctcaagc attaaacaca cttgttaaac aacttagctc taattttggt gcaatttcaa 24360
   gtgtgctaaa tgatatcctt tcgcgacttg ataaagtcga ggcggaggta caaattgaca 24420
   ggttaattac aggcagactt caaagccttc aaacctatgt aacacaacaa ctaatcaggg 24480
   ctgctgaaat cagggcttct gctaatcttg ctgctactaa aatgtctgag tgtgttcttg 24540
   gacaatcaaa aagagttgac ttttgtggaa agggctacca ccttatgtcc ttcccacaag 24600
   cagccccgca tggtgttgtc ttcctacatg tcacgtatgt gccatcccag gagaggaact 24660
   tcaccacagc gccagcaatt tgtcatgaag gcaaagcata cttccctcgt gaaggtgttt 24720
   ttgtgtttaa tggcacttct tggtttatta cacagaggaa cttcttttct ccacaaataa 24780
   ttactacaga caatacattt gtctcaggaa attgtgatgt cgttattggc atcattaaca 24840
   acacagttta tgatcctctg caacctgagc ttgactcatt caaagaagag ctggacaagt 24900
   acttcaaaaa tcatacatca ccagatgttg atcttggcga catttcaggc attaacgctt 24960
   ctgtcgtcaa cattcaaaaa gaaattgacc gcctcaatga ggtcgctaaa aatttaaatg 25020
   aatcactcat tgaccttcaa gaattgggaa aatatgagca atatattaaa tggccttggt 25080
   atgtttggct cggcttcatt gctggactaa ttgccatcgt catggttaca atcttgcttt 25140
   gttgcatgac tagttgttgc agttgcctca agggtgcatg ctcttgtggt tcttgctgca 25200
   agtttgatga ggatgactct gagccagttc tcaagggtgt caaattacat tacacataaa 25260
   cgaacttatg gatttgttta tgagattttt tactcttgga tcaattactg cacagccagt 25320
   aaaaattgac aatgcttctc ctgcaagtac tgttcatgct acagcaacga taccgctaca 25380
   agcctcactc cctttcggat ggcttgttat tggcgttgca tttcttgctg tttttcagag 25440
   cgctaccaaa ataattgcgc tcaataaaag atggcagcta gccctttata agggcttcca 25500
   gttcatttgc aatttactgc tgctatttgt taccatctat tcacatcttt tgcttgtcgc 25560
   tgcaggtatg gaggcgcaat ttttgtacct ctatgccttg atatattttc tacaatgcat 25620
   caacgcatgt agaattatta tgagatgttg gctttgttgg aagtgcaaat ccaagaaccc 25680
   attactttat gatgccaact actttgtttg ctggcacaca cataactatg actactgtat 25740
   accatataac agtgtcacag atacaattgt cgttactgaa ggtgacggca tttcaacacc 25800
   aaaactcaaa gaagactacc aaattggtgg ttattctgag gataggcact caggtgttaa 25860
   agactatgtc gttgtacatg gctatttcac cgaagtttac taccagcttg agtctacaca 25920
   aattactaca gacactggta ttgaaaatgc tacattcttc atctttaaca agcttgttaa 25980
   agacccaccg aatgtgcaaa tacacacaat cgacggctct tcaggagttg ctaatccagc 26040
   aatggatcca atttatgatg agccgacgac gactactagc gtgcctttgt aagcacaaga 26100
   aagtgagtac gaacttatgt actcattcgt ttcggaagaa acaggtacgt taatagttaa 26160
   tagcgtactt ctttttcttg ctttcgtggt attcttgcta gtcacactag ccatccttac 26220
   tgcgcttcga ttgtgtgcgt actgctgcaa tattgttaac gtgagtttag taaaaccaac 26280
   ggtttacgtc tactcgcgtg ttaaaaatct gaactcttct gaaggagttc ctgatcttct 26340
   ggtctaaacg aactaactat tattattatt ctgtttggaa ctttaacatt gcttatcatg 26400
   gcagacaacg gtactattac cgttgaggag cttaaacaac tcctggaaca atggaaccta 26460
   gtaataggtt tcctattcct agcctggatt atgttactac aatttgccta ttctaatcgg 26520
   aacaggtttt tgtacataat aaagcttgtt ttcctctggc tcttgtggcc agtaacactt 26580
   gcttgttttg tgcttgctgc tgtctacaga attaattggg tgactggcgg gattgcgatt 26640
   gcaatggctt gtattgtagg cttgatgtgg cttagctact tcgttgcttc cttcaggctg 26700
   tttgctcgta cccgctcaat gtggtcattc aacccagaaa caaacattct tctcaatgtg 26760
   cctctccggg ggacaattgt gaccagaccg ctcatggaaa gtgaacttgt cattggtgct 26820
   gtgatcattc gtggtcactt gcgaatggcc ggacactccc tagggcgctg tgacattaag 26880
   gacctgccaa aagagatcac tgtggctaca tcacgaacgc tttcttatta caaattagga 26940
   gcgtcgcagc gtgtaggcac tgattcaggt tttgctgcat acaaccgcta ccgtattgga 27000
   aactataaat taaatacaga ccacgccggt agcaacgaca atattgcttt gctagtacag 27060
   taagtgacaa cagatgtttc atcttgttga cttccaggtt acaatagcag agatattgat 27120
   tatcattatg aggactttca ggattgctat ttggaatctt gacgttataa taagttcaat 27180
   agtgagacaa ttatttaagc ctctaactaa gaagaattat tcggagttag atgatgaaga 27240
   acctatggag ttagattatc cataaaacga acatgaaaat tattctcttc ctgacattga 27300
   ttgtatttac atcttgcgag ctatatcact atcaggagtg tgttagaggt acgactgtac 27360
   tactaaaaga accttgccca tcaggaacat acgagggcaa ttcaccattt caccctcttg 27420
   ctgacaataa atttgcacta acttgcacta gcacacactt tgcttttgct tgtgctgacg 27480
   gtactcgaca tacctatcag ctgcgtgcaa gatcagtttc accaaaactt ttcatcagac 27540
   aagaggaggt tcaacaagag ctctactcgc cactttttct cattgttgct gctctagtat 27600
   ttttaatact ttgcttcacc attaagagaa agacagaatg aatgagctca ctttaattga 27660
   cttctatttg tgctttttag cctttctgct attccttgtt ttaataatgc ttattatatt 27720
   ttggttttca ctcgaaatcc aggatctaga agaaccttgt accaaagtct aaacgaacat 27780
   gaaacttctc attgttttga cttgtatttc tctatgcagt tgcatatgca ctgtagtaca 27840
   gcgctgtgca tctaataaac ctcatgtgct tgaagatcct tgtaaggtac aacactaggg 27900
   gtaatactta tagcactgct tggctttgtg ctctaggaaa ggttttacct tttcatagat 27960
   ggcacactat ggttcaaaca tgcacaccta atgttactat caactgtcaa gatccagctg 28020
   gtggtgcgct tatagctagg tgttggtacc ttcatgaagg tcaccaaact gctgcattta 28080
   gagacgtact tgttgtttta aataaacgaa caaattaaaa tgtctgataa tggaccccaa 28140
   tcaaaccaac gtagtgcccc ccgcattaca tttggtggac ccacagattc aactgacaat 28200
   aaccagaatg gaggacgcaa tggggcaagg ccaaaacagc gccgacccca aggtttaccc 28260
   aataatactg cgtcttggtt cacagctctc actcagcatg gcaaggagga acttagattc 28320
   cctcgaggcc agggcgttcc aatcaacacc aatagtggtc cagatgacca aattggctac 28380
   taccgaagag ctacccgacg agttcgtggt ggtgacggca aaatgaaaga gctcagcccc 28440
   agatggtact tctattacct aggaactggc ccagaagctt cacttcccta cggcgctaac 28500
   aaagaaggca tcgtatgggt tgcaactgag ggagccttga atacacccaa agaccacatt 28560
   ggcacccgca atcctaataa caatgctgcc accgtgctac aacttcctca aggaacaaca 28620
   ttgccaaaag gcttctacgc agagggaagc agaggcggca gtcaagcctc ttctcgctcc 28680
   tcatcacgta gtcgcggtaa ttcaagaaat tcaactcctg gcagcagtag gggaaattct 28740
   cctgctcgaa tggctagcgg aggtggtgaa actgccctcg cgctattgct gctagacaga 28800
   ttgaaccagc ttgagagcaa agtttctggt aaaggccaac aacaacaagg ccaaactgtc 28860
   actaagaaat ctgctgctga ggcatctaaa aagcctcgcc aaaaacgtac tgccacaaaa 28920
   cagtacaacg tcactcaagc atttgggaga cgtggtccag aacaaaccca aggaaatttc 28980
   ggggaccaag acctaatcag acaaggaact gattacaaac attggccgca aattgcacaa 29040
   tttgctccaa gtgcctctgc attctttgga atgtcacgca ttggcatgga agtcacacct 29100
   tcgggaacat ggctgactta tcatggagcc attaaattgg atgacaaaga tccacaattc 29160
   aaagacaacg tcatactgct gaacaagcac attgacgcat acaaaacatt cccaccaaca 29220
   gagcctaaaa aggacaaaaa gaaaaagact gatgaagctc agcctttgcc gcagagacaa 29280
   aagaagcagc ccactgtgac tcttcttcct gcggctgaca tggatgattt ctccagacaa 29340
   cttcaaaatt ccatgagtgg agcttctgct gattcaactc aggcataaac actcatgatg 29400
   accacacaag gcagatgggc tatgtaaacg ttttcgcaat tccgtttacg atacatagtc 29460
   tactcttgtg cagaatgaat tctcgtaact aaacagcaca agtaggttta gttaacttta 29520
   atctcacata gcaatcttta atcaatgtgt aacattaggg aggacttgaa agagccacca 29580
   cattttcatc gaggccacgc ggagtacgat cgagggtaca gtgaataatg ctagggagag 29640
   ctgcctatat ggaagagccc taatgtgtaa aattaatttt agtagtgcta tccccatgtg 29700
   attttaatag cttcttagga gaatgacaaa aaaaaaaaaa aaaaaa 29746
<210> 2
   <211> 3945
   <212> DNA
   <213> CORONAVIRUS
<220>
   <221> CDS
   <222> (89)..(3853)
   <223>
<400> 2
   ttctcttctg gaaaaaggta ggcttatcat tagagaaaac aacagagttg tggtttcaag      60
   tgatattctt gttaacaact aaacgaac atg ttt att ttc tta tta ttt ctt        112
                                   Met Phe Ile Phe Leu Leu Phe Leu
                                   1               5
   act ctc act agt ggt agt gac ctt gac cgg tgc acc act ttt gat gat      160
   Thr Leu Thr Ser Gly Ser Asp Leu Asp Arg Cys Thr Thr Phe Asp Asp
       10                  15                  20
   gtt caa gct cct aat tac act caa cat act tca tct atg agg ggg gtt      208
   Val Gln Ala Pro Asn Tyr Thr Gln His Thr Ser Ser Met Arg Gly Val
   25                  30                  35                  40
   tac tat cct gat gaa att ttt aga tca gac act ctt tat tta act cag      256
   Tyr Tyr Pro Asp Glu Ile Phe Arg Ser Asp Thr Leu Tyr Leu Thr Gln
                   45                  50                  55
   gat tta ttt ctt cca ttt tat tct aat gtt aca ggg ttt cat act att      304
   Asp Leu Phe Leu Pro Phe Tyr Ser Asn Val Thr Gly Phe His Thr Ile
               60                  65                  70
   aat cat acg ttt ggc aac cct gtc ata cct ttt aag gat ggt att tat      352
   Asn His Thr Phe Gly Asn Pro Val Ile Pro Phe Lys Asp Gly Ile Tyr
           75                  80                  85
   ttt gct gcc aca gag aaa tca aat gtt gtc cgt ggt tgg gtt ttt ggt      400
   Phe Ala Ala Thr Glu Lys Ser Asn Val Val Arg Gly Trp Val Phe Gly
       90                  95                  100
   tct acc atg aac aac aag tca cag tcg gtg att att att aac aat tct      448
   Ser Thr Met Asn Asn Lys Ser Gln Ser Val Ile Ile Ile Asn Asn Ser
   105                 110                 115                 120
   act aat gtt gtt ata cga gca tgt aac ttt gaa ttg tgt gac aac cct      496
   Thr Asn Val Val Ile Arg Ala Cys Asn Phe Glu Leu Cys Asp Asn Pro
                   125                 130                 135
   ttc ttt gct gtt tct aaa ccc atg ggt aca cag aca cat act atg ata      544
   Phe Phe Ala Val Ser Lys Pro Met Gly Thr Gln Thr His Thr Met Ile
               140                 145                 150
   ttc gat aat gca ttt aat tgc act ttc gag tac ata tct gat gcc ttt      592
   Phe Asp Asn Ala Phe Asn Cys Thr Phe Glu Tyr Ile Ser Asp Ala Phe
           155                 160                 165
   tcg ctt gat gtt tca gaa aag tca ggt aat ttt aaa cac tta cga gag      640
   Ser Leu Asp Val Ser Glu Lys Ser Gly Asn Phe Lys His Leu Arg Glu
       170                 175                 180
   ttt gtg ttt aaa aat aaa gat ggg ttt ctc tat gtt tat aag ggc tat      688
   Phe Val Phe Lys Asn Lys Asp Gly Phe Leu Tyr Val Tyr Lys Gly Tyr
   185                 190                 195                 200
   caa cct ata gat gta gtt cgt gat cta cct tct ggt ttt aac act ttg      736
   Gln Pro Ile Asp Val Val Arg Asp Leu Pro Ser Gly Phe Asn Thr Leu
                   205                 210                 215
   aaa cct att ttt aag ttg cct ctt ggt att aac att aca aat ttt aga     784
   Lys Pro Ile Phe Lys Leu Pro Leu Gly Ile Asn Ile Thr Asn Phe Arg
               220                 225                 230
   gcc att ctt aca gcc ttt tca cct gct caa gac att tgg ggc acg tca     832
   Ala Ile Leu Thr Ala Phe Ser Pro Ala Gln Asp Ile Trp Gly Thr Ser
           235                 240                 245
   gct gca gcc tat ttt gtt ggc tat tta aag cca act aca ttt atg ctc     880
   Ala Ala Ala Tyr Phe Val Gly Tyr Leu Lys Pro Thr Thr Phe Met Leu
       250                 255                 260
   aag tat gat gaa aat ggt aca atc aca gat gct gtt gat tgt tct caa     928
   Lys Tyr Asp Glu Asn Gly Thr Ile Thr Asp Ala Val Asp Cys Ser Gln
   265                 270                 275                 280
   aat cca ctt gct gaa ctc aaa tgc tct gtt aag agc ttt gag att gac     976
   Asn Pro Leu Ala Glu Leu Lys Cys Ser Val Lys Ser Phe Glu Ile Asp
                   285                 290                 295
   aaa gga att tac cag acc tct aat ttc agg gtt gtt ccc tca gga gat    1024
   Lys Gly Ile Tyr Gln Thr Ser Asn Phe Arg Val Val Pro Ser Gly Asp
               300                 305                 310
   gtt gtg aga ttc cct aat att aca aac ttg tgt cct ttt gga gag gtt    1072
   Val Val Arg Phe Pro Asn Ile Thr Asn Leu Cys Pro Phe Gly Glu Val
           315                 320                 325
   ttt aat gct act aaa ttc cct tct gtc tat gca tgg gag aga aaa aaa    1120
   Phe Asn Ala Thr Lys Phe Pro Ser Val Tyr Ala Trp Glu Arg Lys Lys
       330                 335                 340
   att tct aat tgt gtt gct gat tac tct gtg ctc tac aac tca aca ttt    1168
   Ile Ser Asn Cys Val Ala Asp Tyr Ser Val Leu Tyr Asn Ser Thr Phe
   345                 350                 355                 360
   ttt tca acc ttt aag tgc tat ggc gtt tct gcc act aag ttg aat gat    1216
   Phe Ser Thr Phe Lys Cys Tyr Gly Val Ser Ala Thr Lys Leu Asn Asp
                   365                 370                 375
   ctt tgc ttc tcc aat gtc tat gca gat tct ttt gta gtc aag gga gat    1264
   Leu Cys Phe Ser Asn Val Tyr Ala Asp Ser Phe Val Val Lys Gly Asp
               380                 385                 390
   gat gta aga caa ata gcg cca gga caa act ggt gtt att gct gat tat    1312
   Asp Val Arg Gln Ile Ala Pro Gly Gln Thr Gly Val Ile Ala Asp Tyr
           395                 400                 405
   aat tat aaa ttg cca gat gat ttc atg ggt tgt gtc ctt gct tgg aat    1360
   Asn Tyr Lys Leu Pro Asp Asp Phe Met Gly Cys Val Leu Ala Trp Asn
       410                 415                 420
   act agg aac att gat gct act tca act ggt aat tat aat tat aaa tat    1408
   Thr Arg Asn Ile Asp Ala Thr Ser Thr Gly Asn Tyr Asn Tyr Lys Tyr
   425                 430                 435                 440
   agg tat ctt aga cat ggc aag ctt agg ccc ttt gag aga gac ata tct    1456
   Arg Tyr Leu Arg His Gly Lys Leu Arg Pro Phe Glu Arg Asp Ile Ser
                   445                 450                 455
   aat gtg cct ttc tcc cct gat ggc aaa cct tgc acc cca cct gct ctt    1504
   Asn Val Pro Phe Ser Pro Asp Gly Lys Pro Cys Thr Pro Pro Ala Leu
               460                 465                 470
   aat tgt tat tgg cca tta aat gat tat ggt ttt tac acc act act ggc    1552
   Asn Cys Tyr Trp Pro Leu Asn Asp Tyr Gly Phe Tyr Thr Thr Thr Gly
           475                 480                 485
   att ggc tac caa cct tac aga gtt gta gta ctt tct ttt gaa ctt tta      1600
   Ile Gly Tyr Gln Pro Tyr Arg Val Val Val Leu Ser Phe Glu Leu Leu
       490                 495                     500
   aat gca ccg gcc acg gtt tgt gga cca aaa tta tcc act gac ctt att      1648
   Asn Ala Pro Ala Thr Val Cys Gly Pro Lys Leu Ser Thr Asp Leu Ile
   505                 510                 515                 520
   aag aac cag tgt gtc aat ttt aat ttt aat gga ctc act ggt act ggt      1696
   Lys Asn Gln Cys Val Asn Phe Asn Phe Asn Gly Leu Thr Gly Thr Gly
                   525                 530                 535
   gtg tta act cct tct tca aag aga ttt caa cca ttt caa caa ttt ggc      1744
   Val Leu Thr Pro Ser Ser Lys Arg Phe Gln Pro Phe Gln Gln Phe Gly
               540                 545                 550
   cgt gat gtt tct gat ttc act gat tcc gtt cga gat cct aaa aca tct      1792
   Arg Asp Val Ser Asp Phe Thr Asp Ser Val Arg Asp Pro Lys Thr Ser
           555                 560                 565
   gaa ata tta gac att tca cct tgc tct ttt ggg ggt gta agt gta att      1840
   Glu Ile Leu Asp Ile Ser Pro Cys Ser Phe Gly Gly Val Ser Val Ile
       570                 575                 580
   aca cct gga aca aat gct tca tct gaa gtt gct gtt cta tat caa gat      1888
   Thr Pro Gly Thr Asn Ala Ser Ser Glu Val Ala Val Leu Tyr Gln Asp
   585                 590                 595                 600
   gtt aac tgc act gat gtt tct aca gca att cat gca gat caa ctc aca      1936
   Val Asn Cys Thr Asp Val Ser Thr Ala Ile His Ala Asp Gln Leu Thr
                   605                 610                 615
   cca gct tgg cgc ata tat tct act gga aac aat gta ttc cag act caa      1984
   Pro Ala Trp Arg Ile Tyr Ser Thr Gly Asn Asn Val Phe Gln Thr Gln
               620                 625                 630
   gca ggc tgt ctt ata gga gct gag cat gtc gac act tct tat gag tgc      2032
   Ala Gly Cys Leu Ile Gly Ala Glu His Val Asp Thr Ser Tyr Glu Cys
           635                 640                 645
   gac att cct att gga gct ggc att tgt gct agt tac cat aca gtt tct      2080
   Asp Ile Pro Ile Gly Ala Gly Ile Cys Ala Ser Tyr His Thr Val Ser
       650                 655                 660
   tta tta cgt agt act agc caa aaa tct att gtg gct tat act atg tct      2128
   Leu Leu Arg Ser Thr Ser Gln Lys Ser Ile Val Ala Tyr Thr Met Ser
   665                 670                 675                 680
   tta ggt gct gat agt tca att gct tac tct aat aac acc att gct ata      2176
   Leu Gly Ala Asp Ser Ser Ile Ala Tyr Ser Asn Asn Thr Ile Ala Ile
                   685                 690                 695
   cct act aac ttt tca att agc att act aca gaa gta atg cct gtt tct      2224
   Pro Thr Asn Phe Ser Ile Ser Ile Thr Thr Glu Val Met Pro Val Ser
               700                 705                 710
   atg gct aaa acc tcc gta gat tgt aat atg tac atc tgc gga gat tct      2272
   Met Ala Lys Thr Ser Val Asp Cys Asn Met Tyr Ile Cys Gly Asp Ser
           715                 720                 725
   act gaa tgt gct aat ttg ctt ctc caa tat ggt agc ttt tgc aca caa      2320
   Thr Glu Cys Ala Asn Leu Leu Leu Gln Tyr Gly Ser Phe Cys Thr Gln
       730                 735                 740
   cta aat cgt gca ctc tca ggt att gct gct gaa cag gat cgc aac aca      2368
   Leu Asn Arg Ala Leu Ser Gly Ile Ala Ala Glu Gln Asp Arg Asn Thr
   745                 750                 755                 760
   cgt gaa gtg ttc gct caa gtc aaa caa atg tac aaa acc cca act ttg     2416
   Arg Glu Val Phe Ala Gln Val Lys Gln Met Tyr Lys Thr Pro Thr Leu
                   765                 770                 775
   aaa tat ttt ggt ggt ttt aat ttt tca caa ata tta cct gac cct cta     2464
   Lys Tyr Phe Gly Gly Phe Asn Phe Ser Gln Ile Leu Pro Asp Pro Leu
               780                 785                 790
   aag cca act aag agg tct ttt att gag gac ttg ctc ttt aat aag gtg     2512
   Lys Pro Thr Lys Arg Ser Phe Ile Glu Asp Leu Leu Phe Asn Lys Val
           795                 800                 805
   aca ctc gct gat gct ggc ttc atg aag caa tat ggc gaa tgc cta ggt     2560
   Thr Leu Ala Asp Ala Gly Phe Met Lys Gln Tyr Gly Glu Cys Leu Gly
       810                 815                 820
   gat att aat gct aga gat ctc att tgt gcg cag aag ttc aat gga ctt     2608
   Asp Ile Asn Ala Arg Asp Leu Ile Cys Ala Gln Lys Phe Asn Gly Leu
   825                 830                 835                 840
   aca gtg ttg cca cct ctg ctc act gat gat atg att gct gcc tac act     2656
   Thr Val Leu Pro Pro Leu Leu Thr Asp Asp Met Ile Ala Ala Tyr Thr
                   845                 850                 855
   gct gct cta gtt agt ggt act gcc act gct gga tgg aca ttt ggt gct     2704
   Ala Ala Leu Val Ser Gly Thr Ala Thr Ala Gly Trp Thr Phe Gly Ala
               860                 865                 870
   ggc gct gct ctt caa ata cct ttt gct atg caa atg gca tat agg ttc     2752
   Gly Ala Ala Leu Gln Ile Pro Phe Ala Met Gln Met Ala Tyr Arg Phe
           875                 880                 885
   aat ggc att gga gtt acc caa aat gtt ctc tat gag aac caa aaa caa     2800
   Asn Gly Ile Gly Val Thr Gln Asn Val Leu Tyr Glu Asn Gln Lys Gln
       890                 895                 900
   atc gcc aac caa ttt aac aag gcg att agt caa att caa gaa tca ctt     2848
   Ile Ala Asn Gln Phe Asn Lys Ala Ile Ser Gln Ile Gln Glu Ser Leu
   905                 910                 915                 920
   aca aca aca tca act gca ttg ggc aag ctg caa gac gtt gtt aac cag     2896
   Thr Thr Thr Ser Thr Ala Leu Gly Lys Leu Gln Asp Val Val Asn Gln
                   925                 930                 935
   aat gct caa gca tta aac aca ctt gtt aaa caa ctt agc tct aat ttt     2944
   Asn Ala Gln Ala Leu Asn Thr Leu Val Lys Gln Leu Ser Ser Asn Phe
               940                 945                 950
   ggt gca att tca agt gtg cta aat gat atc ctt tcg cga ctt gat aaa     2992
   Gly Ala Ile Ser Ser Val Leu Asn Asp Ile Leu Ser Arg Leu Asp Lys
           955                 960                 965
   gtc gag gcg gag gta caa att gac agg tta att aca ggc aga ctt caa     3040
   Val Glu Ala Glu Val Gln Ile Asp Arg Leu Ile Thr Gly Arg Leu Gln
       970                 975                 980
   agc ctt caa acc tat gta aca caa caa cta atc agg gct gct gaa atc     3088
   Ser Leu Gln Thr Tyr Val Thr Gln Gln Leu Ile Arg Ala Ala Glu Ile
   985                 990                 995                 1000
   agg gct tct gct aat  ctt gct gct act aaa  atg tct gag tgt gtt       3133
   Arg Ala Ser Ala Asn  Leu Ala Ala Thr Lys  Met Ser Glu Cys Val
                   1005                 1010                 1015
   ctt gga caa tca aaa  aga gtt gac ttt tgt  gga aag ggc tac cac       3178
   Leu Gly Gln Ser Lys  Arg Val Asp Phe Cys  Gly Lys Gly Tyr His
                   1020                 1025                 1030
   ctt atg tcc ttc cca  caa gca gcc ccg cat  ggt gtt gtc ttc cta     3223
   Leu Met Ser Phe Pro  Gln Ala Ala Pro His  Gly Val Val Phe Leu
                   1035                 1040                 1045
   cat gtc acg tat gtg  cca tcc cag gag agg  aac ttc acc aca gcg     3268
   His Val Thr Tyr Val  Pro Ser Gln Glu Arg  Asn Phe Thr Thr Ala
                   1050                 1055                 1060
   cca gca att tgt cat  gaa ggc aaa gca tac  ttc cct cgt gaa ggt     3313
   Pro Ala Ile Cys His  Glu Gly Lys Ala Tyr  Phe Pro Arg Glu Gly
                   1065                 1070                 1075
   gtt ttt gtg ttt aat  ggc act tct tgg ttt  att aca cag agg aac     3358
   Val Phe Val Phe Asn  Gly Thr Ser Trp Phe  Ile Thr Gln Arg Asn
                   1080                 1085                 1090
   ttc ttt tct cca caa  ata att act aca gac  aat aca ttt gtc tca     3403
   Phe Phe Ser Pro Gln  Ile Ile Thr Thr Asp  Asn Thr Phe Val Ser
                   1095                 1100                 1105
   gga aat tgt gat gtc  gtt att ggc atc att  aac aac aca gtt tat     3448
   Gly Asn Cys Asp Val  Val Ile Gly Ile Ile  Asn Asn Thr Val Tyr
                   1110                 1115                 1120
   gat cct ctg caa cct  gag ctt gac tca ttc  aaa gaa gag ctg gac     3493
   Asp Pro Leu Gln Pro  Glu Leu Asp Ser Phe  Lys Glu Glu Leu Asp
                   1125                 1130                 1135
   aag tac ttc aaa aat  cat aca tca cca gat  gtt gat ctt ggc gac     3538
   Lys Tyr Phe Lys Asn  His Thr Ser Pro Asp  Val Asp Leu Gly Asp
                   1140                 1145                 1150
   att tca ggc att aac  gct tct gtc gtc aac  att caa aaa gaa att     3583
   Ile Ser Gly Ile Asn  Ala Ser Val Val Asn  Ile Gln Lys Glu Ile
                   1155                 1160                 1165
   gac cgc ctc aat gag  gtc gct aaa aat tta  aat gaa tca ctc att     3628
   Asp Arg Leu Asn Glu  Val Ala Lys Asn Leu  Asn Glu Ser Leu Ile
                   1170                 1175                 1180
   gac ctt caa gaa ttg  gga aaa tat gag caa  tat att aaa tgg cct     3673
   Asp Leu Gln Glu Leu  Gly Lys Tyr Glu Gln  Tyr Ile Lys Trp Pro
                   1185                 1190                 1195
   tgg tat gtt tgg ctc  ggc ttc att gct gga  cta att gcc atc gtc     3718
   Trp Tyr Val Trp Leu  Gly Phe Ile Ala Gly  Leu Ile Ala Ile Val
                   1200                 1205                 1210
   atg gtt aca atc ttg  ctt tgt tgc atg act  agt tgt tgc agt tgc     3763
   Met Val Thr Ile Leu  Leu Cys Cys Met Thr  Ser Cys Cys Ser Cys
                   1215                 1220                 1225
   ctc aag ggt gca tgc  tct tgt ggt tct tgc  tgc aag ttt gat gag     3808
   Leu Lys Gly Ala Cys  Ser Cys Gly Ser Cys  Cys Lys Phe Asp Glu
                   1230                 1235                 1240
   gat gac tct gag cca  gtt ctc aag ggt gtc  aaa tta cat tac aca     3853
   Asp Asp Ser Glu Pro  Val Leu Lys Gly Val  Lys Leu His Tyr Thr
                   1245                 1250                 1255
   taaacgaact tatggatttg tttatgagat tttttactct tggatcaatt actgcacagc  3913
   cagtaaaaat tgacaatgct tctcctgcaa gt                                3945
<210> 3
   <211> 1255
   <212> PRT
   <213> CORONAVIRUS
<400> 3
   Met Phe Ile Phe Leu Leu Phe Leu Thr Leu Thr Ser Gly Ser Asp Leu
   1               5                   10                  15
   Asp Arg Cys Thr Thr Phe Asp Asp Val Gln Ala Pro Asn Tyr Thr Gln
               20                  25                  30
   His Thr Ser Ser Met Arg Gly Val Tyr Tyr Pro Asp Glu Ile Phe Arg
           35                  40                  45
   Ser Asp Thr Leu Tyr Leu Thr Gln Asp Leu Phe Leu Pro Phe Tyr Ser
       50                  55                  60
   Asn Val Thr Gly Phe His Thr Ile Asn His Thr Phe Gly Asn Pro Val
   65                  70                  75                  80
   Ile Pro Phe Lys Asp Gly Ile Tyr Phe Ala Ala Thr Glu Lys Ser Asn
                   85                  90                  95
   Val Val Arg Gly Trp Val Phe Gly Ser Thr Met Asn Asn Lys Ser Gln
               100                 105                 110
   Ser Val Ile Ile Ile Asn Asn Ser Thr Asn Val Val Ile Arg Ala Cys
           115                 120                 125
   Asn Phe Glu Leu Cys Asp Asn Pro Phe Phe Ala Val Ser Lys Pro Met
       130                 135                 140
   Gly Thr Gln Thr His Thr Met Ile Phe Asp Asn Ala Phe Asn Cys Thr
   145                 150                 155                 160
   Phe Glu Tyr Ile Ser Asp Ala Phe Ser Leu Asp Val Ser Glu Lys Ser
                   165                 170                 175
   Gly Asn Phe Lys His Leu Arg Glu Phe Val Phe Lys Asn Lys Asp Gly
               180                 185                 190
   Phe Leu Tyr Val Tyr Lys Gly Tyr Gln Pro Ile Asp Val Val Arg Asp
           195                 200                 205
   Leu Pro Ser Gly Phe Asn Thr Leu Lys Pro Ile Phe Lys Leu Pro Leu
       210                 215                 220
   Gly Ile Asn Ile Thr Asn Phe Arg Ala Ile Leu Thr Ala Phe Ser Pro
   225                 230                 235                 240
   Ala Gln Asp Ile Trp Gly Thr Ser Ala Ala Ala Tyr Phe Val Gly Tyr
                   245                 250                 255
   Leu Lys Pro Thr Thr Phe Met Leu Lys Tyr Asp Glu Asn Gly Thr Ile
               260                 265                 270
   Thr Asp Ala Val Asp Cys Ser Gln Asn Pro Leu Ala Glu Leu Lys Cys
           275                 280                 285
   Ser Val Lys Ser Phe Glu Ile Asp Lys Gly Ile Tyr Gln Thr Ser Asn
       290                 295                 300
   Phe Arg Val Val Pro Ser Gly Asp Val Val Arg Phe Pro Asn Ile Thr
   305                 310                 315                 320
   Asn Leu Cys Pro Phe Gly Glu Val Phe Asn Ala Thr Lys Phe Pro Ser
                   325                 330                 335
   Val Tyr Ala Trp Glu Arg Lys Lys Ile Ser Asn Cys Val Ala Asp Tyr
               340                 345                 350
   Ser Val Leu Tyr Asn Ser Thr Phe Phe Ser Thr Phe Lys Cys Tyr Gly
           355                 360                 365
   Val Ser Ala Thr Lys Leu Asn Asp Leu Cys Phe Ser Asn Val Tyr Ala
       370                 375                 380
   Asp Ser Phe Val Val Lys Gly Asp Asp Val Arg Gln Ile Ala Pro Gly
   385                 390                 395                 400
   Gln Thr Gly Val Ile Ala Asp Tyr Asn Tyr Lys Leu Pro Asp Asp Phe
                   405                 410                 415
   Met Gly Cys Val Leu Ala Trp Asn Thr Arg Asn Ile Asp Ala Thr Ser
               420                 425                 430
   Thr Gly Asn Tyr Asn Tyr Lys Tyr Arg Tyr Leu Arg His Gly Lys Leu
           435                 440                 445
   Arg Pro Phe Glu Arg Asp Ile Ser Asn Val Pro Phe Ser Pro Asp Gly
       450                 455                 460
   Lys Pro Cys Thr Pro Pro Ala Leu Asn Cys Tyr Trp Pro Leu Asn Asp
   465                 470                 475                 480
   Tyr Gly Phe Tyr Thr Thr Thr Gly Ile Gly Tyr Gln Pro Tyr Arg Val
                   485                 490                 495
   Val Val Leu Ser Phe Glu Leu Leu Asn Ala Pro Ala Thr Val Cys Gly
               500                 505                 510
   Pro Lys Leu Ser Thr Asp Leu Ile Lys Asn Gln Cys Val Asn Phe Asn
   515 520 525
   Phe Asn Gly Leu Thr Gly Thr Gly Val Leu Thr Pro Ser Ser Lys Arg
   530 535 540
   Phe Gln Pro Phe Gln Gln Phe Gly Arg Asp Val Ser Asp Phe Thr Asp
   545 550 555 560
   Ser Val Arg Asp Pro Lys Thr Ser Glu Ile Leu Asp Ile Ser Pro Cys
   565 570 575
   Ser Phe Gly Gly Val Ser Val Ile Thr Pro Gly Thr Asn Ala Ser Ser
   580 585 590
   Glu Val Ala Val Leu Tyr Gln Asp Val Asn Cys Thr Asp Val Ser Thr
   595 600 605
   Ala Ile His Ala Asp Gln Leu Thr Pro Ala Trp Arg Ile Tyr Ser Thr
   610 615 620
   Gly Asn Asn Val Phe Gln Thr Gln Ala Gly Cys Leu Ile Gly Ala Glu
   625 630 635 640
   His Val Asp Thr Ser Tyr Glu Cys Asp Ile Pro Ile Gly Ala Gly Ile
   645 650 655
   Cys Ala Ser Tyr His Thr Val Ser Leu Leu Arg Ser Thr Ser Gln Lys
   660 665 670
   Ser Ile Val Ala Tyr Thr Met Ser Leu Gly Ala Asp Ser Ser Ile Ala
   675 680 685
   Tyr Ser Asn Asn Thr Ile Ala Ile Pro Thr Asn Phe Ser Ile Ser Ile
   690 695 700
   Thr Thr Glu Val Met Pro Val Ser Met Ala Lys Thr Ser Val Asp Cys
   705 710 715 720
   Asn Met Tyr Ile Cys Gly Asp Ser Thr Glu Cys Ala Asn Leu Leu Leu
   725 730 735
   Gln Tyr Gly Ser Phe Cys Thr Gln Leu Asn Arg Ala Leu Ser Gly Ile
   740 745 750
   Ala Ala Glu Gln Asp Arg Asn Thr Arg Glu Val Phe Ala Gln Val Lys
   755 760 765
   Gln Met Tyr Lys Thr Pro Thr Leu Lys Tyr Phe Gly Gly Phe Asn Phe
   770 775 780
   Ser Gln Ile Leu Pro Asp Pro Leu Lys Pro Thr Lys Arg Ser Phe Ile
   785                 790                 795                 800
   Glu Asp Leu Leu Phe Asn Lys Val Thr Leu Ala Asp Ala Gly Phe Met
                   805                 810                 815
   Lys Gln Tyr Gly Glu Cys Leu Gly Asp Ile Asn Ala Arg Asp Leu Ile
               820                 825                 830
   Cys Ala Gln Lys Phe Asn Gly Leu Thr Val Leu Pro Pro Leu Leu Thr
           835                 840                 845
   Asp Asp Met Ile Ala Ala Tyr Thr Ala Ala Leu Val Ser Gly Thr Ala
       850                 855                 860
   Thr Ala Gly Trp Thr Phe Gly Ala Gly Ala Ala Leu Gln Ile Pro Phe
   865                 870                 875                 880
   Ala Met Gln Met Ala Tyr Arg Phe Asn Gly Ile Gly Val Thr Gln Asn
                   885                 890                 895
   Val Leu Tyr Glu Asn Gln Lys Gln Ile Ala Asn Gln Phe Asn Lys Ala
               900                 905                 910
   Ile Ser Gln Ile Gln Glu Ser Leu Thr Thr Thr Ser Thr Ala Leu Gly
           915                 920                 925
   Lys Leu Gln Asp Val Val Asn Gln Asn Ala Gln Ala Leu Asn Thr Leu
       930                 935                 940
   Val Lys Gln Leu Ser Ser Asn Phe Gly Ala Ile Ser Ser Val Leu Asn
   945                 950                 955                 960
   Asp Ile Leu Ser Arg Leu Asp Lys Val Glu Ala Glu Val Gln Ile Asp
                   965                 970                 975
   Arg Leu Ile Thr Gly Arg Leu Gln Ser Leu Gln Thr Tyr Val Thr Gln
               980                 985                 990
   Gln Leu Ile Arg Ala Ala Glu Ile  Arg Ala Ser Ala Asn  Leu Ala Ala
           995                 1000                1005
   Thr Lys  Met Ser Glu Cys Val  Leu Gly Gln Ser Lys  Arg Val Asp
       1010                1015                1020
   Phe Cys  Gly Lys Gly Tyr His  Leu Met Ser Phe Pro  Gln Ala Ala
       1025                1030                1035
   Pro His  Gly Val Val Phe Leu  His Val Thr Tyr Val  Pro Ser Gln
       1040                1045                1050
   Glu Arg Asn Phe Thr Thr Ala Pro Ala Ile Cys His Glu Gly Lys
       1055                1060                1065
   Ala Tyr Phe Pro Arg Glu Gly Val Phe Val Phe Asn Gly Thr Ser
       1070                1075                1080
   Trp Phe Ile Thr Gln Arg Asn Phe Phe Ser Pro Gln Ile Ile Thr
       1085                1090                1095
   Thr Asp Asn Thr Phe Val Ser Gly Asn Cys Asp Val Val Ile Gly
       1100                1105                1110
   Ile Ile Asn Asn Thr Val Tyr Asp Pro Leu Gln Pro Glu Leu Asp
       1115                1120                1125
   Ser Phe Lys Glu Glu Leu Asp Lys Tyr Phe Lys Asn His Thr Ser
       1130                1135                1140
   Pro Asp Val Asp Leu Gly Asp Ile Ser Gly Ile Asn Ala Ser Val
       1145                1150                1155
   Val Asn Ile Gln Lys Glu Ile Asp Arg Leu Asn Glu Val Ala Lys
       1160                1165                1170
   Asn Leu Asn Glu Ser Leu Ile Asp Leu Gln Glu Leu Gly Lys Tyr
       1175                1180                1185
   Glu Gln Tyr Ile Lys Trp Pro Trp Tyr Val Trp Leu Gly Phe Ile
       1190                1195                1200
   Ala Gly Leu Ile Ala Ile Val Met Val Thr Ile Leu Leu Cys Cys
       1205                1210                1215
   Met Thr Ser Cys Cys Ser Cys Leu Lys Gly Ala Cys Ser Cys Gly
       1220                1225                1230
   Ser Cys Cys Lys Phe Asp Glu Asp Asp Ser Glu Pro Val Leu Lys
       1235                1240                1245
   Gly Val Lys Leu His Tyr Thr
       1250                1255
<210> 4
   <211> 3943
   <212> DNA
   <213> CORONAVIRUS
<400> 4
   ctcttctgga aaaaggtagg cttatcatta gagaaaacaa cagagttgtg gtttcaagtg     60
   atattcttgt taacaactaa acgaacatgt ttattttctt attatttctt actctcacta    120
   gtggtagtga ccttgaccgg tgcaccactt ttgatgatgt tcaagctcct aattacactc    180
   aacatacttc atctatgagg ggggtttact atcctgatga aatttttaga tcagacactc    240
   tttatttaac tcaggattta tttcttccat tttattctaa tgttacaggg tttcatacta    300
   ttaatcatac gtttggcaac cctgtcatac cttttaagga tggtatttat tttgctgcca    360
   cagagaaatc aaatgttgtc cgtggttggg tttttggttc taccatgaac aacaagtcac    420
   agtcggtgat tattattaac aattctacta atgttgttat acgagcatgt aactttgaat    480
   tgtgtgacaa ccctttcttt gctgtttcta aacccatggg tacacagaca catactatga    540
   tattcgataa tgcatttaat tgcactttcg agtacatatc tgatgccttt tcgcttgatg    600
   tttcagaaaa gtcaggtaat tttaaacact tacgagagtt tgtgtttaaa aataaagatg    660
   ggtttctcta tgtttataag ggctatcaac ctatagatgt agttcgtgat ctaccttctg    720
   gttttaacac tttgaaacct atttttaagt tgcctcttgg tattaacatt acaaatttta    780
   gagccattct tacagccttt tcacctgctc aagacatttg gggcacgtca gctgcagcct     840
   attttgttgg ctatttaaag ccaactacat ttatgctcaa gtatgatgaa aatggtacaa    900
   tcacagatgc tgttgattgt tctcaaaatc cacttgctga actcaaatgc tctgttaaga    960
   gctttgagat tgacaaagga atttaccaga cctctaattt cagggttgtt ccctcaggag   1020
   atgttgtgag attccctaat attacaaact tgtgtccttt tggagaggtt tttaatgcta   1080
   ctaaattccc ttctgtctat gcatgggaga gaaaaaaaat ttctaattgt gttgctgatt   1140
   actctgtgct ctacaactca acattttttt caacctttaa gtgctatggc gtttctgcca   1200
   ctaagttgaa tgatctttgc ttctccaatg tctatgcaga ttcttttgta gtcaagggag   1260
   atgatgtaag acaaatagcg ccaggacaaa ctggtgttat tgctgattat aattataaat   1320
   tgccagatga tttcatgggt tgtgtccttg cttggaatac taggaacatt gatgctactt   1380
   caactggtaa ttataattat aaatataggt atcttagaca tggcaagctt aggccctttg   1440
   agagagacat atctaatgtg cctttctccc ctgatggcaa accttgcacc ccacctgctc   1500
   ttaattgtta ttggccatta aatgattatg gtttttacac cactactggc attggctacc   1560
   aaccttacag agttgtagta ctttcttttg aacttttaaa tgcaccggcc acggtttgtg   1620
   gaccaaaatt atccactgac cttattaaga accagtgtgt caattttaat tttaatggac   1680
   tcactggtac tggtgtgtta actccttctt caaagagatt tcaaccattt caacaatttg   1740
   gccgtgatgt ctctgatttc actgattccg ttcgagatcc taaaacatct gaaatattag   1800
   acatttcacc ttgctctttt gggggtgtaa gtgtaattac acctggaaca aatgcttcat   1860
   ctgaagttgc tgttctatat caagatgtta actgcactga tgtttctaca gcaatccatg   1920
   cagatcaact cacaccagct tggcgcatat attctactgg aaacaatgta ttccagactc   1980
   aagcaggctg tcttatagga gctgagcatg tcgacacttc ttatgagtgc gacattccta   2040
   ttggagctgg catttgtgct agttaccata cagtttcttt attacgtagt actagccaaa 2100
   aatctattgt ggcttatact atgtctttag gtgctgatag ttcaattgct tactctaata 2160
   acaccattgc tatacctact aacttttcaa ttagcattac tacagaagta atgcctgttt 2220
   ctatggctaa aacctccgta gattgtaata tgtacatctg cggagattct actgaatgtg 2280
   ctaatttgct tctccaatat ggtagctttt gcacacaact aaatcgtgca ctctcaggta 2340
   ttgctgctga acaggatcgc aacacacgtg aagtgttcgc tcaagtcaaa caaatgtaca 2400
   aaaccccaac tttgaaatat tttggtggtt ttaatttttc acaaatatta cctgaccctc 2460
   taaagccaac taagaggtct tttattgagg acttgctctt taataaggtg acactcgctg 2520
   atgctggctt catgaagcaa tatggcgaat gcctaggtga tattaatgct agagatctca 2580
   tttgtgcgca gaagttcaat gggcttacag tgttgccacc tctgctcact gatgatatga 2640
   ttgctgccta cactgctgct ctagttagtg gtactgccac tgctggatgg acatttggtg 2700
   ctggcgctgc tcttcaaata ccttttgcta tgcaaatggc atataggttc aatggcattg 2760
   gagttaccca aaatgttctc tatgagaacc aaaaacaaat cgccaaccaa tttaacaagg 2820
   cgattagtca aattcaagaa tcacttacaa caacatcaac tgcattgggc aagctgcaag 2880
   acgttgttaa ccagaatgct caagcattaa acacacttgt taaacaactt agctctaatt 2940
   ttggtgcaat ttcaagtgtg ctaaatgata tcctttcgcg acttgataaa gtcgaggcgg 3000
   aggtacaaat tgacaggcta attacaggca gacttcaaag ccttcaaacc tatgtaacac 3060
   aacaactaat cagggctgct gaaatcaggg cttctgctaa tcttgctgct actaaaatgt 3120
   ctgagtgtgt tcttggacaa tcaaaaagag ttgacttttg tggaaagggc taccacctta 3180
   tgtccttccc acaagcagcc ccgcatggtg ttgtcttcct acatgtcacg tatgtgccat 3240
   cccaggagag gaacttcacc acagcgccag caatttgtca tgaaggcaaa gcatacttcc 3300
   ctcgtgaagg tgtttttgtg tttaatggca cttcttggtt tattacacag aggaacttct 3360
   tttctccaca aataattact acagacaata catttgtctc aggaaattgt gatgtcgtta 3420
   ttggcatcat taacaacaca gtttatgatc ctctgcaacc tgagcttgac tcattcaaag 3480
   aagagctgga caagtacttc aaaaatcata catcaccaga tgttgatctt ggcgacattt 3540
   caggcattaa cgcttctgtc gtcaacattc aaaaagaaat tgaccgcctc aatgaggtcg 3600
   ctaaaaattt aaatgaatca ctcattgacc ttcaagaatt gggaaaatat gagcaatata 3660
   ttaaatggcc ttggtatgtt tggctcggct tcattgctgg actaattgcc atcgtcatgg 3720
   ttacaatctt gctttgttgc atgactagtt gttgcagttg cctcaagggt gcatgctctt 3780
   gtggttcttg ctgcaagttt gatgaggatg actctgagcc agttctcaag ggtgtcaaat 3840
   tacattacac ataaacgaac ttatggattt gtttatgaga ttttttactc ttggatcaat 3900
   tactgcacag ccagtaaaaa ttgacaatgc ttctcctgca agt 3943
<210> 5
   <211> 2049
   <212> DNA
   <213> CORONAVIRUS
<400> 5
   ctcttctgga aaaaggtagg cttatcatta gagaaaacaa cagagttgtg gtttcaagtg      60
   atattcttgt taacaactaa acgaacatgt ttattttctt attatttctt actctcacta     120
   gtggtagtga ccttgaccgg tgcaccactt ttgatgatgt tcaagctcct aattacactc     180
   aacatacttc atctatgagg ggggtttact atcctgatga aatttttaga tcagacactc     240
   tttatttaac tcaggattta tttcttccat tttattctaa tgttacaggg tttcatacta     300
   ttaatcatac gtttggcaac cctgtcatac cttttaagga tggtatttat tttgctgcca     360
   cagagaaatc aaatgttgtc cgtggttggg tttttggttc taccatgaac aacaagtcac     420
   agtcggtgat tattattaac aattctacta atgttgttat acgagcatgt aactttgaat     480
   tgtgtgacaa ccctttcttt gctgtttcta aacccatggg tacacagaca catactatga     540
   tattcgataa tgcatttaat tgcactttcg agtacatatc tgatgccttt tcgcttgatg     600
   tttcagaaaa gtcaggtaat tttaaacact tacgagagtt tgtgtttaaa aataaagatg     660
   ggtttctcta tgtttataag ggctatcaac ctatagatgt agttcgtgat ctaccttctg     720
   gttttaacac tttgaaacct atttttaagt tgcctcttgg tattaacatt acaaatttta     780
   gagccattct tacagccttt tcacctgctc aagacatttg gggcacgtca gctgcagcct      840
   attttgttgg ctatttaaag ccaactacat ttatgctcaa gtatgatgaa aatggtacaa     900
   tcacagatgc tgttgattgt tctcaaaatc cacttgctga actcaaatgc tctgttaaga     960
   gctttgagat tgacaaagga atttaccaga cctctaattt cagggttgtt ccctcaggag    1020
   atgttgtgag attccctaat attacaaact tgtgtccttt tggagaggtt tttaatgcta    1080
   ctaaattccc ttctgtctat gcatgggaga gaaaaaaaat ttctaattgt gttgctgatt    1140
   actctgtgct ctacaactca acattttttt caacctttaa gtgctatggc gtttctgcca    1200
   ctaagttgaa tgatctttgc ttctccaatg tctatgcaga ttcttttgta gtcaagggag    1260
   atgatgtaag acaaatagcg ccaggacaaa ctggtgttat tgctgattat aattataaat    1320
   tgccagatga tttcatgggt tgtgtccttg cttggaatac taggaacatt gatgctactt    1380
   caactggtaa ttataattat aaatataggt atcttagaca tggcaagctt aggccctttg    1440
   agagagacat atctaatgtg cctttctccc ctgatggcaa accttgcacc ccacctgctc    1500
   ttaattgtta ttggccatta aatgattatg gtttttacac cactactggc attggctacc    1560
   aaccttacag agttgtagta ctttcttttg aacttttaaa tgcaccggcc acggtttgtg    1620
   gaccaaaatt atccactgac cttattaaga accagtgtgt caattttaat tttaatggac    1680
   tcactggtac tggtgtgtta actccttctt caaagagatt tcaaccattt caacaatttg    1740
   gccgtgatgt ctctgatttc actgattccg ttcgagatcc taaaacatct gaaatattag    1800
   acatttcacc ttgctctttt gggggtgtaa gtgtaattac acctggaaca aatgcttcat 1860
   ctgaagttgc tgttctatat caagatgtta actgcactga tgtttctaca gcaatccatg 1920
   cagatcaact cacaccagct tggcgcatat attctactgg aaacaatgta ttccagactc 1980
   aagcaggctg tcttatagga gctgagcatg tcgacacttc ttatgagtgc gacattccta 2040
   ttggagctg 2049
<210> 6
   <211> 2027
   <212> DNA
   <213> CORONAVIRUS
<400> 6
   catgcagatc aactcacacc agcttggcgc atatattcta ctggaaacaa tgtattccag 60
   actcaagcag gctgtcttat aggagctgag catgtcgaca cttcttatga gtgcgacatt 120
   cctattggag ctggcatttg tgctagttac catacagttt ctttattacg tagtactagc 180
   caaaaatcta ttgtggctta tactatgtct ttaggtgctg atagttcaat tgcttactct 240
   aataacacca ttgctatacc tactaacttt tcaattagca ttactacaga agtaatgcct 300
   gtttctatgg ctaaaacctc cgtagattgt aatatgtaca tctgcggaga ttctactgaa 360
   tgtgctaatt tgcttctcca atatggtagc ttttgcacac aactaaatcg tgcactctca 420
   ggtattgctg ctgaacagga tcgcaacaca cgtgaagtgt tcgctcaagt caaacaaatg 480
   tacaaaaccc caactttgaa atattttggt ggttttaatt tttcacaaat attacctgac 540
   cctctaaagc caactaagag gtcttttatt gaggacttgc tctttaataa ggtgacactc 600
   gctgatgctg gcttcatgaa gcaatatggc gaatgcctag gtgatattaa tgctagagat 660
   ctcatttgtg cgcagaagtt caatgggctt acagtgttgc cacctctgct cactgatgat 720
   atgattgctg cctacactgc tgctctagtt agtggtactg ccactgctgg atggacattt 780
   ggtgctggcg ctgctcttca aatacctttt gctatgcaaa tggcatatag gttcaatggc 840
   attggagtta cccaaaatgt tctctatgag aaccaaaaac aaatcgccaa ccaatttaac 900
   aaggcgatta gtcaaattca agaatcactt acaacaacat caactgcatt gggcaagctg 960
   caagacgttg ttaaccagaa tgctcaagca ttaaacacac ttgttaaaca acttagctct 1020
   aattttggtg caatttcaag tgtgctaaat gatatccttt cgcgacttga taaagtcgag 1080
   gcggaggtac aaattgacag gttaattaca ggcagacttc aaagccttca aacctatgta 1140
   acacaacaac taatcagggc tgctgaaatc agggcttctg ctaatcttgc tgctactaaa 1200
   atgtctgagt gtgttcttgg acaatcaaaa agagttgact tttgtggaaa gggctaccac 1260
   cttatgtcct tcccacaagc agccccgcat ggtgttgtct tcctacatgt cacgtatgtg 1320
   ccatcccagg agaggaactt caccacagcg ccagcaattt gtcatgaagg caaagcatac 1380
   ttccctcgtg aaggtgtttt tgtgtttaat ggcacttctt ggtttattac acagaggaac   1440
   ttcttttctc cacaaataat tactacagac aatacatttg tctcaggaaa ttgtgatgtc   1500
   gttattggcg tcattaacaa cacagtttat gatcctctgc aacctgagct tgactcattc   1560
   aaagaagagc tggacaagta cttcaaaaat catacatcac cagatgttga tcttggcgac   1620
   atttcaggca ttaacgcttc tgtcgtcaac attcaaaaag aaattgaccg cctcaatgag   1680
   gtcgctaaaa atttaaatga atcactcatt gaccttcaag aattgggaaa atatgagcaa   1740
   tatattaaat ggccttggta tgtttggctc ggcttcattg ctggactaat tgccatcgtc   1800
   atggttacaa tcttgctttg ttgcatgact agttgttgca gttgcctcaa gggtgcatgc   1860
   tcttgtggtt cttgctgcaa gtttgatgag gatgactctg agccagttct caagggtgtc   1920
   aaattacatt acacataaac gaacttatgg atttgtttat gagatttttt actcttggat   1980
   caattactgc acagccagta aaaattgaca atgcttctcc tgcaagt                 2027
<210> 7
   <211> 1096
   <212> DNA
   <213> CORONAVIRUS
<400> 7
   tcttgctttg ttgcatgact agttgttgca gttgcctcaa gggtgcatgc tcttgtggtt     60
   cttgctgcaa gtttgatgag gatgactctg agccagttct caagggtgtc aaattacatt    120
   acacataaac gaacttatgg atttgtttat gagatttttt actcttggat caattactgc    180
   acagccagta aaaattgaca atgcttctcc tgcaagtact gttcatgcta cagcaacgat    240
   accgctacaa gcctcactcc ctttcggatg gcttgttatt ggcgttgcat ttcttgctgt    300
   ttttcagagc gctaccaaaa taattgcgct caataaaaga tggcagctag ccctttataa     360
   gggcttccag ttcatttgca atttactgct gctatttgtt accatctatt cacatctttt    420
   gcttgtcgct gcaggtatgg aggcgcaatt tttgtacctc tatgccttga tatattttct    480
   acaatgcatc aacgcatgta gaattattat gagatgttgg ctttgttgga agtgcaaatc    540
   caagaaccca ttactttatg atgccaacta ctttgtttgc tggcacacac ataactatga    600
   ctactgtata ccatataaca gtgtcacaga tacaattgtc gttactgaag gtgacggcat    660
   ttcaacacca aaactcaaag aagactacca aattggtggt tattctgagg ataggcactc    720
   aggtgttaaa gactatgtcg ttgtacatgg ctatttcacc gaagtttact accagcttga    780
   gtctacacaa attactacag acactggtat tgaaaatgct acattcttca tctttaacaa    840
   gcttgttaaa gacccaccga atgtgcaaat acacacaatc gacggctctt caggagttgc    900
   taatccagca atggatccaa tttatgatga gccgacgacg actactagcg tgcctttgta    960
   agcacaagaa agtgagtacg aacttatgta ctcattcgtt tcggaagaaa caggtacgtt   1020
   aatagttaat agcgtacttc tttttcttgc tttcgtggta ttcttgctag tcacactagc 1080
   catccttact gcgctt 1096
<210> 8
   <211> 1135
   <212> DNA
   <213> CORONAVIRUS
<400> 8
   attgccatcg tcatggttac aatcttgctt tgttgcatga ctagttgttg cagttgcctc 60
   aagggtgcat gctcttgtgg ttcttgctgc aagtttgatg aggatgactc tgagccagtt 120
   ctcaagggtg tcaaattaca ttacacataa acgaacttat ggatttgttt atgagatttt 180
   ttactcttgg atcaattact gcacagccag taaaaattga caatgcttct cctgcaagta 240
   ctgttcatgc tacagcaacg ataccgctac aagcctcact ccctttcgga tggcttgtta 300
   ttggcgttgc atttcttgct gtttttcaga gcgctaccaa aataattgcg ctcaataaaa 360
   gatggcagct agccctttat aagggcttcc agttcatttg caatttactg ctgctatttg 420
   ttaccatcta ttcacatctt ttgcttgtcg ctgcaggtat ggaggcgcaa tttttgtacc 480
   tctatgcctt gatatatttt ctacaatgca tcaacgcatg tagaattatt atgagatgtt 540
   ggctttgttg gaagtgcaaa tccaagaacc cattacttta tgatgccaac tactttgttt 600
   gctggcacac acataactat gactactgta taccatataa cagtgtcaca gatacaattg 660
   tcgttactga aggtgacggc atttcaacac caaaactcaa agaagactac caaattggtg 720
   gttattctga ggataggcac tcaggtgtta aagactatgt cgttgtacat ggctatttca 780
   ccgaagttta ctaccagctt gagtctacac aaattactac agacactggt attgaaaatg 840
   ctacattctt catctttaac aagcttgtta aagacccacc gaatgtgcaa atacacacaa 900
   tcgacggctc ttcaggagtt gctaatccag caatggatcc aatttatgat gagccgacga 960
   cgactactag cgtgcctttg taagcacaag aaagtgagta cgaacttatg tactcattcg 1020
   tttcggaaga aacaggtacg ttaatagtta atagcgtact tctttttctt gctttcgtgg 1080
   tattcttgct agtcacacta gccatcctta ctgcgcttcg attgtgtgcg tactg 1135
<210> 9
   <211> 1096
   <212> DNA
   <213> CORONAVIRUS
<220>
   <221> CDS
   <222> (137)..(958)
   <223>
<400> 9
   tcttgctttg ttgcatgact agttgttgca gttgcctcaa gggtgcatgc tcttgtggtt     60
   cttgctgcaa gtttgatgag gatgactctg agccagttct caagggtgtc aaattacatt    120
   acacataaac gaactt atg gat ttg ttt atg aga ttt ttt act ctt gga tca    172
                     Met Asp Leu Phe Met Arg Phe Phe Thr Leu Gly Ser
                     1               5                   10
   att act gca cag cca gta aaa att gac aat gct tct cct gca agt act      220
   Ile Thr Ala Gln Pro Val Lys Ile Asp Asn Ala Ser Pro Ala Ser Thr
           15                  20                  25
   gtt cat gct aca gca acg ata ccg cta caa gcc tca ctc cct ttc gga      268
   Val His Ala Thr Ala Thr Ile Pro Leu Gln Ala Ser Leu Pro Phe Gly
       30                  35                  40
   tgg ctt gtt att ggc gtt gca ttt ctt gct gtt ttt cag agc gct acc      316
   Trp Leu Val Ile Gly Val Ala Phe Leu Ala Val Phe Gln Ser Ala Thr
   45                  50                  55                  60
   aaa ata att gcg ctc aat aaa aga tgg cag cta gcc ctt tat aag ggc      364
   Lys Ile Ile Ala Leu Asn Lys Arg Trp Gln Leu Ala Leu Tyr Lys Gly
                   65                  70                  75
   ttc cag ttc att tgc aat tta ctg ctg cta ttt gtt acc atc tat tca      412
   Phe Gln Phe Ile Cys Asn Leu Leu Leu Leu Phe Val Thr Ile Tyr Ser
               80                  85                  90
   cat ctt ttg ctt gtc gct gca ggt atg gag gcg caa ttt ttg tac ctc      460
   His Leu Leu Leu Val Ala Ala Gly Met Glu Ala Gln Phe Leu Tyr Leu
           95                  100                 105
   tat gcc ttg ata tat ttt cta caa tgc atc aac gca tgt aga att att      508
   Tyr Ala Leu Ile Tyr Phe Leu Gln Cys Ile Asn Ala Cys Arg Ile Ile
       110                 115                 120
   atg aga tgt tgg ctt tgt tgg aag tgc aaa tcc aag aac cca tta ctt      556
   Met Arg Cys Trp Leu Cys Trp Lys Cys Lys Ser Lys Asn Pro Leu Leu
   125                 130                 135                 140
   tat gat gcc aac tac ttt gtt tgc tgg cac aca cat aac tat gac tac      604
   Tyr Asp Ala Asn Tyr Phe Val Cys Trp His Thr His Asn Tyr Asp Tyr
                   145                 150                 155
   tgt ata cca tat aac agt gtc aca gat aca att gtc gtt act gaa ggt      652
   Cys Ile Pro Tyr Asn Ser Val Thr Asp Thr Ile Val Val Thr Glu Gly
               160                 165                 170
   gac ggc att tca aca cca aaa ctc aaa gaa gac tac caa att ggt ggt      700
   Asp Gly Ile Ser Thr Pro Lys Leu Lys Glu Asp Tyr Gln Ile Gly Gly
           175                 180                 185
   tat tct gag gat agg cac tca ggt gtt aaa gac tat gtc gtt gta cat      748
   Tyr Ser Glu Asp Arg His Ser Gly Val Lys Asp Tyr Val Val Val His
       190                 195                 200
   ggc tat ttc acc gaa gtt tac tac cag ctt gag tct aca caa att act      796
   Gly Tyr Phe Thr Glu Val Tyr Tyr Gln Leu Glu Ser Thr Gln Ile Thr
   205                 210                 215                 220
   aca gac act ggt att gaa aat gct aca ttc ttc atc ttt aac aag ctt      844
   Thr Asp Thr Gly Ile Glu Asn Ala Thr Phe Phe Ile Phe Asn Lys Leu
                   225                 230                 235
   gtt aaa gac cca ccg aat gtg caa ata cac aca atc gac ggc tct tca      892
   Val Lys Asp Pro Pro Asn Val Gln Ile His Thr Ile Asp Gly Ser Ser
               240                 245                 250
   gga gtt gct aat cca gca atg gat cca att tat gat gag ccg acg acg      940
   Gly Val Ala Asn Pro Ala Met Asp Pro Ile Tyr Asp Glu Pro Thr Thr
           255                 260                 265
   act act agc gtg cct ttg taagcacaag aaagtgagta cgaacttatg             988
   Thr Thr Ser Val Pro Leu
       270
   tactcattcg tttcggaaga aacaggtacg ttaatagtta atagcgtact tctttttctt   1048
   gctttcgtgg tattcttgct agtcacacta gccatcctta ctgcgctt                1096
<210> 10
   <211> 274
   <212> PRT
   <213> CORONAVIRUS
<400> 10
   Met Asp Leu Phe Met Arg Phe Phe Thr Leu Gly Ser Ile Thr Ala Gln
   1               5                   10                  15
   Pro Val Lys Ile Asp Asn Ala Ser Pro Ala Ser Thr Val His Ala Thr
               20                  25                  30
   Ala Thr Ile Pro Leu Gln Ala Ser Leu Pro Phe Gly Trp Leu Val Ile
           35                  40                  45
   Gly Val Ala Phe Leu Ala Val Phe Gln Ser Ala Thr Lys Ile Ile Ala
       50                  55                  60
   Leu Asn Lys Arg Trp Gln Leu Ala Leu Tyr Lys Gly Phe Gln Phe Ile
   65                  70                  75                  80
   Cys Asn Leu Leu Leu Leu Phe Val Thr Ile Tyr Ser His Leu Leu Leu
                   85                  90                  95
   Val Ala Ala Gly Met Glu Ala Gln Phe Leu Tyr Leu Tyr Ala Leu Ile
               100                 105                 110
   Tyr Phe Leu Gln Cys Ile Asn Ala Cys Arg Ile Ile Met Arg Cys Trp
           115                 120                 125
   Leu Cys Trp Lys Cys Lys Ser Lys Asn Pro Leu Leu Tyr Asp Ala Asn
       130                 135                 140
   Tyr Phe Val Cys Trp His Thr His Asn Tyr Asp Tyr Cys Ile Pro Tyr
   145 150 155 160
   Asn Ser Val Thr Asp Thr Ile Val Val Thr Glu Gly Asp Gly Ile Ser
   165 170 175
   Thr Pro Lys Leu Lys Glu Asp Tyr Gln Ile Gly Gly Tyr Ser Glu Asp
   180 185 190
   Arg His Ser Gly Val Lys Asp Tyr Val Val Val His Gly Tyr Phe Thr
   195 200 205
   Glu Val Tyr Tyr Gln Leu Glu Ser Thr Gln Ile Thr Thr Asp Thr Gly
   210 215 220
   Ile Glu Asn Ala Thr Phe Phe Ile Phe Asn Lys Leu Val Lys Asp Pro
   225 230 235 240
   Pro Asn Val Gln Ile His Thr Ile Asp Gly Ser Ser Gly Val Ala Asn
   245 250 255
   Pro Ala Met Asp Pro Ile Tyr Asp Glu Pro Thr Thr Thr Thr Ser Val
   260 265 270
   Pro Leu
<210> 11
   <211> 1096
   <212> DNA
   <213> CORONAVIRUS
<220>
   <221> CDS
   <222> (558)..(1019)
   <223>
<400> 11
   tcttgctttg ttgcatgact agttgttgca gttgcctcaa gggtgcatgc tcttgtggtt 60
   cttgctgcaa gtttgatgag gatgactctg agccagttct caagggtgtc aaattacatt 120
   acacataaac gaacttatgg atttgtttat gagatttttt actcttggat caattactgc 180
   acagccagta aaaattgaca atgcttctcc tgcaagtact gttcatgcta cagcaacgat 240
   accgctacaa gcctcactcc ctttcggatg gcttgttatt ggcgttgcat ttcttgctgt 300
   ttttcagagc gctaccaaaa taattgcgct caataaaaga tggcagctag ccctttataa      360
   gggcttccag ttcatttgca atttactgct gctatttgtt accatctatt cacatctttt      420
   gcttgtcgct gcaggtatgg aggcgcaatt tttgtacctc tatgccttga tatatttctt      480
   acaatgcatc aacgcatgta gaattattat gagatgttgg ctttgttgga agtgcaaatc      540
   caagaaccca ttacttt atg atg cca act act ttg ttt gct ggc aca cac         590
                      Met Met Pro Thr Thr Leu Phe Ala Gly Thr His
                      1               5                   10
   ata act atg act act gta tac cat ata aca gtg tca cag ata caa ttg        638
   Ile Thr Met Thr Thr Val Tyr His Ile Thr Val Ser Gln Ile Gln Leu
               15                  20                  25
   tcg tta ctg aag gtg acg gca ttt caa cac caa aac tca aag aag act        686
   Ser Leu Leu Lys Val Thr Ala Phe Gln His Gln Asn Ser Lys Lys Thr
           30                  35                  40
   acc aaa ttg gtg gtt att ctg agg ata ggc act cag gtg tta aag act        734
   Thr Lys Leu Val Val Ile Leu Arg Ile Gly Thr Gln Val Leu Lys Thr
       45                  50                  55
   atg tcg ttg tac atg gct att tca ccg aag ttt act acc agc ttg agt        782
   Met Ser Leu Tyr Met Ala Ile Ser Pro Lys Phe Thr Thr Ser Leu Ser
   60                  65                  70                  75
   cta cac aaa tta cta cag aca ctg gta ttg aaa atg cta cat tct tca        830
   Leu His Lys Leu Leu Gln Thr Leu Val Leu Lys Met Leu His Ser Ser
                   80                  85                  90
   tct tta aca agc ttg tta aag acc cac cga atg tgc aaa tac aca caa        878
   Ser Leu Thr Ser Leu Leu Lys Thr His Arg Met Cys Lys Tyr Thr Gln
               95                  100                 105
   tcg acg gct ctt cag gag ttg cta atc cag caa tgg atc caa ttt atg        926
   Ser Thr Ala Leu Gln Glu Leu Leu Ile Gln Gln Trp Ile Gln Phe Met
           110                 115                 120
   atg agc cga cga cga cta cta gcg tgc ctt tgt aag cac aag aaa gtg        974
   Met Ser Arg Arg Arg Leu Leu Ala Cys Leu Cys Lys His Lys Lys Val
       125                 130                 135
   agt acg aac tta tgt act cat tcg ttt cgg aag aaa cag gta cgt           1019
   Ser Thr Asn Leu Cys Thr His Ser Phe Arg Lys Lys Gln Val Arg
   140                 145                 150
   taatagttaa tagcgtactt ctttttcttg ctttcgtggt attcttgcta gtcacactag     1079
   ccatccttac tgcgctt                                                    1096
<210> 12
   <211> 154
   <212> PRT
   <213> CORONAVIRUS
<400> 12
   Met Met Pro Thr Thr Leu Phe Ala Gly Thr His Ile Thr Met Thr Thr
   1               5                   10                  15
   Val Tyr His Ile Thr Val Ser Gln Ile Gln Leu Ser Leu Leu Lys Val
               20                  25                  30
   Thr Ala Phe Gln His Gln Asn Ser Lys Lys Thr Thr Lys Leu Val Val
           35                  40                  45
   Ile Leu Arg Ile Gly Thr Gln Val Leu Lys Thr Met Ser Leu Tyr Met
       50                  55                  60
   Ala Ile Ser Pro Lys Phe Thr Thr Ser Leu Ser Leu His Lys Leu Leu
   65                  70                  75                  80
   Gln Thr Leu Val Leu Lys Met Leu His Ser Ser Ser Leu Thr Ser Leu
                   85                  90                  95
   Leu Lys Thr His Arg Met Cys Lys Tyr Thr Gln Ser Thr Ala Leu Gln
               100                 105                 110
   Glu Leu Leu Ile Gln Gln Trp Ile Gln Phe Met Met Ser Arg Arg Arg
           115                 120                 125
   Leu Leu Ala Cys Leu Cys Lys His Lys Lys Val Ser Thr Asn Leu Cys
       130                 135                 140
   Thr His Ser Phe Arg Lys Lys Gln Val Arg
   145                 150
<210> 13
   <211> 332
   <212> DNA
   <213> CORONAVIRUS
<220>
   <221> CDS
   <222> (36)..(263)
   <223>
<400> 13
   tgcctttgta agcacaagaa agtgagtacg aactt atg tac tca ttc gtt tcg        53
                                          Met Tyr Ser Phe Val Ser
                                          1               5
   gaa gaa aca ggt acg tta ata gtt aat agc gta ctt ctt ttt ctt gct      101
   Glu Glu Thr Gly Thr Leu Ile Val Asn Ser Val Leu Leu Phe Leu Ala
               10                  15                  20
   ttc gtg gta ttc ttg cta gtc aca cta gcc atc ctt act gcg ctt cga      149
   Phe Val Val Phe Leu Leu Val Thr Leu Ala Ile Leu Thr Ala Leu Arg
           25                  30                  35
   ttg tgt gcg tac tgc tgc aat att gtt aac gtg agt tta gta aaa cca      197
   Leu Cys Ala Tyr Cys Cys Asn Ile Val Asn Val Ser Leu Val Lys Pro
       40                  45                  50
   acg gtt tac gtc tac tcg cgt gtt aaa aat ctg aac tct tct gaa gga      245
   Thr Val Tyr Val Tyr Ser Arg Val Lys Asn Leu Asn Ser Ser Glu Gly
   55                  60                  65                  70
   gtt cct gat ctt ctg gtc taaacgaact aactattatt attattctgt             293
   Val Pro Asp Leu Leu Val
                   75
   ttggaacttt aacattgctt atcatggcag acaacggta                           332
<210> 14
   <211> 76
   <212> PRT
   <213> CORONAVIRUS
<400> 14
   Met Tyr Ser Phe Val Ser Glu Glu Thr Gly Thr Leu Ile Val Asn Ser
   1               5                   10                  15
   Val Leu Leu Phe Leu Ala Phe Val Val Phe Leu Leu Val Thr Leu Ala
               20                  25                  30
   Ile Leu Thr Ala Leu Arg Leu Cys Ala Tyr Cys Cys Asn Ile Val Asn
           35                  40                  45
   Val Ser Leu Val Lys Pro Thr Val Tyr Val Tyr Ser Arg Val Lys Asn
       50                  55                  60
   Leu Asn Ser Ser Glu Gly Val Pro Asp Leu Leu Val
   65                  70                  75
<210> 15
   <211> 332
   <212> DNA
   <213> CORONAVIRUS
<400> 15
   tgcctttgta agcacaagaa agtgagtacg aacttatgta ctcattcgtt tcggaagaaa     60
   caggtacgtt aatagttaat agcgtacttc tttttcttgc tttcgtggta ttcttgctag    120
   tcacactagc catccttact gcgcttcgat tgtgtgcgta ctgctgcaat attgttaacg    180
   tgagtttagt aaaaccaacg gtttacgtct actcgcgtgt taaaaatctg aactcttctg    240
   aaggagttcc tgatcttctg gtctaaacga actaactatt attattattc tgtttggaac    300
   tttaacattg cttatcatgg cagacaacgg ta                                  332
<210> 16
   <211> 708
   <212> DNA
   <213> CORONAVIRUS
<220>
   <221> CDS
   <222> (41)..(703)
   <223>
<400> 16
   tattattatt attctgtttg gaactttaac attgcttatc atg gca gac aac ggt        55
                                               Met Ala Asp Asn Gly
                                               1               5
   act att acc gtt gag gag ctt aaa caa ctc ctg gaa caa tgg aac cta       103
   Thr Ile Thr Val Glu Glu Leu Lys Gln Leu Leu Glu Gln Trp Asn Leu
                   10                  15                  20
   gta ata ggt ttc cta ttc cta gcc tgg att atg tta cta caa ttt gcc       151
   Val Ile Gly Phe Leu Phe Leu Ala Trp Ile Met Leu Leu Gln Phe Ala
               25                  30                  35
   tat tct aat cgg aac agg ttt ttg tac ata ata aag ctt gtt ttc ctc       199
   Tyr Ser Asn Arg Asn Arg Phe Leu Tyr Ile Ile Lys Leu Val Phe Leu
           40                  45                  50
   tgg ctc ttg tgg cca gta aca ctt gct tgt ttt gtg ctt gct gct gtc       247
   Trp Leu Leu Trp Pro Val Thr Leu Ala Cys Phe Val Leu Ala Ala Val
       55                  60                  65
   tac aga att aat tgg gtg act ggc ggg att gcg att gca atg gct tgt       295
   Tyr Arg Ile Asn Trp Val Thr Gly Gly Ile Ala Ile Ala Met Ala Cys
   70                  75                  80                  85
   att gta ggc ttg atg tgg ctt agc tac ttc gtt gct tcc ttc agg ctg       343
   Ile Val Gly Leu Met Trp Leu Ser Tyr Phe Val Ala Ser Phe Arg Leu
                   90                  95                  100
   ttt gct cgt acc cgc tca atg tgg tca ttc aac cca gaa aca aac att       391
   Phe Ala Arg Thr Arg Ser Met Trp Ser Phe Asn Pro Glu Thr Asn Ile
               105                 110                 115
   ctt ctc aat gtg cct ctc cgg ggg aca att gtg acc aga ccg ctc atg       439
   Leu Leu Asn Val Pro Leu Arg Gly Thr Ile Val Thr Arg Pro Leu Met
           120                 125                 130
   gaa agt gaa ctt gtc att ggt gct gtg atc att cgt ggt cac ttg cga       487
   Glu Ser Glu Leu Val Ile Gly Ala Val Ile Ile Arg Gly His Leu Arg
       135                 140                 145
   atg gcc gga cac tcc cta ggg cgc tgt gac att aag gac ctg cca aaa       535
   Met Ala Gly His Ser Leu Gly Arg Cys Asp Ile Lys Asp Leu Pro Lys
   150                     155                     160                     165
   gag atc act gtg gct aca tca cga acg ctt tct tat tac aaa tta gga        583
   Glu Ile Thr Val Ala Thr Ser Arg Thr Leu Ser Tyr Tyr Lys Leu Gly
                   170                 175                 180
   gcg tcg cag cgt gta ggc act gat tca ggt ttt gct gca tac aac cgc        631
   Ala Ser Gln Arg Val Gly Thr Asp Ser Gly Phe Ala Ala Tyr Asn Arg
               185                 190                 195
   tac cgt att gga aac tat aaa tta aat aca gac cac gcc ggt agc aac        679
   Tyr Arg Ile Gly Asn Tyr Lys Leu Asn Thr Asp His Ala Gly Ser Asn
           200                 205                 210
   gac aat att gct ttg cta gta cag taagt                                  708
   Asp Asn Ile Ala Leu Leu Val Gln
       215                 220
<210> 17
   <211> 221
   <212> PRT
   <213> CORONAVIRUS
<400> 17
   Met Ala Asp Asn Gly Thr Ile Thr Val Glu Glu Leu Lys Gln Leu Leu
   1               5                   10                  15
   Glu Gln Trp Asn Leu Val Ile Gly Phe Leu Phe Leu Ala Trp Ile Met
               20                  25                  30
   Leu Leu Gln Phe Ala Tyr Ser Asn Arg Asn Arg Phe Leu Tyr Ile Ile
           35                  40                  45
   Lys Leu Val Phe Leu Trp Leu Leu Trp Pro Val Thr Leu Ala Cys Phe
       50                  55                  60
   Val Leu Ala Ala Val Tyr Arg Ile Asn Trp Val Thr Gly Gly Ile Ala
   65                  70                  75                  80
   Ile Ala Met Ala Cys Ile Val Gly Leu Met Trp Leu Ser Tyr Phe Val
                   85                  90                  95
   Ala Ser Phe Arg Leu Phe Ala Arg Thr Arg Ser Met Trp Ser Phe Asn
               100                 105                 110
   Pro Glu Thr Asn Ile Leu Leu Asn Val Pro Leu Arg Gly Thr Ile Val
           115                 120                 125
   Thr Arg Pro Leu Met Glu Ser Glu Leu Val Ile Gly Ala Val Ile Ile
       130                 135                 140
   Arg Gly His Leu Arg Met Ala Gly His Ser Leu Gly Arg Cys Asp Ile
   145                 150                 155                 160
   Lys Asp Leu Pro Lys Glu Ile Thr Val Ala Thr Ser Arg Thr Leu Ser
                   165                 170                 175
   Tyr Tyr Lys Leu Gly Ala Ser Gln Arg Val Gly Thr Asp Ser Gly Phe
               180                 185                 190
   Ala Ala Tyr Asn Arg Tyr Arg Ile Gly Asn Tyr Lys Leu Asn Thr Asp
           195                 200                 205
   His Ala Gly Ser Asn Asp Asn Ile Ala Leu Leu Val Gln
       210                 215                 220
<210> 18
   <211> 769
   <212> DNA
   <213> CORONAVIRUS
<400> 18
   cctgatcttc tggtctaaac gaactaacta ttattattat tctgtttgga actttaacat      60
   tgcttatcat ggcagacaac ggtactatta ccgttgagga gcttaaacaa ctcctggaac     120
   aatggaacct agtaataggt ttcctattcc tagcctggat tatgttacta caatttgcct     180
   attctaatcg gaacaggttt ttgtacataa taaagcttgt tttcctctgg ctcttgtggc     240
   cagtaacact tgcttgtttt gtgcttgctg ctgtctacag aattaattgg gtgactggcg     300
   ggattgcgat tgcaatggct tgtattgtag gcttgatgtg gcttagctac ttcgttgctt     360
   ccttcaggct gtttgctcgt acccgctcaa tgtggtcatt caacccagaa acaaacattc     420
   ttctcaatgt gcctctccgg gggacaattg tgaccagacc gctcatggaa agtgaacttg     480
   tcattggtgc tgtgatcatt cgtggtcact tgcgaatggc cggacactcc ctagggcgct     540
   gtgacattaa ggacctgcca aaagagatca ctgtggctac atcacgaacg ctttcttatt     600
   acaaattagg agcgtcgcag cgtgtaggca ctgattcagg ttttgctgca tacaaccgct     660
   accgtattgg aaactataaa ttaaatacag accacgccgg tagcaacgac aatattgctt     720
   tgctagtaca gtaagtgaca acagatgttt catcttgttg acttccagg                 769
<210> 19
   <211> 1231
   <212> DNA
   <213> CORONAVIRUS
<400> 19
   taccgtattg gaaactataa attaaataca gaccacgccg gtagcaacga caatattgct      60
   ttgctagtac agtaagtgac aacagatgtt tcatcttgtt gacttccagg ttacaatagc     120
   agagatattg attatcatta tgaggacttt caggattgct atttggaatc ttgacgttat     180
   aataagttca atagtgagac aattatttaa gcctctaact aagaagaatt attcggagtt     240
   agatgatgaa gaacctatgg agttagatta tccataaaac gaacatgaaa attattctct     300
   tcctgacatt gattgtattt acatcttgcg agctatatca ctatcaggag tgtgttagag     360
   gtacgactgt actactaaaa gaaccttgcc catcaggaac atacgagggc aattcaccat     420
   ttcaccctct tgctgacaat aaatttgcac taacttgcac tagcacacac tttgcttttg     480
   cttgtgctga cggtactcga catacctatc agctgcgtgc aagatcagtt tcaccaaaac     540
   ttttcatcag acaagaggag gttcaacaag agctctactc gccacttttt ctcattgttg     600
   ctgctctagt atttttaata ctttgcttca ccattaagag aaagacagaa tgaatgagct     660
   cactttaatt gacttctatt tgtgcttttt agcctttctg ctattccttg ttttaataat     720
   gcttattata ttttggtttt cactcgaaat ccaggatcta gaagaacctt gtaccaaagt     780
   ctaaacgaac atgaaacttc tcattgtttt gacttgtatt tctctatgca gttgcatatg     840
   cactgtagta cagcgctgtg catctaataa acctcatgtg cttgaagatc cttgtaaggt     900
   acaacactag ggtaatactt tatagcactg cttggctttg tgctctagga aaggttttac      960
   cttttcatag atggcacact atggttcaaa catgcacacc taatgttact atcaactgtc    1020
   aagatccagc tggtggtgcg cttatagcta ggtgttggta ccttcatgaa ggtcaccaaa    1080
   ctgctgcatt tagagacgta cttgttgttt taaataaacg aacaaattaa aatgtctgat    1140
   aatggacccc aatcaaacca acgtagtgcc ccccgcatta catttggtgg acccacagat    1200
   tcaactgaca ataaccagaa tggaggacgc a                                   1231
<210> 20
   <211> 1242
   <212> DNA
   <213> CORONAVIRUS
<400> 20
   gcatacaacc gctaccgtat tggaaactat aaattaaata cagaccacgc cggtagcaac      60
   gacaatattg ctttgctagt acagtaagtg acaacagatg tttcatcttg ttgacttcca     120
   ggttacaata gcagagatat tgattatcat tatgaggact ttcaggattg ctatttggaa     180
   tcttgacgtt ataataagtt caatagtgag acagttattt aagcctctaa ctaagaagaa     240
   ttattcggag ttagatgatg aagaacctat ggagttagat tatccataaa acgaacatga     300
   aaattattct cttcctgaca ttgattgtat ttacatcttg cgagctatat cactatcagg     360
   agtgtgttag aggtacgact gtactactaa aagaaccttg cccatcagga acatacgagg     420
   gcaattcacc atttcaccct cttgctgaca ataaatttgc actaacttgc actagcacac     480
   actttgcttt tgcttgtgct gacggtactc gacataccta tcagctgcgt gcaagatcag     540
   tttcaccaaa acttttcatc agacaagagg aggttcaaca agagctctac tcgccacttt     600
   ttctcattgt tgctgctcta gtatttttaa tactttgctt caccattaag agaaagacag     660
   aatgaatgag ctcactttaa ttgacttcta tttgtgcttt ttagcctttc tgctattcct     720
   tgttttaata atgcttatta tattttggtt ttcactcgaa atccaggatc tagaagaacc     780
   ttgtaccaaa gtctaaacga acatgaaact tctcattgtt ttgacttgta tttctctatg     840
   cagttgcata tgcactgtag tacagcgctg tgcatctaat aaacctcatg tgcttgaaga     900
   tccttgtaag gtacaacact aggggtaata cttatagcac tgcttggctt tgtgctctag     960
   gaaaggtttt accttttcat agatggcaca ctatggttca aacatgcaca cctaatgtta    1020
   ctatcaactg tcaagatcca gctggtggtg cgcttatagc taggtgttgg taccttcatg    1080
   aaggtcacca aactgctgca tttagagacg tacttgttgt tttaaataaa cgaacgaatt    1140
   aaaatgtctg ataatggacc ccaatcaaac caacgtagtg ccccccgcat tacatttggt    1200
   ggacccacag attcaactga caataaccag aatggaggac gc                       1242
<210> 21
   <211> 1231
   <212> DNA
   <213> CORONAVIRUS
<220>
   <221> CDS
   <222> (86)..(274)
   <223>
<400> 21
   taccgtattg gaaactataa attaaataca gaccacgccg gtagcaacga caatattgct      60
   ttgctagtac agtaagtgac aacag atg ttt cat ctt gtt gac ttc cag gtt       112
                               Met Phe His Leu Val Asp Phe Gln Val
                               1               5
   aca ata gca gag ata ttg att atc att atg agg act ttc agg att gct       160
   Thr Ile Ala Glu Ile Leu Ile Ile Ile Met Arg Thr Phe Arg Ile Ala
   10                  15                  20                  25
   att tgg aat ctt gac gtt ata ata agt tca ata gtg aga caa tta ttt       208
   Ile Trp Asn Leu Asp Val Ile Ile Ser Ser Ile Val Arg Gln Leu Phe
                   30                  35                  40
   aag cct cta act aag aag aat tat tcg gag tta gat gat gaa gaa cct       256
   Lys Pro Leu Thr Lys Lys Asn Tyr Ser Glu Leu Asp Asp Glu Glu Pro
               45                  50                  55
   atg gag tta gat tat cca taaaacgaac atgaaaatta ttctcttcct          304
   Met Glu Leu Asp Tyr Pro
           60
   gacattgatt gtatttacat cttgcgagct atatcactat caggagtgtg ttagaggtac  364
   gactgtacta ctaaaagaac cttgcccatc aggaacatac gagggcaatt caccatttca  424
   ccctcttgct gacaataaat ttgcactaac ttgcactagc acacactttg cttttgcttg  484
   tgctgacggt actcgacata cctatcagct gcgtgcaaga tcagtttcac caaaactttt  544
   catcagacaa gaggaggttc aacaagagct ctactcgcca ctttttctca ttgttgctgc  604
   tctagtattt ttaatacttt gcttcaccat taagagaaag acagaatgaa tgagctcact  664
   ttaattgact tctatttgtg ctttttagcc tttctgctat tccttgtttt aataatgctt  724
   attatatttt ggttttcact cgaaatccag gatctagaag aaccttgtac caaagtctaa  784
   acgaacatga aacttctcat tgttttgact tgtatttctc tatgcagttg catatgcact  844
   gtagtacagc gctgtgcatc taataaacct catgtgcttg aagatccttg taaggtacaa  904
   cactaggggt aatacttata gcactgcttg gctttgtgct ctaggaaagg ttttaccttt  964
   tcatagatgg cacactatgg ttcaaacatg cacacctaat gttactatca actgtcaaga  1024
   tccagctggt ggtgcgctta tagctaggtg ttggtacctt catgaaggtc accaaactgc  1084
   tgcatttaga gacgtacttg ttgttttaaa taaacgaaca aattaaaatg tctgataatg  1144
   gaccccaatc aaaccaacgt agtgcccccc gcattacatt tggtggaccc acagattcaa  1204
   ctgacaataa ccagaatgga ggacgca                                     1231
<210> 22
   <211> 63
   <212> PRT
   <213> CORONAVIRUS
<400> 22
   Met Phe His Leu Val Asp Phe Gln Val Thr Ile Ala Glu Ile Leu Ile
   1               5                   10                  15
   Ile Ile Met Arg Thr Phe Arg Ile Ala Ile Trp Asn Leu Asp Val Ile
               20                  25                  30
   Ile Ser Ser Ile Val Arg Gln Leu Phe Lys Pro Leu Thr Lys Lys Asn
           35                  40                  45
   Tyr Ser Glu Leu Asp Asp Glu Glu Pro Met Glu Leu Asp Tyr Pro
       50                  55                  60
<210> 23
   <211> 1231
   <212> DNA
   <213> CORONAVIRUS
<220>
   <221> CDS
   <222> (285)..(650)
   <223>
<400> 23
   taccgtattg gaaactataa attaaataca gaccacgccg gtagcaacga caatattgct      60
   ttgctagtac agtaagtgac aacagatgtt tcatcttgtt gacttccagg ttacaatagc     120
   agagatattg attatcatta tgaggacttt caggattgct atttggaatc ttgacgttat     180
   aataagttca atagtgagac aattatttaa gcctctaact aagaagaatt attcggagtt     240
   agatgatgaa gaacctatgg agttagatta tccataaaac gaac atg aaa att att      296
                                                    Met Lys Ile Ile
                                                    1
   ctc ttc ctg aca ttg att gta ttt aca tct tgc gag cta tat cac tat      344
   Leu Phe Leu Thr Leu Ile Val Phe Thr Ser Cys Glu Leu Tyr His Tyr
   5                   10                  15                  20
   cag gag tgt gtt aga ggt acg act gta cta cta aaa gaa cct tgc cca      392
   Gln Glu Cys Val Arg Gly Thr Thr Val Leu Leu Lys Glu Pro Cys Pro
                   25                  30                  35
   tca gga aca tac gag ggc aat tca cca ttt cac cct ctt gct gac aat      440
   Ser Gly Thr Tyr Glu Gly Asn Ser Pro Phe His Pro Leu Ala Asp Asn
               40                  45                  50
   aaa ttt gca cta act tgc act agc aca cac ttt gct ttt gct tgt gct      488
   Lys Phe Ala Leu Thr Cys Thr Ser Thr His Phe Ala Phe Ala Cys Ala
           55                  60                  65
   gac ggt act cga cat acc tat cag ctg cgt gca aga tca gtt tca cca      536
   Asp Gly Thr Arg His Thr Tyr Gln Leu Arg Ala Arg Ser Val Ser Pro
       70                  75                  80
   aaa ctt ttc atc aga caa gag gag gtt caa caa gag ctc tac tcg cca      584
   Lys Leu Phe Ile Arg Gln Glu Glu Val Gln Gln Glu Leu Tyr Ser Pro
   85                  90                  95                  100
   ctt ttt ctc att gtt gct gct cta gta ttt tta ata ctt tgc ttc acc      632
   Leu Phe Leu Ile Val Ala Ala Leu Val Phe Leu Ile Leu Cys Phe Thr
                   105                 110                 115
   att aag aga aag aca gaa tgaatgagct cactttaatt gacttctatt             680
   Ile Lys Arg Lys Thr Glu
               120
   tgtgcttttt agcctttctg ctattccttg ttttaataat gcttattata ttttggtttt    740
   cactcgaaat ccaggatcta gaagaacctt gtaccaaagt ctaaacgaac atgaaacttc    800
   tcattgtttt gacttgtatt tctctatgca gttgcatatg cactgtagta cagcgctgtg    860
   catctaataa acctcatgtg cttgaagatc cttgtaaggt acaacactag gggtaatact     920
   tatagcactg cttggctttg tgctctagga aaggttttac cttttcatag atggcacact     980
   atggttcaaa catgcacacc taatgttact atcaactgtc aagatccagc tggtggtgcg    1040
   cttatagcta ggtgttggta ccttcatgaa ggtcaccaaa ctgctgcatt tagagacgta    1100
   cttgttgttt taaataaacg aacaaattaa aatgtctgat aatggacccc aatcaaacca    1160
   acgtagtgcc ccccgcatta catttggtgg acccacagat tcaactgaca ataaccagaa    1220
   tggaggacgc a                                                         1231
<210> 24
   <211> 122
   <212> PRT
   <213> CORONAVIRUS
<400> 24
   Met Lys Ile Ile Leu Phe Leu Thr Leu Ile Val Phe Thr Ser Cys Glu
   1               5                   10                  15
   Leu Tyr His Tyr Gln Glu Cys Val Arg Gly Thr Thr Val Leu Leu Lys
               20                  25                  30
   Glu Pro Cys Pro Ser Gly Thr Tyr Glu Gly Asn Ser Pro Phe His Pro
           35                  40                  45
   Leu Ala Asp Asn Lys Phe Ala Leu Thr Cys Thr Ser Thr His Phe Ala
       50                  55                  60
   Phe Ala Cys Ala Asp Gly Thr Arg His Thr Tyr Gln Leu Arg Ala Arg
   65                  70                  75                  80
   Ser Val Ser Pro Lys Leu Phe Ile Arg Gln Glu Glu Val Gln Gln Glu
                   85                  90                  95
   Leu Tyr Ser Pro Leu Phe Leu Ile Val Ala Ala Leu Val Phe Leu Ile
               100                 105                 110
   Leu Cys Phe Thr Ile Lys Arg Lys Thr Glu
           115                 120
<210> 25
   <211> 1231
   <212> DNA
   <213> CORONAVIRUS
<220>
   <221> CDS
   <222> (650)..(781)
   <223>
<400> 25
   taccgtattg gaaactataa attaaataca gaccacgccg gtagcaacga caatattgct      60
   ttgctagtac agtaagtgac aacagatgtt tcatcttgtt gacttccagg ttacaatagc     120
   agagatattg attatcatta tgaggacttt caggattgct atttggaatc ttgacgttat     180
   aataagttca atagtgagac aattatttaa gcctctaact aagaagaatt attcggagtt     240
   agatgatgaa gaacctatgg agttagatta tccataaaac gaacatgaaa attattctct     300
   tcctgacatt gattgtattt acatcttgcg agctatatca ctatcaggag tgtgttagag     360
   gtacgactgt actactaaaa gaaccttgcc catcaggaac atacgagggc aattcaccat     420
   ttcaccctct tgctgacaat aaatttgcac taacttgcac tagcacacac tttgcttttg     480
   cttgtgctga cggtactcga catacctatc agctgcgtgc aagatcagtt tcaccaaaac     540
   ttttcatcag acaagaggag gttcaacaag agctctactc gccacttttt ctcattgttg     600
   ctgctctagt atttttaata ctttgcttca ccattaagag aaagacaga atg aat gag      658
                                                         Met Asn Glu
                                                         1
   ctc act tta att gac ttc tat ttg tgc ttt tta gcc ttt ctg cta ttc       706
   Leu Thr Leu Ile Asp Phe Tyr Leu Cys Phe Leu Ala Phe Leu Leu Phe
       5                   10                  15
   ctt gtt tta ata atg ctt att ata ttt tgg ttt tca ctc gaa atc cag       754
   Leu Val Leu Ile Met Leu Ile Ile Phe Trp Phe Ser Leu Glu Ile Gln
   20                  25                  30                  35
   gat cta gaa gaa cct tgt acc aaa gtc taaacgaaca tgaaacttct             801
   Asp Leu Glu Glu Pro Cys Thr Lys Val
                   40
   cattgttttg acttgtattt ctctatgcag ttgcatatgc actgtagtac agcgctgtgc     861
   atctaataaa cctcatgtgc ttgaagatcc ttgtaaggta caacactagg ggtaatactt     921
   atagcactgc ttggctttgt gctctaggaa aggttttacc ttttcataga tggcacacta     981
   tggttcaaac atgcacacct aatgttacta tcaactgtca agatccagct ggtggtgcgc    1041
   ttatagctag gtgttggtac cttcatgaag gtcaccaaac tgctgcattt agagacgtac    1101
   ttgttgtttt aaataaacga acaaattaaa atgtctgata atggacccca atcaaaccaa    1161
   cgtagtgccc cccgcattac atttggtgga cccacagatt caactgacaa taaccagaat    1221
   ggaggacgca                                                           1231
<210> 26
   <211> 44
   <212> PRT
   <213> CORONAVIRUS
<400> 26
   Met Asn Glu Leu Thr Leu Ile Asp Phe Tyr Leu Cys Phe Leu Ala Phe
   1               5                   10                  15
   Leu Leu Phe Leu Val Leu Ile Met Leu Ile Ile Phe Trp Phe Ser Leu
               20                  25                  30
   Glu Ile Gln Asp Leu Glu Glu Pro Cys Thr Lys Val
           35                  40
<210> 27
   <211> 1231
   <212> DNA
   <213> CORONAVIRUS
<220>
   <221> CDS
   <222> (791)..(907)
   <223>
<400> 27
   taccgtattg gaaactataa attaaataca gaccacgccg gtagcaacga caatattgct      60
   ttgctagtac agtaagtgac aacagatgtt tcatcttgtt gacttccagg ttacaatagc     120
   agagatattg attatcatta tgaggacttt caggattgct atttggaatc ttgacgttat     180
   aataagttca atagtgagac aattatttaa gcctctaact aagaagaatt attcggagtt     240
   agatgatgaa gaacctatgg agttagatta tccataaaac gaacatgaaa attattctct     300
   tcctgacatt gattgtattt acatcttgcg agctatatca ctatcaggag tgtgttagag     360
   gtacgactgt actactaaaa gaaccttgcc catcaggaac atacgagggc aattcaccat     420
   ttcaccctct tgctgacaat aaatttgcac taacttgcac tagcacacac tttgcttttg     480
   cttgtgctga cggtactcga catacctatc agctgcgtgc aagatcagtt tcaccaaaac     540
   ttttcatcag acaagaggag gttcaacaag agctctactc gccacttttt ctcattgttg     600
   ctgctctagt atttttaata ctttgcttca ccattaagag aaagacagaa tgaatgagct     660
   cactttaatt gacttctatt tgtgcttttt agcctttctg ctattccttg ttttaataat     720
   gcttattata ttttggtttt cactcgaaat ccaggatcta gaagaacctt gtaccaaagt     780
   ctaaacgaac atg aaa ctt ctc att gtt ttg act tgt att tct cta tgc       829
              Met Lys Leu Leu Ile Val Leu Thr Cys Ile Ser Leu Cys
              1               5                   10
   agt tgc ata tgc act gta gta cag cgc tgt gca tct aat aaa cct cat      877
   Ser Cys Ile Cys Thr Val Val Gln Arg Cys Ala Ser Asn Lys Pro His
       15                  20                  25
   gtg ctt gaa gat cct tgt aag gta caa cac taggggtaat acttatagca        927
   Val Leu Glu Asp Pro Cys Lys Val Gln His
   30                  35
   ctgcttggct ttgtgctcta ggaaaggttt taccttttca tagatggcac actatggttc    987
   aaacatgcac acctaatgtt actatcaact gtcaagatcc agctggtggt gcgcttatag   1047
   ctaggtgttg gtaccttcat gaaggtcacc aaactgctgc atttagagac gtacttgttg   1107
   ttttaaataa acgaacaaat taaaatgtct gataatggac cccaatcaaa ccaacgtagt   1167
   gccccccgca ttacatttgg tggacccaca gattcaactg acaataacca gaatggagga   1227
   cgca                                                                1231
<210> 28
   <211> 39
   <212> PRT
   <213> CORONAVIRUS
<400> 28
   Met Lys Leu Leu Ile Val Leu Thr Cys Ile Ser Leu Cys Ser Cys Ile
   1               5                   10                  15
   Cys Thr Val Val Gln Arg Cys Ala Ser Asn Lys Pro His Val Leu Glu
               20                  25                  30
   Asp Pro Cys Lys Val Gln His
           35
<210> 29
   <211> 1231
   <212> DNA
   <213> CORONAVIRUS
<220>
   <221> CDS
   <222> (876)..(1127)
   <223>
<400> 29
   taccgtattg gaaactataa attaaataca gaccacgccg gtagcaacga caatattgct      60
   ttgctagtac agtaagtgac aacagatgtt tcatcttgtt gacttccagg ttacaatagc     120
   agagatattg attatcatta tgaggacttt caggattgct atttggaatc ttgacgttat     180
   aataagttca atagtgagac aattatttaa gcctctaact aagaagaatt attcggagtt     240
   agatgatgaa gaacctatgg agttagatta tccataaaac gaacatgaaa attattctct     300
   tcctgacatt gattgtattt acatcttgcg agctatatca ctatcaggag tgtgttagag     360
   gtacgactgt actactaaaa gaaccttgcc catcaggaac atacgagggc aattcaccat     420
   ttcaccctct tgctgacaat aaatttgcac taacttgcac tagcacacac tttgcttttg     480
   cttgtgctga cggtactcga catacctatc agctgcgtgc aagatcagtt tcaccaaaac     540
   ttttcatcag acaagaggag gttcaacaag agctctactc gccacttttt ctcattgttg     600
   ctgctctagt atttttaata ctttgcttca ccattaagag aaagacagaa tgaatgagct     660
   cactttaatt gacttctatt tgtgcttttt agcctttctg ctattccttg ttttaataat     720
   gcttattata ttttggtttt cactcgaaat ccaggatcta gaagaacctt gtaccaaagt     780
   ctaaacgaac atgaaacttc tcattgtttt gacttgtatt tctctatgca gttgcatatg     840
   cactgtagta cagcgctgtg catctaataa acctc atg tgc ttg aag atc ctt        893
                                          Met Cys Leu Lys Ile Leu
                                          1               5
   gta agg tac aac act agg ggt aat act tat agc act gct tgg ctt tgt       941
   Val Arg Tyr Asn Thr Arg Gly Asn Thr Tyr Ser Thr Ala Trp Leu Cys
               10                  15                  20
   gct cta gga aag gtt tta cct ttt cat aga tgg cac act atg gtt caa       989
   Ala Leu Gly Lys Val Leu Pro Phe His Arg Trp His Thr Met Val Gln
           25                  30                  35
   aca tgc aca cct aat gtt act atc aac tgt caa gat cca gct ggt ggt      1037
   Thr Cys Thr Pro Asn Val Thr Ile Asn Cys Gln Asp Pro Ala Gly Gly
       40                  45                  50
   gcg ctt ata gct agg tgt tgg tac ctt cat gaa ggt cac caa act gct      1085
   Ala Leu Ile Ala Arg Cys Trp Tyr Leu His Glu Gly His Gln Thr Ala
   55                  60                  65                  70
   gca ttt aga gac gta ctt gtt gtt tta aat aaa cga aca aat              1127
   Ala Phe Arg Asp Val Leu Val Val Leu Asn Lys Arg Thr Asn
                   75                  80
   taaaatgtct gataatggac cccaatcaaa ccaacgtagt gccccccgca ttacatttgg    1187
   tggacccaca gattcaactg acaataacca gaatggagga cgca                      1231
<210> 30
   <211> 84
   <212> PRT
   <213> CORONAVIRUS
<400> 30
   Met Cys Leu Lys Ile Leu Val Arg Tyr Asn Thr Arg Gly Asn Thr Tyr
   1               5                   10                  15
   Ser Thr Ala Trp Leu Cys Ala Leu Gly Lys Val Leu Pro Phe His Arg
               20                  25                  30
   Trp His Thr Met Val Gln Thr Cys Thr Pro Asn Val Thr Ile Asn Cys
           35                  40                  45
   Gln Asp Pro Ala Gly Gly Ala Leu Ile Ala Arg Cys Trp Tyr Leu His
       50                  55                  60
   Glu Gly His Gln Thr Ala Ala Phe Arg Asp Val Leu Val Val Leu Asn
   65                  70                  75                  80
   Lys Arg Thr Asn
<210> 31
   <211> 21221
   <212> DNA
   <213> CORONAVIRUS
<400> 31
   atggagagcc ttgttcttgg tgtcaacgag aaaacacacg tccaactcag tttgcctgtc      60
   cttcaggtta gagacgtgct agtgcgtggc ttcggggact ctgtggaaga ggccctatcg     120
   gaggcacgtg aacacctcaa aaatggcact tgtggtctag tagagctgga aaaaggcgta     180
   ctgccccagc ttgaacagcc ctatgtgttc attaaacgtt ctgatgcctt aagcaccaat     240
   cacggccaca aggtcgttga gctggttgca gaaatggacg gcattcagta cggtcgtagc     300
   ggtataacac tgggagtact cgtgccacat gtgggcgaaa ccccaattgc ataccgcaat     360
   gttcttcttc gtaagaacgg taataaggga gccggtggtc atagctatgg catcgatcta     420
   aagtcttatg acttaggtga cgagcttggc actgatccca ttgaagatta tgaacaaaac     480
   tggaacacta agcatggcag tggtgcactc cgtgaactca ctcgtgagct caatggaggt     540
   gcagtcactc gctatgtcga caacaatttc tgtggcccag atgggtaccc tcttgattgc     600
   atcaaagatt ttctcgcacg cgcgggcaag tcaatgtgca ctctttccga acaacttgat     660
   tacatcgagt cgaagagagg tgtctactgc tgccgtgacc atgagcatga aattgcctgg     720
   ttcactgagc gctctgataa gagctacgag caccagacac ccttcgaaat taagagtgcc     780
   aagaaatttg acactttcaa aggggaatgc ccaaagtttg tgtttcctct taactcaaaa     840
   gtcaaagtca ttcaaccacg tgttgaaaag aaaaagactg agggtttcat ggggcgtata     900
   cgctctgtgt accctgttgc atctccacag gagtgtaaca atatgcactt gtctaccttg 960
   atgaaatgta atcattgcga tgaagtttca tggcagacgt gcgactttct gaaagccact 1020
   tgtgaacatt gtggcactga aaatttagtt attgaaggac ctactacatg tgggtaccta 1080
   cctactaatg ctgtagtgaa aatgccatgt cctgcctgtc aagacccaga gattggacct 1140
   gagcatagtg ttgcagatta tcacaaccac tcaaacattg aaactcgact ccgcaaggga 1200
   ggtaggacta gatgttttgg aggctgtgtg tttgcctatg ttggctgcta taataagcgt 1260
   gcctactggg ttcctcgtgc tagtgctgat attggctcag gccatactgg cattactggt 1320
   gacaatgtgg agaccttgaa tgaggatctc cttgagatac tgagtcgtga acgtgttaac 1380
   attaacattg ttggcgattt tcatttgaat gaagaggttg ccatcatttt ggcatctttc 1440
   tctgcttcta caagtgcctt tattgacact ataaagagtc ttgattacaa gtctttcaaa 1500
   accattgttg agtcctgcgg taactataaa gttaccaagg gaaagcccgt aaaaggtgct 1560
   tggaacattg gacaacagag atcagtttta acaccactgt gtggttttcc ctcacaggct 1620
   gctggtgtta tcagatcaat ttttgcgcgc acacttgatg cagcaaacca ctcaattcct 1680
   gatttgcaaa gagcagctgt caccatactt gatggtattt ctgaacagtc attacgtctt 1740
   gtcgacgcca tggtttatac ttcagacctg ctcaccaaca gtgtcattat tatggcatat 1800
   gtaactggtg gtcttgtaca acagacttct cagtggttgt ctaatctttt gggcactact 1860
   gttgaaaaac tcaggcctat ctttgaatgg attgaggcga aacttagtgc aggagttgaa 1920
   tttctcaagg atgcttggga gattctcaaa tttctcatta caggtgtttt tgacatcgtc 1980
   aagggtcaaa tacaggttgc ttcagataac atcaaggatt gtgtaaaatg cttcattgat 2040
   gttgttaaca aggcactcga aatgtgcatt gatcaagtca ctatcgctgg cgcaaagttg 2100
   cgatcactca acttaggtga agtcttcatc gctcaaagca agggacttta ccgtcagtgt 2160
   atacgtggca aggagcagct gcaactactc atgcctctta aggcaccaaa agaagtaacc 2220
   tttcttgaag gtgattcaca tgacacagta cttacctctg aggaggttgt tctcaagaac 2280
   ggtgaactcg aagcactcga gacgcccgtt gatagcttca caaatggagc tatcgttggc 2340
   acaccagtct gtgtaaatgg cctcatgctc ttagagatta aggacaaaga acaatactgc 2400
   gcattgtctc ctggtttact ggctacaaac aatgtctttc gcttaaaagg gggtgcacca 2460
   attaaaggtg taacctttgg agaagatact gtttgggaag ttcaaggtta caagaatgtg 2520
   agaatcacat ttgagcttga tgaacgtgtt gacaaagtgc ttaatgaaaa gtgctctgtc 2580
   tacactgttg aatccggtac cgaagttact gagtttgcat gtgttgtagc agaggctgtt 2640
   gtgaagactt tacaaccagt ttctgatctc cttaccaaca tgggtattga tcttgatgag 2700
   tggagtgtag ctacattcta cttatttgat gatgctggtg aagaaaactt ttcatcacgt 2760
   atgtattgtt ccttttaccc tccagatgag gaagaagagg acgatgcaga gtgtgaggaa 2820
   gaagaaattg atgaaacctg tgaacatgag tacggtacag aggatgatta tcaaggtctc 2880
   cctctggaat ttggtgcctc agctgaaaca gttcgagttg aggaagaaga agaggaagac 2940
   tggctggatg atactactga gcaatcagag attgagccag aaccagaacc tacacctgaa 3000
   gaaccagtta atcagtttac tggttattta aaacttactg acaatgttgc cattaaatgt 3060
   gttgacatcg ttaaggaggc acaaagtgct aatcctatgg tgattgtaaa tgctgctaac 3120
   atacacctga aacatggtgg tggtgtagca ggtgcactca acaaggcaac caatggtgcc 3180
   atgcaaaagg agagtgatga ttacattaag ctaaatggcc ctcttacagt aggagggtct 3240
   tgtttgcttt ctggacataa tcttgctaag aagtgtctgc atgttgttgg acctaaccta 3300
   aatgcaggtg aggacatcca gcttcttaag gcagcatatg aaaatttcaa ttcacaggac 3360
   atcttacttg caccattgtt gtcagcaggc atatttggtg ctaaaccact tcagtcttta 3420
   caagtgtgcg tgcagacggt tcgtacacag gtttatattg cagtcaatga caaagctctt 3480
   tatgagcagg ttgtcatgga ttatcttgat aacctgaagc ctagagtgga agcacctaaa 3540
   caagaggagc caccaaacac agaagattcc aaaactgagg agaaatctgt cgtacagaag 3600
   cctgtcgatg tgaagccaaa aattaaggcc tgcattgatg aggttaccac aacactggaa 3660
   gaaactaagt ttcttaccaa taagttactc ttgtttgctg atatcaatgg taagctttac 3720
   catgattctc agaacatgct tagaggtgaa gatatgtctt tccttgagaa ggatgcacct 3780
   tacatggtag gtgatgttat cactagtggt gatatcactt gtgttgtaat accctccaaa 3840
   aaggctggtg gcactactga gatgctctca agagctttga agaaagtgcc agttgatgag 3900
   tatataacca cgtaccctgg acaaggatgt gctggttata cacttgagga agctaagact 3960
   gctcttaaga aatgcaaatc tgcattttat gtactacctt cagaagcacc taatgctaag 4020
   gaagagattc taggaactgt atcctggaat ttgagagaaa tgcttgctca tgctgaagag 4080
   acaagaaaat taatgcctat atgcatggat gttagagcca taatggcaac catccaacgt 4140
   aagtataaag gaattaaaat tcaagagggc atcgttgact atggtgtccg attcttcttt 4200
   tatactagta aagagcctgt agcttctatt attacgaagc tgaactctct aaatgagccg 4260
   cttgtcacaa tgccaattgg ttatgtgaca catggtttta atcttgaaga ggctgcgcgc 4320
   tgtatgcgtt ctcttaaagc tcctgccgta gtgtcagtat catcaccaga tgctgttact 4380
   acatataatg gatacctcac ttcgtcatca aagacatctg aggagcactt tgtagaaaca 4440
   gtttctttgg ctggctctta cagagattgg tcctattcag gacagcgtac agagttaggt 4500
   gttgaatttc ttaagcgtgg tgacaaaatt gtgtaccaca ctctggagag ccccgtcgag 4560
   tttcatcttg acggtgaggt tctttcactt gacaaactaa agagtctctt atccctgcgg 4620
   gaggttaaga ctataaaagt gttcacaact gtggacaaca ctaatctcca cacacagctt 4680
   gtggatatgt ctatgacata tggacagcag tttggtccaa catacttgga tggtgctgat 4740
   gttacaaaaa ttaaacctca tgtaaatcat gagggtaaga ctttctttgt actacctagt 4800
   gatgacacac tacgtagtga agctttcgag tactaccata ctcttgatga gagttttctt 4860
   ggtaggtaca tgtctgcttt aaaccacaca aagaaatgga aatttcctca agttggtggt 4920
   ttaacttcaa ttaaatgggc tgataacaat tgttatttgt ctagtgtttt attagcactt 4980
   caacagcttg aagtcaaatt caatgcacca gcacttcaag aggcttatta tagagcccgt 5040
   gctggtgatg ctgctaactt ttgtgcactc atactcgctt acagtaataa aactgttggc 5100
   gagcttggtg atgtcagaga aactatgacc catcttctac agcatgctaa tttggaatct 5160
   gcaaagcgag ttcttaatgt ggtgtgtaaa cattgtggtc agaaaactac taccttaacg 5220
   ggtgtagaag ctgtgatgta tatgggtact ctatcttatg ataatcttaa gacaggtgtt 5280
   tccattccat gtgtgtgtgg tcgtgatgct acacaatatc tagtacaaca agagtcttct 5340
   tttgttatga tgtctgcacc acctgctgag tataaattac agcaaggtac attcttatgt 5400
   gcgaatgagt acactggtaa ctatcagtgt ggtcattaca ctcatataac tgctaaggag 5460
   accctctatc gtattgacgg agctcacctt acaaagatgt cagagtacaa aggaccagtg 5520
   actgatgttt tctacaagga aacatcttac actacaacca tcaagcctgt gtcgtataaa 5580
   ctcgatggag ttacttacac agagattgaa ccaaaattgg atgggtatta taaaaaggat 5640
   aatgcttact atacagagca gcctatagac cttgtaccaa ctcaaccatt accaaatgcg 5700
   agttttgata atttcaaact cacatgttct aacacaaaat ttgctgatga tttaaatcaa 5760
   atgacaggct tcacaaagcc agcttcacga gagctatctg tcacattctt cccagacttg 5820
   aatggcgatg tagtggctat tgactataga cactattcag cgagtttcaa gaaaggtgct 5880
   aaattactgc ataagccaat tgtttggcac attaaccagg ctacaaccaa gacaacgttc 5940
   aaaccaaaca cttggtgttt acgttgtctt tggagtacaa agccagtaga tacttcaaat 6000
   tcatttgaag ttctggcagt agaagacaca caaggaatgg acaatcttgc ttgtgaaagt 6060
   caacaaccca cctctgaaga agtagtggaa aatcctacca tacagaagga agtcatagag 6120
   tgtgacgtga aaactaccga agttgtaggc aatgtcatac ttaaaccatc agatgaaggt 6180
   gttaaagtaa cacaagagtt aggtcatgag gatcttatgg ctgcttatgt ggaaaacaca 6240
   agcattacca ttaagaaacc taatgagctt tcactagcct taggtttaaa aacaattgcc 6300
   actcatggta ttgctgcaat taatagtgtt ccttggagta aaattttggc ttatgtcaaa 6360
   ccattcttag gacaagcagc aattacaaca tcaaattgcg ctaagagatt agcacaacgt 6420
   gtgtttaaca attatatgcc ttatgtgttt acattattgt tccaattgtg tacttttact 6480
   aaaagtacca attctagaat tagagcttca ctacctacaa ctattgctaa aaatagtgtt 6540
   aagagtgttg ctaaattatg tttggatgcc ggcattaatt atgtgaagtc acccaaattt 6600
   tctaaattgt tcacaatcgc tatgtggcta ttgttgttaa gtatttgctt aggttctcta 6660
   atctgtgtaa ctgctgcttt tggtgtactc ttatctaatt ttggtgctcc ttcttattgt 6720
   aatggcgtta gagaattgta tcttaattcg tctaacgtta ctactatgga tttctgtgaa 6780
   ggttcttttc cttgcagcat ttgtttaagt ggattagact cccttgattc ttatccagct 6840
   cttgaaacca ttcaggtgac gatttcatcg tacaagctag acttgacaat tttaggtctg 6900
   gccgctgagt gggttttggc atatatgttg ttcacaaaat tcttttattt attaggtctt 6960
   tcagctataa tgcaggtgtt ctttggctat tttgctagtc atttcatcag caattcttgg 7020
   ctcatgtggt ttatcattag tattgtacaa atggcacccg tttctgcaat ggttaggatg 7080
   tacatcttct ttgcttcttt ctactacata tggaagagct atgttcatat catggatggt 7140
   tgcacctctt cgacttgcat gatgtgctat aagcgcaatc gtgccacacg cgttgagtgt 7200
   acaactattg ttaatggcat gaagagatct ttctatgtct atgcaaatgg aggccgtggc 7260
   ttctgcaaga ctcacaattg gaattgtctc aattgtgaca cattttgcac tggtagtaca 7320
   ttcattagtg atgaagttgc tcgtgatttg tcactccagt ttaaaagacc aatcaaccct 7380
   actgaccagt catcgtatat tgttgatagt gttgctgtga aaaatggcgc gcttcacctc 7440
   tactttgaca aggctggtca aaagacctat gagagacatc cgctctccca ttttgtcaat 7500
   ttagacaatt tgagagctaa caacactaaa ggttcactgc ctattaatgt catagttttt 7560
   gatggcaagt ccaaatgcga cgagtctgct tctaagtctg cttctgtgta ctacagtcag 7620
   ctgatgtgcc aacctattct gttgcttgac caagctcttg tatcagacgt tggagatagt 7680
   actgaagttt ccgttaagat gtttgatgct tatgtcgaca ccttttcagc aacttttagt 7740
   gttcctatgg aaaaacttaa ggcacttgtt gctacagctc acagcgagtt agcaaagggt 7800
   gtagctttag atggtgtcct ttctacattc gtgtcagctg cccgacaagg tgttgttgat 7860
   accgatgttg acacaaagga tgttattgaa tgtctcaaac tttcacatca ctctgactta 7920
   gaagtgacag gtgacagttg taacaatttc atgctcacct ataataaggt tgaaaacatg 7980
   acgcccagag atcttggcgc atgtattgac tgtaatgcaa ggcatatcaa tgcccaagta 8040
   gcaaaaagtc acaatgtttc actcatctgg aatgtaaaag actacatgtc tttatctgaa 8100
   cagctgcgta aacaaattcg tagtgctgcc aagaagaaca acataccttt tagactaact 8160
   tgtgctacaa ctagacaggt tgtcaatgtc ataactacta aaatctcact caagggtggt 8220
   aagattgtta gtacttgttt taaacttatg cttaaggcca cattattgtg cgttcttgct 8280
   gcattggttt gttatatcgt tatgccagta catacattgt caatccatga tggttacaca 8340
   aatgaaatca ttggttacaa agccattcag gatggtgtca ctcgtgacat catttctact 8400
   gatgattgtt ttgcaaataa acatgctggt tttgacgcat ggtttagcca gcgtggtggt 8460
   tcatacaaaa atgacaaaag ctgccctgta gtagctgcta tcattacaag agagattggt 8520
   ttcatagtgc ctggcttacc gggtactgtg ctgagagcaa tcaatggtga cttcttgcat 8580
   tttctacctc gtgtttttag tgctgttggc aacatttgct acacaccttc caaactcatt 8640
   gagtatagtg attttgctac ctctgcttgc gttcttgctg ctgagtgtac aatttttaag 8700
   gatgctatgg gcaaacctgt gccatattgt tatgacacta atttgctaga gggttctatt 8760
   tcttatagtg agcttcgtcc agacactcgt tatgtgctta tggatggttc catcatacag 8820
   tttcctaaca cttacctgga gggttctgtt agagtagtaa caacttttga tgctgagtac 8880
   tgtagacatg gtacatgcga aaggtcagaa gtaggtattt gcctatctac cagtggtaga 8940
   tgggttctta ataatgagca ttacagagct ctatcaggag ttttctgtgg tgttgatgcg 9000
   atgaatctca tagctaacat ctttactcct cttgtgcaac ctgtgggtgc tttagatgtg 9060
   tctgcttcag tagtggctgg tggtattatt gccatattgg tgacttgtgc tgcctactac 9120
   tttatgaaat tcagacgtgt ttttggtgag tacaaccatg ttgttgctgc taatgcactt 9180
   ttgtttttga tgtctttcac tatactctgt ctggtaccag cttacagctt tctgccggga 9240
   gtctactcag tcttttactt gtacttgaca ttctatttca ccaatgatgt ttcattcttg 9300
   gctcaccttc aatggtttgc catgttttct cctattgtgc ctttttggat aacagcaatc 9360
   tatgtattct gtatttctct gaagcactgc cattggttct ttaacaacta tcttaggaaa 9420
   agagtcatgt ttaatggagt tacatttagt accttcgagg aggctgcttt gtgtaccttt 9480
   ttgctcaaca aggaaatgta cctaaaattg cgtagcgaga cactgttgcc acttacacag 9540
   tataacaggt atcttgctct atataacaag tacaagtatt tcagtggagc cttagatact 9600
   accagctatc gtgaagcagc ttgctgccac ttagcaaagg ctctaaatga ctttagcaac 9660
   tcaggtgctg atgttctcta ccaaccacca cagacatcaa tcacttctgc tgttctgcag 9720
   agtggtttta ggaaaatggc attcccgtca ggcaaagttg aagggtgcat ggtacaagta 9780
   acctgtggaa ctacaactct taatggattg tggttggatg acacagtata ctgtccaaga 9840
   catgtcattt gcacagcaga agacatgctt aatcctaact atgaagatct gctcattcgc 9900
   aaatccaacc atagctttct tgttcaggct ggcaatgttc aacttcgtgt tattggccat 9960
   tctatgcaaa attgtctgct taggcttaaa gttgatactt ctaaccctaa gacacccaag 10020
   tataaatttg tccgtatcca acctggtcaa acattttcag ttctagcatg ctacaatggt 10080
   tcaccatctg gtgtttatca gtgtgccatg agacctaatc ataccattaa aggttctttc 10140
   cttaatggat catgtggtag tgttggtttt aacattgatt atgattgcgt gtctttctgc 10200
   tatatgcatc atatggagct tccaacagga gtacacgctg gtactgactt agaaggtaaa 10260
   ttctatggtc catttgttga cagacaaact gcacaggctg caggtacaga cacaaccata 10320
   acattaaatg ttttggcatg gctgtatgct gctgttatca atggtgatag gtggtttctt 10380
   aatagattca ccactacttt gaatgacttt aaccttgtgg caatgaagta caactatgaa 10440
   cctttgacac aagatcatgt tgacatattg ggacctcttt ctgctcaaac aggaattgcc 10500
   gtcttagata tgtgtgctgc tttgaaagag ctgctgcaga atggtatgaa tggtcgtact 10560
   atccttggta gcactatttt agaagatgag tttacaccat ttgatgttgt tagacaatgc 10620
   tctggtgtta ccttccaagg taagttcaag aaaattgtta agggcactca tcattggatg 10680
   cttttaactt tcttgacatc actattgatt cttgttcaaa gtacacagtg gtcactgttt 10740
   ttctttgttt acgagaatgc tttcttgcca tttactcttg gtattatggc aattgctgca 10800
   tgtgctatgc tgcttgttaa gcataagcac gcattcttgt gcttgtttct gttaccttct 10860
   cttgcaacag ttgcttactt taatatggtc tacatgcctg ctagctgggt gatgcgtatc 10920
   atgacatggc ttgaattggc tgacactagc ttgtctggtt ataggcttaa ggattgtgtt 10980
   atgtatgctt cagctttagt tttgcttatt ctcatgacag ctcgcactgt ttatgatgat 11040
   gctgctagac gtgtttggac actgatgaat gtcattacac ttgtttacaa agtctactat 11100
   ggtaatgctt tagatcaagc tatttccatg tgggccttag ttatttctgt aacctctaac 11160
   tattctggtg tcgttacgac tatcatgttt ttagctagag ctatagtgtt tgtgtgtgtt 11220
   gagtattacc cattgttatt tattactggc aacaccttac agtgtatcat gcttgtttat 11280
   tgtttcttag gctattgttg ctgctgctac tttggccttt tctgtttact caaccgttac 11340
   ttcaggctta ctcttggtgt ttatgactac ttggtctcta cacaagaatt taggtatatg 11400
   aactcccagg ggcttttgcc tcctaagagt agtattgatg ctttcaagct taacattaag 11460
   ttgttgggta ttggaggtaa accatgtatc aaggttgcta ctgtacagtc taaaatgtct 11520
   gacgtaaagt gcacatctgt ggtactgctc tcggttcttc aacaacttag agtagagtca 11580
   tcttctaaat tgtgggcaca atgtgtacaa ctccacaatg atattcttct tgcaaaagac 11640
   acaactgaag ctttcgagaa gatggtttct cttttgtctg ttttgctatc catgcagggt 11700
   gctgtagaca ttaataggtt gtgcgaggaa atgctcgata accgtgctac tcttcaggct 11760
   attgcttcag aatttagttc tttaccatca tatgccgctt atgccactgc ccaggaggcc 11820
   tatgagcagg ctgtagctaa tggtgattct gaagtcgttc tcaaaaagtt aaagaaatct 11880
   ttgaatgtgg ctaaatctga gtttgaccgt gatgctgcca tgcaacgcaa gttggaaaag 11940
   atggcagatc aggctatgac ccaaatgtac aaacaggcaa gatctgagga caagagggca 12000
   aaagtaacta gtgctatgca aacaatgctc ttcactatgc ttaggaagct tgataatgat 12060
   gcacttaaca acattatcaa caatgcgcgt gatggttgtg ttccactcaa catcatacca 12120
   ttgactacag cagccaaact catggttgtt gtccctgatt atggtaccta caagaacact 12180
   tgtgatggta acacctttac atatgcatct gcactctggg aaatccagca agttgttgat 12240
   gcggatagca agattgttca acttagtgaa attaacatgg acaattcacc aaatttggct 12300
   tggcctctta ttgttacagc tctaagagcc aactcagctg ttaaactaca gaataatgaa 12360
   ctgagtccag tagcactacg acagatgtcc tgtgcggctg gtaccacaca aacagcttgt 12420
   actgatgaca atgcacttgc ctactataac aattcgaagg gaggtaggtt tgtgctggca 12480
   ttactatcag accaccaaga tctcaaatgg gctagattcc ctaagagtga tggtacaggt 12540
   acaatttaca cagaactgga accaccttgt aggtttgtta cagacacacc aaaagggcct 12600
   aaagtgaaat acttgtactt catcaaaggc ttaaacaacc taaatagagg tatggtgctg 12660
   ggcagtttag ctgctacagt acgtcttcag gctggaaatg ctacagaagt acctgccaat 12720
   tcaactgtgc tttccttctg tgcttttgca gtagaccctg ctaaagcata taaggattac 12780
   ctagcaagtg gaggacaacc aatcaccaac tgtgtgaaga tgttgtgtac acacactggt 12840
   acaggacagg caattactgt aacaccagaa gctaacatgg accaagagtc ctttggtggt 12900
   gcttcatgtt gtctgtattg tagatgccac attgaccatc caaatcctaa aggattctgt 12960
   gacttgaaag gtaagtacgt ccaaatacct accacttgtg ctaatgaccc agtgggtttt 13020
   acacttagaa acacagtctg taccgtctgc ggaatgtgga aaggttatgg ctgtagttgt 13080
   gaccaactcc gcgaaccctt gatgcagtct gcggatgcat caacgttttt aaacgggttt 13140
   gcggtgtaag tgcagcccgt cttacaccgt gcggcacagg cactagtact gatgtcgtct 13200
   acagggcttt tgatatttac aacgaaaaag ttgctggttt tgcaaagttc ctaaaaacta 13260
   attgctgtcg cttccaggag aaggatgagg aaggcaattt attagactct tactttgtag 13320
   ttaagaggca tactatgtct aactaccaac atgaagagac tatttataac ttggttaaag 13380
   attgtccagc ggttgctgtc catgactttt tcaagtttag agtagatggt gacatggtac 13440
   cacatatatc acgtcagcgt ctaactaaat acacaatggc tgatttagtc tatgctctac 13500
   gtcattttga tgagggtaat tgtgatacat taaaagaaat actcgtcaca tacaattgct 13560
   gtgatgatga ttatttcaat aagaaggatt ggtatgactt cgtagagaat cctgacatct 13620
   tacgcgtata tgctaactta ggtgagcgtg tacgccaatc attattaaag actgtacaat 13680
   tctgcgatgc tatgcgtgat gcaggcattg taggcgtact gacattagat aatcaggatc 13740
   ttaatgggaa ctggtacgat ttcggtgatt tcgtacaagt agcaccaggc tgcggagttc 13800
   ctattgtgga ttcatattac tcattgctga tgcccatcct cactttgact agggcattgg 13860
   ctgctgagtc ccatatggat gctgatctcg caaaaccact tattaagtgg gatttgctga 13920
   aatatgattt tacggaagag agactttgtc tcttcgaccg ttattttaaa tattgggacc 13980
   agacatacca tcccaattgt attaactgtt tggatgatag gtgtatcctt cattgtgcaa 14040
   actttaatgt gttattttct actgtgtttc cacctacaag ttttggacca ctagtaagaa 14100
   aaatatttgt agatggtgtt ccttttgttg tttcaactgg ataccatttt cgtgagttag 14160
   gagtcgtaca taatcaggat gtaaacttac atagctcgcg tctcagtttc aaggaacttt 14220
   tagtgtatgc tgctgatcca gctatgcatg cagcttctgg caatttattg ctagataaac 14280
   gcactacatg cttttcagta gctgcactaa caaacaatgt tgcttttcaa actgtcaaac 14340
   ccggtaattt taataaagac ttttatgact ttgctgtgtc taaaggtttc tttaaggaag 14400
   gaagttctgt tgaactaaaa cacttcttct ttgctcagga tggcaacgct gctatcagtg 14460
   attatgacta ttatcgttat aatctgccaa caatgtgtga tatcagacaa ctcctattcg 14520
   tagttgaagt tgttgataaa tactttgatt gttacgatgg tggctgtatt aatgccaacc 14580
   aagtaatcgt taacaatctg gataaatcag ctggtttccc atttaataaa tggggtaagg 14640
   ctagacttta ttatgactca atgagttatg aggatcaaga tgcacttttc gcgtatacta 14700
   agcgtaatgt catccctact ataactcaaa tgaatcttaa gtatgccatt agtgcaaaga 14760
   atagagctcg caccgtagct ggtgtctcta tctgtagtac tatgacaaat agacagtttc 14820
   atcagaaatt attgaagtca atagccgcca ctagaggagc tactgtggta attggaacaa 14880
   gcaagtttta cggtggctgg cataatatgt taaaaactgt ttacagtgat gtagaaactc 14940
   cacaccttat gggttgggat tatccaaaat gtgacagagc catgcctaac atgcttagga 15000
   taatggcctc tcttgttctt gctcgcaaac ataacacttg ctgtaactta tcacaccgtt 15060
   tctacaggtt agctaacgag tgtgcgcaag tattaagtga gatggtcatg tgtggcggct 15120
   cactatatgt taaaccaggt ggaacatcat ccggtgatgc tacaactgct tatgctaata 15180
   gtgtctttaa catttgtcaa gctgttacag ccaatgtaaa tgcacttctt tcaactgatg 15240
   gtaataagat agctgacaag tatgtccgca atctacaaca caggctctat gagtgtctct 15300
   atagaaatag ggatgttgat catgaattcg tggatgagtt ttacgcttac ctgcgtaaac 15360
   atttctccat gatgattctt tctgatgatg ccgttgtgtg ctataacagt aactatgcgg 15420
   ctcaaggttt agtagctagc attaagaact ttaaggcagt tctttattat caaaataatg 15480
   tgttcatgtc tgaggcaaaa tgttggactg agactgacct tactaaagga cctcacgaat 15540
   tttgctcaca gcatacaatg ctagttaaac aaggagatga ttacgtgtac ctgccttacc 15600
   cagatccatc aagaatatta ggcgcaggct gttttgtcga tgatattgtc aaaacagatg 15660
   gtacacttat gattgaaagg ttcgtgtcac tggctattga tgcttaccca cttacaaaac 15720
   atcctaatca ggagtatgct gatgtctttc acttgtattt acaatacatt agaaagttac 15780
   atgatgagct tactggccac atgttggaca tgtattccgt aatgctaact aatgataaca 15840
   cctcacggta ctgggaacct gagttttatg aggctatgta cacaccacat acagtcttgc 15900
   aggctgtagg tgcttgtgta ttgtgcaatt cacagacttc acttcgttgc ggtgcctgta 15960
   ttaggagacc attcctatgt tgcaagtgct gctatgacca tgtcatttca acatcacaca 16020
   aattagtgtt gtctgttaat ccctatgttt gcaatgcccc aggttgtgat gtcactgatg 16080
   tgacacaact gtatctagga ggtatgagct attattgcaa gtcacataag cctcccatta 16140
   gttttccatt atgtgctaat ggtcaggttt ttggtttata caaaaacaca tgtgtaggca 16200
   gtgacaatgt cactgacttc aatgcgatag caacatgtga ttggactaat gctggcgatt 16260
   acatacttgc caacacttgt actgagagac tcaagctttt cgcagcagaa acgctcaaag 16320
   ccactgagga aacatttaag ctgtcatatg gtattgccac tgtacgcgaa gtactctctg 16380
   acagagaatt gcatctttca tgggaggttg gaaaacctag accaccattg aacagaaact 16440
   atgtctttac tggttaccgt gtaactaaaa atagtaaagt acagattgga gagtacacct 16500
   ttgaaaaagg tgactatggt gatgctgttg tgtacagagg tactacgaca tacaagttga 16560
   atgttggtga ttactttgtg ttgacatctc acactgtaat gccacttagt gcacctactc 16620
   tagtgccaca agagcactat gtgagaatta ctggcttgta cccaacactc aacatctcag 16680
   atgagttttc tagcaatgtt gcaaattatc aaaaggtcgg catgcaaaag tactctacac 16740
   tccaaggacc acctggtact ggtaagagtc attttgccat cggacttgct ctctattacc 16800
   catctgctcg catagtgtat acggcatgct ctcatgcagc tgttgatgcc ctatgtgaaa 16860
   aggcattaaa atatttgccc atagataaat gtagtagaat catacctgcg cgtgcgcgcg 16920
   tagagtgttt tgataaattc aaagtgaatt caacactaga acagtatgtt ttctgcactg 16980
   taaatgcatt gccagaaaca actgctgaca ttgtagtctt tgatgaaatc tctatggcta 17040
   ctaattatga cttgagtgtt gtcaatgcta gacttcgtgc aaaacactac gtctatattg 17100
   gcgatcctgc tcaattacca gccccccgca cattgctgac taaaggcaca ctagaaccag 17160
   aatattttaa ttcagtgtgc agacttatga aaacaatagg tccagacatg ttccttggaa 17220
   cttgtcgccg ttgtcctgct gaaattgttg acactgtgag tgctttagtt tatgacaata 17280
   agctaaaagc acacaaggat aagtcagctc aatgcttcaa aatgttctac aaaggtgtta 17340
   ttacacatga tgtttcatct gcaatcaaca gacctcaaat aggcgttgta agagaatttc 17400
   ttacacgcaa tcctgcttgg agaaaagctg tttttatctc accttataat tcacagaacg 17460
   ctgtagcttc aaaaatctta ggattgccta cgcagactgt tgattcatca cagggttctg 17520
   aatatgacta tgtcatattc acacaaacta ctgaaacagc acactcttgt aatgtcaacc 17580
   gcttcaatgt ggctatcaca agggcaaaaa ttggcatttt gtgcataatg tctgatagag 17640
   atctttatga caaactgcaa tttacaagtc tagaaatacc acgtcgcaat gtggctacat 17700
   tacaagcaga aaatgtaact ggacttttta aggactgtag taagatcatt actggtcttc 17760
   atcctacaca ggcacctaca cacctcagcg ttgatataaa gttcaagact gaaggattat 17820
   gtgttgacat accaggcata ccaaaggaca tgacctaccg tagactcatc tctatgatgg 17880
   gtttcaaaat gaattaccaa gtcaatggtt accctaatat gtttatcacc cgcgaagaag 17940
   ctattcgtca cgttcgtgcg tggattggct ttgatgtaga gggctgtcat gcaactagag 18000
   atgctgtggg tactaaccta cctctccagc taggattttc tacaggtgtt aacttagtag 18060
   ctgtaccgac tggttatgtt gacactgaaa ataacacaga attcaccaga gttaatgcaa 18120
   aacctccacc aggtgaccag tttaaacatc ttataccact catgtataaa ggcttgccct 18180
   ggaatgtagt gcgtattaag atagtacaaa tgctcagtga tacactgaaa ggattgtcag 18240
   acagagtcgt gttcgtcctt tgggcgcatg gctttgagct tacatcaatg aagtactttg 18300
   tcaagattgg acctgaaaga acgtgttgtc tgtgtgacaa acgtgcaact tgcttttcta 18360
   cttcatcaga tacttatgcc tgctggaatc attctgtggg ttttgactat gtctataacc 18420
   catttatgat tgatgttcag cagtggggct ttacgggtaa ccttcagagt aaccatgacc 18480
   aacattgcca ggtacatgga aatgcacatg tggctagttg tgatgctatc atgactagat 18540
   gtttagcagt ccatgagtgc tttgttaagc gcgttgattg gtctgttgaa taccctatta 18600
   taggagatga actgagggtt aattctgctt gcagaaaagt acaacacatg gttgtgaagt 18660
   ctgcattgct tgctgataag tttccagttc ttcatgacat tggaaatcca aaggctatca 18720
   agtgtgtgcc tcaggctgaa gtagaatgga agttctacga tgctcagcca tgtagtgaca 18780
   aagcttacaa aatagaggaa ctcttctatt cttatgctac acatcacgat aaattcactg 18840
   atggtgtttg tttgttttgg aattgtaacg ttgatcgtta cccagccaat gcaattgtgt 18900
   gtaggtttga cacaagagtc ttgtcaaact tgaacttacc aggctgtgat ggtggtagtt 18960
   tgtatgtgaa taagcatgca ttccacactc cagctttcga taaaagtgca tttactaatt 19020
   taaagcaatt gcctttcttt tactattctg atagtccttg tgagtctcat ggcaaacaag 19080
   tagtgtcgga tattgattat gttccactca aatctgctac gtgtattaca cgatgcaatt 19140
   taggtggtgc tgtttgcaga caccatgcaa atgagtaccg acagtacttg gatgcatata 19200
   atatgatgat ttctgctgga tttagcctat ggatttacaa acaatttgat acttataacc 19260
   tgtggaatac atttaccagg ttacagagtt tagaaaatgt ggcttataat gttgttaata 19320
   aaggacactt tgatggacac gccggcgaag cacctgtttc catcattaat aatgctgttt 19380
   acacaaaggt agatggtatt gatgtggaga tctttgaaaa taagacaaca cttcctgtta 19440
   atgttgcatt tgagctttgg gctaagcgta acattaaacc agtgccagag attaagatac 19500
   tcaataattt gggtgttgat atcgctgcta atactgtaat ctgggactac aaaagagaag 19560
   ccccagcaca tgtatctaca ataggtgtct gcacaatgac tgacattgcc aagaaaccta 19620
   ctgagagtgc ttgttcttca cttactgtct tgtttgatgg tagagtggaa ggacaggtag 19680
   acctttttag aaacgcccgt aatggtgttt taataacaga aggttcagtc aaaggtctaa 19740
   caccttcaaa gggaccagca caagctagcg tcaatggagt cacattaatt ggagaatcag 19800
   taaaaacaca gtttaactac tttaagaaag tagacggcat tattcaacag ttgcctgaaa 19860
   cctactttac tcagagcaga gacttagagg attttaagcc cagatcacaa atggaaactg 19920
   actttctcga gctcgctatg gatgaattca tacagcgata taagctcgag ggctatgcct 19980
   tcgaacacat cgtttatgga gatttcagtc atggacaact tggcggtctt catttaatga 20040
   taggcttagc caagcgctca caagattcac cacttaaatt agaggatttt atccctatgg 20100
   acagcacagt gaaaaattac ttcataacag atgcgcaaac aggttcatca aaatgtgtgt 20160
   gttctgtgat tgatctttta cttgatgact ttgtcgagat aataaagtca caagatttgt 20220
   cagtgatttc aaaagtggtc aaggttacaa ttgactatgc tgaaatttca ttcatgcttt 20280
   ggtgtaagga tggacatgtt gaaaccttct acccaaaact acaagcaagt caagcgtggc 20340
   aaccaggtgt tgcgatgcct aacttgtaca agatgcaaag aatgcttctt gaaaagtgtg 20400
   accttcagaa ttatggtgaa aatgctgtta taccaaaagg aataatgatg aatgtcgcaa 20460
   agtatactca actgtgtcaa tacttaaata cacttacttt agctgtaccc tacaacatga 20520
   gagttattca ctttggtgct ggctctgata aaggagttgc accaggtaca gctgtgctca 20580
   gacaatggtt gccaactggc acactacttg tcgattcaga tcttaatgac ttcgtctccg 20640
   acgcagattc tactttaatt ggagactgtg caacagtaca tacggctaat aaatgggacc 20700
   ttattattag cgatatgtat gaccctagga ccaaacatgt gacaaaagag aatgactcta 20760
   aagaagggtt tttcacttat ctgtgtggat ttataaagca aaaactagcc ctgggtggtt 20820
   ctatagctgt aaagataaca gagcattctt ggaatgctga cctttacaag cttatgggcc 20880
   atttctcatg gtggacagct tttgttacaa atgtaaatgc atcatcatcg gaagcatttt 20940
   taattggggc taactatctt ggcaagccga aggaacaaat tgatggctat accatgcatg 21000
   ctaactacat tttctggagg aacacaaatc ctatccagtt gtcttcctat tcactctttg 21060
   acatgagcaa atttcctctt aaattaagag gaactgctgt aatgtctctt aaggagaatc 21120
   aaatcaatga tatgatttat tctcttctgg aaaaaggtag gcttatcatt agagaaaaca 21180
   acagagttgt ggtttcaagt gatattcttg ttaacaacta a 21221
<210> 32
   <211> 297
   <212> DNA
   <213> CORONAVIRUS
<400> 32
   atggacccca atcaaaccaa cgtagtgccc cccgcattac atttggtgga cccacagatt      60
   caactgacaa taaccagaat ggaggacgca atggggcaag gccaaaacag cgccgacccc     120
   aaggtttacc caataatact gcgtcttggt tcacagctct cactcagcat ggcaaggagg     180
   aacttagatt ccctcgaggc cagggcgttc caatcaacac caatagtggt ccagatgacc     240
   aaattggcta ctaccgaaga gctacccgac gagttcgtgg tggtgacggc aaaatga        297
<210> 33
   <211> 98
   <212> PRT
   <213> CORONAVIRUS
<400> 33
   Met Asp Pro Asn Gln Thr Asn Val Val Pro Pro Ala Leu His Leu Val
   1               5                   10                  15
   Asp Pro Gln Ile Gln Leu Thr Ile Thr Arg Met Glu Asp Ala Met Gly
               20                  25                  30
   Gln Gly Gln Asn Ser Ala Asp Pro Lys Val Tyr Pro Ile Ile Leu Arg
           35                  40                  45
   Leu Gly Ser Gln Leu Ser Leu Ser Met Ala Arg Arg Asn Leu Asp Ser
       50                  55                  60
   Leu Glu Ala Arg Ala Phe Gln Ser Thr Pro Ile Val Val Gln Met Thr
   65                  70                  75                  80
   Lys Leu Ala Thr Thr Glu Glu Leu Pro Asp Glu Phe Val Val Val Thr
                   85                  90                  95
   Ala Lys
<210> 34
   <211> 213
   <212> DNA
   <213> CORONAVIRUS
<400> 34
   atgctgccac cgtgctacaa cttcctcaag gaacaacatt gccaaaaggc ttctacgcag      60
   agggaagcag aggcggcagt caagcctctt ctcgctcctc atcacgtagt cgcggtaatt     120
   caagaaattc aactcctggc agcagtaggg gaaattctcc tgctcgaatg gctagcggag     180
   gtggtgaaac tgccctcgcg ctattgctgc tag                                  213
<210> 35
   <211> 70
   <212> PRT
   <213> CORONAVIRUS
<400> 35
   Met Leu Pro Pro Cys Tyr Asn Phe Leu Lys Glu Gln His Cys Gln Lys
   1               5                   10                  15
   Ala Ser Thr Gln Arg Glu Ala Glu Ala Ala Val Lys Pro Leu Leu Ala
               20                  25                  30
   Pro His His Val Val Ala Val Ile Gln Glu Ile Gln Leu Leu Ala Ala
           35                  40                  45
   Val Gly Glu Ile Leu Leu Leu Glu Trp Leu Ala Glu Val Val Lys Leu
       50                  55                  60
   Pro Ser Arg Tyr Cys Cys
   65                  70
<210> 36
   <211> 1377
   <212> DNA
   <213> CORONAVIRUS
<220>
   <221> CDS
   <222> (67)..(1335)
   <223>
<400> 36
   atgaaggtca ccaaactgct gcatttagag acgtacttgt tgttttaaat aaacgaacaa      60
   attaaa atg tct gat aat gga ccc caa tca aac caa cgt agt gcc ccc      108
          Met Ser Asp Asn Gly Pro Gln Ser Asn Gln Arg Ser Ala Pro
          1               5                   10
   cgc att aca ttt ggt gga ccc aca gat tca act gac aat aac cag aat     156
   Arg Ile Thr Phe Gly Gly Pro Thr Asp Ser Thr Asp Asn Asn Gln Asn
   15                  20                  25                  30
   gga gga cgc aat ggg gca agg cca aaa cag cgc cga ccc caa ggt tta     204
   Gly Gly Arg Asn Gly Ala Arg Pro Lys Gln Arg Arg Pro Gln Gly Leu
                   35                  40                  45
   ccc aat aat act gcg tct tgg ttc aca gct ctc act cag cat ggc aag     252
   Pro Asn Asn Thr Ala Ser Trp Phe Thr Ala Leu Thr Gln His Gly Lys
               50                  55                  60
   gag gaa ctt aga ttc cct cga ggc cag ggc gtt cca atc aac acc aat     300
   Glu Glu Leu Arg Phe Pro Arg Gly Gln Gly Val Pro Ile Asn Thr Asn
           65                  70                  75
   agt ggt cca gat gac caa att ggc tac tac cga aga gct acc cga cga     348
   Ser Gly Pro Asp Asp Gln Ile Gly Tyr Tyr Arg Arg Ala Thr Arg Arg
       80                  85                  90
   gtt cgt ggt ggt gac ggc aaa atg aaa gag ctc agc ccc aga tgg tac     396
   Val Arg Gly Gly Asp Gly Lys Met Lys Glu Leu Ser Pro Arg Trp Tyr
   95                  100                 105                 110
   ttc tat tac cta gga act ggc cca gaa gct tca ctt ccc tac ggc gct     444
   Phe Tyr Tyr Leu Gly Thr Gly Pro Glu Ala Ser Leu Pro Tyr Gly Ala
                   115                 120                 125
   aac aaa gaa ggc atc gta tgg gtt gca act gag gga gcc ttg aat aca     492
   Asn Lys Glu Gly Ile Val Trp Val Ala Thr Glu Gly Ala Leu Asn Thr
               130                 135                 140
   ccc aaa gac cac att ggc acc cgc aat cct aat aac aat gct gcc acc     540
   Pro Lys Asp His Ile Gly Thr Arg Asn Pro Asn Asn Asn Ala Ala Thr
           145                 150                 155
   gtg cta caa ctt cct caa gga aca aca ttg cca aaa ggc ttc tac gca     588
   Val Leu Gln Leu Pro Gln Gly Thr Thr Leu Pro Lys Gly Phe Tyr Ala
       160                 165                 170
   gag gga agc aga ggc ggc agt caa gcc tct tct cgc tcc tca tca cgt     636
   Glu Gly Ser Arg Gly Gly Ser Gln Ala Ser Ser Arg Ser Ser Ser Arg
   175                 180                 185                 190
   agt cgc ggt aat tca aga aat tca act cct ggc agc agt agg gga aat     684
   Ser Arg Gly Asn Ser Arg Asn Ser Thr Pro Gly Ser Ser Arg Gly Asn
                   195                 200                 205
   tct cct gct cga atg gct agc gga ggt ggt gaa act gcc ctc gcg cta     732
   Ser Pro Ala Arg Met Ala Ser Gly Gly Gly Glu Thr Ala Leu Ala Leu
               210                 215                 220
   ttg ctg cta gac aga ttg aac cag ctt gag agc aaa gtt tct ggt aaa     780
   Leu Leu Leu Asp Arg Leu Asn Gln Leu Glu Ser Lys Val Ser Gly Lys
           225                 230                 235
   ggc caa caa caa caa ggc caa act gtc act aag aaa tct gct gct gag     828
   Gly Gln Gln Gln Gln Gly Gln Thr Val Thr Lys Lys Ser Ala Ala Glu
       240                 245                 250
   gca tct aaa aag cct cgc caa aaa cgt act gcc aca aaa cag tac aac     876
   Ala Ser Lys Lys Pro Arg Gln Lys Arg Thr Ala Thr Lys Gln Tyr Asn
   255                 260                 265                 270
   gtc act caa gca ttt ggg aga cgt ggt cca gaa caa acc caa gga aat         924
   Val Thr Gln Ala Phe Gly Arg Arg Gly Pro Glu Gln Thr Gln Gly Asn
                   275                 280                 285
   ttc ggg gac caa gac cta atc aga caa gga act gat tac aaa cat tgg         972
   Phe Gly Asp Gln Asp Leu Ile Arg Gln Gly Thr Asp Tyr Lys His Trp
               290                 295                 300
   ccg caa att gca caa ttt gct cca agt gcc tct gca ttc ttt gga atg        1020
   Pro Gln Ile Ala Gln Phe Ala Pro Ser Ala Ser Ala Phe Phe Gly Met
           305                 310                 315
   tca cgc att ggc atg gaa gtc aca cct tcg gga aca tgg ctg act tat        1068
   Ser Arg Ile Gly Met Glu Val Thr Pro Ser Gly Thr Trp Leu Thr Tyr
       320                 325                 330
   cat gga gcc att aaa ttg gat gac aaa gat cca caa ttc aaa gac aac        1116
   His Gly Ala Ile Lys Leu Asp Asp Lys Asp Pro Gln Phe Lys Asp Asn
   335                 340                 345                 350
   gtc ata ctg ctg aac aag cac att gac gca tac aaa aca ttc cca cca        1164
   Val Ile Leu Leu Asn Lys His Ile Asp Ala Tyr Lys Thr Phe Pro Pro
                   355                 360                 365
   aca gag cct aaa aag gac aaa aag aaa aag act gat gaa gct cag cct        1212
   Thr Glu Pro Lys Lys Asp Lys Lys Lys Lys Thr Asp Glu Ala Gln Pro
               370                 375                 380
   ttg ccg cag aga caa aag aag cag ccc act gtg act ctt ctt cct gcg        1260
   Leu Pro Gln Arg Gln Lys Lys Gln Pro Thr Val Thr Leu Leu Pro Ala
           385                 390                 395
   gct gac atg gat gat ttc tcc aga caa ctt caa aat tcc atg agt gga        1308
   Ala Asp Met Asp Asp Phe Ser Arg Gln Leu Gln Asn Ser Met Ser Gly
       400                 405                 410
   gct tct gct gat tca act cag gca taa acactcatga tgaccacaca              1355
   Ala Ser Ala Asp Ser Thr Gln Ala
   415                 420
   aggcagatgg gctatgtaaa cg                                               1377
<210> 37
   <211> 422
   <212> PRT
   <213> CORONAVIRUS
<400> 37
   Met Ser Asp Asn Gly Pro Gln Ser Asn Gln Arg Ser Ala Pro Arg Ile
   1               5                   10                  15
   Thr Phe Gly Gly Pro Thr Asp Ser Thr Asp Asn Asn Gln Asn Gly Gly
               20                  25                  30
   Arg Asn Gly Ala Arg Pro Lys Gln Arg Arg Pro Gln Gly Leu Pro Asn
           35                  40                  45
   Asn Thr Ala Ser Trp Phe Thr Ala Leu Thr Gln His Gly Lys Glu Glu
   50 55 60
   Leu Arg Phe Pro Arg Gly Gln Gly Val Pro Ile Asn Thr Asn Ser Gly
   65 70 75 80
   Pro Asp Asp Gln Ile Gly Tyr Tyr Arg Arg Ala Thr Arg Arg Val Arg
   85 90 95
   Gly Gly Asp Gly Lys Met Lys Glu Leu Ser Pro Arg Trp Tyr Phe Tyr
   100 105 110
   Tyr Leu Gly Thr Gly Pro Glu Ala Ser Leu Pro Tyr Gly Ala Asn Lys
   115 120 125
   Glu Gly Ile Val Trp Val Ala Thr Glu Gly Ala Leu Asn Thr Pro Lys
   130 135 140
   Asp His Ile Gly Thr Arg Asn Pro Asn Asn Asn Ala Ala Thr Val Leu
   145 150 155 160
   Gln Leu Pro Gln Gly Thr Thr Leu Pro Lys Gly Phe Tyr Ala Glu Gly
   165 170 175
   Ser Arg Gly Gly Ser Gln Ala Ser Ser Arg Ser Ser Ser Arg Ser Arg
   180 185 190
   Gly Asn Ser Arg Asn Ser Thr Pro Gly Ser Ser Arg Gly Asn Ser Pro
   195 200 205
   Ala Arg Met Ala Ser Gly Gly Gly Glu Thr Ala Leu Ala Leu Leu Leu
   210 215 220
   Leu Asp Arg Leu Asn Gln Leu Glu Ser Lys Val Ser Gly Lys Gly Gln
   225 230 235 240
   Gln Gln Gln Gly Gln Thr Val Thr Lys Lys Ser Ala Ala Glu Ala Ser
   245 250 255
   Lys Lys Pro Arg Gln Lys Arg Thr Ala Thr Lys Gln Tyr Asn Val Thr
   260 265 270
   Gln Ala Phe Gly Arg Arg Gly Pro Glu Gln Thr Gln Gly Asn Phe Gly
   275 280 285
   Asp Gln Asp Leu Ile Arg Gln Gly Thr Asp Tyr Lys His Trp Pro Gln
   290 295 300
   Ile Ala Gln Phe Ala Pro Ser Ala Ser Ala Phe Phe Gly Met Ser Arg
   305 310 315 320
   Ile Gly Met Glu Val Thr Pro Ser Gly Thr Trp Leu Thr Tyr His Gly
   325 330 335
   Ala Ile Lys Leu Asp Asp Lys Asp Pro Gln Phe Lys Asp Asn Val Ile
   340 345 350
   Leu Leu Asn Lys His Ile Asp Ala Tyr Lys Thr Phe Pro Pro Thr Glu
   355 360 365
   Pro Lys Lys Asp Lys Lys Lys Lys Thr Asp Glu Ala Gln Pro Leu Pro
   370 375 380
   Gln Arg Gln Lys Lys Gln Pro Thr Val Thr Leu Leu Pro Ala Ala Asp
   385 390 395 400
   Met Asp Asp Phe Ser Arg Gln Leu Gln Asn Ser Met Ser Gly Ala Ser
   405 410 415
   Ala Asp Ser Thr Gln Ala
   420
<210> 38
   <211> 1377
   <212> DNA
   <213> CORONAVIRUS
<400> 38
   atgaaggtca ccaaactgct gcatttagag acgtacttgt tgttttaaat aaacgaacaa 60
   attaaaatgt ctgataatgg accccaatca aaccaacgta gtgccccccg cattacattt 120
   ggtggaccca cagattcaac tgacaataac cagaatggag gacgcaatgg ggcaaggcca 180
   aaacagcgcc gaccccaagg tttacccaat aatactgcgt cttggttcac agctctcact 240
   cagcatggca aggaggaact tagattccct cgaggccagg gcgttccaat caacaccaat 300
   agtggtccag atgaccaaat tggctactac cgaagagcta cccgacgagt tcgtggtggt 360
   gacggcaaaa tgaaagagct cagccccaga tggtacttct attacctagg aactggccca 420
   gaagcttcac ttccctacgg cgctaacaaa gaaggcatcg tatgggttgc aactgaggga 480
   gccttgaata cacccaaaga ccacattggc acccgcaatc ctaataacaa tgctgccacc 540
   gtgctacaac ttcctcaagg aacaacattg ccaaaaggct tctacgcaga gggaagcaga 600
   ggcggcagtc aagcctcttc tcgctcctca tcacgtagtc gcggtaattc aagaaattca 660
   actcctggca gcagtagggg aaattctcct gctcgaatgg ctagcggagg tggtgaaact 720
   gccctcgcgc tattgctgct agacagattg aaccagcttg agagcaaagt ttctggtaaa 780
   ggccaacaac aacaaggcca aactgtcact aagaaatctg ctgctgaggc atctaaaaag 840
   cctcgccaaa aacgtactgc cacaaaacag tacaacgtca ctcaagcatt tgggagacgt 900
   ggtccagaac aaacccaagg aaatttcggg gaccaagacc taatcagaca aggaactgat 960
   tacaaacatt ggccgcaaat tgcacaattt gctccaagtg cctctgcatt ctttggaatg 1020
   tcacgcattg gcatggaagt cacaccttcg ggaacatggc tgacttatca tggagccatt 1080
   aaattggatg acaaagatcc acaattcaaa gacaacgtca tactgctgaa caagcacatt 1140
   gacgcataca aaacattccc accaacagag cctaaaaagg acaaaaagaa aaagactgat 1200
   gaagctcagc ctttgccgca gagacaaaag aagcagccca ctgtgactct tcttcctgcg 1260
   gctgacatgg atgatttctc cagacaactt caaaattcca tgagtggagc ttctgctgat 1320
   tcaactcagg cataaacact catgatgacc acacaaggca gatgggctat gtaaacg 1377
<210> 39
   <211> 204
   <212> DNA
   <213> CORONAVIRUS
<400> 39
   atattaggtt tttacctacc caggaaaagc caaccaacct cgatctcttg tagatctgtt 60
   ctctaaacga actttaaaat ctgtgtagct gtcgctcggc tgcatgccta gtgcacctac 120
   gcagtataaa caataataaa ttttactgtc gttgacaaga aacgagtaac tcgtccctct 180
   tctgcagact gcttacggtt tcgt 204
<210> 40
   <211> 809
   <212> DNA
   <213> CORONAVIRUS
<400> 40
   actcaagcat ttgggagacg tggtccagaa caaacccaag gaaatttcgg ggaccaagac 60
   ctaatcagac aaggaactga ttacaaacat tggccgcaaa ttgcacaatt tgctccaagt 120
   gcctctgcat tctttggaat gtcacgcatt ggcatggaag tcacaccttc gggaacatgg 180
   ctgacttatc atggagccat taaattggat gacaaagatc cacaattcaa agacaacgtc 240
   atactgctga acaagcacat tgacgcatac aaaacattcc caccaacaga gcctaaaaag 300
   gacaaaaaga aaaagactga tgaagctcag cctttgccgc agagacaaaa gaagcagccc 360
   actgtgactc ttcttcctgc ggctgacatg gatgatttct ccagacaact tcaaaattcc 420
   atgagtggag cttctgctga ttcaactcag gcataaacac tcatgatgac cacacaaggc 480
   agatgggcta tgtaaacgtt ttcgcaattc cgtttacgat acatagtcta ctcttgtgca 540
   gaatgaattc tcgtaactaa acagcacaag taggtttagt taactttaat ctcacatagc 600
   aatctttaat caatgtgtaa cattagggag gacttgaaag agccaccaca ttttcatcga 660
   ggccacgcgg agtacgatcg agggtacagt gaataatgct agggagagct gcctatatgg 720
   aagagcccta atgtgtaaaa ttaattttag tagtgctatc cccatgtgat tttaatagct 780
   tcttaggaga atgacaaaaa aaaaaaaaa 809
<210> 41
   <211> 448
   <212> DNA
   <213> CORONAVIRUS
<400> 41
   aatgaacaca tagggctgtt caagctgggg cagtacgcct ttttccagct ctactagacc 60
   acaagtgcca tttttgaggt gttcacgtgc ctccgatagg gcctcttcca cagagtcccc 120
   gaagccacgc actagcacgt ctctaacctg aaggacaggc aaactgagtt ggacgtgtgt 180
   tttctcgttg acaccaagaa caaggctctc catcttacct ttcggtcaca cccggacgaa 240
   acctaggtat gctgatgatc gactgcaaca cggacgaaac cgtaagcagt ctgcagaaga 300
   gggacgagtt actcgtttct tgtcaacgac agtaaaattt attattgttt atactgcgta 360
   ggtgcactag gcatgcagcc gagcgacagc tacacagatt ttaaagttcg tttagagaac 420
   agatctacaa gagatcgagg ttggttgg 448
<210> 42
   <211> 2033
   <212> DNA
   <213> CORONAVIRUS
<400> 42
   atacctaggt ttcgtccggg tgtgaccgaa aggtaagatg gagagccttg ttcttggtgt 60
   caacgagaaa acacacgtcc aactcagttt gcctgtcctt caggttagag acgtgctagt 120
   gcgtggcttc ggggactctg tggaagaggc cctatcggag gcacgtgaac acctcaaaaa 180
   tggcacttgt ggtctagtag agctggaaaa aggcgtactg ccccagcttg aacagcccta 240
   tgtgttcatt aaacgttctg atgccttaag caccaatcac ggccacaagg tcgttgagct 300
   ggttgcagaa atggacggca ttcagtacgg tcgtagcggt ataacactgg gagtactcgt 360
   gccacatgtg ggcgaaaccc caattgcata ccgcaatgtt cttcttcgta agaacggtaa 420
   taagggagcc ggtggtcata gctatggcat cgatctaaag tcttatgact taggtgacga 480
   gcttggcact gatcccattg aagattatga acaaaactgg aacactaagc atggcagtgg 540
   tgcactccgt gaactcactc gtgagctcaa tggaggtgca gtcactcgct atgtcgacaa 600
   caatttctgt ggcccagatg ggtaccctct tgattgcatc aaagattttc tcgcacgcgc 660
   gggcaagtca atgtgcactc tttccgaaca acttgattac atcgagtcga agagaggtgt 720
   ctactgctgc cgtgaccatg agcatgaaat tgcctggttc actgagcgct ctgataagag 780
   ctacgagcac cagacaccct tcgaaattaa gagtgccaag aaatttgaca ctttcaaagg 840
   ggaatgccca aagtttgtgt ttcctcttaa ctcaaaagtc aaagtcattc aaccacgtgt 900
   tgaaaagaaa aagactgagg gtttcatggg gcgtatacgc tctgtgtacc ctgttgcatc 960
   tccacaggag tgtaacaata tgcacttgtc taccttgatg aaatgtaatc attgcgatga 1020
   agtttcatgg cagacgtgcg actttctgaa agccacttgt gaacattgtg gcactgaaaa 1080
   tttagttatt gaaggaccta ctacatgtgg gtacctacct actaatgctg tagtgaaaat 1140
   gccatgtcct gcctgtcaag acccagagat tggacctgag catagtgttg cagattatca 1200
   caaccactca aacattgaaa ctcgactccg caagggaggt aggactagat gttttggagg 1260
   ctgtgtgttt gcctatgttg gctgctataa taagcgtgcc tactgggttc ctcgtgctag 1320
   tgctgatatt ggctcaggcc atactggcat tactggtgac aatgtggaga ccttgaatga 1380
   ggatctcctt gagatactga gtcgtgaacg tgttaacatt aacattgttg gcgattttca 1440
   tttgaatgaa gaggttgcca tcattttggc atctttctct gcttctacaa gtgcctttat 1500
   tgacactata aagagtcttg attacaagtc tttcaaaacc attgttgagt cctgcggtaa 1560
   ctataaagtt accaagggaa agcccgtaaa aggtgcttgg aacattggac aacagagatc 1620
   agttttaaca ccactgtgtg gttttccctc acaggctgct ggtgttatca gatcaatttt 1680
   tgcgcgcaca cttgatgcag caaaccactc aattcctgat ttgcaaagag cagctgtcac 1740
   catacttgat ggtatttctg aacagtcatt acgtcttgtc gacgccatgg tttatacttc 1800
   agacctgctc accaacagtg tcattattat ggcatatgta actggtggtc ttgtacaaca 1860
   gacttctcag tggttgtcta atcttttggg cactactgtt gaaaaactca ggcctatctt 1920
   tgaatggatt gaggcgaaac ttagtgcagg agttgaattt ctcaaggatg cttgggagat 1980
   tctcaaattt ctcattacag gtgtttttga catcgtcaag ggtcaaatac agg 2033
<210> 43
   <211> 2018
   <212> DNA
   <213> CORONAVIRUS
<400> 43
   ggattgaggc gaaacttagt gcaggagttg aatttctcaa ggatgcttgg gagattctca 60
   aatttctcat tacaggtgtt tttgacatcg tcaagggtca aatacaggtt gcttcagata 120
   acatcaagga ttgtgtaaaa tgcttcattg atgttgttaa caaggcactc gaaatgtgca 180
   ttgatcaagt cactatcgct ggcgcaaagt tgcgatcact caacttaggt gaagtcttca 240
   tcgctcaaag caagggactt taccgtcagt gtatacgtgg caaggagcag ctgcaactac 300
   tcatgcctct taaggcacca aaagaagtaa cctttcttga aggtgattca catgacacag 360
   tacttacctc tgaggaggtt gttctcaaga acggtgaact cgaagcactc gagacgcccg 420
   ttgatagctt cacaaatgga gctatcgttg gcacaccagt ctgtgtaaat ggcctcatgc 480
   tcttagagat taaggacaaa gaacaatact gcgcattgtc tcctggttta ctggctacaa 540
   acaatgtctt tcgcttaaaa gggggtgcac caattaaagg tgtaaccttt ggagaagata 600
   ctgtttggga agttcaaggt tacaagaatg tgagaatcac atttgagctt gatgaacgtg 660
   ttgacaaagt gcttaatgaa aagtgctctg tctacactgt tgaatccggt accgaagtta 720
   ctgagtttgc atgtgttgta gcagaggctg ttgtgaagac tttacaacca gtttctgatc 780
   tccttaccaa catgggtatt gatcttgatg agtggagtgt agctacattc tacttatttg 840
   atgatgctgg tgaagaaaac ttttcatcac gtatgtattg ttccttttac cctccagatg 900
   aggaagaaga ggacgatgca gagtgtgagg aagaagaaat tgatgaaacc tgtgaacatg 960
   agtacggtac agaggatgat tatcaaggtc tccctctgga atttggtgcc tcagctgaaa 1020
   cagttcgagt tgaggaagaa gaagaggaag actggctgga tgatactact gagcaatcag 1080
   agattgagcc agaaccagaa cctacacctg aagaaccagt taatcagttt actggttatt 1140
   taaaacttac tgacaatgtt gccattaaat gtgttgacat cgttaaggag gcacaaagtg 1200
   ctaatcctat ggtgattgta aatgctgcta acatacacct gaaacatggt ggtggtgtag 1260
   caggtgcact caacaaggca accaatggtg ccatgcaaaa ggagagtgat gattacatta 1320
   agctaaatgg ccctcttaca gtaggagggt cttgtttgct ttctggacat aatcttgcta 1380
   agaagtgtct gcatgttgtt ggacctaacc taaatgcagg tgaggacatc cagcttctta 1440
   aggcagcata tgaaaatttc aattcacagg acatcttact tgcaccattg ttgtcagcag 1500
   gcatatttgg tgctaaacca cttcagtctt tacaagtgtg cgtgcagacg gttcgtacac 1560
   aggtttatat tgcagtcaat gacaaagctc tttatgagca ggttgtcatg gattatcttg 1620
   ataacctgaa gcctagagtg gaagcaccta aacaagagga gccaccaaac acagaagatt 1680
   ccaaaactga ggagaaatct gtcgtacaga agcctgtcga tgtgaagcca aaaattaagg 1740
   cctgcattga tgaggttacc acaacactgg aagaaactaa gtttcttacc aataagttac 1800
   tcttgtttgc tgatatcaat ggtaagcttt accatgattc tcagaacatg cttagaggtg 1860
   aagatatgtc tttccttgag aaggatgcac cttacatggt aggtgatgtt atcactagtg 1920
   gtgatatcac ttgtgttgta ataccctcca aaaaggctgg tggcactact gagatgctct 1980
   caagagcttt gaagaaagtg ccagttgatg agtatata 2018
<210> 44
   <211> 1442
   <212> DNA
   <213> CORONAVIRUS
<400> 44
   ttgatgaggt taccacaaca ctggaagaaa ctaagtttct taccaataag ttactcttgt 60
   ttgctgatat caatggtaag ctttaccatg attctcagaa catgcttaga ggtgaagata 120
   tgtctttcct tgagaaggat gcaccttaca tggtaggtga tgttatcact agtggtgata 180
   tcacttgtgt tgtaataccc tccaaaaagg ctggtggcac tactgagatg ctctcaagag 240
   ctttgaagaa agtgccagtt gatgagtata taaccacgta ccctggacaa ggatgtgctg 300
   gttatacact tgaggaagct aagactgctc ttaagaaatg caaatctgca ttttatgtac 360
   taccttcaga agcacctaat gctaaggaag agattctagg aactgtatcc tggaatttga 420
   gagaaatgct tgctcatgct gaagagacaa gaaaattaat gcctatatgc atggatgtta 480
   gagccataat ggcaaccatc caacgtaagt ataaaggaat taaaattcaa gagggcatcg 540
   ttgactatgg tgtccgattc ttcttttata ctagtaaaga gcctgtagct tctattatta 600
   cgaagctgaa ctctctaaat gagccgcttg tcacaatgcc aattggttat gtgacacatg 660
   gttttaatct tgaagaggct gcgcgctgta tgcgttctct taaagctcct gccgtagtgt 720
   cagtatcatc accagatgct gttactacat ataatggata cctcacttcg tcatcaaaga 780
   catctgagga gcactttgta gaaacagttt ctttggctgg ctcttacaga gattggtcct 840
   attcaggaca gcgtacagag ttaggtgttg aatttcttaa gcgtggtgac aaaattgtgt 900
   accacactct ggagagcccc gtcgagtttc atcttgacgg tgaggttctt tcacttgaca 960
   aactaaagag tctcttatcc ctgcgggagg ttaagactat aaaagtgttc acaactgtgg 1020
   acaacactaa tctccacaca cagcttgtgg atatgtctat gacatatgga cagcagtttg 1080
   gtccaacata cttggatggt gctgatgtta caaaaattaa acctcatgta aatcatgagg 1140
   gtaagacttt ctttgtacta cctagtgatg acacactacg tagtgaagct ttcgagtact 1200
   accatactct tgatgagagt tttcttggta ggtacatgtc tgctttaaac cacacaaaga 1260
   aatggaaatt tcctcaagtt ggtggtttaa cttcaattaa atgggctgat aacaattgtt 1320
   atttgtctag tgttttatta gcacttcaac agcttgaagt caaattcaat gcaccagcac 1380
   ttcaagaggc ttattataga gcccgtgctg gtgatgctgc taacttttgt gcactcatac 1440
   tc 1442
<210> 45
   <211> 1050
   <212> DNA
   <213> CORONAVIRUS
<400> 45
   atatgtctat gacatatgga cagcagtttg gtccaacata cttggatggt gctgatgtta 60
   caaaaattaa acctcatgta aatcatgagg gtaagacttt ctttgtacta cctagtgatg 120
   acacactacg tagtgaagct ttcgagtact accatactct tgatgagagt tttcttggta 180
   ggtacatgtc tgctttaaac cacacaaaga aatggaaatt tcctcaagtt ggtggtttaa 240
   cttcaattaa atgggctgat aacaattgtt atttgtctag tgttttatta gcacttcaac 300
   agcttgaagt caaattcaat gcaccagcac ttcaagaggc ttattataga gcccgtgctg 360
   gtgatgctgc taacttttgt gcactcatac tcgcttacag taataaaact gttggcgagc 420
   ttggtgatgt cagagaaact atgacccatc ttctacagca tgctaatttg gaatctgcaa 480
   agcgagttct taatgtggtg tgtaaacatt gtggtcagaa aactactacc ttaacgggtg 540
   tagaagctgt gatgtatatg ggtactctat cttatgataa tcttaagaca ggtgtttcca 600
   ttccatgtgt gtgtggtcgt gatgctacac aatatctagt acaacaagag tcttcttttg 660
   ttatgatgtc tgcaccacct gctgagtata aattacagca aggtacattc ttatgtgcga 720
   atgagtacac tggtaactat cagtgtggtc attacactca tataactgct aaggagaccc 780
   tctatcgtat tgacggagct caccttacaa agatgtcaga gtacaaagga ccagtgactg 840
   atgttttcta caaggaaaca tcttacacta caaccatcaa gcctgtgtcg tataaactcg 900
   atggagttac ttacacagag attgaaccaa aattggatgg gtattataaa aaggataatg 960
   cttactatac agagcagcct atagaccttg taccaactca accattacca aatgcgagtt 1020
   ttgataattt caaactcaca tgttctaaca 1050
<210> 46
   <211> 1995
   <212> DNA
   <213> CORONAVIRUS
<400> 46
   tttgtgcact catactcgct tacagtaata aaactgttgg cgagcttggt gatgtcagag 60
   aaactatgac ccatcttcta cagcatgcta atttggaatc tgcaaagcga gttcttaatg 120
   tggtgtgtaa acattgtggt cagaaaacta ctaccttaac gggtgtagaa gctgtgatgt 180
   atatgggtac tctatcttat gataatctta agacaggtgt ttccattcca tgtgtgtgtg 240
   gtcgtgatgc tacacaatat ctagtacaac aagagtcttc ttttgttatg atgtctgcac 300
   cacctgctga gtataaatta cagcaaggta cattcttatg tgcgaatgag tacactggta 360
   actatcagtg tggtcattac actcatataa ctgctaagga gaccctctat cgtattgacg 420
   gagctcacct tacaaagatg tcagagtaca aaggaccagt gactgatgtt ttctacaagg 480
   aaacatctta cactacaacc atcaagcctg tgtcgtataa actcgatgga gttacttaca 540
   cagagattga accaaaattg gatgggtatt ataaaaagga taatgcttac tatacagagc 600
   agcctataga ccttgtacca actcaaccat taccaaatgc gagttttgat aatttcaaac 660
   tcacatgttc taacacaaaa tttgctgatg atttaaatca aatgacaggc ttcacaaagc      720
   cagcttcacg agagctatct gtcacattct tcccagactt gaatggcgat gtagtggcta      780
   ttgactatag acactattca gcgagtttca agaaaggtgc taaattactg cataagccaa      840
   ttgtttggca cattaaccag gctacaacca agacaacgtt caaaccaaac acttggtgtt      900
   tacgttgtct ttggagtaca aagccagtag atacttcaaa ttcatttgaa gttctggcag      960
   tagaagacac acaaggaatg gacaatcttg cttgtgaaag tcaacaaccc acctctgaag     1020
   aagtagtgga aaatcctacc atacagaagg aagtcataga gtgtgacgtg aaaactaccg     1080
   aagttgtagg caatgtcata cttaaaccat cagatgaagg tgttaaagta acacaagagt     1140
   taggtcatga ggatcttatg gctgcttatg tggaaaacac aagcattacc attaagaaac     1200
   ctaatgagct ttcactagcc ttaggtttaa aaacaattgc cactcatggt attgctgcaa     1260
   ttaatagtgt tccttggagt aaaattttgg cttatgtcaa accattctta ggacaagcag     1320
   caattacaac atcaaattgc gctaagagat tagcacaacg tgtgtttaac aattatatgc     1380
   cttatgtgtt tacattattg ttccaattgt gtacttttac taaaagtacc aattctagaa     1440
   ttagagcttc actacctaca actattgcta aaaatagtgt taagagtgtt gctaaattat     1500
   gtttggatgc cggcattaat tatgtgaagt cacccaaatt ttctaaattg ttcacaatcg     1560
   ctatgtggct attgttgtta agtatttgct taggttctct aatctgtgta actgctgctt     1620
   ttggtgtact cttatctaat tttggtgctc cttcttattg taatggcgtt agagaattgt     1680
   atcttaattc gtctaacgtt actactatgg atttctgtga aggttctttt ccttgcagca     1740
   tttgtttaag tggattagac tcccttgatt cttatccagc tcttgaaacc attcaggtga     1800
   cgatttcatc gtacaagcta gacttgacaa ttttaggtct ggccgctgag tgggttttgg     1860
   catatatgtt gttcacaaaa ttcttttatt tattaggtct ttcagctata atgcaggtgt     1920
   tctttggcta ttttgctagt catttcatca gcaattcttg gctcatgtgg tttatcatta     1980
   gtattgtaca aatgg                                                      1995
<210> 47
   <211> 1884
   <212> DNA
   <213> CORONAVIRUS
<400> 47
   aattcttggc tcatgtggtt tatcattagt attgtacaaa tggcacccgt ttctgcaatg       60
   gttaggatgt acatcttctt tgcttctttc tactacatat ggaagagcta tgttcatatc      120
   atggatggtt gcacctcttc gacttgcatg atgtgctata agcgcaatcg tgccacacgc      180
   gttgagtgta caactattgt taatggcatg aagagatctt tctatgtcta tgcaaatgga      240
   ggccgtggct tctgcaagac tcacaattgg aattgtctca attgtgacac attttgcact      300
   ggtagtacat tcattagtga tgaagttgct cgtgatttgt cactccagtt taaaagacca 360
   atcaacccta ctgaccagtc atcgtatatt gttgatagtg ttgctgtgaa aaatggcgcg 420
   cttcacctct actttgacaa ggctggtcaa aagacctatg agagacatcc gctctcccat 480
   tttgtcaatt tagacaattt gagagctaac aacactaaag gttcactgcc tattaatgtc 540
   atagtttttg atggcaagtc caaatgcgac gagtctgctt ctaagtctgc ttctgtgtac 600
   tacagtcagc tgatgtgcca acctattctg ttgcttgacc aagctcttgt atcagacgtt 660
   ggagatagta ctgaagtttc cgttaagatg tttgatgctt atgtcgacac cttttcagca 720
   acttttagtg ttcctatgga aaaacttaag gcacttgttg ctacagctca cagcgagtta 780
   gcaaagggtg tagctttaga tggtgtcctt tctacattcg tgtcagctgc ccgacaaggt 840
   gttgttgata ccgatgttga cacaaaggat gttattgaat gtctcaaact ttcacatcac 900
   tctgacttag aagtgacagg tgacagttgt aacaatttca tgctcaccta taataaggtt 960
   gaaaacatga cgcccagaga tcttggcgca tgtattgact gtaatgcaag gcatatcaat 1020
   gcccaagtag caaaaagtca caatgtttca ctcatctgga atgtaaaaga ctacatgtct 1080
   ttatctgaac agctgcgtaa acaaattcgt agtgctgcca agaagaacaa catacctttt 1140
   agactaactt gtgctacaac tagacaggtt gtcaatgtca taactactaa aatctcactc 1200
   aagggtggta agattgttag tacttgtttt aaacttatgc ttaaggccac attattgtgc 1260
   gttcttgctg cattggtttg ttatatcgtt atgccagtac atacattgtc aatccatgat 1320
   ggttacacaa atgaaatcat tggttacaaa gccattcagg atggtgtcac tcgtgacatc 1380
   atttctactg atgattgttt tgcaaataaa catgctggtt ttgacgcatg gtttagccag 1440
   cgtggtggtt catacaaaaa tgacaaaagc tgccctgtag tagctgctat cattacaaga 1500
   gagattggtt tcatagtgcc tggcttaccg ggtactgtgc tgagagcaat caatggtgac 1560
   ttcttgcatt ttctacctcg tgtttttagt gctgttggca acatttgcta cacaccttcc 1620
   aaactcattg agtatagtga ttttgctacc tctgcttgcg ttcttgctgc tgagtgtaca 1680
   atttttaagg atgctatggg caaacctgtg ccatattgtt atgacactaa tttgctagag 1740
   ggttctattt cttatagtga gcttcgtcca gacactcgtt atgtgcttat ggatggttcc 1800
   atcatacagt ttcctaacac ttacctggag ggttctgtta gagtagtaac aacttttgat 1860
   gctgagtact gtagacatgg taca 1884
<210> 48
   <211> 2020
   <212> DNA
   <213> CORONAVIRUS
<400> 48
   cactcgttat gtgcttatgg atggttccat catacagttt cctaacactt acctggaggg 60
   ttctgttaga gtagtaacaa cttttgatgc tgagtactgt agacatggta catgcgaaag 120
   gtcagaagta ggtatttgcc tatctaccag tggtagatgg gttcttaata atgagcatta 180
   cagagctcta tcaggagttt tctgtggtgt tgatgcgatg aatctcatag ctaacatctt 240
   tactcctctt gtgcaacctg tgggtgcttt agatgtgtct gcttcagtag tggctggtgg 300
   tattattgcc atattggtga cttgtgctgc ctactacttt atgaaattca gacgtgtttt 360
   tggtgagtac aaccatgttg ttgctgctaa tgcacttttg tttttgatgt ctttcactat 420
   actctgtctg gtaccagctt acagctttct gccgggagtc tactcagtct tttacttgta 480
   cttgacattc tatttcacca atgatgtttc attcttggct caccttcaat ggtttgccat 540
   gttttctcct attgtgcctt tttggataac agcaatctat gtattctgta tttctctgaa 600
   gcactgccat tggttcttta acaactatct taggaaaaga gtcatgttta atggagttac 660
   atttagtacc ttcgaggagg ctgctttgtg tacctttttg ctcaacaagg aaatgtacct 720
   aaaattgcgt agcgagacac tgttgccact tacacagtat aacaggtatc ttgctctata 780
   taacaagtac aagtatttca gtggagcctt agatactacc agctatcgtg aagcagcttg 840
   ctgccactta gcaaaggctc taaatgactt tagcaactca ggtgctgatg ttctctacca 900
   accaccacag acatcaatca cttctgctgt tctgcagagt ggttttagga aaatggcatt 960
   cccgtcaggc aaagttgaag ggtgcatggt acaagtaacc tgtggaacta caactcttaa 1020
   tggattgtgg ttggatgaca cagtatactg tccaagacat gtcatttgca cagcagaaga 1080
   catgcttaat cctaactatg aagatctgct cattcgcaaa tccaaccata gctttcttgt 1140
   tcaggctggc aatgttcaac ttcgtgttat tggccattct atgcaaaatt gtctgcttag 1200
   gcttaaagtt gatacttcta accctaagac acccaagtat aaatttgtcc gtatccaacc 1260
   tggtcaaaca ttttcagttc tagcatgcta caatggttca ccatctggtg tttatcagtg 1320
   tgccatgaga cctaatcata ccattaaagg ttctttcctt aatggatcat gtggtagtgt 1380
   tggttttaac attgattatg attgcgtgtc tttctgctat atgcatcata tggagcttcc 1440
   aacaggagta cacgctggta ctgacttaga aggtaaattc tatggtccat ttgttgacag 1500
   acaaactgca caggctgcag gtacagacac aaccataaca ttaaatgttt tggcatggct 1560
   gtatgctgct gttatcaatg gtgataggtg gtttcttaat agattcacca ctactttgaa 1620
   tgactttaac cttgtggcaa tgaagtacaa ctatgaacct ttgacacaag atcatgttga 1680
   catattggga cctctttctg ctcaaacagg aattgccgtc ttagatatgt gtgctgcttt 1740
   gaaagagctg ctgcagaatg gtatgaatgg tcgtactatc cttggtagca ctattttaga 1800
   agatgagttt acaccatttg atgttgttag acaatgctct ggtgttacct tccaaggtaa 1860
   gttcaagaaa attgttaagg gcactcatca ttggatgctt ttaactttct tgacatcact 1920
   attgattctt gttcaaagta cacagtggtc actgtttttc tttgtttacg agaatgcttt 1980
   cttgccattt actcttggta ttatggcaat tgctgcatgt 2020
<210> 49
   <211> 2040
   <212> DNA
   <213> CORONAVIRUS
<400> 49
   agcatttcca gcctgaagac gtactgtagc agctaaactg cccagcacca tacctctatt      60
   taggttgttt aagcctttga tgaagtacaa gtatttcact ttaggccctt ttggtgtgtc    120
   tgtaacaaac ctacaaggtg gttccagttc tgtgtaaatt gtacctgtac catcactctt    180
   agggaatcta gcccatttga gatcttggtg gtctgatagt aatgccagca caaacctacc    240
   tcccttcgaa ttgttatagt aggcaagtgc attgtcatca gtacaagctg tttgtgtggt    300
   accagccgca caggacatct gtcgtagtgc tactggactc agttcattat tctgtagttt    360
   aacagctgag ttggctctta gagctgtaac aataagaggc caagccaaat ttggtgaatt    420
   gtccatgtta atttcactaa gttgaacaat cttgctatcc gcatcaacaa cttgctggat    480
   ttcccagagt gcagatgcat atgtaaaggt gttaccatca caagtgttct tgtaggtacc    540
   ataatcaggg acaacaacca tgagtttggc tgctgtagtc aatggtatga tgttgagtgg    600
   aacacaacca tcacgcgcat tgttgataat gttgttaagt gcatcattat caagcttcct    660
   aagcatagtg aagagcattg tttgcatagc actagttact tttgccctct tgtcctcaga    720
   tcttgcctgt ttgtacattt gggtcatagc ctgatctgcc atcttttcca acttgcgttg    780
   catggcagca tcacggtcaa actcagattt agccacattc aaagatttct ttaacttttt    840
   gagaacgact tcagaatcac cattagctac agcctgctca taggcctcct gggcagtggc    900
   ataagcggca tatgatggta aagaactaaa ttctgaagca atagcctgaa gagtagcacg    960
   gttatcgagc atttcctcgc acaacctatt aatgtctaca gcaccctgca tggatagcaa   1020
   aacagacaaa agagaaacca tcttctcgaa agcttcagtt gtgtcttttg caagaagaat   1080
   atcattgtgg agttgtacac attgtgccca caatttagaa gatgactcta ctctaagttg   1140
   ttgaagaacc gagagcagta ccacagatgt gcactttacg tcagacattt tagactgtac   1200
   agtagcaacc ttgatacatg gtttacctcc aatacccaac aacttaatgt taagcttgaa   1260
   agcatcaata ctactcttag gaggcaaaag cccctgggag ttcatatacc taaattcttg   1320
   tgtagagacc aagtagtcat aaacaccaag agtaagcctg aagtaacggt tgagtaaaca   1380
   gaaaaggcca aagtagcagc agcaacaata gcctaagaaa caataaacaa gcatgataca   1440
   ctgtaaggtg ttgccagtaa taaataacaa tgggtaatac tcaacacaca caaacactat   1500
   agctctagct aaaaacatga tagtcgtaac gacaccagaa tagttagagg ttacagaaat   1560
   aactaaggcc cacatggaaa tagcttgatc taaagcatta ccatagtaga ctttgtaaac   1620
   aagtgtaatg acattcatca gtgtccaaac acgtctagca gcatcatcat aaacagtgcg   1680
   agctgtcatg agaataagca aaactaaagc tgaagcatac ataacacaat ccttaagcct   1740
   ataaccagac aagctagtgt cagccaattc aagccatgtc atgatacgca tcacccagct 1800
   agcaggcatg tagaccatat taaagtaagc aactgttgca agagaaggta acagaaacaa 1860
   gcacaagaat gcgtgcttat gcttaacaag cagcatagca catgcagcaa ttgccataat 1920
   accaagagta aatggcaaga aagcattctc gtaaacaaag aaaaacagtg accactgtgt 1980
   actttgaaca agaatcaata gtgatgtcaa gaaagttaaa agcatccaat gatgagtgca 2040
<210> 50
   <211> 2012
   <212> DNA
   <213> CORONAVIRUS
<400> 50
   cttgtaggtt tgttacagac acaccaaaag ggcctaaagt gaaatacttg tacttcatca 60
   aaggcttaaa caacctaaat agaggtatgg tgctgggcag tttagctgct acagtacgtc 120
   ttcaggctgg aaatgctaca gaagtacctg ccaattcaac tgtgctttcc ttctgtgctt 180
   ttgcagtaga ccctgctaaa gcatataagg attacctagc aagtggagga caaccaatca 240
   ccaactgtgt gaagatgttg tgtacacaca ctggtacagg acaggcaatt actgtaacac 300
   cagaagctaa catggaccaa gagtcctttg gtggtgcttc atgttgtctg tattgtagat 360
   gccacattga ccatccaaat cctaaaggat tctgtgactt gaaaggtaag tacgtccaaa 420
   tacctaccac ttgtgctaat gacccagtgg gttttacact tagaaacaca gtctgtaccg 480
   tctgcggaat gtggaaaggt tatggctgta gttgtgacca actccgcgaa cccttgatgc 540
   agtctgcgga tgcatcaacg tttttaaacg ggtttgcggt gtaagtgcag cccgtcttac 600
   accgtgcggc acaggcacta gtactgatgt cgtctacagg gcttttgata tttacaacga 660
   aaaagttgct ggttttgcaa agttcctaaa aactaattgc tgtcgcttcc aggagaagga 720
   tgaggaaggc aatttattag actcttactt tgtagttaag aggcatacta tgtctaacta 780
   ccaacatgaa gagactattt ataacttggt taaagattgt ccagcggttg ctgtccatga 840
   ctttttcaag tttagagtag atggtgacat ggtaccacat atatcacgtc agcgtctaac 900
   taaatacaca atggctgatt tagtctatgc tctacgtcat tttgatgagg gtaattgtga 960
   tacattaaaa gaaatactcg tcacatacaa ttgctgtgat gatgattatt tcaataagaa 1020
   ggattggtat gacttcgtag agaatcctga catcttacgc gtatatgcta acttaggtga 1080
   gcgtgtacgc caatcattat taaagactgt acaattctgc gatgctatgc gtgatgcagg 1140
   cattgtaggc gtactgacat tagataatca ggatcttaat gggaactggt acgatttcgg 1200
   tgatttcgta caagtagcac caggctgcgg agttcctatt gtggattcat attactcatt 1260
   gctgatgccc atcctcactt tgactagggc attggctgct gagtcccata tggatgctga 1320
   tctcgcaaaa ccacttatta agtgggattt gctgaaatat gattttacgg aagagagact 1380
   ttgtctcttc gaccgttatt ttaaatattg ggaccagaca taccatccca attgtattaa 1440
   ctgtttggat gataggtgta tccttcattg tgcaaacttt aatgtgttat tttctactgt 1500
   gtttccacct acaagttttg gaccactagt aagaaaaata tttgtagatg gtgttccttt 1560
   tgttgtttca actggatacc attttcgtga gttaggagtc gtacataatc aggatgtaaa 1620
   cttacatagc tcgcgtctca gtttcaagga acttttagtg tatgctgctg atccagctat 1680
   gcatgcagct tctggcaatt tattgctaga taaacgcact acatgctttt cagtagctgc 1740
   actaacaaac aatgttgctt ttcaaactgt caaacccggt aattttaata aagactttta 1800
   tgactttgct gtgtctaaag gtttctttaa ggaaggaagt tctgttgaac taaaacactt 1860
   cttctttgct caggatggca acgctgctat cagtgattat gactattatc gttataatct 1920
   gccaacaatg tgtgatatca gacaactcct attcgtagtt gaagttgttg ataaatactt 1980
   tgattgttac gatggtggct gtattaatgc ca 2012
<210> 51
   <211> 1877
   <212> DNA
   <213> CORONAVIRUS
<400> 51
   gtacttcgcg tacagtggca ataccatatg acagcttaaa tgtttcctca gtggctttga 60
   gcgtttctgc tgcgaaaagc ttgagtctct cagtacaagt gttggcaagt atgtaatcgc 120
   cagcattagt ccaatcacat gttgctatcg cattgaagtc agtgacattg tcactgccta 180
   cacatgtgtt tttgtataaa ccaaaaacct gaccattagc acataatgga aaactaatgg 240
   gaggcttatg tgacttgcaa taatagctca tacctcctag atacagttgt gtcacatcag 300
   tgacatcaca acctggggca ttgcaaacat agggattaac agacaacact aatttgtgtg 360
   atgttgaaat gacatggtca tagcagcact tgcaacatag gaatggtctc ctaatacagg 420
   caccgcaacg aagtgaagtc tgtgaattgc acaatacaca agcacctaca gcctgcaaga 480
   ctgtatgtgg tgtgtacata gcctcataaa actcaggttc ccagtaccgt gaggtgttat 540
   cattagttag cattacggaa tacatgtcca acatgtggcc agtaagctca tcatgtaact 600
   ttctaatgta ttgtaaatac aagtgaaaga catcagcata ctcctgatta ggatgttttg 660
   taagtgggta agcatcaata gccagtgaca cgaacctttc aatcataagt gtaccatctg 720
   ttttgacaat atcatcgaca aaacagcctg cgcctaatat tcttgatgga tctgggtaag 780
   gcaggtacac gtaatcatct ccttgtttaa ctagcattgt atgctgtgag caaaattcgt 840
   gaggtccttt agtaaggtca gtctcagtcc aacattttgc ctcagacatg aacacattat 900
   tttgataata aagaactgcc ttaaagttct taatgctagc tactaaacct tgagccgcat 960
   agttactgtt atagcacaca acggcatcat cagaaagaat catcatggag aaatgtttac 1020
   gcaggtaagc gtaaaactca tccacgaatt catgatcaac atccctattt ctatagagac 1080
   actcatagag cctgtgttgt agattgcgga catacttgtc agctatctta ttaccatcag 1140
   ttgaaagaag tgcatttaca ttggctgtaa cagcttgaca aatgttaaag acactattag 1200
   cataagcagt tgtagcatca ccggatgatg ttccacctgg tttaacatat agtgagccgc 1260
   cacacatgac catctcactt aatacttgcg cacactcgtt agctaacctg tagaaacggt 1320
   gtgataagtt acagcaagtg ttatgtttgc gagcaagaac aagagaggcc attatcctaa 1380
   gcatgttagg catggctctg tcacattttg gataatccca acccataagg tgtggagttt 1440
   ctacatcact gtaaacagtt tttaacatat tatgccagcc accgtaaaac ttgcttgttc 1500
   caattaccac agtagctcct ctagtggcgg ctattgactt caataatttc tgatgaaact 1560
   gtctatttgt catagtacta cagatagaga caccagctac ggtgcgagct ctattctttg 1620
   cactaatggc atacttaaga ttcatttgag ttatagtagg gatgacatta cgcttagtat 1680
   acgcgaaaag tgcatcttga tcctcataac tcattgagtc ataataaagt ctagccttac 1740
   cccatttatt aaatgggaaa ccagctgatt tatccagatt gttaacgatt acttggttgg 1800
   cattaataca gccaccatcg taacaatcaa agtatttatc aacaacttca actacgaata 1860
   ggagttgtct gatatca 1877
<210> 52
   <211> 2051
   <212> DNA
   <213> CORONAVIRUS
<400> 52
   tcaggtccaa tcttgacaaa gtacttcatt gatgtaagct caaagccatg cgcccaaagg 60
   acgaacacga ctctgtctga caatcctttc agtgtatcac tgagcatttg tactatctta 120
   atacgcacta cattccaggg caagccttta tacatgagtg gtataagatg tttaaactgg 180
   tcacctggtg gaggttttgc attaactctg gtgaattctg tgttattttc agtgtcaaca 240
   taaccagtcg gtacagctac taagttaaca cctgtagaaa atcctagctg gagaggtagg 300
   ttagtaccca cagcatctct agttgcatga cagccctcta catcaaagcc aatccacgca 360
   cgaacgtgac gaatagcttc ttcgcgggtg ataaacatat tagggtaacc attgacttgg 420
   taattcattt tgaaacccat catagagatg agtctacggt aggtcatgtc ctttggtatg 480
   cctggtatgt caacacataa tccttcagtc ttgaacttta tatcaacgct gaggtgtgta 540
   ggtgcctgtg taggatgaag accagtaatg atcttactac agtccttaaa aagtccagtt 600
   acattttctg cttgtaatgt agccacattg cgacgtggta tttctagact tgtaaattgc 660
   agtttgtcat aaagatctct atcagacatt atgcacaaaa tgccaatttt tgcccttgtg 720
   atagccacat tgaagcggtt gacattacaa gagtgtgctg tttcagtagt ttgtgtgaat 780
   atgacatagt catattcaga accctgtgat gaatcaacag tctgcgtagg caatcctaag 840
   atttttgaag ctacagcgtt ctgtgaatta taaggtgaga taaaaacagc ttttctccaa 900
   gcaggattgc gtgtaagaaa ttctcttaca acgcctattt gaggtctgtt gattgcagat 960
   gaaacatcat gtgtaataac acctttgtag aacattttga agcattgagc tgacttatcc 1020
   ttgtgtgctt ttagcttatt gtcataaact aaagcactca cagtgtcaac aatttcagca 1080
   ggacaacggc gacaagttcc aaggaacatg tctggaccta ttgttttcat aagtctgcac 1140
   actgaattaa aatattctgg ttctagtgtg cctttagtca gcaatgtgcg gggggctggt 1200
   aattgagcag gatcgccaat atagacgtag tgttttgcac gaagtctagc attgacaaca 1260
   ctcaagtcat aattagtagc catagagatt tcatcaaaga ctacaatgtc agcagttgtt 1320
   tctggcaatg catttacagt gcagaaaaca tactgttcta gtgttgaatt cactttgaat 1380
   ttatcaaaac actctacgcg cgcacgcgca ggtatgattc tactacattt atctatgggc 1440
   aaatatttta atgccttttc acatagggca tcaacagctg catgagagca tgccgtatac 1500
   actatgcgag cagatgggta atagagagca agtccgatgg caaaatgact cttaccagta 1560
   ccaggtggtc cttggagtgt agagtacttt tgcatgccga ccttttgata atttgcaaca 1620
   ttgctagaaa actcatctga gatgttgagt gttgggtaca agccagtaat tctcacatag 1680
   tgctcttgtg gcactagagt aggtgcacta agtggcatta cagtgtgaga tgtcaacaca 1740
   aagtaatcac caacattcaa cttgtatgtc gtagtacctc tgtacacaac agcatcacca 1800
   tagtcacctt tttcaaaggt gtactctcca atctgtactt tactattttt agttacacgg 1860
   taaccagtaa agacatagtt tctgttcaat ggtggtctag gttttccaac ctcccatgaa 1920
   agatgcaatt ctctgtcaga gagtacttcg cgtacagtgg caataccata tgacagctta 1980
   aatgtttcct cagtggcttt gagcgtttct gctgcgaaaa gcttgagtct ctcagtacaa 2040
   gtgttggcaa g 2051
<210> 53
   <211> 2075
   <212> DNA
   <213> CORONAVIRUS
<400> 53
   tgcttgtagt tttgggtaga aggtttcaac atgtccatcc ttacaccaaa gcatgaatga 60
   aatttcagca tagtcaattg taaccttgac cacttttgaa atcactgaca aatcttgtga 120
   ctttattatc tcgacaaagt catcaagtaa aagatcaatc acagaacaca cacattttga 180
   tgaacctgtt tgcgcatctg ttatgaagta atttttcact gtgctgtcca tagggataaa 240
   atcctctaat ttaagtggtg aatcttgtga gcgcttggct aagcctatca ttaaatgaag 300
   accgccaagt tgtccatgac tgaaatctcc ataaacgatg tgttcgaagg catagccctc 360
   gagcttatat cgctgtatga attcatccat agcgagctcg agaaagtcag tttccatttg 420
   tgatctgggc ttaaaatcct ctaagtctct gctctgagta aagtaggttt caggcaactg 480
   ttgaataatg ccgtctactt tcttaaagta gttaaactgt gtttttactg attctccaat 540
   taatgtgact ccattgacgc tagcttgtgc tggtcccttt gaaggtgtta gacctttgac 600
   tgaaccttct gttattaaaa caccattacg ggcgtttcta aaaaggtcta cctgtccttc 660
   cactctacca tcaaacaaga cagtaagtga agaacaagca ctctcagtag gtttcttggc 720
   aatgtcagtc attgtgcaga cacctattgt agatacatgt gctggggctt ctcttttgta 780
   gtcccagatt acagtattag cagcgatatc aacacccaaa ttattgagta tcttaatctc 840
   tggcactggt ttaatgttac gcttagccca aagctcaaat gcaacattaa caggaagtgt 900
   tgtcttattt tcaaagatct ccacatcaat accatctacc tttgtgtaaa cagcattatt 960
   aatgatggaa acaggtgctt cgccggcgtg tccatcaaag tgtcctttat taacaacatt 1020
   ataagccaca ttttctaaac tctgtaacct ggtaaatgta ttccacaggt tataagtatc 1080
   aaattgtttg taaatccata ggctaaatcc agcagaaatc atcatattat atgcatccaa 1140
   gtactgtcgg tactcatttg catggtgtct gcaaacagca ccacctaaat tgcatcgtgt 1200
   aatacacgta gcagatttga gtggaacata atcaatatcc gacactactt gtttgccatg 1260
   agactcacaa ggactatcag aatagtaaaa gaaaggcaat tgctttaaat tagtaaatgc 1320
   acttttatcg aaagctggag tgtggaatgc atgcttattc acatacaaac taccaccatc 1380
   acagcctggt aagttcaagt ttgacaagac tcttgtgtca aacctacaca caattgcatt 1440
   ggctgggtaa cgatcaacgt tacaattcca aaacaaacaa acaccatcag tgaatttatc 1500
   gtgatgtgta gcataagaat agaagagttc ctctattttg taagctttgt cactacatgg 1560
   ctgagcatcg tagaacttcc attctacttc agcctgaggc acacacttga tagcctttgg 1620
   atttccaatg tcatgaagaa ctggaaactt atcagcaagc aatgcagact tcacaaccat 1680
   gtgttgtact tttctgcaag cagaattaac cctcagttca tctcctataa tagggtattc 1740
   aacagaccaa tcaacgcgct taacaaagca ctcatggact gctaaacatc tagtcatgat 1800
   agcatcacaa ctagccacat gtgcatttcc atgtacctgg caatgttggt catggttact 1860
   ctgaaggtta cccgtaaagc cccactgctg aacatcaatc ataaatgggt tatagacata 1920
   gtcaaaaccc acagaatgat tccagcaggc ataagtatct gatgaagtag aaaagcaagt 1980
   tgcacgtttg tcacacagac aacacgttct ttcaggtcca atcttgacaa agtacttcat 2040
   tgatgtaagc tcaaagccat gcgcccaaag gacga 2075
<210> 54
   <211> 1891
   <212> DNA
   <213> CORONAVIRUS
<400> 54
   aagattcacc acttaaatta gaggatttta tccctatgga cagcacagtg aaaaattact 60
   tcataacaga tgcgcaaaca ggttcatcaa aatgtgtgtg ttctgtgatt gatcttttac 120
   ttgatgactt tgtcgagata ataaagtcac aagatttgtc agtgatttca aaagtggtca 180
   aggttacaat tgactatgct gaaatttcat tcatgctttg gtgtaaggat ggacatgttg 240
   aaaccttcta cccaaaacta caagcaagtc aagcgtggca accaggtgtt gcgatgccta 300
   acttgtacaa gatgcaaaga atgcttcttg aaaagtgtga ccttcagaat tatggtgaaa 360
   atgctgttat accaaaagga ataatgatga atgtcgcaaa gtatactcaa ctgtgtcaat 420
   acttaaatac acttacttta gctgtaccct acaacatgag agttattcac tttggtgctg 480
   gctctgataa aggagttgca ccaggtacag ctgtgctcag acaatggttg ccaactggca 540
   cactacttgt cgattcagat cttaatgact tcgtctccga cgcagattct actttaattg 600
   gagactgtgc aacagtacat acggctaata aatgggacct tattattagc gatatgtatg 660
   accctaggac caaacatgtg acaaaagaga atgactctaa agaagggttt ttcacttatc 720
   tgtgtggatt tataaagcaa aaactagccc tgggtggttc tatagctgta aagataacag 780
   agcattcttg gaatgctgac ctttacaagc ttatgggcca tttctcatgg tggacagctt 840
   ttgttacaaa tgtaaatgca tcatcatcgg aagcattttt aattggggct aactatcttg 900
   gcaagccgaa ggaacaaatt gatggctata ccatgcatgc taactacatt ttctggagga 960
   acacaaatcc tatccagttg tcttcctatt cactctttga catgagcaaa tttcctctta 1020
   aattaagagg aactgctgta atgtctctta aggagaatca aatcaatgat atgatttatt 1080
   ctcttctgga aaaaggtagg cttatcatta gagaaaacaa cagagttgtg gtttcaagtg 1140
   atattcttgt taacaactaa acgaacatgt ttattttctt attatttctt actctcacta 1200
   gtggtagtga ccttgaccgg tgcaccactt ttgatgatgt tcaagctcct aattacactc 1260
   aacatacttc atctatgagg ggggtttact atcctgatga aatttttaga tcagacactc 1320
   tttatttaac tcaggattta tttcttccat tttattctaa tgttacaggg tttcatacta 1380
   ttaatcatac gtttggcaac cctgtcatac cttttaagga tggtatttat tttgctgcca 1440
   cagagaaatc aaatgttgtc cgtggttggg tttttggttc taccatgaac aacaagtcac 1500
   agtcggtgat tattattaac aattctacta atgttgttat acgagcatgt aactttgaat 1560
   tgtgtgacaa ccctttcttt gctgtttcta aacccatggg tacacagaca catactatga 1620
   tattcgataa tgcatttaat tgcactttcg agtacatatc tgatgccttt tcgcttgatg 1680
   tttcagaaaa gtcaggtaat tttaaacact tacgagagtt tgtgtttaaa aataaagatg 1740
   ggtttctcta tgtttataag ggctatcaac ctatagatgt agttcgtgat ctaccttctg 1800
   gttttaacac tttgaaacct atttttaagt tgcctcttgg tattaacatt acaaatttta 1860
   gagccattct tacagccttt tcacctgctc a 1891
<210> 55
   <211> 32
   <212> DNA
   <213> ARTIFICIAL SEQUENCE
<220>
   <223> amorce N sens
<400> 55
   cccatatgtc tgataatgga ccccaatcaa ac 32
<210> 56
   <211> 32
   <212> DNA
   <213> artificial sequence
<220>
   <223> amorce N antisens
<400> 56
   cccccgggtg cctgagttga atcagcagaa gc 32
<210> 57
   <211> 31
   <212> DNA
   <213> artificial sequence
<220>
   <223> amorce Sc sens
<400> 57
   cccatatgag tgaccttgac cggtgcacca c 31
<210> 58
   <211> 30
   <212> DNA
   <213> artificial sequence
<220>
   <223> amorce SL sens
<400> 58
   cccatatgaa accttgcacc ccacctgctc 30
<210> 59
   <211> 33
   <212> DNA
   <213> amorce Sc et SL antisens
<400> 59
   cccccgggtt taatatattg ctcatatttt ccc 33
<210> 60
   <211> 16
   <212> DNA
   <213> amorce sens série 1
<400> 60
   ggcatcgtat gggttg 16
<210> 61
   <211> 16
   <212> DNA
   <213> amorce antisens série 2 (28774-28759)
<400> 61
   cagtttcacc acctcc 16
<210> 62
   <211> 16
   <212> DNA
   <213> amorce sens série 2 (28375-28390)
<400> 62
   ggctactacc gaagag 16
<210> 63
   <211> 16
   <212> DNA
   <213> amorce antisens série 2 (28702-28687)
<400> 63
   aattaccgcg actacg 16
<210> 64
   <211> 26
   <212> DNA
   <213> sonde 1/série 1 (28561-28586)
<400> 64
   ggcacccgca atcctaataa caatgc 26
<210> 65
   <211> 21
   <212> DNA
   <213> sonde 2/série 1 (28588-28608)
<400> 65
   gccaccgtgc tacaacttcc t 21
<210> 66
   <211> 23
   <212> DNA
   <213> sonde 1/série 2 /sonde N/FL (28541-28563)
<400> 66
   atacacccaa agaccacatt ggc 23
<210> 67
   <211> 25
   <212> DNA
   <213> sonde 2/série 2/sonde SARS/N/LC705 (28565-28589)
<400> 67
   cccgcaatcc taataacaat gctgc 25
<210> 68
   <211> 30
   <212> DNA
   <213> artificial sequence
<220>
   <223> amorce ancre 14T
<400> 68
   agatgaattc ggtacctttt tttttttttt 30
<210> 69
   <211> 13
   <212> PRT
   <213> artificial sequence
<220>
   <223> peptide M2-14
<400> 69
   Ala Asp Asn Gly Thr Ile Thr Val Glu Glu Leu Lys Gln
   1 5 10
<210> 70
   <211> 12
   <212> PRT
   <213> artificial sequence
<220>
   <223> peptide E1-12
<400> 70
   Met Tyr Ser Phe Val Ser Glu Glu Thr Gly Thr Leu
   1 5 10
<210> 71
   <211> 24
   <212> PRT
   <213> artificial sequence
<220>
   <223> peptide E53-72
<400> 71
   Lys Pro Thr Val Tyr Val Tyr Ser Arg Val Lys Asn Leu Asn Ser Ser
   1 5 10 15
   Glu Gly Val Pro Asp Leu Leu Val
   20
<210> 72
   <211> 153
   <212> DNA
   <213> CORONAVIRUS
<400> 72
   gatattaggt ttttacctac ccaggaaaag ccaaccaacc tcgatctctt gtagatctgt    60
   tctctaaacg aactttaaaa tctgtgtagc tgtcgctcgg ctgcatgcct agtgcaccta   120
   cgcagtataa acaataataa attttactgt cgt                                153
<210> 73
   <211> 410
   <212> DNA
   <213> CORONAVIRUS
<400> 73
   ttctccagac aacttcaaaa ttccatgagt ggagcttctg ctgattcaac tcaggcataa    60
   acactcatga tgaccacaca aggcagatgg gctatgtaaa cgttttcgca attccgttta   120
   cgatacatag tctactcttg tgcagaatga attctcgtaa ctaaacagca caagtaggtt   180
   tagttaactt taatctcaca tagcaatctt taatcaatgt gtaacattag ggaggacttg   240
   aaagagccac cacattttca tcgaggccac gcggagtacg atcgagggta cagtgaataa   300
   tgctagggag agctgcctat atggaagagc cctaatgtgt aaaattaatt ttagtagtgc   360
   tatccccatg tgattttaat agcttcttag gagaatgaca aaaaaaaaaa              410
<210> 74
   <211> 4382
   <212> PRT
   <213> CORONAVIRUS
<400> 74
   Met Glu Ser Leu Val Leu Gly Val Asn Glu Lys Thr His Val Gln Leu
   1               5                   10                  15
   Ser Leu Pro Val Leu Gln Val Arg Asp Val Leu Val Arg Gly Phe Gly
                   20                  25                  30
   Asp Ser Val Glu Glu Ala Leu Ser Glu Ala Arg Glu His Leu Lys Asn
           35                  40                  45
   Gly Thr Cys Gly Leu Val Glu Leu Glu Lys Gly Val Leu Pro Gln Leu
       50                  55                  60
   Glu Gln Pro Tyr Val Phe Ile Lys Arg Ser Asp Ala Leu Ser Thr Asn
   65                  70                  75                  80
   His Gly His Lys Val Val Glu Leu Val Ala Glu Met Asp Gly Ile Gln
                   85                  90                  95
   Tyr Gly Arg Ser Gly Ile Thr Leu Gly Val Leu Val Pro His Val Gly
               100                 105                 110
   Glu Thr Pro Ile Ala Tyr Arg Asn Val Leu Leu Arg Lys Asn Gly Asn
           115                 120                 125
   Lys Gly Ala Gly Gly His Ser Tyr Gly Ile Asp Leu Lys Ser Tyr Asp
       130                 135                 140
   Leu Gly Asp Glu Leu Gly Thr Asp Pro Ile Glu Asp Tyr Glu Gln Asn
   145                 150                 155                 160
   Trp Asn Thr Lys His Gly Ser Gly Ala Leu Arg Glu Leu Thr Arg Glu
                   165                 170                 175
   Leu Asn Gly Gly Ala Val Thr Arg Tyr Val Asp Asn Asn Phe Cys Gly
               180                 185                 190
   Pro Asp Gly Tyr Pro Leu Asp Cys Ile Lys Asp Phe Leu Ala Arg Ala
           195                 200                 205
   Gly Lys Ser Met Cys Thr Leu Ser Glu Gln Leu Asp Tyr Ile Glu Ser
       210                 215                 220
   Lys Arg Gly Val Tyr Cys Cys Arg Asp His Glu His Glu Ile Ala Trp
   225                 230                 235                 240
   Phe Thr Glu Arg Ser Asp Lys Ser Tyr Glu His Gln Thr Pro Phe Glu
                   245                 250                 255
   Ile Lys Ser Ala Lys Lys Phe Asp Thr Phe Lys Gly Glu Cys Pro Lys
               260                 265                 270
   Phe Val Phe Pro Leu Asn Ser Lys Val Lys Val Ile Gln Pro Arg Val
           275                 280                 285
   Glu Lys Lys Lys Thr Glu Gly Phe Met Gly Arg Ile Arg Ser Val Tyr
   290 295 300
   Pro Val Ala Ser Pro Gln Glu Cys Asn Asn Met His Leu Ser Thr Leu
   305 310 315 320
   Met Lys Cys Asn His Cys Asp Glu Val Ser Trp Gln Thr Cys Asp Phe
   325 330 335
   Leu Lys Ala Thr Cys Glu His Cys Gly Thr Glu Asn Leu Val Ile Glu
   340 345 350
   Gly Pro Thr Thr Cys Gly Tyr Leu Pro Thr Asn Ala Val Val Lys Met
   355 360 365
   Pro Cys Pro Ala Cys Gln Asp Pro Glu Ile Gly Pro Glu His Ser Val
   370 375 380
   Ala Asp Tyr His Asn His Ser Asn Ile Glu Thr Arg Leu Arg Lys Gly
   385 390 395 400
   Gly Arg Thr Arg Cys Phe Gly Gly Cys Val Phe Ala Tyr Val Gly Cys
   405 410 415
   Tyr Asn Lys Arg Ala Tyr Trp Val Pro Arg Ala Ser Ala Asp Ile Gly
   420 425 430
   Ser Gly His Thr Gly Ile Thr Gly Asp Asn Val Glu Thr Leu Asn Glu
   435 440 445
   Asp Leu Leu Glu Ile Leu Ser Arg Glu Arg Val Asn Ile Asn Ile Val
   450 455 460
   Gly Asp Phe His Leu Asn Glu Glu Val Ala Ile Ile Leu Ala Ser Phe
   465 470 475 480
   Ser Ala Ser Thr Ser Ala Phe Ile Asp Thr Ile Lys Ser Leu Asp Tyr
   485 490 495
   Lys Ser Phe Lys Thr Ile Val Glu Ser Cys Gly Asn Tyr Lys Val Thr
   500 505 510
   Lys Gly Lys Pro Val Lys Gly Ala Trp Asn Ile Gly Gln Gln Arg Ser
   515 520 525
   Val Leu Thr Pro Leu Cys Gly Phe Pro Ser Gln Ala Ala Gly Val Ile
   530 535 540
   Arg Ser Ile Phe Ala Arg Thr Leu Asp Ala Ala Asn His Ser Ile Pro
   545 550 555 560
   Asp Leu Gln Arg Ala Ala Val Thr Ile Leu Asp Gly Ile Ser Glu Gln
   565 570 575
   Ser Leu Arg Leu Val Asp Ala Met Val Tyr Thr Ser Asp Leu Leu Thr
   580 585 590
   Asn Ser Val Ile Ile Met Ala Tyr Val Thr Gly Gly Leu Val Gln Gln
   595 600 605
   Thr Ser Gln Trp Leu Ser Asn Leu Leu Gly Thr Thr Val Glu Lys Leu
   610 615 620
   Arg Pro Ile Phe Glu Trp Ile Glu Ala Lys Leu Ser Ala Gly Val Glu
   625 630 635 640
   Phe Leu Lys Asp Ala Trp Glu Ile Leu Lys Phe Leu Ile Thr Gly Val
   645 650 655
   Phe Asp Ile Val Lys Gly Gln Ile Gln Val Ala Ser Asp Asn Ile Lys
   660 665 670
   Asp Cys Val Lys Cys Phe Ile Asp Val Val Asn Lys Ala Leu Glu Met
   675 680 685
   Cys Ile Asp Gln Val Thr Ile Ala Gly Ala Lys Leu Arg Ser Leu Asn
   690 695 700
   Leu Gly Glu Val Phe Ile Ala Gln Ser Lys Gly Leu Tyr Arg Gln Cys
   705 710 715 720
   Ile Arg Gly Lys Glu Gln Leu Gln Leu Leu Met Pro Leu Lys Ala Pro
   725 730 735
   Lys Glu Val Thr Phe Leu Glu Gly Asp Ser His Asp Thr Val Leu Thr
   740 745 750
   Ser Glu Glu Val Val Leu Lys Asn Gly Glu Leu Glu Ala Leu Glu Thr
   755 760 765
   Pro Val Asp Ser Phe Thr Asn Gly Ala Ile Val Gly Thr Pro Val Cys
   770 775 780
   Val Asn Gly Leu Met Leu Leu Glu Ile Lys Asp Lys Glu Gln Tyr Cys
   785 790 795 800
   Ala Leu Ser Pro Gly Leu Leu Ala Thr Asn Asn Val Phe Arg Leu Lys
   805 810 815
   Gly Gly Ala Pro Ile Lys Gly Val Thr Phe Gly Glu Asp Thr Val Trp
   820 825 830
   Glu Val Gln Gly Tyr Lys Asn Val Arg Ile Thr Phe Glu Leu Asp Glu
   835 840 845
   Arg Val Asp Lys Val Leu Asn Glu Lys Cys Ser Val Tyr Thr Val Glu
   850 855 860
   Ser Gly Thr Glu Val Thr Glu Phe Ala Cys Val Val Ala Glu Ala Val
   865 870 875 880
   Val Lys Thr Leu Gln Pro Val Ser Asp Leu Leu Thr Asn Met Gly Ile
   885 890 895
   Asp Leu Asp Glu Trp Ser Val Ala Thr Phe Tyr Leu Phe Asp Asp Ala
   900 905 910
   Gly Glu Glu Asn Phe Ser Ser Arg Met Tyr Cys Ser Phe Tyr Pro Pro
   915 920 925
   Asp Glu Glu Glu Glu Asp Asp Ala Glu Cys Glu Glu Glu Glu Ile Asp
   930 935 940
   Glu Thr Cys Glu His Glu Tyr Gly Thr Glu Asp Asp Tyr Gln Gly Leu
   945 950 955 960
   Pro Leu Glu Phe Gly Ala Ser Ala Glu Thr Val Arg Val Glu Glu Glu
   965 970 975
   Glu Glu Glu Asp Trp Leu Asp Asp Thr Thr Glu Gln Ser Glu Ile Glu
   980 985 990
   Pro Glu Pro Glu Pro Thr Pro Glu Glu Pro Val Asn Gln Phe Thr Gly
   995 1000 1005
   Tyr Leu Lys Leu Thr Asp Asn Val Ala Ile Lys Cys Val Asp Ile
   1010 1015 1020
   Val Lys Glu Ala Gln Ser Ala Asn Pro Met Val Ile Val Asn Ala
   1025 1030 1035
   Ala Asn Ile His Leu Lys His Gly Gly Gly Val Ala Gly Ala Leu
   1040 1045 1050
   Asn Lys Ala Thr Asn Gly Ala Met Gln Lys Glu Ser Asp Asp Tyr
   1055 1060 1065
   Ile Lys Leu Asn Gly Pro Leu Thr Val Gly Gly Ser Cys Leu Leu
   1070 1075 1080
   Ser Gly His Asn Leu Ala Lys Lys Cys Leu His Val Val Gly Pro
   1085 1090 1095
   Asn Leu Asn Ala Gly Glu Asp Ile Gln Leu Leu Lys Ala Ala Tyr
   1100 1105 1110
   Glu Asn Phe Asn Ser Gln Asp Ile Leu Leu Ala Pro Leu Leu Ser
   1115 1120 1125
   Ala Gly Ile Phe Gly Ala Lys Pro Leu Gln Ser Leu Gln Val Cys
   1130 1135 1140
   Val Gln Thr Val Arg Thr Gln Val Tyr Ile Ala Val Asn Asp Lys
   1145 1150 1155
   Ala Leu Tyr Glu Gln Val Val Met Asp Tyr Leu Asp Asn Leu Lys
   1160 1165 1170
   Pro Arg Val Glu Ala Pro Lys Gln Glu Glu Pro Pro Asn Thr Glu
   1175 1180 1185
   Asp Ser Lys Thr Glu Glu Lys Ser Val Val Gln Lys Pro Val Asp
   1190 1195 1200
   Val Lys Pro Lys Ile Lys Ala Cys Ile Asp Glu Val Thr Thr Thr
   1205 1210 1215
   Leu Glu Glu Thr Lys Phe Leu Thr Asn Lys Leu Leu Leu Phe Ala
   1220 1225 1230
   Asp Ile Asn Gly Lys Leu Tyr His Asp Ser Gln Asn Met Leu Arg
   1235 1240 1245
   Gly Glu Asp Met Ser Phe Leu Glu Lys Asp Ala Pro Tyr Met Val
   1250 1255 1260
   Gly Asp Val Ile Thr Ser Gly Asp Ile Thr Cys Val Val Ile Pro
   1265 1270 1275
   Ser Lys Lys Ala Gly Gly Thr Thr Glu Met Leu Ser Arg Ala Leu
   1280 1285 1290
   Lys Lys Val Pro Val Asp Glu Tyr Ile Thr Thr Tyr Pro Gly Gln
   1295 1300 1305
   Gly Cys Ala Gly Tyr Thr Leu Glu Glu Ala Lys Thr Ala Leu Lys
   1310 1315 1320
   Lys Cys Lys Ser Ala Phe Tyr Val Leu Pro Ser Glu Ala Pro Asn
   1325 1330 1335
   Ala Lys Glu Glu Ile Leu Gly Thr Val Ser Trp Asn Leu Arg Glu
   1340 1345 1350
   Met Leu Ala His Ala Glu Glu Thr Arg Lys Leu Met Pro Ile Cys
   1355 1360 1365
   Met Asp Val Arg Ala Ile Met Ala Thr Ile Gln Arg Lys Tyr Lys
   1370 1375 1380
   Gly Ile Lys Ile Gln Glu Gly Ile Val Asp Tyr Gly Val Arg Phe
   1385 1390 1395
   Phe Phe Tyr Thr Ser Lys Glu Pro Val Ala Ser Ile Ile Thr Lys
   1400 1405 1410
   Leu Asn Ser Leu Asn Glu Pro Leu Val Thr Met Pro Ile Gly Tyr
   1415 1420 1425
   Val Thr His Gly Phe Asn Leu Glu Glu Ala Ala Arg Cys Met Arg
   1430 1435 1440
   Ser Leu Lys Ala Pro Ala Val Val Ser Val Ser Ser Pro Asp Ala
   1445 1450 1455
   Val Thr Thr Tyr Asn Gly Tyr Leu Thr Ser Ser Ser Lys Thr Ser
   1460 1465 1470
   Glu Glu His Phe Val Glu Thr Val Ser Leu Ala Gly Ser Tyr Arg
   1475 1480 1485
   Asp Trp Ser Tyr Ser Gly Gln Arg Thr Glu Leu Gly Val Glu Phe
   1490 1495 1500
   Leu Lys Arg Gly Asp Lys Ile Val Tyr His Thr Leu Glu Ser Pro
   1505 1510 1515
   Val Glu Phe His Leu Asp Gly Glu Val Leu Ser Leu Asp Lys Leu
   1520 1525 1530
   Lys Ser Leu Leu Ser Leu Arg Glu Val Lys Thr Ile Lys Val Phe
   1535 1540 1545
   Thr Thr Val Asp Asn Thr Asn Leu His Thr Gln Leu Val Asp Met
   1550 1555 1560
   Ser Met Thr Tyr Gly Gln Gln Phe Gly Pro Thr Tyr Leu Asp Gly
   1565 1570 1575
   Ala Asp Val Thr Lys Ile Lys Pro His Val Asn His Glu Gly Lys
   1580 1585 1590
   Thr Phe Phe Val Leu Pro Ser Asp Asp Thr Leu Arg Ser Glu Ala
   1595 1600 1605
   Phe Glu Tyr Tyr His Thr Leu Asp Glu Ser Phe Leu Gly Arg Tyr
   1610 1615 1620
   Met Ser Ala Leu Asn His Thr Lys Lys Trp Lys Phe Pro Gln Val
   1625 1630 1635
   Gly Gly Leu Thr Ser Ile Lys Trp Ala Asp Asn Asn Cys Tyr Leu
   1640 1645 1650
   Ser Ser Val Leu Leu Ala Leu Gln Gln Leu Glu Val Lys Phe Asn
   1655 1660 1665
   Ala Pro Ala Leu Gln Glu Ala Tyr Tyr Arg Ala Arg Ala Gly Asp
   1670 1675 1680
   Ala Ala Asn Phe Cys Ala Leu Ile Leu Ala Tyr Ser Asn Lys Thr
   1685 1690 1695
   Val Gly Glu Leu Gly Asp Val Arg Glu Thr Met Thr His Leu Leu
   1700 1705 1710
   Gln His Ala Asn Leu Glu Ser Ala Lys Arg Val Leu Asn Val Val
   1715 1720 1725
   Cys Lys His Cys Gly Gln Lys Thr Thr Thr Leu Thr Gly Val Glu
   1730 1735 1740
   Ala Val Met Tyr Met Gly Thr Leu Ser Tyr Asp Asn Leu Lys Thr
   1745 1750 1755
   Gly Val Ser Ile Pro Cys Val Cys Gly Arg Asp Ala Thr Gln Tyr
   1760 1765 1770
   Leu Val Gln Gln Glu Ser Ser Phe Val Met Met Ser Ala Pro Pro
   1775 1780 1785
   Ala Glu Tyr Lys Leu Gln Gln Gly Thr Phe Leu Cys Ala Asn Glu
   1790 1795 1800
   Tyr Thr Gly Asn Tyr Gln Cys Gly His Tyr Thr His Ile Thr Ala
   1805 1810 1815
   Lys Glu Thr Leu Tyr Arg Ile Asp Gly Ala His Leu Thr Lys Met
   1820 1825 1830
   Ser Glu Tyr Lys Gly Pro Val Thr Asp Val Phe Tyr Lys Glu Thr
   1835 1840 1845
   Ser Tyr Thr Thr Thr Ile Lys Pro Val Ser Tyr Lys Leu Asp Gly
   1850 1855 1860
   Val Thr Tyr Thr Glu Ile Glu Pro Lys Leu Asp Gly Tyr Tyr Lys
       1865                    1870                    1875
   Lys Asp Asn Ala Tyr Tyr Thr Glu Gln Pro Ile Asp Leu Val Pro
       1880                    1885                    1890
   Thr Gln Pro Leu Pro Asn Ala Ser Phe Asp Asn Phe Lys Leu Thr
       1895                    1900                    1905
   Cys Ser Asn Thr Lys Phe Ala Asp Asp Leu Asn Gln Met Thr Gly
       1910                    1915                    1920
   Phe Thr Lys Pro Ala Ser Arg Glu Leu Ser Val Thr Phe Phe Pro
       1925                    1930                    1935
   Asp Leu Asn Gly Asp Val Val Ala Ile Asp Tyr Arg His Tyr Ser
       1940                    1945                    1950
   Ala Ser Phe Lys Lys Gly Ala Lys Leu Leu His Lys Pro Ile Val
       1955                    1960                    1965
   Trp His Ile Asn Gln Ala Thr Thr Lys Thr Thr Phe Lys Pro Asn
       1970                    1975                    1980
   Thr Trp Cys Leu Arg Cys Leu Trp Ser Thr Lys Pro Val Asp Thr
       1985                    1990                    1995
   Ser Asn Ser Phe Glu Val Leu Ala Val Glu Asp Thr Gln Gly Met
       2000                    2005                    2010
   Asp Asn Leu Ala Cys Glu Ser Gln Gln Pro Thr Ser Glu Glu Val
       2015                    2020                    2025
   Val Glu Asn Pro Thr Ile Gln Lys Glu Val Ile Glu Cys Asp Val
       2030                    2035                    2040
   Lys Thr Thr Glu Val Val Gly Asn Val Ile Leu Lys Pro Ser Asp
       2045                    2050                    2055
   Glu Gly Val Lys Val Thr Gln Glu Leu Gly His Glu Asp Leu Met
       2060                    2065                    2070
   Ala Ala Tyr Val Glu Asn Thr Ser Ile Thr Ile Lys Lys Pro Asn
       2075                    2080                    2085
   Glu Leu Ser Leu Ala Leu Gly Leu Lys Thr Ile Ala Thr His Gly
       2090                    2095                    2100
   Ile Ala Ala Ile Asn Ser Val Pro Trp Ser Lys Ile Leu Ala Tyr
       2105                    2110                    2115
   Val Lys Pro Phe Leu Gly Gln Ala Ala Ile Thr Thr Ser Asn Cys
   2120 2125 2130
   Ala Lys Arg Leu Ala Gln Arg Val Phe Asn Asn Tyr Met Pro Tyr
   2135 2140 2145
   Val Phe Thr Leu Leu Phe Gln Leu Cys Thr Phe Thr Lys Ser Thr
   2150 2155 2160
   Asn Ser Arg Ile Arg Ala Ser Leu Pro Thr Thr Ile Ala Lys Asn
   2165 2170 2175
   Ser Val Lys Ser Val Ala Lys Leu Cys Leu Asp Ala Gly Ile Asn
   2180 2185 2190
   Tyr Val Lys Ser Pro Lys Phe Ser Lys Leu Phe Thr Ile Ala Met
   2195 2200 2205
   Trp Leu Leu Leu Leu Ser Ile Cys Leu Gly Ser Leu Ile Cys Val
   2210 2215 2220
   Thr Ala Ala Phe Gly Val Leu Leu Ser Asn Phe Gly Ala Pro Ser
   2225 2230 2235
   Tyr Cys Asn Gly Val Arg Glu Leu Tyr Leu Asn Ser Ser Asn Val
   2240 2245 2250
   Thr Thr Met Asp Phe Cys Glu Gly Ser Phe Pro Cys Ser Ile Cys
   2255 2260 2265
   Leu Ser Gly Leu Asp Ser Leu Asp Ser Tyr Pro Ala Leu Glu Thr
   2270 2275 2280
   Ile Gln Val Thr Ile Ser Ser Tyr Lys Leu Asp Leu Thr Ile Leu
   2285 2290 2295
   Gly Leu Ala Ala Glu Trp Val Leu Ala Tyr Met Leu Phe Thr Lys
   2300 2305 2310
   Phe Phe Tyr Leu Leu Gly Leu Ser Ala Ile Met Gln Val Phe Phe
   2315 2320 2325
   Gly Tyr Phe Ala Ser His Phe Ile Ser Asn Ser Trp Leu Met Trp
   2330 2335 2340
   Phe Ile Ile Ser Ile Val Gln Met Ala Pro Val Ser Ala Met Val
   2345 2350 2355
   Arg Met Tyr Ile Phe Phe Ala Ser Phe Tyr Tyr Ile Trp Lys Ser
   2360 2365 2370
   Tyr Val His Ile Met Asp Gly Cys Thr Ser Ser Thr Cys Met Met
   2375 2380 2385
   Cys Tyr Lys Arg Asn Arg Ala Thr Arg Val Glu Cys Thr Thr Ile
   2390 2395 2400
   Val Asn Gly Met Lys Arg Ser Phe Tyr Val Tyr Ala Asn Gly Gly
   2405 2410 2415
   Arg Gly Phe Cys Lys Thr His Asn Trp Asn Cys Leu Asn Cys Asp
   2420 2425 2430
   Thr Phe Cys Thr Gly Ser Thr Phe Ile Ser Asp Glu Val Ala Arg
   2435 2440 2445
   Asp Leu Ser Leu Gln Phe Lys Arg Pro Ile Asn Pro Thr Asp Gln
   2450 2455 2460
   Ser Ser Tyr Ile Val Asp Ser Val Ala Val Lys Asn Gly Ala Leu
   2465 2470 2475
   His Leu Tyr Phe Asp Lys Ala Gly Gln Lys Thr Tyr Glu Arg His
   2480 2485 2490
   Pro Leu Ser His Phe Val Asn Leu Asp Asn Leu Arg Ala Asn Asn
   2495 2500 2505
   Thr Lys Gly Ser Leu Pro Ile Asn Val Ile Val Phe Asp Gly Lys
   2510 2515 2520
   Ser Lys Cys Asp Glu Ser Ala Ser Lys Ser Ala Ser Val Tyr Tyr
   2525 2530 2535
   Ser Gln Leu Met Cys Gln Pro Ile Leu Leu Leu Asp Gln Ala Leu
   2540 2545 2550
   Val Ser Asp Val Gly Asp Ser Thr Glu Val Ser Val Lys Met Phe
   2555 2560 2565
   Asp Ala Tyr Val Asp Thr Phe Ser Ala Thr Phe Ser Val Pro Met
   2570 2575 2580
   Glu Lys Leu Lys Ala Leu Val Ala Thr Ala His Ser Glu Leu Ala
   2585 2590 2595
   Lys Gly Val Ala Leu Asp Gly Val Leu Ser Thr Phe Val Ser Ala
   2600 2605 2610
   Ala Arg Gln Gly Val Val Asp Thr Asp Val Asp Thr Lys Asp Val
   2615 2620 2625
   Ile Glu Cys Leu Lys Leu Ser His His Ser Asp Leu Glu Val Thr
   2630 2635 2640
   Gly Asp Ser Cys Asn Asn Phe Met Leu Thr Tyr Asn Lys Val Glu
   2645 2650 2655
   Asn Met Thr Pro Arg Asp Leu Gly Ala Cys Ile Asp Cys Asn Ala
   2660 2665 2670
   Arg His Ile Asn Ala Gln Val Ala Lys Ser His Asn Val Ser Leu
   2675 2680 2685
   Ile Trp Asn Val Lys Asp Tyr Met Ser Leu Ser Glu Gln Leu Arg
   2690 2695 2700
   Lys Gln Ile Arg Ser Ala Ala Lys Lys Asn Asn Ile Pro Phe Arg
   2705 2710 2715
   Leu Thr Cys Ala Thr Thr Arg Gln Val Val Asn Val Ile Thr Thr
   2720 2725 2730
   Lys Ile Ser Leu Lys Gly Gly Lys Ile Val Ser Thr Cys Phe Lys
   2735 2740 2745
   Leu Met Leu Lys Ala Thr Leu Leu Cys Val Leu Ala Ala Leu Val
   2750 2755 2760
   Cys Tyr Ile Val Met Pro Val His Thr Leu Ser Ile His Asp Gly
   2765 2770 2775
   Tyr Thr Asn Glu Ile Ile Gly Tyr Lys Ala Ile Gln Asp Gly Val
   2780 2785 2790
   Thr Arg Asp Ile Ile Ser Thr Asp Asp Cys Phe Ala Asn Lys His
   2795 2800 2805
   Ala Gly Phe Asp Ala Trp Phe Ser Gln Arg Gly Gly Ser Tyr Lys
   2810 2815 2820
   Asn Asp Lys Ser Cys Pro Val Val Ala Ala Ile Ile Thr Arg Glu
   2825 2830 2835
   Ile Gly Phe Ile Val Pro Gly Leu Pro Gly Thr Val Leu Arg Ala
   2840 2845 2850
   Ile Asn Gly Asp Phe Leu His Phe Leu Pro Arg Val Phe Ser Ala
   2855 2860 2865
   Val Gly Asn Ile Cys Tyr Thr Pro Ser Lys Leu Ile Glu Tyr Ser
   2870 2875 2880
   Asp Phe Ala Thr Ser Ala Cys Val Leu Ala Ala Glu Cys Thr Ile
   2885 2890 2895
   Phe Lys Asp Ala Met Gly Lys Pro Val Pro Tyr Cys Tyr Asp Thr
   2900 2905 2910
   Asn Leu Leu Glu Gly Ser Ile Ser Tyr Ser Glu Leu Arg Pro Asp
   2915 2920 2925
   Thr Arg Tyr Val Leu Met Asp Gly Ser Ile Ile Gln Phe Pro Asn
   2930 2935 2940
   Thr Tyr Leu Glu Gly Ser Val Arg Val Val Thr Thr Phe Asp Ala
   2945 2950 2955
   Glu Tyr Cys Arg His Gly Thr Cys Glu Arg Ser Glu Val Gly Ile
   2960 2965 2970
   Cys Leu Ser Thr Ser Gly Arg Trp Val Leu Asn Asn Glu His Tyr
   2975 2980 2985
   Arg Ala Leu Ser Gly Val Phe Cys Gly Val Asp Ala Met Asn Leu
   2990 2995 3000
   Ile Ala Asn Ile Phe Thr Pro Leu Val Gln Pro Val Gly Ala Leu
   3005 3010 3015
   Asp Val Ser Ala Ser Val Val Ala Gly Gly Ile Ile Ala Ile Leu
   3020 3025 3030
   Val Thr Cys Ala Ala Tyr Tyr Phe Met Lys Phe Arg Arg Val Phe
   3035 3040 3045
   Gly Glu Tyr Asn His Val Val Ala Ala Asn Ala Leu Leu Phe Leu
   3050 3055 3060
   Met Ser Phe Thr Ile Leu Cys Leu Val Pro Ala Tyr Ser Phe Leu
   3065 3070 3075
   Pro Gly Val Tyr Ser Val Phe Tyr Leu Tyr Leu Thr Phe Tyr Phe
   3080 3085 3090
   Thr Asn Asp Val Ser Phe Leu Ala His Leu Gln Trp Phe Ala Met
   3095 3100 3105
   Phe Ser Pro Ile Val Pro Phe Trp Ile Thr Ala Ile Tyr Val Phe
   3110 3115 3120
   Cys Ile Ser Leu Lys His Cys His Trp Phe Phe Asn Asn Tyr Leu
   3125 3130 3135
   Arg Lys Arg Val Met Phe Asn Gly Val Thr Phe Ser Thr Phe Glu
   3140 3145 3150
   Glu Ala Ala Leu Cys Thr Phe Leu Leu Asn Lys Glu Met Tyr Leu
   3155 3160 3165
   Lys Leu Arg Ser Glu Thr Leu Leu Pro Leu Thr Gln Tyr Asn Arg
   3170 3175 3180
   Tyr Leu Ala Leu Tyr Asn Lys Tyr Lys Tyr Phe Ser Gly Ala Leu
   3185 3190 3195
   Asp Thr Thr Ser Tyr Arg Glu Ala Ala Cys Cys His Leu Ala Lys
   3200 3205 3210
   Ala Leu Asn Asp Phe Ser Asn Ser Gly Ala Asp Val Leu Tyr Gln
   3215 3220 3225
   Pro Pro Gln Thr Ser Ile Thr Ser Ala Val Leu Gln Ser Gly Phe
   3230 3235 3240
   Arg Lys Met Ala Phe Pro Ser Gly Lys Val Glu Gly Cys Met Val
   3245 3250 3255
   Gln Val Thr Cys Gly Thr Thr Thr Leu Asn Gly Leu Trp Leu Asp
   3260 3265 3270
   Asp Thr Val Tyr Cys Pro Arg His Val Ile Cys Thr Ala Glu Asp
   3275 3280 3285
   Met Leu Asn Pro Asn Tyr Glu Asp Leu Leu Ile Arg Lys Ser Asn
   3290 3295 3300
   His Ser Phe Leu Val Gln Ala Gly Asn Val Gln Leu Arg Val Ile
   3305 3310 3315
   Gly His Ser Met Gln Asn Cys Leu Leu Arg Leu Lys Val Asp Thr
   3320 3325 3330
   Ser Asn Pro Lys Thr Pro Lys Tyr Lys Phe Val Arg Ile Gln Pro
   3335 3340 3345
   Gly Gln Thr Phe Ser Val Leu Ala Cys Tyr Asn Gly Ser Pro Ser
   3350 3355 3360
   Gly Val Tyr Gln Cys Ala Met Arg Pro Asn His Thr Ile Lys Gly
   3365 3370 3375
   Ser Phe Leu Asn Gly Ser Cys Gly Ser Val Gly Phe Asn Ile Asp
   3380 3385 3390
   Tyr Asp Cys Val Ser Phe Cys Tyr Met His His Met Glu Leu Pro
   3395 3400 3405
   Thr Gly Val His Ala Gly Thr Asp Leu Glu Gly Lys Phe Tyr Gly
   3410 3415 3420
   Pro Phe Val Asp Arg Gln Thr Ala Gln Ala Ala Gly Thr Asp Thr
   3425 3430 3435
   Thr Ile Thr Leu Asn Val Leu Ala Trp Leu Tyr Ala Ala Val Ile
   3440 3445 3450
   Asn Gly Asp Arg Trp Phe Leu Asn Arg Phe Thr Thr Thr Leu Asn
   3455 3460 3465
   Asp Phe Asn Leu Val Ala Met Lys Tyr Asn Tyr Glu Pro Leu Thr
   3470 3475 3480
   Gln Asp His Val Asp Ile Leu Gly Pro Leu Ser Ala Gln Thr Gly
   3485 3490 3495
   Ile Ala Val Leu Asp Met Cys Ala Ala Leu Lys Glu Leu Leu Gln
   3500 3505 3510
   Asn Gly Met Asn Gly Arg Thr Ile Leu Gly Ser Thr Ile Leu Glu
   3515 3520 3525
   Asp Glu Phe Thr Pro Phe Asp Val Val Arg Gln Cys Ser Gly Val
   3530 3535 3540
   Thr Phe Gln Gly Lys Phe Lys Lys Ile Val Lys Gly Thr His His
   3545 3550 3555
   Trp Met Leu Leu Thr Phe Leu Thr Ser Leu Leu Ile Leu Val Gln
   3560 3565 3570
   Ser Thr Gln Trp Ser Leu Phe Phe Phe Val Tyr Glu Asn Ala Phe
   3575 3580 3585
   Leu Pro Phe Thr Leu Gly Ile Met Ala Ile Ala Ala Cys Ala Met
   3590 3595 3600
   Leu Leu Val Lys His Lys His Ala Phe Leu Cys Leu Phe Leu Leu
   3605 3610 3615
   Pro Ser Leu Ala Thr Val Ala Tyr Phe Asn Met Val Tyr Met Pro
   3620 3625 3630
   Ala Ser Trp Val Met Arg Ile Met Thr Trp Leu Glu Leu Ala Asp
   3635 3640 3645
   Thr Ser Leu Ser Gly Tyr Arg Leu Lys Asp Cys Val Met Tyr Ala
   3650 3655 3660
   Ser Ala Leu Val Leu Leu Ile Leu Met Thr Ala Arg Thr Val Tyr
   3665 3670 3675
   Asp Asp Ala Ala Arg Arg Val Trp Thr Leu Met Asn Val Ile Thr
   3680 3685 3690
   Leu Val Tyr Lys Val Tyr Tyr Gly Asn Ala Leu Asp Gln Ala Ile
   3695 3700 3705
   Ser Met Trp Ala Leu Val Ile Ser Val Thr Ser Asn Tyr Ser Gly
   3710 3715 3720
   Val Val Thr Thr Ile Met Phe Leu Ala Arg Ala Ile Val Phe Val
   3725 3730 3735
   Cys Val Glu Tyr Tyr Pro Leu Leu Phe Ile Thr Gly Asn Thr Leu
   3740 3745 3750
   Gln Cys Ile Met Leu Val Tyr Cys Phe Leu Gly Tyr Cys Cys Cys
   3755 3760 3765
   Cys Tyr Phe Gly Leu Phe Cys Leu Leu Asn Arg Tyr Phe Arg Leu
   3770 3775 3780
   Thr Leu Gly Val Tyr Asp Tyr Leu Val Ser Thr Gln Glu Phe Arg
   3785 3790 3795
   Tyr Met Asn Ser Gln Gly Leu Leu Pro Pro Lys Ser Ser Ile Asp
   3800 3805 3810
   Ala Phe Lys Leu Asn Ile Lys Leu Leu Gly Ile Gly Gly Lys Pro
   3815 3820 3825
   Cys Ile Lys Val Ala Thr Val Gln Ser Lys Met Ser Asp Val Lys
   3830 3835 3840
   Cys Thr Ser Val Val Leu Leu Ser Val Leu Gln Gln Leu Arg Val
   3845 3850 3855
   Glu Ser Ser Ser Lys Leu Trp Ala Gln Cys Val Gln Leu His Asn
   3860 3865 3870
   Asp Ile Leu Leu Ala Lys Asp Thr Thr Glu Ala Phe Glu Lys Met
   3875 3880 3885
   Val Ser Leu Leu Ser Val Leu Leu Ser Met Gln Gly Ala Val Asp
   3890 3895 3900
   Ile Asn Arg Leu Cys Glu Glu Met Leu Asp Asn Arg Ala Thr Leu
   3905 3910 3915
   Gln Ala Ile Ala Ser Glu Phe Ser Ser Leu Pro Ser Tyr Ala Ala
   3920 3925 3930
   Tyr Ala Thr Ala Gln Glu Ala Tyr Glu Gln Ala Val Ala Asn Gly
   3935 3940 3945
   Asp Ser Glu Val Val Leu Lys Lys Leu Lys Lys Ser Leu Asn Val
   3950 3955 3960
   Ala Lys Ser Glu Phe Asp Arg Asp Ala Ala Met Gln Arg Lys Leu
   3965 3970 3975
   Glu Lys Met Ala Asp Gln Ala Met Thr Gln Met Tyr Lys Gln Ala
   3980 3985 3990
   Arg Ser Glu Asp Lys Arg Ala Lys Val Thr Ser Ala Met Gln Thr
   3995 4000 4005
   Met Leu Phe Thr Met Leu Arg Lys Leu Asp Asn Asp Ala Leu Asn
   4010 4015 4020
   Asn Ile Ile Asn Asn Ala Arg Asp Gly Cys Val Pro Leu Asn Ile
   4025 4030 4035
   Ile Pro Leu Thr Thr Ala Ala Lys Leu Met Val Val Val Pro Asp
   4040 4045 4050
   Tyr Gly Thr Tyr Lys Asn Thr Cys Asp Gly Asn Thr Phe Thr Tyr
   4055 4060 4065
   Ala Ser Ala Leu Trp Glu Ile Gln Gln Val Val Asp Ala Asp Ser
   4070 4075 4080
   Lys Ile Val Gln Leu Ser Glu Ile Asn Met Asp Asn Ser Pro Asn
   4085 4090 4095
   Leu Ala Trp Pro Leu Ile Val Thr Ala Leu Arg Ala Asn Ser Ala
   4100 4105 4110
   Val Lys Leu Gln Asn Asn Glu Leu Ser Pro Val Ala Leu Arg Gln
   4115 4120 4125
   Met Ser Cys Ala Ala Gly Thr Thr Gln Thr Ala Cys Thr Asp Asp
   4130 4135 4140
   Asn Ala Leu Ala Tyr Tyr Asn Asn Ser Lys Gly Gly Arg Phe Val
   4145 4150 4155
   Leu Ala Leu Leu Ser Asp His Gln Asp Leu Lys Trp Ala Arg Phe
   4160 4165 4170
   Pro Lys Ser Asp Gly Thr Gly Thr Ile Tyr Thr Glu Leu Glu Pro
   4175 4180 4185
   Pro Cys Arg Phe Val Thr Asp Thr Pro Lys Gly Pro Lys Val Lys
   4190 4195 4200
   Tyr Leu Tyr Phe Ile Lys Gly Leu Asn Asn Leu Asn Arg Gly Met
   4205 4210 4215
   Val Leu Gly Ser Leu Ala Ala Thr Val Arg Leu Gln Ala Gly Asn
   4220 4225 4230
   Ala Thr Glu Val Pro Ala Asn Ser Thr Val Leu Ser Phe Cys Ala
   4235 4240 4245
   Phe Ala Val Asp Pro Ala Lys Ala Tyr Lys Asp Tyr Leu Ala Ser
   4250 4255 4260
   Gly Gly Gln Pro Ile Thr Asn Cys Val Lys Met Leu Cys Thr His
   4265 4270 4275
   Thr Gly Thr Gly Gln Ala Ile Thr Val Thr Pro Glu Ala Asn Met
   4280 4285 4290
   Asp Gln Glu Ser Phe Gly Gly Ala Ser Cys Cys Leu Tyr Cys Arg
   4295 4300 4305
   Cys His Ile Asp His Pro Asn Pro Lys Gly Phe Cys Asp Leu Lys
   4310 4315 4320
   Gly Lys Tyr Val Gln Ile Pro Thr Thr Cys Ala Asn Asp Pro Val
   4325 4330 4335
   Gly Phe Thr Leu Arg Asn Thr Val Cys Thr Val Cys Gly Met Trp
   4340 4345 4350
   Lys Gly Tyr Gly Cys Ser Cys Asp Gln Leu Arg Glu Pro Leu Met
   4355 4360 4365
   Gln Ser Ala Asp Ala Ser Thr Phe Leu Asn Gly Phe Ala Val
   4370 4375 4380
<210> 75
   <211> 2695
   <212> PRT
   <213> CORONAVIRUS
<400> 75
   Arg Val Cys Gly Val Ser Ala Ala Arg Leu Thr Pro Cys Gly Thr Gly
   1               5                   10                  15
   Thr Ser Thr Asp Val Val Tyr Arg Ala Phe Asp Ile Tyr Asn Glu Lys
               20                  25                  30
   Val Ala Gly Phe Ala Lys Phe Leu Lys Thr Asn Cys Cys Arg Phe Gln
           35                  40                  45
   Glu Lys Asp Glu Glu Gly Asn Leu Leu Asp Ser Tyr Phe Val Val Lys
       50                  55                  60
   Arg His Thr Met Ser Asn Tyr Gln His Glu Glu Thr Ile Tyr Asn Leu
   65                  70                  75                  80
   Val Lys Asp Cys Pro Ala Val Ala Val His Asp Phe Phe Lys Phe Arg
                   85                  90                  95
   Val Asp Gly Asp Met Val Pro His Ile Ser Arg Gln Arg Leu Thr Lys
               100                 105                 110
   Tyr Thr Met Ala Asp Leu Val Tyr Ala Leu Arg His Phe Asp Glu Gly
           115                 120                 125
   Asn Cys Asp Thr Leu Lys Glu Ile Leu Val Thr Tyr Asn Cys Cys Asp
       130                 135                 140
   Asp Asp Tyr Phe Asn Lys Lys Asp Trp Tyr Asp Phe Val Glu Asn Pro
   145                 150                 155                 160
   Asp Ile Leu Arg Val Tyr Ala Asn Leu Gly Glu Arg Val Arg Gln Ser
                   165                 170                 175
   Leu Leu Lys Thr Val Gln Phe Cys Asp Ala Met Arg Asp Ala Gly Ile
               180                 185                 190
   Val Gly Val Leu Thr Leu Asp Asn Gln Asp Leu Asn Gly Asn Trp Tyr
           195                 200                 205
   Asp Phe Gly Asp Phe Val Gln Val Ala Pro Gly Cys Gly Val Pro Ile
       210                 215                 220
   Val Asp Ser Tyr Tyr Ser Leu Leu Met Pro Ile Leu Thr Leu Thr Arg
   225                 230                 235                 240
   Ala Leu Ala Ala Glu Ser His Met Asp Ala Asp Leu Ala Lys Pro Leu
                   245                 250                 255
   Ile Lys Trp Asp Leu Leu Lys Tyr Asp Phe Thr Glu Glu Arg Leu Cys
   260 265 270
   Leu Phe Asp Arg Tyr Phe Lys Tyr Trp Asp Gln Thr Tyr His Pro Asn
   275 280 285
   Cys Ile Asn Cys Leu Asp Asp Arg Cys Ile Leu His Cys Ala Asn Phe
   290 295 300
   Asn Val Leu Phe Ser Thr Val Phe Pro Pro Thr Ser Phe Gly Pro Leu
   305 310 315 320
   Val Arg Lys Ile Phe Val Asp Gly Val Pro Phe Val Val Ser Thr Gly
   325 330 335
   Tyr His Phe Arg Glu Leu Gly Val Val His Asn Gln Asp Val Asn Leu
   340 345 350
   His Ser Ser Arg Leu Ser Phe Lys Glu Leu Leu Val Tyr Ala Ala Asp
   355 360 365
   Pro Ala Met His Ala Ala Ser Gly Asn Leu Leu Leu Asp Lys Arg Thr
   370 375 380
   Thr Cys Phe Ser Val Ala Ala Leu Thr Asn Asn Val Ala Phe Gln Thr
   385 390 395 400
   Val Lys Pro Gly Asn Phe Asn Lys Asp Phe Tyr Asp Phe Ala Val Ser
   405 410 415
   Lys Gly Phe Phe Lys Glu Gly Ser Ser Val Glu Leu Lys His Phe Phe
   420 425 430
   Phe Ala Gln Asp Gly Asn Ala Ala Ile Ser Asp Tyr Asp Tyr Tyr Arg
   435 440 445
   Tyr Asn Leu Pro Thr Met Cys Asp Ile Arg Gln Leu Leu Phe Val Val
   450 455 460
   Glu Val Val Asp Lys Tyr Phe Asp Cys Tyr Asp Gly Gly Cys Ile Asn
   465 470 475 480
   Ala Asn Gln Val Ile Val Asn Asn Leu Asp Lys Ser Ala Gly Phe Pro
   485 490 495
   Phe Asn Lys Trp Gly Lys Ala Arg Leu Tyr Tyr Asp Ser Met Ser Tyr
   500 505 510
   Glu Asp Gln Asp Ala Leu Phe Ala Tyr Thr Lys Arg Asn Val Ile Pro
   515 520 525
   Thr Ile Thr Gln Met Asn Leu Lys Tyr Ala Ile Ser Ala Lys Asn Arg
   530 535 540
   Ala Arg Thr Val Ala Gly Val Ser Ile Cys Ser Thr Met Thr Asn Arg
   545 550 555 560
   Gln Phe His Gln Lys Leu Leu Lys Ser Ile Ala Ala Thr Arg Gly Ala
   565 570 575
   Thr Val Val Ile Gly Thr Ser Lys Phe Tyr Gly Gly Trp His Asn Met
   580 585 590
   Leu Lys Thr Val Tyr Ser Asp Val Glu Thr Pro His Leu Met Gly Trp
   595 600 605
   Asp Tyr Pro Lys Cys Asp Arg Ala Met Pro Asn Met Leu Arg Ile Met
   610 615 620
   Ala Ser Leu Val Leu Ala Arg Lys His Asn Thr Cys Cys Asn Leu Ser
   625 630 635 640
   His Arg Phe Tyr Arg Leu Ala Asn Glu Cys Ala Gln Val Leu Ser Glu
   645 650 655
   Met Val Met Cys Gly Gly Ser Leu Tyr Val Lys Pro Gly Gly Thr Ser
   660 665 670
   Ser Gly Asp Ala Thr Thr Ala Tyr Ala Asn Ser Val Phe Asn Ile Cys
   675 680 685
   Gln Ala Val Thr Ala Asn Val Asn Ala Leu Leu Ser Thr Asp Gly Asn
   690 695 700
   Lys Ile Ala Asp Lys Tyr Val Arg Asn Leu Gln His Arg Leu Tyr Glu
   705 710 715 720
   Cys Leu Tyr Arg Asn Arg Asp Val Asp His Glu Phe Val Asp Glu Phe
   725 730 735
   Tyr Ala Tyr Leu Arg Lys His Phe Ser Met Met Ile Leu Ser Asp Asp
   740 745 750
   Ala Val Val Cys Tyr Asn Ser Asn Tyr Ala Ala Gln Gly Leu Val Ala
   755 760 765
   Ser Ile Lys Asn Phe Lys Ala Val Leu Tyr Tyr Gln Asn Asn Val Phe
   770 775 780
   Met Ser Glu Ala Lys Cys Trp Thr Glu Thr Asp Leu Thr Lys Gly Pro
   785 790 795 800
   His Glu Phe Cys Ser Gln His Thr Met Leu Val Lys Gln Gly Asp Asp
   805 810 815
   Tyr Val Tyr Leu Pro Tyr Pro Asp Pro Ser Arg Ile Leu Gly Ala Gly
   820 825 830
   Cys Phe Val Asp Asp Ile Val Lys Thr Asp Gly Thr Leu Met Ile Glu
   835 840 845
   Arg Phe Val Ser Leu Ala Ile Asp Ala Tyr Pro Leu Thr Lys His Pro
   850 855 860
   Asn Gln Glu Tyr Ala Asp Val Phe His Leu Tyr Leu Gln Tyr Ile Arg
   865 870 875 880
   Lys Leu His Asp Glu Leu Thr Gly His Met Leu Asp Met Tyr Ser Val
   885 890 895
   Met Leu Thr Asn Asp Asn Thr Ser Arg Tyr Trp Glu Pro Glu Phe Tyr
   900 905 910
   Glu Ala Met Tyr Thr Pro His Thr Val Leu Gln Ala Val Gly Ala Cys
   915 920 925
   Val Leu Cys Asn Ser Gln Thr Ser Leu Arg Cys Gly Ala Cys Ile Arg
   930 935 940
   Arg Pro Phe Leu Cys Cys Lys Cys Cys Tyr Asp His Val Ile Ser Thr
   945 950 955 960
   Ser His Lys Leu Val Leu Ser Val Asn Pro Tyr Val Cys Asn Ala Pro
   965 970 975
   Gly Cys Asp Val Thr Asp Val Thr Gln Leu Tyr Leu Gly Gly Met Ser
   980 985 990
   Tyr Tyr Cys Lys Ser His Lys Pro Pro Ile Ser Phe Pro Leu Cys Ala
   995 1000 1005
   Asn Gly Gln Val Phe Gly Leu Tyr Lys Asn Thr Cys Val Gly Ser
   1010 1015 1020
   Asp Asn Val Thr Asp Phe Asn Ala Ile Ala Thr Cys Asp Trp Thr
   1025 1030 1035
   Asn Ala Gly Asp Tyr Ile Leu Ala Asn Thr Cys Thr Glu Arg Leu
   1040 1045 1050
   Lys Leu Phe Ala Ala Glu Thr Leu Lys Ala Thr Glu Glu Thr Phe
   1055 1060 1065
   Lys Leu Ser Tyr Gly Ile Ala Thr Val Arg Glu Val Leu Ser Asp
   1070 1075 1080
   Arg Glu Leu His Leu Ser Trp Glu Val Gly Lys Pro Arg Pro Pro
   1085 1090 1095
   Leu Asn Arg Asn Tyr Val Phe Thr Gly Tyr Arg Val Thr Lys Asn
   1100 1105 1110
   Ser Lys Val Gln Ile Gly Glu Tyr Thr Phe Glu Lys Gly Asp Tyr
   1115 1120 1125
   Gly Asp Ala Val Val Tyr Arg Gly Thr Thr Thr Tyr Lys Leu Asn
   1130 1135 1140
   Val Gly Asp Tyr Phe Val Leu Thr Ser His Thr Val Met Pro Leu
   1145 1150 1155
   Ser Ala Pro Thr Leu Val Pro Gln Glu His Tyr Val Arg Ile Thr
   1160 1165 1170
   Gly Leu Tyr Pro Thr Leu Asn Ile Ser Asp Glu Phe Ser Ser Asn
   1175 1180 1185
   Val Ala Asn Tyr Gln Lys Val Gly Met Gln Lys Tyr Ser Thr Leu
   1190 1195 1200
   Gln Gly Pro Pro Gly Thr Gly Lys Ser His Phe Ala Ile Gly Leu
   1205 1210 1215
   Ala Leu Tyr Tyr Pro Ser Ala Arg Ile Val Tyr Thr Ala Cys Ser
   1220 1225 1230
   His Ala Ala Val Asp Ala Leu Cys Glu Lys Ala Leu Lys Tyr Leu
   1235 1240 1245
   Pro Ile Asp Lys Cys Ser Arg Ile Ile Pro Ala Arg Ala Arg Val
   1250 1255 1260
   Glu Cys Phe Asp Lys Phe Lys Val Asn Ser Thr Leu Glu Gln Tyr
   1265 1270 1275
   Val Phe Cys Thr Val Asn Ala Leu Pro Glu Thr Thr Ala Asp Ile
   1280 1285 1290
   Val Val Phe Asp Glu Ile Ser Met Ala Thr Asn Tyr Asp Leu Ser
   1295 1300 1305
   Val Val Asn Ala Arg Leu Arg Ala Lys His Tyr Val Tyr Ile Gly
   1310 1315 1320
   Asp Pro Ala Gln Leu Pro Ala Pro Arg Thr Leu Leu Thr Lys Gly
   1325 1330 1335
   Thr Leu Glu Pro Glu Tyr Phe Asn Ser Val Cys Arg Leu Met Lys
   1340 1345 1350
   Thr Ile Gly Pro Asp Met Phe Leu Gly Thr Cys Arg Arg Cys Pro
   1355 1360 1365
   Ala Glu Ile Val Asp Thr Val Ser Ala Leu Val Tyr Asp Asn Lys
   1370 1375 1380
   Leu Lys Ala His Lys Asp Lys Ser Ala Gln Cys Phe Lys Met Phe
   1385 1390 1395
   Tyr Lys Gly Val Ile Thr His Asp Val Ser Ser Ala Ile Asn Arg
   1400 1405 1410
   Pro Gln Ile Gly Val Val Arg Glu Phe Leu Thr Arg Asn Pro Ala
   1415 1420 1425
   Trp Arg Lys Ala Val Phe Ile Ser Pro Tyr Asn Ser Gln Asn Ala
   1430 1435 1440
   Val Ala Ser Lys Ile Leu Gly Leu Pro Thr Gln Thr Val Asp Ser
   1445 1450 1455
   Ser Gln Gly Ser Glu Tyr Asp Tyr Val Ile Phe Thr Gln Thr Thr
   1460 1465 1470
   Glu Thr Ala His Ser Cys Asn Val Asn Arg Phe Asn Val Ala Ile
   1475 1480 1485
   Thr Arg Ala Lys Ile Gly Ile Leu Cys Ile Met Ser Asp Arg Asp
   1490 1495 1500
   Leu Tyr Asp Lys Leu Gln Phe Thr Ser Leu Glu Ile Pro Arg Arg
   1505 1510 1515
   Asn Val Ala Thr Leu Gln Ala Glu Asn Val Thr Gly Leu Phe Lys
   1520 1525 1530
   Asp Cys Ser Lys Ile Ile Thr Gly Leu His Pro Thr Gln Ala Pro
   1535 1540 1545
   Thr His Leu Ser Val Asp Ile Lys Phe Lys Thr Glu Gly Leu Cys
   1550 1555 1560
   Val Asp Ile Pro Gly Ile Pro Lys Asp Met Thr Tyr Arg Arg Leu
   1565 1570 1575
   Ile Ser Met Met Gly Phe Lys Met Asn Tyr Gln Val Asn Gly Tyr
   1580 1585 1590
   Pro Asn Met Phe Ile Thr Arg Glu Glu Ala Ile Arg His Val Arg
   1595 1600 1605
   Ala Trp Ile Gly Phe Asp Val Glu Gly Cys His Ala Thr Arg Asp
   1610 1615 1620
   Ala Val Gly Thr Asn Leu Pro Leu Gln Leu Gly Phe Ser Thr Gly
   1625 1630 1635
   Val Asn Leu Val Ala Val Pro Thr Gly Tyr Val Asp Thr Glu Asn
   1640 1645 1650
   Asn Thr Glu Phe Thr Arg Val Asn Ala Lys Pro Pro Pro Gly Asp
   1655 1660 1665
   Gln Phe Lys His Leu Ile Pro Leu Met Tyr Lys Gly Leu Pro Trp
   1670 1675 1680
   Asn Val Val Arg Ile Lys Ile Val Gln Met Leu Ser Asp Thr Leu
   1685 1690 1695
   Lys Gly Leu Ser Asp Arg Val Val Phe Val Leu Trp Ala His Gly
   1700 1705 1710
   Phe Glu Leu Thr Ser Met Lys Tyr Phe Val Lys Ile Gly Pro Glu
   1715 1720 1725
   Arg Thr Cys Cys Leu Cys Asp Lys Arg Ala Thr Cys Phe Ser Thr
   1730 1735 1740
   Ser Ser Asp Thr Tyr Ala Cys Trp Asn His Ser Val Gly Phe Asp
   1745 1750 1755
   Tyr Val Tyr Asn Pro Phe Met Ile Asp Val Gln Gln Trp Gly Phe
   1760 1765 1770
   Thr Gly Asn Leu Gln Ser Asn His Asp Gln His Cys Gln Val His
   1775 1780 1785
   Gly Asn Ala His Val Ala Ser Cys Asp Ala Ile Met Thr Arg Cys
   1790 1795 1800
   Leu Ala Val His Glu Cys Phe Val Lys Arg Val Asp Trp Ser Val
   1805 1810 1815
   Glu Tyr Pro Ile Ile Gly Asp Glu Leu Arg Val Asn Ser Ala Cys
   1820 1825 1830
   Arg Lys Val Gln His Met Val Val Lys Ser Ala Leu Leu Ala Asp
   1835 1840 1845
   Lys Phe Pro Val Leu His Asp Ile Gly Asn Pro Lys Ala Ile Lys
   1850 1855 1860
   Cys Val Pro Gln Ala Glu Val Glu Trp Lys Phe Tyr Asp Ala Gln
   1865 1870 1875
   Pro Cys Ser Asp Lys Ala Tyr Lys Ile Glu Glu Leu Phe Tyr Ser
   1880 1885 1890
   Tyr Ala Thr His His Asp Lys Phe Thr Asp Gly Val Cys Leu Phe
   1895 1900 1905
   Trp Asn Cys Asn Val Asp Arg Tyr Pro Ala Asn Ala Ile Val Cys
   1910 1915 1920
   Arg Phe Asp Thr Arg Val Leu Ser Asn Leu Asn Leu Pro Gly Cys
   1925 1930 1935
   Asp Gly Gly Ser Leu Tyr Val Asn Lys His Ala Phe His Thr Pro
   1940 1945 1950
   Ala Phe Asp Lys Ser Ala Phe Thr Asn Leu Lys Gln Leu Pro Phe
   1955 1960 1965
   Phe Tyr Tyr Ser Asp Ser Pro Cys Glu Ser His Gly Lys Gln Val
   1970 1975 1980
   Val Ser Asp Ile Asp Tyr Val Pro Leu Lys Ser Ala Thr Cys Ile
   1985 1990 1995
   Thr Arg Cys Asn Leu Gly Gly Ala Val Cys Arg His His Ala Asn
   2000 2005 2010
   Glu Tyr Arg Gln Tyr Leu Asp Ala Tyr Asn Met Met Ile Ser Ala
   2015 2020 2025
   Gly Phe Ser Leu Trp Ile Tyr Lys Gln Phe Asp Thr Tyr Asn Leu
   2030 2035 2040
   Trp Asn Thr Phe Thr Arg Leu Gln Ser Leu Glu Asn Val Ala Tyr
   2045 2050 2055
   Asn Val Val Asn Lys Gly His Phe Asp Gly His Ala Gly Glu Ala
   2060 2065 2070
   Pro Val Ser Ile Ile Asn Asn Ala Val Tyr Thr Lys Val Asp Gly
   2075 2080 2085
   Ile Asp Val Glu Ile Phe Glu Asn Lys Thr Thr Leu Pro Val Asn
   2090 2095 2100
   Val Ala Phe Glu Leu Trp Ala Lys Arg Asn Ile Lys Pro Val Pro
   2105 2110 2115
   Glu Ile Lys Ile Leu Asn Asn Leu Gly Val Asp Ile Ala Ala Asn
   2120 2125 2130
   Thr Val Ile Trp Asp Tyr Lys Arg Glu Ala Pro Ala His Val Ser
   2135 2140 2145
   Thr Ile Gly Val Cys Thr Met Thr Asp Ile Ala Lys Lys Pro Thr
   2150 2155 2160
   Glu Ser Ala Cys Ser Ser Leu Thr Val Leu Phe Asp Gly Arg Val
   2165 2170 2175
   Glu Gly Gln Val Asp Leu Phe Arg Asn Ala Arg Asn Gly Val Leu
   2180 2185 2190
   Ile Thr Glu Gly Ser Val Lys Gly Leu Thr Pro Ser Lys Gly Pro
   2195 2200 2205
   Ala Gln Ala Ser Val Asn Gly Val Thr Leu Ile Gly Glu Ser Val
   2210 2215 2220
   Lys Thr Gln Phe Asn Tyr Phe Lys Lys Val Asp Gly Ile Ile Gln
   2225 2230 2235
   Gln Leu Pro Glu Thr Tyr Phe Thr Gln Ser Arg Asp Leu Glu Asp
   2240 2245 2250
   Phe Lys Pro Arg Ser Gln Met Glu Thr Asp Phe Leu Glu Leu Ala
   2255 2260 2265
   Met Asp Glu Phe Ile Gln Arg Tyr Lys Leu Glu Gly Tyr Ala Phe
   2270 2275 2280
   Glu His Ile Val Tyr Gly Asp Phe Ser His Gly Gln Leu Gly Gly
   2285 2290 2295
   Leu His Leu Met Ile Gly Leu Ala Lys Arg Ser Gln Asp Ser Pro
   2300 2305 2310
   Leu Lys Leu Glu Asp Phe Ile Pro Met Asp Ser Thr Val Lys Asn
   2315 2320 2325
   Tyr Phe Ile Thr Asp Ala Gln Thr Gly Ser Ser Lys Cys Val Cys
   2330 2335 2340
   Ser Val Ile Asp Leu Leu Leu Asp Asp Phe Val Glu Ile Ile Lys
   2345 2350 2355
   Ser Gln Asp Leu Ser Val Ile Ser Lys Val Val Lys Val Thr Ile
   2360 2365 2370
   Asp Tyr Ala Glu Ile Ser Phe Met Leu Trp Cys Lys Asp Gly His
   2375 2380 2385
   Val Glu Thr Phe Tyr Pro Lys Leu Gln Ala Ser Gln Ala Trp Gln
   2390 2395 2400
   Pro Gly Val Ala Met Pro Asn Leu Tyr Lys Met Gln Arg Met Leu
   2405 2410 2415
   Leu Glu Lys Cys Asp Leu Gln Asn Tyr Gly Glu Asn Ala Val Ile
   2420 2425 2430
   Pro Lys Gly Ile Met Met Asn Val Ala Lys Tyr Thr Gln Leu Cys
   2435 2440 2445
   Gln Tyr Leu Asn Thr Leu Thr Leu Ala Val Pro Tyr Asn Met Arg
   2450 2455 2460
   Val Ile His Phe Gly Ala Gly Ser Asp Lys Gly Val Ala Pro Gly
   2465 2470 2475
   Thr Ala Val Leu Arg Gln Trp Leu Pro Thr Gly Thr Leu Leu Val
   2480 2485 2490
   Asp Ser Asp Leu Asn Asp Phe Val Ser Asp Ala Asp Ser Thr Leu
   2495 2500 2505
   Ile Gly Asp Cys Ala Thr Val His Thr Ala Asn Lys Trp Asp Leu
   2510 2515 2520
   Ile Ile Ser Asp Met Tyr Asp Pro Arg Thr Lys His Val Thr Lys
   2525 2530 2535
   Glu Asn Asp Ser Lys Glu Gly Phe Phe Thr Tyr Leu Cys Gly Phe
   2540 2545 2550
   Ile Lys Gln Lys Leu Ala Leu Gly Gly Ser Ile Ala Val Lys Ile
   2555 2560 2565
   Thr Glu His Ser Trp Asn Ala Asp Leu Tyr Lys Leu Met Gly His
   2570 2575 2580
   Phe Ser Trp Trp Thr Ala Phe Val Thr Asn Val Asn Ala Ser Ser
   2585 2590 2595
   Ser Glu Ala Phe Leu Ile Gly Ala Asn Tyr Leu Gly Lys Pro Lys
   2600 2605 2610
   Glu Gln Ile Asp Gly Tyr Thr Met His Ala Asn Tyr Ile Phe Trp
   2615 2620 2625
   Arg Asn Thr Asn Pro Ile Gln Leu Ser Ser Tyr Ser Leu Phe Asp
   2630 2635 2640
   Met Ser Lys Phe Pro Leu Lys Leu Arg Gly Thr Ala Val Met Ser
   2645 2650 2655
   Leu Lys Glu Asn Gln Ile Asn Asp Met Ile Tyr Ser Leu Leu Glu
   2660 2665 2670
   Lys Gly Arg Leu Ile Ile Arg Glu Asn Asn Arg Val Val Val Ser
   2675 2680 2685
   Ser Asp Ile Leu Val Asn Asn
   2690 2695
<210> 76
   <211> 20
   <212> DNA
   <213> Artificial sequence
<220>
   <223> amorce S/L3/+/4932
<400> 76
   ccacacacag cttgtggata 20
<210> 77
   <211> 20
   <212> DNA
   <213> Artificial sequence
<220>
   <223> amorce S/L4/+/6401
<400> 77
   ccgaagttgt aggcaatgtc 20
<210> 78
   <211> 20
   <212> DNA
   <213> Artificial sequence
<220>
   <223> amorce S/L4/+/6964
<400> 78 20
   tttggtgctc cttcttattg 20
<210> 79
   <211> 20
   <212> DNA
   <213> Artificial sequence
<220>
   <223> amorce S/L4/-/6817
<400> 79 20
   ccggcatcca aacataattt 20
<210> 80
   <211> 20
   <212> DNA
   <213> Artificial sequence
<220>
   <223> amorce S/L5/-/7633
<400> 80
   tggtcagtag ggttgattgg 20
<210> 81
   <211> 20
   <212> DNA
   <213> Artificial sequence
<220>
   <223> S/L5/-/8127
<400> 81
   catcctttgt gtcaacatcg 20
<210> 82
   <211> 20
   <212> DNA
   <213> Artificial sequence
<220>
   <223> amorce S/L5/-/8633
<400> 82
   gtcacgagtg acaccatcct 20
<210> 83
   <211> 20
   <212> DNA
   <213> Artificial sequence
<220>
   <223> amorce S/L5/+/7839
<400> 83
   atgcgacgag tctgcttcta 20
<210> 84
   <211> 20
   <212> DNA
   <213> Artificial sequence
<220>
   <223> amorce S/L5/+/8785
<400> 84
   ttcatagtgc ctggcttacc 20
<210> 85
   <211> 20
   <212> DNA
   <213> Artificial sequence
<220>
   <223> amorce S/L5/+/8255
<400> 85
   atcttggcgc atgtattgac 20
<210> 86
   <211> 20
   <212> DNA
   <213> Artificial sequence
<220>
   <223> amorce S/L6/-/9422
<400> 86
   tgcattagca gcaacaacat 20
<210> 87
   <211> 20
   <212> DNA
   <213> Artificial sequence
<220>
   <223> amorce 5/L6/-/9966
<400> 87
   tctgcagaac agcagaagtg 20
<210> 88
   <211> 20
   <212> DNA
   <213> Artificial sequence
<220>
   <223> amorce S/L6/-/10542
<400> 88
   cctgtgcagt ttgtctgtca 20
<210> 89
   <211> 20
   <212> DNA
   <213> Artificial sequence
<220>
   <223> amorce S/L6/+/10677
<400> 89
   ccttgtggca atgaagtaca 20
<210> 90
   <211> 20
   <212> DNA
   <213> Artificial sequence
<220>
   <223> amorce S/L6/+/10106
<400> 90
   atgtcatttg cacagcagaa 20
<210> 91
   <211> 20
   <212> DNA
   <213> Artificial sequence
<220>
   <223> amorce S/L6/+/9571
<400> 91
   cttcaatggt ttgccatgtt 20
<210> 92
   <211> 20
   <212> DNA
   <213> Artificial sequence
<220>
   <223> amorce S/L7/-/11271
<400> 92
   tgcgagctgt catgagaata 20
<210> 93
   <211> 20
   <212> DNA
   <213> Artificial sequence
<220>
   <223> amorce S/L7/-/11801
<400> 93
   aaccgagagc agtaccacag 20
<210> 94
   <211> 20
   <212> DNA
   <213> Artificial sequence
<220>
   <223> amorce S/L7/-/12383
<400> 94
   tttggctgct gtagtcaatg 20
<210> 95
   <211> 20
   <212> DNA
   <213> Artificial sequence
<220>
   <223> amorce S/L7/+/12640
<400> 95
   ctacgacaga tgtcctgtgc 20
<210> 96
   <211> 20
   <212> DNA
   <213> Artificial sequence
<220>
   <223> amorce S/L7/+/12088
<400> 96
   gagcaggctg tagctaatgg 20
<210> 97
   <211> 20
   <212> DNA
   <213> Artificial sequence
<220>
   <223> amorce S/L7/+/11551
<400> 97
   ttaggctatt gttgctgctg 20
<210> 98
   <211> 20
   <212> DNA
   <213> Artificial sequence
<220>
   <223> amorce S/L8/-/13160
<400> 98
   cagacaacat gaagcaccac 20
<210> 99
   <211> 20
   <212> DNA
   <213> Artificial sequence
<220>
   <223> amorce S/L8/-/13704
<400> 99
   cgctgacgtg atatatgtgg 20
<210> 100
   <211> 20
   <212> DNA
   <213> Artificial sequence
<220>
   <223> amorce S/L8/-/14284
<400> 100
   tgcacaatga aggatacacc 20
<210> 101
   <211> 20
   <212> DNA
   <213> Artificial sequence
<220>
   <223> amorce S/L8/+/14453
<400> 101
   acatagctcg cgtctcagtt 20
<210> 102
   <211> 20
   <212> DNA
   <213> Artificial sequence
<220>
   <223> amorce S/L8/+/13968
<400> 102
   ggcattgtag gcgtactgac 20
<210> 103
   <211> 19
   <212> DNA
   <213> Artificial sequence
<220>
   <223> amorce S/L8/+/13401
<400> 103
   gtttgcggtg taagtgcag 19
<210> 104
   <211> 20
   <212> DNA
   <213> Artificial sequence
<220>
   <223> amorce S/L9/-/15098
<400> 104
   tagtggcggc tattgacttc 20
<210> 105
   <211> 20
   <212> DNA
   <213> Artificial sequence
<220>
   <223> amorce S/L9/-/15677
<400> 105
   ctaaaccttg agccgcatag 20
<210> 106
   <211> 20
   <212> DNA
   <213> Artificial sequence
<220>
   <223> amorce S/L9/-/16247
<400> 106
   catggtcata gcagcacttg 20
<210> 107
   <211> 21
   <212> DNA
   <213> Artificial sequence
<220>
   <223> amorce S/L9/+/16323
<400> 107
   ccaggttgtg atgtcactga t 21
<210> 108
   <211> 20
   <212> DNA
   <213> Artificial sequence
<220>
   <223> amorce S/L9/+/15858
<400> 108
   ccttacccag atccatcaag 20
<210> 109
   <211> 20
   <212> DNA
   <213> Artificial sequence
<220>
   <223> amorce S/L9/+/15288
<400> 109
   cgcaaacata acacttgctg 20
<210> 110
   <211> 20
   <212> DNA
   <213> Artificial sequence
<220>
   <223> amorce S/L10/-/16914
<400> 110
   agtgttgggt acaagccagt 20
<210> 111
   <211> 20
   <212> DNA
   <213> Artificial sequence
<220>
   <223> amorce S/L10/-/17466
<400> 111
   gttccaagga acatgtctgg 20
<210> 112
   <211> 20
   <212> DNA
   <213> Artificial sequence
<220>
   <223> amorce S/L10/-/18022
<400> 112
   aggtgcctgt gtaggatgaa 20
<210> 113
   <211> 20
   <212> DNA
   <213> Artificial sequence
<220>
   <223> amorce S/L10/+/18245
<400> 113
   gggctgtcat gcaactagag 20
<210> 114
   <211> 20
   <212> DNA
   <213> Artificial sequence
<220>
   <223> amorce S/L10/+/17663
<400> 114
   tcttacacgc aatcctgctt 20
<210> 115
   <211> 20
   <212> DNA
   <213> Artificial sequence
<220>
   <223> amorce S/L10/+/17061
<400> 115
   tacccatctg ctcgcatagt 20
<210> 116
   <211> 20
   <212> DNA
   <213> Artificial sequence
<220>
   <223> amorce S/L11/-/18877
<400> 116
   gcaagcagaa ttaaccctca 20
<210> 117
   <211> 20
   <212> DNA
   <213> Artificial sequence
<220>
   <223> amorce S/L11/-/19396
<400> 117
   agcaccacct aaattgcatc 20
<210> 118
   <211> 20
   <212> DNA
   <213> Artificial sequence
<220>
   <223> amorce S/L11/-/20002
<400> 118
   tggtcccttt gaaggtgtta 20
<210> 119
   <211> 20
   <212> DNA
   <213> Artificial sequence
<220>
   <223> amorce S/L11/+/20245
<400> 119
   tcgaacacat cgtttatgga 20
<210> 120
   <211> 20
   <212> DNA
   <213> Artificial sequence
<220>
   <223> amorce S/L11/+/19611
<400> 120
   gaagcacctg tttccatcat 20
<210> 121
   <211> 20
   <212> DNA
   <213> Artificial sequence
<220>
   <223> amorce S/L11/+/19021
<400> 121
   acgatgctca gccatgtagt 20
<210> 122
   <211> 20
   <212> DNA
   <213> Artificial sequence
<220>
   <223> amorce SARS/L1/F3/+/800
<400> 122
   gaggtgcagt cactcgctat 20
<210> 123
   <211> 20
   <212> DNA
   <213> Artificial sequence
<220>
   <223> amorce SARS/L1/F4/+/1391
<400> 123
   cagagattgg acctgagcat 20
<210> 124
   <211> 20
   <212> DNA
   <213> Artificial sequence
<220>
   <223> amorce SARS/L1/F5/+/1925
<400> 124
   cagcaaacca ctcaattcct 20
<210> 125
   <211> 20
   <212> DNA
   <213> Artificial sequence
<220>
   <223> amorce SARS/L1/R3/-/1674
<400> 125
   aaatgatggc aacctcttca 20
<210> 126
   <211> 20
   <212> DNA
   <213> Artificial sequence
<220>
   <223> amorce SARS/L1/R4/-/1107
<400> 126
   cacgtggttg aatgactttg 20
<210> 127
   <211> 20
   <212> DNA
   <213> Artificial sequence
<220>
   <223> amorce SARS/L1/R5/-/520
<400> 127
   atttctgcaa ccagctcaac 20
<210> 128
   <211> 20
   <212> DNA
   <213> Artificial sequence
<220>
   <223> amorce SARS/L2/F3/+/2664
<400> 128
   cgcattgtct cctggtttac 20
<210> 129
   <211> 20
   <212> DNA
   <213> Artificial sequence
<220>
   <223> amorce SARS/L2/F4/+/3232
<400> 129
   gagattgagc cagaaccaga 20
<210> 130
   <211> 20
   <212> DNA
   <213> Artificial sequence
<220>
   <223> amorce SARS/L2/F5/+/3746
<400> 130
   atgagcaggt tgtcatggat 20
<210> 131
   <211> 20
   <212> DNA
   <213> Artificial sequence
<220>
   <223> amorce SARS/L2/R3/-/3579
<400> 131
   ctgccttaag aagctggatg 20
<210> 132
   <211> 20
   <212> DNA
   <213> Artificial sequence
<220>
   <223> amorce SARS/L2/R4/-/2991
<400> 132
   tttcttcacc agcatcatca 20
<210> 133
   <211> 20
   <212> DNA
   <213> Artificial sequence
<220>
   <223> amorce SARS/L2/R5/-/2529
<400> 133
   caccgttctt gagaacaacc 20
<210> 134
   <211> 20
   <212> DNA
   <213> Artificial sequence
<220>
   <223> amorce SARS/L3/F3/+/4708
<400> 134
   tctttggctg gctcttacag 20
<210> 135
   <211> 20
   <212> DNA
   <213> Artificial sequence
<220>
   <223> amorce SRAS/L3/F4/+/5305
<400> 135
   gctggtgatg ctgctaactt 20
<210> 136
   <211> 20
   <212> DNA
   <213> Artificial sequence
<220>
   <223> amorce SARS/L3/F5/+/5822
<400> 136
   ccatcaagcc tgtgtcgtat 20
<210> 137
   <211> 20
   <212> DNA
   <213> Artificial sequence
<220>
   <223> amorce SARS/L3/R3/-/5610
<400> 137
   caggtggtgc agacatcata 20
<210> 138
   <211> 20
   <212> DNA
   <213> Artificial sequence
<220>
   <223> amorce SARS/L3/R4/-/4988
<400> 138
   aacatcagca ccatccaagt 20
<210> 139
   <211> 20
   <212> DNA
   <213> Artificial sequence
<220>
   <223> amorce SARS/L3/R5/-/4437
<400> 139
   atcggacacc atagtcaacg 20
<210> 140
   <211> 7788
   <212> DNA
   <213> Artificial sequence
<220>
   <223> gène s synthétique
<400> 140
   tcaatattgg ccattagcca tattattcat tggttatata gcataaatca atattggcta     60
   ttggccattg catacgttgt atctatatca taatatgtac atttatattg gctcatgtcc    120
   aatatgaccg ccatgttggc attgattatt gactagttat taatagtaat caattacggg    180
   gtcattagtt catagcccat atatggagtt ccgcgttaca taacttacgg taaatggccc    240
   gcctggctga ccgcccaacg acccccgccc attgacgtca ataatgacgt atgttcccat    300
   agtaacgcca atagggactt tccattgacg tcaatgggtg gagtatttac ggtaaactgc    360
   ccacttggca gtacatcaag tgtatcatat gccaagtccg ccccctattg acgtcaatga    420
   cggtaaatgg cccgcctggc attatgccca gtacatgacc ttacgggact ttcctacttg    480
   gcagtacatc tacgtattag tcatcgctat taccatggtg atgcggtttt ggcagtacac    540
   caatgggcgt ggatagcggt ttgactcacg gggatttcca agtctccacc ccattgacgt    600
   caatgggagt ttgttttggc accaaaatca acgggacttt ccaaaatgtc gtaataaccc    660
   cgccccgttg acgcaaatgg gcggtaggcg tgtacggtgg gaggtctata taagcagagc    720
   tcgtttagtg aaccgtcaga tcactagaag ctttattgcg gtagtttatc acagttaaat    780
   tgctaacgca gtcagtgctt ctgacacaac agtctcgaac ttaagctgca gaagttggtc    840
   gtgaggcact gggcaggtaa gtatcaaggt tacaagacag gtttaaggag accaatagaa    900
   actgggcttg tcgagacaga gaagactctt gcgtttctga taggcaccta ttggtcttac 960
   tgacatccac tttgcctttc tctccacagg tgtccactcc cagttcaatt acagctctta 1020
   aggctagagt acttaatacg actcactata ggctagcgga tccaccatgt tcatcttcct 1080
   gctgttcctg accctgacca gcggcagcga cctggaccgg tgcaccacct tcgacgacgt 1140
   gcaggccccc aactacaccc agcacaccag cagcatgcgg ggcgtgtact accccgacga 1200
   gatctttcgg agcgacaccc tgtacctgac ccaggacctg ttcctgccct tctacagcaa 1260
   cgtgaccggc ttccacacca tcaaccacac cttcggcaac cccgtgatcc ccttcaagga 1320
   cggcatctac ttcgccgcca ccgagaagag caacgtggtg cggggctggg tgttcggcag 1380
   caccatgaac aacaagagcc agagcgtgat catcatcaac aacagcacca acgtggtgat 1440
   ccgggcctgc aacttcgagc tgtgcgacaa ccccttcttc gccgtgtcca aacccatggg 1500
   cacccagacc cacaccatga tcttcgacaa cgccttcaac tgcaccttcg agtacatcag 1560
   cgacgccttc agcctggacg tgagcgagaa gagcggcaac ttcaagcacc tgcgggagtt 1620
   cgtgttcaag aacaaggacg gcttcctgta cgtgtacaag ggctaccagc ccatcgacgt 1680
   ggtgagagac ctgcccagcg gcttcaacac cctgaagccc atcttcaagc tgcccctggg 1740
   catcaacatc accaacttcc gggccatcct gaccgccttt agccctgccc aggacatctg 1800
   gggcaccagc gccgccgcct acttcgtggg ctacctgaag cctaccacct tcatgctgaa 1860
   gtacgacgag aacggcacca tcaccgacgc cgtggactgc agccagaacc ccctggccga 1920
   gctgaagtgc agcgtgaaga gcttcgagat cgacaagggc atctaccaga ccagcaactt 1980
   cagagtggtg cctagcggcg atgtggtgcg gttccccaat atcaccaacc tgtgcccctt 2040
   cggcgaagtg ttcaacgcca ccaagttccc cagcgtgtac gcctgggagc ggaagaagat 2100
   cagcaactgc gtggccgact acagcgtgct gtacaactcc accttcttca gcaccttcaa 2160
   gtgctacggc gtgagcgcca ccaagctgaa cgacctgtgc ttcagcaacg tgtacgccga 2220
   cagcttcgtg gtgaagggcg acgacgtgag acagatcgcc cctggccaga ccggcgtgat 2280
   cgccgactac aactacaagc tgcccgacga cttcatgggc tgcgtgctgg cctggaacac 2340
   ccggaacatc gacgccacaa gcaccggcaa ctacaattac aagtaccgct acctgcggca 2400
   cggcaagctg cggcccttcg agcgggacat ctccaacgtg cccttcagcc ccgacggcaa 2460
   gccctgcacc ccccctgccc tgaactgcta ctggcccctg aacgactacg gcttctacac 2520
   caccaccggc atcggctatc agccctacag agtggtggtg ctgagcttcg agctgctgaa 2580
   cgcccctgcc accgtgtgcg gccccaagct gagcaccgac ctgatcaaga accagtgcgt 2640
   gaacttcaac ttcaacggcc tgaccggcac cggcgtgctg acccccagca gcaagcgctt 2700
   ccagcccttc cagcagttcg gccgggatgt gagcgacttc accgacagcg tgcgggaccc 2760
   caagaccagc gagatcctgg acatcagccc ctgcagcttc ggcggcgtgt ccgtgatcac 2820
   ccccggcacc aacgccagca gcgaagtggc cgtgctgtac caggacgtga actgcaccga 2880
   cgtgagcacc gccatccacg ccgaccagct gacccccgcc tggcggatct acagcaccgg 2940
   gaacaacgtg ttccagaccc aggccggctg cctgatcggc gccgagcacg tggacaccag 3000
   ctacgagtgc gacatcccca ttggcgccgg aatctgcgcc agctaccaca ccgtgagcct 3060
   gctgcggagc accagccaga agtccatcgt ggcctacacc atgagcctgg gcgccgacag 3120
   cagcatcgcc tacagcaaca acaccatcgc catccccacc aacttcagca tctccatcac 3180
   caccgaagtg atgcccgtga gcatggccaa gacaagcgtg gattgcaaca tgtacatctg 3240
   cggcgacagc accgagtgcg ccaacctgct gctgcagtac ggcagcttct gcacccagct 3300
   gaaccgggcc ctgagcggca tcgccgccga gcaggaccgg aacaccagag aagtgttcgc 3360
   ccaagtgaag cagatgtata agacccccac cctgaagtac ttcgggggct tcaacttctc 3420
   tcagatcctg cccgaccctc tgaagcccac caagcgctcc ttcatcgagg acctgctgtt 3480
   caacaaagtg accctggccg acgccggctt tatgaagcag tacggcgagt gcctgggcga 3540
   catcaacgcc cgggacctga tctgcgccca gaagtttaac gggctgaccg tgctgccccc 3600
   cctgctgacc gacgacatga tcgccgccta tacagccgcc ctggtgagcg gcaccgccac 3660
   cgccggctgg accttcggag ccggagccgc cctgcagatc cccttcgcca tgcagatggc 3720
   ctaccggttc aacggcatcg gcgtgaccca gaacgtgctg tacgagaacc agaagcagat 3780
   cgccaaccag ttcaacaagg ccatcagcca gatccaggag agcctgacca caaccagcac 3840
   cgccctgggc aagctgcagg acgtggtgaa ccagaacgcc caggccctga acaccctggt 3900
   gaagcagctg agcagcaact tcggcgccat cagctctgtg ctgaacgaca tcctgagcag 3960
   gctggacaaa gtggaggccg aagtgcagat cgaccggctg atcaccggac gcctgcagtc 4020
   cctgcagacc tacgtgaccc agcagctgat cagagccgcc gagatccggg ccagcgccaa 4080
   tctggccgcc accaagatga gcgagtgcgt gctgggccag agcaagagag tggacttctg 4140
   cggcaagggc tatcacctga tgagcttccc ccaggccgcc ccccacggcg tggtgttcct 4200
   gcacgtgacc tacgtgccta gccaggagcg gaacttcacc accgccccag ccatctgcca 4260
   cgagggcaag gcctacttcc cccgggaggg cgtgttcgtg tttaacggca ccagctggtt 4320
   catcacccag cgcaacttct tcagccccca gatcatcacc acagacaaca ccttcgtgtc 4380
   cggcaactgt gatgtggtga tcggcatcat caataacacc gtgtacgacc ccctgcagcc 4440
   cgagctggac agcttcaagg aggagctgga caaatacttc aagaaccaca cctcccccga 4500
   cgtggacctg ggcgatatca gcggcatcaa cgcctccgtg gtgaacatcc agaaggagat 4560
   cgacagactg aacgaagtgg ccaagaacct gaacgagagc ctgatcgacc tgcaggagct 4620
   gggcaagtac gagcagtaca tcaagtggcc ctggtacgtg tggctgggct tcatcgccgg 4680
   cctgatcgcc atcgtgatgg tgaccatcct gctgtgctgc atgaccagct gctgtagctg 4740
   cctgaaaggc gcctgcagct gtggcagctg ctgcaagttc gacgaggacg acagcgagcc 4800
   cgtgctgaag ggcgtgaagc tgcactacac ctgataactc gagaattcac gcgtggtacc 4860
   tctagagtcg acccgggcgg ccgcttcgag cagacatgat aagatacatt gatgagtttg 4920
   gacaaaccac aactagaatg cagtgaaaaa aatgctttat ttgtgaaatt tgtgatgcta 4980
   ttgctttatt tgtaaccatt ataagctgca ataaacaagt taacaacaac aattgcattc 5040
   attttatgtt tcaggttcag ggggagatgt gggaggtttt ttaaagcaag taaaacctct 5100
   acaaatgtgg taaaatcgat aaggatccgg gctggcgtaa tagcgaagag gcccgcaccg 5160
   atcgcccttc ccaacagttg cgcagcctga atggcgaatg gacgcgccct gtagcggcgc 5220
   attaagcgcg gcgggtgtgg tggttacgcg cagcgtgacc gctacacttg ccagcgccct 5280
   agcgcccgct cctttcgctt tcttcccttc ctttctcgcc acgttcgccg gctttccccg 5340
   tcaagctcta aatcgggggc tccctttagg gttccgattt agagctttac ggcacctcga 5400
   ccgcaaaaaa cttgatttgg gtgatggttc acgtagtggg ccatcgccct gatagacggt 5460
   ttttcgccct ttgacgttgg agtccacgtt ctttaatagt ggactcttgt tccaaactgg 5520
   aacaacactc aaccctatct cggtctattc ttttgattta taagggattt tgccgatttc 5580
   ggcctattgg ttaaaaaatg agctgattta acaaatattt aacgcgaatt ttaacaaaat 5640
   attaacgttt acaatttcgc ctgatgcggt attttctcct tacgcatctg tgcggtattt 5700
   cacaccgcat atggtgcact ctcagtacaa tctgctctga tgccgcatag ttaagccagc 5760
   cccgacaccc gccaacaccc gctgacgcgc cctgacgggc ttgtctgctc ccggcatccg 5820
   cttacagaca agctgtgacc gtctccggga gctgcatgtg tcagaggttt tcaccgtcat 5880
   caccgaaacg cgcgagacga aagggcctcg tgatacgcct atttttatag gttaatgtca 5940
   tgataataat ggtttcttag acgtcaggtg gcacttttcg gggaaatgtg cgcggaaccc 6000
   ctatttgttt atttttctaa atacattcaa atatgtatcc gctcatgaga caataaccct 6060
   gataaatgct tcaataatat tgaaaaagga agagtatgag tattcaacat ttccgtgtcg 6120
   cccttattcc cttttttgcg gcattttgcc ttcctgtttt tgctcaccca gaaacgctgg 6180
   tgaaagtaaa agatgctgaa gatcagttgg gtgcacgagt gggttacatc gaactggatc 6240
   tcaacagcgg taagatcctt gagagttttc gccccgaaga acgttttcca atgatgagca 6300
   cttttaaagt tctgctatgt ggcgcggtat tatcccgtat tgacgccggg caagagcaac 6360
   tcggtcgccg catacactat tctcagaatg acttggttga gtactcacca gtcacagaaa 6420
   agcatcttac ggatggcatg acagtaagag aattatgcag tgctgccata accatgagtg 6480
   ataacactgc ggccaactta cttctgacaa cgatcggagg accgaaggag ctaaccgctt 6540
   ttttgcacaa catgggggat catgtaactc gccttgatcg ttgggaaccg gagctgaatg 6600
   aagccatacc aaacgacgag cgtgacacca cgatgcctgt agcaatggca acaacgttgc 6660
   gcaaactatt aactggcgaa ctacttactc tagcttcccg gcaacaatta atagactgga 6720
   tggaggcgga taaagttgca ggaccacttc tgcgctcggc ccttccggct ggctggttta 6780
   ttgctgataa atctggagcc ggtgagcgtg ggtctcgcgg tatcattgca gcactggggc 6840
   cagatggtaa gccctcccgt atcgtagtta tctacacgac ggggagtcag gcaactatgg 6900
   atgaacgaaa tagacagatc gctgagatag gtgcctcact gattaagcat tggtaactgt 6960
   cagaccaagt ttactcatat atactttaga ttgatttaaa acttcatttt taatttaaaa 7020
   ggatctaggt gaagatcctt tttgataatc tcatgaccaa aatcccttaa cgtgagtttt 7080
   cgttccactg agcgtcagac cccgtagaaa agatcaaagg atcttcttga gatccttttt 7140
   ttctgcgcgt aatctgctgc ttgcaaacaa aaaaaccacc gctaccagcg gtggtttgtt 7200
   tgccggatca agagctacca actctttttc cgaaggtaac tggcttcagc agagcgcaga 7260
   taccaaatac tgtccttcta gtgtagccgt agttaggcca ccacttcaag aactctgtag 7320
   caccgcctac atacctcgct ctgctaatcc tgttaccagt ggctgctgcc agtggcgata 7380
   agtcgtgtct taccgggttg gactcaagac gatagttacc ggataaggcg cagcggtcgg 7440
   gctgaacggg gggttcgtgc acacagccca gcttggagcg aacgacctac accgaactga 7500
   gatacctaca gcgtgagcta tgagaaagcg ccacgcttcc cgaagggaga aaggcggaca 7560
   ggtatccggt aagcggcagg gtcggaacag gagagcgcac gagggagctt ccagggggaa 7620
   acgcctggta tctttatagt cctgtcgggt ttcgccacct ctgacttgag cgtcgatttt 7680
   tgtgatgctc gtcagggggg cggagcctat ggaaaaacgc cagcaacgcg gcctttttac 7740
   ggttcctggc cttttgctgg ccttttgctc acatggctcg acagatct 7788
<210> 141
   <211> 23
   <212> DNA
   <213> Artificial sequence
<220>
   <223> amorce SNE-S1
<400> 141
   ggttgggatt atccaaaatg tga 23
<210> 142
   <211> 24
   <212> DNA
   <213> Artificial sequence
<220>
   <223> amorce SNE-AS1
<400> 142
   gcatcatcag aaagaatcat catg 24
<210> 143
   <211> 21
   <212> DNA
   <213> Artificial sequence
<220>
   <223> amorce SAR1-s
<400> 143
   cctctcttgt tcttgctcgc a 21
<210> 144
   <211> 21
   <212> DNA
   <213> Artificial sequence
<220>
   <223> amorce SAR1-AS
<400> 144
   tatagtgagc cgccacacat g 21
<210> 145
   <211> 45
   <212> DNA
   <213> Artificial sequence
<220>
   <223> amorce PCR
<400> 145
   ataggatcca ccatgtttat tttcttatta tttcttactc tcact 45
<210> 146
   <211> 37
   <212> DNA
   <213> Artificial sequence
<220>
   <223> amorce PCR
<400> 146
   atactcgagt tatgtgtaat gtaatttgac acccttg 37
<210> 147
   <211> 45
   <212> DNA
   <213> Artificial sequence
<220>
   <223> amorce PCR
<400> 147
   ataggatcca ccatgtttat tttcttatta tttcttactc tcact 45
<210> 148
   <211> 36
   <212> DNA
   <213> Artificial sequence
<220>
   <223> amorce PCR
<400> 148
   acctccggat ttaatatatt gctcatattt tcccaa 36
<210> 149
   <211> 13
   <212> PRT
   <213> Artificial sequence
<220>
   <223> extrémité N-terminale de la protéine s du SRAS-CoV (acides amines 1 à 13)
<400> 149
   Met Phe Ile Phe Leu Leu Phe Leu Thr Leu Thr Ser Gly
   1 5 10
<210> 150
   <211> 10
   <212> PRT
   <213> Artificial sequence
<220>
   <223> oligopeptide
<400> 150
   Ser Gly Asp Tyr Lys Asp Asp Asp Asp Lys
   1 5 10
<210> 151
   <211> 34
   <212> DNA
   <213> Artificial sequence
<220>
   <223> amorce PCR
<400> 151
   actagctagc ggatccacca tgttcatctt cctg 34
<210> 152
   <211> 33
   <212> DNA
   <213> Artificial sequence
<220>
   <223> amorce PCR
<400> 152
   agtatccgga cttgatgtac tgctcgtact tgc 33
<210> 153
   <211> 59
   <212> DNA
   <213> Artificial sequence
<220>
   <223> oligonucléotide
<400> 153
   tatgagcttt tttttttttt tttttttggc atataaatag actcggcgcg ccatctgca 59
<210> 154
   <211> 53
   <212> DNA
   <213> Artificial sequence
<220>
   <223> oligonucléotide
<400> 154
   gatggcgcgc cgagtctatt tatatgccaa aaaaaaaaaa aaaaaaaagc tca 53
<210> 155
   <211> 45
   <212> DNA
   <213> Artificial sequence
<220>
   <223> amorce PCR
<400> 155
   atacgtacga ccatgtttat tttcttatta tttcttactc tcact 45
<210> 156
   <211> 40
   <212> DNA
   <213> Artificial sequence
<220>
   <223> amorce PCR
<400> 156
   atagcgcgct cattatgtgt aatgtaattt gacacccttg 40
<210> 157
   <211> 20
   <212> DNA
   <213> Artificial sequence
<220>
   <223> amorce PCR
<400> 157
   ccatttcaac aatttggccg 20
<210> 158
   <211> 45
   <212> DNA
   <213> Artificial sequence
<220>
   <223> amorce PCR
<400> 158
   ataggatccg cgcgctcatt atttatcgtc gtcatcttta taatc 45